(19) Europäisches Patentamt
European Patent Office
Office européen des brevets

(11) EP 4 617 371 A1

(12) # EUROPEAN PATENT APPLICATION
published in accordance with Art. 153(4) EPC

(43) Date of publication:
**17.09.2025 Bulletin 2025/38**

(21) Application number: **23900635.6**

(22) Date of filing: **05.12.2023**

(51) International Patent Classification (IPC):
***C12Q 1/00*** *(2006.01)* ***C12M 1/34*** *(2006.01)*
***C12Q 1/25*** *(2006.01)* ***C12Q 1/37*** *(2006.01)*
***C12Q 1/44*** *(2006.01)* ***G01N 21/64*** *(2006.01)*
***G01N 33/50*** *(2006.01)* ***G01N 33/53*** *(2006.01)*

(52) Cooperative Patent Classification (CPC):
**C12M 1/34; C12Q 1/00; C12Q 1/25; C12Q 1/37; C12Q 1/44; G01N 21/64; G01N 33/50; G01N 33/53**

(86) International application number:
**PCT/JP2023/043377**

(87) International publication number:
**WO 2024/122520 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.12.2022 JP 2022195994**
**06.07.2023 JP 2023111738**
**30.11.2023 JP 2023203313**

(71) Applicant: **CANON KABUSHIKI KAISHA**
**Tokyo 146-8501 (JP)**

(72) Inventors:
- **YANO, Tetsuya**
  **Tokyo 146-8501 (JP)**
- **OGAWA, Satoshi**
  **Tokyo 146-8501 (JP)**
- **TORII, Sota**
  **Tokyo 146-8501 (JP)**
- **BABA, Kyoko**
  **Tokyo 146-8501 (JP)**

(74) Representative: **WESER & Kollegen Patentanwälte PartmbB**
**Radeckestraße 43**
**81245 München (DE)**

(54) **METHOD, KIT AND DEVICE FOR DETECTING EXTRACELLULAR VESICLES**

(57) An object of the present invention is to simply detect an extracellular vesicle with high sensitivity. Specifically, provided is a method of detecting an extracellular vesicle having a target enzyme, the method including: a distribution step of distributing an extracellular vesicle and a reagent containing a reporter molecule to be modified by the target enzyme to emit a signal into a plurality of individual separated compartments; a signal generation step of allowing the target enzyme and the reporter molecule to react with each other to generate a signal; a signal detection step of detecting the signal; and an identification step including determining, based on a detection result obtained in the signal detection step, a signal intensity of each of the individual separated compartments, and identifying each of the individual separated compartments having a signal intensity exceeding a predetermined threshold value.

EP 4 617 371 A1

FIG. 1

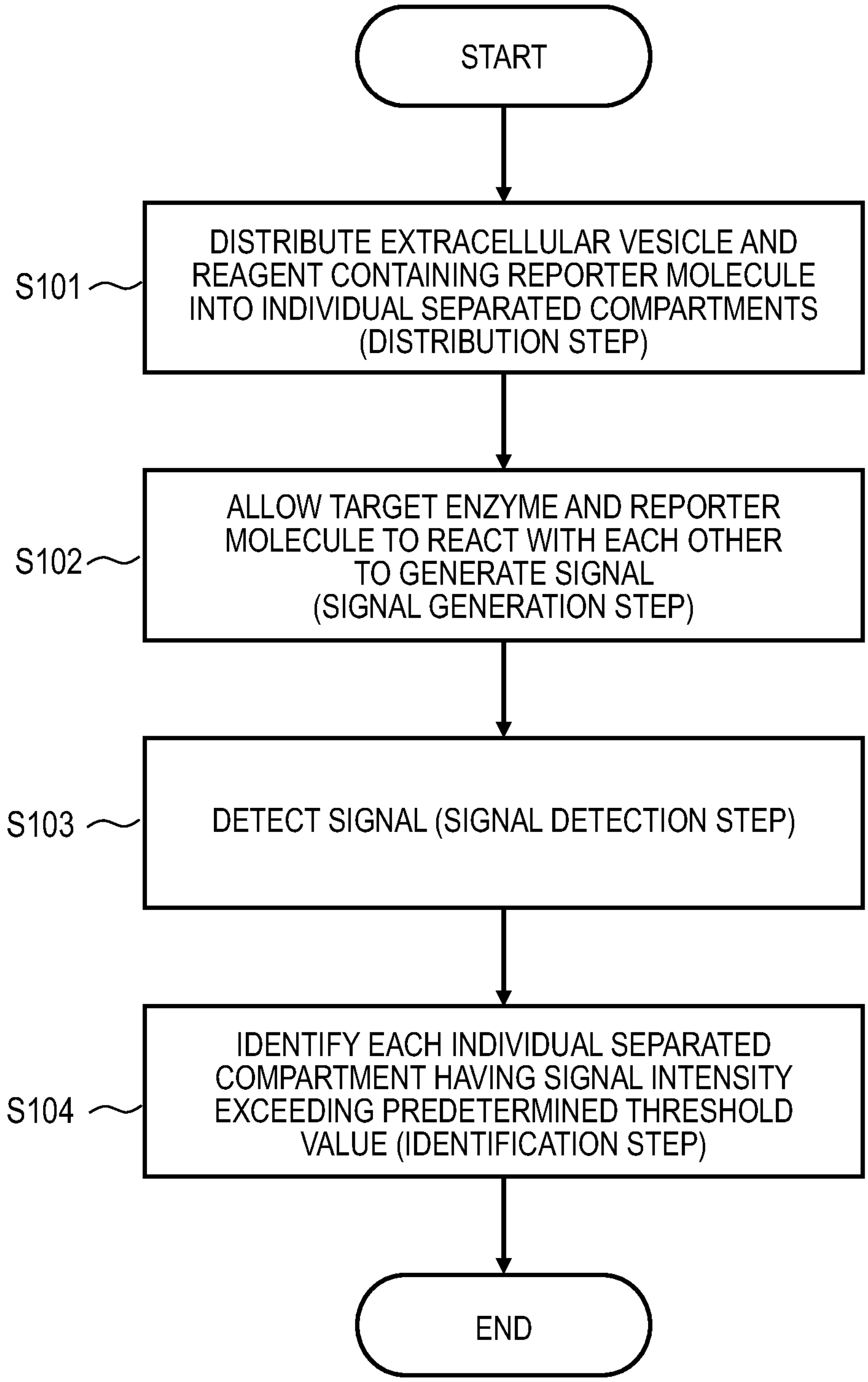
START
S101
DISTRIBUTE EXTRACELLULAR VESICLE AND REAGENT CONTAINING REPORTER MOLECULE INTO INDIVIDUAL SEPARATED COMPARTMENTS (DISTRIBUTION STEP)
S102
ALLOW TARGET ENZYME AND REPORTER MOLECULE TO REACT WITH EACH OTHER TO GENERATE SIGNAL (SIGNAL GENERATION STEP)
S103
DETECT SIGNAL (SIGNAL DETECTION STEP)
S104
IDENTIFY EACH INDIVIDUAL SEPARATED COMPARTMENT HAVING SIGNAL INTENSITY EXCEEDING PREDETERMINED THRESHOLD VALUE (IDENTIFICATION STEP)
END

## Description

[Technical Field]

The present invention relates to a method, kit, apparatus, and program for detecting an extracellular vesicle.

[Background Art]

Extracellular vesicles are the generic term for nucleus-free vesicles that are secreted from cells and have a lipid bilayer structure, and are broadly classified, based on differences in their intracellular production mechanisms, into three kinds: an exosome, a microvesicle, and an apoptosis body. Attention has been paid to the extracellular vesicles serving as intercellular communication tools for delivering a functional molecule having physiological activity (e.g., a protein and a nucleic acid) from cells to other cells, the extracellular vesicles are also present in body fluids (e.g., blood, urine, and spinal fluid), and hence medical application research for the application to liquid biopsy is progressing.

Vinita Gupta et al. at Genentech, Inc. have disclosed assays for the detection and/or quantification of membrane proteins, for example, circulating CD20 (cCD20), incorporating an extracellular vesicle-based calibrator including a membrane-associated tumor antigen, and the use of such assays in the detection and treatment of hyperproliferative disorders (Patent Literature 1). In this literature, there is a disclosure of an ELISA assay using a combination of a capture antibody that binds to an extracellular vesicle including a membrane protein, to thereby generate a capture antibody-extracellular vesicle complex and is immobilized on a solid phase, and a detection antibody that binds to the capture antibody-extracellular vesicle complex, to thereby form a detectable bound complex.

[Citation List]

[Patent Literature]

PTL 1: Japanese Patent Laid-Open No. 2022-524327

[Summary of Invention]

[Technical Problem]

In Patent Literature 1, there is a disclosure that the presence of a membrane protein serving as a detection target is detected by an ELISA assay for an extracellular vesicle. However, an assay that, when the detection target is an enzyme, detects the presence of an active enzyme utilizing the activity of the enzyme, and an assay that detects an extracellular vesicle in which the active enzyme is present are not disclosed.

An object of the present invention is to simply detect an extracellular vesicle having enzymatic activity with high sensitivity by detecting the activity of an enzyme molecule included in the extracellular vesicle.

[Solution to Problem]

There is provided a method of detecting an extracellular vesicle having a target enzyme, the method including: a distribution step of distributing an extracellular vesicle and a reporter molecule to be modified by the target enzyme to emit a signal into a plurality of individual separated compartments; a signal generation step of allowing the target enzyme and the reporter molecule to react with each other to generate a signal; a signal detection step of detecting the signal; and an identification step including determining, based on a detection result obtained in the signal detection step, a signal intensity of each of the individual separated compartments, and identifying each of the individual separated compartments having a signal intensity exceeding a predetermined threshold value.

There is provided a reagent kit for detecting an extracellular vesicle having a target enzyme in an individual separated compartment, the reagent kit including: a reagent containing a reporter molecule to be modified by the target enzyme to emit a signal; and a container for providing a plurality of individual separated compartments.

There is provided an apparatus for detecting an extracellular vesicle having a target enzyme, the apparatus including: a distribution unit configured to distribute an extracellular vesicle and a reagent containing a reporter molecule to be modified by the target enzyme to emit a signal into a plurality of individual separated compartments; a signal generation unit configured to allow the target enzyme and the reporter molecule to react with each other to generate a signal; a signal detection unit configured to detect the signal; and an identification unit configured to determine, based on a detection result obtained with the signal detection unit, a signal intensity of each of the individual separated compartments, and identify each of the individual separated compartments having a signal intensity exceeding a predetermined threshold value.

[Advantageous Effects of Invention]

According to the present invention, it is possible to provide the detection method, kit, apparatus, and program that easily detect an extracellular vesicle having enzymatic activity by detecting the activity of an enzyme included in the extracellular vesicle.

[Brief Description of Drawings]

[FIG. 1]
FIG. 1 is a flow chart for illustrating a flow of a method of detecting an extracellular vesicle of an embodiment of the present invention.
[FIG. 2]
FIG. 2 is a flow chart for illustrating a flow of the method of detecting an extracellular vesicle in the case of using liquid droplets as individual separated compartments.
[FIG. 3]
FIG. 3 is a flow chart for illustrating a flow of the method of detecting an extracellular vesicle in the case of using wells as individual separated compartments.
[FIG. 4]
FIG. 4 is a flow chart for illustrating a flow of the method of detecting an extracellular vesicle in the case of performing background correction through use of a mask image.
[FIG. 5A]
FIG. 5A is a cross-sectional view of a well array.
[FIG. 5B]
FIG. 5B is a cross-sectional view of the well array with its wells filled with a particle complex formed of a capture antibody-immobilized particle and an extracellular vesicle.
[FIG. 6]
FIG. 6 is a function block diagram for illustrating an example of an extracellular vesicle detection apparatus of an embodiment of the present invention.
[FIG. 7]
FIG. 7 is a function block diagram for illustrating an example of an extracellular vesicle detection apparatus of an embodiment of the present invention.
[FIG. 8]
FIG. 8 is a block diagram for illustrating a hardware configuration example of the extracellular vesicle detection apparatus according to the embodiment of the present invention.
[FIG. 9A]
FIG. 9A shows results of Example 1 in which Recombinant human MMP14 protein was measured with SensoLyte 520 MMP-14 Assay Kit through use of a 96-well plate.
[FIG. 9B]
FIG. 9B shows results of Example 1 in which Recombinant human MMP14 protein was measured with SensoLyte 520 MMP-14 Assay Kit through use of a 96-well plate.
[FIG. 10]
FIG. 10 shows results of Example 2 in which MDA-MB-231-derived extracellular vesicles were measured with SensoLyte 520 MMP-14 Assay Kit through use of a 96-well plate.
[FIG. 11]
FIG. 11 shows results of Example 3 in which MDA-MB-231-derived extracellular vesicles were measured with MMP-14 Substrate I through use of a 96-well plate (temporal changes).
[FIG. 12]
FIG. 12 shows results of Example 4 in which MDA-MB-231-derived extracellular vesicles were measured with Custom #1, Custom #2, or SensoLyte 520 MMP14 through use of a 96-well plate (temporal changes).
[FIG. 13A]
FIG. 13A shows results of Example 5 in which MDA-MB-231-derived extracellular vesicles were measured with Custom #1 or Custom #2 through use of a 96-well plate (temporal changes).
[FIG. 13B]
FIG. 13B shows results of Example 5 in which MDA-MB-231-derived extracellular vesicles were measured with Custom #1 or Custom #2 through use of a 96-well plate (temporal changes).
[FIG. 14A]

FIG. 14A shows results of Example 6 in which MDA-MB-231-derived extracellular vesicles were measured with Custom #1 through use of a 96-well plate (investigation of reaction solution composition, temporal changes).

[FIG. 14B]

FIG. 14B shows results of Example 6 in which MDA-MB-231-derived extracellular vesicles were measured with Custom #1 through use of a 96-well plate (investigation of reaction solution composition, temporal changes).

[FIG. 14C]

FIG. 14C shows results of Example 6 in which MDA-MB-231-derived extracellular vesicles were measured with Custom #1 through use of a 96-well plate (investigation of reaction solution composition, temporal changes).

[FIG. 15A]

FIG. 15A shows results of Example 7 in which MDA-MB-231-derived extracellular vesicles were measured with Custom #1 through use of a 96-well plate (investigation of reaction solution composition, temporal changes).

[FIG. 15B]

FIG. 15B shows results of Example 7 in which MDA-MB-231-derived extracellular vesicles were measured with Custom #2 through use of a 96-well plate (investigation of reaction solution composition, temporal changes).

[FIG. 16A]

FIG. 16A shows results of Example 8 in which MDA-MB-231-derived extracellular vesicles were measured with Custom #1 or Custom #2 through use of a 96-well plate (varied extracellular vesicle concentrations, temporal changes).

[FIG. 16B]

FIG. 16B shows results of Example 8 in which MDA-MB-231-derived extracellular vesicles were measured with Custom #1 or Custom #2 through use of a 96-well plate (varied extracellular vesicle concentrations).

[FIG. 17A]

FIG. 17A shows results of Example 9 in which MDA-MB-231-derived extracellular vesicles were measured with Custom #1 through use of a 96-well plate (varied reporter molecule concentrations, temporal changes).

[FIG. 17B]

FIG. 17B shows results of Example 9 in which MDA-MB-231-derived extracellular vesicles were measured with Custom #2 through use of a 96-well plate (varied reporter molecule concentrations, temporal changes).

[FIG. 17C]

FIG. 17C shows results of Example 9 in which MDA-MB-231-derived extracellular vesicles were measured with Custom #1 or Custom #2 through use of a 96-well plate (Lineweaver-Burk plots).

[FIG. 18A]

FIG. 18A shows results of Example 10 in which MDA-MB-231-derived extracellular vesicles were measured with Custom #1 or Custom #2 through use of a 96-well plate (temporal changes).

[FIG. 18B]

FIG. 18B shows results of Example 10 in which A549-derived extracellular vesicles were measured with Custom #1 or Custom #2 through use of a 96-well plate (temporal changes).

[FIG. 18C]

FIG. 18C shows results of Example 10 in which COLO201-derived extracellular vesicles were measured with Custom #1 or Custom #2 through use of a 96-well plate (temporal changes).

[FIG. 19]

FIG. 19 shows results of Example 11 in which AChE was measured with Amplite Fluorimetric Acetylcholinesterase Assay Kit through use of a 96-well plate (varied AChE concentrations, temporal changes).

[FIG. 20A]

FIG. 20A shows results of Example 12 in which MDA-MB-231-derived extracellular vesicles were measured with Amplite Fluorimetric Acetylcholinesterase Assay Kit through use of a 96-well plate (temporal changes).

[FIG. 20B]

FIG. 20B shows results of Example 12 in which A549-derived extracellular vesicles were measured with Amplite Fluorimetric Acetylcholinesterase Assay Kit through use of a 96-well plate (temporal changes).

[FIG. 21]

FIG. 21 shows results of Example 13 in which MDA-MB-231-derived extracellular vesicles were measured with Amplite Fluorimetric Acetylcholinesterase Assay Kit through use of a 96-well plate (varied reporter molecule concentrations, temporal changes).

[FIG. 22]

FIG. 22 shows results of Example 14 in which AChE was measured with SensoLyte 520 Acetylcholinesterase Activity Assay Kit through use of a 96-well plate (varied AChE concentrations, temporal changes).

[FIG. 23A]

FIG. 23A shows results of Example 15 in which MDA-MB-231-derived extracellular vesicles were measured with SensoLyte 520 Acetylcholinesterase Activity Assay Kit through use of a 96-well plate (temporal changes).

[FIG. 23B]
FIG. 23B shows results of Example 15 in which A549-derived extracellular vesicles were measured with SensoLyte 520 Acetylcholinesterase Activity Assay Kit through use of a 96-well plate (temporal changes).
[FIG. 24]
FIG. 24 shows results of Example 16 in which MDA-MB-231-derived extracellular vesicles were measured with SensoLyte 520 Acetylcholinesterase Activity Assay Kit through use of a 96-well plate (varied reporter molecule concentrations, temporal changes).
[FIG. 25A]
FIG. 25A shows results of Example 17 in which MMP14 was measured with Custom #1 through use of a 96-well plate (varied buffers, temporal changes).
[FIG. 25B]
FIG. 25B shows results of Example 17 in which MMP14 was measured with Custom #2 through use of a 96-well plate (varied buffers, temporal changes).
[FIG. 25C]
FIG. 25C shows results of Example 17 in which AChE was measured with Amplite Fluorimetric Acetylcholinesterase Assay Kit through use of a 96-well plate (varied buffers, temporal changes).
[FIG. 25D]
FIG. 25D shows results of Example 17 in which AChE was measured with SensoLyte 520 Acetylcholinesterase Activity Assay Kit through use of a 96-well plate (varied buffers, temporal changes).
[FIG. 26A]
FIG. 26A shows results of Example 18 in which MDA-MB-231-derived extracellular vesicles were measured with Custom #2 through use of a 96-well plate (temporal change).
[FIG. 26B]
FIG. 26B shows a fluorescence microscope image of wells in Example 18 (sealed with AsahiKlin AE-3000 and Fomblin Y25 serving as hydrophobic solvents).
[FIG. 26C]
FIG. 26C shows a fluorescence microscope image of wells in Example 18 (sealed with Simoa SR-X Sealing Oil serving as a hydrophobic solvent).
[FIG. 26D]
FIG. 26D shows a histogram obtained from the fluorescence microscope image of wells in Example 18 (sealed with AsahiKlin AE-3000 and Fomblin Y25 serving as hydrophobic solvents).
[FIG. 26E]
FIG. 26E shows a histogram obtained from the fluorescence microscope image of wells in Example 18 (sealed with Simoa SR-X Sealing Oil serving as a hydrophobic solvent).
[FIG. 27]
FIG. 27 shows results (fluorescence intensities from a reporter molecule, temporal changes) of Example 19 in which MDA-MB-231-derived extracellular vesicles were measured with Custom #2 through use of a 96-well plate.
[FIG. 28A]
FIG. 28A shows a fluorescence microscope image of wells in Example 19 (Custom #2, after 3 hours of reaction).
[FIG. 28B]
FIG. 28B shows a fluorescence microscope image of wells in Example 19 (Custom #2, after 3 hours of reaction).
[FIG. 28C]
FIG. 28C shows a fluorescence microscope image of wells in Example 19 (Custom #2, after 3 hours of reaction).
[FIG. 28D]
FIG. 28D shows a fluorescence microscope image of wells in Example 19 (Custom #2, after 3 hours of reaction).
[FIG. 28E]
FIG. 28E shows a fluorescence microscope image of wells in Example 19 (Custom #2, after 3 hours of reaction).
[FIG. 28F]
FIG. 28F shows a fluorescence microscope image of wells in Example 19 (Custom #2, after 3 hours of reaction).
[FIG. 28G]
FIG. 28G shows a fluorescence microscope image of wells in Example 19 (standard fluorescent substance, after 3 hours of reaction).
[FIG. 28H]
FIG. 28H shows a fluorescence microscope image of wells in Example 19 (standard fluorescent substance, after 3 hours of reaction).
[FIG. 28I]
FIG. 28I shows a fluorescence microscope image of wells in Example 19 (standard fluorescent substance, after 3 hours of reaction).

[FIG. 28J]
FIG. 28J shows a fluorescence microscope image of wells in Example 19 (standard fluorescent substance, after 3 hours of reaction).
[FIG. 28K]
FIG. 28K shows a fluorescence microscope image of wells in Example 19 (standard fluorescent substance, after 3 hours of reaction).
[FIG. 28L]
FIG. 28L shows a fluorescence microscope image of wells in Example 19 (standard fluorescent substance, after 3 hours of reaction).
[FIG. 29A]
FIG. 29A shows a fluorescence microscope image of wells in Example 19 (Custom #2, after 7 hours of reaction).
[FIG. 29B]
FIG. 29B shows a fluorescence microscope image of wells in Example 19 (Custom #2, after 7 hours of reaction).
[FIG. 29C]
FIG. 29C shows a fluorescence microscope image of wells in Example 19 (Custom #2, after 7 hours of reaction).
[FIG. 29D]
FIG. 29D shows a fluorescence microscope image of wells in Example 19 (Custom #2, after 7 hours of reaction).
[FIG. 29E]
FIG. 29E shows a fluorescence microscope image of wells in Example 19 (Custom #2, after 7 hours of reaction).
[FIG. 29F]
FIG. 29F shows a fluorescence microscope image of wells in Example 19 (Custom #2, after 7 hours of reaction).
[FIG. 29G]
FIG. 29G shows a fluorescence microscope image of wells in Example 19 (standard fluorescent substance, after 7 hours of reaction).
[FIG. 29H]
FIG. 29H shows a fluorescence microscope image of wells in Example 19 (standard fluorescent substance, after 7 hours of reaction).
[FIG. 29I]
FIG. 29I shows a fluorescence microscope image of wells in Example 19 (standard fluorescent substance, after 7 hours of reaction).
[FIG. 29J]
FIG. 29J shows a fluorescence microscope image of wells in Example 19 (standard fluorescent substance, after 7 hours of reaction).
[FIG. 29K]
FIG. 29K shows a fluorescence microscope image of wells in Example 19 (standard fluorescent substance, after 7 hours of reaction).
[FIG. 29L]
FIG. 29L shows a fluorescence microscope image of wells in Example 19 (standard fluorescent substance, after 7 hours of reaction).
[FIG. 30A]
FIG. 30A shows a histogram obtained from the fluorescence microscope image of wells in Example 19 (after 3 hours of reaction).
[FIG. 30B]
FIG. 30B shows a histogram obtained from the fluorescence microscope image of wells in Example 19 (after 3 hours of reaction).
[FIG. 30C]
FIG. 30C shows a histogram obtained from the fluorescence microscope image of wells in Example 19 (after 3 hours of reaction).
[FIG. 30D]
FIG. 30D shows a histogram obtained from the fluorescence microscope image of wells in Example 19 (after 3 hours of reaction).
[FIG. 30E]
FIG. 30E shows a histogram obtained from the fluorescence microscope image of wells in Example 19 (after 3 hours of reaction).
[FIG. 31A]
FIG. 31A shows a histogram obtained from the fluorescence microscope image of wells in Example 19 (after 7 hours of reaction).
[FIG. 31B]

FIG. 31B shows a histogram obtained from the fluorescence microscope image of wells in Example 19 (after 7 hours of reaction).
[FIG. 31C]
FIG. 31C shows a histogram obtained from the fluorescence microscope image of wells in Example 19 (after 7 hours of reaction).
[FIG. 31D]
FIG. 31D shows a histogram obtained from the fluorescence microscope image of wells in Example 19 (after 7 hours of reaction).
[FIG. 31E]
FIG. 31E shows a histogram obtained from the fluorescence microscope image of wells in Example 19 (after 7 hours of reaction).
[FIG. 32]
FIG. 32 shows plots of an expected value λ with respect to an extracellular vesicle concentration calculated from the results of Example 19.
[FIG. 33]
FIG. 33 shows results (fluorescence intensities from a reporter molecule, temporal changes) of Example 20 in which MDA-MB-231-derived extracellular vesicles were measured with Custom #1 through use of a 96-well plate.
[FIG. 34A]
FIG. 34A shows a fluorescence microscope image of wells in Example 20 (Custom #1, after 5 hours of reaction).
[FIG. 34B]
FIG. 34B shows a fluorescence microscope image of wells in Example 20 (Custom #1, after 5 hours of reaction).
[FIG. 34C]
FIG. 34C shows a fluorescence microscope image of wells in Example 20 (Custom #1, after 5 hours of reaction).
[FIG. 34D]
FIG. 34D shows a fluorescence microscope image of wells in Example 20 (Custom #1, after 5 hours of reaction).
[FIG. 34E]
FIG. 34E shows a fluorescence microscope image of wells in Example 20 (Custom #1, after 5 hours of reaction).
[FIG. 34F]
FIG. 34F shows a fluorescence microscope image of wells in Example 20 (Custom #1, after 5 hours of reaction).
[FIG. 34G]
FIG. 34G shows a fluorescence microscope image of wells in Example 20 (standard fluorescent substance, after 5 hours of reaction).
[FIG. 34H]
FIG. 34H shows a fluorescence microscope image of wells in Example 20 (standard fluorescent substance, after 5 hours of reaction).
[FIG. 34I]
FIG. 34I shows a fluorescence microscope image of wells in Example 20 (standard fluorescent substance, after 5 hours of reaction).
[FIG. 34J]
FIG. 34J shows a fluorescence microscope image of wells in Example 20 (standard fluorescent substance, after 5 hours of reaction).
[FIG. 34K]
FIG. 34K shows a fluorescence microscope image of wells in Example 20 (standard fluorescent substance, after 5 hours of reaction).
[FIG. 34L]
FIG. 34L shows a fluorescence microscope image of wells in Example 20 (standard fluorescent substance, after 5 hours of reaction).
[FIG. 35A]
FIG. 35A shows a histogram obtained from the fluorescence microscope image of wells in Example 20 (after 5 hours of reaction).
[FIG. 35B]
FIG. 35B shows a histogram obtained from the fluorescence microscope image of wells in Example 20 (after 5 hours of reaction).
[FIG. 35C]
FIG. 35C shows a histogram obtained from the fluorescence microscope image of wells in Example 20 (after 5 hours of reaction).
[FIG. 35D]
FIG. 35D shows a histogram obtained from the fluorescence microscope image of wells in Example 20 (after 5 hours of reaction).

[FIG. 35E]

FIG. 35E shows a histogram obtained from the fluorescence microscope image of wells in Example 20 (after 5 hours of reaction).

[FIG. 35F]

FIG. 35F shows a histogram obtained from the fluorescence microscope image of wells in Example 20 (after 5 hours of reaction).

[FIG. 36]

FIG. 36 shows plots of an expected value λ with respect to an extracellular vesicle concentration calculated from the results of Example 20.

[FIG. 37]

FIG. 37 shows results of Example 21 in whichA-549-derived extracellular vesicles were measured with Custom #1 through use of a 96-well plate (fluorescence intensity from a reporter molecule, temporal changes).

[FIG. 38A]

FIG. 38A shows a histogram obtained from the fluorescence microscope image of wells in Example 21 (after 5 hours of reaction).

[FIG. 38B]

FIG. 38B shows a histogram obtained from the fluorescence microscope image of wells in Example 21 (after 5 hours of reaction).

[FIG. 38C]

FIG. 38C shows a histogram obtained from the fluorescence microscope image of wells in Example 21 (after 5 hours of reaction).

[FIG. 38D]

FIG. 38D shows a histogram obtained from the fluorescence microscope image of wells in Example 21 (after 5 hours of reaction).

[FIG. 38E]

FIG. 38E shows a histogram obtained from the fluorescence microscope image of wells in Example 21 (after 5 hours of reaction).

[FIG. 38F]

FIG. 38F shows a histogram obtained from the fluorescence microscope image of wells in Example 21 (after 5 hours of reaction).

[FIG. 39]

FIG. 39 shows plots of an expected value λ with respect to an extracellular vesicle concentration calculated from the results of Example 21.

[FIG. 40A]

FIG. 40A shows a histogram obtained from the fluorescence microscope image of wells in Example 22 in which MDA-MB-231-derived extracellular vesicles were measured with Custom #1 or Custom #2 through use of Simoa Discs (after 1 hour of reaction, Custom #1).

[FIG. 40B]

FIG. 40B shows a histogram obtained from the fluorescence microscope image of wells in Example 22 (after 1 hour of reaction, Custom #1).

[FIG. 40C]

FIG. 40C shows a histogram obtained from the fluorescence microscope image of wells in Example 22 (after 1 hour of reaction, Custom #1).

[FIG. 40D]

FIG. 40D shows a histogram obtained from the fluorescence microscope image of wells in Example 22 (after 1 hour of reaction, Custom #1).

[FIG. 40E]

FIG. 40E shows a histogram obtained from the fluorescence microscope image of wells in Example 22 (after 2 hours of reaction, Custom #1).

[FIG. 40F]

FIG. 40F shows a histogram obtained from the fluorescence microscope image of wells in Example 22 (after 2 hours of reaction, Custom #1).

[FIG. 40G]

FIG. 40G shows a histogram obtained from the fluorescence microscope image of wells in Example 22 (after 2 hours of reaction, Custom #1).

[FIG. 40H]

FIG. 40H shows a histogram obtained from the fluorescence microscope image of wells in Example 22 (after 2 hours of reaction, Custom #1).

[FIG. 41A]

FIG. 41A shows a histogram obtained from the fluorescence microscope image of wells in Example 22 (after 1 hour of reaction, Custom #2).

[FIG. 41B]

FIG. 41B shows a histogram obtained from the fluorescence microscope image of wells in Example 22 (after 1 hour of reaction, Custom #2).

[FIG. 41C]

FIG. 41C shows a histogram obtained from the fluorescence microscope image of wells in Example 22 (after 1 hour of reaction, Custom #2).

[FIG. 41D]

FIG. 41D shows a histogram obtained from the fluorescence microscope image of wells in Example 22 (after 1 hour of reaction, Custom #2).

[FIG. 41E]

FIG. 41E shows a histogram obtained from the fluorescence microscope image of wells in Example 22 (after 2 hours of reaction, Custom #2).

[FIG. 41F]

FIG. 41F shows a histogram obtained from the fluorescence microscope image of wells in Example 22 (after 2 hours of reaction, Custom #2).

[FIG. 41G]

FIG. 41G shows a histogram obtained from the fluorescence microscope image of wells in Example 22 (after 2 hours of reaction, Custom #2).

[FIG. 41H]

FIG. 40H shows a histogram obtained from the fluorescence microscope image of wells in Example 22 (after 2 hours of reaction, Custom #2).

[FIG. 42A]

FIG. 42A shows plots of an expected value λ with respect to an extracellular vesicle concentration calculated from the results of Example 22 (Custom #1).

[FIG. 42B]

FIG. 42B shows plots of an expected value λ with respect to an extracellular vesicle concentration calculated from the results of Example 22 (Custom #2).

[FIG. 43]

FIG. 43 shows results of Example 23 in which MDA-MB-231-derived extracellular vesicles were measured with SensoLyte 520 MMP-14 Assay Kit through use of a 96-well plate (fluorescence intensity from a reporter molecule, temporal changes).

[FIG. 44A]

FIG. 44A shows a histogram obtained from the fluorescence microscope image of wells in Example 23 (after 1 hour of reaction).

[FIG. 44B]

FIG. 44B shows a histogram obtained from the fluorescence microscope image of wells in Example 23 (after 1 hour of reaction).

[FIG. 44C]

FIG. 44C shows a histogram obtained from the fluorescence microscope image of wells in Example 23 (after 1 hour of reaction).

[FIG. 44D]

FIG. 44D shows a histogram obtained from the fluorescence microscope image of wells in Example 23 (after 1 hour of reaction).

[FIG. 44E]

FIG. 44E shows a histogram obtained from the fluorescence microscope image of wells in Example 23 (after 1 hour of reaction).

[FIG. 45]

FIG. 45 shows plots of an expected value λ with respect to an extracellular vesicle concentration calculated from the results of Example 23.

[FIG. 46]

FIG. 46 shows results of Example 24 in which MDA-MB-231-derived extracellular vesicles were measured with Amplite Fluorimetric Acetylcholinesterase Assay Kit through use of a 96-well plate (fluorescence intensity from a reporter molecule, temporal changes).

[FIG. 47A]

FIG. 47A shows a fluorescence microscope image of wells in Example 24 (after 3 hours of reaction, reporter molecule).

[FIG. 47B]

FIG. 47B shows a fluorescence microscope image of wells in Example 24 (after 3 hours of reaction, reporter molecule).

[FIG. 47C]

FIG. 47C shows a fluorescence microscope image of wells in Example 24 (after 3 hours of reaction, reporter molecule).

[FIG. 47D]

FIG. 47D shows a fluorescence microscope image of wells in Example 24 (after 3 hours of reaction, standard fluorescent substance).

[FIG. 47E]

FIG. 47E shows a fluorescence microscope image of wells in Example 24 (after 3 hours of reaction, standard fluorescent substance).

[FIG. 47F]

FIG. 47F shows a fluorescence microscope image of wells in Example 24 (after 3 hours of reaction, standard fluorescent substance).

[FIG. 48A]

FIG. 48A shows a histogram obtained from the fluorescence microscope image of wells in Example 24 (after 3 hours of reaction, reporter molecule).

[FIG. 48B]

FIG. 48B shows a histogram obtained from the fluorescence microscope image of wells in Example 24 (after 3 hours of reaction, reporter molecule).

[FIG. 48C]

FIG. 48C shows a histogram obtained from the fluorescence microscope image of wells in Example 24 (after 3 hours of reaction, reporter molecule).

[FIG. 49]

FIG. 49 shows plots of an expected value $\lambda$ with respect to an extracellular vesicle concentration calculated from the results of Example 24.

[FIG. 50]

FIG. 50 shows results of Example 25 in which A549-derived extracellular vesicles were measured with Amplite Fluorimetric Acetylcholinesterase Assay Kit through use of a 96-well plate (fluorescence intensity from a reporter molecule, temporal changes).

[FIG. 51A]

FIG. 51A shows a histogram obtained from the fluorescence microscope image of wells in Example 25 (after 1.5 hours of reaction).

[FIG. 51B]

FIG. 51B shows a histogram obtained from the fluorescence microscope image of wells in Example 25 (after 1.5 hours of reaction).

[FIG. 51C]

FIG. 51C shows a histogram obtained from the fluorescence microscope image of wells in Example 25 (after 1.5 hours of reaction).

[FIG. 51D]

FIG. 51D shows a histogram obtained from the fluorescence microscope image of wells in Example 25 (after 1.5 hours of reaction).

[FIG. 52]

FIG. 52 shows plots of an expected value $\lambda$ with respect to an extracellular vesicle concentration calculated from the results of Example 25.

[FIG. 53]

FIG. 53 shows results of Example 26 in which A549-derived extracellular vesicles were measured with SensoLyte 520 Acetylcholinesterase Activity Assay Kit through use of a 96-well plate (fluorescence intensity from a reporter molecule, temporal changes).

[FIG. 54A]

FIG. 54A shows a histogram obtained from the fluorescence microscope image of wells in Example 26 (after 5 hours of reaction).

[FIG. 54B]

FIG. 54B shows a histogram obtained from the fluorescence microscope image of wells in Example 26 (after 5 hours of reaction).

[FIG. 54C]
FIG. 54C shows a histogram obtained from the fluorescence microscope image of wells in Example 26 (after 5 hours of reaction).
[FIG. 54D]
FIG. 54D shows a histogram obtained from the fluorescence microscope image of wells in Example 26 (after 5 hours of reaction).
[FIG. 55]
FIG. 55 shows plots of an expected value λ with respect to an extracellular vesicle concentration calculated from the results of Example 26.
[FIG. 56]
FIG. 56 shows results of Example 27 in which A549-derived extracellular vesicles were measured with Custom #2 through use of a 96-well plate (fluorescence intensity from a reporter molecule, temporal changes).
[FIG. 57A]
FIG. 57A shows a histogram obtained from the fluorescence microscope image of wells in Example 27 (after 5 hours of reaction).
[FIG. 57B]
FIG. 57B shows a histogram obtained from the fluorescence microscope image of wells in Example 27 (after 5 hours of reaction).
[FIG. 57C]
FIG. 57C shows a histogram obtained from the fluorescence microscope image of wells in Example 27 (after 5 hours of reaction).
[FIG. 57D]
FIG. 57D shows a histogram obtained from the fluorescence microscope image of wells in Example 27 (after 5 hours of reaction).
[FIG. 58]
FIG. 58 shows plots of an expected value λ with respect to an extracellular vesicle concentration calculated from the results of Example 27.
[FIG. 59A]
FIG. 59A shows a fluorescence microscope image of wells in Example 28 in which MDA-MB-231-derived extracellular vesicles were measured with Custom #2 through use of an antibody-immobilized particle and Simoa Discs (after 5 hours of reaction, reporter molecule).
[FIG. 59B]
FIG. 59B shows a fluorescence microscope image of wells in Example 28 (after 5 hours of reaction, reporter molecule).
[FIG. 59C]
FIG. 59C shows a fluorescence microscope image of wells in Example 28 (after 5 hours of reaction, particle).
[FIG. 59D]
FIG. 59D shows a fluorescence microscope image of wells in Example 28 (after 5 hours of reaction, particle).
[FIG. 60A]
FIG. 60A shows a histogram obtained from the fluorescence microscope image of wells in Example 28 (after 5 hours of reaction).
[FIG. 60B]
FIG. 60B shows a histogram obtained from the fluorescence microscope image of wells in Example 28 (after 5 hours of reaction).
[FIG. 61A]
FIG. 61A shows a fluorescence microscope image of wells in Example 29 in which MDA-MB-231-derived extracellular vesicles were measured with Amplite Fluorimetric Acetylcholinesterase Assay Kit through use of antibody-immobilized particles and Simoa Discs (after 3 hours of reaction, reporter molecule).
[FIG. 61B]
FIG. 61B shows a fluorescence microscope image of wells in Example 29 (after 3 hours of reaction, reporter molecule).
[FIG. 61C]
FIG. 61C shows a fluorescence microscope image of wells in Example 29 (after 3 hours of reaction, particle).
[FIG. 61D]
FIG. 61D shows a fluorescence microscope image of wells in Example 29 (after 3 hours of reaction, particle).
[FIG. 62A]
FIG. 62A shows a histogram obtained from the fluorescence microscope image of wells in Example 29 (after 3 hours of reaction).

[FIG. 62B]
FIG. 62B shows a histogram obtained from the fluorescence microscope image of wells in Example 29 (after 3 hours of reaction).
[FIG. 63A]
FIG. 63A shows a histogram obtained from the fluorescence microscope image of wells in Example 30 in which MDA-MB-231-derived extracellular vesicles were measured with SensoLyte 520 Acetylcholinesterase Activity Assay Kit through use of antibody-immobilized particles and Simoa Discs (after 5 hours of reaction).
[FIG. 63B]
FIG. 63B shows a histogram obtained from the fluorescence microscope image of wells in Example 30 (after 5 hours of reaction).
[FIG. 64A]
FIG. 64A shows a histogram obtained from the fluorescence microscope image of wells in Example 31 in which MDA-MB-231-derived extracellular vesicles were measured with Custom #2 through use of antibody-immobilized particles and Simoa Discs (after 5 hours of reaction).
[FIG. 64B]
FIG. 64B shows a histogram obtained from the fluorescence microscope image of wells in Example 31 (after 5 hours of reaction).
[FIG. 65A]
FIG. 65A shows a fluorescence microscope image of Example 32 in which MDA-MB-231-derived extracellular vesicles were measured with Custom #1 through use of liquid droplets (liquid droplets generated with an SPG pumping connector (pore diameter: 10 μm), 4 hours of reaction, bright field).
[FIG. 65B]
FIG. 65B shows a fluorescence microscope image of Example 32 (liquid droplets generated with an SPG pumping connector (pore diameter: 10 μm), 4 hours of reaction, Custom #1).
[FIG. 65C]
FIG. 65C shows a fluorescence microscope image of Example 32 (liquid droplets generated with an SPG pumping connector (pore diameter: 10 μm), 4 hours of reaction, standard fluorescent substance).
[FIG. 66A]
FIG. 66A shows a fluorescence microscope image of Example 32 (liquid droplets generated with an SPG pumping connector (pore diameter: 5 μm), 4 hours of reaction, bright field).
[FIG. 66B]
FIG. 66B shows a fluorescence microscope image of Example 32 (liquid droplets generated with an SPG pumping connector (pore diameter: 5 μm), 4 hours of reaction, Custom #1).
[FIG. 66C]
FIG. 66C shows a fluorescence microscope image of Example 32 (liquid droplets generated with an SPG pumping connector (pore diameter: 5 μm), 4 hours of reaction, standard fluorescent substance).
[FIG. 67]
FIG. 67 shows a histogram of a liquid droplet diameter distribution obtained from the fluorescence microscope image of liquid droplets in Example 32 (liquid droplets generated with an SPG pumping connector (pore diameter: 10 μm), 4 hours of reaction, standard fluorescent substance).
[FIG. 68A]
FIG. 68A shows results of Example 33 in which MDA-MB-231-derived extracellular vesicles were measured with Custom #2 through use of a 96-well plate (fluorescence intensity from a reporter molecule, temporal changes).
[FIG. 68B]
FIG. 68B shows results of Example 33 in which MDA-MB-231-derived extracellular vesicles were measured with resorufin β-D-galactopyranoside through use of a 96-well plate (fluorescence intensity from a reporter molecule, temporal changes).
[FIG. 69A]
FIG. 69A shows a fluorescence microscope image of wells in Example 33 in which MDA-MB-231-derived extracellular vesicles were measured with Custom #2 through use of antibody-immobilized particles and Simoa Discs (after 4 hours of reaction, reporter molecule).
[FIG. 69B]
FIG. 69B shows a fluorescence microscope image of wells in Example 33 in which MDA-MB-231-derived extracellular vesicles were measured with resorufin β-D-galactopyranoside through use of antibody-immobilized particles and Simoa Discs (after 4 hours of reaction, reporter molecule).
[FIG. 69C]
FIG. 69C shows a fluorescence microscope image of wells in Example 33 (after 4 hours of reaction, particle).
[FIG. 70A]

FIG. 70A shows a histogram obtained from the fluorescence microscope image of wells in Example 33 in which measurement was performed with Custom #2 (after 4 hours of reaction).

[FIG. 70B]

FIG. 70B shows a histogram obtained from the fluorescence microscope image of wells in Example 33 in which measurement was performed with resorufin β-D-galactopyranoside (after 4 hours of reaction).

[FIG. 71A]

FIG. 71A shows plots of an expected value λ with respect to an extracellular vesicle concentration calculated from the results of Example 33.

[FIG. 71B]

FIG. 71B shows plots of an expected value λ with respect to an extracellular vesicle concentration calculated from the results of Example 33.

[FIG. 72]

FIG. 72 shows plots of an expected value λ with respect to an extracellular vesicle concentration calculated from the results of Example 49 in which MDA-MB-231-derived extracellular vesicles were measured with Custom #1 through use of Tim4-immobilized magnetic particles (PS Capture Exosome Flow Cytometry Kit) and Simoa Discs.

[FIG. 73]

FIG. 73 shows plots of an expected value λ with respect to an extracellular vesicle concentration calculated from the results of Example 49 in which MDA-MB-231-derived extracellular vesicles were measured with Custom #2 through use of Tim4-immobilized magnetic particles (PS Capture Exosome Flow Cytometry Kit) and Simoa Discs.

[FIG. 74]

FIG. 74 shows plots of an expected value λ with respect to an extracellular vesicle concentration calculated through BG correction from the results of Example 49 in which MDA-MB-231-derived extracellular vesicles were measured with Custom #1 through use of Tim4-immobilized magnetic particles (PS Capture Exosome Flow Cytometry Kit) and Simoa Discs.

[FIG. 75]

FIG. 75 shows plots of an expected value λ with respect to an extracellular vesicle concentration calculated through BG correction from the results of Example 49 in which MDA-MB-231-derived extracellular vesicles were measured with Custom #2 through use of Tim4-immobilized magnetic particles (PS Capture Exosome Flow Cytometry Kit) and Simoa Discs.

[FIG. 76]

FIG. 76 shows plots of an expected value λ with respect to an extracellular vesicle concentration calculated from the results of Example 50 in which MDA-MB-231-derived extracellular vesicles were measured with Custom #2 through use of Tim4-immobilized magnetic particles (MS300S) and Simoa Discs.

[FIG. 77]

FIG. 77 shows plots of an expected value λ with respect to an extracellular vesicle concentration calculated through BG correction from the results of Example 50 in which MDA-MB-231-derived extracellular vesicles were measured with Custom #2 through use of Tim4-immobilized magnetic particles (MS300S) and Simoa Discs.

[Description of Embodiments]

Exemplary embodiments of the present invention are now described with reference to the drawings, but the present invention is not limited to the following examples.

Like elements or corresponding elements are denoted by the same reference numerals in the drawings, and description thereof may be omitted or simplified.

In the following sections, not only bonds, such as a covalent bond and a coordinate bond, but also bindings, such as an affinity interaction and surface adsorption, as well as binding modes including combinations thereof are all described as "binding".

According to an embodiment of the present invention, there is provided a method of detecting an extracellular vesicle having a target enzyme, the method including: a distribution step of distributing an extracellular vesicle and a reagent containing a reporter molecule to be modified by the target enzyme to emit a signal into a plurality of individual separated compartments; a signal generation step of allowing the target enzyme and the reporter molecule to react with each other to generate a signal; a signal detection step of detecting the signal; and an identification step including determining, based on a detection result obtained in the signal detection step, a signal intensity of each of the individual separated compartments, and identifying each of the individual separated compartments having a signal intensity exceeding a predetermined threshold value.

FIG. 1 is a flow chart for illustrating a flow of the method of detecting an extracellular vesicle according to the embodiment of the present invention.

The method of detecting an extracellular vesicle according to the embodiment includes: a distribution step S101 of distributing an extracellular vesicle and a reagent containing a reporter molecule to be modified by the target enzyme to emit a signal into a plurality of individual separated compartments; a signal generation step S102 of allowing the target enzyme and the reporter molecule to react with each other to generate a signal; a signal detection step S103 of detecting the signal; and an identification step S104 including determining, based on a detection result obtained in the signal detection step, a signal intensity of each of the individual separated compartments, and identifying each of the individual separated compartments having a signal intensity exceeding a predetermined threshold value.

The signal detection step may include an image acquisition step of acquiring an image containing the individual separated compartments.

The identification step may include identifying an individual separated compartment having a signal intensity exceeding a predetermined threshold value by processing the image acquired in the image acquisition step.

The identification step may include identifying, based on at least any one of a ratio or a difference between the signal intensity in a reference compartment and the signal intensity in each of the individual separated compartments, each of the individual separated compartments having a signal intensity exceeding a predetermined threshold value. The reference compartment may be a compartment that is free of the extracellular vesicle and contains a sample of the reagent containing the reporter molecule.

In addition, a further step may be incorporated before the distribution step or at the same time as the distribution step.

An example of the further step is a step of mixing an extracellular vesicle and a capture protein to be bound to the extracellular vesicle to form a complex.

Another example of the further step may be a recovery step including mixing an extracellular vesicle and a capture protein to be bound to the extracellular vesicle to form a complex, and recovering the resultant complex. Further, a step of mixing the complex and a second capture protein to be bound to the extracellular vesicle may be incorporated.

According to another embodiment of the present invention, there is provided a reagent kit for detecting an extracellular vesicle having a target enzyme in an individual separated compartment, the reagent kit including: a reagent containing a reporter molecule to be modified by the target enzyme to emit a signal; and a container for providing a plurality of individual separated compartments.

In the following, the embodiments described above are described further specifically.

(Reagent)

The reagent includes an aqueous liquid containing a reporter molecule capable of being converted by the activity of a target enzyme to emit a signal. An example of an aqueous solution is a buffer. The reporter molecule and the buffer are described later.

When there are a plurality of kinds of enzymes to be detected, the reagent may contain two or more kinds of reporter molecules. In this case, the two or more kinds of reporter molecules may each generate a different signal, and the signal intensities of the signals emitted by the two or more kinds of reporter molecules may be separately detected. For example, when a signal to be emitted is fluorescence, signals based on a plurality of kinds of enzymatic activities may be acquired by changing the wavelengths of fluorescence emitted by a plurality of reporter molecules.

In the case where the reagent is used, for example, in the diagnosis of diseases by detecting the plurality of kinds of enzymatic activities as described above, when the origin of an extracellular vesicle is more accurately determined, a primary site is easily identified. In addition, improvement in sensitivity and specificity of inspection may be expected together.

The reagent may further contain a capture protein to be bound to an extracellular vesicle. Alternatively, in the further step, a further reagent containing a capture protein to be bound to an extracellular vesicle may be used. In this case, the extracellular vesicle and the capture protein are bound to each other to form a complex. Thus, a protein and the like included in the extracellular vesicle can be detected in addition to the enzymatic activity. In that case, a capture protein having affinity to the protein and the like included in the extracellular vesicle (a capture target), for example, an antibody may be used. Improvement in detection precision may be expected by simultaneously detecting the enzymatic activity of the target enzyme and the capture target through use of the capture protein. In this case, the capture target may be the target enzyme, or the capture target may be a molecule different from the target enzyme. When the capture target is the target enzyme, for example, a ratio of an active enzyme to an inactive enzyme thereof, and the like can be detected. The capture protein is described later. Additionally, a second capture protein to be bound to an extracellular vesicle may be further bound to the complex formed of the capture protein and the extracellular vesicle.

Further, a capture protein labeled with a particle (a capture protein-immobilized particle) may be used as the capture protein. For example, an aqueous liquid in which an antibody labeled with a particle (an antibody-immobilized particle) is dispersed may be used. In this case, the capture protein is preferably an antibody in which the capture target is a general-purpose protein, carbohydrate chain, or the like expressed in many extracellular vesicles, or a mixture of a plurality of kinds of such antibodies, from the viewpoint of capture efficiency. The use of a particle enables recovery through a magnetic force or a centrifugal force, and hence B/F separation (separation of a bound form and a free form) is facilitated. The particle is described later. The second capture protein to be bound to an extracellular vesicle may be further bound to a particle complex recovered by the particle.

(Target Enzyme)

The target enzyme includes all enzymes included in the surface of an extracellular vesicle or inside the extracellular vesicle. Examples of the target enzyme may include: hydrolases typified by various proteases including MMP families, such as a matrix metalloproteinase (MMP), a disintegrin and metalloproteinase (ADAM), and ADAM with thrombospondin motifs (ADAMTS), matriptase, β-secretase, an endothelin-converting enzyme (ECE), calpain, dipeptidyl peptidase-4 (DPPIV), an angiotensin-converting enzyme 1 (ACE1), and an angiotensin-converting enzyme 2 (ACE2), various esterases including acetylcholinesterase (AChE), autotaxin, lipase, phospholipase, and phosphatase, and various glycosidases, such as β-galactosidase; oxidoreductases typified by various oxidases including monoamine oxidase (MAO), various peroxidases including myeloperoxidase (MPO), catalase, and superoxide dismutase; transferases typified by various acetylases/deacetylases including histone acetyltransferase (HAT) and histone deacetylase (HDAC), kinase, protein kinase, and glycosyltransferase; and isomerases typified by various cis-trans isomerases including peptidylprolyl isomerase (PPIase, Pin1), racemase, and mutase. Any enzyme may be selected as the target enzyme in accordance with the purpose, and the target enzyme is not limited to the above-mentioned examples. In the following, some enzymes usable in the embodiments of the present invention are described in detail.

MMPs are a family of enzymes that function in extracellular matrix protein remodeling, are enzymes essential for various normal physiological processes, such as embryogenesis, morphogenesis, reproduction, tissue resorption, and tissue reconstruction, and are also associated with pathological processes, such as cancer, inflammation, arthritis, cardiovascular diseases, and fibrosis. The MMPs include the following kinds: MMP1, MMP2, MMP3, MMP7, MMP8, MMP9, MMP10, MMP11, MMP12, MMP13, MMP14, MMP15, MMP16, MMP17, MMP18, MMP19, MMP20, MMP21, MMP23, MMP24, MMP25, MMP26, MMP27, and MMP28. In particular, the expression of MMP14 (MT1-MMP) is known to be high expression in highly invasive cancer cells. In cancer cells, dysregulation of gene expression with epithelial-mesenchymal transition causes the aberrant expression of MMP14. It is known that as a result, the proliferation, invasion, and metastasis of cancer cells are promoted by protease-dependent and -independent actions of MMP14. Specific inhibition of MMP14 activity significantly suppresses the proliferation, invasion, and metastasis of cancer cells and angiogenesis, and hence there are growing expectations for a therapy using a humanized antibody.

The ADAM family has 25 kinds of genes identified through human genome analysis, and of those, 4 kinds are pseudogenes. Accordingly, it is known that 21 kinds of ADAM proteins are produced in humans, and of those, 13 kinds are protease-type ADAM molecules, and the remaining 8 kinds are non-protease-type ADAM molecules. ADAM9, ADAM10, and ADAM17 are proteases belonging to the ADAM family and are found to be α-secretases. ADAM9, ADAM10, and ADAM17 act as sheddases and cleave a cell surface protein associated with nerve pathology, an inflammatory response, and tumor progression. In addition, ADAM9, ADAM10, and ADAM17 are physiologically associated as α-secretases against an amyloid precursor protein, and play a critical role in the molecular etiology of Alzheimer's disease. In addition, it has been reported that ADAM28 is highly expressed in a cancer tissue-specific manner in human breast cancer and non-small cell lung cancer tissues.

Aggrecanase is a protease belonging to the ADAMTS family, and degrades aggrecan, which is a major component of cartilage. The degradation of the core protein of cartilage makes differences in normal dynamic properties, such as compressibility and elasticity, and is known to be one of the causes of various symptoms of arthritis. ADAMTS4 is critical aggrecanase in human osteoarthritic cartilage.

Matriptase is a kind of membrane protein, and is a trypsin-like protease belonging to the type II transmembrane serine protease family, a substrate of the matriptase includes an extracellular matrix protein, a cell adhesion molecule, an ion channel, a growth factor-like protein, or another protease, and processing, activation, or degradation of a protein is caused by the action of matriptase. Matriptase is known to be associated with the onset of ovarian cancer, prostate cancer, and cervical cancer.

β-secretase is a kind of membrane protein, is a transmembrane aspartic acid protease present in an acidic intracellular vesicle, and plays a critical role in the production of a β-amyloid peptide through the cleavage of a β-amyloid precursor protein. In senile plaques in the brains of patients with Alzheimer's disease, which is age-related cognitive impairment, accumulation of a neurotoxic β-amyloid peptide is observed.

ECE degrades an amyloid β peptide. It has been reported that Aβ degradation is reduced in the brains of ECE homozygous knockout mice and Aβ-40 and Aβ-42 levels are significantly increased.

Calpain is a family of intracellular cysteine proteases, and regulates the biological activities of many proteins through calcium-dependent selective cleavage. Previous studies show that calpain is involved in a variety of calcium-regulatory intracellular mechanisms, such as platelet activation, apoptosis, cell cycle, cell proliferation, differentiation, and signal transduction, and dysfunction of calpain is known to be involved in various diseases, such as atherosclerosis, Alzheimer's disease, diabetes, and cancer.

DPPIV is a kind of protease known as a prolyl protease that cleaves a protein, and is a serine-dipeptidyl protease that cleaves alanine and proline at the N terminus of a target polypeptide, such as a chemokine or a peptide hormone. Attention has been paid to the relationships between DPPIV and an extracellular matrix that affects the activation of T cells and between DPPIV and adenosine deaminase, and it has been suggested that DPPIV plays a critical role in diabetes, cancer, and an autoimmune disease.

ACE1 is a kind of zinc metalloprotease that degrades angiotensin-1 (AT-1) to produce angiotensin-II (AT-II), and is a membrane protein present on a cell membrane. The AT-II is responsible for a blood pressure regulation mechanism in which the AT-II is bound to an AT receptor in a biological tissue to express vasoconstriction activity. It is known that the overexpression of ACE activity causes extreme vasoconstriction due to the production of a large amount of AT-II to abnormally increase the blood pressure, and as a result, induces vascular diseases, such as myocardial infarction, cerebral infarction, cerebral hemorrhage.

ACE2 is an enzyme of a renin-angiotensin system (RAS), and is known to be a zinc metalloprotease that plays a central role in the control of an angiotensin peptide. ACE2 is directly associated with a cardiac function, and is mainly expressed in cardiac and renal vascular endothelial cells. In addition, it has been suggested that ACE2 may protect the kidney in the early stage of diabetes. ACE2 is known to play a critical role in the regulation of hypertension. In addition, ACE2 is known to be a target receptor of a coronavirus that causes severe acute respiratory syndrome (SARS).

AChE is a serine hydrolase belonging to the carboxylesterase family, and hydrolyzes acetylcholine in the synaptic cleft into choline and acetic acid. In Alzheimer's disease and myasthenia gravis, a reduction in acetylcholine is recognized, and an AChE inhibitor for alleviating a symptom has been developed. AChE is inhibited by an organic phosphorus compound and the like.

Autotaxin hydrolyzes phosphodiester bonds of various nucleotides and nucleotide derivatives. The expression of autotaxin is known to increase in melanoma, breast cancer, renal cell carcinoma, non-small cell lung cancer, neuroblastoma, hepatocellular carcinoma, glioblastoma multiforme, and thyroid carcinoma. In addition, it has been reported that autotaxin is involved in invasiveness of breast cancer cells, and is one of the top 40 genes highly expressed in highly metastatic breast cancer. Further, it has been also reported that autotaxin induces the mobility of some cultured cancer cell lines by increasing the production of LPA.

MAO belongs to the family of flavin-containing amine oxidoreductases, and catalyzes the oxidation of a monoamine. MAO plays a major role in the inactivation of a neurotransmitter. It is known that the dysfunction of MAO is associated with depression, drug dependence, migraine, schizophrenia, attention deficit disorder, Parkinson's disease, Alzheimer's disease, and the like.

MPO is a heme-based peroxidase exhibiting antimicrobial activity against various organisms, and is present in neutrophils, monocytes, and macrophages in soft tissues. MPO is frequently expressed in stimulated neutrophils, and catalyzes the production of a hypohalous acid such as hypochlorous acid from hydrogen peroxide and chloride ions or other halides. MPO is associated with symptoms of many diseases including arthritis, cancer, renal disorders, and cystic fibrosis, and it is known that MPO deficiency, which is a genetic disease, causes immunodeficiency. A relationship between an increase in MPO level and a coronary artery disease is corroborated, and it has been reported that MPO functions as a predictor of myocardial infarction in a certain patient.

HAT regulates the acetylation of histone and nonhistone proteins, and an ε-amino group of lysine on a histone tail is associated with not only transcriptional activity but also DNA replication, DNA repair, and a protein-protein interaction. HAT plays a major role in the control of cell fate, and the dysfunction of HAT is known to be associated with tumorigenesis. P300/CBP-associated factor (pCAF) specifically acetylates lysine residues at the N termini of histone H3 and histone H4. pCAF is also known to be a transcription coactivator of a tumor suppressor p53.

HDAC belongs to a large number of epigenetic enzyme families that play a critical role in gene silencing that controls gene expression, and is important to maintain a chromatin structure and gene transcription. HDAC plays a critical role in cellular processes, such as cell proliferation, cell cycle regulation, apoptosis, and cell differentiation, and hence the expression change or mutation of HDAC is associated with many human diseases, such as cancer, inflammation, neuropsychiatric diseases, skeletogeny, and cardiovascular growth.

Pin1 catalyzes the cis-trans isomerization of a peptide bond in phosphoserine/threonine-proline. Pin1 is known to play a critical role in the regulation of cell cycle, and the expression of Pin1 is increased in various kinds of cancers. In addition, it has been reported that Pin1 is associated with the onset of Alzheimer's disease. It has been suggested that Pin1 is bound to a phosphorylated Thr-212/231 residue of a tau protein to promote dephosphorylation, to thereby inhibit the formation of neurofibrillary tangles.

(Reporter Molecule)

The reporter molecule is not particularly limited as long as the reporter molecule is modified by the target enzyme to emit a signal. The emission of a signal includes a change from a state in which the signal is absent to a state in which the signal is present, a change from a state in which the signal is present to a state in which the signal is absent, or a change in kind or intensity of the signal. For example, when the signal is luminescence, a reporter may be provided by any one of a change in luminescence intensity or a change in luminescence wavelength. In addition, a change in signal may be a change in signal based on a multistep reaction utilizing a reaction product of an enzymatic reaction. In this case, a plurality of kinds of reporter molecules may be simultaneously used. In that case, the selection of a combination where reaction mechanisms do not interfere with each other is desired.

A reporter molecule in which the luminescence intensity is increased by a change in structure caused by enzymatic activity may be, for example, a molecule labeled with a fluorescent substance and a quencher. Such molecule may be, for example, a molecule (FRET substrate) that has a fluorescent substance and a quencher, respectively, on both termini or amino acid residues capable of being modified in the reporter molecule, and contains a peptide serving as a substrate for the target enzyme therebetween. In the FRET substrate, before undergoing enzyme cleavage, the fluorescent substance is sufficiently close to the quencher, and hence the fluorescent substance is quenched, and when the peptide is cleaved by enzymatic activity, the quenching by the quencher is released, and fluorescence is emitted. When the FRET substrate is used as the reporter molecule, the presence or absence of the target enzyme may be observed as a large change in brightness of fluorescence. It is preferred that the peptide included in the FRET substrate have 3 or more residues from the viewpoint of reactivity and have 30 or less residues in order to obtain a quenching effect. In addition, such a reporter molecule that a non-fluorescent substance is converted to a fluorescent substance by a change in chemical structure caused by enzymatic activity is also preferred from the viewpoint that a large change in brightness is achieved.

For example, a reporter molecule of each of the following commercially available kits may be used as such reporter molecule, but the reporter molecule is not limited to the following examples.

SensoLyte (trademark) 390/490/520/570 series (AnaSpec, Inc.)
MMP activity measurement kit SensoLyte (AnaSpec, Inc.)
SensoLyte 520 ADAM10 activity assay kit (AnaSpec, Inc.)
SensoLyte 520 Aggrecanase-1 activity assay kit (AnaSpec, Inc.)
SensoLyte Rh110 matriptase activity assay kit (AnaSpec, Inc.)
SensoLyte 520 β-secretase measurement assay (AnaSpec, Inc.)
SensoLyte 520 ECEs activity assay kit (AnaSpec, Inc.)
SensoLyte 520 calpain activity measurement assay (AnaSpec, Inc.)
SensoLyte Rh110 DPP4 activity measurement assay kit (AnaSpec, Inc.)
SensoLyte 390 ACE2 activity assay kit (AnaSpec, Inc.)
SensoLyte 520 acetylcholinesterase activity assay kit (AnaSpec, Inc.)
SensoLyte HAT (p300)/HAT (pCAF) assay (AnaSpec, Inc.)
SensoLyte 520 HDAC activity assay kit (AnaSpec, Inc.)
SensoLyte Green Pin1 activity assay kit (AnaSpec, Inc.)
Amplite series (AAT Bioquest, Inc. (trademark))
Universal MMP activity measurement kit Amplite (AAT Bioquest, Inc.)
Amplite (trademark) Universal Fluorimetric MMP Activity Assay Kit (Green Fluorescence) (AAT Bioquest, Inc.)
Amplite Fluorimetric Acetylcholinesterase Assay Kit (Green Fluorescence) (AAT Bioquest, Inc.)
Amplite monoamine oxidase assay kit (AAT Bioquest, Inc.)

Others

DPPIV/CD26 measurement assay kit (Enzo Life Sciences Inc.)
Angiotensin 1-converting enzyme (ACE1) activity measurement kit (Life Laboratory Company)
Autotaxin activity measurement assay kit (Echelon Biosciences Inc.)
OxiSelect myeloperoxidase chlorination activity measurement assay (Cell Biolabs, Inc.)
FLUOR DE LYS (trademark) HDAC activity assay kit (Enzo Life Sciences Inc.)

In addition, the reporter molecule may be custom-synthesized, based on the substrate sequence of the target enzyme, so as to have a combination of the fluorescent substance and the quencher at both termini of the substrate sequence or in the substrate sequence.

In addition, examples of a reporter molecule for MMP14 detection may include custom-synthesized FRET peptides used in the following Examples. Custom #1 and Custom #2 used in the following Examples are each a reporter molecule for MMP14 detection including a substrate having FAM and TQ2, which are a FRET pair, conjugated to both termini of the peptide sequence.

Custom #1: [5-FAM]GRIGFLRTAK(TQ2)[OH]
Custom #2: [5-FAM]GGPLGLAGGK(TQ2)[OH]

Custom #3 and Custom #4 are each a reporter molecule for MMP14 detection having a different fluorescence wavelength obtained by replacing FAM and TQ2, which are a FRET pair of Custom #1 and Custom #2, by TAMRA and TQ3. Those reporter molecules can be shared with another reporter molecule that emits fluorescence with the same wavelength range as that of FAM, and are useful in multiplex detection.

Custom #3: [5-TAMRA]GRIGFLRTAK(TQ3)[OH]
Custom #4: [5-TAMRA]GGPLGLAGGK(TQ3)[OH]

In addition, the reporter molecule may be, for example, a peptide that serves as a substrate for the target enzyme and is labeled with a fluorescent substance or a chromogenic dye. An example of such dye is a p-nitroaniline derivative. A substrate containing p-nitroanilide is almost colorless, but p-nitroaniline cleaved out by the enzyme is yellow. In addition, an example of a fluorescent dye is a coumarin derivative serving as a quenching fluorescent reagent. A substrate containing methylcoumarin amide does not emit fluorescence, but aminomethylcoumarin cleaved out by an enzymatic reaction emits strong fluorescence. An example of a similar compound is aminotrifluoromethylcoumarin.

(Reaction Buffer)

The reagent preferably contains a reaction buffer. In addition, the reaction buffer may be used in the further step. An example of the reaction buffer is a Tris-based buffer, which has proven use in an enzymatic reaction using MMP. A specific example thereof is an incubation buffer for MMP gelatin zymography having the following composition (Abcam plc.).

50 mM Tris-HCl (pH: 7.5)
5 mM $CaCl_2$
1 $\mu$M $ZnCl_2$
1% Triton X-100

With regard to AChE, an example of the buffer is a buffer having the following composition as described in the Grant-in-Aid for Scientific Research, Research Category No. 24928013 "Research on clinical application of acetylcholinesterase measurement using selective substrate" by Shirata et al. at Yamagata University.

0.1 M phosphate buffer solution (pH: 7.4)
0.1% Tween 20

When a plurality of target enzymes are detected, it is necessary to optimize the composition of the reaction buffer so that the enzymatic activities of the respective enzymes may be simultaneously detected.

In general, it is preferred that a reaction temperature be from about 20°C to about 37°C, but the optimum temperature only needs to be selected in accordance with the enzymes. When a plurality of target enzymes are detected, it is necessary to optimize the reaction temperature so that the enzymatic activities of the respective enzymes may be simultaneously detected.

For example, when MMP14 and AChE are simultaneously detected, a reaction buffer having the following composition may be used.

50 mM Tris-HCl (pH: 7.5)
10 mM $CaCl_2$
0.01% Brij35

(Capture Protein and Detection Reagent Therefor)

The reagent may further contain a compound that detects a protein and the like included in an extracellular vesicle in addition to the reporter molecule capable of being converted by the activity of the target enzyme to emit a signal. Alternatively, in the further step, a further reagent containing a compound that detects a protein and the like included in an extracellular vesicle may be used.

An example of the compound that detects a protein and the like is a capture protein to be bound to the extracellular vesicle. An antibody may be used as an example thereof. In addition, a complex having a so-called sandwich structure in which the extracellular vesicle is captured by two kinds of capture proteins may be formed by using the two or more kinds of capture proteins.

Examples of a molecule that is a protein or the like common to extracellular vesicles and becomes a capture target of the capture protein include actin, tubulin, GAPDH, flotillin, which is a lipid raft-associated protein, apoptosis-linked gene 2 (ALG-2)-interacting protein X (ALIX) and syntenin, which are ESCRT-associated proteins, tumor susceptibility gene 101 protein (TSG101), heat shock proteins (HSP70 and HSP90), soluble-N-ethylmaleimide sensitive factor attachment protein receptor (SNARE) involved in extracellular vesicle secretion enhancement, and RAB and Annexin involved in membrane transportation and fusion. The examples also include: tetraspanins, such as CD9, CD63, and CD81, which are membrane proteins known to be localized on the surface of an extracellular vesicle and are a family of 4-pass transmembrane proteins; and cell adhesion molecules, such as integrin, selectin, and EpCAM, which are transmembrane proteins that mediate cell-cell adhesion. The examples also include disease-specific proteins that are included in an extracellular vesicle.

Examples of the capture protein include an anti-CD9 antibody, an anti-CD63 antibody, an anti-CD81 antibody, an anti-flotillin antibody, an anti-ALIX antibody, an anti-syntenin antibody, an anti-TSG101 antibody, an anti-HSP70 antibody, an anti-HSP90 antibody, an anti-SNARE antibody, an anti-Rab antibody, an anti-annexin antibody, an anti-MMP antibody, an anti-ADAM antibody, an anti-AChE antibody, an anti-integrin antibody, an anti-selectin antibody, and an anti-EpCAM antibody. In addition, with regard to a large number of disease-specific proteins, an antibody that recognizes each of the proteins as a capture target may be used. In grasping the total number of extracellular vesicles, any one of the anti-CD9 antibody, the anti-CD63 antibody, the anti-CD81 antibody, the anti-flotillin antibody, the anti-ALIX antibody, the anti-syntenin antibody, the anti-TSG101 antibody, the anti-HSP70 antibody, the anti-HSP90 antibody, the anti-SNARE antibody, the anti-Rab antibody, the anti-annexin antibody, the anti-integrin antibody, the anti-selectin antibody, and the anti-EpCAM antibody, or a plurality of kinds thereof may be used. In addition, when the disease-specific protein or the like is a capture target, the target enzyme may be the capture target, or the capture target may be different from the target enzyme. When the capture target is the target enzyme, for example, a ratio of an active enzyme to an inactive enzyme thereof, and the like can be detected.

T cell immunoglobulin and mucin domain-containing protein 4 (Tim-4) having affinity to a phospholipid (phosphatidylserine) of an extracellular vesicle may also be used as the capture protein. In this case, the extracellular vesicle can be widely detected, and hence Tim-4 is useful in grasping the total number of extracellular vesicles.

In addition, a lectin may also be used as the capture protein to detect a carbohydrate chain included in an extracellular vesicle.

The capture protein may be labeled, and is labeled with, for example, a fluorescent substance, a dye, an enzyme, or a particle.

The reagent may further contain a detection reagent for detecting a labeling molecule. Alternatively, in the further step, a detection reagent for detecting a labeling molecule may be used. As an ordinary method, a method in which the capture protein is labeled with an enzyme, such as alkaline phosphatase (AP), horseradish peroxidase (HRP), or β-galactosidase (β-Gal), and a substrate for an enzyme with which an antibody is labeled is contained as the detection reagent is known. Such enzyme or substrate may be used as the detection reagent. In addition, a reagent including a secondary antibody having an enzyme, such as AP, HRP, or β-Gal, bound thereto, or a substrate for the enzyme may be further used.

(Staining Reagent)

A staining reagent capable of staining a protein included in an extracellular vesicle or a membrane itself to detect the protein or the membrane may be further added, in addition to the above-mentioned reporter molecule capable of being converted by the activity of the target enzyme to emit a signal. In that case, a staining reagent having affinity to the protein or a staining reagent having affinity to the membrane may be used.

For example, the following staining reagents may be used.

Exosome fluorescent staining reagent (ExoSparkler series) (manufactured by Dojindo Laboratories)
Exo-Glow EV Labeling Kit series (manufactured by System Biosciences, LLC)
Lipid membrane staining fluorescent dye CellBrite Steady & CellBrite Fix series (manufactured by Biotium)
ExoBrite EV Membrane Staining Kit series (manufactured by Biotium)
MemGlow series (manufactured by Cytoskeleton, Inc.)
PKH series (manufactured by Sigma-Aldrich Co. LLC)
Wheat Germ Agglutinin, Alexa Fluor series (manufactured by Thermo Fisher Scientific Inc.)
Lipophilic Carbocyanine Dye series (e.g., Dilinoleyl DiI/DiO) (manufactured by PromoCell GmbH)
Near-infrared fluorescent probe for exosome staining Sciforiem (trademark) FI 7510 (manufactured by Toyo Ink Co., Ltd.)

ACOERELA lipid membrane fluorescent labeling kit (manufactured by ACOERELA)

When the staining reagents are used, the number of the extracellular vesicles having target enzyme activity can be detected while the total number of extracellular vesicles is grasped. For example, the staining reagents are useful in the determination of the fraction, presence ratio, and the like of the extracellular vesicle having target enzyme activity.

(Particle)

A capture protein labeled with a particle (capture protein-immobilized particle) may be used as the capture protein. The kind of the particle may be a primary particle or a secondary particle in which the primary particles are aggregated as long as the capture protein can be bound to the particle.

The binding between the capture protein and the particle is not limited, and examples of the binding include a covalent bond and physical adsorption. A covalent bond is preferred. The capture protein or a linker described later and the particle are easily bound to each other, and hence it is preferred that the binding be formed by a reaction utilizing a carboxyl group originally present in the particle. That is, a binding portion between the capture protein and the particle preferably has a structure derived from the carboxyl group of the particle.

The capture protein and the particle may be bound to each other via an amide bond. In this case, there is obtained a structure in which C=O derived from a carboxyl group in the particle and N-H derived from an amino group in the protein form the amide bond.

The capture protein and the particle may be bound to each other via a linker. In this case, the "linker" refers to a structure that forms the binding between the capture protein and the particle.

As a binding portion between the capture protein and a capture protein-side linker, there are given: an ε-amino group of a lysine residue in the capture protein; an α-amino group at the N-terminus of the capture protein; various tag peptide sequences or tag proteins artificially inserted into the N-terminus of the capture protein; and the like.

Examples of the tag peptides include a His tag, a HA tag, and a DDDDK tag (FLAG (trademark)). In addition, an example of the tag proteins is Halo-tag (trademark).

As a binding portion between the particle and the linker, various binding portions are given depending on the kind of the linker.

As an example, when the linker is bound to the ε-amino group of a lysine residue of the capture protein or to the α-amino group at the N-terminus of the capture protein on the capture protein side, a carboxyl group, an aldehyde group, or the like is given as a structure to be utilized for binding to a particle-side linker.

In addition, for example, when the linker causes an amino group in the capture protein and a carboxyl group in the particle to form an amide bond on the capture protein side, a condensation reaction using N-hydroxysuccinimide (NHS)/water-soluble carbodiimide (WSC) may be used for the binding to the particle-side linker.

Examples of the linker include: any of various tag peptide sequences or tag proteins and a moiety having affinity therefor; a complex of avidin and biotin; and PEG having any of various functional groups at an end thereof. In addition, the capture protein may be bound to the particle through, for example, physical adsorption between the capture protein and the surface of the particle.

A position in the capture protein to be bound to the particle is preferably such a position that the function of the capture protein is not inhibited.

As a material for the particle, there are given, for example, a polymer resin (e.g., styrene resin or acrylic resin) particle, a silica particle, a resin particle, an agarose-based resin particle, a metal particle, and a latex particle.

Examples of the commercially available particle include magnetic particles subjected to measurement with Magnosphere (trademark) MS300, Magnosphere MS160, PureProteome (trademark) Nickel Magnetic Beads, Simoa (trademark), and Homebrew assay kit (Quanterix). As the material for the magnetic particle, a particle containing a paramagnetic material, a ferromagnetic material, a superparamagnetic material, or the like, such as iron, nickel, and magnetite, is preferably used, but other materials may also be used. When the magnetic particle is used, recovery can be performed through application of a magnetic field.

The particle diameter of the particle is preferably 10 nm or more, more preferably 1 μm or more and 10 μm or less. Specific examples of a method of measuring the particle diameter include observation with an optical microscope or an electron microscope, a laser diffraction method, a dynamic light scattering method, and a centrifugal sedimentation method, but another method of measuring the particle diameter may also be used.

When the capture protein-immobilized particle is used, an extracellular vesicle can be recovered through use of the property of the particle. That is, in this embodiment, as the further step, a recovery step including mixing an extracellular vesicle and a capture protein to be bound to the extracellular vesicle to form a complex, and recovering the resultant complex may be further incorporated before the distribution step or at the same time as the distribution step, and the capture protein may be labeled with a particle. When a magnetic particle is used as the particle, B/F separation of the capture protein can be performed, and the recovery step can be more simply performed. The recovery step may further include a step of washing the complex. In addition, the recovered complex may be bound to a second capture protein to be bound to an extracellular vesicle to form a complex having a so-called sandwich structure in which the extracellular vesicle is captured by the two kinds of capture proteins.

In the recovery step, after the extracellular vesicle has been recovered from the sample, the extracellular vesicle may be substantially concentrated by being dispersed in a medium having a smaller volume than the original sample.

For example, when the recovery step is incorporated, even an extracellular vesicle to be discarded without being caused to fill the individual separated compartments may be recovered by allowing the capture protein labeled with the particle to act on the extracellular vesicle. After the recovery, the extracellular vesicle may be detected by filling the individual separated compartments with the particle that has captured the extracellular vesicle (a particle complex). In addition, for example, a trace amount of the extracellular vesicle that dissolves in a specimen such as blood or an aqueous solution may be captured by the capture protein labeled with the particle and recovered.

In addition, for example, when the sample containing the extracellular vesicle is a large-volume solution, the extracellular vesicle may be captured by the capture protein labeled with the particle before filling minute individual separated compartments, to fill the individual separated compartments with the particle that has captured the extracellular vesicle (a particle complex). Thus, the extracellular vesicle can be distributed to the individual separated compartments with a reduced loss of the extracellular vesicle, and the extracellular vesicle can be detected with high sensitivity.

In addition, when a magnetic particle is used as the particle, the extracellular vesicle can be simply recovered through utilization of magnetism.

The capture protein is as described above. Even when the particle is used as a label, the capture target may be the target enzyme, or the capture target may be different from a target protein. When a substance other than the target protein is the capture target, colocalization of the capture target and the target enzyme may be detected. When the capture target is the target enzyme, improvement in detection precision of the target enzyme may be expected.

Tim-4 having affinity to a phospholipid (phosphatidylserine) of the extracellular vesicle can be used as the capture protein. In this case, the extracellular vesicle can be widely recovered, which is useful in the recovery of the extracellular vesicle.

In addition, a lectin can be used to capture a carbohydrate chain included in the extracellular vesicle.

(Blocking Agent)

The reagent may further contain a blocking agent. In the further step, the blocking agent may be used. For example, at the time of the binding of the particle and the capture protein to each other, part of the linker binding portion of the particle to which the capture protein has not been bound may be sealed with the blocking agent to suppress non-specific binding or undesired binding. For example, when an amino group of the capture protein and a carboxyl group of the particle are subjected to a condensation reaction through use of NHS/WSC, the particle may have an unreacted carboxyl group after the reaction. Thus, a carboxyl group that has not been bonded to the capture protein may be subjected to a reaction with ethanolamine, PEG having an amino group, or the like serving as the blocking agent. Examples of the blocking agent include Blockmaster (trademark) CE series (manufactured by Medical & Biological Laboratories Co., Ltd.). Additionally, a publicly known substance may be used as the blocking agent without restriction, and for example, albumin, casein, skim milk, Blockmaster PA series (manufactured by Medical & Biological Laboratories Co., Ltd.), Blockmaster DB series (manufactured by Medical & Biological Laboratories Co., Ltd.), or the like may be used. In addition, the blocking agent may be incorporated into the detection reagent containing the reporter molecule. Non-specific binding of the reporter molecule to a modification portion of the particle surface may be suppressed. A publicly known substance may be used as the blocking agent without restriction, and for example, albumin, casein, skim milk, or the like may be used.

(Specimen and Recovery Method for Extracellular Vesicle)

Examples of a specimen to be applied by the detection method of the present invention include blood (serum and plasma), urine, cerebral spinal fluid, ascites, amniotic fluid, milk, saliva, and lacrimal fluid that are used for liquid biopsy, and various specimens may be applied. In addition, a culture supernatant is also a target in the field of basic study. The "liquid biopsy" is widely defined as diagnosis and inspection by a biologically derived marker in body fluids, and is an inspection method having a reduced invasiveness through use of the above-mentioned body fluids as compared to past tissue biopsy. The liquid biopsy enables the acquisition of various kinds of information, such as genetic information, protein information, and metabolism information, without sampling a tumor site, and diagnosis and prediction of a therapeutic effect are performed based on the information.

When extracellular vesicles are utilized in the liquid biopsy, a step of recovering an exosome from a body fluid is essentially required. Examples of a major recovery method include an ultracentrifugation method, a density-gradient centrifugation method, a polymer precipitation method, an ultrafiltration method, a size exclusion chromatography method, an ion exchange chromatography method, an immunoaffinity chromatography method, an immunoaffinity capture method (magnetic beads), a tangential flow filtration method, and a microfluidic separation method.

The exosome recovery method most commonly used to date is an ultracentrifugation method, which has been recognized as the gold standard of the recovery method. However, the ultracentrifugation method requires time and includes a complicated operation, and hence the ultracentrifugation method is unsuitable for high-throughput treatment. In addition, the ultracentrifugation method generates substantial loss, and hence the ultracentrifugation method is also unsuitable for recovery from a small amount of the body fluid. Examples of a method capable of recovering an exosome with high yield through a simple operation include a polymer precipitation method, an ultrafiltration method, a size exclusion chromatography method, an ion exchange chromatography method, an immunoaffinity chromatography method, an immunoaffinity capture method (magnetic beads), a tangential flow filtration method, and a microfluidic separation method.

More specific examples of the polymer precipitation method include Total Exosome Isolation series (manufactured by Thermo Fisher Scientific Inc.) and ExoQuick series (manufactured by System Biosciences, LLC). Examples of the ultrafiltration method include Amicon Ultra series (manufactured by Merck KGaA). Examples of the size exclusion chromatography include PURE-EV series (manufactured by HansaBioMed Life Sciences Ltd), EV Second series (manufactured by GL Sciences Inc.), SmartSEC series (manufactured by System Biosciences, LLC), and qEV series (manufactured by Izon Science Limited). Examples of the ion exchange chromatography method include CIM Monolithic series (manufactured by Resonac Corporation) and EVs Quick Filter series (manufactured by Human Metabolome Technologies, Inc.). Examples of the immunoaffinity chromatography method include Fab-TACS Exosome Isolation Kit series (manufactured by IBA Lifesciences GmbH). Examples of the immunoaffinity capture method (magnetic beads) include Exo-Flow Exosome IP Kit series (manufactured by System Biosciences, LLC) and MagCapture Exosome Isolation Kit series (manufactured by FUJIFILM Corporation). Examples of the tangential flow filtration method include TFF-EVs series (manufactured by HansaBioMed Life Sciences Ltd), TFF-Easy series (manufactured by HansaBioMed Life Sciences Ltd), and tangential flow filtration Minimate EVO TFF system (manufactured by Cytiva). Development of the microfluid separation method is advancing at research institutions.

(Cells)

Cells from which an extracellular vesicle is collected in the present invention are not particularly limited. Mammalian cells are preferably used. Cells present in the living body, cultured cells, cells of a tissue section, cells extracted from a tissue or a biological sample, or the like become target cells. Normal cells, cancer cells, degenerated cells, a mixed culture of cancer cells and normal cells, a mixed culture of cancer cells, a mixed culture of normal cells, cells in a mixed tissue or blood, or the like become target cells. In addition, slow-twitch muscle fibers and fast-twitch muscle fibers of the same differentiated cell type, or cells that are predicted to undergo changes in the phenotype of cells of the same type, such as epithelial-mesenchymal transition of cancer cells, differentiation induction from undifferentiated cells, degeneration of cells, or senescence of cells, or the like also become a target.

(Individual Separated Compartments)

An extracellular vesicle and the reagent containing the reporter molecule to be modified by the target enzyme to emit a signal are distributed into a plurality of individual separated compartments. Thus, the extracellular vesicle and the reagent are individually separated and comparted. When an extracellular vesicle is distributed into individual separated compartments, there are individual separated compartments into which the extracellular vesicle is distributed and individual separated compartments into which the extracellular vesicle is not distributed. Accordingly, the extracellular vesicle is concentrated in the individual separated compartments into which the extracellular vesicle is distributed, and hence weak activity of few enzymes in the extracellular vesicle can be detected, and the time when a signal is saturated can be shortened. Further, when the volume per compartment of the individual separated compartments is sufficiently reduced, the extracellular vesicle included in one compartment can be set to one or less extracellular vesicle, and when the number of compartments from which signals are obtained is counted, a concentration of the extracellular vesicle having target enzymatic activity in the sample can be calculated.

The term "individual separated compartment" refers to a compartment individually separated. A chemical reaction is performed in each compartment, and in principle, substances in different compartments are not mixed with each other or are not affected by each other. Each of the individual separated compartments preferably includes a solvent. Each of the individual separated compartments preferably includes 0.1 fL or more and 1,400 fL or less of the solvent, and more preferably includes 0.8 fL or more and 600 fL or less of the solvent. The solvent is preferably a water-soluble solvent.

Liquid droplets or wells may be used as the individual separated compartments. A water-in-oil type emulsion (W/O emulsion) is preferably used as each of the liquid droplets. In addition, for example, wells included in a well array having such a structure as illustrated in FIG. 5A and FIG. 5B may be used as the wells. In addition, specific examples of the wells include wells on Simoa Discs (Quanterix Corporation).

FIG. 5A is a cross-sectional view of a well array 200, and FIG. 5B is a cross-sectional view of the well array 200 having wells 204 filled with a particle complex 206 that is a capture protein-immobilized particle that has captured the extracellular vesicle. The well array 200 includes a lower substrate 201, an upper substrate 202, an injection port portion (not shown), and a discharge port portion (not shown), and the lower substrate 201 has hydrophobic partition walls 203 formed thereon. On the lower substrate 201, a plurality of the wells 204 are separated from each other by the partition walls 203.

The lower substrate 201 preferably has a hydrophilic surface, and for example, glass, silicon, or a polymer resin may be used as a material for the lower substrate 201. In addition, an upper surface (surface opposed to the lower substrate 201) of the upper substrate 202 is preferably hydrophobic. For example, a hydrophobic resin, a water-repellent resin, or a fluorinated polymer resin may be used as a material for the partition walls 203. When the bottom surface of each of the wells 204 is hydrophilic and the top surface of each of the partition walls 203 is hydrophobic, the wells 204 can be efficiently filled with a solution, and in a step of removing an excess of the solution with a hydrophobic solvent, the hydrophobic solvent can be prevented from entering the wells 204. In addition, the lower substrate 201, the upper substrate 202, and the partition walls 203 may be formed from the same material, and an example of the material is a polymer resin such as a cycloolefin polymer (COP).

The wells 204 are recesses for accommodating the solution, and are separated from each other by the partition walls 203. The wells 204 use the lower substrate 201 as their bottom surfaces, and the shape of a region surrounded by the bottom surface and side surfaces of each of the wells 204 may be, for example, a columnar shape or a prismatic shape. In the well array 200 illustrated in FIG. 5A and FIG. 5B, the depth of each of the wells 204 is equal to the height of each of the partition walls 203.

When the shape of each of the wells 204 is a columnar shape having a circle bottom surface, it is preferred that the diameter of the bottom surface of each of the wells 204 be 0.5 μm or more and 12 μm or less, and the depth of each of the wells 204 be 0.5 μm or more and 12 μm or less. In addition, it is more preferred that the diameter of the bottom surface of each of the wells 204 be 1 μm or more and 9 μm or less, and the depth of each of the wells 204 be 1 μm or more and 9 μm or less.

The upper substrate 202 is opposed to the openings of the wells 204 and the top surfaces of the partition walls 203 across a space 205. The space 205 serves as a channel through which various liquids flow, and the various liquids can flow from the injection port portion toward the discharge port portion. After the wells 204 have been filled with the solution, the space 205 is filled with the hydrophobic solvent. When the particle complex 206 is used, the wells 204 are filled with the particle complex 206, and the space 205 is filled with the hydrophobic solvent. For example, a fluorine oil or an aliphatic hydrocarbon may be used as the hydrophobic solvent.

The volume of each of the individual separated compartments is preferably 0.1 fL or more and 1,400 fL or less, more preferably 0.8 fL or more and 600 fL or less.

When the volume of each of the individual separated compartments is 0.1 fL or more, liquid droplets or wells each having such volume may be formed without difficulty. In addition, when the volume of each of the individual separated compartments is 1,400 fL or less, the detection time can be made sufficiently short.

Specific examples of the method of detecting an extracellular vesicle according to this embodiment are described below for a case in which the individual separated compartments are liquid droplets and a case in which the individual separated compartments are wells.

FIG. 2 is a flow chart for illustrating a flow of the method of detecting an extracellular vesicle according to this embodiment in the case where the individual separated compartments are liquid droplets.

(Step S201)

Liquid droplets each containing the sample and the reporter molecule are prepared. The liquid droplets are each preferably a water-in-oil type emulsion.

(Step S202)

The liquid droplets each containing the sample and the reporter molecule are placed in a tube, and are incubated in an incubator at 37°C. However, the reaction temperature may be set to any temperature, and is not limited to 37°C.

Through the incubation, an enzymatic reaction progresses, and a signal is generated from the reporter molecule.

(Step S203)

The incubation is ended after a reaction time set in advance, and an observation chamber is filled with the liquid droplets. The observation chamber is preferably a plate for sedimentation.

(Step S204)

An example in which the signal is fluorescence is described below.

A fluorescence image of each of the liquid droplets with which the observation chamber is filled is acquired with a fluorescence microscope. The fluorescence image of the liquid droplet is acquired with an image pickup device such as a CCD camera mounted onto the fluorescence microscope. Through the acquisition of the fluorescence image of the liquid droplet, the fluorescence is detected.

(Step S205)

Based on the detection result of the signal, the signal intensity of each of the liquid droplets is determined, and each liquid droplet having a signal intensity exceeding a predetermined threshold value is identified.

Each liquid droplet having a signal intensity exceeding the predetermined threshold value may be identified based on at least any one of a ratio or a difference between the signal intensity of a reference liquid droplet and the signal intensity of each of the liquid droplets containing the sample and the reporter molecule. The signal intensity of the reference liquid droplet may be a signal intensity acquired for a liquid droplet that is free of the extracellular vesicle and contains the reporter molecule.

The signal intensity of each liquid droplet is determined by subjecting the signal image of the liquid droplet taken with the image pickup device to predetermined image processing. For example, the signal intensity of each liquid droplet may be determined by using Image J (manufactured by the American National Institutes of Health) or the like as image processing software.

A case in which fluorescence is used as the signal is also described below.

The predetermined image processing has a function of binarizing a fluorescence image based on brightness information.

The negative liquid droplets are liquid droplets free of the extracellular vesicle, and are liquid droplets that do not generate fluorescence derived from the fluorescent substance in the reporter molecule. In addition, the positive liquid droplets are liquid droplets containing the extracellular vesicle containing a target enzyme, and are liquid droplets that generate fluorescence derived from the fluorescent substance in the reporter molecule. The fluorescence intensity of each liquid droplet may be determined by binarizing the fluorescence image (grayscale image) through use of predetermined image processing.

(Step S206)

After the enzymatic reaction through the incubation in Step S202, the concentration of the extracellular vesicle is calculated from the number of liquid droplets generating a signal. When the number of extracellular vesicles included in the sample is large, one liquid droplet may contain two or more extracellular vesicles. Thus, the number of extracellular vesicles may fail to match the number of the liquid droplets generating a signal.

For the above-mentioned reason, it is preferred to calculate the concentration of the extracellular vesicle through calculation that takes the Poisson distribution into consideration. In the Poisson distribution, when the average number of molecules per liquid droplet is represented by λ, a ratio P(k) of the liquid droplets generating a signal can be expressed by the following equation (1).

$$P(k)=(\lambda k/k!)e^{-\lambda}\ (k=0, 1, 2, ...)$$

... equation (1)

From the number of liquid droplets generating the signal, P(k) can be determined and the expected value λ can be calculated. Thus, through use of the equation (1), the concentration of the extracellular vesicle can be calculated from the number of liquid droplets in which the signal has been detected among all the liquid droplets.

FIG. 3 is a flow chart for illustrating a flow of the method of detecting an extracellular vesicle according to this embodiment in the case where the individual separated compartments are wells.

(Step S301)

The well array 200 schematically illustrated in FIG. 5A and FIG. 5B is used as the well array. In the well array 200, the injection port portion (not shown) and the discharge port portion (not shown) are opened, and a reaction solution containing the sample and the reporter molecule is fed from the injection port portion into the space 205.

(Step S302)

The wells 204 are filled with the reaction solution. As a reaction solution filling method, for example, there is given a method including leaving the well array 200 under reduced pressure and degassing the space 205. Specifically, it is preferred to leave the well array 200 in a vacuum desiccator at 0.1 atm for a predetermined time period. Through the degassing, the air in the wells 204 is removed, and thus the wells 204 can be efficiently filled with the reaction solution. A degassing time is not particularly limited, and may be freely set. The reaction solution filling method is not limited to a method based on the degassing. For example, the wells may be efficiently filled with the reaction solution even by performing suction treatment from the discharge port portion.

(Step S303)

The space 205 was fed with a hydrophobic solvent to be sealed. That is, the reaction solution present in the space 205 above the wells 204 is replaced with the hydrophobic solvent. As the hydrophobic solvent, there may be used, for example, a fluorinated oil, a saturated aliphatic hydrocarbon, an unsaturated aliphatic hydrocarbon, an aromatic hydrocarbon, and a silicone oil. Examples of the fluorinated oil may include Fluorinert (manufactured by 3M), AsahiKlin AE-3000 (manufactured by AGC Inc.), Fomblin (manufactured by Solvay S.A.), and Krytox (manufactured by DuPont). Examples of the saturated hydrocarbon include Isopar (manufactured by Exxon Mobil Corporation) and a mineral oil.

(Step S304)

The well array 200 filled with the reaction solution is incubated in an incubator at 37°C. However, the reaction temperature may be set to any temperature, and is not limited to 37°C.
Through the incubation, an enzymatic reaction progresses, and a signal is generated from the reporter molecule.

(Step S305)

The incubation is ended after a reaction time set in advance, and a fluorescence image of each of the wells 204 is acquired with a fluorescence microscope in the case where the signal is fluorescence, for example.

(Step S306)

The signal intensity of each of the wells 204 is determined, and each well 204 having a signal intensity exceeding a predetermined threshold value is identified. Each of the wells 204 having a signal intensity exceeding the predetermined threshold value may be identified based on at least any one of a ratio or a difference between the signal intensity of a reference well and the signal intensity of each of the wells 204 containing the reporter molecule and the sample. The signal intensity of the reference well means a signal intensity acquired for each of the wells 204 that is free of the extracellular vesicle and contains the reporter molecule.
Predetermined image processing is used for the determination of the signal intensity of each of the wells 204. The above-mentioned Image J may be used as image processing software, and the signal intensity of each of the wells 204 may be determined by an operation similar to that in the case where the individual separated compartments are liquid droplets.

(Step S307)

The concentration of the extracellular vesicle may be calculated by an operation similar to that in the case where the individual separated compartments are liquid droplets.
FIG. 4 is a flow chart for illustrating a flow of the method of detecting an extracellular vesicle according to this embodiment when background correction is performed through use of a mask image. An example in which the signal is fluorescence is described.

(Step S401)

A mask image indicating an individual separated compartment position is produced from a fluorescence microscope image of a standard fluorescent substance.

(Step S402)

A background of a fluorescence microscope image of the reporter molecule is corrected through use of the mask image.

(Step S403)

The average brightness value of each of the individual separated compartments is acquired through use of the corrected fluorescence image of the reporter molecule and the mask image, and a histogram is created.

(Step S404)

A predetermined threshold value is applied to the histogram to judge negativity or positivity and aggregate.

(Other Step)

The method of detecting an extracellular vesicle according to this embodiment may include a step other than the above-mentioned steps.

An example of the other step is a step of acquiring a fluorescence image serving as a reference (hereinafter abbreviated as "reference fluorescence image") in the case where the signal is fluorescence.

For example, when fluorescence derived from a substance other than the fluorescent substance for detecting the extracellular vesicle is included in the fluorescence image acquired in Step S204 or Step S305, the concentration of the extracellular vesicle may not be correctly calculated. A conceivable example of the fluorescence of an origin other than the fluorescent substance for detecting the extracellular vesicle is autofluorescence emitted by the wells.

The timing at which the reference fluorescence image is acquired in the step of acquiring a reference fluorescence image only needs to be before the presence of the enzyme causes the above-mentioned reporter molecule to be cleaved to emit fluorescence. For example, the reference fluorescence image may be acquired before the wells or the liquid droplets are filled with the sample or the reagent containing the reporter molecule, before filling with a sealing oil for sealing the individual separated compartments, such as the wells or the liquid droplets, or before the incubation of Step S202 or Step S304 is performed (before heating is performed). In addition, when the timing at which the reference fluorescence image is acquired is after the wells or the liquid droplets have been filled with the reporter molecule, and before incubation is performed (before heating is performed), the reference fluorescence image may be acquired after a predetermined time period from the time that the filling of the wells or the liquid droplets with the reporter molecule is started.

When the timing at which the reference fluorescence image is acquired is set to be before incubation is performed (before heating is performed), the reference fluorescence image may be acquired before heating means that has received a driving signal starts heating operation. In addition, the timing at which the reference fluorescence image is acquired may be determined by using means for monitoring the state of the filling of the wells or the liquid droplets with the sample or the reporter molecule.

The reference fluorescence image may be used as a reference in calculating the intensity of fluorescence based on the presence of the enzyme in each well or each liquid droplet in the fluorescence image obtained in Step S204 or Step S305. That is, when to what degree the fluorescence intensity is increased from the fluorescence intensity of the reference fluorescence image acquired in advance is calculated, the intensity of fluorescence derived from a component other than the fluorescent substance for detecting the extracellular vesicle in each well or each liquid droplet can be excluded. Thus, the fluorescence intensity derived from the fluorescent substance of the reporter molecule for detecting the extracellular vesicle can be more accurately obtained.

In addition, for example, for the fluorescence image obtained in Step S204 or Step S305, it can be more correctly judged whether the pixel value (fluorescence intensity) of a well or liquid droplet of interest is equal to or more than the predetermined threshold value, or less than the predetermined threshold value. The predetermined threshold value only needs to be determined prior to the judgment of the number of the wells or the number of the liquid droplets. For example, the predetermined threshold value may be a value fixed in the extracellular vesicle detection apparatus, or may be a value to be set by input from a user. Being judged to be equal to or more than the predetermined threshold value may be expressed as being judged to be positive, and being judged to be less than the predetermined threshold value may be expressed as being judged to be negative.

In addition, for example, the reference fluorescence image may be acquired in order to detect a state in which a well or liquid droplet to be filled with the sample or the reporter molecule has not been filled therewith owing to a failure or the like in the well or the liquid droplet, or in an operation of filling the well or the liquid droplet. In this case, for example, a well or a liquid droplet may be filled with a substance that emits light having a wavelength different from that of the fluorescent substance to be used for the detection of the extracellular vesicle (hereinafter referred to as "reference substance"), and a fluorescence image thereof may be acquired to acquire a fluorescence distribution. In this case, a difference between the center wavelength of the emission wavelength (fluorescence wavelength) of the reference substance and the center wavelength of the emission wavelength (fluorescence wavelength) of the reporter fluorescent substance is preferably 30 nm or more, more preferably 50 nm or more, still more preferably 100 nm or more.

When fluorescence is not detected as a result of the acquisition of the reference fluorescence image, it may be appropriate to start over from the first step, to display an error message, or to determine whether to start over or continue measurement in accordance with the number of wells or liquid droplets in which fluorescence is not detected. It may be appropriate not to count the fluorescence of the wells or the liquid droplets when the number of wells or liquid droplets in which fluorescence is not detected is equal to or less than a predetermined threshold value. The predetermined threshold value only needs to be determined prior to the judgment of the number of the wells or the number of the liquid droplets. For example, the predetermined threshold value may be a value fixed in the extracellular vesicle detection apparatus, or may be a value to be set by input from a user. In addition, for example, it may be appropriate to allow a user to appropriately make a choice regarding operation in the case where fluorescence is not detected as a result of the acquisition of the reference fluorescence image in the extracellular vesicle detection apparatus for carrying out the method of detecting an extracellular vesicle according to this embodiment.

The extracellular vesicle detection apparatus for carrying out the method of detecting an extracellular vesicle according to this embodiment may be configured to be switchable between a mode including the above-mentioned step of acquiring a reference fluorescence image and a mode not including the step of acquiring a reference fluorescence image.

As described above, the step of acquiring a reference fluorescence image has been described in the method of detecting an extracellular vesicle according to this embodiment, but may be applied to the extracellular vesicle detection apparatus according to the above-mentioned embodiment of the present invention. In this case, the reference fluorescence image may be acquired with the fluorescence detection unit or the image acquisition unit in the extracellular vesicle detection apparatus according to this embodiment, or the reference fluorescence image may be acquired by other means (e.g., a reference fluorescence image acquisition unit).

In addition, according to another embodiment of the present invention, there is provided an apparatus for detecting an extracellular vesicle having a target enzyme, the apparatus including: a distribution unit configured to distribute an extracellular vesicle and a reagent containing a reporter molecule to be modified by the target enzyme to emit a signal into a plurality of individual separated compartments; a signal generation unit configured to allow the target enzyme and the reporter molecule to react with each other to generate a signal; a signal detection unit configured to detect the signal; and an identification unit configured to determine, based on a detection result obtained with the signal detection unit, a signal intensity of each of the individual separated compartments, and identify each of the individual separated compartments having a signal intensity exceeding a predetermined threshold value.

The detection apparatus of this embodiment is described further specifically with reference to FIG. 6.

(Distribution Unit)

When the individual separated compartments are liquid droplets, for example, the distribution unit 101 includes an emulsification membrane or microchannels. When the distribution unit 101 includes the emulsification membrane, the liquid droplets may be prepared by, for example, using a direct membrane emulsification method or pumping method with a Shirasu porous glass (SPG) membrane of SPG Technology Co., Ltd. or the like. When the distribution unit 101 including the emulsification membrane is used, for example, liquid droplets each having a diameter of from 1 μm to 10 μm may be prepared in a combination of Isopar L (aliphatic hydrocarbon, manufactured by Exxon Mobil Corporation) and KF-6038 (surfactant, manufactured by Shin-Etsu Chemical Co., Ltd.). In addition, when emulsification membranes having different pore diameters are used, the average liquid droplet diameter may be changed.

When the distribution unit 101 includes the microchannels, for example, microchannels of Dolomite Microfluidics may be used as the microchannels. When the distribution unit 101 including the microchannels is used, for example, nearly monodisperse liquid droplets each having a diameter of from 1 μm to 10 μm may be prepared in each of the following three combinations. In addition, when microchannels having different nozzle sizes are used, the average liquid droplet diameter may be changed.

·A combination of Isopar L (aliphatic hydrocarbon, manufactured by Exxon Mobil Corporation) and KF-6038 (surfactant, manufactured by Shin-Etsu Chemical Co., Ltd.)
·A combination of a mineral oil (aliphatic hydrocarbon) and SPAN-80 (surfactant, manufactured by Tokyo Chemical Industry Co., Ltd.)
·Automated Droplet Generator oil (manufactured by Bio-Rad Laboratories, Inc) alone

When the individual separated compartments are wells, the distribution unit 101 includes an injection port portion, and the solution is injected into the wells from the injection port portion of the well array, for example, via a nozzle through use of the injection port portion.

In the distribution unit 101, the sample containing the extracellular vesicle, and the reagent containing the reporter molecule may be mixed in advance before being distributed into the individual separated compartments, thus being distributed as a reaction solution, or may be each individually distributed so as to be mixed with each other in the individual separated compartments. The sample containing the extracellular vesicle, and the reporter molecule are preferably mixed in advance before being distributed to the individual separated compartments because a uniformly mixed reaction solution can be easily obtained.

(Signal Generation Unit)

The signal generation unit 102 modifies the reporter molecule with the enzyme through appropriate adjustment of an environment such as a temperature in accordance with the sample, the kind of the target enzyme, the kind of the reporter molecule, and the like, to thereby generate a signal. For example, an incubator may be used as the signal generation unit 102.

(Signal Detection Unit)

The signal detection unit 103 detects the signal generated with the signal generation unit 102. Any device may be used as the signal detection unit 103 as long as the device can detect the signal in the individual separated compartments, but examples thereof include a plate reader and a fluorescence microscope.

(Identification Unit)

The identification unit 104 may include an extraction unit 105, a determination unit 106, a judgment unit 107, a calculation unit 108, a display unit 109, and a storage unit 110. In the extraction unit 105, the extraction unit 105 extracts information on the number and relative positional relationship of the plurality of individual separated compartments from detection results obtained with the signal detection unit 103. The determination unit 106 determines the signal intensity of each of the individual separated compartments identified with the extraction unit 105 based on the detection results obtained with the signal detection unit 103. The judgment unit 107 judges each individual separated compartment having a signal intensity exceeding the predetermined threshold value based on the signal intensity determined with the determination unit 106. Thus, each individual separated compartment having a signal intensity exceeding the predetermined threshold value is identified.

The identification unit 104 is preferably configured to identify each individual separated compartment having a signal intensity exceeding the predetermined threshold value based on at least any one of a ratio or a difference between the signal intensity of a reference compartment and the signal intensity of each individual separated compartment. Further, the signal intensity of the reference compartment is preferably a signal intensity acquired with a sample free of the extracellular vesicle, and the reporter molecule. The signal intensity of the reference compartment may be determined in the same manner as with the individual separated compartments except that the reference compartment is free of the extracellular vesicle.

The ratio between the signal intensity of the reference compartment and the signal intensity of each individual separated compartment is calculated by, for example, using the signal intensity of the reference compartment as a denominator and the signal intensity of each individual separated compartment as a numerator. The difference between the signal intensity of the reference compartment and the signal intensity of each individual separated compartment is calculated by, for example, subtracting the signal intensity of each individual separated compartment from the signal intensity of the reference compartment. When the value obtained based on the ratio and/or difference between the signal intensity of the reference compartment and the signal intensity of an individual separated compartment is less than the predetermined threshold value, the judgment unit 107 deems the signal intensity of that individual separated compartment to be equivalent to that of the reference compartment, and judges the individual separated compartment to be negative. In addition, when the value obtained based on the ratio and/or difference between the signal intensity of the reference compartment and the signal intensity of an individual separated compartment is equal to or more than the predetermined threshold value, the judgment unit 107 judges that individual separated compartment to be positive.

The calculation unit 108 calculates the concentration of the extracellular vesicle in the sample based on the identified number of individual separated compartments each having a signal intensity exceeding the predetermined threshold value. When the concentration of the extracellular vesicle in the sample is not calculated, the identification unit 104 does not need to include the calculation unit 108.

The display unit 109 displays information acquired or extracted with the extraction unit 105, the determination unit 106, the judgment unit 107, and the calculation unit 108. The storage unit 110 stores data and the like acquired or extracted with the extraction unit 105, the determination unit 106, the judgment unit 107, and the calculation unit 108.

In the extracellular vesicle detection apparatus according to this embodiment, the signal detection unit 103 may be an image acquisition unit. FIG. 7 is a functional block diagram for illustrating an extracellular vesicle detection apparatus 20 including an image acquisition unit 111 as the signal detection unit 103 in a configuration similar to that of the above-mentioned extracellular vesicle detection apparatus 10.

The image acquisition unit 111 acquires an image containing the individual separated compartments or a particle complex of the extracellular vesicle and the particle to which the capture protein is bound. The image to be acquired with the image acquisition unit 111 is an image containing the signal generated with the signal generation unit 102 as image information. For example, a fluorescence microscope may be used as the image acquisition unit 111 in the case where the signal is fluorescence.

In the extracellular vesicle detection apparatus 20, the extraction unit 105 included in the identification unit 104 preferably extracts information on the number and relative positional relationship of the plurality of individual separated compartments based on the image acquired with the image acquisition unit 111. That is, in the extracellular vesicle detection apparatus 20, the identification unit 104 preferably identifies each individual separated compartment having a signal intensity exceeding the predetermined threshold value by processing the image acquired with the image acquisition unit 111.

For example, the extraction unit 105 extracts information on the individual separated compartments through use of a region extraction method using brightness information in the case where the signal is fluorescence. In particular, when the individual separated compartments are liquid droplets, regions corresponding to the liquid droplets on the image each have an outline, and hence the extraction unit 105 may perform processing for extracting the edge of the outline as a closed curve. In addition, the extraction unit 105 may extract the regions corresponding to the liquid droplets on the image by binarizing the image based on brightness information.

Further, the determination unit 106 determines the signal intensity of each of the individual separated compartments based on brightness information that each individual separated compartment on the image has.

FIG. 8 is a block diagram for illustrating a hardware configuration example of the extracellular vesicle detection apparatus 10 according to this embodiment. The extracellular vesicle detection apparatus 10 includes a distribution device 401, a signal generation device 402, a signal detection device 403, and an information processing system 404. The information processing system 404 may be, for example, an individual separated compartment identification device.

The distribution device 401, the signal generation device 402, and the signal detection device 403 are devices for executing the functions of the distribution unit 101, the signal generation unit 102, and the signal detection unit 103, respectively.

The information processing system 404 has functions of a computer. For example, the information processing system 404 may be configured unitarily with a desktop personal computer (PC), a laptop PC, a tablet PC, a smartphone, or the like. The information processing system 404 has a function of identifying each individual separated compartment having a signal intensity exceeding a predetermined threshold value. In addition, the information processing system 404 may further have a function of controlling the operations of the distribution device 401, the signal generation device 402, and the signal detection device 403 in accordance with a predetermined program.

The information processing system 404 includes, in order to implement functions as a computer that performs arithmetic operation and storage, a central processing unit (CPU) 406, a random-access memory (RAM) 407, a read-only memory (ROM) 408, and a hard disk drive (HDD) 409. In addition, the information processing system 404 also includes a communication interface (I/F) 410, a display device 411, and an input device 412. The CPU 406, the RAM 407, the ROM 408, the HDD 409, the communication I/F 410, the display device 411, and the input device 412 are connected to each other via a bus 405. The display device 411 and the input device 412 may be connected to the bus 405 via a drive device (not shown) for driving those devices.

In FIG. 8, the various components forming the information processing system 404 are illustrated as an integrated device, but part of the functions of those components may be implemented by an external device. For example, the display device 411 and the input device 412 may be external devices different from the components implementing the functions of the computer including the CPU 406 and the like.

The CPU 406 performs predetermined operations in accordance with programs stored in, for example, the RAM 407 and the HDD 409, and also has a function of controlling each component of the information processing system 404. The RAM 407 is built from a volatile storage medium, and provides a temporary memory area required for the operations of the CPU 406. The ROM 408 is built from a non-volatile storage medium, and stores required information such as programs to be used for the operations of the information processing system 404. The HDD 409 is a storage device which is built from a non-volatile storage medium, and which stores information on, for example, the number of individual separate compartments and positions thereof and signal intensities.

The communication I/F 410 is a communication interface based on a standard, such as Wi-Fi (trademark) or 4G, and is a module for communicating to and from another device. The display device 411 is, for example, a liquid crystal display or an organic light emitting diode (OLED) display, and is used for displaying moving images, still images, characters, and the like. The input device 412 is, for example, a button, a touch panel, a keyboard, or a pointing device, and is used by a user to operate the information processing system 404. The display device 411 and the input device 412 may be integrally formed as a touch panel.

The hardware configuration illustrated in FIG. 8 is an example, and devices other than the illustrated devices may be added, or part of the illustrated devices may be omitted. In addition, part of the devices may be substituted with another device having the same function. Further, part of the functions may be provided by another device via a network, and the functions for implementing the embodiments may be shared and implemented by a plurality of devices. For example, the HDD 409 may be substituted with a solid state drive (SSD) using a semiconductor element such as a flash memory, or may be substituted with cloud storage.

The CPU 406 implements functions of the extraction unit 105, the determination unit 106, the judgment unit 107, and the calculation unit 108 by loading the program stored in the ROM 408 or another place onto the RAM 407 and executing the program. The CPU 406 also implements a function of the display unit 109 by controlling the display device 411. The CPU 406 implements a function of the storage unit 110 as well by controlling the HDD 409.

As a hardware configuration example of the extracellular vesicle detection apparatus 20 according to this embodiment, there is given an example including as the fluorescence detection device 403 an image acquisition device for executing the function of the image acquisition unit 111 in the hardware configuration example of the extracellular vesicle detection apparatus 10 described above. The hardware configuration of the extracellular vesicle detection apparatus 20 may be the same as that of the extracellular vesicle detection apparatus 10 except the foregoing.

According to still another embodiment of the present invention, there is provided a program for causing a computer included in an extracellular vesicle detection apparatus to execute the method of detecting an extracellular vesicle so as to cause the extracellular vesicle detection apparatus to execute the method.

[Examples]

The present invention is described in more detail below by way of Examples. However, the present invention is by no means limited to Examples described below.

(Materials and the like)

Extracellular vesicles, reporter molecules, and standard fluorescent substances used in Examples are described.

<Extracellular Vesicles (sometimes abbreviated as "EV" or "EVs")>

The following commercially available purified exosomes (manufactured by System Biosciences, LLC) were used as extracellular vesicles. EXOP-105A-1: Frozen exosomes (concentration: >$1\times10^6$) from MDA-MB-231 (Human Breast Cancer, Aggressive/Invasive/Metastatic Cell Line): EXOP-120A-1: Frozen exosomes (concentration: >$1\times10^6$) from A549 (Human Non-small Cell Lung Cancer Cell Line):
In addition, the following commercially available purified exosomes (manufactured by FUJIFILM Wako Pure Chemical Corporation) were also used as extracellular vesicles.
052-09301, Exosomes, from COLO201 cells, purified (50 μL):
Exosome concentration: 10 μg/mL (protein concentration estimated from a CD63 signal value)

<Reporter Molecule for MMP Detection>

For detection of the enzymatic activity of a MMP, the following commercially available MMP activity measurement kit SensoLyte (manufactured by AnaSpec, Inc.) and MMP-14 Substrate I available from Merck KGaA were used.

A reporter molecule of SensoLyte is included in SensoLyte 520 MMP-14 Assay Kit (Fluorimetric) AS-72025, is a FRET peptide having 5-FAM serving as a fluorescent substance and QXL520 (trademark) serving as a quencher, and emits fluorescence with Ex/Em=490 nm/520 nm by cleavage of a peptide chain by MMP14.

In addition, for detection of various MMPs, SensoLyte 520 MMP Substrate Sampler Kit (Fluorimetric) AS-71170 was used. Each of the substrates is a FRET peptide having 5-FAM serving as a fluorescent substance and QXL520 serving as a quencher, and emits fluorescence with Ex/Em=490 nm/520 nm by cleavage of a peptide chain by the various MMPs.

Among the 16 kinds of substrates, the following 6 kinds were used.

SB2 MMP-1/7/8/12/13
QXL520-Pro-Leu-Ala-Leu-Trp-Ala-Arg-Lys(5-FAM)-$NH_2$
SB6 MMP-2/13

QXL520-Pro-Leu-Gly-Met-Trp-Ser-Arg-Lys(5-FAM)-$NH_2$
SB7 MMP-7/12/13
QXL520-Pro-Tyr-Ala-Tyr-Trp-Met-Arg-Lys(5-FAM)-$NH_2$
SB9 MMP-1/2/7/8/12/13
QXL520-Arg-Pro-Leu-Ala-Leu-Trp-Arg-Lys(5-FAM)-$NH_2$
SB10 MMP-13
QXL520-Pro-Leu-Ala-Tyr-Trp-Ala-Arg-Lys(5-FAM)-$NH_2$
SB14 MMP-1/2/3/7/8/9/10/12/13/14/15/16/24
QXL520-γ-Abu-Pro-Cha-Abu-Smc-His-Ala-Dab(5-FAM)-Ala-Lys-$NH_2$
(Smc=S-Methyl-L-cysteine)

In addition, a reporter molecule (MMP-14 Substrate I) of MMP-14 Substrate I, Fluorogenic (Calbiochem: 444258) is a FRET peptide having MCA serving as a fluorescent substance and DPA serving as a quencher, and emits fluorescence with Ex/Em=325 nm/392 nm by cleavage of a peptide chain by MMP14. That is, the reporter molecule has the following sequence.

Peptide sequence: MCA-Pro-Leu-Ala-Cys(p-OMeBz)-Trp-Ala-Arg(Dpa)-$NH_2$
Dpa represents $N^3$-(2,4-dinitrophenyl)-L-2,3-diaminopropionyl.

Bz represents benzyl.

DMSO was added to a lyophilizate of each of the peptides to prepare a 1 mM solution.

In addition, the following custom-synthesized FRET peptides obtained through contract synthesis by Cosmo Bio Co., Ltd., were used. The peptides are called Custom #1, Custom #2, Custom #11, Custom #12, Custom #13, Custom #14, Custom #15, and Custom #16, respectively. Each of the peptides is a FRET peptide having 5-FAM serving as a fluorescent substance and TQ2 serving as a quencher, and emits fluorescence with Ex/Em=490 nm/520 nm by cleavage of a peptide chain by MMP14.

Custom #1: [5-FAM]GRIGFLRTAK(TQ2)[OH]
Custom #2: [5-FAM]GGPLGLAGGK(TQ2)[OH]
Custom #11: TQ2-LAPLGLQRRK(5-FAM)
Custom #12: TQ2-FMPLGLRGIK(5-FAM)
Custom #13: TQ2-WYPAGLRMVK(5-FAM)
Custom #14: TQ2-DLPAGLQARK(5-FAM)
Custom #15: TQ2-YPPRPLLARK(5-FAM)
Custom #16: TQ2-WPHGSLQAAK(5-FAM)

In addition, the following custom-synthesized FRET peptides obtained through contract synthesis by Cosmo Bio Co., Ltd., were used as different FRET dye pairs of Custom #2. The peptides are called Custom #2T and Custom #2TF3, respectively. Custom #2T is a FRET peptide having TAMRA serving as a fluorescent substance and TQ3 serving as a quencher, and emits fluorescence with Ex/Em=553 nm/575 nm by cleavage of a peptide chain by MMP14. In addition, Custom #2TF3 is a FRET peptide having TF3 serving as a fluorescent substance and TQ3 serving as a quencher, and emits fluorescence with Ex/Em=555 nm/584 nm by cleavage of a peptide chain by MMP14.

Custom #2T: TAMRA-GGPLGLAGGK(TQ3)
Custom #2TF3: TF3-GGPLGLAGGK(TQ3)
TF3 (Tide Fluor 3)
TQ3 (Tide Quencher 3)

DMSO was added to a lyophilizate of each of the peptides to prepare a 1 mM solution.

<Reporter Molecule for AChE>

For detection of the enzymatic activity of AChE, the following commercially available AChE activity measurement kits SensoLyte (manufactured by AnaSpec, Inc.) and Amplite (manufactured by AAT Bioquest, Inc.) were used.

A reporter molecule of SensoLyte is included in 520 Acetylcholinesterase Activity Assay Kit (AS-72242), is a FRET peptide having 5-FAM serving as a fluorescent substance and QXL520 serving as a quencher, and emits fluorescence with Ex/Em=490 nm/520 nm by cleavage of a peptide chain by AChE.

A reporter molecule of Amplite is included in Amplite Fluorimetric Acetylcholinesterase Assay Kit (Green Fluorescence) 11401, and reacts with a hydrolyzed thiocholine that reflects AChE activity in a sample solution to cause a green fluorescent dye Tiolite Green to emit fluorescence with Ex/Em=510 nm/525 nm.

<Reporter Molecule for ADAM>

For detection of the enzymatic activity of ADAM, the following commercially available ADAM activity measurement substrates (manufactured by BioZyme Inc.) were used. Each of the substrates is a FRET peptide having 5-FAM serving as a fluorescent substance and Dabcyl serving as a quencher, and emits fluorescence with Ex/Em=490 nm/520 nm by cleavage of a peptide chain by ADAM.

ADAM Substrate II (Fluorogenic)-PEPDAB010
Dabcyl-SPLAQAVRSSK(5FAM)-$NH_2$
(Dabcyl=4-((4-(dimethylamino)phenyl)azo)benzoic acid)
ADAM17 Selective Substrate (Fluorogenic)-PEPDAB014
Dabcyl-EHADLLAVVAK(5FAM)-$NH_2$
DMSO was added to a lyophilizate of each of the substrates to prepare a 1 mM solution.

<Reporter Molecule for Cathepsin D>

In addition, for detection of the enzymatic activity of cathepsin D, commercially available Cathepsin D Assay Kit, Fluorimetric, SensoLyte 520 AS-72170 (manufactured by AnaSpec, Inc.) was used. A reporter molecule is a FRET peptide having HiLyte Fluor (trademark) 488 serving as a fluorescent substance and QXL520 serving as a quencher, and emits fluorescence with Ex/Em=497 nm/525 nm by cleavage of a peptide chain by cathepsin D.

<Standard Fluorescent Substance>

Alexa Fluor (trademark) 647 (manufactured by Molecular Probes (trademark)) serving as a standard fluorescent substance for recognizing liquid droplets or wells to be described later was dissolved in DMSO to prepare a standard fluorescent substance solution (1 mM) to be used.

Example 1

(Detection of MMP-14 Activity with SensoLyte 520 MMP-14 Assay Kit (Fluorimetric))

10 μg of a MMP14 lyophilizate using Recombinant human MMP14 protein (Active) (manufactured by Abcam plc.): ab285993 as a MMP14 standard was dissolved in 100 μL of purified water. 5 μL of the solution was aliquoted, and Assay buffer (Component D) included in the kit was added to provide 120 μL of a solution. The MMP14 concentration of the solution is 4.17 μg/mL. The concentration of the solution was set to x1, and 3-fold serial dilution was performed to prepare standard samples with seven concentrations. Further, a sample free of MMP14 was prepared similarly, and used as a sample for background (BG) measurement.

In addition, for detection of MMP14 activity, 4.95 mL of Assay buffer (Component D) was added to 50 μL of a MMP-14 substrate (Component A) included in the kit to provide 5 mL of a MMP14 detection solution.

50 μL of the MMP14 detection solution was added to 50 μL of each of the samples, a reaction was performed at 37°C, and MMP14 activity was evaluated.

(Evaluation of MMP14 Activity with 96-well Plate)

The MMP14 activity was evaluated with a 96-well plate (manufactured by Thermo Fisher Scientific Inc., model number: 137101). A fluorescence intensity was measured with a fluorescence plate reader (Synergy MX, manufactured by BioTek Instruments, Inc.) at 37°C every 5 min for 1 hour. For measurement wavelengths, an excitation wavelength of 485 ±20 nm and a fluorescence wavelength of 528±20 nm were used.

A relative fluorescence unit difference ΔRFU was calculated by subtracting the measured value of the sample for BG measurement from the measured value of each of the samples.

FIG. 9A shows the MMP14 concentration and the fluorescence intensity after 1 hour. As shown in FIG. 9A, MMP14 activity depending on the MMP14 concentration was able to be detected. In addition, FIG. 9B shows five plots on the low concentration side and an approximated straight line. It is found that a low concentration region has satisfactory linearity.

Example 2

(Detection of MMP-14 Activity of MDA-MB-231-derived Extracellular Vesicles with SensoLyte 520 MMP-14 Assay Kit (Fluorimetric))

5 μL (corresponding to 5 μg) of the MDA-MB-231-derived extracellular vesicles (50 μg of protein in 50 μL of PBS) described in the section "Materials and the like" were aliquoted, and Assay buffer (Component D) included in the kit was added to the aliquot to provide 120 μL of a solution. The concentration of the solution is 41.7 μg/mL. The concentration of the solution was set to x1, and 2-fold serial dilution was performed to prepare standard samples with six concentrations. Further, a sample free of extracellular vesicles was prepared similarly, and used as a sample for BG measurement.

In addition, for detection of MMP14 activity, 4.95 mL of Assay buffer (Component D) was added to 50 μL of a MMP-14 substrate (Component A) included in the kit to provide 5 mL of a MMP14 detection solution.

50 μL of the MMP14 detection solution was added to 50 μL of each of the samples, a reaction was performed at 37°C, and MMP14 activity was evaluated.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 10 shows the MDA-MB-231-derived extracellular vesicle concentration and the fluorescence intensity after 1 hour. As shown in FIG. 10, MMP14 activity depending on the extracellular vesicle concentration was able to be detected. Satisfactory linearity is found in the evaluated concentration region. From comparison to the slope of the approximated straight line in Example 1, about 0.86% of the weight of an extracellular vesicle protein may be estimated to correspond to MMP14 in terms of recombinant MMP14 with a molecular weight of 24 kDa in Example 1. In addition, 2.5% thereof may be estimated to correspond to MMP14 in terms of intact MMP14 with a molecular weight of 70 kDa.

Example 3

(Detection of MMP-14 Activity of MDA-MB-231-derived Extracellular Vesicles with Calbiochem MMP-14 Substrate I, Fluorogenic)

2 μL (corresponding to 2 μg) of the MDA-MB-231-derived extracellular vesicles (50 μg of protein in 50 μL of PBS) sterile 1x PBS with 50 μg exosomes protein) described in the section "Materials and the like" were aliquoted, and Assay buffer (Component D) included in SensoLyte 520 MMP-14 Assay Kit was added to the aliquot to provide 200 μL of a solution. The concentration of the solution is 10 μg/mL. Further, a sample free of extracellular vesicles was prepared similarly, and used as a sample for BG measurement.

In addition, for detection of MMP14 activity, 1 mM MMP-14 Substrate I was diluted with Assay buffer (Component D) included in SensoLyte 520 MMP-14 Assay Kit to prepare 20 μM, 10 μM, and 5 μM MMP14 detection solutions.

50 μL of each of the MMP14 detection solutions was added to 50 μL of each of the samples, a reaction was performed at 37°C, and MMP14 activity was evaluated.

In this case, the concentration of the extracellular vesicles in each of the reaction solutions was 5 μg/mL, and the concentration of the reporter molecule in each of the reaction solutions was 10 μM, 5 μM, or 2.5 μM.

(Evaluation of MMP14 Activity with 96-well Plate)

Evaluation was performed in the same manner as in Example 1 except that: as the measurement wavelengths, the excitation wavelength was set to 325±20 nm and the fluorescence wavelength was set to 392±20 nm; and the reaction time was set to 2 hours. ΔRFU was calculated by subtracting the measured value of the sample for BG measurement from the measured value of each of the samples.

FIG. 11 shows temporal changes in fluorescence intensity from the MMP-14 Substrate I by the MDA-MB-231-derived extracellular vesicles. As shown in FIG. 11, it is found that the fluorescence intensity was increased with the reaction time, and MMP14 activity was able to be detected.

Example 4

(Detection of MMP-14 Activity of MDA-MB-231-derived Extracellular Vesicle with Reporter Molecule: Custom #1 or Custom #2)

2 μL (corresponding to 2 μg) of the MDA-MB-231-derived extracellular vesicles (50 μg of protein in 50 μL of PBS) described in the section "Materials and the like" were aliquoted, and Assay buffer (Component D) included in SensoLyte 520 MMP-14 Assay Kit was added to the aliquot to provide 200 μL of a solution. The concentration of the solution is 10 μg/mL. Further, a sample free of extracellular vesicles was prepared similarly, and used as a sample for BG measurement.

In addition, for detection of MMP14 activity, 1 mM Custom #1 or Custom #2 was diluted with Assay buffer (Component D) included in SensoLyte 520 MMP-14 Assay Kit to prepare a 10 μM MMP14 detection solution. In addition, the SensoLyte 520 MMP-14 detection solution of Example 1 was used as a control.

50 μL of each of the MMP14 detection solutions was added to 50 μL of each of the samples, a reaction was performed at 37°C, and MMP14 activity was evaluated.

In this case, the concentration of the extracellular vesicles in each of the reaction solutions is 5 μg/mL, and the concentration of the reporter molecule in each of the reaction solutions is 5 μM.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 12 shows temporal changes in fluorescence intensities from the various reporter molecules by the MDA-MB-231-derived extracellular vesicles. As shown in FIG. 12, it is found that the fluorescence intensity was increased with the reaction time, and MMP14 activity was able to be detected. In addition, the increase in fluorescence intensity from each of Custom #1 and Custom #2 is more significant than that from SensoLyte 520 MMP-14.

Example 5

(Detection of MMP-14 Activity of MDA-MB-231-derived Extracellular Vesicle with Reporter Molecule: Custom #1 or Custom #2)

4 μL (corresponding to 4 μg) of the MDA-MB-231-derived extracellular vesicles (50 μg of protein in 50 μL of PBS) described in the section "Materials and the like" were aliquoted, and Assay buffer (Component D) included in SensoLyte 520 MMP-14 Assay Kit was added to the aliquot to provide 200 μL of a solution. The concentration of the solution is 20 μg/mL. The concentration of the solution was set to x1, and 3-fold serial dilution was performed to prepare standard samples with three concentrations. Further, a sample free of extracellular vesicles was prepared similarly, and used as a sample for BG measurement.

In addition, for detection of MMP14 activity, 1 mM Custom #1 or Custom #2 was diluted with Assay buffer (Component D) included in SensoLyte 520 MMP-14 Assay Kit to prepare a 10 μM MMP14 detection solution.

50 μL of the MMP14 detection solution was added to 50 μL of each of the samples, a reaction was performed at 37°C, and MMP14 activity was evaluated.

In this case, the highest concentration of the extracellular vesicles in each of the reaction solutions is 10 μg/mL, and the concentrations of the reporter molecule in the reaction solutions are all 5 μM.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 13A shows temporal changes in fluorescence intensities from the various reporter molecules by differences in MDA-MB-231-derived extracellular vesicle concentration. As shown in FIG. 13A, it is found that the fluorescence intensity varies depending on the extracellular vesicle concentration. In addition, it is found that the fluorescence intensity from Custom #1 is higher than the fluorescence intensity from Custom #2 at the same extracellular vesicle concentration.

The temporal increase in fluorescence intensity within 1 hour of reaction is approximated linearly, and plots of the slope of the increase (a reaction rate) with regard to each of the reporter molecules and each of the extracellular vesicle concentrations are shown in FIG. 13B. From the approximated straight lines, the reaction rates per 1 μg/mL with regard to Custom #1 and Custom #2 may be estimated to be 101.5 ΔRFU/min and 83.3 ARFU/min, respectively.

Example 6

(Investigation of Reaction Solution Composition for Detection of MMP-14 Activity with Reporter Molecule: Custom #1)

5 μL (corresponding to 5 μg) of the MDA-MB-231-derived extracellular vesicles (50 μg of protein in 50 μL of PBS) described in the section "Materials and the like" were aliquoted, and the following buffer (1) was added to the aliquot to provide 500 μL of a solution.

Buffer (1)
50 mM Tris-Cl (pH: 7.6)
10 mM $CaCl_2$

The concentration of the solution is 10 μg/mL. Further, a sample free of extracellular vesicles was prepared similarly, and used as a sample for BG measurement.
In addition, for detection of MMP14 activity, 1 mM Custom #1 was diluted with the buffer (1) to prepare a 10 μM MMP14 detection solution.
500 μL of the MMP14 detection solution was added to 500 μL of the sample and mixed therein, and the mixture was then aliquoted into 100 μL portions. To each of the portions, NaCl and Brij35 were added as described below. The amounts (μL) of NaCl and Brij35 added to 100 μL of each reaction solution are described below.

| Composition | 3 M NaCl | 1% Brij35 |
|---|---|---|
| 1 | 5 (final concentration: 150 mM) | 5 (final concentration: 0.05%) |
| 2 | 5 | 2.5 (final concentration: 0.025%) |
| 3 | 5 | 1.25 (final concentration: 0.0125%) |
| 4 | 5 | 0 (final concentration: 0%) |
| 5 | 0 (final concentration: 0 mM) | 5 |
| 6 | 0 | 2.5 |
| 7 | 0 | 1.25 |
| 8 | 0 | 0 |

After the mixing, a reaction was performed at 37°C, and MMP14 activity was evaluated.
In this case, the concentration of the extracellular vesicles in each of the reaction solutions is 5 μg/mL, and the concentration of the reporter molecule in each of the reaction solutions is 5 μM.

(Evaluation of MMP14 Activity with 96-well Plate)

Evaluation was performed in the same manner as in Example 1.
FIG. 14A shows temporal changes in fluorescence intensity from Custom #1 by differences in Brij35 concentration in the case where NaCl is contained at a final concentration of 150 mM. It is found that the fluorescence intensity is reduced even when the Brij35 concentration is high or zero. In addition, FIG. 14B shows temporal changes in fluorescence intensity from Custom #1 by differences in Brij35 concentration in the case where NaCl is not contained. It is found that the fluorescence intensity is reduced even when the Brij35 concentration is high or zero similarly to the case where NaCl is contained at a final concentration of 150 mM. FIG. 14C shows temporal changes in fluorescence intensity from Custom #1 by the presence or absence of NaCl in the case where Brij35 is contained at a final concentration of 0.0125%. It is found that the fluorescence intensity in the case where NaCl is not contained becomes higher.

Example 7

(Investigation of Reaction Solution Composition for Detection of MMP-14 Activity with Reporter Molecule: Custom #1 or Custom #2)

5 μL (corresponding to 5 μg) of the MDA-MB-231-derived extracellular vesicles (50 μg of protein in 50 μL of PBS) described in the section "Materials and the like" were aliquoted, and the above-mentioned buffer (1) was added to the aliquot to provide 500 μL of a solution.
The concentration of the solution is 10 μg/mL. Further, a sample free of extracellular vesicles was prepared similarly, and used as a sample for BG measurement.
In addition, for detection of MMP14 activity, 1 mM Custom #1 or Custom #2 was diluted with the buffer (1) to prepare a 10 μM MMP14 detection solution.
500 μL of the MMP14 detection solution was added to 500 μL of the sample and mixed therein, and the mixture was then aliquoted into 100 μL portions. To each of the portions, a 0.5% Brij35 solution was added as described below. The amounts (μL) of the 0.5% Brij35 solution added to 100 μL of each reaction solution are described below.

| Composition | 0.5% Brij35 | Custom |
|---|---|---|
| 9 | 4 (final concentration: 0.02%) | #1 |

(continued)

| Composition | 0.5% Brij35 | Custom |
|---|---|---|
| 10 | 3 (final concentration: 0.015%) | #1 |
| 11 | 2 (final concentration: 0.01%) | #1 |
| 12 | 1 (final concentration: 0.005%) | #1 |
| 13 | 4 | #2 |
| 14 | 3 | #2 |
| 15 | 2 | #2 |
| 16 | 1 | #2 |

After the mixing, a reaction was performed at 37°C, and MMP14 activity was evaluated.

In this case, the concentration of the extracellular vesicles in each of the reaction solutions is 5 μg/mL, and the concentration of the reporter molecule in each of the reaction solutions is 5 μM.

(Evaluation of MMP14 Activity with 96-well Plate)

Evaluation was performed in the same manner as in Example 1.

FIG. 15A shows temporal changes in fluorescence intensity from Custom #1 by differences in Brij35 concentration. It is found that the fluorescence intensity is reduced when the Brij35 concentration is high. In addition, FIG. 15B shows temporal changes in fluorescence intensity from Custom #2 by differences in Brij35 concentration. It is found that the differences in Brij35 concentration in this range only slightly affect the fluorescence intensity unlike the case of Custom #1.

Example 8

(Detection of MMP-14 Activity of MDA-MB-231-derived Extracellular Vesicle with Reporter Molecule: Custom #1 or Custom #2)

4 μL (corresponding to 4 μg) of the MDA-MB-231-derived extracellular vesicles (50 μg of protein in 50 μL of PBS) described in the section "Materials and the like" were aliquoted, and the following buffer (2) was added to the aliquot to provide 200 μL of a solution. The concentration of the solution is 20 μg/mL. The concentration of the solution was set to x1, and 3-fold serial dilution was performed to prepare standard samples with three concentrations. Further, a sample free of extracellular vesicles was prepared similarly, and used as a sample for BG measurement.

Buffer (2)
50 mM Tris-Cl (pH: 7.6)
10 mM $CaCl_2$
0.0125% Brij35

In addition, for detection of MMP14 activity, 1 mM Custom #1 or Custom #2 was diluted with the buffer (2) to prepare a 10 μM MMP14 detection solution.

50 μL of the MMP14 detection solution was added to 50 μL of each of the samples, a reaction was performed at 37°C, and MMP14 activity was evaluated.

In this case, the concentration of the extracellular vesicles in the reaction solution was 10 μg/mL, and the concentrations of the reporter molecules in the reaction solution were all 5 μM.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 16A shows temporal changes in fluorescence intensities from the various reporter molecules by differences in MDA-MB-231-derived extracellular vesicle concentration. As shown in FIG. 16A, it is found that the fluorescence intensity varies depending on the extracellular vesicle concentration. In addition, it is found that the fluorescence intensity from Custom #1 is higher than the fluorescence intensity from Custom #2 at the same extracellular vesicle concentration.

The temporal increase in fluorescence intensity within 1 hour of reaction is approximated linearly, and plots of the slope of the increase (a reaction rate) with regard to each of the reporter molecules and each of the extracellular vesicle concentrations are shown in FIG. 16B. From the approximated straight lines, the reaction rates per 1 μg/mL with regard to Custom #1 and Custom #2 may be estimated to be 198 ΔRFU/min and 99.0 ΔRFU/min, respectively. It is found that both of the reaction rates with regard to Custom #1 and Custom #2 are improved in the buffer (2) as compared to 101.5 ΔRFU/min and 83.3 ΔRFU/min, respectively, in Assay buffer (Component D) included in SensoLyte 520 MMP-14 Assay Kit in Example 5.

Example 9

(Detection of MMP-14 Activity of MDA-MB-231-derived Extracellular Vesicle with Reporter Molecule: Custom #1 or Custom #2)

4 μL (corresponding to 4 μg) of the MDA-MB-231-derived extracellular vesicles (50 μg of protein in 50 μL of PBS) described in the section "Materials and the like" were aliquoted, and the following buffer (3) was added to the aliquot to provide 500 μL of a solution. The concentration of the solution is 8 μg/mL.

Buffer (3)
50 mM Tris-Cl (pH: 7.6)
10 mM $CaCl_2$
0.005% Brij35

In addition, for detection of MMP14 activity, 1 mM Custom #1 or Custom #2 was diluted with the buffer (3) to prepare a 20 μM MMP14 detection solution. The concentration of the solution was set to x1, and 2-fold serial dilution was performed to prepare MMP14 detection solutions with five concentrations.
50 μL of each of the MMP14 detection solutions was added to 50 μL of the sample, a reaction was performed at 37°C, and MMP14 activity was evaluated.
In this case, the concentration of the extracellular vesicles in each of the reaction solutions is 4 μg/mL, and the concentration of the reporter molecule in each of the reaction solutions is 10 μM, 5 μM, 2.5 μM, 1.25 μM, or 0.625 μM.

(Evaluation of MMP14 Activity with 96-well Plate)

Evaluation was performed in the same manner as in Example 1. ΔRFU was calculated by subtracting the measured value at 0 minutes from the measured value of each of the samples.
FIG. 17A shows temporal changes in fluorescence intensity by differences in Custom #1 concentration. As shown in FIG. 17A, it is found that the fluorescence intensity varies depending on the Custom #1 concentration. In addition, it is found that the fluorescence intensity largely varies at 2.5 μM or less.
FIG. 17B shows temporal changes in fluorescence intensity by differences in Custom #2 concentration. As shown in FIG. 17B, it is found that the fluorescence intensity varies depending on the Custom #2 concentration. In addition, it is found that the fluorescence intensity largely varies at 5 μM or less.
The temporal increase in fluorescence intensity within 1 hour of reaction is approximated linearly, and Lineweaver-Burk plots from the slope of the increase (a reaction rate) with regard to each of the reporter molecule concentrations are shown in FIG. 17C. In addition, values of Vmax, Km, and Vmax/Km calculated from the Lineweaver-Burk plots are shown below.

[Table 1]

Table 1

| Reporter molecule | Custom #1 | Custom #2 |
|---|---|---|
| Vmax (ΔRFU/min) | 1,278.77 | 582.411 |
| Km (μM) | 0.44306 | 0.51116 |
| Vmax/Km (ΔRFU/min·μM) | 2,886.23 | 1,139.38 |

It is found that Custom #1 is cleaved about 2.5 times more easily than Custom #2 under the above-mentioned reaction conditions.

Example 10

(Detection of MMP-14 Activity of MDA-MB-231, A549, or COLO201-derived Extracellular Vesicle with Reporter Molecule: Custom #1 or Custom #2)

2 μL (corresponding to 2 μg for the MDA-MB-231 and A549-derived extracellular vesicles, and corresponding to 0.4 μg for the COLO201-derived extracellular vesicles) of each of the MDA-MB-231-derived extracellular vesicles (50 μg of protein in 50 μL of PBS), the A549-derived extracellular vesicles (50 μg of protein in 50 μL of PBS), and the COLO201-derived extracellular vesicles (10 μg of protein/mL) described in the section "Materials and the like" was aliquoted, and the buffer (3) was added to the aliquot to provide 200 μL of each solution. The concentrations of the extracellular vesicles in the solutions were as follows: 10 μg/mL for each of the MDA-MB-231 and A549-derived extracellular vesicles; and 2 μg/mL for the concentration of the COLO201-derived extracellular vesicles. The concentration of 20 μg/mL was set to x1 in Example 8, and hence, with regard to each of the MDA-MB-231 and A549-derived extracellular vesicles, the concentration of the solution was set to x1/2, and 3-fold serial dilution was performed to prepare standard samples with three concentrations. With regard to the COLO201-derived extracellular vesicles, the concentration of the solution was set to x1/10, and 3-fold serial dilution was performed to prepare standard samples with three concentrations.

In addition, for detection of MMP14 activity, 1 mM Custom #1 or Custom #2 was diluted with the buffer (3) to prepare a 10 μM MMP14 detection solution.

50 μL of the MMP14 detection solution was added to 50 μL of each of the samples, a reaction was performed at 37°C, and MMP14 activity was evaluated.

In this case, the highest concentration of the extracellular vesicles in the reaction solutions was 5 μg/mL for the MDA-MB-231 and A549-derived extracellular vesicles, and 1 μg/mL for the COLO201-derived extracellular vesicles, and the concentrations of each of the reporter molecules in the reaction solutions were all 5 μM.

(Evaluation of MMP14 Activity with 96-well Plate)

Evaluation was performed in the same manner as in Example 1.

FIG. 18A shows temporal changes in fluorescence intensity from the various reporter molecules by differences in MDA-MB-231-derived extracellular vesicle concentration. As shown in FIG. 18A, it is found that the fluorescence intensity varies depending on the extracellular vesicle concentration. In addition, it is found that the fluorescence intensity from Custom #1 is higher than the fluorescence intensity from Custom #2 at the same extracellular vesicle concentration. The results satisfactorily reproduce the results of Example 8.

FIG. 18B shows temporal changes in fluorescence intensity from the various reporter molecules by differences in A549-derived extracellular vesicle concentration. As shown in FIG. 18B, it is found that the fluorescence intensity varies depending on the extracellular vesicle concentration. It is found that the fluorescence intensity from Custom #2 is higher than the fluorescence intensity from Custom #1 at the same extracellular vesicle concentration. In this case, in the case of Custom #2, a change in fluorescence intensity almost equivalent to the change in fluorescence intensity in the MDA-MB-231-derived extracellular vesicles is obtained.

FIG. 18C shows temporal changes in fluorescence intensity from the various reporter molecules by differences in COLO201-derived extracellular vesicle concentration. As shown in FIG. 18C, no change in fluorescence intensity was able to be observed. No literatures related to MMP14 with regard to COLO201 were found in PubMed search (by the search formula: COLO201 × MMP, only one literature related to MMP-7 was found), and hence the results are conceived to be reasonable.

Example 11

(Detection of AChE Activity with Amplite Fluorimetric Acetylcholinesterase Assay Kit (Green Fluorescence))

First, the following reagents were prepared.

Thiolite Green stock solution (200-fold)

50 μL of DMSO (Component E) was added to Thiolite Green (Component A) to provide a 200-fold stock solution.

Acetylcholine stock solution (500-fold)

0.6 mL of purified water was added to acetylcholine (Component C) to provide a 500-fold stock solution.

Acetylcholinesterase standard stock solution (50 U/mL)

100 μL of purified water containing 0.1% BSA was added to Acetylcholinesterase Standard (Component D) to provide a 50 U/mL acetylcholinesterase standard solution.

980 μL of Assay Buffer (Component B) was added to 20 μL of the above-mentioned AChE standard solution to prepare a 1,000 mU/mL AChE solution. Further, 10-fold dilution was performed with Assay Buffer (Component B) to provide a 100 mU/mL AChE solution. The concentration of the AChE solution was set to x1, and 3-fold serial dilution was performed to prepare standard samples with seven concentrations. Further, a sample free of AChE was prepared similarly, and used as a sample for BG measurement.

In addition, for detection of AChE activity, 1 μL of the acetylcholine stock solution (500-fold) and 2.5 μL of the Thiolite Green stock solution (200-fold) were added to 500 μL of Assay Buffer (Component B) to provide 503.5 μL of an AChE detection solution.

50 μL of the AChE detection solution was added to 50 μL of each of the samples, a reaction was performed at 37°C, and AChE activity was evaluated. In this case, the highest concentration of AChE in the reaction solutions was 50 mU/mL.

(Evaluation of AChE Activity with 96-well Plate)

Evaluation was performed in the same manner as in Example 1. ΔRFU was calculated by subtracting the measured value of the sample for BG measurement from the measured value of each of the samples.

FIG. 19 shows the AChE concentration and temporal changes in fluorescence intensity from the reporter molecule. As shown in FIG. 19, it is found that the fluorescence intensity varies depending on the AChE concentration. The concentrations of x1/9, x1/27, x1/81, and x1/243 at which activity can be satisfactorily detected correspond to 5.56 mU/mL, 1.85 mU/mL, 0.62 mU/mL, and 0.21 mU/mL, respectively.

Example 12

(Detection of AChE Activity of MDA-MB-231 or A549-derived Extracellular Vesicles with Amplite Fluorimetric Acetylcholinesterase Assay Kit (Green Fluorescence))

4 μL (corresponding to 4 μg for each of the MDA-MB-231 and A549-derived extracellular vesicles) of each of the MDA-MB-231-derived extracellular vesicles (50 μg of protein in 50 μL of PBS) and the A549-derived extracellular vesicles (50 μg of protein in 50 μL of PBS) described in the section "Materials and the like" were aliquoted, and Assay Buffer (Component B) used in Example 11 was added to the aliquot to provide 200 μL of each solution. The concentration of the solution is 20 μg/mL for each of the MDA-MB-231 and A549-derived extracellular vesicles. The concentration of 20 μg/mL was set to x1 in Example 8, and hence the concentration of the solution was set to x1 for each of the MDA-MB-231 and A549-derived extracellular vesicles, and 2-fold serial dilution was performed to prepare standard samples with seven concentrations. Further, a sample free of extracellular vesicles was prepared similarly, and used as a sample for BG measurement.

In addition, for detection of AChE activity, 2 μL of the acetylcholine stock solution (500-fold) and 5 μL of the Thiolite Green stock solution (200-fold) were added to 1,000 μL of Assay Buffer (Component B) to provide 1,007 μL of an AChE detection solution.

50 μL of the AChE detection solution was added to 50 μL of each of the samples, a reaction was performed at 30°C, and AChE activity was evaluated. In this case, the highest concentration of the extracellular vesicles in the reaction solutions was 10 μg/mL for each of the MDA-MB-231 and A549-derived extracellular vesicles.

(Evaluation of AChE Activity with 96-well Plate)

Evaluation was performed in the same manner as in Example 1. ΔRFU was calculated by subtracting the measured value of the sample for BG measurement from the measured value of each of the samples.

FIG. 20A shows the MDA-MB-231-derived extracellular vesicle concentration and temporal changes in fluorescence intensity from the reporter molecule. As shown in FIG. 20A, it is found that the fluorescence intensity varies depending on the extracellular vesicle concentration. In addition, FIG. 20B shows the A549-derived extracellular vesicle concentration and temporal changes in fluorescence intensity from the reporter molecule. As shown in FIG. 20B, it is found that the fluorescence intensity varies depending on the extracellular vesicle concentration. From comparison between the MDA-MB-231 and A549-derived extracellular vesicles, it is found that the AChE activity of the A549-derived extracellular vesicles is strong.

Example 13

(Detection of AChE Activity of MDA-MB-231-derived Extracellular Vesicles with Amplite Fluorimetric Acetylcholinesterase Assay Kit (Green Fluorescence))

1 μL (corresponding to 1 μg) of the MDA-MB-231-derived extracellular vesicles (50 μg of protein in 50 μL of PBS) described in the section "Materials and the like" were aliquoted, and Assay Buffer (Component B) used in Example 11 was added to the aliquot to provide 250 μL of a solution. The concentration of the solution is 4 μg/mL. The concentration of 20 μg/mL was set to x1 in Example 8, and hence the concentration of the solution is x1/5.

In addition, for detection of AChE activity, 1 μL of the acetylcholine stock solution (500-fold) and 2.5 μL of the Thiolite Green stock solution (200-fold) were added to 250 μL of Assay Buffer (Component B) to provide 253.5 μL of an AChE detection solution. The concentration of the solution is twice the concentration specified in the kit. The concentration of the solution was set to x2, and 2-fold serial dilution was performed to provide AChE detection solutions with four concentrations.

50 μL of each of the AChE detection solutions was added to 50 μL of the sample, a reaction was performed at 30°C, and AChE activity was evaluated. In this case, the concentration of the extracellular vesicles in the reaction solution is 2 μg/mL.

(Evaluation of AChE Activity with 96-well Plate)

Evaluation was performed in the same manner as in Example 1.

FIG. 21 shows temporal changes in fluorescence intensity by differences in reporter molecule concentration. As shown in FIG. 21, it is found that the fluorescence intensity varies depending on the reporter molecule concentration. A change in fluorescence intensity can be controlled by changing the reporter molecule concentration.

Example 14

(Detection of AChE Activity with SensoLyte 520 Acetylcholinesterase Activity Assay Kit (Fluorimetric))

980 μL of Assay Buffer (Component B) was added to 20 μL of the AChE standard solution in the same manner as in Example 11 to prepare a 1,000 mU/mL AChE solution. Further, 10-fold dilution was performed with Assay Buffer (Component B) to provide a 100 mU/mL AChE solution. The concentration of the AChE solution was set to x1, and 3-fold serial dilution was performed to prepare standard samples with seven concentrations. Further, a sample free of AChE was prepared similarly, and used as a sample for BG measurement.

Solutions in SensoLyte 520 Acetylcholinesterase Activity Assay Kit were used for the following AChE detection solution. For detection of AChE activity, 5 μL of an acetylcholine solution (Component C) and 5 μL of an AChE substrate solution (Component A) were added to 490 μL of Assay Buffer (Component D) to provide 500 μL of an AChE detection solution.

50 μL of the AChE detection solution was added to 50 μL of each of the samples, a reaction was performed at 30°C, and AChE activity was evaluated. In this case, the highest concentration of AChE in the reaction solutions is 50 mU/mL.

(Evaluation of AChE Activity with 96-well Plate)

Evaluation was performed in the same manner as in Example 1. ΔRFU was calculated by subtracting the measured value of the sample for BG measurement from the measured value of each of the samples.

FIG. 22 shows the AChE concentration and temporal changes in fluorescence intensity from the reporter molecule. As shown in FIG. 22, it is found that the fluorescence intensity varies depending on the AChE concentration. The concentrations of x1, x1/3, x1/9, and x1/27 at which activity can be satisfactorily detected correspond to 50 mU/mL, 16.7 mU/mL, 5.56 mU/mL, and 1.85 mU/mL, respectively. In addition, it is found that the change in fluorescence intensity from the reporter molecule of Amplite Fluorimetric Acetylcholinesterase Assay Kit is larger than that of SensoLyte 520 Acetylcholinesterase Activity Assay Kit.

Example 15

(Detection of AChE Activity of MDA-MB-231 or A549-derived Extracellular Vesicles with SensoLyte 520 Acetylcholinesterase Activity Assay Kit (Fluorimetric))

4 μL (corresponding to 4 μg for each of the MDA-MB-231 and A549-derived extracellular vesicles) of each of the MDA-MB-231-derived extracellular vesicles (50 μg of protein in 50 μL of PBS) and the A549-derived extracellular vesicles (50 μg of protein in 50 μL of PBS) described in the section "Materials and the like" were aliquoted, and Assay Buffer (Component D) included in the kit was added to the aliquot to provide 200 μL of each solution. The concentration of the solution is 20 μg/mL for each of the MDA-MB-231 and A549-derived extracellular vesicles. The concentration of 20 μg/mL was set to x1 in Example 8, and hence the concentration of the solution was set to x1, and 2-fold serial dilution was performed to prepare standard samples with seven concentrations for each of the MDA-MB-231 and A549-derived extracellular vesicles. Further, a sample free of extracellular vesicles was prepared similarly, and used as a sample for BG measurement.

For detection of AChE activity, 5 μL of the acetylcholine solution (Component C) and 5 μL of the AChE substrate solution (Component A) were added to 490 μL of Assay Buffer (Component D) to provide 500 μL of an AChE detection solution.

50 μL of the AChE detection solution was added to 50 μL of each of the samples, a reaction was performed at 30°C, and AChE activity was evaluated. In this case, the highest concentration of the extracellular vesicles in the reaction solutions is 10 μg/mL for each of the MDA-MB-231 and A549-derived extracellular vesicles.

(Evaluation of AChE Activity with 96-well Plate)

Evaluation was performed in the same manner as in Example 1. ΔRFU was calculated by subtracting the measured value of the sample for BG measurement from the measured value of each of the samples.

FIG. 23A shows the MDA-MB-231-derived extracellular vesicle concentration and temporal changes in fluorescence intensity from the reporter molecule. As shown in FIG. 23A, it is found that the fluorescence intensity varies depending on the extracellular vesicle concentration. In addition, FIG. 23B shows the A549-derived extracellular vesicle concentration and temporal changes in fluorescence intensity from the reporter molecule. As shown in FIG. 23B, it is found that the fluorescence intensity varies depending on the extracellular vesicle concentration. From comparison between the MDA-MB-231 and A549-derived extracellular vesicles, it is found that the AChE activity of the A549-derived extracellular vesicles is strong. This is the same as the results of Example 12.

Example 16

(Detection of AChE Activity of MDA-MB-231-derived Extracellular Vesicles with SensoLyte 520 Acetylcholinesterase Activity Assay Kit (Fluorimetric))

1 μL (corresponding to 1 μg) of the MDA-MB-231-derived extracellular vesicles (50 μg of protein in 50 μL of PBS) in Example 1 were aliquoted, and Assay Buffer (Component D) used in Example 14 was added to the aliquot to provide 250 μL of a solution. The concentration of the solution is 4 μg/mL. The concentration of 20 μg/mL was set to x1 in Example 8, and hence the concentration of the solution is x1/5.

In addition, for detection of AChE activity, 2 μL of the acetylcholine solution (Component C) and 2 μL of the AChE substrate solution (Component A) were added to 96 μL of Assay Buffer (Component D) to provide 100 μL of an AChE detection solution. The concentration of the solution is twice the concentration specified in the kit. Similarly, 4 μL of the acetylcholine solution (Component C) and 4 μL of the AChE substrate solution (Component A) were added to 92 μL of Assay Buffer (Component D) to provide 100 μL of an AChE detection solution (4-fold concentration), and 6 μL of the acetylcholine solution (Component C) and 6 μL of the AChE substrate solution (Component A) were added to 88 μL of Assay Buffer (Component D) to provide 100 μL of an AChE detection solution (6-fold concentration). Thus, three kinds of AChE detection solutions with three concentrations were prepared.

50 μL of each of the AChE detection solutions was added to 50 μL of the sample, a reaction was performed at 30°C, and AChE activity was evaluated. In this case, the concentrations of the extracellular vesicles in the reaction solutions are 2 μg/mL.

(Evaluation of AChE Activity with 96-well Plate)

Evaluation was performed in the same manner as in Example 1.

FIG. 24 shows temporal changes in fluorescence intensity by differences in reporter molecule concentration. As shown in FIG. 24, it is found that the fluorescence intensity varies depending on the reporter molecule concentration. A change in fluorescence intensity can be controlled by changing the reporter molecule concentration.

Example 17

(Detection of MMP14 Activity or AChE Activity of MDA-MB-231-derived Extracellular Vesicles with Custom #1, Custom #2, Amplite Fluorimetric Acetylcholinesterase Assay Kit (Green Fluorescence), or SensoLyte 520 Acetylcholinesterase Activity Assay Kit (Fluorimetric))

8 μL (corresponding to 8 μg) of the MDA-MB-231-derived extracellular vesicles (50 μg of protein in 50 μL of PBS) described in the section "Materials and the like" were aliquoted, and purified water was added to the aliquot to provide 80 μL of a solution. The concentration of the solution is 100 μg/mL. The concentration of 20 μg/mL was set to x1 in Example 8, and hence the concentration of the solution is x5.
The following three kinds of buffers were prepared for the detection of the respective activities.

·MMP 14 buffer

50 mM Tris-Cl (pH: 7.6)
10 mM $CaCl_2$
0.005% Brij35

·Amplite Fluorimetric Acetylcholinesterase Assay Kit (Green Fluorescence)

Assay Buffer (Component B)
hereinafter abbreviated as "ALG buffer"

·SensoLyte 520 Acetylcholinesterase Activity Assay Kit (Fluorimetric)

Assay Buffer (Component D)
hereinafter abbreviated as "SL520 buffer"

For detection of MMP14 activity, 1 μL of 1 mM Custom #1 was added to 100 μL of each of the MMP14 buffer, the ALG buffer, and the SL520 buffer. Thus, MMP14 detection solutions (1) each having a final concentration of 10 μM were obtained.
For detection of MMP14 activity, 1 μL of 1 mM Custom #2 was added to 100 μL of each of the MMP14 buffer, the ALG buffer, and the SL520 buffer. Thus, MMP14 detection solutions (2) each having a final concentration of 10 μM were obtained.
For detection of AChE activity (Amplite Fluorimetric Acetylcholinesterase Assay Kit (Green Fluorescence)), 1 μL of the acetylcholine stock solution (500-fold) and 2.5 μL of the Thiolite Green stock solution (200-fold) were added to 500 μL of each of the MMP 14 buffer, the ALG buffer, and the SL520 buffer. Thus, AChE detection solutions (1) were obtained.
For detection of AChE activity (SensoLyte 520 Acetylcholinesterase Activity Assay Kit (Fluorimetric)), 1 μL of the acetylcholine solution (Component C) and 1 μL of the AChE substrate solution (Component A) were added to 100 μL of each of the MMP14 buffer, the ALG buffer, and the SL520 buffer. Thus, AChE detection solutions (2) were obtained.
4 μL of the above-mentioned sample was added to 100 μL of each of the above-mentioned detection solutions, a reaction was performed at 30°C, and MMP14 activity and AChE activity were evaluated. In this case, the concentration of the extracellular vesicles in each of the reaction solutions is 4 μg/mL, and the final concentration corresponds to x2/5 in Example 8. In addition, in the standard specification of each of the detection solutions, the same volume of the sample is to be added. In this Example, the volume of the sample to be added is almost negligible, and hence the reporter molecule concentration in each of the detection solutions is about twice the standard specification.

(Evaluation of MMP14 or AChE Activity with 96-well Plate)

Evaluation was performed in the same manner as in Example 1.
FIG. 25A shows temporal changes in fluorescence intensity by differences in assay buffer in the case of using the MMP14 detection solutions (1). As shown in FIG. 25A, it is found that the fluorescence intensity varies depending on differences in buffer to be used. Although activity was recognized in all cases, the MMP14 buffer, the SL520 buffer, and the ALG buffer were ranked in descending order of change in fluorescence intensity.
FIG. 25B shows temporal changes in fluorescence intensity by differences in assay buffer in the case of using the MMP14 detection solutions (2). As shown in FIG. 25B, it is found that the fluorescence intensity varies depending on differences in buffer to be used. Although activity was recognized in all cases, the MMP14 buffer, the SL520 buffer, and the ALG buffer were ranked in descending order of change in fluorescence intensity, which was the same order as in the case of the MMP14 detection solutions (1).

FIG. 25C shows temporal changes in fluorescence intensity by differences in assay buffer in the case of using the AChE detection solutions (1). As shown in FIG. 25C, it is found that the fluorescence intensity varies depending on differences in buffer to be used. Although activity was recognized in all cases, the SL520 buffer, the MMP14 buffer, and the ALG buffer were ranked in descending order of change in fluorescence intensity.

FIG. 25D shows temporal changes in fluorescence intensity by differences in assay buffer in the case of using the AChE detection solutions (2). As shown in FIG. 25D, it is found that the fluorescence intensity varies depending on differences in buffer to be used. Although activity was recognized in all cases, the MMP14 buffer, the SL520 buffer, and the ALG buffer were ranked in descending order of change in fluorescence intensity, which was the same order as in the cases of the MMP14 detection solutions (1) and (2).

Accordingly, when MMP14 activity and AChE activity are simultaneously detected, any buffer may be used. The MMP14 buffer and the SL520 buffer in each of which activity is broadly improved through use of various reporter molecules are preferred, and the MMP14 buffer in which activity is most highly improved is more preferred.

Example 18

(Production of Wells)

The wells 204 illustrated in FIG. 5A and FIG. 5B were produced through a CYTOP application step, a photolithography step, and an etching/resist removal step.

In the CYTOP application step, a quartz substrate (synthetic quartz substrate, AQ grade, thickness: 1 mm, manufactured by AGC Inc.) was used as the lower substrate 201 and treated with a silane coupling agent (KBE-903, manufactured by Shin-Etsu Silicone), and then CYTOP (CTL-809A, manufactured by AGC Inc.) was applied.

In the photolithography step, a positive photoresist (AZ P4903, AZ Electronic Materials) was applied. Next, developing treatment with an alkali was performed by UV exposure from above through a photomask having a desired pattern. Only in portions irradiated with UV light by the developing treatment, the photoresist was dissolved to expose a hydrophobic resin layer.

In the etching/resist removal step, hydrophobic partition walls were formed by removing part of the resin layer through etching with oxygen plasma via the photoresist part of which had been dissolved.

Finally, the desired wells 204 were formed by dissolving the photoresist with an organic solvent. The wells 204 each had a diameter of 5 μm, a depth of 4 μm, and a volume of 78.5 fL, and had a pitch of 10 μm, and the number of the wells was about 1,000,000.

(Production of Well Array)

The well array 200 illustrated in FIG. 5A and FIG. 5B was produced so as to include the lower substrate 201 having the above-mentioned wells 204 formed thereon, the upper substrate 202, an injection port portion (not shown), and a discharge port portion (not shown). Polycarbonate (thickness: 1 mm) was used as the upper substrate 202, and the upper substrate 202 was opposed to the openings of the wells 204 and the top surfaces of the partition walls 203 across the space 205. The distance in the space 205 from the top surface of each of the partition walls 203 to the upper substrate 202 was 250 μm.

(Preparation of Reaction Solution for Wells)

A reaction solution for filling the wells 204 was prepared. 1 μL (corresponding to 1 μg) of the MDA-MB-231-derived extracellular vesicles (50 μg of protein in 50 μL of PBS) described in the section "Materials and the like" were aliquoted, and the following buffer (4) was added to the aliquot to provide 150 μL of a solution. The concentration of the solution is 6.7 μg/mL. The concentration of 20 μg/mL was set to x1 in Example 8, and hence the concentration of the solution is x1/3.

Buffer (4)
50 mM Tris-Cl (pH: 7.6)
10 mM $CaCl_2$
0.01% Brij35

In addition, for detection of MMP 14 activity, 1 mM Custom #2 was diluted with the buffer (4) to prepare an MMP14 detection solution containing 10 μM Custom #2.

100 μL of the MMP14 detection solution was added to 100 μL of the sample to provide a reaction solution for wells. In this case, the concentration of the extracellular vesicles in the reaction solution is 3.3 μg/mL, and the concentration of the reporter molecule in the reaction solution is 5 μM. 100 μL of 200 μL of the prepared reaction solution for wells was used in the measurement of MMP14 activity with a 96-well plate.

(Evaluation of MMP14 Activity with 96-well Plate)

Evaluation was performed in the same manner as in Example 1.

FIG. 26A shows a temporal change in fluorescence intensity from the reporter molecule by the MDA-MB-231-derived extracellular vesicles. As shown in FIG. 26A, it is found that the fluorescence intensity was increased with the reaction time, and MMP14 activity was able to be detected.

(Filling of Wells with Reaction Solution)

The reaction solution was injected from the injection port portion on the upper substrate 202, and was fed so that the reaction solution covered the wells 204. Next, the wells were filled with the reaction solution by leaving the well array 200 to stand under reduced pressure to degas the space 205. After that, the space 205 was fed with hydrophobic solvents to be sealed. Fluorinated oils AsahiKlin AE-3000 (manufactured by AGC Inc.) and Fomblin Y-25 (manufactured by Solvay S.A.) were used as the hydrophobic solvents. A fluorinated oil Simoa (trademark) SR-X Sealing Oil: 102767 (manufactured by Quanterix Corporation) was further used as another hydrophobic solvent.

(Fluorescence Microscope Observation)

The well array prepared in the foregoing was subjected to a reaction through incubation at 37°C to promote the generation of fluorescence. After that, observation was performed with a fluorescence microscope (BZ-X800 (manufactured by Keyence Corporation)).

The observation of fluorescence derived from each fluorescent substance was performed under the following conditions to acquire a fluorescence image.

Custom #2
GFP: OP-87763 (manufactured by Keyence Corporation)
Model: OP-87763
Excitation wavelength: 470/40 nm
Absorption wavelength: 525/50 nm
Dichroic wavelength: 495 nm

FIG. 26B and FIG. 26C show fluorescence microscope images of the wells. FIG. 26B is the fluorescence microscope image of the wells sealed with AsahiKlin AE-3000 (manufactured by AGC Inc.) and Fomblin Y-25 (manufactured by Solvay S.A.) serving as hydrophobic solvents after 3 hours of reaction. This image contains both of the fluorescence of 5-FAM derived from Custom #2 after release of quenching and the background fluorescence of 5-FAM derived from Custom #2 under quenching. In addition, FIG. 26C is the fluorescence microscope image of the wells sealed with Simoa SR-X Sealing Oil (manufactured by Quanterix Corporation) serving as a hydrophobic solvent after 3 hours of reaction. Similarly to the foregoing, this image contains both of the fluorescence of 5-FAM derived from Custom #2 after release of quenching and the background fluorescence of 5-FAM derived from Custom #2 under quenching.

As shown in FIG. 26B and FIG. 26C, a well in which the fluorescence of 5-FAM derived from Custom #2 after release of quenching was observed and a well in which only the background fluorescence of 5-FAM derived from Custom #2 under quenching was observed were able to be recognized. Thus, it was able to be recognized that the wells were filled with the sample and the reagent containing the reporter molecule and a cleavage reaction of Custom #2 by MMP14 in the MDA-MB-231-derived extracellular vesicles worked in a microcompartment.

Next, the fluorescence microscope images after 3 hours of reaction of Example described above were subjected to predetermined image processing to provide histograms shown in FIG. 26D and FIG. 26E. In this case, FIG. 26D is the histogram obtained by analyzing the fluorescence microscope image of the wells sealed with AsahiKlin AE-3000 (manufactured by AGC Inc.) and Fomblin Y-25 (manufactured by Solvay S.A.) serving as hydrophobic solvents, and FIG. 26E is the histogram obtained by analyzing the fluorescence microscope image of the wells sealed with Simoa SR-X Sealing Oil. The histograms shown in FIG. 26D and FIG. 26E each show the number of wells each showing a fluorescence intensity included in each fraction of fluorescence intensity obtained by dividing fluorescence intensities into intervals of a constant value.

As shown in FIG. 26D and FIG. 26E, each of the histograms was divided into three regions A, B, and C according to the fluorescence intensity. Of those, the region A having the smallest fluorescence intensity is attributed to the background fluorescence of the reporter molecule. In addition, wells falling within the category of the region B were identified as positive wells B, and wells falling within the category of the region C were identified as positive wells C. When the fluorescence intensities of the positive wells B and the positive wells C are compared to each other, the fluorescence intensity of the positive wells C is stronger, and hence it is presumed that most of the positive wells B are filled with one extracellular vesicle per well, and most of the positive wells C are filled with a plurality of extracellular vesicles per well. In the present invention, it is conceived that activity from "a plurality of" MMP14 molecules in "one" extracellular vesicle is detected, and it is presumed that well-defined integer multiple peaks are not observed owing to the fluctuations of, for example, the sizes of extracellular vesicles and the molecule density of MMP14 in each of the extracellular vesicles, in addition to the fluctuation of reaction efficiency.

Further, when the concentration of the extracellular vesicles having MMP14 activity was calculated through use of the above-mentioned equation (1) from the total number of wells and the number of positive wells identified from each of the histograms, the concentration was able to be estimated as described below.

[Table 2]

Table 2

| Fluorescence microscope image | Oil | Number of wells | Number of positives | Positive ratio | Expected value λ |
|---|---|---|---|---|---|
| FIG. 26D | AE-3000 Y25 | 14,756 | 3,094 | 0.21 | 0.235 |
| FIG. 26E | Simoa | 14,767 | 2,529 | 0.171 | 0.188 |

In a system using AE-3000 and Y25 as oils, λ is 0.235, the volume of the microcompartment is 78.5 fL, and hence the extracellular vesicle concentration can be calculated to be $3.0\times10^9$/mL.

The reaction solution concentration is 3.3 μg of protein/mL, and hence the extracellular vesicle concentration is $9.1\times10^8$/μg of protein. Accordingly, it is estimated that 1 μL of the MDA-MB-231-derived extracellular vesicles (50 μg of protein in 50 μL of PBS) contain $9.1\times10^8$ extracellular vesicles in which MMP14 activity can be detected with Custom #2.

Similarly, in a system using Simoa as an oil, λ is 0.188, the volume of the microcompartment is 78.5 fL, and hence the extracellular vesicle concentration can be calculated to be $2.4\times10^9$/mL. The reaction solution concentration is 3.3 μg of protein/mL, and hence the extracellular vesicle concentration is $7.3\times10^8$/μg of protein. Accordingly, it is estimated that 1 μL of the MDA-MB-231-derived extracellular vesicles (50 μg of protein in 50 μL of PBS) contain $7.3\times10^8$ extracellular vesicles in which MMP14 activity can be detected with Custom #2.

Consequently, it was shown that digital detection of the extracellular vesicles was able to be performed through use of MMP14 activity.

Example 19

(Wells and Well Array)

Simoa Discs (16 discs): 100001 (manufactured by Quanterix Corporation) was used as a well array. The specification is as described below.

Well diameter: 4.25 μm
Well depth: 3.25 μm
Well capacity: 50 fL
Number of wells in array: 239,000
Array range: 3 mm×4 mm

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) used in Example 18 was added to the aliquot to provide 200 μL of a solution. The concentration of the solution is 20 μg/mL. The concentration of 20 μg/mL was set to x1 in Example 8, and hence the concentration of the solution is also x1. The concentration of the solution was set to x1, and 10-fold serial dilution was performed to prepare standard samples with five concentrations. Further, a sample free of extracellular vesicles was prepared similarly, and used as a sample for BG measurement.

In addition, for detection of MMP14 activity, 1 mM Custom #2 and 1 mM Alexa Fluor 647 (manufactured by Molecular Probes) serving as a standard fluorescent substance were diluted with the buffer (4) to prepare a MMP14 detection solution containing 10 μM Custom #2 and 1 μM Alexa Fluor 647.

100 μL of the MMP14 detection solution was added to 100 μL of each of the samples to provide a reaction solution for wells. In this case, the highest concentration of the extracellular vesicles in the reaction solutions is 10 μg/mL, and the concentrations of the reporter molecule and the standard fluorescent substance in each of the reaction solutions are 5 μM and 0.5 μM, respectively. 100 μL of 200 μL of each of the prepared reaction solutions for wells was used in the measurement of MMP14 activity with a 96-well plate.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 27 shows temporal changes in the fluorescence intensity from Custom #2 by the MDA-MB-231-derived extracellular vesicles. As shown in FIG. 27, it is found that the fluorescence intensity was increased with the reaction time, and MMP14 activity was able to be detected at concentrations of x1 and x1/10, but the MMP14 activity was not able to be detected at concentrations of x1/100 or less.

(Filling of Wells with Reaction Solution)

The reaction solution was injected from the injection port portion on the upper substrate 202, and was fed so that the reaction solution covered the wells 204. Next, the wells were filled with the reaction solution by leaving the well array 200 to stand under reduced pressure to degas the space 205. After that, the space 205 was fed with hydrophobic solvents to be sealed. A fluorinated oil SR-X Sealing Oil was used as a hydrophobic solvent.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the following conditions to acquire a fluorescence image.

Reporter molecule (Custom #2)
GFP: OP-87763 (manufactured by Keyence Corporation)
Model: OP-87763
Excitation wavelength: 470/40 nm
Absorption wavelength: 525/50 nm
Dichroic wavelength: 495 nm

In this Example, Custom #2 was used as a reporter molecule. In subsequent Examples, when the same conditions as those in Example 19 are described, measurement was also performed on reporter molecules, such as Custom #1, SenosoLyte, and Amplite, under the same conditions as described above unless otherwise indicated.

Alexa Fluor 647
Cy5: OP-87766 (manufactured by Keyence Corporation)
Model: OP-87766
Excitation wavelength: 620/60 nm
Absorption wavelength: 700/75 nm
Dichroic wavelength: 660 nm

FIG. 28A to FIG. 28F and FIG. 28G to FIG. 28L show fluorescence microscope images of the wells. FIG. 28A to FIG. 28F each show the fluorescence microscope image containing both of the fluorescence of 5-FAM derived from Custom #2 and the background fluorescence of Custom #2 after 3 hours of reaction. In addition, FIG. 28G to FIG. 28L each show the fluorescence microscope image containing the fluorescence of the standard fluorescent substance after 3 hours of reaction.

Similarly, FIG. 29A to FIG. 29F and FIG. 29G to FIG. 29L show fluorescence microscope images of the wells. FIG.

29A to FIG. 29F each show the fluorescence microscope image containing both of the fluorescence of 5-FAM derived from Custom #2 and the background fluorescence of Custom #2 after 7 hours of reaction. In addition, FIG. 29G to FIG. 29L each show the fluorescence microscope image containing the fluorescence of the standard fluorescent substance after 7 hours of reaction.

As shown in FIG. 28G to FIG. 28L and FIG. 29G to FIG. 29L, the wells were able to be recognized based on the fluorescence of the standard fluorescent substance. In addition, as shown in FIG. 28A to FIG. 28E and FIG. 29A to FIG. 29E, it is found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #2 by MMP14 in the MDA-MB-231-derived extracellular vesicles worked in a microcompartment.

Next, the fluorescence microscope images after 3 hours of reaction of Example described above were subjected to predetermined image processing to provide histograms shown in FIG. 30A to FIG. 30E. Similarly, histograms shown in FIG. 31A to FIG. 31E after 7 hours of reaction were obtained. Those histograms each show the number of wells each showing a fluorescence intensity included in each fraction of fluorescence intensity obtained by dividing fluorescence intensities into intervals of a constant value.

The expected values ($\lambda$) calculated with respect to the concentration of the extracellular vesicles subjected to the test through use of the above-mentioned equation (1) from the total number of wells and the number of positive wells identified from each of the histograms after 3 hours and 7 hours of reaction are shown. In this case, in a sample free of extracellular vesicles, no positive well was observed at any of the reaction times. FIG. 32 shows plots in a low positive ratio region where separation between positivity and negativity is more satisfactory. A power approximation can be performed satisfactorily. The extracellular vesicle concentration was able to be accurately quantified. In this case, under the assumption that there are no artifacts in the extracellular vesicle concentration, such as the aggregation of extracellular vesicles in a higher concentration region, it is presumed that an approximation with an exponent closer to 1 after a reaction time of 7 hours is more accurate.

As shown in FIG. 27 described in this Example, only MMP14 activity at an extracellular vesicle concentration of x1/10 (corresponding to 1 $\mu$g/mL) or more was able to be detected with a 96-well plate, but MMP14 activity at an extracellular vesicle concentration of x1/10,000 (corresponding to 1 ng/mL) or more was able to be detected in the microcompartments. Accordingly, it was found that the use of the microcompartment improved detection sensitivity by about three orders of magnitude.

[Table 3]

Table 3

| Fluorescence microscope image | Reaction time | Concentration [a.u.] | Number of wells | Number of positives | Positive ratio | Expected value $\lambda$ |
|---|---|---|---|---|---|---|
| FIG. 30A | 3 h | 1 | 28,414 | 14,539 | 0.512 | 0.717 |
| FIG. 30B | 3 h | 0.1 | 29,031 | 2,305 | 0.0794 | 0.0827 |
| FIG. 30C | 3 h | 0.01 | 28,971 | 264 | 0.00911 | 0.00915 |
| FIG. 30D | 3 h | 0.001 | 29,089 | 32 | 0.0011 | 0.0011 |
| FIG. 30E | 3 h | 0.0001 | 28,896 | 4 | 0.000138 | 0.000138 |
| FIG. 31A | 7 h | 1 | 28,916 | 26,093 | 0.902 | 2.33 |
| FIG. 31B | 7 h | 0.1 | 28,971 | 2,571 | 0.0887 | 0.0929 |
| FIG. 31C | 7 h | 0.01 | 28,709 | 263 | 0.00916 | 0.0092 |
| FIG. 31D | 7 h | 0.001 | 28,984 | 24 | 0.000828 | 0.000828 |
| FIG. 31E | 7 h | 0.0001 | 28,940 | 3 | 0.000104 | 0.000104 |

From the approximation after a reaction time of 3 hours, $\lambda$ at x1 is 0.6727, the volume of the microcompartment is 50 fL, and hence the extracellular vesicle concentration can be calculated to be $1.3\times10^{10}$/mL. The reaction solution concentration is 10 $\mu$g of protein/mL, and hence the extracellular vesicle concentration is $1.3\times10^{9}/\mu$g of protein. Accordingly, it is estimated that 1 $\mu$L of the MDA-MB-231-derived extracellular vesicles (50 $\mu$g of protein in 50 $\mu$L of

PBS) contain $1.3\times10^9$ extracellular vesicles in which MMP14 activity can be detected with Custom #2.

Similarly, from the approximation after a reaction time of 7 hours, λ at x1 is 0.8756, and the extracellular vesicle concentration can be calculated to be $1.8\times10^{10}$/mL. Accordingly, it is estimated that 1 μL of the MDA-MB-231-derived extracellular vesicles (50 μg of protein in 50 μL of PBS) contain $1.8\times10^9$ extracellular vesicles in which MMP14 activity can be detected with Custom #2.

Consequently, it was shown that digital detection of the extracellular vesicles was able to be performed through use of MMP 14 activity even in a different well array.

Example 20

(Wells and Well Array)

A well array of Simoa Discs (manufactured by Quanterix Corporation) was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 200 μL of a solution. The concentration of the solution is 20 μg/mL. The concentration of 20 μg/mL was set to x1 in Example 8, and hence the concentration of the solution is also x1. The concentration of the solution was set to x1, and 10-fold serial dilution was performed to prepare standard samples with five concentrations. Further, a sample free of extracellular vesicles was prepared similarly, and used as a sample for BG measurement.

In addition, for detection of MMP14 activity, 1 mM Custom #1 and 1 mM Alexa Fluor 647 serving as a standard fluorescent substance were diluted with the buffer (4) to prepare a MMP14 detection solution containing 10 μM Custom #1 and 5 μM Alexa Fluor 647.

100 μL of the MMP14 detection solution was added to 100 μL of each of the samples to provide a reaction solution for wells. In this case, the highest concentration of the extracellular vesicles in each of the reaction solutions is 10 μg/mL, and the concentrations of the reporter molecule and the standard fluorescent substance in each of the reaction solutions are 5 μM and 2.5 μM, respectively. 100 μL of 200 μL of each of the prepared reaction solutions for wells was used in the measurement of MMP14 activity with a 96-well plate.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 33 shows temporal changes in fluorescence intensity from Custom #1 by the MDA-MB-231-derived extracellular vesicles. As shown in FIG. 33, it is found that the fluorescence intensity was increased with the reaction time, and MMP14 activity was able to be detected at concentrations of x1 and x1/10, but the MMP14 activity was not able to be detected at concentrations of x1/100 or less.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

FIG. 34A to FIG. 34F and FIG. 34G to FIG. 34L show fluorescence microscope images of the wells. FIG. 34A to FIG. 34F show fluorescence microscope images each containing both of the fluorescence of 5-FAM derived from Custom #1 and the background fluorescence of Custom #1 after 5 hours of reaction. In addition, FIG. 34G to FIG. 34L show fluorescence microscope images each containing the fluorescence of the standard fluorescent substance after 5 hours of reaction.

As shown in FIG. 34G to FIG. 34L, the wells were able to be recognized based on the fluorescence of the standard fluorescent substance. In addition, as shown in FIG. 34A to FIG. 34E, it is found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the MDA-MB-231-derived extracellular vesicles worked in a microcompartment. In this case, the fluorescence of 5-FAM was also observed in FIG. 34F that was free of extracellular vesicles, and hence it is found that a pseudopositive result occurred, through its frequency is extremely low.

Next, the fluorescence microscope images after 5 hours of reaction of Example described above were subjected to predetermined image processing to provide histograms shown in FIG. 35A to FIG. 35F. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 5 hours of reaction is shown. In this case, the number of pseudopositive wells nearly equal to that at x1/10,000 was also observed in the sample free of extracellular vesicles, and hence it is found that detection at x1/10,000 is difficult. The expected value (λ) obtained by subtracting the number of positive wells when the extracellular vesicle concentration is zero is collectively shown. In addition, FIG. 36 shows plots of the expected value λ with respect to the extracellular vesicle concentration. A power approximation can be performed satisfactorily. The extracellular vesicle concentration was able to be accurately quantified. In this case, under the assumption that there are no artifacts in the extracellular vesicle concentration, such as the aggregation of extracellular vesicles in a higher concentration region, it is presumed that an approximation with an exponent closer to 1 of the expected value (λ) that is obtained by subtracting the number of positive wells when the extracellular vesicle concentration is zero is more accurate.

As shown in FIG. 33 described in this Example, only MMP14 activity at an extracellular vesicle concentration of x1/10 (corresponding to 1 μg/mL) or more was able to be detected with a 96-well plate, but MMP14 activity at an extracellular vesicle concentration of at least x1/1,000 (corresponding to 10 ng/mL) was able to be detected in the microcompartments. Accordingly, it was found that the use of the microcompartment improved detection sensitivity by at least two orders of magnitude. In addition, it is found that the detection sensitivity in a low concentration region of Custom #2 in Example 19 is higher than that of Custom #1 by about one order of magnitude.

[Table 4]

Table 4

| Fluorescence microscope image | ΔBG | Concentration [a.u.] | Number of wells | Number of positives | Positive ratio | Expected value λ |
|---|---|---|---|---|---|---|
| FIG. 34A | Absent | 1 | 57,742 | 39,601 | 0.686 | 1.16 |
| FIG. 34B | Absent | 0.1 | 57,791 | 7,404 | 0.128 | 0.137 |
| FIG. 34C | Absent | 0.01 | 57,938 | 661 | 0.0114 | 0.0115 |
| FIG. 34D | Absent | 0.001 | 57,949 | 165 | 0.00285 | 0.00285 |
| FIG. 34E | Absent | 0.0001 | 58,005 | 31 | 0.000534 | 0.000535 |
| FIG. 34F | BG | 0 | 57,865 | 32 | 0.000553 | 0.000553 |
| FIG. 34A | Present | 1 | 57,742 | 39,569 | 0.685 | 1.16 |
| FIG. 34B | Present | 0.1 | 57,791 | 7,372 | 0.128 | 0.136 |
| FIG. 34C | Present | 0.01 | 57,938 | 629 | 0.0109 | 0.0109 |
| FIG. 34D | Present | 0.001 | 57,949 | 133 | 0.0023 | 0.0023 |
| FIG. 34E | Present | 0.0001 | 58,005 | -1 | - | - |

In an approximation without BG correction, λ at x1 is 1.03, the volume of the microcompartment is 50 fL, and hence the extracellular vesicle concentration can be calculated to be $2.1 \times 10^{10}$/mL. The reaction solution concentration is 10 μg of protein/mL, and hence the extracellular vesicle concentration is $2.1 \times 10^{9}$/μg of protein.

Accordingly, it is estimated that 1 μL of the MDA-MB-231-derived extracellular vesicles (50 μg of protein in 50 μL of PBS) contain $2.1 \times 10^{9}$ extracellular vesicles in which MMP14 activity can be detected with Custom #1.

Similarly, in an approximation with BG correction, $\lambda$ at x1 is 1.07, and the extracellular vesicle concentration can be calculated to be $2.1\times10^{10}$/mL like the approximation without correction. Accordingly, it is estimated that 1 μL of the MDA-MB-231-derived extracellular vesicles (50 μg of protein in 50 μL of PBS) contain $2.1\times10^{9}$ extracellular vesicles in which MMP14 activity can be detected with Custom #1.

Consequently, it was shown that digital detection of the extracellular vesicles was able to be performed through use of MMP14 activity even in different reporter molecules.

Example 21

(Wells and Well Array)

A well array of Simoa Discs was used.

(Preparation of Reaction Solution for Wells)

A reaction solution for filling the wells 204 was prepared. 4 μL (corresponding to 4 μg) of the A549-derived extracellular vesicles (50 μg of protein in 50 μL of PBS) described in the section "Materials and the like" were aliquoted, and the buffer (4) was added to the aliquot to provide 200 μL of a solution. The concentration of the solution is 20 μg/mL. The concentration of 20 μg/mL was set to x1 in Example 8, and hence the concentration of the solution is also x1. The concentration of the solution was set to x1, and 10-fold serial dilution was performed to prepare standard samples with five concentrations. Further, a sample free of extracellular vesicles was prepared similarly, and used as a sample for BG measurement.

In addition, for detection of MMP14 activity, 1 mM Custom #1 and 1 mM Alexa Fluor 647 serving as a standard fluorescent substance were diluted with the buffer (4) to prepare a MMP14 detection solution containing 10 μM Custom #1 and 5 μM Alexa Fluor 647.

100 μL of the MMP14 detection solution was added to 100 μL of each of the samples to provide a reaction solution for wells. In this case, the highest concentration of the extracellular vesicles in each of the reaction solutions is 10 μg/mL, and the concentrations of the reporter molecule and the standard fluorescent substance in each of the reaction solutions are 5 μM and 2.5 μM, respectively. 100 μL of 200 μL of each of the prepared reaction solutions for wells was used in the measurement of MMP14 activity with a 96-well plate.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 37 shows temporal changes in fluorescence intensity from Custom #1 by the A549-derived extracellular vesicles. As shown in FIG. 37, it is found that the fluorescence intensity was increased with the reaction time, and MMP14 activity was able to be detected at a concentration of x1, but the MMP14 activity was not able to be detected at concentrations of x1/10 or less.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from Custom #1 and the background fluorescence of Custom #1 after 5 hours of reaction, and a fluorescence microscope image containing the fluorescence of the standard fluorescent substance after 5 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the A549-derived extracellular vesicles worked in a microcompartment. In this case, the fluorescence of 5-FAM was also observed in the fluorescence microscope image of the wells free of extracellular vesicles, and hence it is found that a pseudopositive result occurred, though its frequency is extremely low.

Next, the fluorescence microscope images after 5 hours of reaction of Example described above were subjected to predetermined image processing to provide histograms shown in FIG. 38A to FIG. 38F. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 5 hours of reaction is shown. In this case, substantially the same number of pseudopositive wells as those at x1/10,000 and x1/1,000 were observed even in the sample free of extracellular vesicles, and hence it is found that detection at x1/10,000 and x1/1,000 is difficult. An expected value (λ) obtained by subtracting the number of positive wells when the extracellular vesicle concentration is zero is collectively shown. In addition, FIG. 39 shows plots of the expected value λ with respect to the extracellular vesicle concentration. A power approximation can be performed satisfactorily. The extracellular vesicle concentration was able to be accurately quantified. In this case, under the assumption that there are no artifacts in the extracellular vesicle concentration, such as the aggregation of extracellular vesicles in a higher concentration region, it is presumed that an approximation with an exponent closer to 1 of the expected value (λ) obtained by subtracting the number of positive wells when the extracellular vesicle concentration is zero is more accurate.

As shown in FIG. 37 described in this Example, only MMP14 activity at an extracellular vesicle concentration of x1 (corresponding to 10 μg/mL) or more was able to be detected with a 96-well plate, but MMP14 activity at an extracellular vesicle concentration of at least x1/100 (corresponding to 100 ng/mL) was able to be detected in the microcompartment. Accordingly, it was found that the use of the microcompartment improved detection sensitivity by at least two orders of magnitude. In addition, it is found that the detection sensitivity in a low concentration region of Custom #2 in Example 19 is higher than that of Custom #1 by approximately one order of magnitude.

[Table 5]

Table 5

| Fluorescence microscope image | ΔBG | Concentration [a.u.] | Number of wells | Number of positives | Positive ratio | Expected value λ |
|---|---|---|---|---|---|---|
| Not displayed | Absent | 1 | 57,981 | 8,036 | 0.139 | 0.149 |
| Not displayed | Absent | 0.1 | 58,003 | 789 | 0.0136 | 0.0137 |
| Not displayed | Absent | 0.01 | 57,685 | 119 | 0.00206 | 0.00207 |
| Not displayed | Absent | 0.001 | 57,901 | 57 | 0.000984 | 0.000985 |
| Not displayed | Absent | 0.0001 | 57,990 | 42 | 0.000724 | 0.000725 |
| Not displayed | BG | 0 | 57,920 | 38 | 0.000656 | 0.000656 |
| Not displayed | Present | 1 | 57,981 | 7,998 | 0.138 | 0.148 |
| Not displayed | Present | 0.1 | 58,003 | 751 | 0.0129 | 0.013 |
| Not displayed | Present | 0.01 | 57,685 | 81 | 0.0014 | 0.00141 |
| Not displayed | Present | 0.001 | 57,901 | 19 | 0.000328 | 0.000328 |
| Not displayed | Present | 0.0001 | 57,990 | 4 | 0.000069 | 0.000069 |

In an approximation without BG correction, λ at x1 is 0.102, the volume of the microcompartment is 50 fL, and hence the extracellular vesicle concentration can be calculated to be $2.0\times10^9$/mL. The reaction solution concentration is 10 μg of protein/mL, and hence the extracellular vesicle concentration is $2.0\times10^8$/μg of protein. Accordingly, it is estimated that 1 μL of the A549-derived extracellular vesicles (50 μg of protein in 50 μL of PBS) contain $2.0\times10^8$ extracellular vesicles in which MMP14 activity can be detected with Custom #1.

Similarly, in an approximation with BG correction, λ at x1 is 0.120, and the extracellular vesicle concentration can be calculated to be $2.4\times10^9$/mL like the approximation without correction. Accordingly, it is estimated that 1 μL of the A549-derived extracellular vesicles (50 μg of protein in 50 μL of PBS) contain $2.4\times10^8$ extracellular vesicles in which MMP14 activity can be detected with Custom #1.

Consequently, it was shown that digital detection of the extracellular vesicles was able to be performed through use of MMP14 activity even in the cell-derived extracellular vesicles.

Example 22

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (2) was added to the aliquot to provide 200 μL of a solution. The concentration of the solution is 20 μg/mL. The concentration of 20 μg/mL was set to x1 in Example 8, and hence the concentration of the solution is also x1. The concentration of the solution was set to x1, and 3-fold serial dilution was performed to prepare standard samples with three concentrations. Further, a sample free of extracellular vesicles was prepared similarly, and used as a sample for BG measurement.

In addition, for detection of MMP14 activity, 1 mM Custom #1 or Custom #2 and 1 mM Alexa Fluor 647 serving as a standard fluorescent substance were diluted with the buffer (2) to prepare a MMP14 detection solution containing 10 μM Custom #1 or Custom #2 and 5 μM Alexa Fluor 647.

100 μL of the MMP14 detection solution was added to 100 μL of each of the samples to provide a reaction solution for wells. In this case, the highest concentration of the extracellular vesicles in each of the reaction solutions is 10 μg/mL, and the concentrations of the reporter molecule and the standard fluorescent substance in each of the reaction solutions are 5 μM and 2.5 μM, respectively. 100 μL of 200 μL of each of the prepared reaction solutions for wells was used in the measurement of MMP14 activity with a 96-well plate.

(Evaluation of MMP14 Activity with 96-well Plate)

Evaluation was performed in the same manner as in Example 1. ΔRFU was calculated by subtracting the measured value of the sample for BG measurement from the measured value of each of the samples.

The reaction conditions in this Example are exactly the same as those in Example 8, and MMP14 activity was able to be recognized in exactly the same manner as in Example 8.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, fluorescence microscope images each containing both of the fluorescence of 5-FAM derived from Custom #1 and the background fluorescence of Custom #1 after 1 hour of reaction and after 2 hours of reaction were acquired. In addition, fluorescence microscope images each containing the fluorescence of the standard fluorescent substance after 1 hour of reaction and after 2 hours of reaction were acquired.

Although the illustration is omitted, fluorescence microscope images each containing both of the fluorescence of 5-FAM derived from Custom #2 and the background fluorescence of Custom #2 after 1 hour of reaction and after 2 hours of reaction were acquired similarly. In addition, fluorescence microscope images each containing the fluorescence of the standard fluorescent substance after 1 hour of reaction and after 2 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #2 by MMP14 in the MDA-MB-231-derived extracellular vesicles worked in the microcompartments. In this case, the fluorescence of 5-FAM was also observed in the fluorescence microscope image of the wells free of extracellular vesicles, and hence it is found that a pseudopositive result occurred, though its frequency is extremely low, in a system of Custom #1. Meanwhile, pseudopositive wells were not observed in a system of Custom #2. It is found that Custom #2 is more suitable when the extracellular vesicles at a lower concentration are detected.

Next, the above-mentioned fluorescence microscope images were subjected to predetermined image processing to provide histograms shown in FIG. 40A to FIG. 40D in 1 hour of reaction with Custom #1, FIG. 40E to FIG. 40H in 2 hours of reaction with Custom #1, FIG. 41A to FIG. 41D in 1 hour of reaction with of Custom #2, and FIG. 41E to FIG. 41H in 2 hours of reaction with Custom #2. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 1 hour of reaction and 2 hours of reaction with each of the reporter molecules are shown. In addition, FIG. 42A shows plots of the expected value λ with respect to the extracellular vesicle concentration for Custom #1, and FIG. 42B shows plots of the expected value λ with respect to the extracellular vesicle concentration for Custom #2. A power approximation can be performed satisfactorily. The extracellular vesicle concentration was able to be accurately quantified. The results using the 96-well plate were reflected, and almost the same detection results were achieved after 1 hour and after 2 hours through use of Custom #1 with stronger activity. Meanwhile, a larger number of positive wells were able to be detected with Custom #2 after 2 hours. When rapid results are desired, Custom #1 is preferred. As described above, a pseudopositive result is observed, though its frequency is extremely low, in Custom #1, and hence, when accurate detection in a lower concentration region is desired, Custom #2 is preferred, and it is advisable to use a reporter molecule in consideration of its characteristics.

The expected values λ at x1 after 1 hour with regard to Custom #1 and Custom #2 are 0.661 and 0.5098, respectively, and there is a slight difference therebetween. However, the expected values λ at x1 after 2 hours with regard to Custom #1 and Custom #2 are 0.7145 and 0.7649, respectively, which are almost the same.

System of Custom #1

[Table 6]

Table 6

| Fluorescence microscope image | Reaction time | Concentration [a.u.] | Number of wells | Number of positives | Positive ratio | Expected value λ |
|---|---|---|---|---|---|---|
| Not displayed | 1 h | 1 | 28,977 | 13,855 | 0.478 | 0.65 |
| Not displayed | 1 h | 0.333 | 28,980 | 6,117 | 0.211 | 0.237 |
| Not displayed | 1 h | 0.111 | 28,986 | 2,186 | 0.0754 | 0.0784 |
| Not displayed | 1 h | 0 | 28,983 | 21 | 0.000725 | 0.000725 |
| Not displayed | 2 h | 1 | 28,972 | 14,860 | 0.513 | 0.719 |
| Not displayed | 2 h | 0.333 | 28,971 | 6,143 | 0.212 | 0.238 |
| Not displayed | 2 h | 0.111 | 28,974 | 2,287 | 0.0789 | 0.0822 |
| Not displayed | 2 h | 0 | 28,983 | 23 | 0.000794 | 0.000794 |

System of Custom #2

[Table 7]

Table 7

| Fluorescence microscope image | Reaction time | Concentration [a.u.] | Number of wells | Number of positives | Positive ratio | Expected value λ |
|---|---|---|---|---|---|---|
| Not displayed | 1 h | 1 | 28,988 | 11,360 | 0.392 | 0.497 |
| Not displayed | 1 h | 0.333 | 28,983 | 5,094 | 0.176 | 0.193 |
| Not displayed | 1 h | 0.111 | 28,994 | 1,819 | 0.0627 | 0.0648 |
| Not displayed | 1 h | 0 | 28,907 | 0 | 0 | 0 |
| Not displayed | 2h | 1 | 28,967 | 15,608 | 0.539 | 0.774 |
| Not displayed | 2 h | 0.333 | 28,975 | 6,788 | 0.234 | 0.267 |
| Not displayed | 2h | 0.111 | 28,979 | 2,727 | 0.0941 | 0.0988 |
| Not displayed | 2h | 0 | 28,984 | 0 | 0 | 0 |

Example 23

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and Assay buffer (Component D) included in SensoLyte 520 MMP-14 Assay Kit (Fluorimetric) was added to the aliquot to provide 200 μL of a solution. The concentration of the solution is 20 μg/mL. The concentration of 20 μg/mL was set to x1 in Example 8, and hence the concentration of the solution is also x1. The concentration of the solution was set to x1, and 2-fold serial dilution was performed to prepare standard samples with four concentrations. Further, a sample free of MMP14 was prepared similarly, and used as a sample for BG measurement.

In addition, for detection of MMP14 activity, 4.95 mL of Assay buffer (Component D) was added to 50 μL of the MMP-14 substrate (Component A) included in the kit to provide 5 mL of a MMP14 detection solution. 500 μL thereof was aliquoted, and 2.5 μL of 1 mM Alexa Fluor 647 serving as a standard fluorescent substance was added to the aliquot to prepare a MMP14 detection solution further containing 5 μM Alexa Fluor 647.

100 μL of the MMP14 detection solution was added to 100 μL of each of the samples to provide a reaction solution for wells. In this case, the highest concentration of the extracellular vesicles in each of the reaction solutions is 10 μg/mL, and the concentration of the standard fluorescent substance in each of the reaction solutions is 2.5 μM. 100 μL of 200 μL of each of the prepared reaction solutions for wells was used in the measurement of MMP14 activity with a 96-well plate.

(Evaluation of MMP14 Activity with 96-well Plate)

Evaluation was performed in the same manner as in Example 1. ΔRFU was calculated by subtracting the measured value of the sample for BG measurement from the measured value of each of the samples.

FIG. 43 shows temporal changes in fluorescence intensity from the reporter molecule by the MDA-MB-231-derived extracellular vesicles. As shown in FIG. 43, it is found that the fluorescence intensity was increased with the reaction time, and MMP14 activity was able to be detected.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from the SensoLyte reporter molecule and the background fluorescence of the SensoLyte reporter molecule after 1 hour of reaction was acquired. In addition, a fluorescence microscope image containing the fluorescence of the standard fluorescent substance after 1 hour of reaction was acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of the SensoLyte reporter molecule by MMP14 in the MDA-MB-231-derived extracellular vesicles worked in the microcompartments. In this case, in a fluorescence microscope image of wells free of extracellular vesicles, the fluorescence of 5-FAM was not observed.

Next, the above-mentioned fluorescence microscope images were subjected to predetermined image processing to provide histograms shown in FIG. 44A to FIG. 44E in 1 hour of reaction with the SensoLyte reporter molecule. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 1 hour of reaction is shown. In addition, FIG. 45 shows plots of the expected value λ with respect to the extracellular vesicle concentration. A power approximation can be performed satisfactorily. The extracellular vesicle concentration was able to be accurately quantified.

The expected value λ at x1 with regard to SensoLyte by the plot approximation is 0.469, and the expected values λ with regard to Custom #1 and Custom #2 in the same reaction time in Example 22 are 0.661 and 0.5098, respectively, which are slightly low values. It is presumed that this is affected by the MMP14 detection sensitivity of the SensoLyte reporter molecule that is slightly lower than those of Custom #1 and Custom #2 as shown in FIG. 12 of Example 4.

Accordingly, it was shown that digital detection of the extracellular vesicles was able to be performed through use of MMP14 activity even in a further different reporter molecule.

System of SensoLyte Reporter Molecule

[Table 8]

Table 8

| Fluorescence microscope image | Reaction time | Concentration [a.u.] | Number of wells | Number of positives | Positive ratio | Expected value λ |
|---|---|---|---|---|---|---|
| Not displayed | 1 h | 1 | 29,005 | 10,134 | 0.349 | 0.43 |
| Not displayed | 1 h | 0.5 | 28,987 | 7,295 | 0.252 | 0.29 |
| Not displayed | 1 h | 0.25 | 28,923 | 3,462 | 0.12 | 0.127 |
| Not displayed | 1 h | 0.125 | 28,991 | 1,929 | 0.0665 | 0.0689 |
| Not displayed | 1 h | 0 | 29,007 | 0 | 0 | 0 |

Example 24

(Wells and Well Array)

A well array of Simoa Discs was used.

(Preparation of Reaction Solution for Wells)

A reaction solution for filling the wells 204 was prepared. 2 μL (corresponding to 2 μg) of the MDA-MB-231-derived extracellular vesicles (50 μg of protein in 50 μL of PBS) described in the section "Materials and the like" were aliquoted, and the buffer (4) used in Example 18 was added to the aliquot to provide 200 μL of a solution. The concentration of the solution is 10 μg/mL. The concentration of 20 μg/mL was set to x1 in Example 8, and hence the concentration of the solution is x1/2. The solution was subjected to 10-fold serial dilution to prepare standard samples with three concentrations. Further, a sample free of extracellular vesicles was prepared similarly, and used as a sample for BG measurement.

In addition, for detection of AChE activity, 1 μL of the acetylcholine stock solution (500-fold) and 2.5 μL of the Thiolite Green stock solution (200-fold) included in Amplite Fluorimetric Acetylcholinesterase Assay Kit (Green Fluorescence), and 5 μL of 1 mM Alexa Fluor 647 serving as a standard fluorescent substance were added to 1,000 μL of the buffer (4) to provide 1,008.5 μL of an AChE detection solution. The concentration of the solution is x1/2 of the concentration specified in the kit.

100 μL of the AChE detection solution was added to 100 μL of each of the samples to provide a reaction solution for wells. In this case, the highest concentration of the extracellular vesicles in each of the reaction solutions is 5 μg/mL, and the concentration of the reporter molecule in each of the reaction solutions is x1/2 of the concentration specified in the kit, and the concentration of the standard fluorescent substance in each of the reaction solutions is 2.5 μM. 100 μL of 200 μL of each of the prepared reaction solutions for wells was used in the measurement of AChE activity with a 96-well plate.

(Evaluation of AChE Activity with 96-well Plate)

Evaluation was performed in the same manner as in Example 1 except that the reaction temperature was set to 30°C. ΔRFU was calculated by subtracting the measured value of the sample for BG measurement from the measured value of each of the samples.

FIG. 46 shows temporal changes in fluorescence intensity from the reporter molecule by the MDA-MB-231-derived extracellular vesicles. As shown in FIG. 46, it is found that the fluorescence intensity was increased with the reaction time, and AChE activity was able to be detected. In this Example, the expression "x1" represents half of the concentration represented by the expression "x1" in Example 8.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

FIG. 47A to FIG. 47F show fluorescence microscope images of the wells. FIG. 47A to FIG. 47C show fluorescence microscope images each containing both of the fluorescence derived from the Amplite reporter molecule and the background fluorescence of the Amplite reporter molecule after 3 hours of reaction. In addition, FIG. 47D to FIG. 47F show fluorescence microscope images each containing the fluorescence of the standard fluorescent substance after 3 hours of reaction.

As shown in FIG. 47D to FIG. 47F, the wells were able to be recognized based on the fluorescence of the standard fluorescent substance. In addition, as shown in FIG. 47A to FIG. 47C, it is found that, in the wells in which the fluorescence of the Amplite reporter molecule was observed, the fluorescence of the standard fluorescent substance was also observed. Thus, it was able to be recognized that the wells were filled with the sample and the reagent containing the reporter molecule, and a reaction of the Amplite reporter molecule by AChE in the MDA-MB-231-derived extracellular vesicles worked in the microcompartments.

Next, the above-mentioned fluorescence microscope images were subjected to predetermined image processing to provide histograms shown in FIG. 48A to FIG. 48C in 3 hours of reaction with the Amplite reporter molecule. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 3 hours of reaction is shown. In addition, FIG. 49 shows plots of the expected value λ with respect to the extracellular vesicle concentration. A power approximation can be performed satisfactorily. The extracellular vesicle concentration was able to be accurately quantified.

System of Amplite Reporter Molecule

[Table 9]

| Fluorescence microscope image | Reaction time | Concentration [a.u.] | Number of wells | Number of positives | Positive ratio | Expected value λ |
|---|---|---|---|---|---|---|
| FIG. 47A | 3 h | 1 | 86,863 | 3,460 | 0.039832 | 0.040647 |
| FIG. 47B | 3 h | 0.1 | 86,811 | 517 | 0.005955 | 0.005973 |
| FIG. 47C | 3 h | 0.01 | 86,981 | 104 | 0.001195 | 0.001196 |

In the approximation of FIG. 49, λ at x1 is 0.0386, the volume of the microcompartment is 50 fL, and hence the concentration of the extracellular vesicles that can be detected with the Amplite reporter molecule based on the AChE activity can be calculated to be $7.7\times10^8$/mL. The reaction solution concentration is 5 μg of protein/mL, and hence the extracellular vesicle concentration is $1.5\times10^8$/μg of protein. Accordingly, it is estimated that 1 μL of the MDA-MB-231-derived extracellular vesicles (50 μg of protein in 50 μL of PBS) contain $1.5\times10^8$ extracellular vesicles in which AChE activity can be detected with the Amplite reporter molecule.

It was shown that digital detection of the extracellular vesicles was able to be performed through use of AChE activity in addition to the digital detection of the extracellular vesicles through use of MMP 14 activity described in the preceding Examples.

Example 25

(Wells and Well Array)

A well array of Simoa Discs was used.

(Preparation of Reaction Solution for Wells)

A reaction solution for filling the wells 204 was prepared. 2 μL (corresponding to 2 μg) of the A549-derived extracellular vesicles (50 μg of protein in 50 μL of PBS) described in the section "Materials and the like" were aliquoted, and the buffer (4) was added to the aliquot to provide 200 μL of a solution. The concentration of the solution is 10 μg/mL. The concentration of 20 μg/mL was set to x1 in Example 8, and hence the concentration of the solution is x1/2. The solution was subjected to 10-fold serial dilution to prepare standard samples with three concentrations. Further, a sample free of extracellular vesicles was prepared similarly, and used as a sample for BG measurement.

In addition, for detection of AChE activity, 1 μL of the acetylcholine stock solution (500-fold) and 2.5 μL of the Thiolite Green stock solution (200-fold) included in Amplite Fluorimetric Acetylcholinesterase Assay Kit (Green Fluorescence), and 5 μL of 1 mM Alexa Fluor 647 serving as a standard fluorescent substance were added to 1,000 μL of the buffer (4) to provide 1,008.5 μL of an AChE detection solution. The concentration of the solution is x1/2 of the concentration specified in the kit.

100 μL of the AChE detection solution was added to 100 μL of each of the samples to provide a reaction solution for wells. In this case, the highest concentration of the extracellular vesicles in each of the reaction solutions is 5 μg/mL, and the concentration of the reporter molecule in each of the reaction solutions is x1/2 of the concentration specified in the kit, and the concentration of the standard fluorescent substance in each of the reaction solutions is 2.5 μM. 100 μL of 200 μL of each of the prepared reaction solutions for wells was used in the measurement of AChE activity with a 96-well plate.

(Evaluation of AChE Activity with 96-well Plate)

Evaluation was performed in the same manner as in Example 1 except that the reaction temperature was set to 23°C. ΔRFU was calculated by subtracting the measured value of the sample for BG measurement from the measured value of each of the samples.

FIG. 50 shows temporal changes in fluorescence intensity from the reporter molecule by the A549-derived extracellular vesicles. As shown in FIG. 50, it is found that the fluorescence intensity was increased with the reaction time, and AChE activity was able to be detected at concentrations of x1 and x1/10, but the AChE activity was not able to be detected at a concentration of x1/100. In this Example, the expression "x1" represents half of the concentration represented by the expression "x1" in Example 8.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence derived from the Amplite reporter molecule and the background fluorescence of the Amplite reporter molecule after 1.5 hours of reaction was acquired. In addition, a fluorescence microscope image containing the fluorescence of the standard fluorescent substance after 1.5 hours of reaction was acquired.

The wells were able to be recognized based on the fluorescence of the standard fluorescent substance. In addition, it was found that, in the wells in which the fluorescence of the Amplite reporter molecule was observed, the fluorescence of the standard fluorescent substance was also observed. Thus, it was able to be recognized that the wells were filled with the sample and the reagent containing the reporter molecule, and a reaction of the Amplite reporter molecule by AChE in the A549-derived extracellular vesicles worked in the microcompartments.

Next, the above-mentioned fluorescence microscope images were subjected to predetermined image processing to provide histograms shown in FIG. 51A to FIG. 51D in 1.5 hours of reaction with the Amplite reporter molecule. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 1.5 hours of reaction is shown. In addition, FIG. 52 shows plots of the expected value λ with respect to the extracellular vesicle concentration. A power approximation can be performed satisfactorily. The extracellular vesicle concentration was able to be accurately quantified.

System of Amplite Reporter Molecule

[Table 10]

Table 10

| Fluorescence microscope image | Reaction time | Concentration [a.u.] | Number of wells | Number of positives | Positive ratio | Expected value λ |
|---|---|---|---|---|---|---|
| Not displayed | 1.5 h | 1 | 86,847 | 3,790 | 0.043639 | 0.04462 |
| Not displayed | 1.5 h | 0.1 | 86,751 | 474 | 0.005463 | 0.005478 |
| Not displayed | 1.5 h | 0.01 | 86,888 | 67 | 0.000771 | 0.000771 |
| Not displayed | 1.5 h | 0 | 86,877 | 3 | 0.000035 | 0.000035 |

In the approximation of FIG. 52, λ at x1 is 0.0436, the volume of the microcompartment is 50 fL, and hence the concentration of the extracellular vesicles that can be detected with the Amplite reporter molecule based on the AChE activity can be calculated to be $8.7\times10^8$/mL. The reaction solution concentration is 5 μg of protein/mL, and hence the extracellular vesicle concentration is $1.7\times10^8$/μg of protein. Accordingly, it is estimated that 1 μL of the A549-derived extracellular vesicles (50 μg of protein in 50 μL of PBS) contain $1.7\times10^8$ extracellular vesicles in which AChE activity can be detected with the Amplite reporter molecule.

It was shown that digital detection of the A549-derived extracellular vesicles was able to be performed through use of the AChE activity in addition to the digital detection of the MDA-MB-231-derived extracellular vesicles described in the preceding Examples. In addition, the detection was able to be sufficiently performed even in 1.5 hours of reaction at room temperature.

Example 26

(Wells and Well Array)

A well array of Simoa Discs was used.

(Preparation of Reaction Solution for Wells)

A reaction solution for filling the wells 204 was prepared. 2 μL (corresponding to 2 μg) of the A549-derived extracellular vesicles (50 μg of protein in 50 μL of PBS) described in the section "Materials and the like" were aliquoted, and the buffer (4) used in Example 18 was added to the aliquot to provide 200 μL of a solution. The concentration of the solution is 10 μg/mL. The concentration of 10 μg/mL was set to x1 in Example 8, and hence the concentration of the solution is x1/2. The solution was subjected to 10-fold serial dilution to prepare standard samples with three concentrations. Further, a sample free of extracellular vesicles was prepared similarly, and used as a sample for BG measurement.

In addition, for detection of AChE activity, 10 μL of the acetylcholine stock solution (Component C) and 10 μL of the AChE substrate solution (Component A) included in SensoLyte 520 Acetylcholinesterase Activity Assay Kit, and 2.5 μL of 1 mM Alexa Fluor 647 serving as a standard fluorescent substance were added to 480 μL of the buffer (4) to provide 502.5 μL of an AChE detection solution. The concentration of the solution is x2 of the concentration specified in the kit.

100 μL of the AChE detection solution was added to 100 μL of each of the samples to provide a reaction solution for wells. In this case, the highest concentration of the extracellular vesicles in each of reaction solutions is 5 μg/mL, and the concentration of the reporter molecule in each of the reaction solutions is x2 of the concentration specified in the kit, and the concentration of the standard fluorescent substance in each of the reaction solutions is 2.5 μM. 100 μL of 200 μL of each of the prepared reaction solutions for wells was used in the measurement of AChE activity with a 96-well plate.

(Evaluation of AChE Activity with 96-well Plate)

Evaluation was performed in the same manner as in Example 1 except that the reaction temperature was set to 23°C. ΔRFU was calculated by subtracting the measured value of the sample for BG measurement from the measured value of each of the samples.

FIG. 53 shows temporal changes in fluorescence intensity from the reporter molecule by the A549-derived extracellular vesicles. As shown in FIG. 53, it is found that the fluorescence intensity was increased with the reaction time, and AChE activity was able to be detected at a concentration of x1, but the AChE activity was not able to be detected at concentrations of x1/10 and x1/100. In this Example, the expression "x1" represents half of the concentration represented by the expression "x1" in Example 8.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence derived from the SensoLyte reporter molecule and the background fluorescence of the SensoLyte reporter molecule after 5 hours of reaction was acquired. In addition, a fluorescence microscope image containing the fluorescence of the standard fluorescent substance after 5 hours of reaction was acquired.

The wells were able to be recognized based on the fluorescence of the standard fluorescent substance. In addition, it was found that, in the wells in which the fluorescence of the SensoLyte reporter molecule was observed, the fluorescence of the standard fluorescent substance was also observed. Thus, it was able to be recognized that the wells were filled with the sample and the reagent containing the reporter molecule, and a reaction of the SensoLyte reporter molecule by AChE in the A549-derived extracellular vesicles worked in the microcompartments.

Next, the above-mentioned fluorescence microscope images were subjected to predetermined image processing to provide histograms shown in FIG. 54A to FIG. 54D in 5 hours of reaction with the SensoLyte reporter molecule. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 5 hours of reaction is shown. In addition, FIG. 55 shows plots of the expected value λ with respect to the extracellular vesicle concentration. A power approximation can be performed satisfactorily. The extracellular vesicle concentration was able to be accurately quantified.

System of SensoLyte Reporter Molecule

[Table 11]

Table 11

| Fluorescence microscope image | Reaction time | Concentration [a.u.] | Number of wells | Number of positives | Positive ratio | Expected value λ |
|---|---|---|---|---|---|---|
| Not displayed | 5 h | 1 | 28,880 | 512 | 0.017729 | 0.017888 |
| Not displayed | 5 h | 0.1 | 29,039 | 55 | 0.001894 | 0.001896 |
| Not displayed | 5 h | 0.01 | 29,018 | 5 | 0.000172 | 0.000172 |
| Not displayed | 5 h | 0 | 28,887 | 3 | 0.000103 | 0.000103 |

In the approximation of FIG. 55, λ at x1 is 0.0184, the volume of the microcompartment is 50 fL, and hence the concentration of the extracellular vesicles that can be detected with the SensoLyte reporter molecule based on the AChE activity can be calculated to be $3.7 \times 10^8$/mL. The reaction solution concentration is 5 μg of protein/mL, and hence the extracellular vesicle concentration is $7.4 \times 10^7$/μg of protein. Accordingly, it is estimated that 1 μL of the A549-derived extracellular vesicles (50 μg of protein in 50 μL of PBS) contain $7.4 \times 10^7$ extracellular vesicles in which AChE activity can be detected with the SensoLyte reporter molecule. In Example 25, it was estimated that 1 μL of the A549-derived extracellular vesicles contained $1.7 \times 10^8$ extracellular vesicles in which AChE activity was able to be detected with the Amplite reporter molecule. However, a value for the number of the extracellular vesicles in which AChE activity can be detected with the SensoLyte reporter molecule is about 40% of the number of the extracellular vesicles in which AChE activity can be detected with the Amplite reporter molecule. The SNR between positive and negative wells with regard to the Amplite reporter molecule is higher than that with regard to the SensoLyte reporter molecule, and positivity and negativity can be clearly separated. It is presumed that this contributes to improvement in detection sensitivity.

It was shown that digital detection of the extracellular vesicles with the SensoLyte reporter molecule was able to be performed through use of the AChE activity in addition to the digital detection of the extracellular vesicles with the Amplite reporter molecule described in the preceding Examples.

Example 27

(Wells and Well Array)

A well array of Simoa Discs was used.

(Preparation of Reaction Solution for Wells)

A reaction solution for filling the wells 204 was prepared. 2 μL (corresponding to 2 μg) of the A549-derived extracellular vesicles (50 μg of protein in 50 μL of PBS) described in the section "Materials and the like" were aliquoted, and the buffer (4) used in Example 18 was added to the aliquot to provide 200 μL of a solution. The concentration of the solution is 10 μg/mL. The concentration of 20 μg/mL was set to x1 in Example 8, and hence the concentration of the solution is x1/2. The solution was subjected to 10-fold serial dilution to prepare standard samples with three concentrations. Further, a sample free of extracellular vesicles was prepared similarly, and used as a sample for BG measurement.

In addition, for detection of MMP14 activity, 10 μL of 1 mM Custom #2 and 2.5 μL of 1 mM Alexa Fluor 647 serving as a standard fluorescent substance were added to 490 μL of the buffer (4) to provide 502.5 μL of a MMP14 detection solution.

100 μL of the MMP14 detection solution was added to 100 μL of each of the samples to provide a reaction solution for wells. In this case, the highest concentration of the extracellular vesicles in each of the reaction solutions is 5 μg/mL, the concentration of the reporter molecule in each of the reaction solutions is 10 μM, and the concentration of the standard fluorescent substance in each of the reaction solutions is 2.5 μM. 100 μL of 200 μL of each of the prepared reaction solutions for wells was used in the measurement of MMP14 activity with a 96-well plate.

(Evaluation of AChE Activity with 96-well Plate)

Evaluation was performed in the same manner as in Example 1 except that the reaction temperature was set to 23°C. ΔRFU was calculated by subtracting the measured value of the sample for BG measurement from the measured value of each of the samples.

FIG. 56 shows temporal changes in fluorescence intensity from the reporter molecule by the A549-derived extracellular vesicles. As shown in FIG. 56, it is found that the fluorescence intensity was increased with the reaction time, and MMP14 activity was able to be detected at a concentration of x1, but the MMP14 activity was not able to be detected at concentrations of x1/10 and x1/100. In this Example, the expression "x1" represents half of the concentration represented by the expression "x1" in Example 8.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence derived from Custom #2 and the background fluorescence of Custom #2 after 5 hours of reaction was acquired. In addition, a fluorescence microscope image containing the fluorescence of the standard fluorescent substance after 5 hours of reaction was acquired.

The wells were able to be recognized based on the fluorescence of the standard fluorescent substance. In addition, it was found that, in the wells in which the fluorescence of Custom #2 was observed, the fluorescence of the standard fluorescent substance was also observed. Thus, it was able to be recognized that the wells were filled with the sample and the reagent containing the reporter molecule, and a reaction of Custom #2 by MMP14 in the A549-derived extracellular vesicles worked in the microcompartments.

Next, the above-mentioned fluorescence microscope images were subjected to predetermined image processing to provide histograms shown in FIG. 57A to FIG. 57D in 5 hours of reaction with Custom #2. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 5 hours of reaction is shown. In addition, FIG. 58 shows plots of the expected value λ with respect to the extracellular vesicle concentration. A power approximation can be performed satisfactorily. The extracellular vesicle concentration was able to be accurately quantified.

System of Custom #2

[Table 12]

Table 12

| Fluorescence microscope image | Reaction time | Concentration [a.u.] | Number of wells | Number of positives | Positive ratio | Expected value λ |
|---|---|---|---|---|---|---|
| Not displayed | 5 h | 1 | 86,803 | 20,982 | 0.24172 | 0.276702 |
| Not displayed | 5 h | 0.1 | 86,881 | 2,766 | 0.031837 | 0.032354 |
| Not displayed | 5 h | 0.01 | 86,890 | 357 | 0.004109 | 0.004117 |
| Not displayed | 5 h | 0 | 86,832 | 2 | 0.000023 | 0.000023 |

In the approximation of FIG. 58, λ at x1 is 0.2767, the volume of the microcompartment is 50 fL, and hence the concentration of the extracellular vesicles that can be detected with Custom #2 based on the MMP14 activity can be calculated to be $5.5\times10^9$/mL. The reaction solution concentration is 5 μg of protein/mL, and hence the extracellular vesicle concentration is $1.1\times10^9$/μg of protein. Accordingly, it is estimated that 1 μL of the A549-derived extracellular vesicles (50 μg of protein in 50 μL of PBS) contain $1.1\times10^9$ extracellular vesicles in which MMP14 activity can be detected with Custom #2.

Example 28

(Production of Antibody-immobilized Particles)

Homebrew 2.0 Multiplex Beads 647 L1.5:103527 (manufactured by Quanterix Corporation) were used as magnetic particles. To the magnetic particles buffer-exchanged into Bead Conjugation Buffer (manufactured by Quanterix Corporation), 1-ethyl-3-(3-dimethylaminopropyl)carbodiimide hydrochloride (EDC) (manufactured by Thermo Fisher Scientific Inc.) dissolved in the same Bead Conjugation Buffer was added, the mixture was stirred at 4°C for 30 minutes, and the magnetic particles were then recovered with a magnet. The recovered magnetic particles were dispersed in Bead Conjugation Buffer, and an anti-CD9 antibody (ab58989 manufactured by Abcam plc.), an anti-CD63 antibody (ab59479 manufactured by Abcam plc.), and an anti-CD81 antibody (ab59477 manufactured by Abcam plc.) were added. After that, the mixture was stirred at 4°C for 2 hours. After that, the mixture was washed with Bead Wash Buffer (manufactured by Quanterix Corporation), Bead Blocking Buffer (manufactured by Quanterix Corporation) was then added, and the mixture was stirred at room temperature for 45 minutes. The magnetic particles were recovered with a magnet, washed with Bead Wash Buffer, and then dispersed in Bead Diluent Buffer (manufactured by Quanterix Corporation) to provide an antibody-immobilized particle solution. The antibody-immobilized particle solution was stored at 4°C until use.

(Wells and Well Array)

A well array of Simoa Discs was used.

(Preparation of Reaction Solution for Wells)

A reaction solution for filling the wells 204 was prepared. 2 μL (corresponding to 2 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 200 μL of a solution serving as a sample. The concentration of the solution is 10 μg/mL. Further, a sample free of extracellular vesicles was prepared similarly, and used as a sample for BG measurement.

Each of the above-mentioned samples and the following were mixed.

Each of the samples prepared above: 100 μL

The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.

For detection of MMP14 activity, 1 mM Custom #2 was diluted with the buffer (4) to prepare a MMP14 detection solution containing 10 μM Custom #2.

25 μL of the MMP14 detection solution was added to the recovered complexes to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

FIG. 59A to FIG. 59D show fluorescence microscope images of the wells acquired after 5 hours of reaction. FIG. 59A and FIG. 59B show fluorescence microscope images each containing both of the fluorescence of 5-FAM derived from the reporter molecule and the background fluorescence of the reporter molecule. In addition, FIG. 59C and FIG. 59D show fluorescence microscope images each containing the fluorescence of Multiplex Beads 647. From FIG. 59C and FIG. 59D, the wells filled with the particles were able to be recognized. In addition, it is found from FIG. 59A that the fluorescence of 5-FAM is observed from the wells filled with the particles. Thus, it was able to be recognized that the MDA-MB-231-derived extracellular vesicles were captured on the particles filling the microcompartments, and a reaction of Custom #2 by MMP14 in the extracellular vesicles worked.

Next, the above-mentioned fluorescence microscope images were subjected to predetermined image processing to provide histograms shown in FIG. 60A and FIG. 60B in 5 hours of reaction with Custom #2. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the number of wells filled with the particles and the number of positive wells identified from each of the histograms after 5 hours of reaction is shown.

System of Custom #2

[Table 13]

Table 13

| Fluorescence microscope image | Reaction time | Concentration [a.u.] | Number of particles | Number of positives | Positive ratio | Expected value λ |
|---|---|---|---|---|---|---|
| FIG. 59A | 5 h | 1 | 2,287 | 338 | 0.147792 | 0.159924 |
| FIG. 59B | 5 h | 0 | 2,026 | 1 | 0.000493 | 0.000493 |

The concentration of the sample was 10 μg/mL, 100 μL of the sample was used, and hence the extracellular vesicles with 1 μg of protein were used in the reaction. 25 μL of the particles at a concentration of $2.0\times10^7$ particles/mL were used, and hence the number of the particles subjected to the reaction is $5.0\times10^5$ The expected value Δλ per particle (value obtained by subtracting BG) is 0.159431, and hence the extracellular vesicle concentration is estimated to be $8.0\times10^4$ extracellular vesicles/μg of protein.

Accordingly, the extracellular vesicles were captured on the particles to which a capture antibody including a cocktail of the anti-CD9 antibody, the anti-CD63 antibody, and the anti-CD81 antibody was bound, and the extracellular vesicles were able to be detected and quantified by the MMP14 activity of the extracellular vesicles.

Example 29

(Production of Antibody-immobilized Particles)

An antibody-immobilized particle solution was prepared and stored at 4°C until use, in the same manner as in Example 28.

(Wells and Well Array)

A well array of Simoa Discs was used.

(Preparation of Reaction Solution for Wells)

A reaction solution for filling the wells 204 was prepared. 2 μL (corresponding to 2 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 200 μL of a solution serving as a sample. The concentration of the solution is 10 μg/mL. Further, a sample free of extracellular vesicles was prepared similarly, and used as a sample for BG measurement.
Each of the above-mentioned samples and the following were mixed.

Each of the samples prepared above: 100 μL
The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.
For detection of AChE activity, 1 μL of the acetylcholine stock solution (500-fold) and 2.5 μL of the Thiolite Green stock solution (200-fold) included in Amplite Fluorimetric Acetylcholinesterase Assay Kit (Green Fluorescence) were added to 1,000 μL of the buffer (4) to provide 1,003.5 μL of an AChE detection solution. The concentration of the solution is x1/2 of the concentration specified in the kit, but an equal amount of a sample solution is not added at the time of a reaction, and hence the final concentration during the reaction is the concentration specified in the kit.
25 μL of the AChE detection solution was added to the recovered complexes to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).
The observation of fluorescence derived from each fluorescent substance was performed under the same conditions as in Example 19 to acquire a fluorescence image.
FIG. 61A to FIG. 61D show fluorescence microscope images of the wells acquired after 3 hours of reaction. FIG. 61A and FIG. 61B each show a fluorescence microscope image containing both of the fluorescence derived from the reporter molecule and the background fluorescence of the reporter molecule. In addition, FIG. 61C and FIG. 61D each show a fluorescence microscope image containing the fluorescence of Multiplex Beads 647. From FIG. 61C and FIG. 61D, the wells filled with the particles were able to be recognized. In addition, it is found from FIG. 61A that the fluorescence is observed from the wells filled with the particles. Thus, it was able to be recognized that the MDA-MB-231-derived extracellular vesicles were captured on the particles filling the microcompartments, and a reaction of the Amplite reporter molecule by the AChE activity of the extracellular vesicles worked.
Next, the above-mentioned fluorescence microscope images were subjected to predetermined image processing to provide histograms shown in FIG. 62A and FIG. 62B in 3 hours of reaction with the Amplite reporter molecule. Those histograms each show the number of wells each showing a fluorescence intensity included in each fraction of fluorescence intensity obtained by dividing fluorescence intensities into intervals of a constant value.
The expected value (λ) calculated with respect to the concentration of the extracellular vesicles subjected to the test through use of the above-mentioned equation (1) from the number of wells filled with the particles and the number of positive wells identified from each of the histograms after 3 hours of reaction is shown.

System of Amplite Reporter Molecule

[Table 14]

Table 14

| Fluorescence microscope image | Reaction time | Concentration [a.u.] | Number of particles | Number of positives | Positive ratio | Expected value λ |
|---|---|---|---|---|---|---|
| FIG. 61A | 3 h | 1 | 861 | 52 | 0.060395 | 0.062296 |
| FIG. 61B | 3 h | 0 | 13,263 | 36 | 0.002714 | 0.002718 |

The concentration of the sample was 10 μg/mL, 100 μL of the sample was used, and hence the extracellular vesicles with 1 μg of protein were used in the reaction. 25 μL of the particles at a concentration of $2.0\times10^7$ particles/mL were used, and hence the number of the particles subjected to the reaction is $5.0\times10^5$ The expected value $\Delta\lambda$ per particle (value obtained by subtracting BG) is 0.059578, and hence the extracellular vesicle concentration is estimated to be $3.0\times10^4$ extracellular vesicles/μg of protein.

Accordingly, the extracellular vesicles were captured on the particles to which a capture antibody including a cocktail of the anti-CD9 antibody, the anti-CD63 antibody, and the anti-CD81 antibody was bound, and the extracellular vesicles were able to be detected and quantified by the AChE activity of the extracellular vesicles.

Example 30

(Production of Antibody-immobilized Particles)

An antibody-immobilized particle solution was prepared and stored at 4°C until use, in the same manner as in Example 28.

(Wells and Well Array)

A well array of Simoa Discs was used.

(Preparation of Reaction Solution for Wells)

A reaction solution for filling the wells 204 was prepared. 2 μL (corresponding to 2 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 200 μL of a solution serving as a sample. The concentration of the solution is 10 μg/mL. Further, a sample free of extracellular vesicles was prepared similarly, and used as a sample for BG measurement.

Each of the above-mentioned samples and the following were mixed.

Each of the samples prepared above: 100 μL
The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.

For detection of AChE activity, 5 μL of the acetylcholine solution (Component C) and 5 μL of the AChE substrate solution (Component A) included in SensoLyte 520 Acetylcholinesterase Activity Assay Kit were added to 490 μL of the buffer (4) to provide 500 μL of an AChE detection solution. The concentration of the solution is the concentration specified in the kit. An equal amount of a sample solution is not added at the time of a reaction, and hence the final concentration during the reaction is x2 of the concentration specified in the kit.

25 μL of the AChE detection solution was added to the recovered complexes to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence derived from the reporter molecule and the background fluorescence of the reporter molecule was acquired. In addition, a fluorescence microscope image containing the fluorescence of Multiplex Beads 647 was acquired. The wells filled with the particles were able to be recognized. In addition, it was found that fluorescence was observed from the wells filled with the particles. Thus, it was able to be recognized that the MDA-MB-231-derived extracellular vesicles were captured on the particles filling the microcompartments, and a reaction of the SensoLyte reporter molecule by the AChE activity of the extracellular vesicles worked.

Next, the above-mentioned fluorescence microscope images were subjected to predetermined image processing to provide histograms shown in FIG. 63A and FIG. 63B in 5 hours of reaction with the SensoLyte reporter molecule. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the number of wells filled with the particles and the number of positive wells identified from each of the histograms after 5 hours of reaction is shown.

System of SensoLyte Reporter Molecule

[Table 15]

Table 15

| Fluorescence microscope image | Reaction time | Concentration [a.u.] | Number of particles | Number of positives | Positive ratio | Expected value λ |
|---|---|---|---|---|---|---|
| Not displayed | 5 h | 1 | 851 | 43 | 0.050529 | 0.05185 |
| Not displayed | 5 h | 0 | 471 | 3 | 0.006369 | 0.00639 |

The concentration of the sample was 10 μg/mL, 100 μL of the sample was used, and hence the extracellular vesicles with 1 μg of protein were used in the reaction. 25 μL of the particles at a concentration of $2.0\times10^7$ particles/mL were used, and hence the number of the particles subjected to the reaction is $5.0\times10^5$ The expected value Δλ per particle (value obtained by subtracting BG) is 0.04546, and hence the extracellular vesicle concentration is estimated to be $2.3\times10^4$ extracellular vesicles/μg of protein.

Accordingly, the extracellular vesicles were captured on the particles to which a capture antibody including a cocktail of the anti-CD9 antibody, the anti-CD63 antibody, and the anti-CD81 antibody was bound, and the extracellular vesicles were able to be detected and quantified by the AChE activity of the extracellular vesicles.

Example 31

(Production of Antibody-immobilized Particles)

An antibody-immobilized particle solution was prepared and stored at 4°C until use, in the same manner as in Example 28.

(Wells and Well Array)

A well array of Simoa Discs was used.

(Preparation of Reaction Solution for Wells)

A reaction solution for filling the wells 204 was prepared. 2 μL (corresponding to 2 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 200 μL of a solution serving as a sample. The concentration of the solution is 10 μg/mL. Further, a sample free of extracellular vesicles was prepared similarly, and used as a sample for BG measurement.
Each of the above-mentioned samples and the following were mixed.

Each of the samples prepared above: 50 μL
The antibody-immobilized particles ($4.0\times10^7$ particles/mL) produced above: 50 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.
For detection of MMP14 activity, 1 mM Custom #2 was diluted with the buffer (4) to prepare a MMP14 detection solution containing 10 μM Custom #2.
100 μL of the MMP14 detection solution was added to the recovered complexes to provide a reaction solution for wells, and 25 μL of the reaction solution was used to fill wells. The number of the particles was increased 4-fold, and a reaction was allowed to proceed. However, the number and concentration of the particles used to fill the wells are finally the same as those in other Examples.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 37°C to promote the generation of fluorescence. After that, observation was performed with a fluorescence microscope (BZ-X800).
The observation of fluorescence derived from each fluorescent substance was performed under the same conditions as in Example 19 to acquire a fluorescence image.
Although the illustration is omitted, a fluorescence microscope image containing both of the fluorescence of 5-FAM derived from the reporter molecule and the background fluorescence of the reporter molecule was acquired. In addition, a fluorescence microscope image containing the fluorescence of Multiplex Beads 647 was acquired. The wells filled with the particles were able to be recognized. In addition, it was found that the fluorescence of 5-FAM was observed from the wells filled with the particles. Thus, it was able to be recognized that the MDA-MB-231-derived extracellular vesicles were captured on the particles filling the microcompartments, and a reaction of Custom #2 by MMP14 in the extracellular vesicles worked.
Next, the above-mentioned fluorescence microscope images were subjected to predetermined image processing to provide histograms shown in FIG. 64A and FIG. 64B in 5 hours of reaction with Custom #2. Those histograms each show the number of wells each showing a fluorescence intensity included in each fraction of fluorescence intensity obtained by dividing fluorescence intensities into intervals of a constant value.
The expected value (λ) calculated with respect to the concentration of the extracellular vesicles subjected to the test through use of the above-mentioned equation (1) from the number of wells filled with the particles and the number of positive wells identified from each of the histograms after 5 hours of reaction is shown. With regard to BG, the value of Example 28 was used.

System of Custom #2

[Table 16]

Table 16

| Fluorescence microscope image | Reaction time | Concentration [a.u.] | Number of particles | Number of positives | Positive ratio | Expected value λ |
|---|---|---|---|---|---|---|
| Not displayed | 5 h | 1 | 9,220 | 402 | 0.043601 | 0.04458 |
| Not displayed | 5 h | 1 | 9,295 | 410 | 0.04411 | 0.045112 |
| Not displayed | 5 h | 1 | 18,515 | 812 | 0.043856 | 0.044847 |
| FIG. 59B | 5 h | 0 | 2,026 | 1 | 0.000493 | 0.000493 |

The concentration of the sample was 10 μg/mL, 50 μL of the sample was used, and hence the extracellular vesicles with 0.5 μg of protein were used in the reaction. 50 μL of the particles at a concentration of $4.0\times10^7$ particles/mL were used, and hence the number of the particles subjected to the reaction is $2.0\times10^6$. The expected value Δλ per particle (value obtained by subtracting BG) can be calculated from the sum of the numbers of particles and the numbers of positives in the two images, to be 0.044354, and hence the extracellular vesicle concentration is estimated to be $1.8\times10^5$ extracellular vesicles/μg of protein. This extracellular vesicle concentration is larger than $8.0\times10^4$ extracellular vesicles/μg of protein that is the value estimated in Example 28. This is presumably because the capture efficiency of the extracellular vesicles was improved by the reaction with the 4-fold number of particles at the 5-fold particle concentration.

Accordingly, the extracellular vesicles were captured on the particles to which a capture antibody including a cocktail of the anti-CD9 antibody, the anti-CD63 antibody, and the anti-CD81 antibody was bound, and the extracellular vesicles were able to be detected and quantified by the MMP14 activity of the extracellular vesicles.

Example 32

In this Example, liquid droplets were used as individual separated compartments.

(Preparation of Sample for forming Liquid Droplets)

A reaction solution for forming liquid droplets was prepared as described below.

1.5 μL (corresponding to 1.5 μg) of the MDA-MB-231-derived extracellular vesicles (50 μg of protein in 50 μL of PBS) described in the section "Materials and the like" were aliquoted, and the buffer (4) was added to the aliquot to provide 300 μL of a solution. The concentration of the solution is 5 μg/mL.

In addition, for detection of MMP14 activity, 1.5 μL of 1 mM Custom #1 and 1.5 μL of 1 mM Alexa Fluor 647 serving as a standard fluorescent substance were added to the above-mentioned sample to provide a MMP14 detection solution containing Custom #1 at a final concentration of 5 μM and Alexa Fluor 647 at a final concentration of 5 μM.

(Preparation of Liquid Droplets)

Liquid droplets were prepared by a pumping method of an SPG emulsification membrane through use of the reaction solution for forming liquid droplets prepared in the foregoing. A dispersed phase and a continuous phase are shown below.

·Dispersed phase: the above-mentioned reaction solution for forming liquid droplets 80 μL
·Continuous phase: an aliphatic hydrocarbon (product name: Isopar L, manufactured by Exxon Mobil Corporation) having a surfactant (product name: KF-6038, manufactured by Shin-Etsu Chemical Co., Ltd.) dissolved therein at a concentration of 4% 2.5 mL

An SPG pumping connector (pore diameter: 10 μm, pore diameter: 5 μm, manufactured by SPG Technology Co., Ltd.) was used as the SPG emulsification membrane, and the number of times of pumping reciprocations was set to 10. Thus, liquid droplets each having a diameter of from about 1 μm to about 10 μm were obtained.

(Fluorescence Microscope Observation)

The emulsified liquid prepared in the foregoing were subjected to a reaction through incubation at 37°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

FIG. 65A to FIG. 65C are a bright-field image and fluorescence microscope images acquired after 4 hours of reaction of the liquid droplets produced with an SPG pumping connector (pore diameter: 10 μm). FIG. 65A shows a bright-field image of the liquid droplets. FIG. 65B shows a fluorescence microscope image containing both of the fluorescence of 5-FAM derived from the reporter molecule and the background fluorescence of the reporter molecule. In addition, FIG. 65C shows a fluorescence microscope image containing the fluorescence of the standard fluorescent substance.

FIG. 66A to FIG. 66C are a bright-field image and fluorescence microscope images acquired after 4 hours of reaction of the liquid droplets produced with an SPG pumping connector (pore diameter: 5 μm). FIG. 66A shows a bright-field image of the liquid droplets. FIG. 66B shows a fluorescence microscope image containing both of the fluorescence of 5-FAM derived from the reporter molecule and the background fluorescence of the reporter molecule. In addition, FIG. 66C shows a fluorescence microscope image containing the fluorescence of the standard fluorescent substance.

The liquid droplets were able to be recognized based on the fluorescence of the standard fluorescent substance similarly to Example 19. It is found that, in the liquid droplets in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the MDA-MB-231-derived extracellular vesicles worked in the microcompartments.

Next, the images acquired with an SPG pumping connector with a pore diameter of 10 μm were analyzed in the same manner as in Example 19. The above-mentioned fluorescence microscope images after 4 hours of reaction were subjected to predetermined image processing to provide a particle diameter distribution of the liquid droplets shown in FIG. 67, and obtain a histogram representing the number of liquid droplets each showing a fluorescence intensity included in each fraction of fluorescence intensity obtained by dividing fluorescence intensities into intervals of a constant value in each particle diameter range.

Further, when the concentration of the extracellular vesicles having MMP14 activity was calculated through use of the above-mentioned equation (1) from the total number of liquid droplets in each particle diameter range and the number of positive liquid droplets identified from the histogram, the concentration was able to be estimated as described below.

[Table 17]

Table 17

| Particle diameter (μm) | Number of particles | Number of positives | Positive ratio | Expected value λ | Average volume (fL) | Concentration (/mL) |
|---|---|---|---|---|---|---|
| <3 | 173 | 8 | 0.046 | 0.047 | 4.55 | 1.04E+10 |
| 3-4 | 77 | 15 | 0.195 | 0.217 | 20.5 | 1.06E+10 |
| 4-5 | 47 | 16 | 0.340 | 0.416 | 46.0 | 9.05E+09 |
| 5-6 | 52 | 29 | 0.558 | 0.816 | 82.8 | 9.85E+09 |
| 6-7 | 51 | 37 | 0.725 | 1.293 | 143.2 | 9.03E+09 |
| 7-8 | 27 | 23 | 0.852 | 1.910 | 211.9 | 9.01E+09 |
| >8 | 27 | 26 | 0.963 | 3.296 | 379.5 | 8.68E+09 |

From the above-mentioned estimation, the average and standard deviation of the extracellular vesicle concentrations can be calculated to be $9.5\times10^9$/mL and $7.0\times10^8$/mL, respectively. The reaction solution concentration is 5.0 μg of protein/mL, and hence the extracellular vesicle concentration is $1.9\times10^9$/μg of protein. Accordingly, it is estimated that 1 μL of the MDA-MB-231-derived extracellular vesicles (50 μg of protein in 50 μL of PBS) contain $1.9\times10^9$ extracellular vesicles in which MMP14 activity can be detected with the reporter molecule Custom #1. This is a result similar to the estimated result of $2.1\times10^9$ extracellular vesicles in the system in which the wells were used as the microcompartments in Example 20. Accordingly, it is found that a similar result is obtained in a similar reaction system irrespective of whether the wells or the liquid droplets are used as the microcompartments.

Example 33

(Production of Antibody-immobilized Particles)

An antibody-immobilized particle solution was prepared and stored at 4°C until use, in the same manner as in Example 28.

(Production of Detection Antibody in which Antibody is conjugated with Biotin)

An anti-CD9 antibody (ab58989), an anti-CD63 antibody (ab59479), and an anti-CD81 antibody (ab59477) were buffer-exchanged into Biotinylation Reaction Buffer to provide an antibody solution. A biotinylation reagent NHS-PEG4-Biotin was added to the solution, and the mixture was stirred and then subjected to a reaction at room temperature for 30 minutes. After that, the resultant was washed with Biotinylation Reaction Buffer, and then recovered with Biotinylation Reaction Buffer to provide a biotinylated detection antibody solution. The biotinylated detection antibody solution was stored at 4°C until use.

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 400 μL of a solution serving as a sample. The concentration of the solution is 10 μg/mL. The solution was subjected to 3-fold serial dilution to prepare standard samples with seven concentrations. Further, a sample free of extracellular vesicles was prepared similarly, and used as a sample for BG measurement.
Each of the above-mentioned samples and the antibody-immobilized particles were mixed as described below.

Each of the samples prepared above: 100 μL
The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.
The recovered composite particles were subjected to a reaction with the following.
Biotinylated detection antibody (0.3 μg/mL): 100 μL
The resultant mixed solution was subjected to a reaction with shaking at 30°C for 10 minutes to form sandwich-type particle complexes in which the biotinylated detection antibody was further bound to each of the particle complexes of the antibody-immobilized particles and the extracellular vesicles. After that, washing was performed with a plate washer and the complexes were then recovered with a magnet.
The recovered composite particles were subjected to a reaction with the following.
Streptavidin-conjugated β-galactosidase (SBG) (150 pM): 100 μL
The resultant mixed solution was subjected to a reaction with shaking at 30°C for 10 minutes to form complexes in which streptavidin-conjugated β-galactosidase was further bound to the biotinylated detection antibody of each of the complexes described above. After that, washing was performed with a plate washer, and the complexes were then recovered with a magnet and used in a subsequent reaction.
In addition, part of the complexes prepared in the foregoing were subjected to evaluation for reference through use of Simoa SR-X. The evaluation through use of Simoa SR-X was performed according to the operation procedure for Simoa SR-X.
For detection of MMP14 activity, 1 mM Custom #2 and a 100 μM resorufin β-D-galactopyranoside solution were diluted with the buffer (4) to prepare a MMP14 and CD9/CD63/CD81 cocktail detection solution (1) containing 10 μM Custom #2 and 5 μM resorufin β-D-galactopyranoside. In addition, the solution was diluted 2-fold with the buffer (4) to prepare a detection solution (2). Nonfluorescent resorufin β-D-galactopyranoside is degraded by β-galactosidase to produce fluorescent resorufin. In this case, the maximum wavelengths of excitation and fluorescence of resorufin are 570 nm and 585 nm, respectively.
50 μL of the detection solution (2) was added to the recovered complexes to provide a reaction solution for wells.
In addition, 50 μL of the detection solution (1) was added to 50 μL of each of the prepared standard samples with seven concentrations and the sample for BG measurement, and the resultant solution was used in measurement of MMP14 activity and β-galactosidase activity with a 96-well plate. Equal amounts of each of the samples and the detection solution (1) are added, and hence the final concentration of the reporter molecule is the same as that of the detection solution (2).

(Evaluation of MMP14 Activity and β-Galactosidase Activity in 96-well Plate)

Evaluation was performed in the same manner as in Example 1 except that the reaction temperature was set to 30°C. ΔRFU was calculated by subtracting the measured value of the sample for BG measurement from the measured value of each of the samples. In this case, resorufin produced by β-galactosidase activity was simultaneously measured at an excitation wavelength of 545±20 nm and a fluorescence wavelength of 590±20 nm, and ΔRFU was calculated similarly.

FIG. 68A shows temporal changes in fluorescence intensity from the MMP14 activity reporter molecule by the MDA-MB-231-derived extracellular vesicles. As shown in FIG. 68A, it is found that the fluorescence intensity was increased with the reaction time, and MMP14 activity was able to be detected at concentrations of x1/9 or more, but the MMP14 activity was not able to be detected at concentrations of x1/27 or less. In this Example, the expression "x1" represents half of the concentration represented by the expression "x1" in Example 8. FIG. 68B shows temporal changes in fluorescence intensity from the β-galactosidase activity reporter molecule (resorufin β-D-galactopyranoside) by the MDA-MB-231-derived extracellular vesicles. It is found that the activity was not able to be detected at all as shown in FIG. 68B. This system does not include a CD9/CD63/CD81 detection system, and hence it is found that the β-galactosidase activity inherent in the MDA-MB-231-derived extracellular vesicles is equal to or less than the detection limit of this system, and it is also found that this system does not affect the evaluation of β-galactosidase activity used in the CD9/CD63/CD81 detection system.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image. In this case, a TRITC filter was used in the observation of the fluorescence from resorufin.

Resorufin

TRITC: OP-87764 (manufactured by Keyence Corporation)
Model: OP-87764
Excitation wavelength: 545/25 nm
Absorption wavelength: 605/70 nm
Dichroic wavelength: 565 nm

FIG. 69A to FIG. 69C each show an example of fluorescence microscope images of the wells acquired after 4 hours of reaction. The images obtained from the standard sample at a concentration of x1/9 are shown in the figures. FIG. 69A shows a fluorescence microscope image containing both of the fluorescence of 5-FAM derived from Custom #2 and the background fluorescence of the reporter molecule. In addition, FIG. 69B shows a fluorescence microscope image containing both of the fluorescence of resorufin derived from resorufin β-D-galactopyranoside and the background fluorescence of the reporter molecule. FIG. 69C shows a fluorescence microscope image containing the fluorescence of Multiplex Beads 647. The wells filled with the particles were able to be recognized from FIG. 69C. In addition, it is found from FIG. 69A and FIG. 69B that the fluorescence of 5-FAM and the fluorescence of resorufin are observed from the wells filled with the particles. Thus, it was able to be recognized that: the MDA-MB-231-derived extracellular vesicles were captured on the particles filling the microcompartments, and a reaction of Custom #2 by MMP14 in the extracellular vesicles worked; and the biotinylated detection antibody was bound to CD9/CD63/CD81 in the extracellular vesicles, and a reaction of the reporter molecule (resorufin β-D-galactopyranoside) worked by streptavidin-conjugated β-galactosidase further bound to biotin. Accordingly, it was able to be recognized that the extracellular vesicles captured on the particles were able to be detected in a multiplex manner through use of enzymatic activity and a surface antigen, which were biomarkers having different properties.

Next, the above-mentioned fluorescence microscope images were subjected to predetermined image processing to provide histograms shown in FIG. 70A (5-FAM) and FIG. 70B (resorufin) from the fluorescence derived from each of the reporter molecules. Those histograms each show the number of wells each showing a fluorescence intensity included in each fraction of fluorescence intensity obtained by dividing fluorescence intensities into intervals of a constant value. Similarly, with regard to standard samples of other dilution series, the fluorescence microscope images were analyzed to provide histograms.

The expected value (λ) calculated with respect to the concentration of the extracellular vesicles subjected to the test through use of the above-mentioned equation (1) from the number of wells filled with the particles and the number of positive wells identified from each of the histograms after 4 hours of reaction is shown. In addition, results obtained by evaluating, with Simoa SR-X, samples prepared similarly are collectively shown (the column "Simoa").

[Table 18]

Table 18

| | | MMP14 | | CD9/CD63/ CD81 | | |
|---|---|---|---|---|---|---|
| Concentration [a.u.] | Number of particles | Number of positives | Expected value λ | Number of positives | Expected value λ | Simoa |
| X1 | 2,298 | 298 | 0.139 | 2,166 | 2.86 | 5.09 |
| X1/3 | 2,739 | 218 | 0.0829 | 2,315 | 1.87 | 3.44 |
| X1/9 | 2,927 | 162 | 0.0569 | 1,544 | 0.750 | 0.643 |
| X1/27 | 2,519 | 78 | 0.0315 | 725 | 0.339 | 0.324 |
| X1/81 | 1,625 | 19 | 0.0118 | 144 | 0.0928 | 0.0864 |
| X1/243 | 1,865 | 20 | 0.0108 | 78 | 0.0427 | 0.0413 |
| X1/729 | 1,853 | 22 | 0.0120 | 44 | 0.0240 | 0.0247 |
| 0 | 2,168 | 9 | 0.00428 | 20 | 0.00927 | 0.0182 |

FIG. 71A shows plots of the expected value λ (for Simoa, AEB) of each of the standard samples. It is found that the expected value λ obtained through use of CD9/CD63/CD81 and an approximation curve thereof that are obtained from the fluorescence microscope images are satisfactorily coincident with those obtained with Simoa SR-X. In this case, for Simoa, analog correction based on the brightness values of wells is performed in a high AEB region, and hence it is presumed that a higher value is obtained, and it is found that a value obtained for λ or AEB of less than 1 is extremely satisfactorily consistent with that obtained from the fluorescence microscope image. In addition, it is found that the number of extracellular vesicles that can be detected through use of MMP 14 is about one order of magnitude smaller than the number of extracellular vesicles that can be detected through use of CD9/CD63/CD81. BG of the expected value λ in this system can be estimated to be about 0.01, and hence FIG. 71B shows five plots of the expected value at a higher concentration side.

The number of the extracellular vesicles that can be detected by MMP14 activity is changed at a nearly constant ratio with respect to the number of the extracellular vesicles that can be detected through use of CD9/CD63/CD81, and hence it is able to be recognized that this multiplex detection system effectively works in a dynamic range of about two orders of magnitude. In addition, the multiplex detection system enables detection sensitivity about one order of magnitude higher than the detection sensitivity of MMP14 activity with a 96-well plate.

For example, it was able to be recognized that 99.7% of the extracellular vesicles that were able to be detected with MMP14 at a concentration [a.u.] of x1 were present in wells (particles) containing the extracellular vesicles that were able to be detected with at least any one of CD9, CD63, and CD81, which were commonly used biomarkers for extracellular vesicles. It was able to be recognized that this multiplex detection system effectively worked as means for recognizing the colocalization of biomarkers.

The embodiments according to the present invention may be achieved by a method to be executed by a computer of a system or an apparatus, by executing one or a plurality of functions of the above-mentioned embodiments with a computer of a system or apparatus (e.g., an application-specific integrated circuit (ASIC)) configured to read and execute a computer-executable instruction (e.g., one or more programs) recorded on a storage medium, and/or by reading and executing a computer-executable instruction from a storage medium with a computer of a system or apparatus including one or a plurality of circuits configured to execute one or a plurality of functions of the above-mentioned embodiments and for the purpose of, for example, executing one or a plurality of functions of the above-mentioned embodiments, and/or by controlling one or a plurality of circuits to execute one or a plurality of functions of the above-mentioned embodiments. The computer may include one or more processors (e.g., a central processing unit (CPU) or a microprocessor unit (MPU)), and may include a network of individual computers or individual processors to read and execute the computer-executable instruction. The computer-executable instruction may be, for example, provided from the network or the storage medium to the computer. The storage medium may include one or more of, for example, a hard disk, a random access memory (RAM), a read only memory (ROM), a storage apparatus of a distributed computing system, an optical disc (e.g., a compact disc (CD), a digital versatile disc (DVD), or a Blu-ray Disc (BD)), a flash memory device, and a memory card.

Example 34

(Detection of MMP Activity of MDA-MB-231 or A549-derived Extracellular Vesicles with Reporter Molecule: SB2, SB6, SB7, SB9, SB10, or SB14)

4 μL (corresponding to 4 μg for each of the MDA-MB-231 and A549-derived extracellular vesicles) of each of the MDA-MB-231-derived extracellular vesicles (50 μg of protein in 50 μL of PBS) and the A549-derived extracellular vesicles (50 μg of protein in 50 μL of PBS) described in the section "Materials and the like" were aliquoted, and the buffer (4) was added to the aliquot to provide 500 μL of each solution. The concentration of the solution is 8 μg/mL for each of the MDA-MB-231 and A549-derived extracellular vesicles. The concentration of 20 μg/mL was set to x1 in Example 8, and hence the concentration of the solution is x1/2.5 for each of the MDA-MB-231 and A549-derived extracellular vesicles. Further, a sample free of extracellular vesicles was prepared similarly, and used as a sample for BG measurement.
In addition, for detection of MMP activity, 100 μM SB2, SB6, SB7, SB9, SB10, or SB14 of SensoLyte 520 MMP Substrate Sampler Kit (Fluorimetric) AS-71170 was diluted with the buffer (4) to prepare a 10 μM MMP detection solution.
50 μL of the MMP detection solution was added to 50 μL of each of the samples, a reaction was performed at 30°C, and MMP activity was evaluated.
In this case, the concentration of the extracellular vesicles in each of the reaction solutions is 4 μg/mL for each of the MDA-MB-231 and A549-derived extracellular vesicles, and the concentrations of the reporter molecule in the reaction solutions are all 5 μM.

(Evaluation of MMP Activity with 96-well Plate)

Evaluation was performed in the same manner as in Example 1.
Table 19 shows changes in fluorescence intensity after 1 hour from the various reporter molecules by the MDA-MB-231-derived extracellular vesicles. As shown in Table 19, it is found that the fluorescence intensity is changed by the MDA-MB-231-derived extracellular vesicles.

[Table 19]

Table 19

| Reporter molecule | Fluorescence change after 1 hour of reaction at 30°C (ΔRFU) |
|---|---|
| SB2 | 32,088 |
| SB6 | 52,130 |
| SB7 | 39,031 |
| SB9 | 62,330 |
| SB10 | 36,239 |
| SB14 | 6,831 |

Table 20 shows changes in fluorescence intensity after 1 hour from the various reporter molecules by the A549-derived extracellular vesicles. As shown in Table 20, it is found that the fluorescence intensity is changed by the A549-derived extracellular vesicles.

[Table 20]

Table 20

| Reporter molecule | Fluorescence change after 1 hour of reaction at 30°C (ΔRFU) |
|---|---|
| SB2 | 1,154 |
| SB6 | 2,043 |
| SB7 | 1,228 |
| SB9 | 1,775 |
| SB10 | 1,220 |
| SB14 | 801 |

From Table 19 and Table 20, it is found that the change in fluorescence intensity by the MDA-MB-231-derived extracellular vesicles is about 30 times as large as the change in fluorescence intensity by the A549-derived extracellular vesicles (in the case of SB14, slightly less than 10 times).

Example 35

(Wells and Well Array)

A well array of Simoa Discs was used.

(Preparation of Reaction Solution for Wells)

A reaction solution for filling the wells 204 was prepared. 12 μL (corresponding to 12 μg) of the MDA-MB-231-derived extracellular vesicles (50 μg of protein in 50 μL of PBS) described in the section "Materials and the like" were aliquoted, and the buffer (4) was added to the aliquot to provide 1,200 μL of a solution. The concentration of the solution is 10 μg/mL. The concentration of 20 μg/mL was set to x1 in Example 8, and hence the concentration of the solution is x1/2. The solution was further diluted 2-fold to prepare a standard sample at a concentration of x1/4, further diluted 3-fold to prepare a standard sample at a concentration of x1/12, and further diluted 3-fold to prepare a standard sample at a concentration of x1/36. Thus, the standard samples with four concentrations were prepared. Further, a sample free of extracellular vesicles was prepared similarly, and used as a sample for BG measurement.

In addition, for detection of MMP activity, 100 μM SB2, SB6, SB7, SB9, SB10, or SB14 of SensoLyte 520 MMP Substrate Sampler Kit (Fluorimetric) AS-71170 and 1 mM Alexa Fluor 647 serving as a standard fluorescent substance were diluted with the buffer (4) to prepare a MMP detection solution containing 10 μM SB2, SB6, SB7, SB9, SB10, or SB14 and 2 μM Alexa Fluor 647.

50 μL of each of the MMP detection solutions was added to 50 μL of each of the samples to provide a reaction solution for wells.

In this case, the highest concentration of the extracellular vesicles in each of the reaction solutions is 5 μg/mL, and the concentrations of the reporter molecule and the standard fluorescent substance in each of the reaction solutions are 5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from each of SB2, SB6, SB7, SB9, SB10, and SB14 and the background fluorescence of each of SB2, SB6, SB7, SB9, SB10, and SB14 after 6 hours of reaction, and a fluorescence microscope image containing the fluorescence of the standard fluorescent substance after 6 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of each of SB2, SB6, SB7, SB9, SB10 and SB14 by MMP in the MDA-MB-231-derived extracellular vesicles worked in the microcompartments. In this case, the fluorescence of 5-FAM was observed even in the fluorescence microscope image of the wells free of extracellular vesicles, and hence it is found that a pseudopositive result occurred, though its frequency is extremely low.

Next, although the illustration is omitted, the fluorescence microscope images after 6 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected value ($\lambda$) calculated with respect to the concentration of the extracellular vesicles subjected to the test through use of the above-mentioned equation (1) from the total number of wells and the number of positive wells identified from each of the histograms after 6 hours of reaction is shown in Table 21. A power approximation can be satisfactorily applied to plots of the expected value $\lambda$ with respect to the extracellular vesicle concentration. The extracellular vesicle concentration was able to be accurately quantified. Approximation equations and $R^2$ are shown in Table 22. Further, the extracellular vesicle concentration at a concentration of x1 calculated from $\lambda$ at a concentration of x1 and the volume of the microcompartment that is 50 fL is collectively shown in Table 22.

[Table 21]

Table 21

| Fluorescence image | Reporter | Concentration [a.u.] | Number of wells | Number of positives | Positive ratio | Expected value $\lambda$ |
|---|---|---|---|---|---|---|
| Not displayed | SB2 | 1/2 | 28,080 | 20,623 | 0.7344 | 1.326 |
| Not displayed | SB2 | 1/4 | 28,080 | 8,382 | 0.298504 | 0.35454 |
| Not displayed | SB2 | 1/12 | 28,080 | 2,699 | 0.096118 | 0.101057 |
| Not displayed | SB2 | 1/36 | 28,080 | 811 | 0.028882 | 0.029307 |
| Not displayed | SB2 | 0 | 28,080 | 1 | 3.56E-05 | 3.56E-05 |
| Not displayed | SB6 | 1/2 | 28,002 | 17,761 | 0.6343 | 1.006 |
| Not displayed | SB6 | 1/4 | 28,080 | 11,797 | 0.420121 | 0.544936 |
| Not displayed | SB6 | 1/12 | 28,080 | 2,533 | 0.090207 | 0.094538 |
| Not displayed | SB6 | 1/36 | 28,080 | 1,310 | 0.046652 | 0.047776 |
| Not displayed | SB6 | 0 | 28,002 | 1 | 3.57E-05 | 3.57E-05 |
| Not displayed | SB7 | 1/2 | 28,080 | 22,181 | 0.7899 | 1.56 |
| Not displayed | SB7 | 1/4 | 28,080 | 8,663 | 0.308511 | 0.368909 |
| Not displayed | SB7 | 1/12 | 28,080 | 986 | 0.035114 | 0.035745 |
| Not displayed | SB7 | 1/36 | 28,002 | 536 | 0.019141 | 0.019327 |
| Not displayed | SB7 | 0 | 28,080 | 3 | 0.000107 | 0.000107 |
| Not displayed | SB9 | 1/2 | 28,080 | 17,697 | 0.6302 | 0.9949 |
| Not displayed | SB9 | 1/4 | 28,002 | 7,010 | 0.250339 | 0.288135 |

(continued)

| Fluorescence image | Reporter | Concentration [a.u.] | Number of wells | Number of positives | Positive ratio | Expected value λ |
|---|---|---|---|---|---|---|
| Not displayed | SB9 | 1/12 | 28,002 | 442 | 0.015785 | 0.01591 |
| Not displayed | SB9 | 1/36 | 28,080 | 349 | 0.012429 | 0.012507 |
| Not displayed | SB9 | 0 | 28,080 | 1 | 3.56E-05 | 3.56E-05 |
| Not displayed | SB10 | 1/2 | 28,080 | 19,627 | 0.699 | 1.201 |
| Not displayed | SB10 | 1/4 | 28,080 | 11,057 | 0.393768 | 0.500492 |
| Not displayed | SB10 | 1/12 | 28,080 | 3,336 | 0.118803 | 0.126475 |
| Not displayed | SB10 | 1/36 | 28,080 | 1,350 | 0.048077 | 0.049271 |
| Not displayed | SB10 | 0 | 28,080 | 1 | 3.56E-05 | 3.56E-05 |
| Not displayed | SB14 | 1/2 | 28,080 | 3,693 | 0.1315 | 0.141 |
| Not displayed | SB14 | 1/4 | 28,080 | 1,266 | 0.045085 | 0.046133 |
| Not displayed | SB14 | 1/12 | 28,080 | 1,549 | 0.055164 | 0.056744 |
| Not displayed | SB14 | 1/36 | 28,080 | 204 | 0.007265 | 0.007291 |
| Not displayed | SB14 | 0 | 28,080 | 3 | 0.000107 | 0.000107 |

[Table 22]

Table 22

| Reporter | Power approximation equation | $R^2$ | Expected value λ (×1) | Extracellular vesicle concentration (×$10^{10}$/mL) |
|---|---|---|---|---|
| SB2 | $y = 2.6406\times^{1.2839}$ | 0.9866 | 2.6406 | 5.3 |
| SB6 | $y = 2.1589\times^{1.1143}$ | 0.9691 | 2.1589 | 4.3 |
| SB7 | $y = 3.4196\times^{1.5646}$ | 0.9416 | 3.4196 | 6.8 |
| SB9 | $y = 2.3593\times^{1.6207}$ | 0.9033 | 2.3593 | 4.7 |
| SB10 | $y = 2.3991\times^{1.1153}$ | 0.9919 | 2.3991 | 4.8 |
| SB14 | $y = 0.2451\times^{0.8839}$ | 0.8142 | 0.2451 | 0.49 |

Accordingly, it was shown that digital detection of the extracellular vesicles was able to be performed through use of MMP activity in different reporter molecules.

Example 36

(Wells and Well Array)

A well array of Simoa Discs was used.

(Preparation of Reaction Solution for Wells)

A reaction solution for filling the wells 204 was prepared. 4 μL (corresponding to 4 μg) of the A549-derived extracellular vesicles (50 μg of protein in 50 μL of PBS) described in the section "Materials and the like" were aliquoted, and the buffer (4) was added to the aliquot to provide 400 μL of a solution. The concentration of the solution is 10 μg/mL. The concentration of 20 μg/mL was set to x1 in Example 8, and hence the concentration of the solution is x1/2.

In addition, for detection of MMP activity, 100 μM SB2, SB6, SB7, SB9, SB10, or SB14 of SensoLyte 520 MMP Substrate Sampler Kit (Fluorimetric) AS-71170 and 1 mM Alexa Fluor 647 serving as a standard fluorescent substance were diluted with the buffer (4) to prepare a MMP detection solution containing 10 μM SB2, SB6, SB7, SB9, SB10, or SB14 and 2 μM Alexa Fluor 647.

50 μL of each of the MMP detection solutions was added to 50 μL of the sample to provide a reaction solution for wells.

In this case, the concentration of the extracellular vesicles in each of the reaction solutions is 5 μg/mL, and the concentrations of the reporter molecule and the standard fluorescent substance in each of the reaction solutions were 5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from each of SB2, SB6, SB7, SB9, SB10, and SB14 and the background fluorescence of each of SB2, SB6, SB7, SB9, SB10, and SB14 after 4 hours and 24 hours of reaction, and a fluorescence microscope image containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of SB2, SB6, SB7, SB9, SB10, or SB14 by MMP in the A549-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 23. Further, the extracellular vesicle concentration at a concentration of x1 calculated from λ at a concentration of x1 and the volume of the microcompartment that is 50 fL is shown in Table 24.

[Table 23]

Table 23

| Fluorescence image | Reporter | Time (hr) | Number of wells | Number of positives | Positive ratio | Expected value λ |
|---|---|---|---|---|---|---|
| Not displayed | SB2 | 4 | 28,080 | 1,823 | 0.06492 | 0.06712 |
| Not displayed | SB2 | 24 | 28,080 | 112 | 0.00399 | 0.004 |
| Not displayed | SB6 | 4 | 28,080 | 1,911 | 0.06806 | 0.07048 |
| Not displayed | SB6 | 24 | 28,080 | 1,345 | 0.0479 | 0.04908 |
| Not displayed | SB7 | 4 | 28,080 | 395 | 0.01407 | 0.01417 |
| Not displayed | SB7 | 24 | 28,080 | 37 | 0.00132 | 0.00132 |
| Not displayed | SB9 | 4 | 28,080 | 1,594 | 0.05677 | 0.05844 |
| Not displayed | SB9 | 24 | 28,080 | 153 | 0.00545 | 0.00546 |
| Not displayed | SB10 | 4 | 28,080 | 617 | 0.02197 | 0.02222 |

(continued)

| Fluorescence image | Reporter | Time (hr) | Number of wells | Number of positives | Positive ratio | Expected value λ |
|---|---|---|---|---|---|---|
| Not displayed | SB10 | 24 | 28,002 | 59 | 0.00211 | 0.00211 |
| Not displayed | SB14 | 4 | 28,080 | 297 | 0.01058 | 0.01063 |
| Not displayed | SB14 | 24 | 28,080 | 0 | 0 | 0 |

[Table 24]

Table 24

| Reporter | Reaction (hr) | Expected value λ (×1) | Extracellular vesicle concentration (×$10^9$/mL) |
|---|---|---|---|
| SB2 | 4 | 0.13425 | 2.685 |
| SB2 | 24 | 0.00799 | 0.15986 |
| SB6 | 4 | 0.14096 | 2.81928 |
| SB6 | 24 | 0.09817 | 1.96336 |
| SB7 | 4 | 0.02833 | 0.56667 |
| SB7 | 24 | 0.00264 | 0.05274 |
| SB9 | 4 | 0.11688 | 2.33765 |
| SB9 | 24 | 0.01093 | 0.21854 |
| SB10 | 4 | 0.04444 | 0.88872 |
| SB10 | 24 | 0.00422 | 0.08437 |
| SB14 | 4 | 0.02127 | 0.42533 |
| SB14 | 24 | 0 | 0 |

Accordingly, it was shown that digital detection was able to be performed through use of MMP activity in different reporter molecules and extracellular vesicles derived from different cell strains.

Example 37

(Detection of MMP14 Activity of MDA-MB-231-derived Extracellular Vesicles with Reporter Molecule: Custom #11, Custom #12, Custom #13, Custom #14, Custom #15, or Custom #16)

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 400 μL of a solution. The concentration of the MDA-MB-231-derived extracellular vesicles in the solution is 10 μg/mL. The concentration of 20 μg/mL was set to x1 in Example 8, and hence the concentration of the MDA-MB-231-derived extracellular vesicles in the solution is x1/2. Further, a sample free of extracellular vesicles was prepared similarly, and used as a sample for BG measurement.

In addition, for detection of MMP14 activity, 1 mM Custom #11, Custom #12, Custom #13, Custom #14, Custom #15, or Custom #16 was diluted with the buffer (4) to prepare a 10 μM MMP14 detection solution.

50 μL of the MMP14 detection solution was added to 50 μL of each of the samples, a reaction was performed at 30°C, and MMP14 activity was evaluated.

In this case, the concentration of the MDA-MB-231-derived extracellular vesicles in each of the reaction solutions is 5 μg/mL, and the concentrations of the reporter molecule in the reaction solutions are all 5 μM.

(Evaluation of MMP Activity with 96-well Plate)

Evaluation was performed in the same manner as in Example 1.

Table 25 shows changes in fluorescence intensity after 2 hours from the various reporter molecules by the MDA-MB-231-derived extracellular vesicles. As shown in Table 25, it is found that the fluorescence intensity is changed by the MDA-MB-231-derived extracellular vesicles.

[Table 25]

Table 25

| Reporter molecule | Fluorescence change after 2 hours of reaction at 30°C (ΔRFU) |
|---|---|
| Custom #11 | 33,665 |
| Custom #12 | 32,801 |
| Custom #13 | 19,279 |
| Custom #14 | 34,878 |
| Custom #15 | 16,774 |
| Custom #16 | 4,424 |

Example 38

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 800 μL of a solution. The concentration of the solution is 5 μg/mL. The concentration of 20 μg/mL was set to x1 in Example 8, and hence the concentration of the solution is x1/4.

In addition, for detection of MMP14 activity, 1 mM Custom #11, Custom #12, Custom #13, Custom #14, Custom #15, or Custom #16 and 1 mM Alexa Fluor 647 serving as a standard fluorescent substance were diluted with the buffer (4) to prepare a MMP14 detection solution containing 10 μM Custom #11, Custom #12, Custom #13, Custom #14, Custom #15, or Custom #16 and 2 μM Alexa Fluor 647.

50 μL of the MMP14 detection solution was added to 50 μL of the sample to provide a reaction solution for wells.

In this case, the concentration of the extracellular vesicles in the reaction solution is 2.5 μg/mL, and the concentrations of the reporter molecule and the standard fluorescent substance in the reaction solution are 5 μM and 1 μM, respectively.

(Filling of Wells with Reaction Solution)

A fluorinated oil SR-X Sealing Oil was used as a hydrophobic solvent and wells were filled with the reaction solution in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, fluorescence microscope images each containing both of the fluorescence of 5-FAM derived from Custom #11, Custom #12, Custom #13, Custom #14, Custom #15, or Custom #16 and the background fluorescence of Custom #11, Custom #12, Custom #13, Custom #14, Custom #15, or Custom #16 after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #11, Custom #12, Custom #13, Custom #14, Custom #15, or Custom #16 by MMP14 in the MDA-MB-231-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 26. Further, the extracellular vesicle concentration at a concentration of x1 calculated from λ at a concentration of x1 and the volume of the microcompartment that is 50 fL is shown in Table 27.

[Table 26]

Table 26

| Fluorescence image | Reporter | Reaction (hr) | Number of wells | Number of positives | Positive ratio | Expected value λ |
|---|---|---|---|---|---|---|
| Not displayed | Custom #11 | 4 | 28,080 | 3,566 | 0.12699 | 0.13581 |
| Not displayed | Custom #11 | 24 | 28,080 | 7,100 | 0.25285 | 0.29149 |
| Not displayed | Custom #12 | 4 | 28,080 | 3,416 | 0.12165 | 0.12971 |
| Not displayed | Custom #12 | 24 | 28,002 | 7,423 | 0.26509 | 0.308 |
| Not displayed | Custom #13 | 4 | 28,029 | 3,573 | 0.12748 | 0.13636 |
| Not displayed | Custom #13 | 24 | 28,080 | 6,485 | 0.23095 | 0.2626 |
| Not displayed | Custom #14 | 4 | 28,061 | 6,120 | 0.2181 | 0.24602 |
| Not displayed | Custom #14 | 24 | 28,080 | 9,120 | 0.32479 | 0.39273 |
| Not displayed | Custom #15 | 4 | 28,080 | 3,295 | 0.11734 | 0.12482 |
| Not displayed | Custom #15 | 24 | 28,080 | 6,988 | 0.24886 | 0.28616 |
| Not displayed | Custom #16 | 4 | 28,080 | 471 | 0.01677 | 0.01692 |
| Not displayed | Custom #16 | 24 | 28,080 | 2,623 | 0.09341 | 0.09807 |

[Table 27]

Table 27

| Reporter | Reaction (hr) | Expected value λ (×1) | Extracellular vesicle concentration (×$10^{10}$/mL) |
|---|---|---|---|
| Custom #11 | 4 | 0.54325 | 1.08651 |
| Custom #11 | 24 | 1.16595 | 2.3319 |
| Custom #12 | 4 | 0.51885 | 1.0377 |
| Custom #12 | 24 | 1.23202 | 2.46404 |
| Custom #13 | 4 | 0.54546 | 1.09091 |
| Custom #13 | 24 | 1.05038 | 2.10077 |
| Custom #14 | 4 | 0.98409 | 1.96819 |

(continued)

| Reporter | Reaction (hr) | Expected value λ (×1) | Extracellular vesicle concentration (×$10^{10}$/mL) |
|---|---|---|---|
| Custom #14 | 24 | 1.5709 | 3.14181 |
| Custom #15 | 4 | 0.49928 | 0.99855 |
| Custom #15 | 24 | 1.14466 | 2.28931 |
| Custom #16 | 4 | 0.06766 | 0.13533 |
| Custom #16 | 24 | 0.39227 | 0.78453 |

Accordingly, it was shown that digital detection of the extracellular vesicles was able to be performed through use of MMP14 activity in different reporter molecules.

Example 39

(Detection of ADAM Activity of MDA-MB-231-derived Extracellular Vesicles with Reporter Molecule: PEPDAB010 or PEPDAB014, and Detection of Cathepsin D Activity of MDA-MB-231-derived Extracellular Vesicles with Reporter Molecule for Cathepsin D of Cathepsin D Assay Kit, Fluorimetric, SensoLyte 520 AS-72170 (hereinafter abbreviated as "CathD"))

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 600 μL of a solution. The concentration of the MDA-MB-231-derived extracellular vesicles in the solution is 6.7 μg/mL. The concentration of 20 μg/mL was set to x1 in Example 8, and hence the concentration of the MDA-MB-231-derived extracellular vesicles in the solution is x1/3. Further, a sample free of extracellular vesicles was prepared similarly, and used as a sample for BG measurement.
In addition, for detection of ADAM activity, 1 mM EPDAB010 or PEPDAB014 was diluted with the buffer (4) to prepare a 10 μM ADAM detection solution. In addition, for detection of Cathepsin D activity, 1 mM CathD was diluted with the buffer (4) to prepare a 10 μM Cathepsin D detection solution.
50 μL of the ADAM detection solution or the Cathepsin D detection solution was added to 50 μL of each of the samples, a reaction was performed at 30°C, and activity was evaluated.
In this case, the concentration of the extracellular vesicles in each of the reaction solutions is 3.3 μg/mL for the MDA-MB-231-derived extracellular vesicles, and the concentrations of the reporter molecule in the reaction solutions are all 5 μM.

(Evaluation of MMP Activity with 96-well Plate)

Evaluation was performed in the same manner as in Example 1.
Table 28 shows changes in fluorescence intensity after 2 hours from the various reporter molecules by the MDA-MB-231-derived extracellular vesicles. As shown in Table 28, it is found that the fluorescence intensity is changed by the MDA-MB-231-derived extracellular vesicles.

[Table 28]

Table 28

| Reporter molecules | Fluorescence change after 2 hours of reaction at 30°C (ΔRFU) |
|---|---|
| PEPDAB010 | 74,285 |
| PEPDAB014 | 6,939 |
| CathD | 12,868 |

Example 40

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 1,000 μL of a solution. The concentration of the solution is 4 μg/mL. The concentration of 20 μg/mL was set to x1 in Example 8, and hence the concentration of the solution is x1/5.
In addition, for detection of ADAM or Cathepsin D activity, 1 mM PEPDAB010 or PEPDAB014, or 1 mM CathD and 1 mM Alexa Fluor 647 serving as a standard fluorescent substance were diluted with the buffer (4) to prepare an ADAM or Cathepsin D detection solution containing 10 μM PEPDAB010 or PEPDAB014, or 10 μM CathD and 2 μM Alexa Fluor 647.
50 μL of the ADAM or Cathepsin D detection solution was added to 50 μL of the sample to provide a reaction solution for wells.
In this case, the concentration of the extracellular vesicles in each of the reaction solutions is 2 μg/mL, and the concentration of each of the reporter molecules and the standard fluorescent substance in each of the reaction solutions are 5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).
The observation of fluorescence derived from each fluorescent substance was performed under the same conditions as in Example 19 to acquire a fluorescence image.
Although the illustration is omitted, fluorescence microscope images each containing both of the fluorescence of 5-FAM derived from PEPDAB010, PEPDAB014, or CathD, or HiLyte Fluor 488 and the background fluorescence of PEPDAB010, PEPDAB014, or CathD after 4 hours and 24 hours of reaction, and fluorescence microscope images each containing the fluorescence of the standard fluorescent substance after 4 hours and 24 hours of reaction were acquired.
The wells were able to be recognized based on the fluorescence of the standard fluorescent substance. In addition, it was found that, in the wells in which the fluorescence of 5-FAM or HiLyte Fluor 488 was observed, the fluorescence of the standard fluorescent substance was also observed. Thus, it was able to be recognized that the wells were filled with the sample and the reagent containing the reporter molecule, and a cleavage reaction of PEPDAB010 or PEPDAB014 by ADAM in the MDA-MB-231-derived extracellular vesicles worked in the microcompartments, and a cleavage reaction of CathD by Cathepsin D in the MDA-MB-231-derived extracellular vesicles worked in the microcompartments.
Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.
The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 29. Further, the extracellular vesicle concentration at a concentration of x1 calculated from λ at a concentration of x1 and the volume of the microcompartment that is 50 fL is shown in Table 30.

[Table 29]

Table 29

| Fluorescence image | Reporter | Reaction (hr) | Number of wells | Number of positives | Positive ratio | Expected value λ |
|---|---|---|---|---|---|---|
| Not displayed | PEPDAB010 | 4 | 28,080 | 2,664 | 0.09487 | 0.09968 |
| Not displayed | PEPDAB010 | 24 | 28,080 | 4,394 | 0.15648 | 0.17017 |
| Not displayed | PEPDAB014 | 4 | 28,080 | 1,536 | 0.0547 | 0.05625 |
| Not displayed | PEPDAB014 | 24 | 28,080 | 2,204 | 0.07849 | 0.08174 |
| Not displayed | CathD | 4 | 28,002 | 4,013 | 0.14331 | 0.15468 |
| Not displayed | CathD | 24 | 28,080 | 6,306 | 0.22457 | 0.25434 |

[Table 30]

Table 30

| Reporter | Reaction (hr) | Expected value λ (×1) | Extracellular vesicle concentration (×$10^{10}$/mL) |
|---|---|---|---|
| PEPDAB010 | 4 | 0.49839341 | 0.99679 |
| PEPDAB010 | 24 | 0.850867114 | 1.70173 |
| PEPDAB014 | 4 | 0.281269229 | 0.56254 |
| PEPDAB014 | 24 | 0.408708405 | 0.81742 |
| CathD | 4 | 0.773402721 | 1.54681 |
| CathD | 24 | 1.271704908 | 2.54341 |

Accordingly, it was shown that digital detection of the extracellular vesicles was able to be performed through use of ADAM activity or Cathepsin D activity in different reporter molecules.

Example 41

(Detection of MMP14 Activity of MDA-MB-231-derived Extracellular Vesicles with Reporter Molecule: Custom #2T or Custom #2TF3)

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 400 μL of a solution. The concentration of the MDA-MB-231-derived extracellular vesicles in the solution was 10 μg/mL. The concentration of 20 μg/mL was set to x1 in Example 8, and hence the concentration of the MDA-MB-231-derived extracellular vesicles in the solution is x1/2. Further, a sample free of extracellular vesicles was prepared similarly, and used as a sample for BG measurement.

In addition, for detection of MMP14 activity, 1 mM Custom #2T or Custom #2TF3 was diluted with the buffer (4) to prepare a 10 μM MMP14 detection solution.

50 μL of the MMP14 detection solution was added to 50 μL of each of the samples, a reaction was performed at 30°C, and MMP14 activity was evaluated.

In this case, the concentration of the extracellular vesicles in each of the reaction solutions is 5 μg/mL for the MDA-MB-231-derived extracellular vesicles, and the concentrations of the reporter molecule in the reaction solutions are all 5 μM.

(Evaluation of MMP Activity with 96-well Plate)

The MMP14 activity of the extracellular vesicles was evaluated with a 96-well plate (137101). A fluorescence intensity was measured with a fluorescence plate reader (Synergy) at 30°C every 5 minutes for 2 hours. For measurement wavelengths, an excitation wavelength of 545±20 nm and a fluorescence wavelength of 588±20 nm were used. A relative fluorescence unit difference (ΔRFU) was calculated by subtracting the measured value of the sample for BG measurement from the measured value of each of the samples.

Table 31 shows changes in fluorescence intensity after 2 hours from the various reporter molecules by the MDA-MB-231-derived extracellular vesicles. As shown in Table 31, it is found that the fluorescence intensity is changed by the MDA-MB-231-derived extracellular vesicles.

[Table 31]

Table 31

| Reporter molecule | Fluorescence change after 2 hours of reaction at 30°C (ΔRFU) |
|---|---|
| Custom #2T | 6,455 |
| Custom #2TF3 | 3,791 |

Example 42

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 800 μL of a solution. The concentration of the solution is 5 μg/mL. The concentration of 20 μg/mL was set to x1 in Example 8, and hence the concentration of the solution is x1/4.

In addition, for detection of MMP14 activity, 1 mM Custom #2T or Custom #2TF3 and 1 mM Alexa Fluor 647 serving as a standard fluorescent substance were diluted with the buffer (4) to prepare a MMP14 detection solution containing 10 μM Custom #2T or Custom #2TF3 and 2 μM Alexa Fluor 647.

50 μL of the MMP14 detection solution was added to 50 μL of the sample to provide a reaction solution for wells.

In this case, the concentration of the extracellular vesicles in the reaction solution is 2.5 μg/mL, and the concentrations of the reporter molecule and the standard fluorescent substance in the reaction solution are 5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 except that a filter was changed to the following filter, to acquire a fluorescence image.

TRITC: OP-87764 (manufactured by Keyence Corporation)
Model: OP-87764
Excitation wavelength: 545/25 nm
Absorption wavelength: 605/70 nm
Dichroic wavelength: 565 nm

Although the illustration is omitted, fluorescence microscope images each containing both of the fluorescence of TAMRA or TF3 derived from Custom #2T or Custom #2TF3 and the background fluorescence of Custom #2T or Custom #2TF3 after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of TAMRA or TF3 was observed, the fluorescence of the standard fluorescent substance was also observed. Thus, it was recognized that the wells were filled with the sample and the reagent containing the reporter molecule, and a cleavage reaction of Custom #2T or Custom #2TF3 by MMP14 in the MDA-MB-231-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 32. Further, the extracellular vesicle concentration at a concentration of x1 calculated from λ at a concentration of x1 and the volume of the microcompartment that is 50 fL is shown in Table 33.

[Table 32]

Table 32

| Fluorescence image | Reporter | Reaction (hr) | Number of wells | Number of positives | Positive ratio | Expected value λ |
|---|---|---|---|---|---|---|
| Not displayed | Custom #2T | 4 | 28,080 | 3,313 | 0.117984 | 0.125545 |
| Not displayed | Custom #2T | 24 | 28,080 | 3,518 | 0.125285 | 0.133857 |
| Not displayed | Custom #2TF3 | 4 | 28,080 | 3,746 | 0.1334 | 0.14318 |
| Not displayed | Custom #2TF3 | 24 | 28,080 | 3,783 | 0.13472 | 0.1447 |

[Table 33]

Table 33

| Reporter | Reaction (hr) | Expected value λ (×1) | Extracellular vesicle concentration |
|---|---|---|---|
| | | | (×$10^{10}$/mL) |
| Custom #2T | 4 | 0.50218 | 1.0044 |
| Custom #2T | 24 | 0.53543 | 1.0709 |
| Custom #2TF3 | 4 | 0.57272 | 1.1454 |
| Custom #2TF3 | 24 | 0.5788 | 1.1576 |

Accordingly, it was shown that digital detection of the extracellular vesicles was able to be performed through use of MMP14 activity even in reporter molecules in which the dye species in the FRET pair were different.

Example 43

(Multiplex (hereinafter abbreviated as "MP") Detection of MMP14 Activity of MDA-MB-231-derived Extracellular Vesicles with Reporter Molecules: Custom #1 and Custom #2T, and Custom #2 and Custom #2T)

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 600 μL of a solution. The concentration of the MDA-MB-231-derived extracellular vesicles in the solution is 6.7 μg/mL. The concentration of 20 μg/mL was set to x1 in Example 8, and hence the concentration of the MDA-MB-231-derived extracellular vesicles in the solution is x1/3. Further, a sample free of extracellular vesicles was prepared similarly, and used as a sample for BG measurement.

In addition, for detection of MMP14 activity, Custom #1 and Custom #2T, and Custom #2 and Custom #2T were each diluted with the buffer (4) to prepare four kinds of MMP14 detection solutions in which Custom #1 and Custom #2T were each mixed at 5 μM or 2.5 μM, and Custom #2 and Custom #2T were each mixed at 5 μM or 2.5 μM.

50 μL of each of the MMP14 detection solutions was added to 50 μL of each of the samples, a reaction was performed at 30°C, and MMP14 activity was evaluated.

In this case, the concentration of the extracellular vesicles in each of the reaction solutions is 3.3 μg/mL for the MDA-MB-231-derived extracellular vesicles, and the concentrations of the respective reporter molecules in each of the reaction solutions are a combination of 2.5 μM and 2.5 μM or a combination of 1.25 μM and 1.25 μM.

(Evaluation of MMP Activity with 96-well Plate)

With regard to Custom #1 and Custom #2, evaluation was performed in the same manner as in Example 1. In addition, with regard to Custom #2T, evaluation was performed in the same manner as in Example 41.

Table 31 shows changes in fluorescence intensity after 2 hours from the various reporter molecules by the MDA-MB-231-derived extracellular vesicles. As shown in Table 31, it is found that the fluorescence intensity is changed by the MDA-MB-231-derived extracellular vesicles.

[Table 34]

Table 34

| Reporter molecules | Fluorescence change after 2 hours of reaction at 30°C (ΔRFU) | |
|---|---|---|
| MP | Custom #1 or Custom #2 | Custom #2T |
| 2.5 μM #1 2.5 μM #2T | 44,030 | 1,118 |
| 2.5 μM #2 2.5 μM #2T | 9,620 | 2,196 |
| 1.25 μM #1 1.25 μM #2T | 34,477 | 1,015 |
| 1.25 μM #2 1.25 μM #2T | 9,235 | 1,734 |

Example 44

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 600 μL of a solution. The concentration of the solution is 6.7 μg/mL. The concentration of 20 μg/mL was set to x1 in Example 8, and hence the concentration of the solution is x1/3.

In addition, for detection of MMP14 activity, Custom #1 and Custom #2T, and Custom #2 and Custom #2T were each diluted with the buffer (4) to prepare four kinds of MMP14 detection solutions in which Custom #1 and Custom #2T were each mixed at 5 μM or 2.5 μM, and Custom #2 and Custom #2T were each mixed at 5 μM or 2.5 μM. In addition, 1 mM Alexa Fluor 647 was used as a standard fluorescent substance, and each of the MMP14 detection solutions contained the standard fluorescent substance at a final concentration of 2 μM.

50 μL of each of the MMP14 detection solutions was added to 50 μL of the sample to provide a reaction solution for wells.

In this case, the concentration of the extracellular vesicles in each of the reaction solutions is 3.3 μg/mL, and the concentrations of the respective reporter molecules in each of the reaction solutions are a combination of 2.5 μM and 2.5 μM or a combination of 1.25 μM and 1.25 μM. In addition, the concentrations of the standard fluorescent substance in the reaction solutions are all 1 μM.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 for Custom #1 and Custom #2, and under the same conditions as in Example 42 for Custom #2T, to acquire fluorescence images.

Although the illustration is omitted, fluorescence microscope images each containing both of the fluorescence of 5-FAM derived from Custom #1 or Custom #2, the fluorescence of TAMRA derived from Custom #2T, and the background fluorescence of Custom #1 or Custom #2 and Custom #2T after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM and the fluorescence of TAMRA were observed, the fluorescence of the standard fluorescent substance was also observed. Thus, it was able to be recognized that the wells were filled with the sample and the reagent containing the reporter molecules, and cleavage reactions in the same reaction solution of Custom #1 or Custom #2 and Custom #2T by MMP14 in the MDA-MB-231-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 35. In addition, the ratio of double-positives with respect to all the positives (double-positive ratio 1), the ratio of double-positives with respect to the positives for Custom #1 or Custom #2 (double-positive ratio 2), and the ratio of double-positives with respect to the positives for Custom #2T (double-positive ratio 3) are shown in Table 36. Further, the extracellular vesicle concentration at a concentration of x1 calculated from λ at a concentration of x1 and the volume of the microcompartment that is 50 fL is shown in Table 37.

[Table 35]

Table 35

| Combination | Reporter | Reaction (hr) | Number of wells | Number of positives | Positive ratio | Expected value λ |
|---|---|---|---|---|---|---|
| 1 | 2.5 μM #1 | 4 | 28,078 | 3,151 | 0.11222 | 0.11903 |
| | 2.5 μM #1 | 24 | 28,364 | 7,953 | 0.28039 | 0.32905 |
| | 2.5 μM #2T | 4 | 28,078 | 1,438 | 0.05121 | 0.05257 |
| | 2.5 μM #2T | 24 | 28,364 | 3,642 | 0.1284 | 0.13743 |
| 2 | 2.5 μM #2 | 4 | 28,373 | 4,216 | 0.14859 | 0.16086 |
| | 2.5 μM #2 | 24 | 28,445 | 4,195 | 0.14748 | 0.15956 |
| | 2.5 μM #2T | 4 | 28,373 | 4,292 | 0.15127 | 0.16401 |
| | 2.5 μM #2T | 24 | 28,445 | 4,140 | 0.14554 | 0.15729 |

(continued)

| Combination | Reporter | Reaction (hr) | Number of wells | Number of positives | Positive ratio | Expected value λ |
|---|---|---|---|---|---|---|
| 3 | 1.25 μM #1 | 4 | 28,589 | 10,538 | 0.3686 | 0.45982 |
| | 1.25 μM #1 | 24 | 28,503 | 10,933 | 0.38357 | 0.48382 |
| | 1.25 μM #2T | 4 | 28,589 | 4,904 | 0.17153 | 0.18818 |
| | 1.25 μM #2T | 24 | 28,503 | 5,694 | 0.19977 | 0.22285 |
| 4 | 1.25 μM #2 | 4 | 29,188 | 5,159 | 0.17675 | 0.1945 |
| | 1.25 μM #2 | 24 | 28,913 | 5,601 | 0.19372 | 0.21532 |
| | 1.25 μM #2T | 4 | 29,188 | 5,152 | 0.17651 | 0.1942 |
| | 1.25 μM #2T | 24 | 28,913 | 6,275 | 0.21703 | 0.24466 |

[Table 36]

Table 36

| Combination | Reporter | Reaction (hr) | Double-positive ratio 1 | Double-positive ratio 2 | Double-positive ratio 3 |
|---|---|---|---|---|---|
| 1 | 2.5 μM #1 | 4 | 0.06129 | 0.0841 | 0.18428 |
| | 2.5 μM #2T | 24 | 0.12311 | 0.15981 | 0.34898 |
| 2 | 2.5 μM #2 | 4 | 0.75785 | 0.87002 | 0.85461 |
| | 2.5 μM #2T | 24 | 0.9021 | 0.94231 | 0.95483 |
| 3 | 1.25 μM #1 | 4 | 0.16905 | 0.2119 | 0.45534 |
| | 1.25 μM #2T | 24 | 0.21694 | 0.27111 | 0.52055 |
| 4 | 1.25 μM #2 | 4 | 0.8826 | 0.937 | 0.93828 |
| | 1.25 μM #2T | 24 | 0.7926 | 0.93751 | 0.83681 |

[Table 37]

Table 37

| Combination | Reporter | Reaction (hr) | Expected value λ (×1) | Extracellular vesicle concentration (×$10^{10}$/mL) |
|---|---|---|---|---|
| 1 | 2.5 μM #1 | 4 | 0.3571 | 0.71421 |
| | 2.5 μM #1 | 24 | 0.98714 | 1.97428 |
| | 2.5 μM #2T | 4 | 0.15772 | 0.31544 |
| | 2.5 μM #2T | 24 | 0.41228 | 0.82456 |
| 2 | 2.5 μM #2 | 4 | 0.48259 | 0.96518 |
| | 2.5 μM #2 | 24 | 0.47867 | 0.95733 |
| | 2.5 μM #2T | 4 | 0.49204 | 0.98409 |
| | 2.5 μM #2T | 24 | 0.47187 | 0.94374 |

(continued)

| Combination | Reporter | Reaction (hr) | Expected value λ (×1) | Extracellular vesicle concentration (×$10^{10}$/mL) |
|---|---|---|---|---|
| 3 | 1.25 μM #1 | 4 | 1.37946 | 2.75893 |
| | 1.25 μM #1 | 24 | 1.45145 | 2.9029 |
| | 1.25 μM #2T | 4 | 0.56454 | 1.12908 |
| | 1.25 μM #2T | 24 | 0.66856 | 1.33712 |
| 4 | 1.25 μM #2 | 4 | 0.58349 | 1.16698 |
| | 1.25 μM #2 | 24 | 0.64597 | 1.29194 |
| | 1.25 μM #2T | 4 | 0.58261 | 1.16523 |
| | 1.25 μM #2T | 24 | 0.73398 | 1.46797 |

Accordingly, it was shown that multiplex-digital detection of the extracellular vesicles was able to be performed through use of MMP14 activity even when different reporter molecules were contained in the same reaction solution.

Example 45

(MP Detection of MMP14 Activity of MDA-MB-231-derived Extracellular Vesicles with Reporter Molecules: Custom #11, Custom #12, Custom #13, Custom #14, Custom #15, or Custom #16 and Custom #2TF3)

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 600 μL of a solution. The concentration of the MDA-MB-231-derived extracellular vesicles in the solution is 6.7 μg/mL. The concentration of 20 μg/mL was set to x1 in Example 8, and hence the concentration of the MDA-MB-231-derived extracellular vesicles in the solution is x1/3. Further, a sample free of extracellular vesicles was prepared similarly, and used as a sample for BG measurement.

In addition, for detection of MMP14 activity, 1 mM Custom #11, Custom #12, Custom #13, Custom #14, Custom #15, or Custom #16 and 1 mM Custom #2TF3 were diluted with the buffer (4) to prepare 12 kinds of MMP14 detection solutions in which Custom #11, Custom #12, Custom #13, Custom #14, Custom #15, or Custom #16 and Custom #2TF3 were each mixed at 5 μM, and Custom #11, Custom #12, Custom #13, Custom #14, Custom #15, or Custom #16 and Custom #2TF3 were mixed at 2.5 μM and 10 μM, respectively.

50 μL of each of the MMP14 detection solutions was added to 50 μL of each of the samples, a reaction was performed at 30°C, and MMP14 activity was evaluated.

In this case, the concentration of the extracellular vesicles in each of the reaction solutions is 3.3 μg/mL for the MDA-MB-231-derived extracellular vesicles, and the concentrations of the respective reporter molecules in each of the reaction solutions are a combination of 2.5 μM and 2.5 μM, or a combination of 1.25 μM for Custom #11, Custom #12, Custom #13, Custom #14, Custom #15, or Custom #16 and 5 μM for Custom #2TF3.

(Evaluation of MMP Activity with 96-well Plate)

With regard to Custom #11, Custom #12, Custom #13, Custom #14, Custom #15, and Custom #16, evaluation was performed in the same manner as in Example 1. In addition, with regard to Custom #2TF3, evaluation was performed in the same manner as in Example 41.

Table 38 shows changes in fluorescence intensity after 2 hours from the various reporter molecules by the MDA-MB-231-derived extracellular vesicles. As shown in Table 38, it is found that the fluorescence intensity is changed by the MDA-MB-231-derived extracellular vesicles.

[Table 38]

Table 38

| Reporter molecules | | Fluorescence change after 2 hours of reaction at 30°C (ΔRFU) | |
|---|---|---|---|
| MP | | Custom #11 to Custom #16 | Custom #2TF3 |
| 2.5 μM Custom #11 | 2.5 μM Custom #2TF3 | 18,065 | 313 |
| 2.5 μM Custom #12 | | 19,102 | 319 |
| 2.5 μM Custom #13 | | 9,939 | 452 |
| 2.5 μM Custom #14 | | 14,104 | 397 |
| 2.5 μM Custom #15 | | 10,890 | 551 |
| 2.5 μM Custom #16 | | 1,489 | 1,085 |
| 1.25 μM Custom #11 | 5 μM Custom #2TF3 | 12,156 | 772 |
| 1.25 μM Custom #12 | | 13,343 | 618 |
| 1.25 μM Custom #13 | | 7,073 | 814 |
| 1.25 μM Custom #14 | | 9,504 | 714 |
| 1.25 μM Custom #15 | | 9,131 | 1,023 |
| 1.25 μM Custom #16 | | 852 | 1,857 |

Example 46

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 600 μL of a solution. The concentration of the solution is 6.7 μg/mL. The concentration of 20 μg/mL was set to x1 in Example 8, and hence the concentration of the solution is x1/3.

In addition, for detection of MMP14 activity, 1 mM Custom #11, Custom #12, Custom #13, Custom #14, Custom #15, or Custom #16 and 1 mM Custom #2TF3 were diluted with the buffer (4) to prepare 12 kinds of MMP14 detection solutions in which Custom #11, Custom #12, Custom #13, Custom #14, Custom #15, or Custom #16 and Custom #2TF3 were each mixed at 5 μM, and Custom #11, Custom #12, Custom #13, Custom #14, Custom #15, or Custom #16 and Custom #2TF3 were mixed at 2.5 μM and 10 μM, respectively. In addition, 1 mM Alexa Fluor 647 was used as a standard fluorescent substance, and each of the MMP14 detection solutions contained the standard fluorescent substance at a final concentration of 2 μM.

50 μL of each of the MMP14 detection solutions was added to 50 μL of the sample to provide a reaction solution for wells.

In this case, the concentration of the extracellular vesicles in each of the reaction solutions is 3.3 μg/mL, and the concentrations of the respective reporter molecules in each of the reaction solutions are a combination of 2.5 μM and 2.5 μM, or a combination of 1.25 μM for Custom #11, Custom #12, Custom #13, Custom #14, Custom #15, or Custom #16 and 5 μM for Custom #2TF3. In addition, the concentrations of the standard fluorescent substance in the reaction solutions are all 1 μM.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 for Custom #11, Custom #12, Custom #13, Custom #14, Custom #15, and Custom #16, and under the same conditions as in Example 42 for Custom #2TF3, to acquire fluorescence images.

Although the illustration is omitted, fluorescence microscope images each containing all of the fluorescence of 5-FAM derived from any one of Custom #11, Custom #12, Custom #13, Custom #14, Custom #15, and Custom #16, the fluorescence of TF3 derived from Custom #2TF3, and the background fluorescence of any one of Custom #11, Custom #12, Custom #13, Custom #14, Custom #15, and Custom #16 and Custom #2TF3 after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM and the fluorescence of TF3 were observed, the fluorescence of the standard fluorescent substance was also observed. Thus, it was able to be recognized that the wells were filled with the sample and the reagent containing the reporter molecules, and cleavage reactions in the same reaction solution of any one of Custom #11, Custom #12, Custom #13, Custom #14, Custom #15, and Custom #16 and Custom #2TF3 by MMP14 in the MDA-MB-231-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) after 24 hours of reaction calculated with respect to the concentration of the extracellular vesicles subjected to the test through use of the above-mentioned equation (1) from the total number of wells and the number of positive wells identified from each of the histograms after 4 hours and 24 hours of reaction are shown in Table 39 and Table 40. Table 39 shows the expected value (λ) in any one of Custom #11, Custom #12, Custom #13, Custom #14, Custom #15, and Custom #16 when any one of Custom #11, Custom #12, Custom #13, Custom #14, Custom #15, and Custom #16, and Custom #2TF3 were subjected to the reaction in the same reaction solution, and Table 40 shows the expected value (λ) in Custom #2TF3 when any one of Custom #11, Custom #12, Custom #13, Custom #14, Custom #15, and Custom #16, and Custom #2TF3 were subjected to the reaction in the same reaction solution. In addition, the ratio of double-positives with respect to all the positives (double-positive ratio 1), the ratio of double-positives with respect to the positives for any one of Custom #11, Custom #12, Custom #13, Custom #14, Custom #15, and Custom #16 (double-positive ratio 2), and the ratio of double-positives with respect to the positives for Custom #2TF3 (double-positive ratio 3) are shown in Table 41. Further, the extracellular vesicle concentration at a concentration of x1 calculated from λ at a concentration of x1 and the volume of the microcompartment that is 50 fL is shown in Table 42 and Table 43.

[Table 39]

Table 39

| Results of #11 to #16 | | | | | | |
|---|---|---|---|---|---|---|
| Reporter molecules | | Reaction (hr) | Number of wells | Number of positives | Positive ratio | Expected value λ |
| 2.5 μM #11 | 2.5 μM #2TF3 | 24 | 28,236 | 8,099 | 0.28683 | 0.33804 |
| 2.5 μM #12 | | 24 | 28,278 | 11,898 | 0.42075 | 0.54602 |
| 2.5 μM #13 | | 24 | 28,385 | 10,495 | 0.36974 | 0.46162 |
| 2.5 μM #14 | | 24 | 28,195 | 12,150 | 0.43093 | 0.56375 |
| 2.5 μM #15 | | 24 | 28,270 | 9,694 | 0.34291 | 0.41993 |
| 2.5 μM #16 | | 24 | 28,232 | 4,025 | 0.14257 | 0.15381 |
| 1.25 μM #11 | 5 μM #2TF3 | 24 | 28,330 | 8,198 | 0.28938 | 0.34161 |
| 1.25 μM #12 | | 24 | 28,280 | 12,040 | 0.42574 | 0.55468 |

(continued)

| Results of #11 to #16 | | | | | | |
|---|---|---|---|---|---|---|
| Reporter molecules | | Reaction (hr) | Number of wells | Number of positives | Positive ratio | Expected value λ |
| 1.25 μM #13 | | 24 | 28,360 | 11,078 | 0.39062 | 0.49531 |
| 1.25 μM #14 | | 24 | 28,159 | 12,311 | 0.4372 | 0.57482 |
| 1.25 μM #15 | | 24 | 28,242 | 10,131 | 0.35872 | 0.44429 |
| 1.25 μM #16 | | 24 | 28,343 | 15,76 | 0.0556 | 0.05721 |

[Table 40]

Table 40

| Results of #2TF3 | | | | | | |
|---|---|---|---|---|---|---|
| Reporter molecules | | Reaction (hr) | Number of wells | Number of positives | Positive ratio | Expected value λ |
| 2.5 μM #11 | 2.5 μM #2TF3 | 24 | 28,236 | 5,424 | 0.1921 | 0.21331 |
| 2.5 μM #12 | | 24 | 28,278 | 6,916 | 0.24457 | 0.28047 |
| 2.5 μM #13 | | 24 | 28,385 | 6,815 | 0.24009 | 0.27456 |
| 2.5 μM #14 | | 24 | 28,195 | 7,302 | 0.25898 | 0.29973 |
| 2.5 μM #15 | | 24 | 28,270 | 7,234 | 0.25588 | 0.29557 |
| 2.5 μM #16 | | 24 | 28,232 | 6,470 | 0.22917 | 0.26029 |
| 1.25 μM #11 | 5 μM #2TF3 | 24 | 28,330 | 6,672 | 0.23551 | 0.26855 |
| 1.25 μM #12 | | 24 | 28,280 | 7,341 | 0.25958 | 0.30054 |
| 1.25 μM #13 | | 24 | 28,360 | 6,598 | 0.23265 | 0.26481 |
| 1.25 μM #14 | | 24 | 28,159 | 6,320 | 0.22444 | 0.25417 |
| 1.25 μM #15 | | 24 | 28,242 | 6,878 | 0.24354 | 0.2791 |
| 1.25 μM #16 | | 24 | 28,343 | 6,443 | 0.22732 | 0.25789 |

[Table 41]

Table 41

| Reporter molecules | | Reaction (hr) | Double-positive ratio 1 | Double-positive ratio 2 | Double-positive ratio 3 |
|---|---|---|---|---|---|
| 2.5 μM #11 | 2.5 μM #2TF3 | 24 | 0.193030437 | 0.270156809 | 0.40339233 |
| 2.5 μM #12 | | 24 | 0.26344772 | 0.329719281 | 0.567235396 |
| 2.5 μM #13 | | 24 | 0.213629671 | 0.290328728 | 0.447101981 |
| 2.5 μM #14 | | 24 | 0.269381363 | 0.339753086 | 0.565324569 |
| 2.5 μM #15 | | 24 | 0.209574848 | 0.302558283 | 0.405446503 |
| 2.5 μM #16 | | 24 | 0.137916079 | 0.316024845 | 0.196599691 |

(continued)

| Reporter molecules | | Reaction (hr) | Double-positive ratio 1 | Double-positive ratio 2 | Double-positive ratio 3 |
|---|---|---|---|---|---|
| 1.25 μM #11 | 5 μM #2TF3 | 24 | 0.227100182 | 0.335691632 | 0.412470024 |
| 1.25 μM #12 | | 24 | 0.269137581 | 0.341362126 | 0.559869228 |
| 1.25 μM #13 | | 24 | 0.213927615 | 0.28118794 | 0.472112761 |
| 1.25 μM #14 | | 24 | 0.231313198 | 0.284298595 | 0.553797468 |
| 1.25 μM #15 | | 24 | 0.224021301 | 0.307274701 | 0.452602501 |
| 1.25 μM #16 | | 24 | 0.084674692 | 0.397208122 | 0.097159708 |

[Table 42]

Table 42

| Results of #11 to #16 | | | | |
|---|---|---|---|---|
| Reporter molecules | | Reaction (hr) | Expected value λ (×1) | Extracellular vesicle concentration (×$10^{10}$/mL) |
| 2.5 μM #11 | 2.5 μM #2TF3 | 24 | 1.01412 | 2.028233 |
| 2.5 μM #12 | | 24 | 1.63807 | 3.2761382 |
| 2.5 μM #13 | | 24 | 1.38486 | 2.7697136 |
| 2.5 μM #14 | | 24 | 1.69124 | 3.3824843 |
| 2.5 μM #15 | | 24 | 1.25979 | 2.5195845 |
| 2.5 μM #16 | | 24 | 0.46144 | 0.9228854 |
| 1.25 μM #11 | 5 μM #2TF3 | 24 | 1.02483 | 2.0496643 |
| 1.25 μM #12 | | 24 | 1.66403 | 3.328065 |
| 1.25 μM #13 | | 24 | 1.48594 | 2.9718852 |
| 1.25 μM #14 | | 24 | 1.72447 | 3.4489423 |
| 1.25 μM #15 | | 24 | 1.33287 | 2.6657445 |
| 1.25 μM #16 | | 24 | 0.17163 | 0.3432618 |

[Table 43]

Table 43

| Results of #2TF3 | | | | |
|---|---|---|---|---|
| Reporter molecules | | Reaction (hr) | Expected value λ (×1) | Extracellular vesicle concentration ($\times 10^{10}$/mL) |
| 2.5 μM #11 | 2.5 μM #2TF3 | 24 | 0.639933139 | 1.279866277 |
| 2.5 μM #12 | | 24 | 0.841411424 | 1.682822847 |
| 2.5 μM #13 | | 24 | 0.823672129 | 1.647344257 |
| 2.5 μM #14 | | 24 | 0.899191448 | 1.798382896 |
| 2.5 μM #15 | | 24 | 0.886697748 | 1.773395496 |
| 2.5 μM #16 | | 24 | 0.780872271 | 1.561744541 |
| 1.25 μM #11 | 5 μM #2TF3 | 24 | 0.805639238 | 1.611278476 |
| 1.25 μM #12 | | 24 | 0.901624177 | 1.803248353 |
| 1.25 μM #13 | | 24 | 0.794443116 | 1.588886233 |
| 1.25 μM #14 | | 24 | 0.762508971 | 1.525017942 |
| 1.25 μM #15 | | 24 | 0.837308908 | 1.674617815 |
| 1.25 μM #16 | | 24 | 0.773680348 | 1.547360696 |

Accordingly, it was shown that multiplex-digital detection of the extracellular vesicles was able to be performed through use of MMP14 activity even when different reporter molecules were contained in the same reaction solution.

Example 47

(MP Detection of MMP Activity of MDA-MB-231-derived Extracellular Vesicles with Reporter Molecules: SB2, SB6, SB7, SB9, or SB10 and Custom #2T)

4 μL of the MDA-MB-231-derived extracellular vesicles (50 μg of protein in 50 μL of PBS) (corresponding to 4 μg of the MDA-MB-231-derived extracellular vesicles) described in the section "Materials and the like" were aliquoted, and the buffer (4) was added to the aliquot to provide 600 μL of a solution. The concentration of the MDA-MB-231-derived extracellular vesicles in the solution is 6.7 μg/mL. The concentration of 20 μg/mL was set to x1 in Example 8, and hence the concentration of the MDA-MB-231-derived extracellular vesicles in the solution is x1/3. Further, a sample free of extracellular vesicles was prepared similarly, and used as a sample for BG measurement.
In addition, for detection of MMP activity, 1 mM SB2, SB6, SB7, SB9, or SB10 and 1 mM Custom #2T were diluted with the buffer (4) to prepare 10 kinds of MMP detection solutions in which any one of SB2, SB6, SB7, SB9, and SB10 and Custom #2T were each mixed at 10 μM or 2.5 μM.
50 μL of each of the MMP detection solutions was added to 50 μL of each of the samples, a reaction was performed at 30°C, and MMP activity was evaluated.
In this case, the concentration of the extracellular vesicles in each of the reaction solutions is 3.3 μg/mL for the MDA-MB-231-derived extracellular vesicles, and the concentrations of the respective reporter molecules in each of the reaction solutions are a combination of 5 μM and 5 μM or a combination of 1.25 μM and 1.25 μM.

(Evaluation of MMP Activity with 96-well Plate)

With regard to SB2, SB6, SB7, SB9, and SB10, evaluation was performed in the same manner as in Example 1. In addition, with regard to Custom #2T, evaluation was performed in the same manner as in Example 41.
Table 44 shows changes in fluorescence intensity after 2 hours from the various reporter molecules by the MDA-MB-231-derived extracellular vesicles. As shown in Table 44, it is found that the fluorescence intensity is changed by the MDA-MB-231-derived extracellular vesicles.

[Table 44]

Table 44

| Reporter molecules | | Fluorescence change after 2 hours of reaction at 30°C (ΔRFU) | |
|---|---|---|---|
| MP | | SB2 to SB10 | Custom #2T |
| 5 μM SB2 | 5 μM Custom #2T | 31,832 | 710 |
| 5 μM SB6 | | 57,158 | 1,161 |
| 5 μM SB7 | | 32,014 | 288 |
| 5 μM SB9 | | 40,247 | 153 |
| 5 μM SB10 | | 33,411 | 592 |
| 1.25 μM SB2 | 1.25 μM Custom #2T | 21,733 | 1,319 |
| 1.25 μM SB6 | | 37,893 | 1,527 |
| 1.25 μM SB7 | | 28,167 | 626 |
| 1.25 μM SB9 | | 39,228 | 469 |
| 1.25 μM SB10 | | 28,057 | 1,030 |

Example 48

(Wells and Well Array)

A well array of Simoa Discs was used.

(Preparation of Reaction Solution for Wells)

A reaction solution for filling the wells 204 was prepared. 6 μL (corresponding to 6 μg) of the MDA-MB-231-derived extracellular vesicles (50 μg of protein in 50 μL of PBS) described in the section "Materials and the like" were aliquoted, and the buffer (4) was added to the aliquot to provide 900 μL of a solution. The concentration of the solution is 6.7 μg/mL. The concentration of 20 μg/mL was set to x1 in Example 8, and hence the concentration of the solution is x1/3.

In addition, for detection of MMP activity, 1 mM SB2, SB6, SB7, SB9, or SB10 and 1 mM Custom #2T were diluted with the buffer (4) to prepare 15 kinds of MMP detection solutions in which any one of SB2, SB6, SB7, SB9, and SB10 and Custom #2T were each mixed at 10 μM, 5 μM, or 2.5 μM. In addition, 1 mM Alexa Fluor 647 was used as a standard fluorescent substance, and each of the MMP14 detection solutions contained the standard fluorescent substance at a final concentration of 2 μM.

50 μL of each of the MMP detection solutions was added to 50 μL of each of the samples, a reaction was performed at 30°C, and MMP activity was evaluated.

In this case, the concentration of the extracellular vesicles in each of the reaction solutions is 3.3 μg/mL for the MDA-MB-231-derived extracellular vesicles, and the concentrations of the respective reporter molecules in each of the reaction solutions are a combination of 5 μM and 5 μM, a combination of 2.5 μM and 2.5 μM, or a combination of 1.25 μM and 1.25 μM.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 for SB2, SB6, SB7, SB9, and SB10, and under the same conditions as in Example 42 for Custom #2T, to acquire fluorescence images.

Although the illustration is omitted, fluorescence microscope images each containing both of the fluorescence of 5-FAM derived from any one of SB2, SB6, SB7, SB9, and SB10, the fluorescence of TAMRA derived from Custom #2T, and the background fluorescence of any one of SB2, SB6, SB7, SB9, and SB10, and Custom #2T after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM and the fluorescence of TAMRA were observed, the fluorescence of the standard fluorescent substance was also observed. Thus, it was able to be recognized that the wells were filled with the sample and the reagent containing the reporter molecules, and cleavage reactions in the same reaction solution of any one of SB2, SB6, SB7, SB9, and SB10, and Custom #2T by MMP in the MDA-MB-231-derived extracellular vesicles worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) after 24 hours of reaction calculated with respect to the concentration of the extracellular vesicles subjected to the test through use of the above-mentioned equation (1) from the total number of wells and the number of positive wells identified from each of the histograms after 4 hours and 24 hours of reaction are shown in Table 45 and Table 46. Table 45 shows the expected value (λ) in any one of SB2, SB6, SB7, SB9, and SB10 when any one of SB2, SB6, SB7, SB9, and SB10, and Custom #2T were subjected to the reaction in the same reaction solution, and Table 46 shows the expected value (λ) in Custom #2T when any one of SB2, SB6, SB7, SB9, and SB10, and Custom #2T were subjected to the reaction in the same reaction solution. Further, the average and standard deviation of the expected values (λ) in the results under the three conditions are shown in Table 47.

In addition, the ratio of double-positives with respect to all the positives (double-positive ratio 1), the ratio of double-positives with respect to the positives for any one of SB2, SB6, SB7, SB9, and SB10 (double-positive ratio 2), and the ratio of double-positives with respect to the positives for Custom #2T (double-positive ratio 3) are shown in Table 48. Further, the averages and standard deviations of the double-positive ratios 1, the double-positive ratios 2, and the double-positive ratios 3 in the results under the three conditions are shown in Table 49. Finally, the extracellular vesicle concentration at a concentration of x1 calculated from λ at a concentration of x1 and the volume of the microcompartment that is 50 fL is shown in Table 50 and Table 51.

[Table 45]

Table 45

| Results of SB2, SB6, SB7, SB9, and SB10 | | | | | | |
|---|---|---|---|---|---|---|
| Reporter molecules | | Reaction (hr) | Number of wells | Number of positives | Positive ratio | Expected value λ |
| 5 μM SB2 | 5 μM #2T | 24 | 28,321 | 13,115 | 0.46308 | 0.62191 |
| 5 μM SB6 | | 24 | 28,414 | 13,872 | 0.48821 | 0.66984 |
| 5 μM SB7 | | 24 | 28,253 | 19,812 | 0.70124 | 1.2081 |
| 5 μM SB9 | | 24 | 28,330 | 11,014 | 0.38878 | 0.49229 |
| 5 μM SB10 | | 24 | 32,669 | 8,892 | 0.27218 | 0.31771 |
| 2.5 μM SB2 | 2.5 μM #2T | 24 | 28,277 | 11,435 | 0.40439 | 0.51817 |
| 2.5 μM SB6 | | 24 | 28,390 | 17,610 | 0.62029 | 0.96834 |
| 2.5 μM SB7 | | 24 | 28,373 | 21,473 | 0.75681 | 1.41392 |
| 2.5 μM SB9 | | 24 | 28,316 | 13,100 | 0.46264 | 0.62108 |
| 2.5 μM SB10 | | 24 | 28,339 | 14,733 | 0.51988 | 0.73373 |

(continued)

| Results of SB2, SB6, SB7, SB9, and SB10 | | | | | | |
|---|---|---|---|---|---|---|
| Reporter molecules | | Reaction (hr) | Number of wells | Number of positives | Positive ratio | Expected value λ |
| 1.25 μM SB2 | 1.25 μM #2T | 24 | 28,402 | 12,625 | 0.44451 | 0.58791 |
| 1.25 μM SB6 | | 24 | 28,335 | 11,927 | 0.42093 | 0.54633 |
| 1.25 μM SB7 | | 24 | 28,374 | 13,220 | 0.46592 | 0.62721 |
| 1.25 μM SB9 | | 24 | 28,300 | 9,168 | 0.32396 | 0.3915 |
| 1.25 μM SB10 | | 24 | 28,309 | 12,233 | 0.43212 | 0.56585 |

[Table 46]

| Results of Custom #2T | | | | | | |
|---|---|---|---|---|---|---|
| Reporter molecules | | Reaction (hr) | Number of wells | Number of positives | Positive ratio | Expected value λ |
| 5 μM SB2 | 5 μM #2T | 24 | 28,321 | 4,098 | 0.1447 | 0.1563 |
| 5 μM SB6 | | 24 | 28,414 | 3,661 | 0.12884 | 0.13794 |
| 5 μM SB7 | | 24 | 28,253 | 3,356 | 0.11878 | 0.12645 |
| 5 μM SB9 | | 24 | 28,330 | 4,962 | 0.17515 | 0.19255 |
| 5 μM SB10 | | 24 | 32,669 | 3,671 | 0.11237 | 0.1192 |
| 2.5 μM SB2 | 2.5 μM #2T | 24 | 28,277 | 4,617 | 0.16328 | 0.17826 |
| 2.5 μM SB6 | | 24 | 28,390 | 5,057 | 0.17813 | 0.19617 |
| 2.5 μM SB7 | | 24 | 28,373 | 4,363 | 0.15377 | 0.16697 |
| 2.5 μM SB9 | | 24 | 28,316 | 5,748 | 0.20299 | 0.22689 |
| 2.5 μM SB10 | | 24 | 28,339 | 5,655 | 0.19955 | 0.22258 |
| 1.25 μM SB2 | 1.25 μM #2T | 24 | 28,402 | 4,970 | 0.17499 | 0.19236 |
| 1.25 μM SB6 | | 24 | 28,335 | 4,874 | 0.17201 | 0.18876 |
| 1.25 μM SB7 | | 24 | 28,374 | 3,938 | 0.13879 | 0.14942 |
| 1.25 μM SB9 | | 24 | 28,300 | 4,693 | 0.16583 | 0.18132 |
| 1.25 μM SB10 | | 24 | 28,309 | 5,696 | 0.20121 | 0.22465 |

[Table 47]

Table 47

| Reporter molecules | | SB | | #2T | |
|---|---|---|---|---|---|
| | | Expected value λ | | Expected value λ | |
| | | Average | Standard deviation | Average | Standard deviation |
| SB2 | #2T | 0.576 | 0.04318 | 0.17564 | 0.01484 |
| SB6 | | 0.72817 | 0.17716 | 0.17429 | 0.02588 |
| SB7 | | 1.08307 | 0.33312 | 0.14761 | 0.01659 |
| SB9 | | 0.50162 | 0.09396 | 0.20026 | 0.01939 |
| SB10 | | 0.5391 | 0.17089 | 0.18881 | 0.04923 |

[Table 48]

Table 48

| Reporter molecules | | Reaction (hr) | Double-positive ratio 1 | Double-positive ratio 2 | Double-positive ratio 3 |
|---|---|---|---|---|---|
| 5 μM SB2 | 5 μM #2T | 24 | 0.14707 | 0.16828 | 0.53856 |
| 5 μM SB6 | | 24 | 0.14028 | 0.15549 | 0.58918 |
| 5 μM SB7 | | 24 | 0.12932 | 0.13391 | 0.79052 |
| 5 μM SB9 | | 24 | 0.16037 | 0.20047 | 0.44498 |
| 5 μM SB10 | | 24 | 0.12997 | 0.16251 | 0.39363 |
| 2.5 μM SB2 | 2.5 μM #2T | 24 | 0.09855 | 0.12593 | 0.31191 |
| 2.5 μM SB6 | | 24 | 0.09501 | 0.11168 | 0.38893 |
| 2.5 μM SB7 | | 24 | 0.10927 | 0.11852 | 0.58327 |
| 2.5 μM SB9 | | 24 | 0.14175 | 0.17863 | 0.40707 |
| 2.5 μM SB10 | | 24 | 0.12643 | 0.15532 | 0.40466 |
| 1.25 μM SB2 | 1.25 μM #2T | 24 | 0.16139 | 0.19366 | 0.49195 |
| 1.25 μM SB6 | | 24 | 0.16117 | 0.19552 | 0.47846 |
| 1.25 μM SB7 | | 24 | 0.14425 | 0.16362 | 0.54926 |
| 1.25 μM SB9 | | 24 | 0.13979 | 0.18543 | 0.36224 |
| 1.25 μM SB10 | | 24 | 0.18743 | 0.23134 | 0.49684 |

[Table 49]

Table 49

| Reporter molecules | | Double-positive ratio 1 | | Double-positive ratio 2 | | Double-positive ratio 3 | |
|---|---|---|---|---|---|---|---|
| | | Average | Standard deviation | Average | Standard deviation | Average | Standard deviation |
| SB2 | #2T | 0.13567 | 0.02689 | 0.16262 | 0.02794 | 0.44747 | 0.09773 |
| SB6 | | 0.13215 | 0.02762 | 0.15423 | 0.03424 | 0.48552 | 0.08191 |
| SB7 | | 0.12761 | 0.01433 | 0.13868 | 0.01872 | 0.64102 | 0.10662 |
| SB9 | | 0.1473 | 0.00928 | 0.18818 | 0.00913 | 0.40477 | 0.03382 |
| SB10 | | 0.14794 | 0.02796 | 0.18305 | 0.03427 | 0.43171 | 0.04628 |

[Table 50]

Table 50

| Results of SB2, SB6, SB7, SB9, and SB10 | | | | |
|---|---|---|---|---|
| Reporter molecules | | Reaction (hr) | Expected value λ (×1) | Extracellular vesicle concentration (×$10^{10}$/mL) |
| 5 μM SB2 | 5 μM #2T | 24 | 1.86574 | 3.73148 |
| 5 μM SB6 | | 24 | 2.00952 | 4.01905 |
| 5 μM SB7 | | 24 | 3.6243 | 7.24859 |
| 5 μM SB9 | | 24 | 1.47687 | 2.95374 |
| 5 μM SB10 | | 24 | 0.95312 | 1.90625 |
| 2.5 μM SB2 | 2.5 μM #2T | 24 | 1.55452 | 3.10904 |
| 2.5 μM SB6 | | 24 | 2.90503 | 5.81007 |
| 2.5 μM SB7 | | 24 | 4.24175 | 8.4835 |
| 2.5 μM SB9 | | 24 | 1.86324 | 3.72648 |
| 2.5 μM SB10 | | 24 | 2.20118 | 4.40237 |
| 1.25 μM SB2 | 1.25 μM #2T | 24 | 1.76372 | 3.52744 |
| 1.25 μM SB6 | | 24 | 1.63899 | 3.27797 |
| 1.25 μM SB7 | | 24 | 1.88163 | 3.76325 |
| 1.25 μM SB9 | | 24 | 1.1745 | 2.349 |
| 1.25 μM SB10 | | 24 | 1.69756 | 3.39511 |

[Table 51]

Table 51

| Results of #2T | | | | |
|---|---|---|---|---|
| Reporter molecules | | Reaction (hr) | Expected value λ (×1) | Extracellular vesicle concentration (×$10^{10}$/mL) |
| 5 μM SB2 | 5 μM #2T | 24 | 0.4689 | 0.93781 |
| 5 μM SB6 | | 24 | 0.41381 | 0.82761 |
| 5 μM SB7 | | 24 | 0.37936 | 0.75871 |
| 5 μM SB9 | | 24 | 0.57766 | 1.15532 |
| 5 μM SB10 | | 24 | 0.3576 | 0.7152 |
| 2.5 μM SB2 | 2.5 μM #2T | 24 | 0.53479 | 1.06958 |
| 2.5 μM SB6 | | 24 | 0.5885 | 1.17701 |
| 2.5 μM SB7 | | 24 | 0.5009 | 1.00181 |
| 2.5 μM SB9 | | 24 | 0.68068 | 1.36136 |
| 2.5 μM SB10 | | 24 | 0.66774 | 1.33547 |
| 1.25 μM SB2 | 1.25 μM #2T | 24 | 0.57707 | 1.15414 |
| 1.25 μM SB6 | | 24 | 0.56627 | 1.13255 |
| 1.25 μM SB7 | | 24 | 0.44825 | 0.89649 |
| 1.25 μM SB9 | | 24 | 0.54396 | 1.08791 |
| 1.25 μM SB10 | | 24 | 0.67396 | 1.34793 |

Accordingly, it was shown that multiplex-digital detection of the extracellular vesicles was able to be performed through use of MMP activity even when different reporter molecules were contained in the same reaction solution.

Example 49

(Preparation of Particles)

Magnetic particles included in PS Capture (trademark) Exosome Flow Cytometry Kit (manufactured by FUJIFILM Corporation) were used as magnetic particles. In this kit, binding of an extracellular vesicle to a Tim4-immobilized magnetic particle is utilized.

800 μL of Exosome Capture Beads were aliquoted in a 1.5 mL microtube, vortexed for 5 seconds, spun down in a benchtop centrifuge, and then placed on a magnetic stand. After still standing for 1 minute, the supernatant was removed by pipetting. 1 mL of x1 WB (+Enhancer) prepared from x10 Washing Buffer and x100 Exosome Binding Enhancer included in the kit was added to the residue, the mixture was vortexed for 5 seconds, spun down in a benchtop centrifuge, and then placed on a magnetic stand. After still standing for 1 minute, the supernatant was removed by pipetting. This operation was repeated three times.

800 μL of the buffer (4) was added to the magnetic particle pellets, and the mixture was vortexed for 30 seconds to provide a homogeneous magnetic particle suspension. 60 μL of the suspension was aliquoted into each of twelve 1.5 mL microtubes.

(Formation of Particle Complexes of Tim4-immobilized Magnetic Particles and Extracellular Vesicles)

2 μL (corresponding to 2 μg) of the MDA-MB-231-derived extracellular vesicles (50 μg of protein in 50 μL of PBS) described in the section "Materials and the like" were aliquoted, and the buffer (4) was added to the aliquot to provide 500 μL of a solution. The concentration of the solution is 4 μg/mL. The concentration of 20 μg/mL was set to x1 in Example 8, and hence the concentration of the solution is x1/5. Further, the concentration of the x1/5 solution was set to be the highest concentration, and 10-fold dilution was performed in four stages. Thus, 10-fold dilution samples in a total of five stages were obtained. In addition, a sample free of extracellular vesicles was prepared similarly, and used as a sample for BG measurement.

The magnetic particle suspension that had been aliquoted in each amount of 60 μL in the section "(Preparation of Particles)" was vortexed for 5 seconds, spun down in a benchtop centrifuge, and then placed on a magnetic stand. After still standing for 1 minute, the supernatant was removed by pipetting. 150 μL of each of the above-mentioned samples was added to the magnetic particle pellets, and the resultant mixed solution was subjected to a reaction with shaking at room temperature for 60 minutes to form particle complexes of Tim4-immobilized magnetic particles and the extracellular vesicles.

After that, the above-mentioned mixed solution was spun down in a benchtop centrifuge, then placed on a magnetic stand, and left to stand still for 1 minute. After that, the supernatant was removed by pipetting, 300 μL of the buffer (4) was added to the residue, and the mixture was vortexed for 5 seconds. The mixture was spun down in a benchtop centrifuge, then placed on a magnetic stand, and left to stand still for 1 minute. The same operation was further repeated twice.

(Wells and Well Array)

A well array of Simoa Discs was used.

(Preparation of Reaction Solution for Wells)

For detection of MMP14 activity, 1 mM Custom #1 and Custom #2 were diluted with the buffer (4) to prepare MMP14 detection solutions containing 5 μM Custom #1 and Custom #2, respectively.

The supernatant of the mixed solution of the magnetic particles and the extracellular vesicles subjected to the washing was removed by pipetting, and 60 μL of each of the MMP14 detection solutions was added to each of the magnetic particle pellets to prepare a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from Custom #1 or Custom #2 and the background fluorescence of Custom #1 or Custom #2 after 4 hours of reaction was acquired. In addition, a bright field image for observation of particles was acquired together.

The wells filled with the particles were able to be recognized from the bright field image, and it was found that the fluorescence of 5-FAM was observed from the wells filled with the particles. Thus, it was able to be recognized that the MDA-MB-231-derived extracellular vesicles were captured on the particles filling the microcompartments, and a reaction of Custom #1 or Custom #2 by MMP14 in the extracellular vesicles worked.

Next, although the illustration is omitted, the fluorescence microscope images after 4 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the number of wells filled with the particles and the number of positive wells identified from each of the histograms after 4 hours of reaction is shown in Table 52. In addition, FIG. 72 shows plots of the expected value λ for Custom #1, and FIG. 73 shows plots of the expected value λ for Custom #2. In addition, the expected value (λ) calculated by subtracting the number of positive wells containing the particles in BG from the number of positive wells containing the particles at each concentration of the extracellular vesicles subjected to the test is shown in Table 53. In addition, FIG. 74 shows plots of the expected value λ for Custom #1, and FIG. 75 shows plots of the expected value λ for Custom #2.

[Table 52]

Table 52

| Reporter molecule | MB231 concentration (a.u.) | Number of wells | Number of particles | Particle | Particle | Particle |
|---|---|---|---|---|---|---|
| | | | | Number of positives | Positive ratio | Expected value λ |
| Custom #1 | x1/5 | 27,643 | 2,197 | 1,240 | 0.56441 | 0.83104 |
| Custom #1 | x1/50 | 27,823 | 2,452 | 165 | 0.06729 | 0.06966 |
| Custom #1 | x1/500 | 27,745 | 2732 | 89 | 0.03258 | 0.03312 |
| Custom #1 | x1/5,000 | 27,745 | 3,071 | 41 | 0.01335 | 0.01344 |
| Custom #1 | x1/50,000 | 27,745 | 2,581 | 30 | 0.01162 | 0.01169 |
| Custom #1 | 0 | 27,822 | 3,798 | 28 | 0.00737 | 0.0074 |
| Custom #2 | x1/5 | 27,758 | 2,321 | 557 | 0.23998 | 0.27441 |
| Custom #2 | x1/50 | 27,822 | 2,677 | 246 | 0.09189 | 0.09639 |
| Custom #2 | x1/500 | 27,789 | 4,874 | 181 | 0.03714 | 0.03784 |
| Custom #2 | x1/5,000 | 27,725 | 3,073 | 37 | 0.01204 | 0.01211 |
| Custom #2 | x1/50,000 | 27,781 | 3,096 | 17 | 0.00549 | 0.00551 |
| Custom #2 | 0 | 27,721 | 3,224 | 5 | 0.00155 | 0.00155 |

[Table 53]

Table 53

| Reporter molecule | MB231 concentration (a.u.) | Number of wells | Number of particles | Particle Δ Number of positives | Particle Δ Positive ratio | Particle Expected value Δλ |
|---|---|---|---|---|---|---|
| Custom #1 | x1/5 | 27,643 | 2,197 | 1,212 | 0.55166 | 0.80221 |
| Custom #1 | x1/50 | 27,823 | 2,452 | 137 | 0.05587 | 0.05749 |
| Custom #1 | x1/500 | 27,745 | 2,732 | 61 | 0.02233 | 0.02258 |
| Custom #1 | x1/5,000 | 27,745 | 3,071 | 13 | 0.00423 | 0.00424 |
| Custom #1 | x1/50,000 | 27,745 | 2,581 | 2 | 0.00077 | 0.00078 |
| Custom #1 | 0 | 27,822 | 3,798 | 0 | 0 | 0 |
| Custom #2 | x1/5 | 27,758 | 2,321 | 552 | 0.23783 | 0.27158 |
| Custom #2 | x1/50 | 27,822 | 2,677 | 241 | 0.09003 | 0.09434 |
| Custom #2 | x1/500 | 27,789 | 4,874 | 176 | 0.03611 | 0.03678 |
| Custom #2 | x1/5,000 | 27,725 | 3,073 | 32 | 0.01041 | 0.01047 |
| Custom #2 | x1/50,000 | 27,781 | 3,096 | 12 | 0.00388 | 0.00388 |
| Custom #2 | 0 | 27,721 | 3,224 | 0 | 0 | 0 |

The concentration of the sample was 4 μg/mL, 150 μL of the sample was used, and hence the extracellular vesicles with 0.6 μg of protein, which are converted into the extracellular vesicles with 3 μg of protein at a concentration of x1, were used in the reaction. In addition, $6\times10^5$ particles were subjected to the reaction. From FIG. 74 for Custom #1, the expected value Δλ per particle (value obtained by subtracting BG) at a concentration of x1 is 1.7373, and hence the number of the extracellular vesicles is $1.04\times10^6$, and is estimated to be $3.47\times10^5$/μg of protein. In addition, from FIG. 75 for Custom #2, the number of the extracellular vesicles may be similarly estimated to be $1.18\times10^5$/μg of protein.

Accordingly, the extracellular vesicles were captured on the particles modified with Tim4 serving as a capture molecule, and the extracellular vesicles were able to be detected and quantified by the MMP14 activity of the extracellular vesicles.

Example 50

(Preparation of Particles)

Magnosphere (trademark) MS300/Streptavidin (manufactured by JSR Corporation) (hereinafter abbreviated as "MS300S") was used as magnetic particles. The magnetic particles were modified with Tim4 through use of Biotin-labeled Exosome Capture included in MagCapture (trademark) Exosome Isolation Kit PS Ver. 2 (manufactured by FUJIFILM Corporation) (hereinafter abbreviated as "PS2") to prepare Tim4-immobilized magnetic particles.

100 μL ($6\times10^7$ particles) of MS300S was aliquoted in a 1.5 mL microtube, vortexed for 5 seconds, spun down in a benchtop centrifuge, and then placed on a magnetic stand.

After still standing for 1 minute, the supernatant was removed by pipetting. 500 μL of x1 I/WB (+Enhancer) prepared from x10 Exosome Immobilizing/Washing Buffer and x500 Exosome Binding Enhancer included in the PS2 kit was added to the residue, and the mixture was vortexed for 5 seconds, spun down in a benchtop centrifuge, and then placed on a magnetic stand. After still standing for 1 minute, the supernatant was removed by pipetting.

500 μL of x1 I/WB (+Enhancer) was added to the magnetic particle pellets, and the mixture was vortexed for 30 seconds to provide a magnetic particle suspension. 10 μL of Biotin-labeled Exosome Capture was added to the suspension, and the mixture was vortexed for 5 seconds so that the contents were mixed. The mixture was subjected to a reaction for 10 minutes while the contents were mixed by inversion at room temperature with a rotator. After that, the resultant was spun down in a benchtop centrifuge, and then placed on a magnetic stand. After still standing for 1 minute, the supernatant was removed by pipetting. 500 μL of x1 I/WB (+Enhancer) was added to the residue, and the mixture was vortexed for 5 seconds, spun down in a benchtop centrifuge, and then placed on a magnetic stand. After still standing for 1 minute, the supernatant was removed by pipetting. This operation was further repeated twice.

600 μL of x1 I/WB (+Enhancer) was added to the magnetic particle pellets ($1\times10^8$ particles/mL), and the mixture was vortexed for 30 seconds to provide a Tim4-immobilized magnetic particle suspension.

In use, 6 μL of the Tim4-immobilized magnetic particle suspension was added to 120 μL of the buffer (4), and the mixture was vortexed for 30 seconds to provide a homogeneous magnetic particle suspension. The suspension was spun down in a benchtop centrifuge, then placed on a magnetic stand, and left to stand still for 1 minute, and then the supernatant was removed by pipetting.

(Formation of Particle Complexes of Tim4-immobilized Magnetic Particles and Extracellular Vesicles)

4 μL (corresponding to 4 μg) of the MDA-MB-231-derived extracellular vesicles (50 μg of protein in 50 μL of PBS) described in the section "Materials and the like" were aliquoted, and the buffer (4) was added to the aliquot to provide 200 μL of a solution. The concentration of the solution is 20 μg/mL. The concentration of 20 μg/mL was set to x1 in Example 8, and hence the concentration of the solution is x1. Further, the concentration of the x1 solution was set to be the highest concentration, and 5-fold dilution was performed in six stages. Thus, 5-fold dilution samples in a total of seven stages were obtained. In addition, a sample free of extracellular vesicles was prepared similarly, and used as a sample for BG measurement.

100 μL of each of the above-mentioned samples was added to the magnetic particle pellets prepared in the section "(Preparation of Particles)", and the mixture was vortexed for 30 seconds so that the contents were mixed. The resultant mixed solution was subjected to a reaction with shaking at room temperature for 60 minutes to form particle complexes of the Tim4-immobilized magnetic particles and the extracellular vesicles.

After that, the above-mentioned mixed solution was spun down in a benchtop centrifuge, then placed on a magnetic stand, and left to stand still for 1 minute. After that, the supernatant was removed by pipetting, 300 μL of the buffer (4) was added to the residue, and the mixture was vortexed for 5 seconds. The mixture was spun down in a benchtop centrifuge, then placed on a magnetic stand, and left to stand still for 1 minute. The same operation was further repeated twice.

(Wells and Well Array)

A well array of Simoa Discs was used.

(Preparation of Reaction Solution for Wells)

For detection of MMP14 activity, 1 mM Custom #2 was diluted with the buffer (4) to prepare a MMP14 detection solution containing 5 μM Custom #2.

The supernatant of the mixed solution of the magnetic particles and the extracellular vesicles subjected to the washing was removed by pipetting, and 60 μL of the MMP14 detection solution was added to each of the magnetic particle pellets to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from Custom #2 and the background fluorescence of Custom #2 after 4 hours of reaction was acquired. In addition, a bright field image for observation of particles was acquired together.

The wells filled with the particles were able to be recognized from the bright field image, and it was found that the fluorescence of 5-FAM was observed from the wells filled with the particles. Thus, it was able to be recognized that the MDA-MB-231-derived extracellular vesicles were captured on the particles filling the microcompartments, and a reaction of Custom #2 by MMP14 in the extracellular vesicles worked.

Next, although the illustration is omitted, the fluorescence microscope images after 4 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the number of wells filled with the particles and the number of positive wells identified from each of the histograms after 4 hours of reaction is shown in Table 54. In addition, FIG. 76 is a graph in which the expected value λ is plotted. In addition, the expected value (λ) calculated by subtracting the number of positive wells containing the particles in BG from the number of positive wells containing the particles at each concentration of the extracellular vesicles subjected to the test is shown in Table 55. In addition, FIG. 77 is a graph in which the expected value λ is plotted.

[Table 54]

Table 54

| Reporter molecule | MB231 concentration (a.u.) | Number of wells | Number of particles | Particle Number of positives | Particle Positive ratio | Particle Expected value λ |
|---|---|---|---|---|---|---|
| Custom #2 | x1 | 27,789 | 6,903 | 1,744 | 0.25264 | 0.29121 |
| Custom #2 | x1/5 | 27,822 | 7,166 | 1,235 | 0.17234 | 0.18915 |
| Custom #2 | x1/25 | 27,819 | 6,488 | 643 | 0.09911 | 0.10437 |
| Custom #2 | x1/125 | 27,766 | 6,509 | 385 | 0.05915 | 0.06097 |
| Custom #2 | x1/625 | 27,745 | 6,912 | 225 | 0.03255 | 0.03309 |
| Custom #2 | x1/3,125 | 27,819 | 7,531 | 166 | 0.02204 | 0.02229 |
| Custom #2 | x1/15,625 | 27,722 | 5,414 | 88 | 0.01625 | 0.01639 |
| Custom #2 | 0 | 27,758 | 4,726 | 23 | 0.00487 | 0.00488 |

[Table 55]

Table 55

| Reporter molecule | MB231 concentration (a.u.) | Number of wells | Number of particles | Particle Δ Number of positives | Particle Δ Positive ratio | Particle Expected value Δλ |
|---|---|---|---|---|---|---|
| Custom #2 | x1 | 27,789 | 6,903 | 1,721 | 0.24931 | 0.28677 |
| Custom #2 | x1/5 | 27,822 | 7,166 | 1,212 | 0.16913 | 0.18528 |
| Custom #2 | x1/25 | 27,819 | 6,488 | 620 | 0.09556 | 0.10044 |
| Custom #2 | x1/125 | 27,766 | 6,509 | 362 | 0.05562 | 0.05722 |
| Custom #2 | x1/625 | 27,745 | 6,912 | 202 | 0.02922 | 0.02966 |
| Custom #2 | x1/3,125 | 27,819 | 7,531 | 143 | 0.01899 | 0.01917 |
| Custom #2 | x1/15,625 | 27,722 | 5,414 | 65 | 0.01201 | 0.01208 |
| Custom #2 | 0 | 27,758 | 4,726 | 0 | 0 | 0 |

The concentration of the sample was 20 μg/mL, 100 μL of the sample was used, and hence the extracellular vesicles with 2 μg of protein were used in the reaction. In addition, the number of the particles subjected to the reaction is $6\times10^5$. From FIG. 77, the expected value Δλ per particle (value obtained by subtracting BG) at a concentration of x1 is 0.2953, and hence the number of the extracellular vesicles is $1.77\times10^5$, and is estimated to be $8.86\times10^4$/μg of protein.

Accordingly, the extracellular vesicles were captured on the particles immobilized with Tim4 serving as a capture molecule, and the extracellular vesicles were able to be detected and quantified by the MMP14 activity of the extracellular vesicles.

Example 51

(Production of Antibody-immobilized Particles)

An antibody-immobilized particle solution was prepared and stored at 4°C until use, in the same manner as in Example 28.
In addition, an antibody-immobilized particle solution was prepared and stored at 4°C until use, in the same manner as in Example 28 except that Magnosphere MS300/Carboxyl (manufactured by JSR Corporation) (hereinafter abbreviated as "MS300C") was used as magnetic particles.

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 400 μL of a solution serving as a sample. The concentration of the solution is 10 μg/mL. Further, a sample free of extracellular vesicles was prepared similarly, and used as a sample for BG measurement.
Each of the above-mentioned samples and the following were mixed.

Each of the samples prepared above: 100 μL
The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.
For detection of MMP14 activity, 1 mM Custom #2 was diluted with the buffer (4) to prepare a MMP14 detection solution containing 5 μM Custom #2. In addition, 1 mM Custom #2T was diluted with the buffer (4) to prepare a MMP14 detection solution containing 2.5 μM Custom #2T.
50 μL of each of the MMP14 detection solutions was added to the recovered complexes to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).
The observation of fluorescence derived from each fluorescent substance was performed under the same conditions as in Example 19 for Custom #2 and under the same conditions as in Example 42 for Custom #2T, to acquire fluorescence images.
Although the illustration is omitted, a fluorescence microscope image containing both of the fluorescence of 5-FAM derived from Custom #2 and the background fluorescence of Custom #2 after 24 hours of reaction was acquired. Similarly, a fluorescence microscope image containing both of the fluorescence of TAMRA derived from Custom #2T and the background fluorescence of Custom #2T was acquired. In addition, a bright field image for observation of particles was acquired together.
The wells filled with the particles were able to be recognized from the bright field image, and it was found that the fluorescence of 5-FAM or TAMRA was observed from the wells filled with the particles. Thus, it was able to be recognized that the MDA-MB-231-derived extracellular vesicles were captured on the particles filling the microcompartments, and a reaction of Custom #2 or Custom #2T by MMP14 in the extracellular vesicles worked.
Next, although the illustration is omitted, the fluorescence microscope images after 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the number of wells filled with the particles and the number of positive wells identified from each of the histograms after 24 hours of reaction is shown in Table 56.

[Table 56]

Table 56

| Reporter molecule | Particle | MB231 | Number of wells | Number of particles | Particle | Particle | Particle |
|---|---|---|---|---|---|---|---|
| | | | | | Number of positives | Positive ratio | Expected value λ |
| Custom #2 | Simoa | Present | 27,820 | 4,699 | 1,234 | 0.26261 | 0.30464 |
| Custom #2 | Simoa | Absent | 27,773 | 6,569 | 8 | 0.00122 | 0.00122 |
| Custom #2T | Simoa | Present | 27,823 | 4,815 | 359 | 0.07456 | 0.07748 |
| Custom #2T | Simoa | Absent | 27,768 | 6,637 | 13 | 0.00196 | 0.00196 |
| Custom #2 | MS300C | Present | 27,756 | 5,118 | 1,288 | 0.25166 | 0.2899 |
| Custom #2 | MS300C | Absent | 27,788 | 7,100 | 16 | 0.00225 | 0.00226 |
| Custom #2T | MS300C | Present | 27,823 | 5,736 | 1,460 | 0.25453 | 0.29374 |
| Custom #2T | MS300C | Absent | 27,745 | 5,515 | 11 | 0.00199 | 0.002 |

The concentration of the sample was 10 μg/mL, 100 μL of the sample was used, and hence the extracellular vesicles with 1 μg of protein were used in the reaction. 25 μL of the particles at a concentration of $2.0\times10^7$ particles/mL were used, and hence the number of the particles subjected to the reaction is $5.0\times10^5$. The expected value λ per particle is as shown in Table 56, and hence the extracellular vesicle concentration is estimated as shown in Table 57.

[Table 57]

Table 57

| Reporter molecule | Particle | MB231 | Particle expected value λ | Extracellular vesicle concentration |
|---|---|---|---|---|
| | | | | ($\times10^5$/μg of protein) |
| Custom #2 | Simoa | Present | 0.30464 | 1.5232 |
| Custom #2 | Simoa | Absent | 0.00122 | 0.0061 |
| Custom #2T | Simoa | Present | 0.07748 | 0.3874 |
| Custom #2T | Simoa | Absent | 0.00196 | 0.0098 |
| Custom #2 | MS300C | Present | 0.2899 | 1.4495 |
| Custom #2 | MS300C | Absent | 0.00226 | 0.0113 |
| Custom #2T | MS300C | Present | 0.29374 | 1.4687 |
| Custom #2T | MS300C | Absent | 0.002 | 0.01 |

Accordingly, the extracellular vesicles were captured on the particles to which a capture antibody including a cocktail of the anti-CD9 antibody, the anti-CD63 antibody, and the anti-CD81 antibody was bound, and the extracellular vesicles were able to be detected and quantified by the MMP14 activity of the extracellular vesicles. In this case, in the system using Simoa particles, the extracellular vesicle concentration is estimated to be lower in Custom #2T than in Custom #2. A possible reason for this is that the autofluorescence of the particles resulted in background noise to affect quantitative accuracy. Meanwhile, with regard to both Custom #2 with reduced influence of autofluorescence and Custom #2T in MS300C, almost the same quantitative value is obtained. Accordingly, it is conceivable that MS300C is preferred in a multiplex assay.

Example 52

(Production of Antibody-immobilized Particles)

An antibody-immobilized particle solution was prepared and stored at 4°C until use, in the same manner as in Example 28 except that: MS300C was used as magnetic particles; and blocking treatment was performed at 4°C or room temperature through use of Blockmaster CE210 or CE510 (manufactured by Medical & Biological Laboratories Co., Ltd.) instead of Bead Blocking Buffer (manufactured by Quanterix Corporation).

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 400 μL of a solution serving as a sample. The concentration of the solution is 10 μg/mL. Further, a sample free of extracellular vesicles was prepared similarly, and used as a sample for BG measurement.

Each of the above-mentioned samples and the following were mixed.

Each of the samples prepared above: 100 μL
The antibody-immobilized particles ($2.0\times10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.

For detection of MMP14 activity, 1 mM Custom #2 and 1 mM Custom #2T were diluted with the buffer (4) to prepare a MMP14 detection solution containing 5 μM Custom #2 and 2.5 μM Custom #2T serving as the reporter molecules in the same system.

50 μL of the MMP14 detection solution was added to the recovered complexes to provide a reaction solution for wells.

(Filling of Wells with Reaction Solution)

A fluorinated oil SR-X Sealing Oil was used as a hydrophobic solvent and wells were filled with the reaction solution in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).

The observation of fluorescence derived from each fluorescent substance was performed under the same conditions as in Example 19 for Custom #2 and under the same conditions as in Example 42 for Custom #2T, to acquire fluorescence images.

Although the illustration is omitted, a fluorescence microscope image containing both of the fluorescence of 5-FAM derived from Custom #2 and the background fluorescence of Custom #2 after 24 hours of reaction was acquired. Similarly, a fluorescence microscope image containing both of the fluorescence of TAMRA derived from Custom #2T and the background fluorescence of Custom #2T was acquired. In addition, a bright field image for observation of particles was acquired together.

The wells filled with the particles were able to be recognized from the bright field image, and it was found that the fluorescence of 5-FAM or TAMRA was observed from the wells filled with the particles. Thus, it was able to be recognized that the MDA-MB-231-derived extracellular vesicles were captured on the particles filling the microcompartments, and a reaction of Custom #2 or Custom #2T by MMP14 in the extracellular vesicles worked.

Next, although the illustration is omitted, the fluorescence microscope images after 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

From the number of wells filled with the particles and the number of positive wells identified from each of the histograms after 24 hours of reaction, the double-positive ratio of positives for both of Custom #2 and Custom #2T with respect to all the positives (the total number of wells that are positive for any one of Custom #2 and Custom #2T or positive for both thereof), and the expected value (λ) of particles exhibiting double-positivity calculated with respect to the concentration of the extracellular vesicles subjected to the test through use of the above-mentioned equation (1) are shown in Table 58.

[Table 58]

Table 58

| Blocking | MB23 1 | Number of wells | Number of particl es | Total number of positives | Number of double-positives | Double positive ratio | Expected value λ of particles exhibiting double-positivity |
|---|---|---|---|---|---|---|---|
| CE210 Treatment at 4°C | Present | 28,080 | 1,469 | 36 | 36 | 1 | 0.02481 |
| | Absent | 28,002 | 6,705 | 5 | 1 | 0.2 | 0.00015 |
| CE210 Treatment at room temperature | Present | 27,922 | 5,719 | 142 | 136 | 0.95775 | 0.02407 |
| | Absent | 28,080 | 6,706 | 5 | 2 | 0.4 | 0.0003 |
| CE510 Treatment at 4°C | Present | 28,080 | 3,542 | 85 | 77 | 0.90588 | 0.02198 |
| | Absent | 28,002 | 7,172 | 4 | 0 | 0 | 0 |
| CE510 Treatment at room temperature | Present | 28,077 | 1,354 | 36 | 34 | 0.94444 | 0.02543 |
| | Absent | 28,080 | 2,781 | 2 | 1 | 0.5 | 0.00036 |

When any blocking treatment is used in Custom #2 and Custom #2T, which differ from each other only in FRET pair and have the same substrate sequence, a double-positive ratio of 90% or more is achieved, and it is found that multiplex detection satisfactorily works. In this case, the double-positive ratio in a system without MB231 is presumed to be due to false positivity, and hence the ratio is not considered.

The concentration of the sample was 10 μg/mL, 100 μL of the sample was used, and hence the extracellular vesicles with 1 μg of protein were used in the reaction. 25 μL of the particles at a concentration of $1\times10^8$ particles/mL were used, and hence the number of the particles subjected to the reaction is $2.5\times10^6$. The expected value λ per particle is as shown in Table 58, and hence the extracellular vesicle concentration exhibiting double-positivity in Custom #2 and Custom #2T is estimated as shown in Table 59.

[Table 59]

Table 59

| Blocking | MB231 | Expected value λ of particles exhibiting double-positivity | Extracellular vesicle concentration (×$10^4$/μg of protein) |
|---|---|---|---|
| CE210 Treatment at 4°C | Present | 0.02481 | 6.2029 |
| | Absent | 0.00015 | 0.0373 |
| CE210 Treatment at room temperature | Present | 0.02407 | 6.0169 |
| | Absent | 0.00031 | 0.0746 |
| CE510 Treatment at 4°C | Present | 0.02198 | 5.4947 |
| | Absent | 0 | 0 |
| CE510 Treatment at room temperature | Present | 0.02543 | 6.3579 |
| | Absent | 0.00036 | 0.0899 |

Accordingly, the extracellular vesicles were captured on the particles to which a capture antibody including a cocktail of the anti-CD9 antibody, the anti-CD63 antibody, and the anti-CD81 antibody was bound, and multiplex detection and quantification of the extracellular vesicles were able to be performed by the MMP14 activity of the extracellular vesicles through use of homo reporter molecules.

Example 53

(Production of Antibody-immobilized Particles)

An antibody-immobilized particle solution was prepared and stored at 4°C until use, in the same manner as in Example 52.

(Wells and Well Array)

A well array of Simoa Discs was used.

(Preparation of Reaction Solution for Wells)

A reaction solution for filling the wells 204 was prepared. 4 μL (corresponding to 4 μg) of the MDA-MB-231-derived extracellular vesicles (50 μg of protein in 50 μL of PBS) described in the section "Materials and the like" were aliquoted, and Sample Diluent Buffer (manufactured by Quanterix Corporation) was added to the aliquot to provide 400 μL of a solution serving as a sample. The concentration of the solution is 10 μg/mL. Further, a sample free of extracellular vesicles was prepared similarly, and used as a sample for BG measurement.
Each of the above-mentioned samples and the following were mixed.

Each of the samples prepared above: 100 μL
The antibody-immobilized particles (2.0×$10^7$ particles/mL) produced above: 25 μL

The resultant mixed solution was subjected to a reaction with shaking at 30°C for 30 minutes to form particle complexes of the antibody-immobilized particles and the extracellular vesicles. After that, washing was performed with a plate washer and the complexes were then recovered with a magnet.
For detection of MMP14 activity, 1 mM Custom #1 and 1 mM Custom #2T were diluted with the buffer (4) to prepare a MMP14 detection solution containing 5 μM Custom #1 and 2.5 μM Custom #2T.
50 μL of the MMP14 detection solution was added to the recovered complexes to provide a reaction solution for wells.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 23°C to promote the generation of fluorescence. After that, observation was performed with a fluorescence microscope (BZ-X800).

The observation of fluorescence derived from each fluorescent substance was performed under the same conditions as in Example 19 for Custom #1 and under the same conditions as in Example 42 for Custom #2T, to acquire fluorescence images.

Although the illustration is omitted, a fluorescence microscope image containing both of the fluorescence of 5-FAM derived from Custom #1 and the background fluorescence of Custom #1 after 24 hours of reaction was acquired. Similarly, a fluorescence microscope image containing both of the fluorescence of TAMRA derived from Custom #2T and the background fluorescence of Custom #2T was acquired. In addition, a bright field image for observation of particles was acquired together.

The wells filled with the particles were able to be recognized from the bright field image, and it was found that the fluorescence of 5-FAM or TAMRA was observed from the wells filled with the particles. Thus, it was able to be recognized that the MDA-MB-231-derived extracellular vesicles were captured on the particles filling the microcompartments, and a reaction of Custom #1 or Custom #2T by MMP14 in the extracellular vesicles worked.

Next, although the illustration is omitted, the fluorescence microscope images after 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

From the number of wells filled with the particles and the number of positive wells identified from each of the histograms after 24 hours of reaction, the double-positive ratio of positives for both of Custom #1 and Custom #2T with respect to all the positives (the total number of wells that are positive for any one of Custom #1 or Custom #2T or positive for both thereof), and the expected value ($\lambda$) of particles exhibiting double-positivity calculated with respect to the concentration of the extracellular vesicles subjected to the test through use of the above-mentioned equation (1) are shown in Table 60.

[Table 60]

Table 60

| Blocking | MB231 | Number of wells | Number of particles | Total number of positives | Number of double-positives | Double-positive ratio | Expected value $\lambda$ of particles exhibiting double-positivity |
|---|---|---|---|---|---|---|---|
| CE210 Treatment at 4°C | Present | 28,080 | 5,109 | 152 | 76 | 0.5 | 0.01499 |
| | Absent | 27,995 | 10,431 | 5 | 1 | 0.2 | 0.00048 |
| CE210 Treatment at room temperature | Present | 28,080 | 7,642 | 216 | 121 | 0.56019 | 0.01596 |
| | Absent | 28,029 | 11,296 | 4 | 1 | 0.25 | 0.00035 |
| CE510 Treatment at 4°C | Present | 28,080 | 12,020 | 365 | 189 | 0.51781 | 0.01585 |
| | Absent | 28,080 | 12,403 | 3 | 0 | 0 | 0.00024 |
| CE510 Treatment at room temperature | Present | 28,002 | 10,262 | 262 | 150 | 0.57252 | 0.01472 |
| | Absent | 28,080 | 8,935 | 3 | 1 | 0.33333 | 0.00034 |

When any blocking treatment is used in Custom #1 and Custom #2T, a double-positive ratio of from about 50% to about 60% is achieved, and it is found that multiplex detection satisfactorily works. In this case, the double-positive ratio is lower than the double-positive ratio in Custom #2 and Custom #2T of Example 52. It is presumed that this is because there is a slight difference in activity for MMP14 (easiness in cleavage) and substrate specificity (activity for another MMP) because of a difference in substrate sequence between Custom #1 and Custom #2T. In this case, the double-positive ratio in a system without MB231 is presumed to be due to false positivity, and hence the ratio is not considered.

The concentration of the sample was 10 μg/mL, 100 μL of the sample was used, and hence the extracellular vesicles with 1 μg of protein were used in the reaction. 25 μL of the particles at a concentration of $1\times10^8$ particles/mL were used, and hence the number of the particles subjected to the reaction is $2.5\times10^6$. The expected value λ per particle is as shown in Table 60, and hence the extracellular vesicle concentration exhibiting double-positivity in Custom #1 and Custom #2T is estimated as shown in Table 61.

[Table 61]

Table 61

| Blocking | MB231 | Expected value λ of particles exhibiting double-positivity | Extracellular vesicle concentration ($\times10^4$/μg of protein) |
|---|---|---|---|
| CE210 at 4°C | Present | 0.01499 | 3.7469 |
| | Absent | 0.00048 | 0.11986 |
| CE210 at room temperature | Present | 0.01596 | 3.9901 |
| | Absent | 0.00035 | 0.08854 |
| CE510 at 4°C | Present | 0.01585 | 3.9622 |
| | Absent | 0.00024 | 0.06048 |
| CE510 at room temperature | Present | 0.01472 | 3.6812 |
| | Absent | 0.00034 | 0.08395 |

Accordingly, the extracellular vesicles were captured on the particles to which a capture antibody including a cocktail of the anti-CD9 antibody, the anti-CD63 antibody, and the anti-CD81 antibody was bound, and multiplex detection and quantification of the extracellular vesicles were able to be performed by the MMP14 activity of the extracellular vesicles through use of hetero reporter molecules.

Example 54

(Detection of MMP14 Activity of HT1080-derived Extracellular Vesicles with Reporter Molecule: Custom #1)

(Culture of Human Fibrosarcoma Cells)

Human fibrosarcoma HT1080 cells (KAC Co., Ltd.) were cultured in three kinds of cell culture media described later. In addition, 100 mm cell culture Dish (Nunclon Delta Surface) (manufactured by Thermo Fisher Scientific Inc.) was used as a cell culture container.

The first culture medium is a Minimum Essential Medium Eagle (manufactured by MP Biomedicals, LLC) supplemented with 1% of a penicillin-streptomycin solution (×100) (manufactured by FUJIFILM Wako Pure Chemical Corporation), 1% of a MEM non-essential amino acid solution (×100) (manufactured by FUJIFILM Wako Pure Chemical Corporation), and 1% of a 200 mmol/L L-glutamine solution (×100) (manufactured by FUJIFILM Wako Pure Chemical Corporation) (hereinafter referred to as "MEM").

The second culture medium is a MEM supplemented with 10% of Exo-FBS Exosome-Depleted FBS (manufactured by System Biosciences, LLC) (hereinafter referred to as "Exo-FBS").

The third culture medium is Cell Growth Medium No. 104 (E-MEM+NEAA+10% FBS) (manufactured by KAC Co., Ltd.) (hereinafter referred to as "FBS").

In the case of culture in the Exo-FBS or FBS culture medium, the suspension of the cells was seeded into a dish and incubated at 37°C in 5% $CO_2$ for several days until 50% or more of the culture surface area of the dish was covered with the cells.

In the case of culture in the MEM culture medium, the cells were incubated in advance in the FBS culture medium at 37°C in 5% $CO_2$ for several days until 50% or more of the culture surface area of the dish was covered with the cells. After that, the culture supernatant was removed, PBS was added to the dish and spread over the entire surface, and the PBS was then removed. The MEM culture medium was added to this dish, and the cells were incubated at 37°C in 5% $CO_2$ for 48 hours.

(Recovery of Culture Supernatant)

Each of the culture supernatants was recovered from each of the dishes in a centrifuge tube, and centrifuged at 2,000×g for 10 minutes at 4°C. Each of the supernatants after the centrifugation was recovered in a fresh container with a small amount of the culture medium remaining so as not to incorporate the precipitated cells. Each of the culture supernatants was filtered through a syringe with a 0.22 μm filter (manufactured by Merck KGaA) so that contaminants such as cell fragments were removed. After that, the filtrate was recovered in a fresh container, and stored at 4°C.

(Preparation of Sample)

From each of the HT1080 culture supernatants described in the section "(Recovery of Culture Supernatant)", 100 μL of each of the supernatants after centrifugation and 100 μL of each of the supernatants after filtration through a 0.22 μm filter were each aliquoted, and the buffer (4) was added to the aliquots to provide 1 mL of solutions. The concentration of each of the solutions is 1/10 of the concentration of each of the HT1080 culture supernatants. Further, 100 μL of each of 3 kinds of culture media where the HT1080 cells were not cultured was aliquoted, and the buffer (4) was added to the aliquot to provide 1 mL of a solution.

In addition, for detection of MMP14 activity, 1 mM Custom #1 was diluted with the buffer (4) to prepare a 5 μM MMP14 detection solution.

50 μL of the MMP14 detection solution was added to 50 μL of each of the samples, a reaction was performed at 30°C, and MMP14 activity was evaluated. In this case, the concentrations of each of the HT1080 culture supernatants and each of the culture medium samples are 1/20 of the original concentrations, and the concentration of the reporter molecule in each of the reaction solutions is 2.5 μM.

In the HT1080 culture supernatants with the MEM, the activity was low, and hence a sample obtained without dilution of the culture supernatant was also investigated. The concentration of the sample is 1/2 of the original concentration. This concentration is 10 times the concentration of each of the other samples, and is hence referred to as "x10" in Table.

(Evaluation of MMP Activity with 96-well Plate)

Evaluation was performed in the same manner as in Example 1.

Table 62 shows changes in fluorescence intensity after 2 hours from Custom #1 in the HT1080 culture supernatants and the culture media. As shown in Table 62, it is found that the fluorescence intensity is changed by the HT1080 culture supernatants and the culture medium samples.

[Table 62]

Table 62

| Culture medium | HT1080 culture | Treatment (experiment conditions) | Fluorescence change (ΔRFU) |
|---|---|---|---|
| FBS | Present | Only centrifugation (I) | 64,773 |
| | | Centrifugation+filtration (II) | 65,200 |
| | Absent | None (III) | 66,945 |
| Exo-FBS | Present | Only centrifugation (IV) | 46,172 |
| | | Centrifugation+filtration (V) | 45,429 |
| | Absent | None (VI) | 50,117 |
| MEM | Present | Only centrifugation (VII) | -240 |
| | | Centrifugation+filtration (VIII) | 186 |
| | Absent | None (IX) | -311 |

(continued)

| Culture medium | HT1080 culture | Treatment (experiment conditions) | Fluorescence change (ΔRFU) |
|---|---|---|---|
| MEM (x10) | Present | Only centrifugation | 2,494 |
| | | Centrifugation+filtration | 1,515 |

Example 55

(Wells and Well Array)

A well array of Simoa Discs was used.

(Preparation of Reaction Solution for Wells)

A reaction solution for filling the wells 204 was prepared in the same manner as in Example 54.

In addition, for detection of MMP14 activity, 1 mM Custom #1 was diluted with the buffer (4) to prepare a MMP14 detection solution containing 5 μM Custom #1 and 2 μM Alexa Fluor 647.

50 μL of the MMP14 detection solution was added to 50 μL of each of the samples to provide a reaction solution for wells.

In this case, the concentrations of the HT1080 culture supernatants and the culture medium samples are 1/20 of the original concentrations, and the concentrations of the reporter molecule and the standard fluorescent substance in each of the reaction solutions are 2.5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, fluorescence microscope images each containing both of the fluorescence of 5-FAM derived from Custom #1 and the background fluorescence of Custom #1 after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the HT1080 culture supernatants and the culture medium samples worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 63. Further, the concentration of a MMP14 active component in each of the HT1080 culture supernatants and each of the culture medium samples calculated from λ at a concentration of x1 and the volume of the microcompartment that is 50 fL is shown in Table 64. In this case, the experiment conditions correspond to from (I) to (IX) of Example 54.

[Table 63]

Table 63

| Experiment condition | Reaction (hr) | Number of wells | Number of positives | Positive ratio | Expected value λ |
|---|---|---|---|---|---|
| (I) | 4 | 28,080 | 16,696 | 0.59459 | 0.90285 |
| | 24 | 28,080 | 17,091 | 0.60865 | 0.93816 |
| (II) | 4 | 28,080 | 14,954 | 0.53255 | 0.76046 |
| | 24 | 27,975 | 15,051 | 0.53802 | 0.77229 |
| (III) | 4 | 28,080 | 13,549 | 0.48251 | 0.65877 |
| | 24 | 28,002 | 13,900 | 0.49639 | 0.68596 |
| (IV) | 4 | 28,080 | 11,105 | 0.39548 | 0.50332 |
| | 24 | 28,002 | 10,939 | 0.39065 | 0.49536 |
| (V) | 4 | 28,080 | 12,180 | 0.43376 | 0.56874 |
| | 24 | 28,080 | 12,313 | 0.4385 | 0.57714 |
| (VI) | 4 | 28,077 | 12,998 | 0.46294 | 0.62165 |
| | 24 | 28,080 | 12,698 | 0.45221 | 0.60186 |
| (VII) | 4 | 28,080 | 94 | 0.00335 | 0.00335 |
| | 24 | 28,002 | 280 | 0.01005 | 0.01005 |
| (VIII) | 4 | 28,080 | 144 | 0.00513 | 0.00514 |
| | 24 | 28,080 | 309 | 0.01107 | 0.01107 |
| (IX) | 4 | 28,002 | 3 | 0.00011 | 0.00011 |
| | 24 | 28,002 | 4 | 0.00014 | 0.00014 |

[Table 64]

Table 64

| Experiment condition | Reaction (hr) | Expected value λ | MMP14 active component concentration (×$10^{11}$/mL) |
|---|---|---|---|
| (I) | 4 | 0.90285 | 3.61139 |
| | 24 | 0.93816 | 3.75265 |
| (II) | 4 | 0.76046 | 3.04185 |
| | 24 | 0.77223 | 3.0889 |
| (III) | 4 | 0.65877 | 2.63509 |
| | 24 | 0.68596 | 2.74384 |
| (IV) | 4 | 0.50332 | 2.01326 |
| | 24 | 0.49536 | 1.98145 |
| (V) | 4 | 0.56874 | 2.27495 |
| | 24 | 0.57714 | 2.30855 |
| (VI) | 4 | 0.62165 | 2.48659 |
| | 24 | 0.60186 | 2.40744 |
| (VII) | 4 | 0.00335 | 0.01341 |
| | 24 | 0.01005 | 0.0402 |
| (VIII) | 4 | 0.00514 | 0.02057 |
| | 24 | 0.01107 | 0.04426 |

(continued)

| Experiment condition | Reaction (hr) | Expected value λ | MMP14 active component concentration (×$10^{11}$/mL) |
|---|---|---|---|
| (IX) | 4 | 0.00011 | 0.00043 |
| | 24 | 0.00014 | 0.00057 |

Accordingly, it was shown that digital detection of the MMP14 active component was able to be performed through use of MMP14 activity in reporter molecules. In this case, a high concentration of the MMP14 active component was also detected in each of the FBS-containing culture medium (III) and the Exo-FBS-containing culture medium (VI) where the HT1080 cells were not cultured. In contrast, a slightly small amount of the MMP14 active component was only detected in the culture medium (IX) containing no FBS. Accordingly, it is found that, when a test using a cultured cell supernatant is performed, the MEM is preferably used as the culture medium in order to eliminate the influence of FBS.

In addition, the MMP14 active component can be recovered in (VII) and (VIII) at about two orders of magnitude higher than in (IX). It is presumed that this component includes extracellular vesicles and other components released from the HT1080 cells to the culture supernatant.

In the experiment system of Example 55, the culture supernatant was used as it was as the sample, recovery based on the features of extracellular vesicles was not performed, and hence the description is limited to the term "MMP14 active component."

Example 56

(Detection of MMP14 Activity of HT1080-derived Extracellular Vesicles with Reporter Molecule: Custom #1)

(Culture of Human Fibrosarcoma Cells and Recovery of Culture Supernatant)

HT1080 cells were cultured in each of the Exo-FBS and the MEM, and 3 mL of each of the culture supernatants was recovered and then stored at 4°C, in the same manner as in Example 54.

(Recovery of Extracellular Vesicles with exoEasy Maxi Kit (manufactured by QIAGEN N.V.) and Preparation of Sample)

Extracellular vesicles were recovered from 3 mL of each of the recovered culture supernatants with exoEasy Maxi Kit. In this case, the recovery was performed according to the protocol included in the kit. From 3 mL of each of the culture supernatants, 450 μL of each of recovered liquids was obtained. Each of the recovered liquids has a concentration 6.67 times the original concentration of each of the culture supernatants. Each of the recovered liquids was diluted 66.7-fold to prepare 100 μL of a sample at 1/10 of the original concentration of each of the culture supernatants.

In addition, for detection of MMP14 activity, 1 mM Custom #1 was diluted with the buffer (4) to prepare a 5 μM MMP14 detection solution.

50 μL of the MMP14 detection solution was added to 50 μL of each of the samples, a reaction was performed at 30°C, and MMP14 activity was evaluated. In this case, the concentration of each of the samples is 1/20 of the original concentration in each of the culture supernatants, and the concentration of the reporter molecule in each of the reaction solutions is 2.5 μM.

(Evaluation of MMP Activity with 96-well Plate)

Evaluation was performed in the same manner as in Example 1.

Table 65 shows changes in fluorescence intensity after 2 hours from Custom #1 in the exoEasy recovered liquid. As shown in Table 65, the fluorescence intensity was changed in the exoEasy recovered liquid from the Exo-FBS, but no change in fluorescence intensity was recognized in the exoEasy recovered liquid from the MEM.

[Table 65]

Table 65

| Culture medium | HT1080 culture | Fluorescence change (ΔRFU) |
|---|---|---|
| Exo-FBS | Present | 6,663 |
| MEM | Present | -313 |

Example 57

(Wells and Well Array)

A well array of Simoa Discs was used.

(Preparation of Reaction Solution for Wells)

A reaction solution for filling the wells 204 was prepared in the same manner as in Example 56.

In addition, for detection of MMP14 activity, 1 mM Custom #1 was diluted with the buffer (4) to prepare a MMP14 detection solution containing 5 μM Custom #1 and 2 μM Alexa Fluor 647.

50 μL of the MMP14 detection solution was added to 50 μL of each of the samples to provide a reaction solution for wells.

In this case, the concentration of each of the samples is 1/20 of the original concentration in each of the culture supernatants, and the concentrations of the reporter molecule and the standard fluorescent substance in each of the reaction solutions are 2.5 μM and 1 μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, fluorescence microscope images each containing both of the fluorescence of 5-FAM derived from Custom #1 and the background fluorescence of Custom #1 after 4 hours and 24 hours of reaction, and fluorescence microscope images each containing the fluorescence of the standard fluorescent substance after 4 hours and 24 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the sample worked in the microcompartments.

Next, although the illustration is omitted, the fluorescence microscope images after 4 hours and 24 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected values (λ) calculated with respect to the concentration of the extracellular vesicles subjected to the test through use of the above-mentioned equation (1) from the total number of wells and the number of positive wells identified from each of the histograms after 4 hours and 24 hours of reaction are shown in Table 66. Further, the concentration of a MMP14 active component in each of the HT1080 culture supernatants recovered with the exoEasy, the concentration being calculated from λ at a concentration of x1 and the volume of the microcompartment that is 50 fL, is shown in Table 67.

[Table 66]

Table 66

| Culture medium | Reaction (hr) | Number of wells | Number of positives | Positive ratio | Expected value λ |
|---|---|---|---|---|---|
| Exo-FBS | 4 | 28,080 | 8,245 | 0.29363 | 0.34761 |
| | 24 | 28,080 | 11,272 | 0.40142 | 0.5132 |
| MEM | 4 | 28,080 | 132 | 0.0047 | 0.00471 |
| | 24 | 27,975 | 308 | 0.01101 | 0.01107 |

[Table 67]

Table 67

| Experiment condition | Reaction (hr) | Expected value λ | MMP14 extracellular vesicle concentration ($\times 10^{11}$/mL) |
|---|---|---|---|
| Exo-FBS | 4 | 0.34761 | 1.39044 |
| | 24 | 0.5132 | 2.05281 |
| MEM | 4 | 0.00471 | 0.01885 |
| | 24 | 0.01107 | 0.04428 |

It was found that extracellular vesicles having MMP14 activity were able to be recovered from each of the HT1080 culture supernatants with exoEasy. Even in the MEM culture supernatant in which no activity had been able to be detected in the bulk test of Example 56, quantification was able to be performed in a digital test.

Example 58

(Detection of MMP14 Activity of HT1080-derived Extracellular Vesicles with Reporter Molecule: Custom #1)

(Culture of Human Fibrosarcoma Cells and Recovery of Culture Supernatant)

HT1080 cells were cultured in each of the Exo-FBS and the MEM, and 3 mL of each of the culture supernatants was recovered and then stored at 4°C, in the same manner as in Example 54.

(Recovery of Extracellular Vesicles with qEV (manufactured by Izon Science Limited) and Preparation of Sample)

Extracellular vesicles were recovered from 3 mL of each of the recovered culture supernatants with qEV. In this case, the recovery was performed according to the protocol included in the kit. From 3 mL of each of the culture supernatants, 700 μL of each of fraction recovered liquids in series was obtained. Each of the recovered liquids was used as a sample as it was.

In addition, for detection of MMP14 activity, 1 mM Custom #1 was diluted with the buffer (4) to prepare a 5 μM MMP14 detection solution.

50 μL of the MMP14 detection solution was added to 50 μL of each of the samples, a reaction was performed at 30°C, and MMP14 activity was evaluated. In this case, the concentration of each of the samples is 1/2 of the original concentration in each of the recovered liquids, and the concentration of the reporter molecule in each of the reaction solutions is 2.5 μM.

(Evaluation of MMP Activity with 96-well Plate)

Evaluation was performed in the same manner as in Example 1.

Table 68 shows changes in fluorescence intensity after 2 hours from Custom #1 in the qEV recovered liquid. As shown in Table 68, a change in fluorescence intensity in each of the qEV recovered liquids was recognized.

[Table 68]

Table 68

| Culture medium | HT1080 culture | Fraction # | Fluorescence change (ΔRFU) |
|---|---|---|---|
| Exo-FBS | Present | 1 | 336 |
| | | 2 | 318 |
| | | 3 | 271 |
| | | 4 | 285 |
| | | 5 | 302 |
| | | 6 | 325 |
| | | 7 | 1,869 |
| | | 8 | 7,249 |
| | | 9 | 9,417 |
| | | 10 | 17,731 |
| | | 11 | 30,226 |
| | | 12 | 44,826 |
| | | 13 | 61,704 |
| | | 14 | 76,810 |
| MEM | Present | 1 | 245 |
| | | 2 | 285 |
| | | 3 | 245 |
| | | 4 | 304 |
| | | 5 | 1,450 |
| | | 6 | 697 |
| | | 7 | 10,198 |
| | | 8 | 11,966 |
| | | 9 | 1,424 |
| | | 10 | 550 |
| | | 11 | 506 |
| | | 12 | 536 |
| | | 13 | 489 |
| | | 14 | 446 |

Example 59

(Wells and Well Array)

A well array of Simoa Discs was used.

(Preparation of Reaction Solution for Wells)

A reaction solution for filling the wells 204 was prepared in the same manner as in Example 58.

In addition, for detection of MMP14 activity, 1 mM Custom #1 was diluted with the buffer (4) to prepare a MMP14 detection solution containing 5 μM Custom #1 and 2 μM Alexa Fluor 647.

50 μL of the MMP14 detection solution was added to 50 μL of each of the samples to provide a reaction solution for wells.

In this case, the concentration of each of the samples is 1/2 of the original concentration in each of the recovered liquids, and the concentrations of the reporter molecule and the standard fluorescent substance in each of the reaction solutions are 2.5 μM and 1μM, respectively.

(Filling of Wells with Reaction Solution)

A fluorinated oil SR-X Sealing Oil was used as a hydrophobic solvent and wells were filled with each of the reaction solutions in the same manner as in Example 19.

(Fluorescence Microscope Observation)

The well array prepared in the foregoing was subjected to a reaction through incubation at 30°C to promote the generation of fluorescence. After that, observation was performed with a fluorescence microscope (BZ-X800).

The observation of fluorescence derived from each fluorescent substance was performed under the same conditions as in Example 19 to acquire a fluorescence image.

Although the illustration is omitted, a fluorescence microscope image containing both of the fluorescence of 5-FAM derived from Custom #1 and the background fluorescence of Custom #1 after 4 hours of reaction, and a fluorescence microscope image containing the fluorescence of the standard fluorescent substance after 4 hours of reaction were acquired.

The wells were able to be recognized based on the fluorescence of the standard fluorescent substance. In addition, it was found that, in the wells in which the fluorescence of 5-FAM was observed, the fluorescence of the standard fluorescent substance was also observed. Thus, it was able to be recognized that the wells were filled with the sample and the reagent containing the reporter molecule, and a cleavage reaction of Custom #1 by MMP14 in the sample worked in the microcompartments.

Next, although the illustration is omitted, the fluorescence microscope images after 4 hours of reaction of Example described above were subjected to predetermined image processing to provide histograms. Those histograms each show the number of wells each showing a fluorescence intensity included in each fraction of fluorescence intensity obtained by dividing fluorescence intensities into intervals of a constant value.

The expected value (λ) calculated with respect to the concentration of the extracellular vesicles subjected to the test through use of the above-mentioned equation (1) from the total number of wells and the number of positive wells identified from each of the histograms after 4 hours of reaction is shown in Table 69. Further, the concentration of a MMP14 active component in each of the qEV recovered liquids calculated from λ at a concentration of x1 and the volume of the microcompartment that is 50 fL is shown in Table 70.

[Table 69]

Table 69

| Culture medium | Fraction # | Number of wells | Number of positives | Positive ratio | Expected value λ |
|---|---|---|---|---|---|
| Exo-FBS | 8 | 27,975 | 7,164 | 0.25609 | 0.29583 |
| | 9 | 27,975 | 14,130 | 0.50509 | 0.70339 |
| | 10 | 28,029 | 21,264 | 0.75864 | 1.42148 |
| | 11 | 28,080 | 21,512 | 0.7661 | 1.45285 |
| | 12 | 28,080 | 21,591 | 0.76891 | 1.46495 |
| | 13 | 28,080 | 20,505 | 0.73024 | 1.3102 |
| | 14 | 28,015 | 24,077 | 0.85943 | 1.96207 |
| | 15 | 28,080 | 26,856 | 0.95641 | 3.13293 |

(continued)

| Culture medium | Fraction # | Number of wells | Number of positives | Positive ratio | Expected value λ |
|---|---|---|---|---|---|
| MEM | 7 | 28,080 | 9,216 | 0.32821 | 0.3978 |
| | 8 | 28,047 | 4,791 | 0.17082 | 0.18732 |
| | 9 | 28,046 | 4,129 | 0.14722 | 0.15926 |
| | 10 | 28,080 | 490 | 0.01745 | 0.0176 |
| | 11 | 28,046 | 308 | 0.01098 | 0.01104 |
| | 12 | 28,080 | 324 | 0.01154 | 0.01161 |
| | 13 | 28,080 | 678 | 0.02415 | 0.02444 |
| | 14 | 28,080 | 21 | 0.00075 | 0.00075 |

[Table 70]

Table 70

| Culture medium | Fraction # | Expected value λ | MMP14 extracellular vesicle concentration ($\times 10^{10}$/mL) |
|---|---|---|---|
| Exo-FBS | 8 | 0.29583 | 1.1833 |
| | 9 | 0.70339 | 2.8135 |
| | 10 | 1.42148 | 5.6859 |
| | 11 | 1.45285 | 5.8114 |
| | 12 | 1.46495 | 5.8598 |
| | 13 | 1.3102 | 5.2408 |
| | 14 | 1.96207 | 7.8483 |
| | 15 | 3.13293 | 12.532 |
| MEM | 7 | 0.3978 | 1.5912 |
| | 8 | 0.18732 | 0.7493 |
| | 9 | 0.15926 | 0.637 |
| | 10 | 0.0176 | 0.0704 |
| | 11 | 0.01104 | 0.0442 |
| | 12 | 0.01161 | 0.0464 |
| | 13 | 0.02444 | 0.0978 |
| | 14 | 0.00075 | 0.003 |

It was found that extracellular vesicles having MMP14 activity were able to be recovered from the HT1080 culture supernatant with qEV. The fractions immediately after the void volume of the column correspond to Fraction #8 and Fraction #9 of the Exo-FBS, and Fraction #7 and Fraction #8 of the MEM. The volume of each of the fractions is 0.7 mL, and hence the amounts of the extracellular vesicles in the respective fractions are $0.82831 \times 10^{10}$ and $1.96945 \times 10^{10}$ in Fraction #8 and Fraction #9 of the Exo-FBS, respectively. The extracellular vesicles are recovered from 3 mL of the culture supernatant, and hence the original concentration in the culture supernatant is $9.3259 \times 10^{9}$/mL. Similarly, the amounts of the extracellular vesicles in Fraction #7 and Fraction #8 of the MEM are $1.11384 \times 10^{10}$ and $0.52451 \times 10^{10}$, respectively. The extracellular vesicles are recovered from 3 mL of the culture supernatant, and hence the original concentration in the culture supernatant is $5.4612 \times 10^{9}$/mL.

When the MMP14 active component concentration in each of the fractions is considered, in the case of the MEM, the concentration of the fraction immediately after the void in which the extracellular vesicles are to be eluted is high, and the concentration of the subsequent fraction is one order of magnitude lower. From the principle of size-exclusion chromatography, it is presumed that the extracellular vesicles are a main component in the MEM. Accordingly, it is conceivable that the concentration of the extracellular vesicles having MMP14 activity is $5.4612 \times 10^9$/mL. Meanwhile, in the case of the Exo-FBS, as the elution becomes slower, the concentration of the MMP14 active component is increased. This means that the MMP14 active component, which has a size smaller than those of the extracellular vesicles, accounts for a large proportion of the culture supernatant. From the results, when the use of the culture supernatant is investigated, the use of the MEM is preferred, and when the Exo-FBS is used, the use of separation and purification means for extracellular vesicles, such as size-exclusion chromatography, is preferred.

Consequently, it was found that extracellular vesicles having MMP14 activity were able to be recovered from the HT1080 culture supernatant with qEV and digital quantification was able to be performed.

The present invention is not limited to the embodiments described above, and various changes and modifications may be made without departing from the spirit and scope of the present invention. Accordingly, the following claims are appended hereto in order to make the scope of the present invention public.

The present application claims priority based on Japanese Patent Application No. 2022-195994 filed on December 7, 2022, Japanese Patent Application No. 2023-111738 filed on July 6, 2023, and Japanese Patent Application No. 2023-203313 filed on November 30, 2023, the entire contents thereof are incorporated herein by reference.

[Reference Signs List]

| | |
|---|---|
| 10, 20 | extracellular vesicle detection apparatus |
| 101 | distribution unit |
| 102 | signal generation unit |
| 103 | signal detection unit |
| 104 | identification unit |
| 105 | extraction unit |
| 106 | determination unit |
| 107 | judgment unit |
| 108 | calculation unit |
| 109 | display unit |
| 110 | storage unit |
| 111 | image acquisition unit |
| 200 | well array |

## Claims

**1.** A method of detecting an extracellular vesicle having a target enzyme, the method comprising:

a distribution step of distributing an extracellular vesicle and a reagent containing a reporter molecule to be modified by the target enzyme to emit a signal into a plurality of individual separated compartments;
a signal generation step of allowing the target enzyme and the reporter molecule to react with each other to generate a signal;
a signal detection step of detecting the signal; and
an identification step including determining, based on a detection result obtained in the signal detection step, a signal intensity of each of the individual separated compartments, and identifying each of the individual separated compartments having a signal intensity exceeding a predetermined threshold value.

**2.** The method of detecting an extracellular vesicle according to claim 1, wherein the signal detection step includes an image acquisition step of acquiring an image containing the individual separated compartments.

**3.** The method of detecting an extracellular vesicle according to claim 2, wherein the identification step includes identifying each of the individual separated compartments having a signal intensity exceeding the predetermined threshold value by processing the image acquired in the image acquisition step.

**4.** The method of detecting an extracellular vesicle according to any one of claims 1 to 3, wherein the reporter molecule is labeled.

**5.** The method of detecting an extracellular vesicle according to any one of claims 1 to 4, wherein the reporter molecule is labeled with a fluorescent substance.

6. The method of detecting an extracellular vesicle according to any one of claims 1 to 5, wherein the reporter molecule is labeled with a quencher and a fluorescent substance.

7. The method of detecting an extracellular vesicle according to any one of claims 1 to 6, wherein the reporter molecule is a molecule that reacts with any one selected from the group consisting of: a hydrolase; an oxidoreductase; a transferase; and an isomerase to emit a signal.

8. The method of detecting an extracellular vesicle according to any one of claims 1 to 7, wherein the reporter molecule is a molecule that reacts with any one selected from the group consisting of: a protease; an esterase; a glycosidase; an oxidase; a peroxidase; a catalase; a superoxide dismutase; an acetylase and a deacetylase; a kinase; a protein kinase; a glycosyltransferase; a cis-trans isomerase; a racemase; and a mutase to emit a signal.

9. The method of detecting an extracellular vesicle according to any one of claims 1 to 8, wherein the reporter molecule is a molecule that reacts with a molecule belonging to a MMP family to emit a signal.

10. The method of detecting an extracellular vesicle according to any one of claims 1 to 9, wherein the reporter molecule is a molecule that reacts with MMP14 to emit a signal.

11. The method of detecting an extracellular vesicle according to any one of claims 1 to 8, wherein the reporter molecule is a molecule that reacts with AChE to emit a signal.

12. The method of detecting an extracellular vesicle according to any one of claims 1 to 11, further comprising a step of mixing the extracellular vesicle and a capture protein to be bound to the extracellular vesicle to form a complex.

13. The method of detecting an extracellular vesicle according to claim 12, wherein the capture protein is to be bound to a membrane protein of the extracellular vesicle.

14. The method of detecting an extracellular vesicle according to claim 12 or 13, wherein the capture protein is any one selected from the group consisting of: an anti-CD9 antibody; an anti-CD63 antibody; an anti-CD81 antibody; an anti-Flotillin antibody; an anti-ALIX antibody; an anti-Syntenin antibody; an anti-TSG101 antibody; an anti-HSP70 antibody; an anti-HSP90 antibody; an anti-SNARE antibody; an anti-Rab antibody; an anti-Annexin antibody; an anti-MMP antibody; an anti-ADAM antibody; an anti-AChE antibody; an anti-integrin antibody; an anti-selectin antibody; and an anti-EpCAM antibody.

15. The method of detecting an extracellular vesicle according to any one of claims 12 to 14, wherein the capture protein is labeled.

16. The method of detecting an extracellular vesicle according to any one of claims 12 to 15, wherein the capture protein is labeled with a particle.

17. The method of detecting an extracellular vesicle according to claim 16, wherein the particle has a particle diameter of 1 μm or more and 10 μm or less.

18. The method of detecting an extracellular vesicle according to any one of claims 1 to 16, further comprising a recovery step including mixing the extracellular vesicle and a capture protein to be bound to the extracellular vesicle to form a complex, and recovering the formed complex.

19. The method of detecting an extracellular vesicle according to claim 18, further comprising a step of mixing the complex and a second capture protein to be bound to the extracellular vesicle.

20. The method of detecting an extracellular vesicle according to any one of claims 1 to 19, wherein the reagent contains two or more kinds of reporter molecules.

21. The method of detecting an extracellular vesicle according to claim 20, wherein the two or more kinds of reporter molecules each generate a different signal.

**22.** The method of detecting an extracellular vesicle according to claim 21, wherein the signal detection step includes separately detecting a signal intensity of a signal emitted from each of the two or more kinds of reporter molecules.

**23.** The method of detecting an extracellular vesicle according to any one of claims 1 to 22, further comprising a step of staining at least any one of a membrane or a protein of the extracellular vesicle.

**24.** The method of detecting an extracellular vesicle according to any one of claims 1 to 23, wherein the identification step includes identifying, based on at least any one of a ratio or a difference between a signal intensity of a reference compartment and a signal intensity of each of the individual separated compartments, each of the individual separated compartments having a signal intensity exceeding the predetermined threshold value.

**25.** The method of detecting an extracellular vesicle according to claim 24, wherein the reference compartment is a compartment free of the extracellular vesicle and including a sample containing a reporter molecule.

**26.** The method of detecting an extracellular vesicle according to any one of claims 1 to 25, wherein each of the individual separated compartments is a liquid droplet.

**27.** The method of detecting an extracellular vesicle according to any one of claims 1 to 26, wherein each of the individual separated compartments is a well.

**28.** The method of detecting an extracellular vesicle according to any one of claims 1 to 27, wherein each of the individual separated compartments has a volume of 0.1 fL or more and 1,400 fL or less.

**29.** A reagent kit for detecting an extracellular vesicle having a target enzyme in an individual separated compartment, the reagent kit comprising:

a reagent containing a reporter molecule to be modified by the target enzyme to emit a signal; and
a container for providing a plurality of individual separated compartments.

**30.** An apparatus for detecting an extracellular vesicle having a target enzyme, the apparatus comprising:

a distribution unit configured to distribute an extracellular vesicle and a reagent containing a reporter molecule to be modified by the target enzyme to emit a signal into a plurality of individual separated compartments;
a signal generation unit configured to allow the target enzyme and the reporter molecule to react with each other to generate a signal;
a signal detection unit configured to detect the signal; and
an identification unit configured to determine, based on a detection result obtained with the signal detection unit, a signal intensity of each of the individual separated compartments, and identify each of the individual separated compartments having a signal intensity exceeding a predetermined threshold value.

**31.** A program for causing a computer included in an apparatus for detecting an extracellular vesicle to execute the method of detecting an extracellular vesicle of any one of claims 1 to 28 so as to cause the apparatus for detecting an extracellular vesicle to execute the method.

FIG. 1

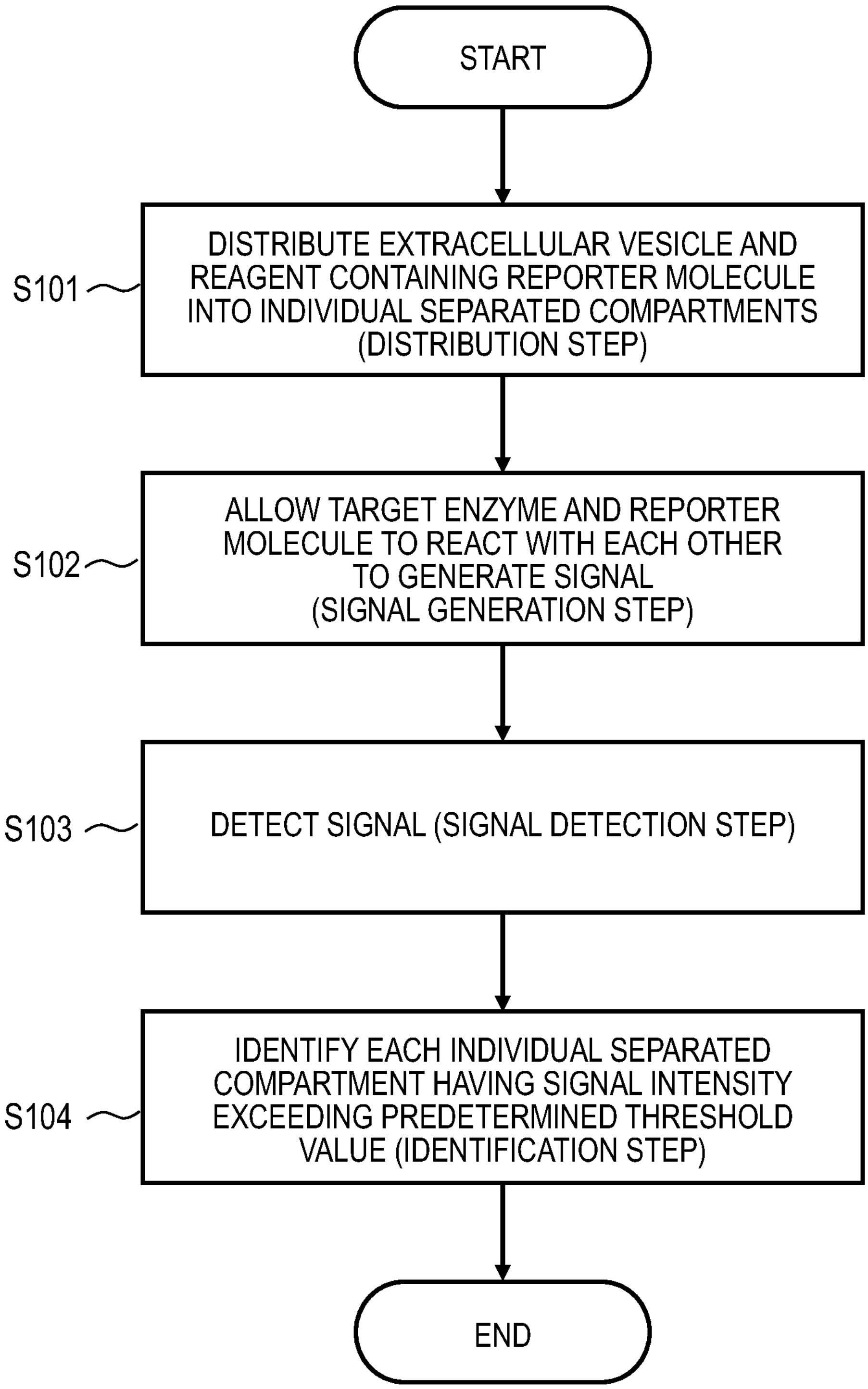
START
S101
DISTRIBUTE EXTRACELLULAR VESICLE AND REAGENT CONTAINING REPORTER MOLECULE INTO INDIVIDUAL SEPARATED COMPARTMENTS (DISTRIBUTION STEP)
S102
ALLOW TARGET ENZYME AND REPORTER MOLECULE TO REACT WITH EACH OTHER TO GENERATE SIGNAL (SIGNAL GENERATION STEP)
S103
DETECT SIGNAL (SIGNAL DETECTION STEP)
S104
IDENTIFY EACH INDIVIDUAL SEPARATED COMPARTMENT HAVING SIGNAL INTENSITY EXCEEDING PREDETERMINED THRESHOLD VALUE (IDENTIFICATION STEP)
END

FIG. 2

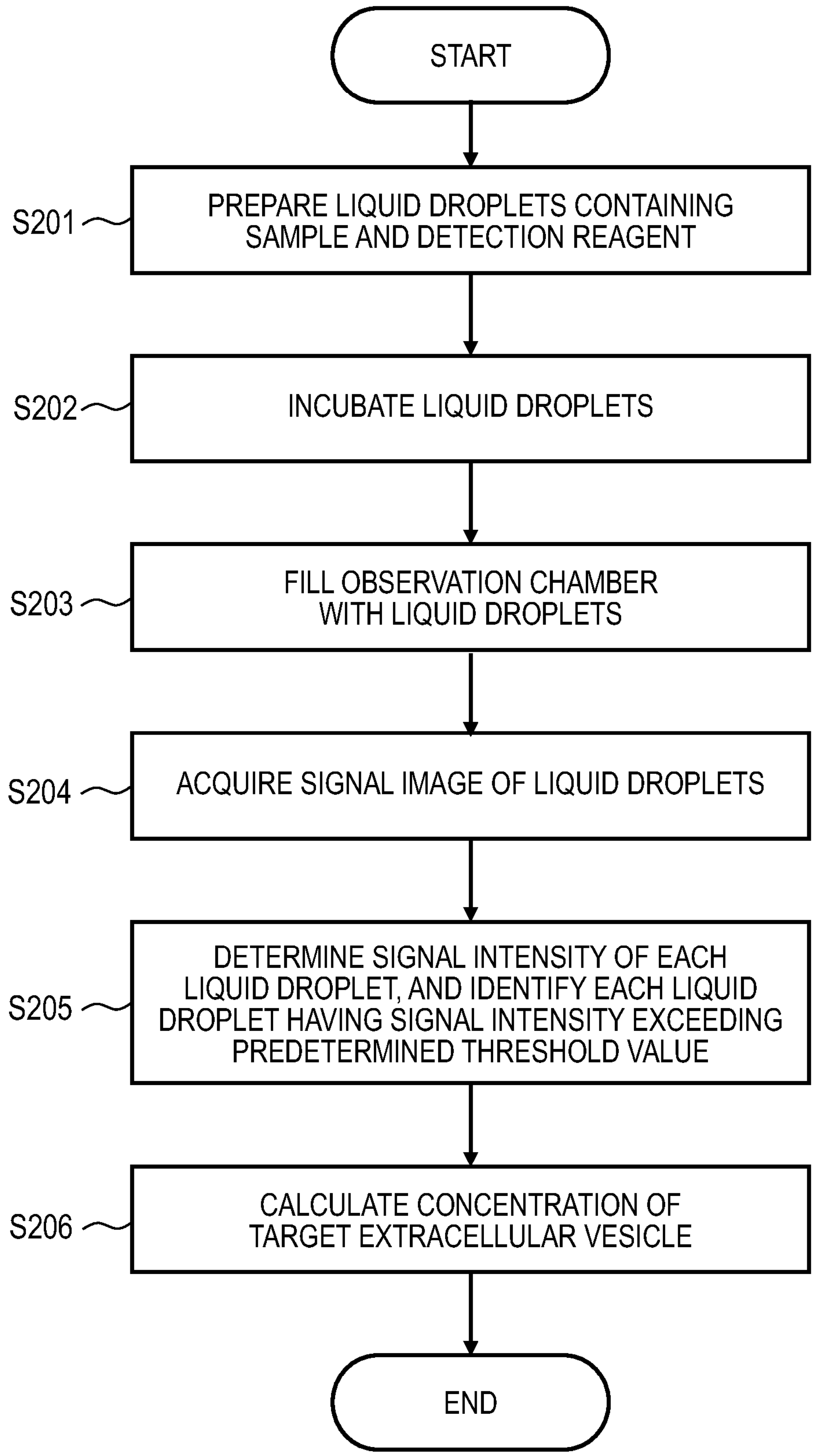
START
S201
PREPARE LIQUID DROPLETS CONTAINING SAMPLE AND DETECTION REAGENT
S202
INCUBATE LIQUID DROPLETS
S203
FILL OBSERVATION CHAMBER WITH LIQUID DROPLETS
S204
ACQUIRE SIGNAL IMAGE OF LIQUID DROPLETS
S205
DETERMINE SIGNAL INTENSITY OF EACH LIQUID DROPLET, AND IDENTIFY EACH LIQUID DROPLET HAVING SIGNAL INTENSITY EXCEEDING PREDETERMINED THRESHOLD VALUE
S206
CALCULATE CONCENTRATION OF TARGET EXTRACELLULAR VESICLE
END

FIG. 3

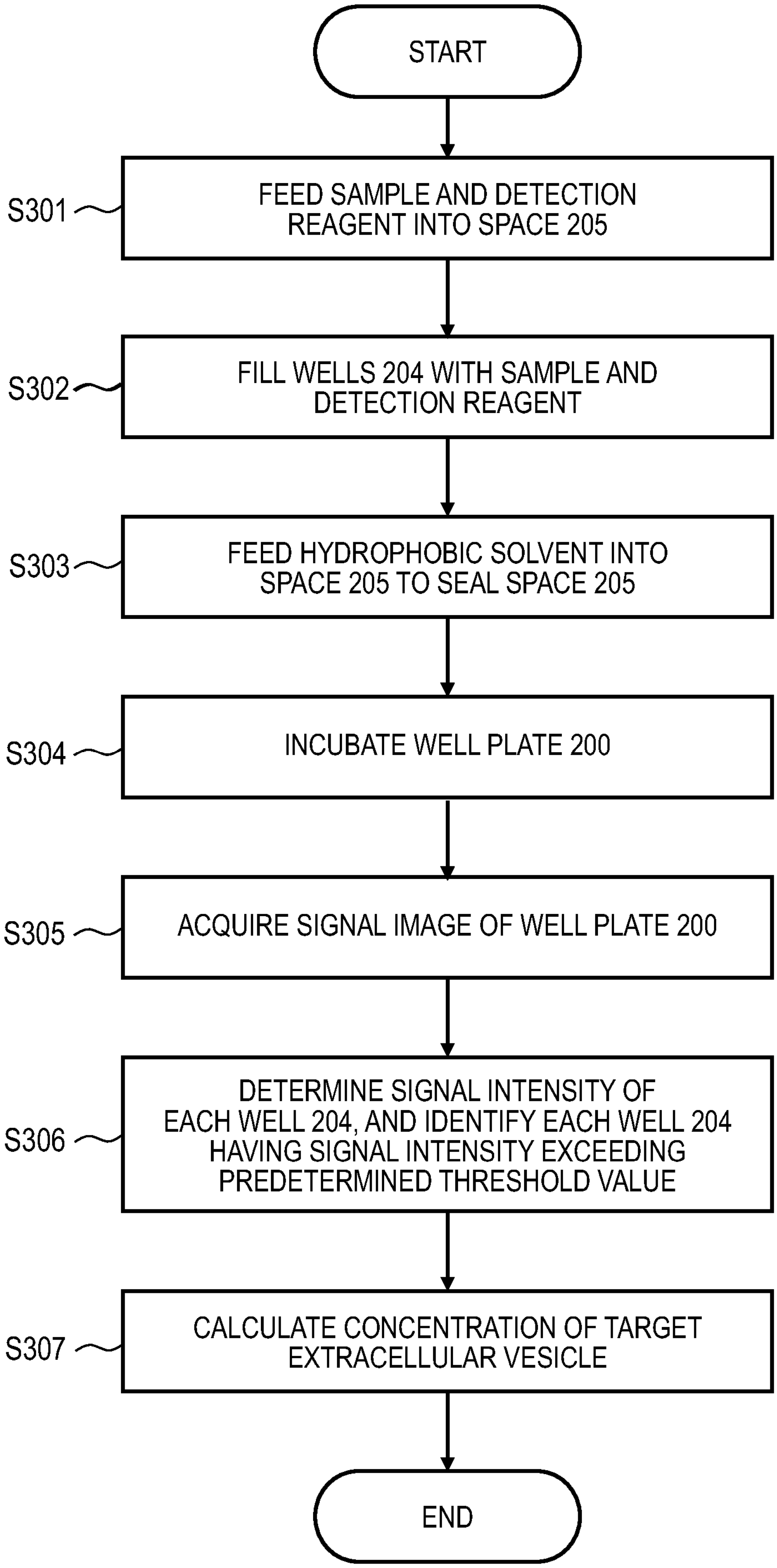
START
S301
FEED SAMPLE AND DETECTION REAGENT INTO SPACE 205
S302
FILL WELLS 204 WITH SAMPLE AND DETECTION REAGENT
S303
FEED HYDROPHOBIC SOLVENT INTO SPACE 205 TO SEAL SPACE 205
S304
INCUBATE WELL PLATE 200
S305
ACQUIRE SIGNAL IMAGE OF WELL PLATE 200
S306
DETERMINE SIGNAL INTENSITY OF EACH WELL 204, AND IDENTIFY EACH WELL 204 HAVING SIGNAL INTENSITY EXCEEDING PREDETERMINED THRESHOLD VALUE
S307
CALCULATE CONCENTRATION OF TARGET EXTRACELLULAR VESICLE
END

FIG. 4

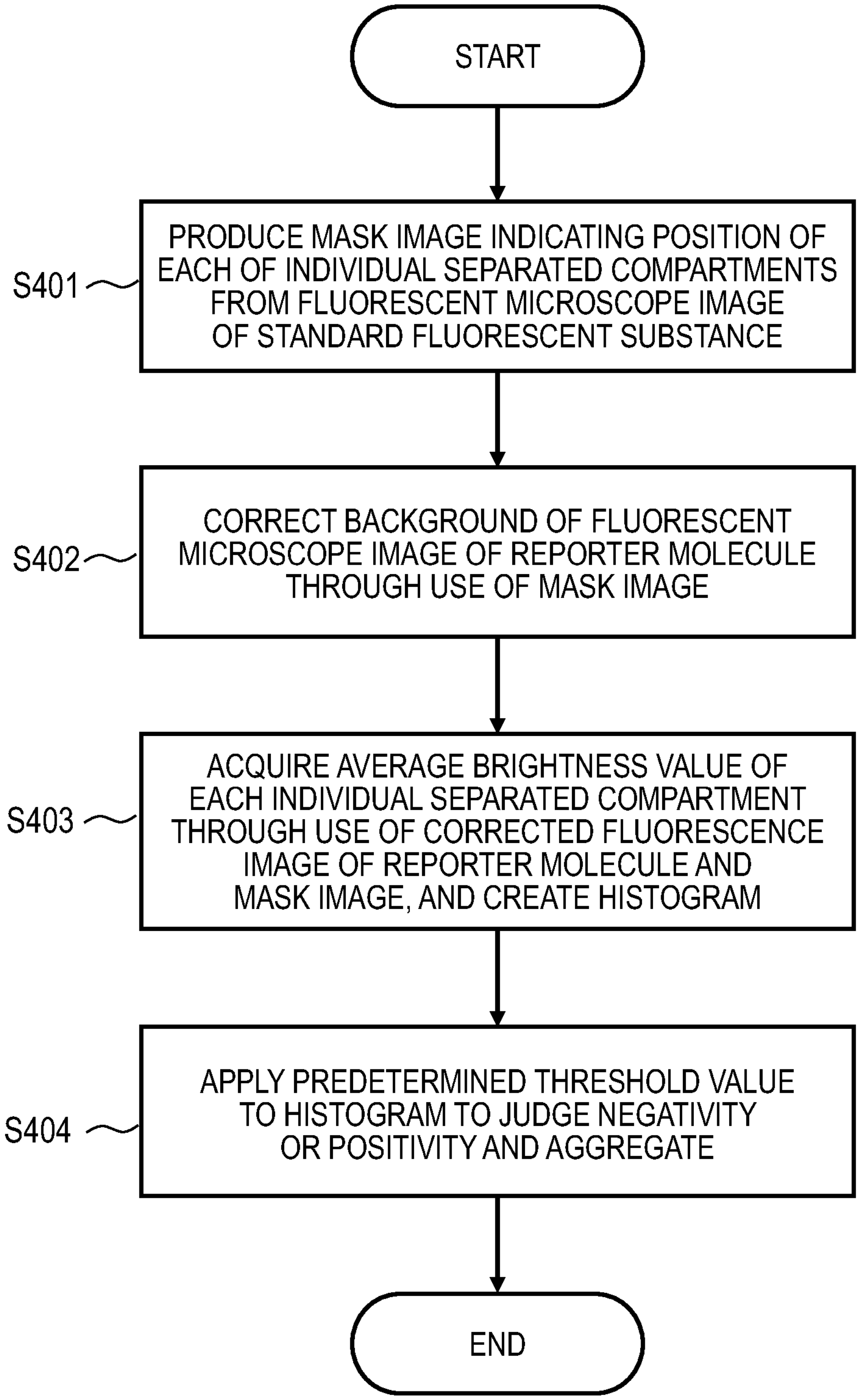
START
S401
PRODUCE MASK IMAGE INDICATING POSITION OF EACH OF INDIVIDUAL SEPARATED COMPARTMENTS FROM FLUORESCENT MICROSCOPE IMAGE OF STANDARD FLUORESCENT SUBSTANCE
S402
CORRECT BACKGROUND OF FLUORESCENT MICROSCOPE IMAGE OF REPORTER MOLECULE THROUGH USE OF MASK IMAGE
S403
ACQUIRE AVERAGE BRIGHTNESS VALUE OF EACH INDIVIDUAL SEPARATED COMPARTMENT THROUGH USE OF CORRECTED FLUORESCENCE IMAGE OF REPORTER MOLECULE AND MASK IMAGE, AND CREATE HISTOGRAM
S404
APPLY PREDETERMINED THRESHOLD VALUE TO HISTOGRAM TO JUDGE NEGATIVITY OR POSITIVITY AND AGGREGATE
END

FIG. 5A

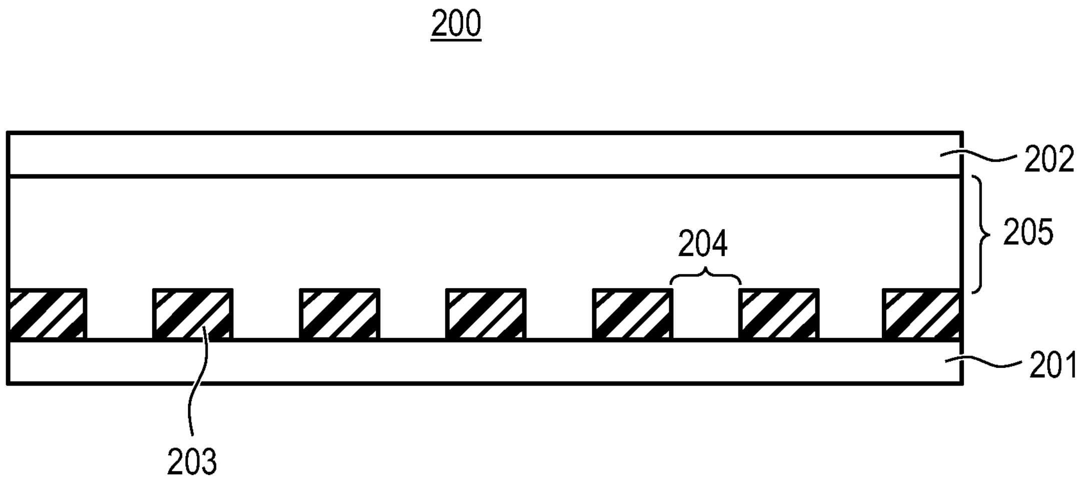

FIG. 5B

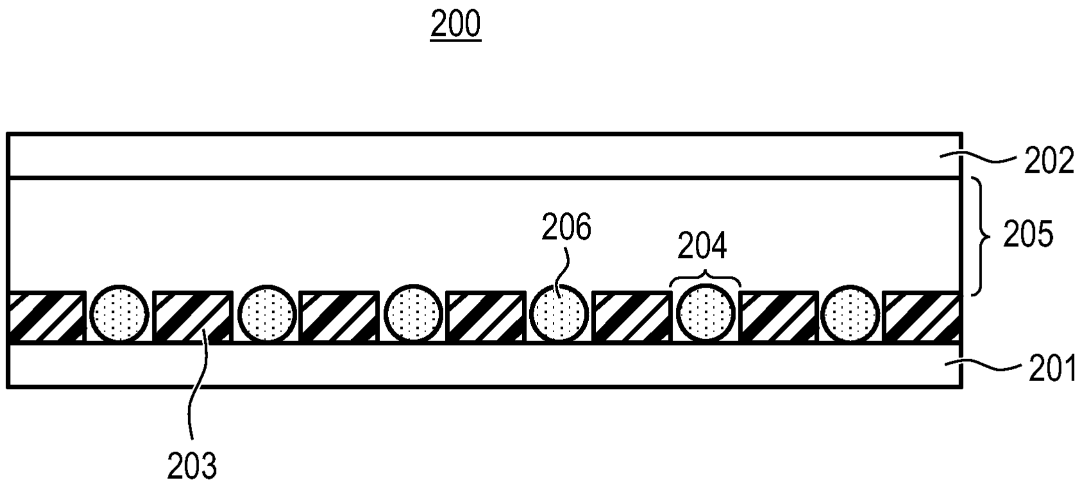

FIG. 6

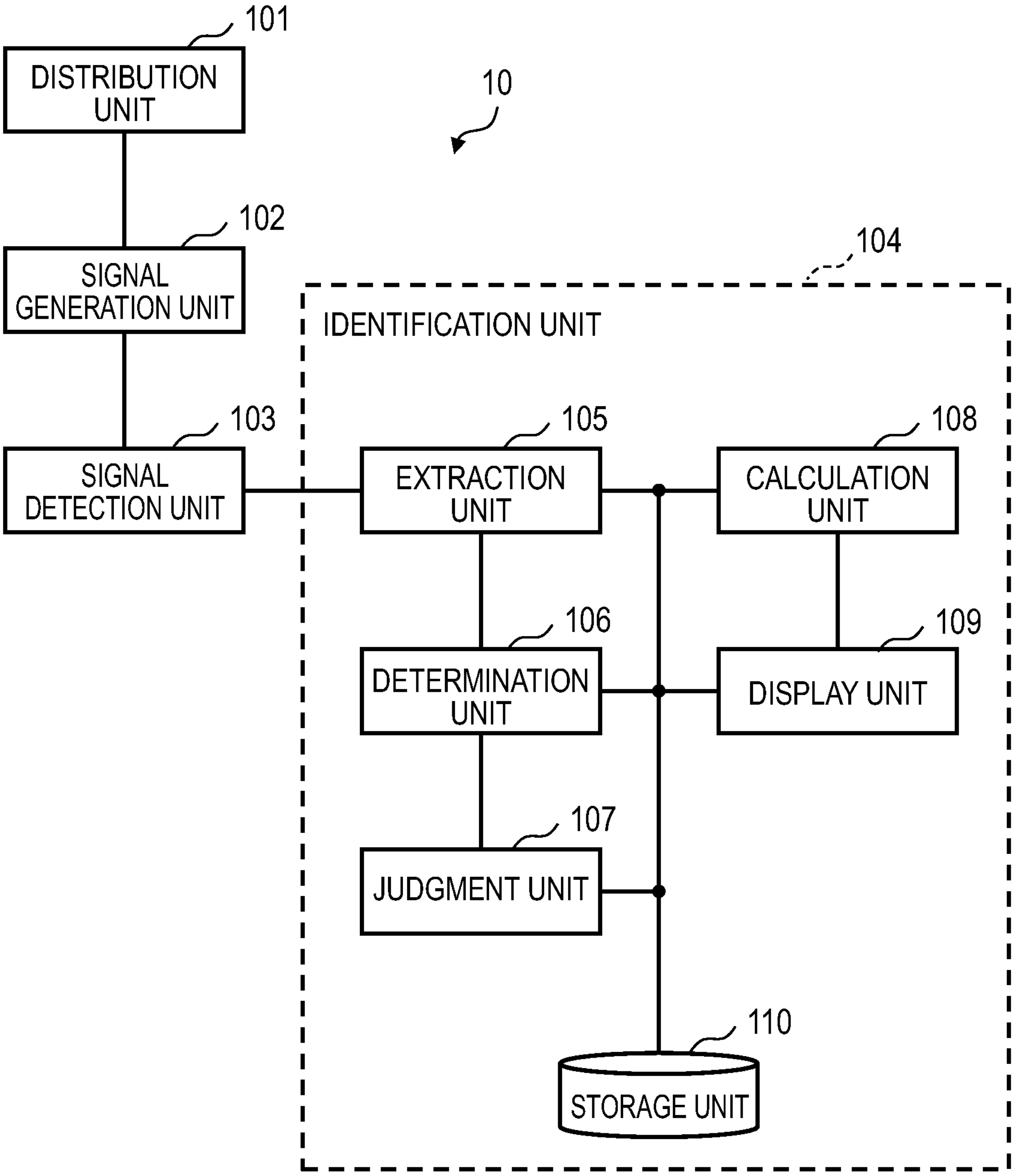
10
101
DISTRIBUTION UNIT
102
SIGNAL GENERATION UNIT
103
SIGNAL DETECTION UNIT
104
IDENTIFICATION UNIT
105
EXTRACTION UNIT
106
DETERMINATION UNIT
107
JUDGMENT UNIT
108
CALCULATION UNIT
109
DISPLAY UNIT
110
STORAGE UNIT

FIG. 7

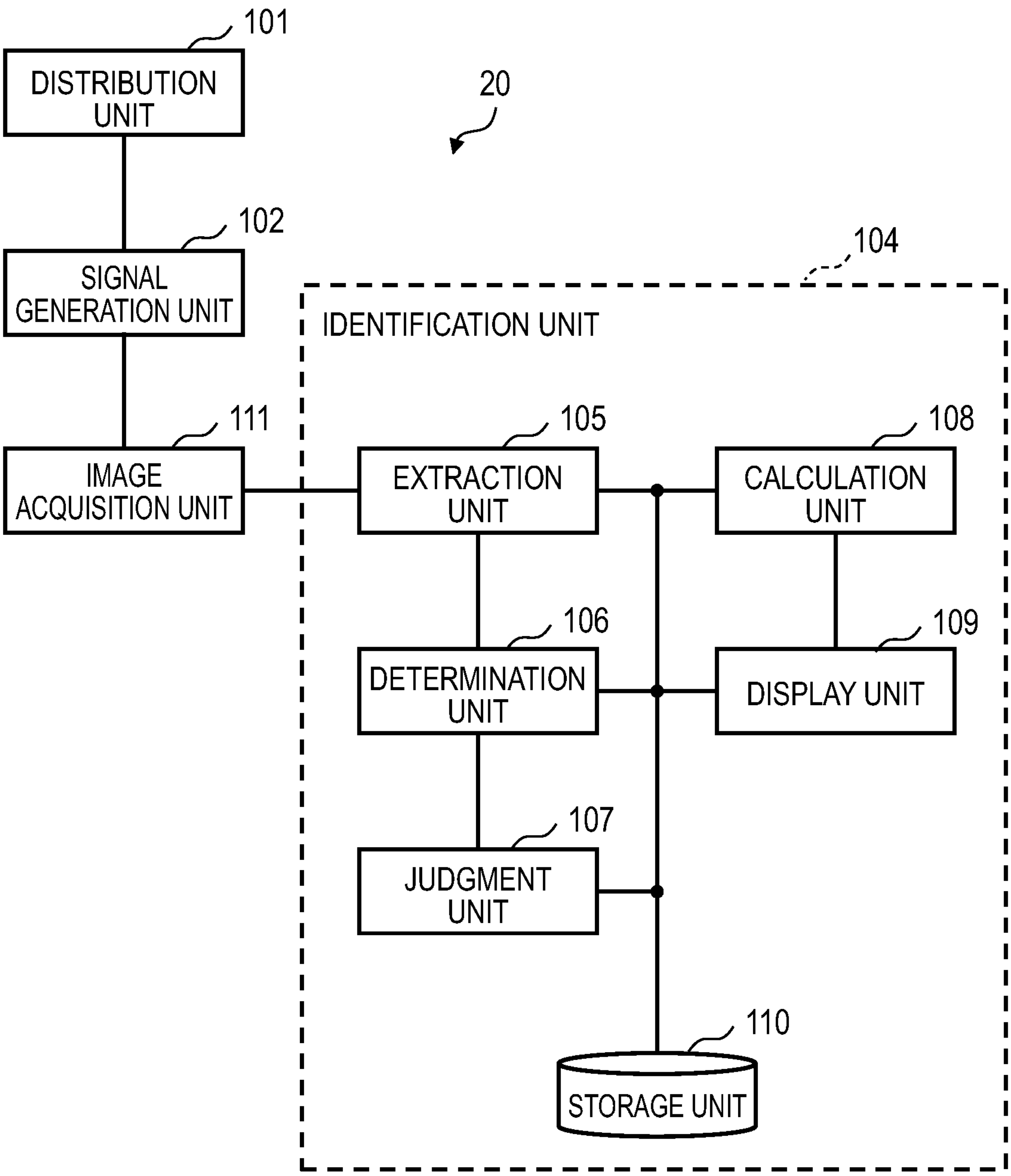
101
DISTRIBUTION UNIT
20
102
SIGNAL GENERATION UNIT
104
IDENTIFICATION UNIT
111
IMAGE ACQUISITION UNIT
105
EXTRACTION UNIT
108
CALCULATION UNIT
106
DETERMINATION UNIT
109
DISPLAY UNIT
107
JUDGMENT UNIT
110
STORAGE UNIT

FIG. 8

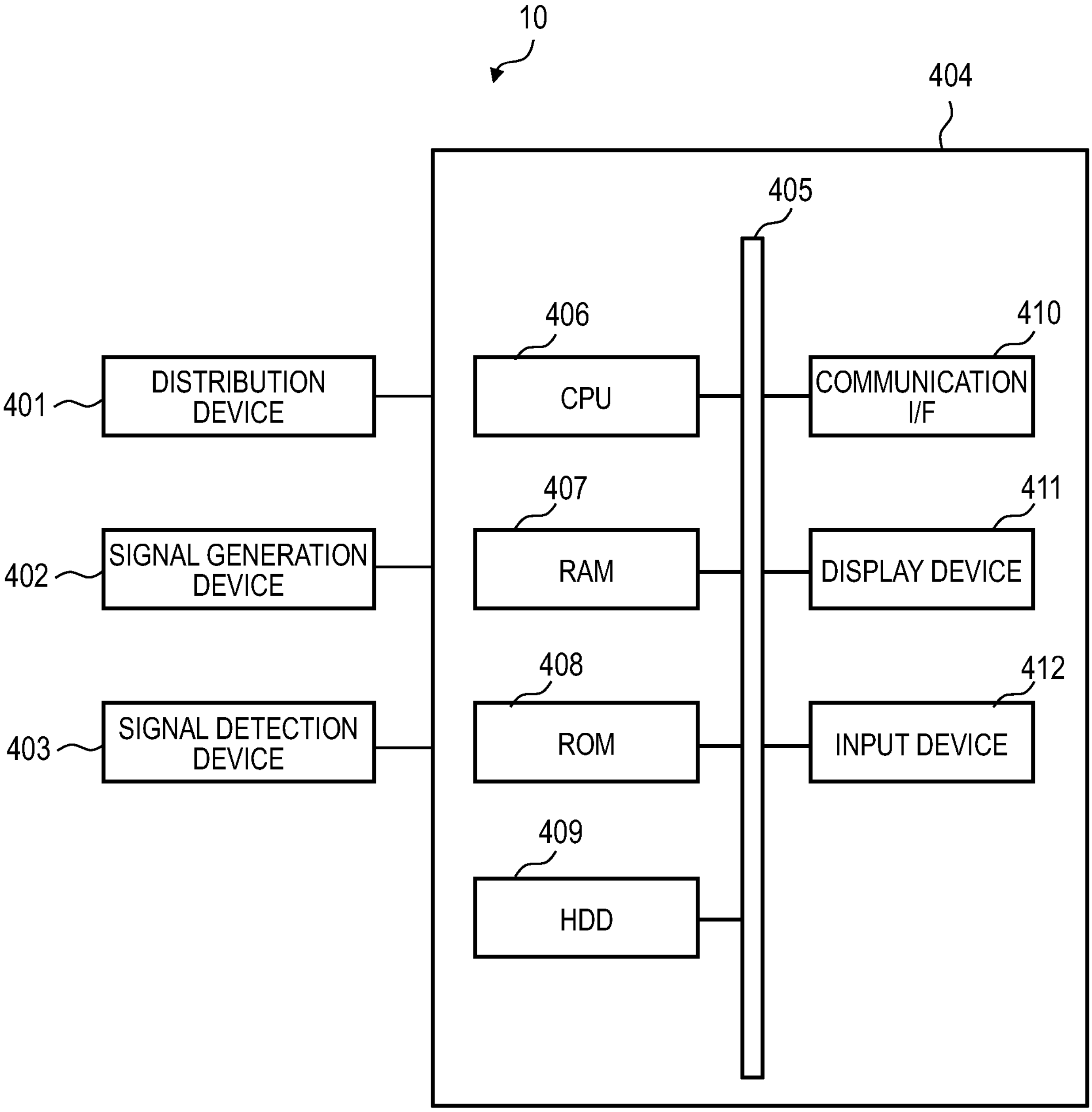
10
404
405
406
CPU
407
RAM
408
ROM
409
HDD
410
COMMUNICATION I/F
411
DISPLAY DEVICE
412
INPUT DEVICE
401
DISTRIBUTION DEVICE
402
SIGNAL GENERATION DEVICE
403
SIGNAL DETECTION DEVICE

FIG. 9A

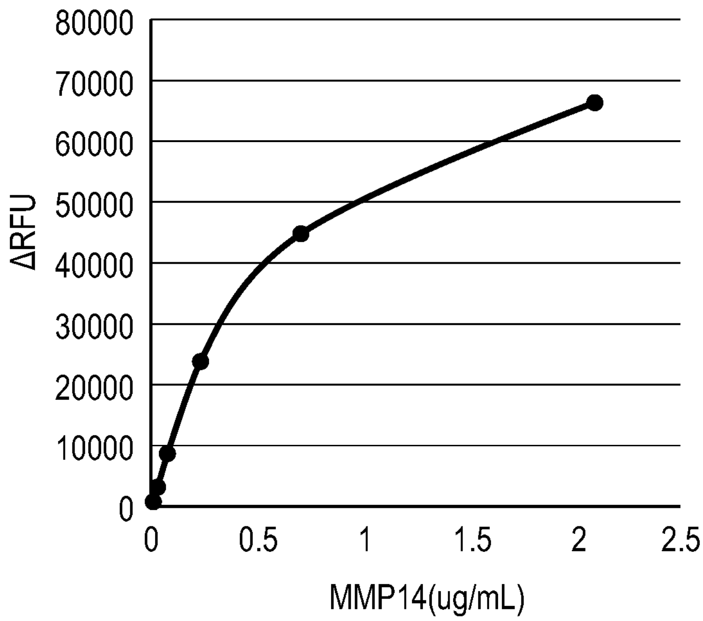
80000
70000
60000
50000
40000
30000
20000
10000
0
ΔRFU
0
0.5
1
1.5
2
2.5
MMP14(ug/mL)

FIG. 9B

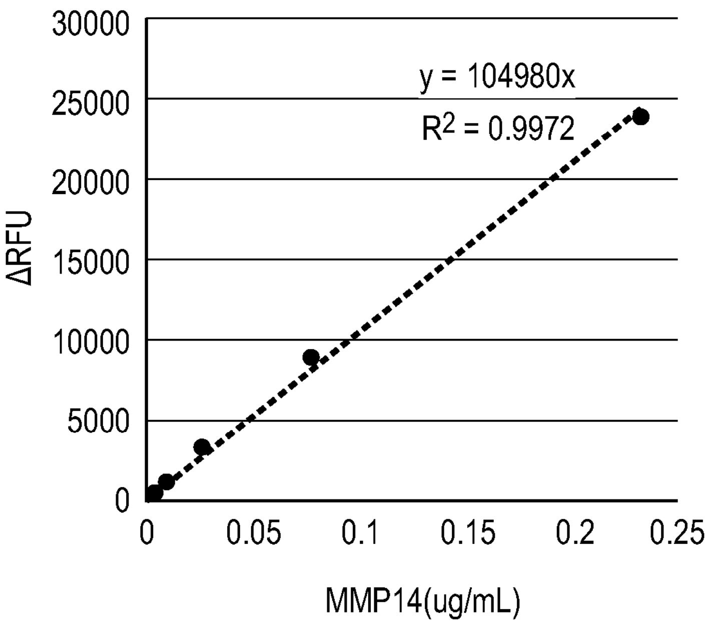
30000
25000
20000
15000
10000
5000
0
ΔRFU
y = 104980x
R2 = 0.9972
0
0.05
0.1
0.15
0.2
0.25
MMP14(ug/mL)

FIG. 10

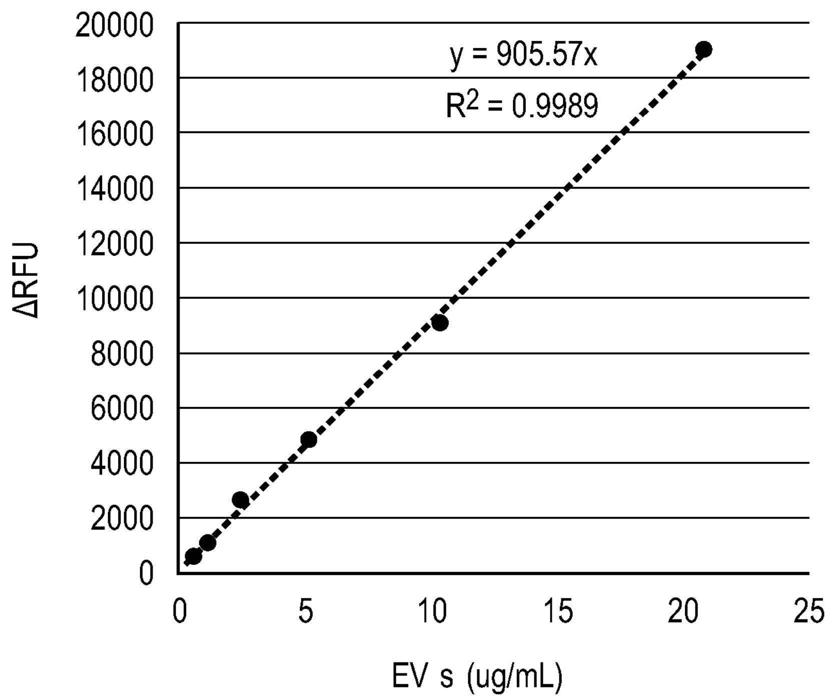
y = 905.57x
$R^2$ = 0.9989
20000
18000
16000
14000
12000
10000
8000
6000
4000
2000
0
ΔRFU
0
5
10
15
20
25
EV s (ug/mL)

FIG. 11

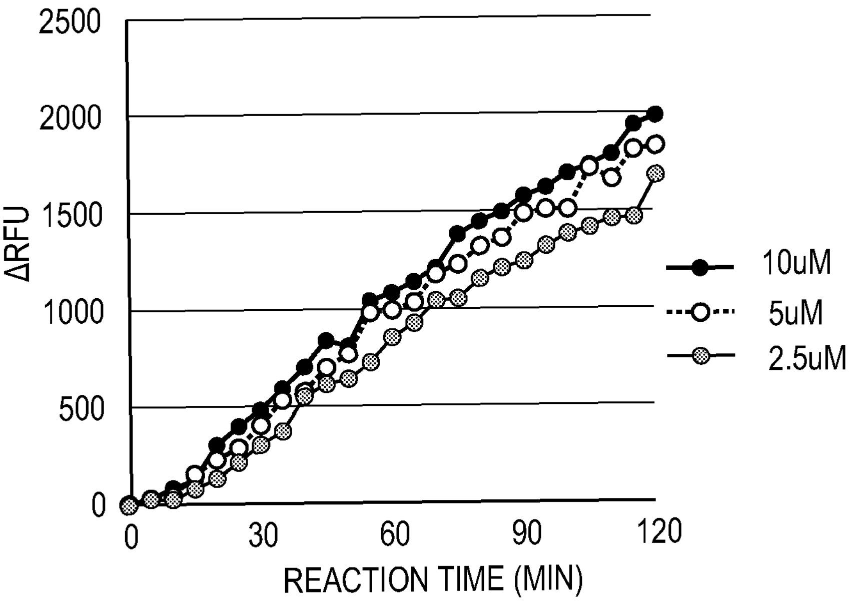
2500
2000
1500
1000
500
0
ΔRFU
0
30
60
90
120
REACTION TIME (MIN)
10uM
5uM
2.5uM

FIG. 12

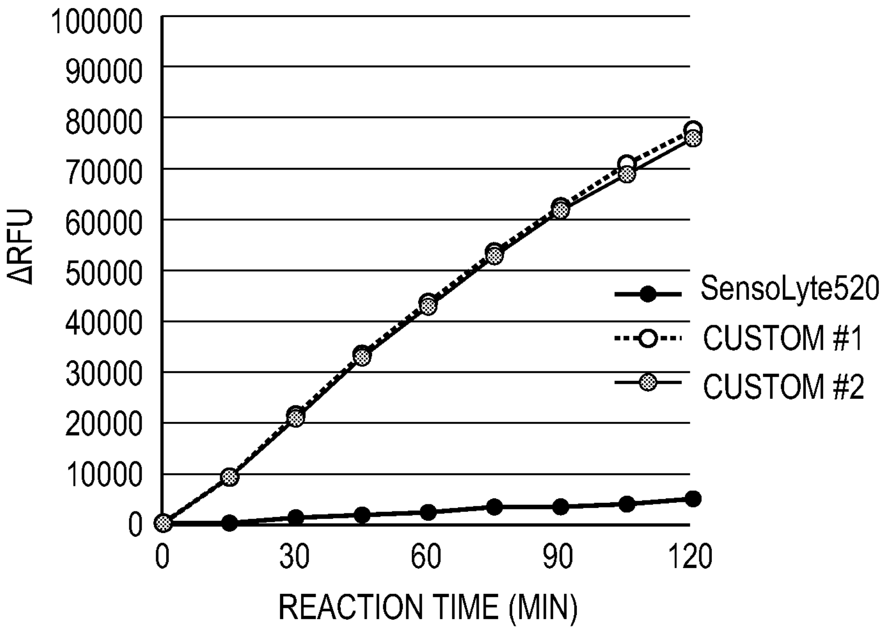
100000
90000
80000
70000
60000
50000
40000
30000
20000
10000
0
ΔRFU
0
30
60
90
120
REACTION TIME (MIN)
SensoLyte520
CUSTOM #1
CUSTOM #2

FIG. 13A

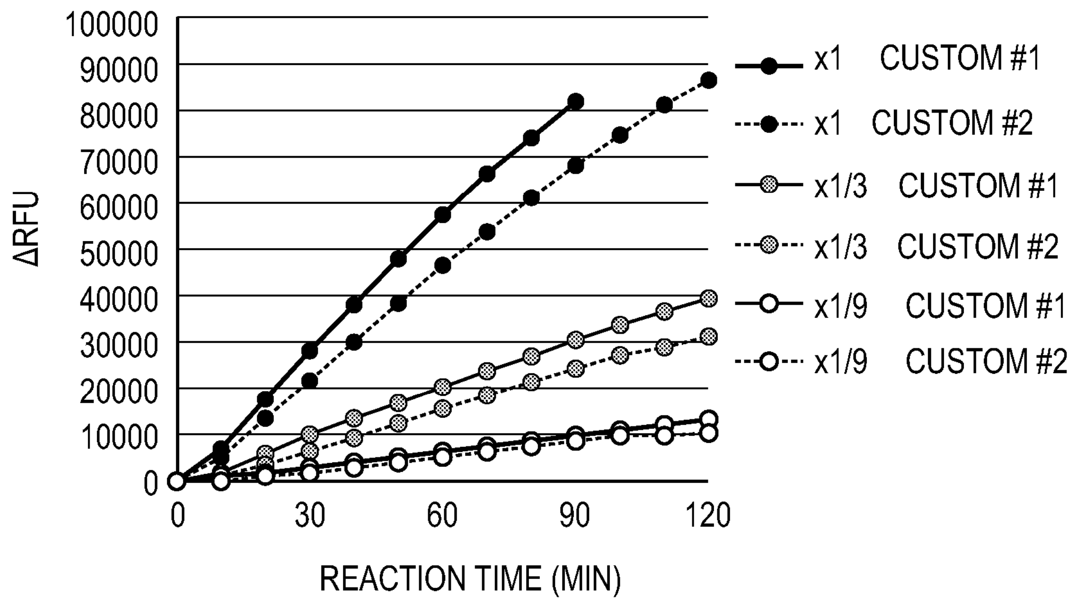
100000
90000
80000
70000
60000
50000
40000
30000
20000
10000
0
ΔRFU
0
30
60
90
120
REACTION TIME (MIN)
x1 CUSTOM #1
x1 CUSTOM #2
x1/3 CUSTOM #1
x1/3 CUSTOM #2
x1/9 CUSTOM #1
x1/9 CUSTOM #2

FIG. 13B

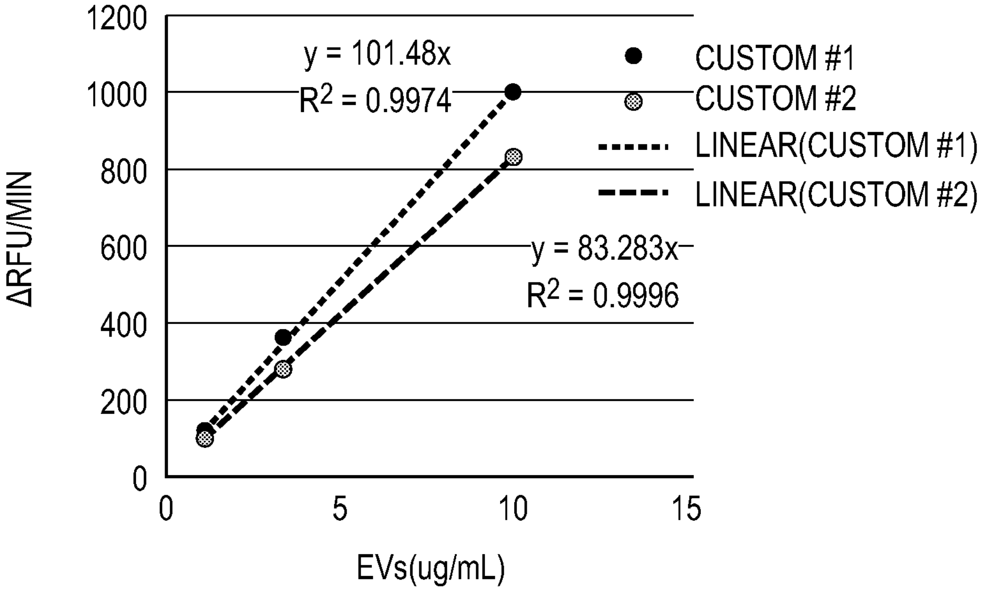
1200
1000
800
600
400
200
0
0
5
10
15
ΔRFU/MIN
EVs(ug/mL)
y = 101.48x
R2 = 0.9974
y = 83.283x
R2 = 0.9996
CUSTOM #1
CUSTOM #2
LINEAR(CUSTOM #1)
LINEAR(CUSTOM #2)

FIG. 14A

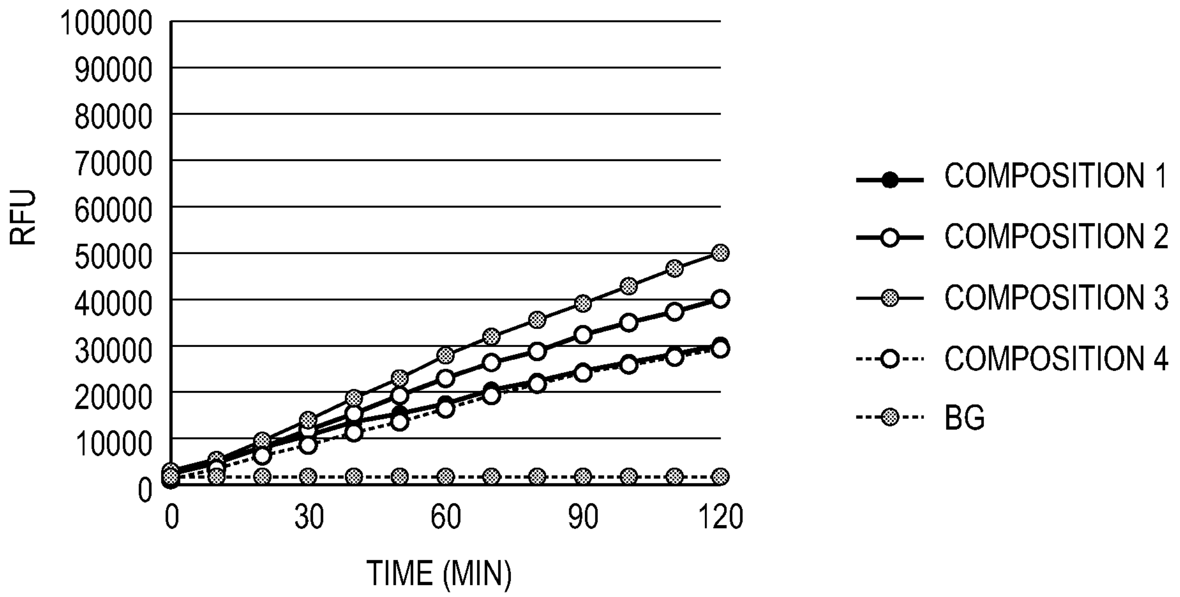
100000
90000
80000
70000
60000
50000
40000
30000
20000
10000
0
RFU
0
30
60
90
120
TIME (MIN)
COMPOSITION 1
COMPOSITION 2
COMPOSITION 3
COMPOSITION 4
BG

FIG. 14B

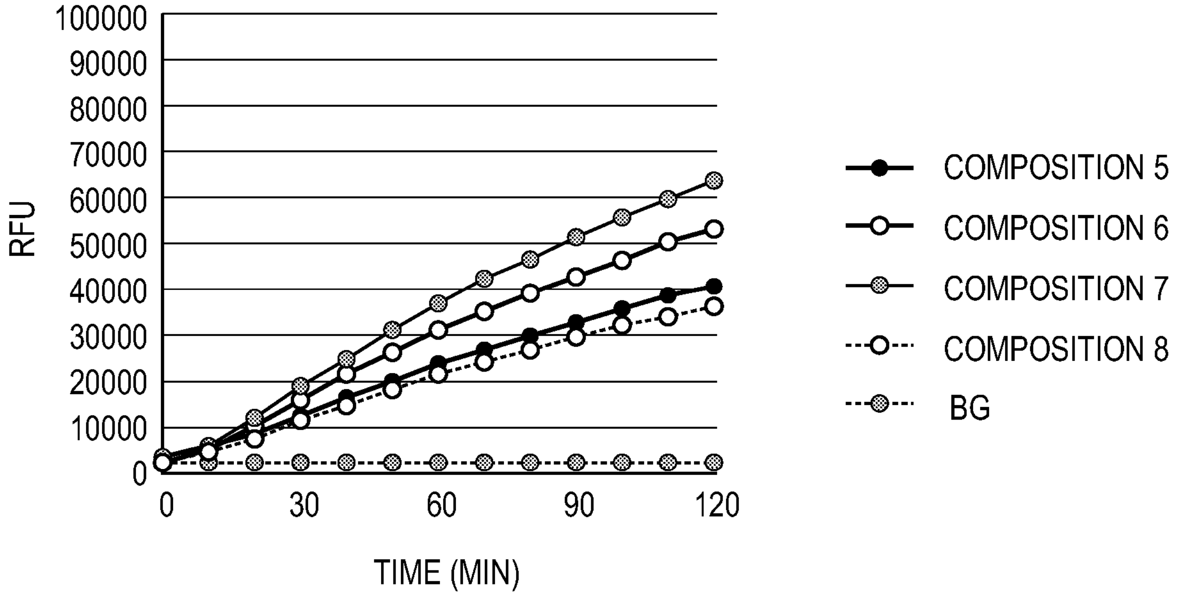
RFU
100000
90000
80000
70000
60000
50000
40000
30000
20000
10000
0
0
30
60
90
120
TIME (MIN)
COMPOSITION 5
COMPOSITION 6
COMPOSITION 7
COMPOSITION 8
BG

FIG. 14C

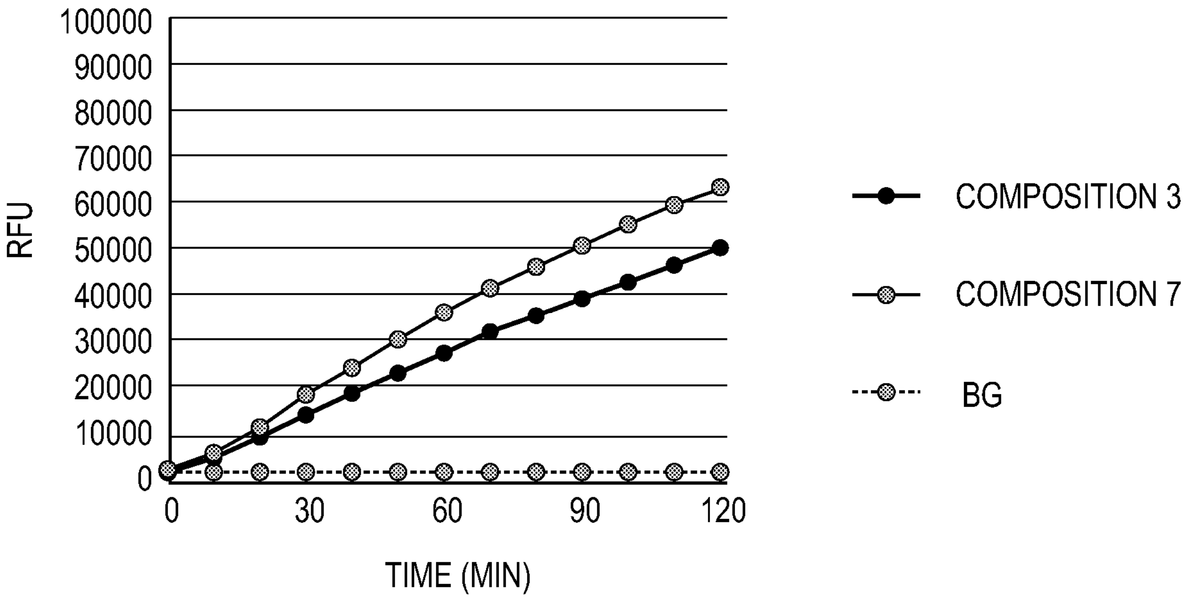
100000
90000
80000
70000
60000
50000
40000
30000
20000
10000
0
RFU
0
30
60
90
120
TIME (MIN)
COMPOSITION 3
COMPOSITION 7
BG

FIG. 15A

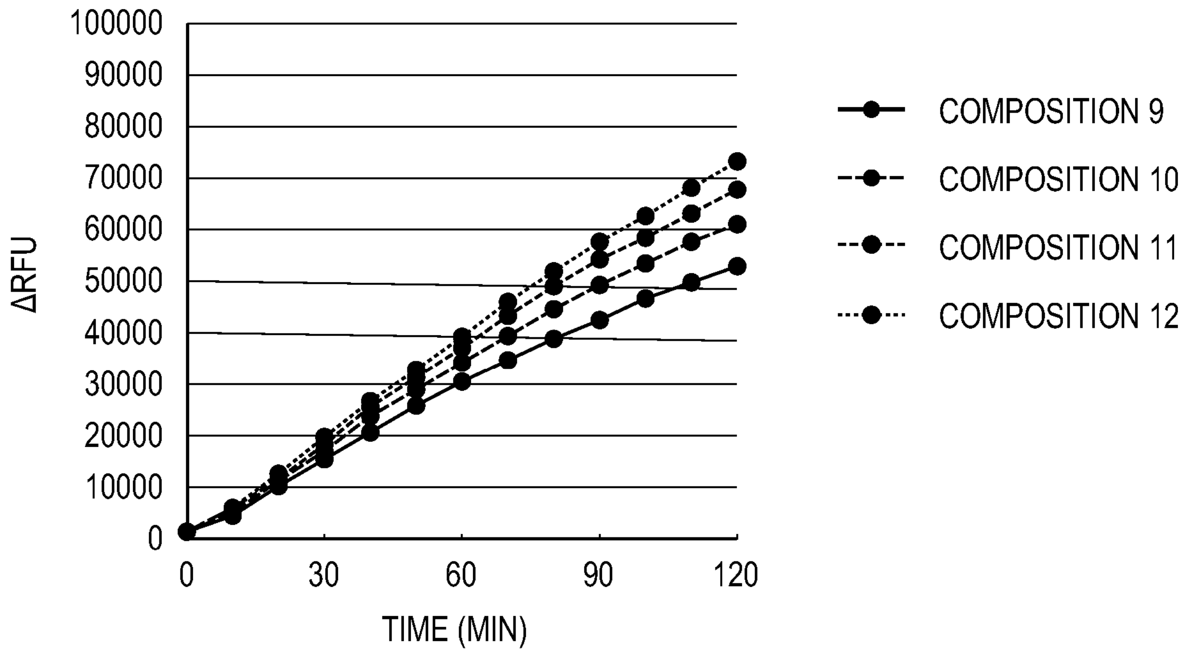
100000
90000
80000
70000
60000
50000
40000
30000
20000
10000
0
ΔRFU
0
30
60
90
120
TIME (MIN)
COMPOSITION 9
COMPOSITION 10
COMPOSITION 11
COMPOSITION 12

FIG. 15B

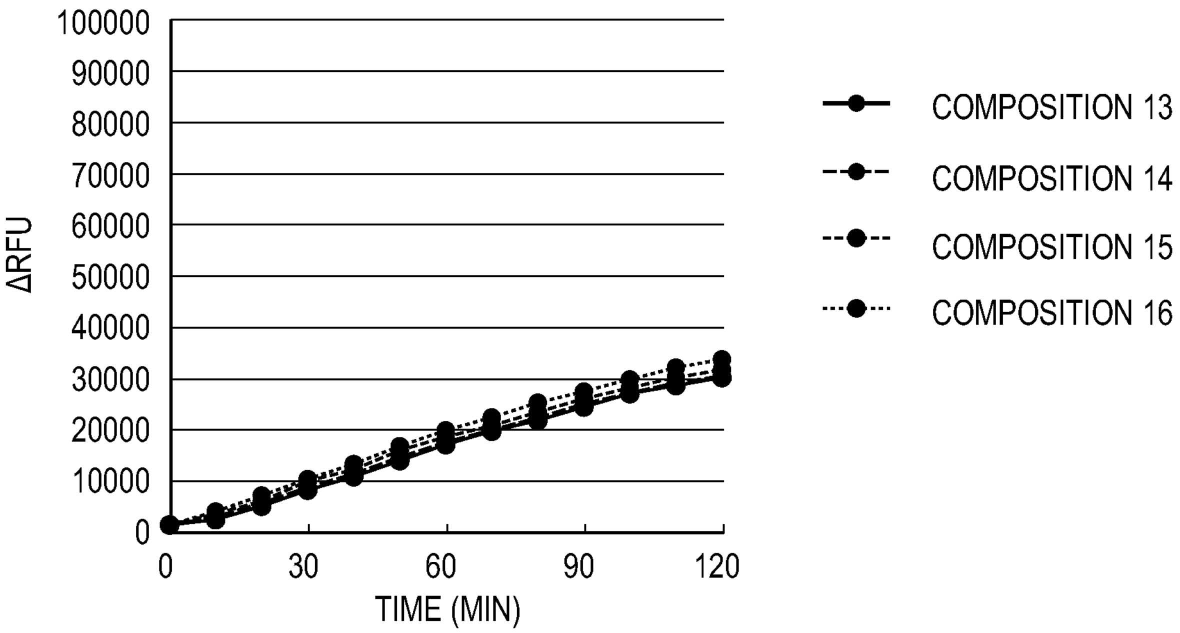
100000
90000
80000
70000
60000
50000
40000
30000
20000
10000
0
ΔRFU
0
30
60
90
120
TIME (MIN)
COMPOSITION 13
COMPOSITION 14
COMPOSITION 15
COMPOSITION 16

FIG. 16A

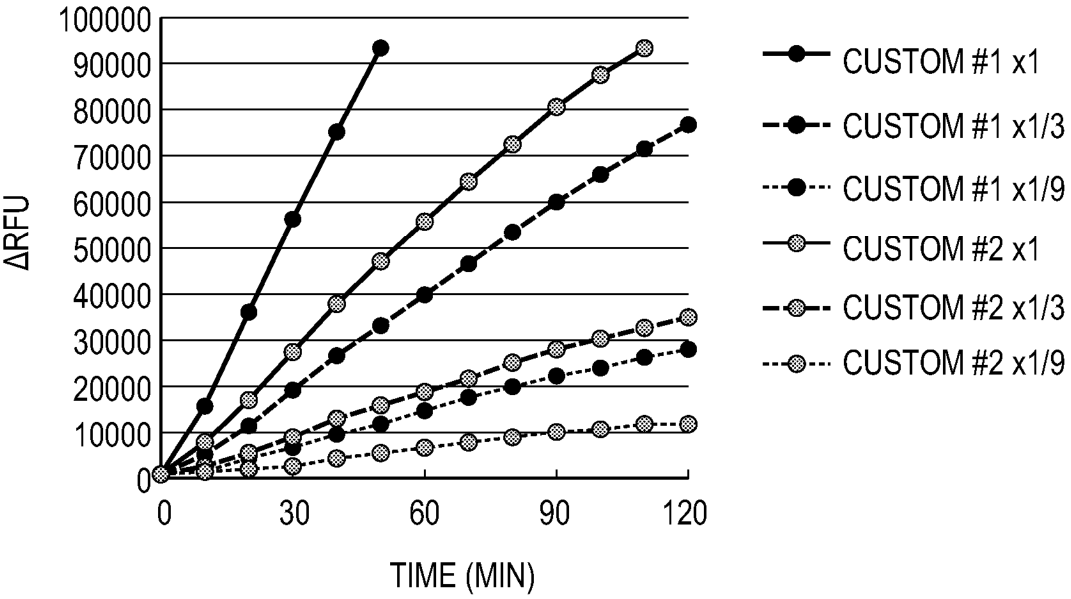
100000
90000
80000
70000
60000
50000
40000
30000
20000
10000
0
ΔRFU
0
30
60
90
120
TIME (MIN)
CUSTOM #1 x1
CUSTOM #1 x1/3
CUSTOM #1 x1/9
CUSTOM #2 x1
CUSTOM #2 x1/3
CUSTOM #2 x1/9

FIG. 16B

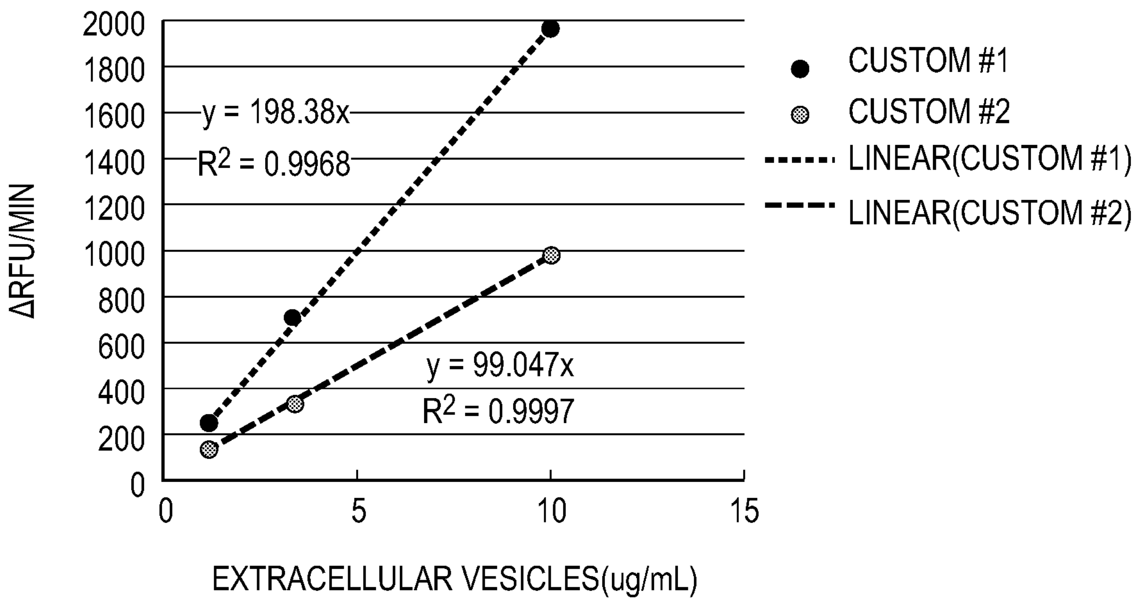
2000
1800
1600
1400
1200
1000
800
600
400
200
0
ΔRFU/MIN
0
5
10
15
EXTRACELLULAR VESICLES(ug/mL)
y = 198.38x
R2 = 0.9968
y = 99.047x
R2 = 0.9997
CUSTOM #1
CUSTOM #2
LINEAR(CUSTOM #1)
LINEAR(CUSTOM #2)

FIG. 17A

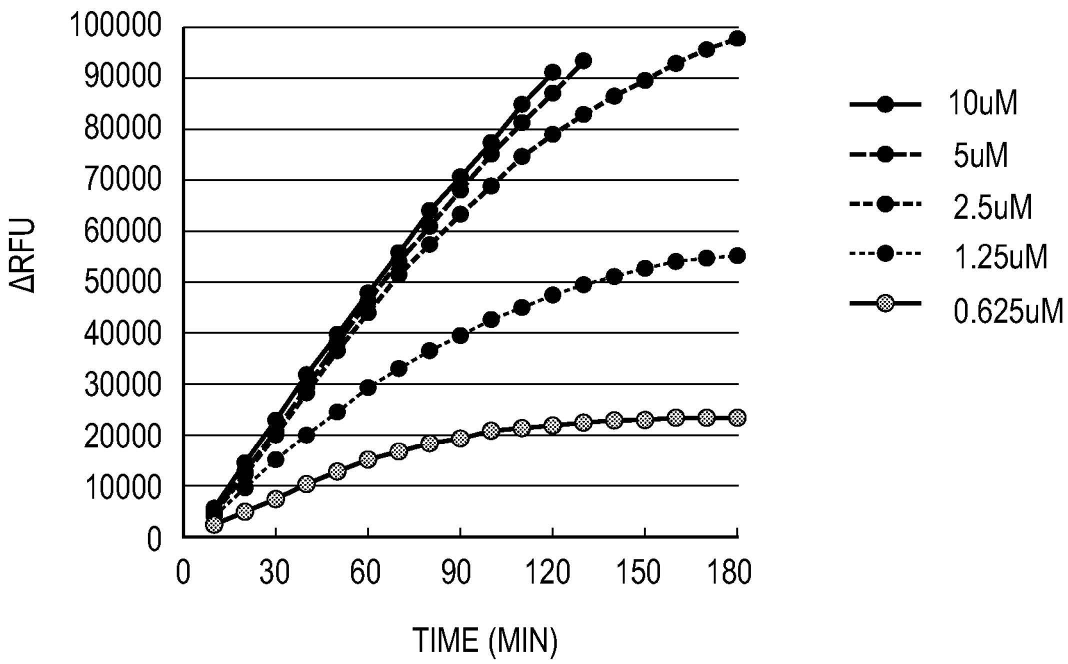
100000
90000
80000
70000
60000
50000
40000
30000
20000
10000
0
ΔRFU
0
30
60
90
120
150
180
TIME (MIN)
10uM
5uM
2.5uM
1.25uM
0.625uM

FIG. 17B

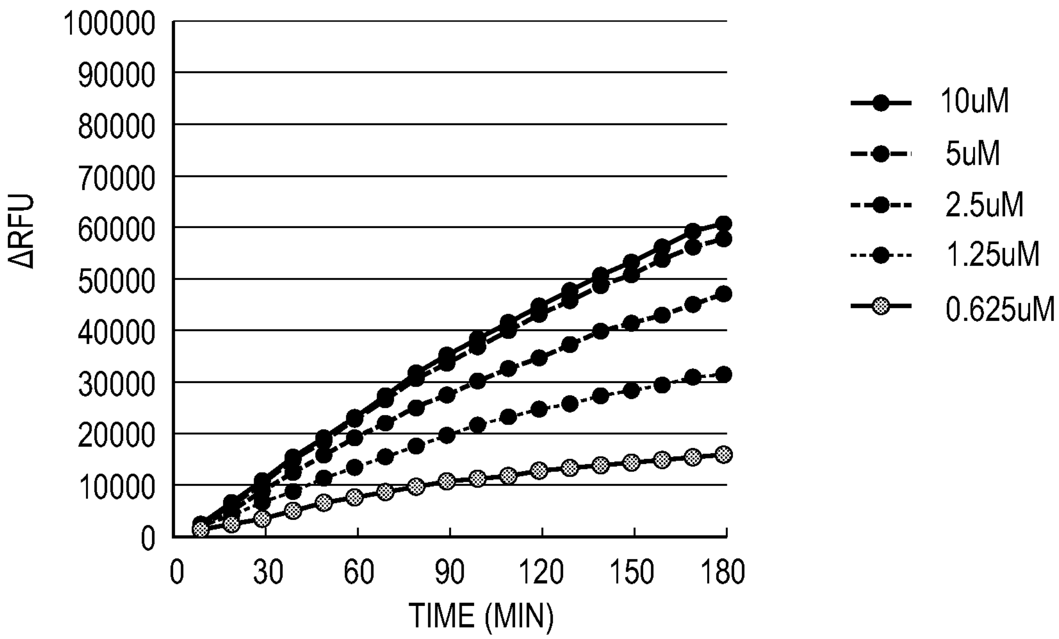
100000
90000
80000
70000
60000
50000
40000
30000
20000
10000
0
ΔRFU
0
30
60
90
120
150
180
TIME (MIN)
10uM
5uM
2.5uM
1.25uM
0.625uM

FIG. 17C

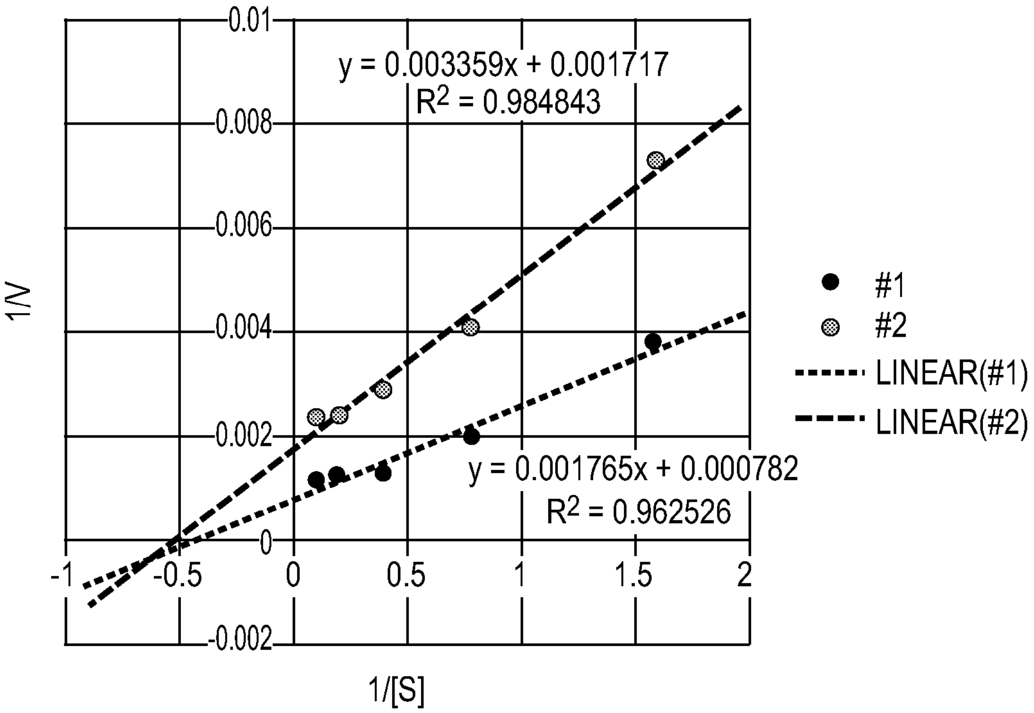
y = 0.003359x + 0.001717
R2 = 0.984843
y = 0.001765x + 0.000782
R2 = 0.962526
0.01
0.008
0.006
0.004
0.002
0
-0.002
-1
-0.5
0
0.5
1
1.5
2
1/V
1/[S]
#1
#2
LINEAR(#1)
LINEAR(#2)

FIG. 18A

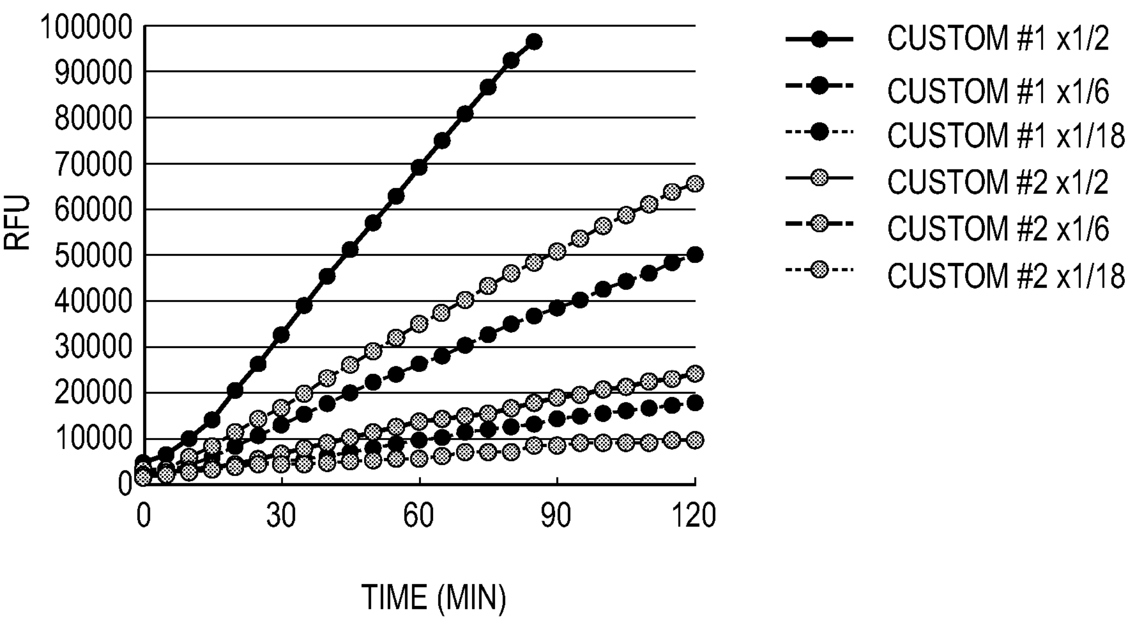
100000
90000
80000
70000
60000
50000
40000
30000
20000
10000
0
RFU
0
30
60
90
120
TIME (MIN)
CUSTOM #1 x1/2
CUSTOM #1 x1/6
CUSTOM #1 x1/18
CUSTOM #2 x1/2
CUSTOM #2 x1/6
CUSTOM #2 x1/18

FIG. 18B

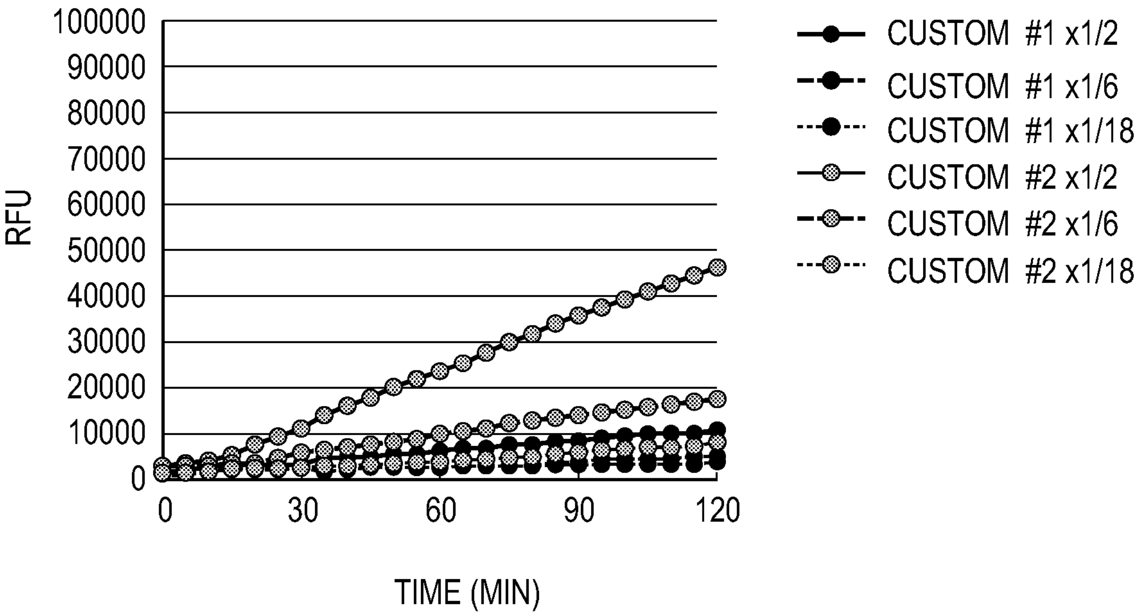

RFU
100000
90000
80000
70000
60000
50000
40000
30000
20000
10000
0
0
30
60
90
120
TIME (MIN)
CUSTOM #1 x1/2
CUSTOM #1 x1/6
CUSTOM #1 x1/18
CUSTOM #2 x1/2
CUSTOM #2 x1/6
CUSTOM #2 x1/18

FIG. 18C

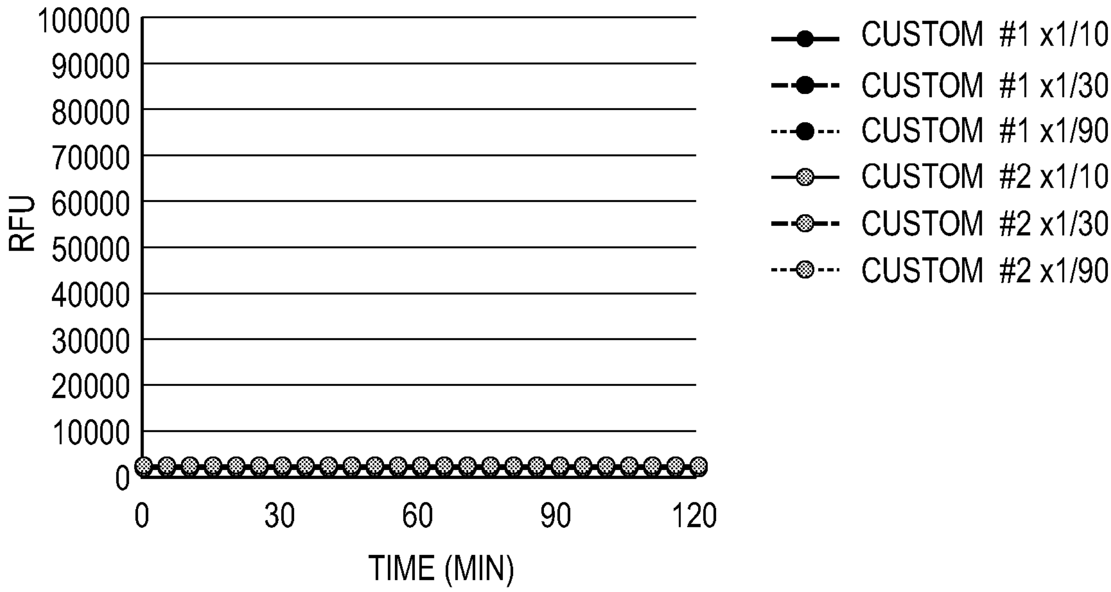
100000
90000
80000
70000
60000
50000
40000
30000
20000
10000
0
RFU
0
30
60
90
120
TIME (MIN)
CUSTOM #1 x1/10
CUSTOM #1 x1/30
CUSTOM #1 x1/90
CUSTOM #2 x1/10
CUSTOM #2 x1/30
CUSTOM #2 x1/90

FIG. 19

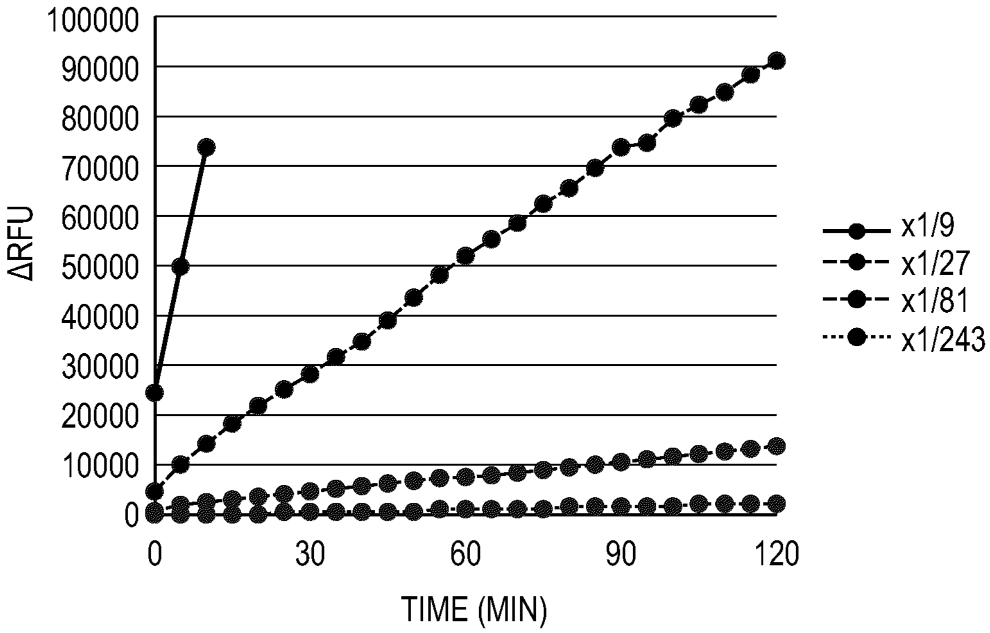
100000
90000
80000
70000
60000
50000
40000
30000
20000
10000
0
ΔRFU
0
30
60
90
120
TIME (MIN)
x1/9
x1/27
x1/81
x1/243

FIG. 20A

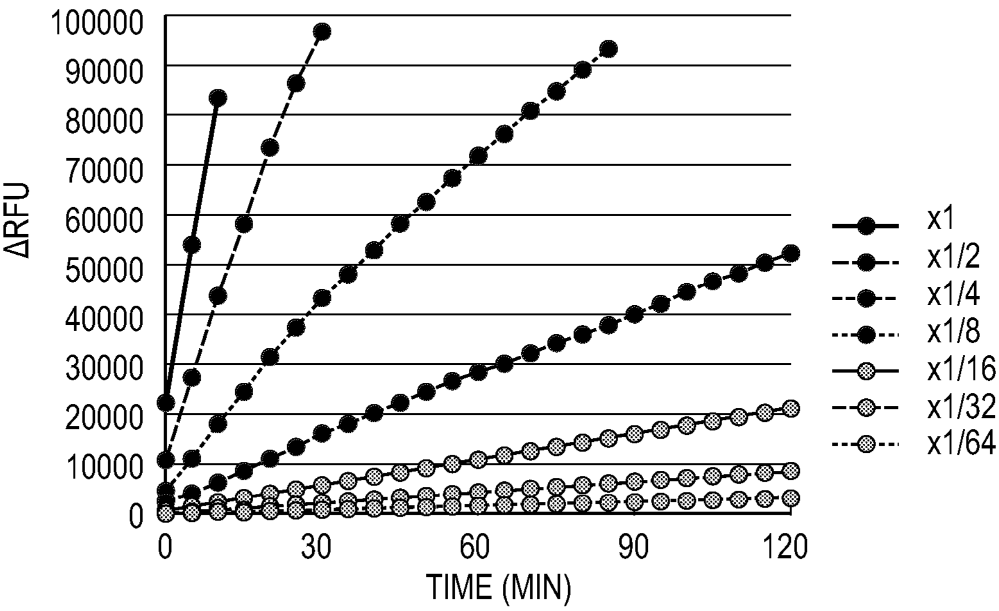
100000
90000
80000
70000
60000
50000
40000
30000
20000
10000
0
ΔRFU
0
30
60
90
120
TIME (MIN)
x1
x1/2
x1/4
x1/8
x1/16
x1/32
x1/64

FIG. 20B

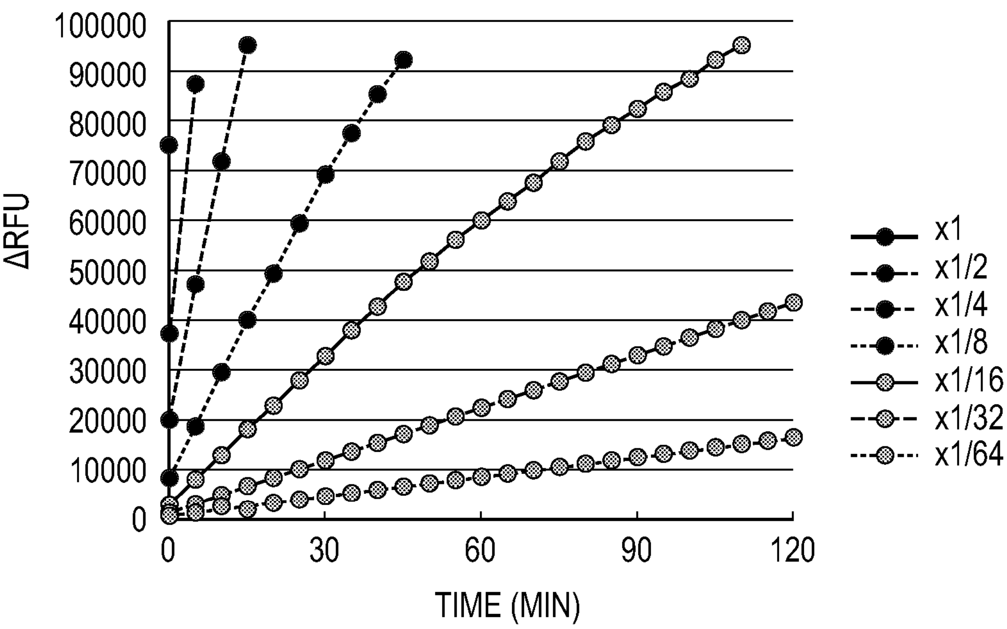
100000
90000
80000
70000
60000
50000
40000
30000
20000
10000
0
ΔRFU
0
30
60
90
120
TIME (MIN)
x1
x1/2
x1/4
x1/8
x1/16
x1/32
x1/64

FIG. 21

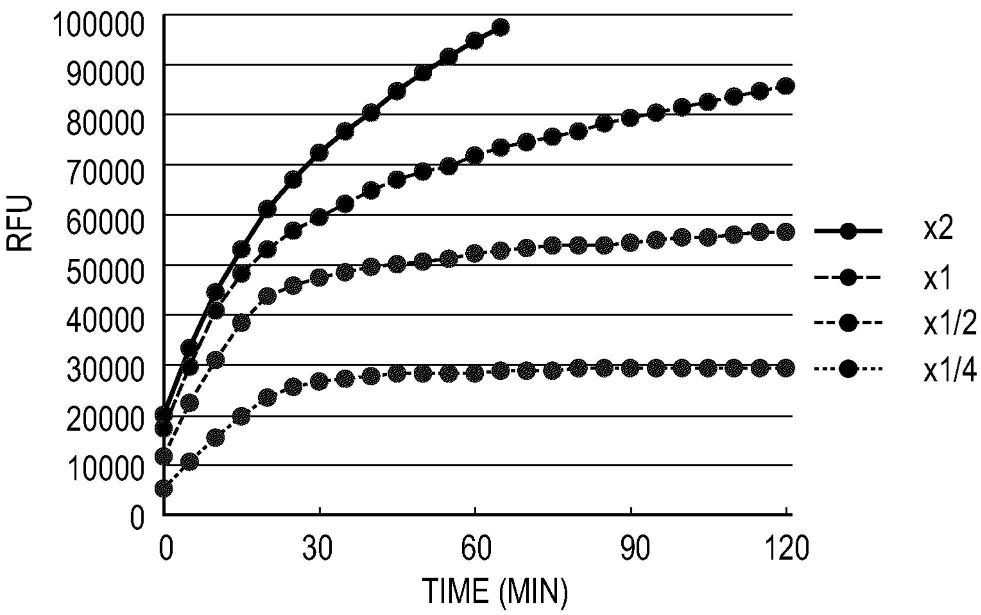
100000
90000
80000
70000
60000
50000
40000
30000
20000
10000
0
RFU
0
30
60
90
120
TIME (MIN)
x2
x1
x1/2
x1/4

FIG. 22

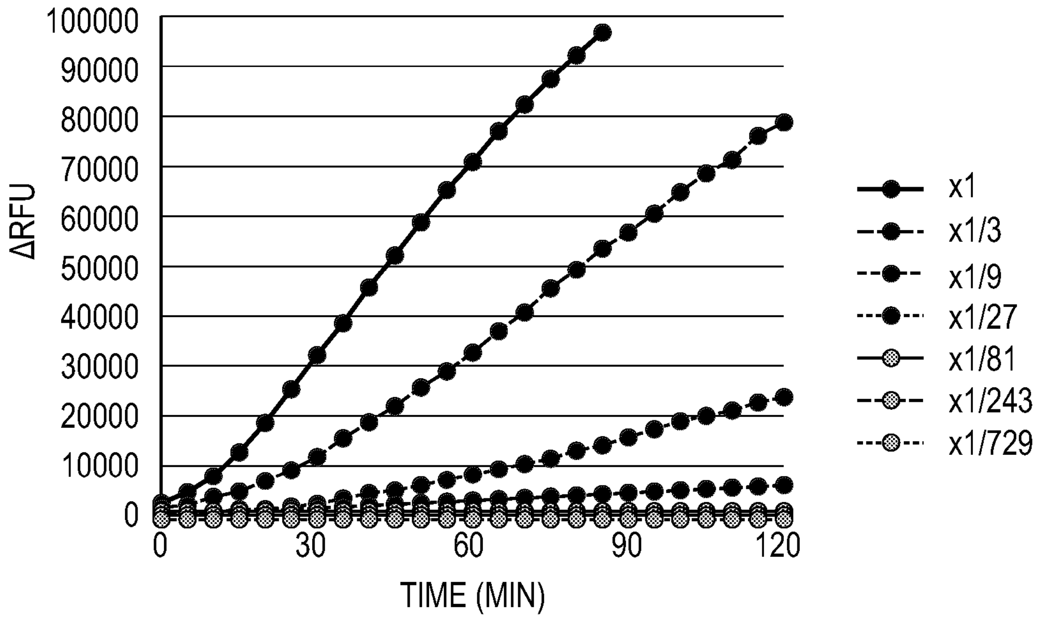
ΔRFU
100000
90000
80000
70000
60000
50000
40000
30000
20000
10000
0
0
30
60
90
120
TIME (MIN)
x1
x1/3
x1/9
x1/27
x1/81
x1/243
x1/729

FIG. 23A

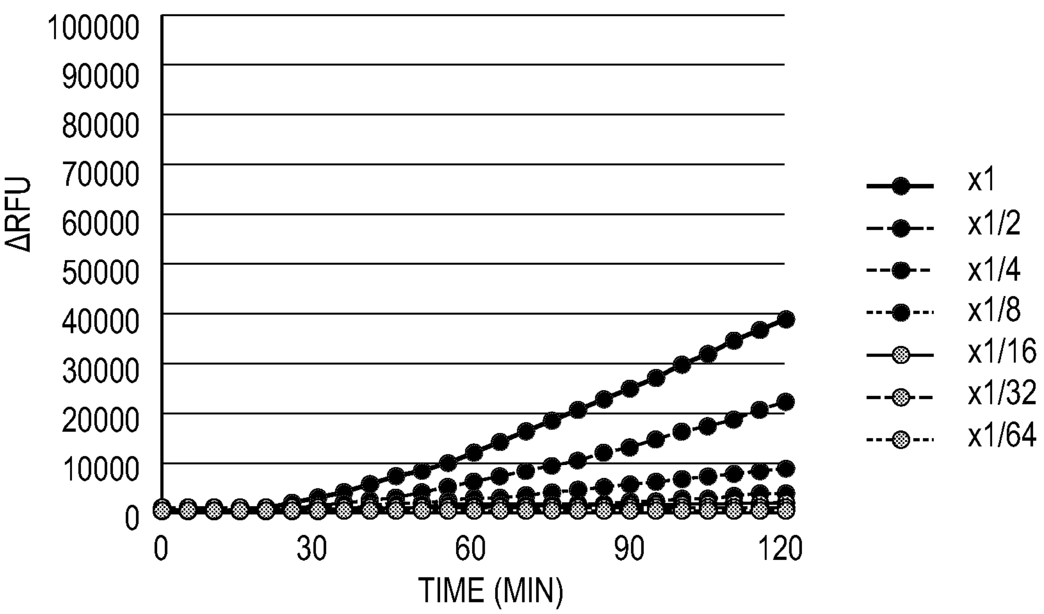
100000
90000
80000
70000
60000
50000
40000
30000
20000
10000
0
ΔRFU
0
30
60
90
120
TIME (MIN)
x1
x1/2
x1/4
x1/8
x1/16
x1/32
x1/64

FIG. 23B

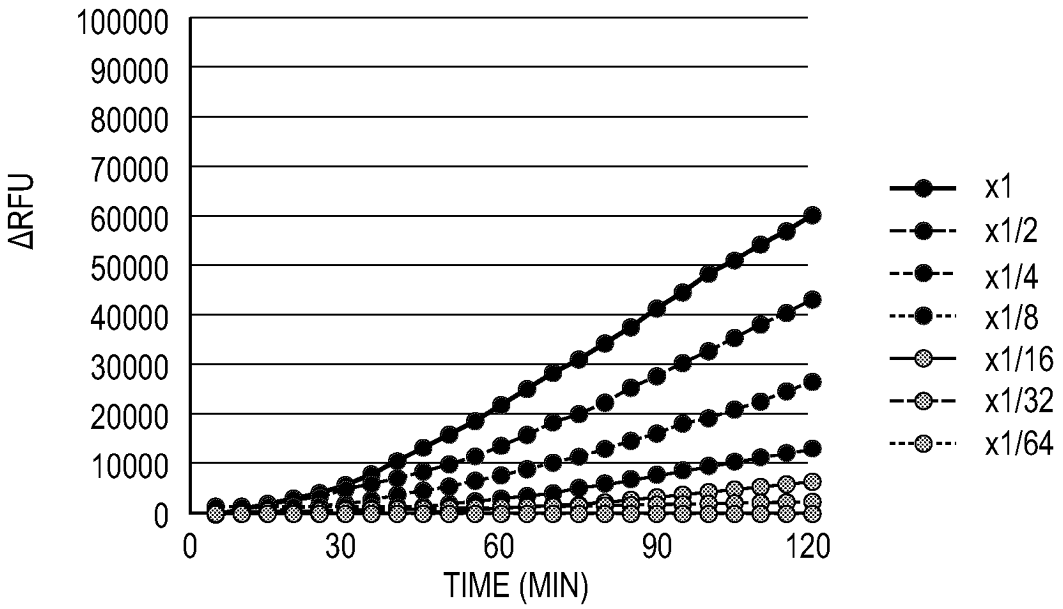
ΔRFU
100000
90000
80000
70000
60000
50000
40000
30000
20000
10000
0
0
30
60
90
120
TIME (MIN)
x1
x1/2
x1/4
x1/8
x1/16
x1/32
x1/64

FIG. 24

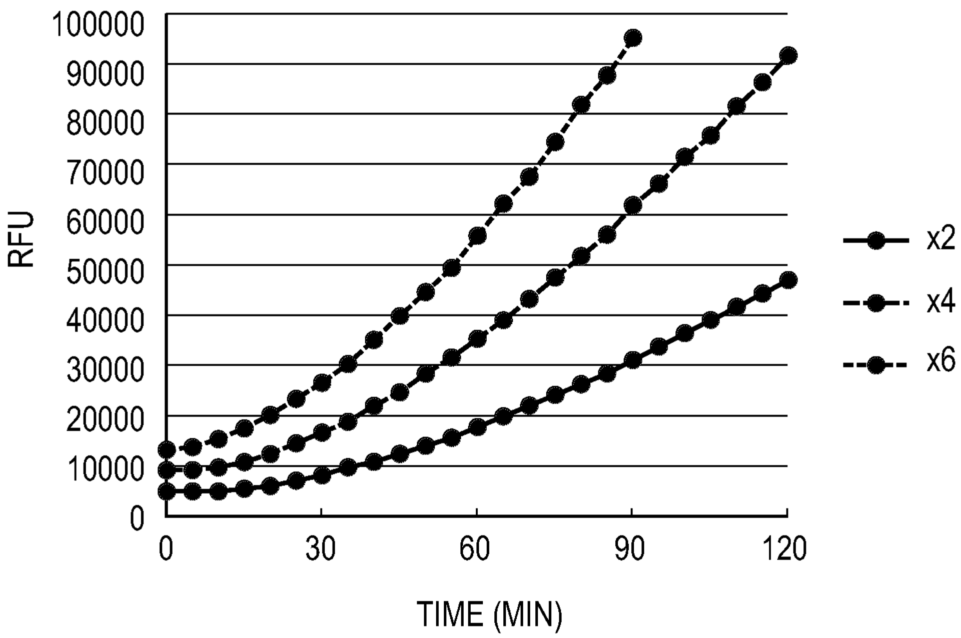
100000
90000
80000
70000
60000
50000
40000
30000
20000
10000
0
RFU
0
30
60
90
120
TIME (MIN)
x2
x4
x6

FIG. 25A

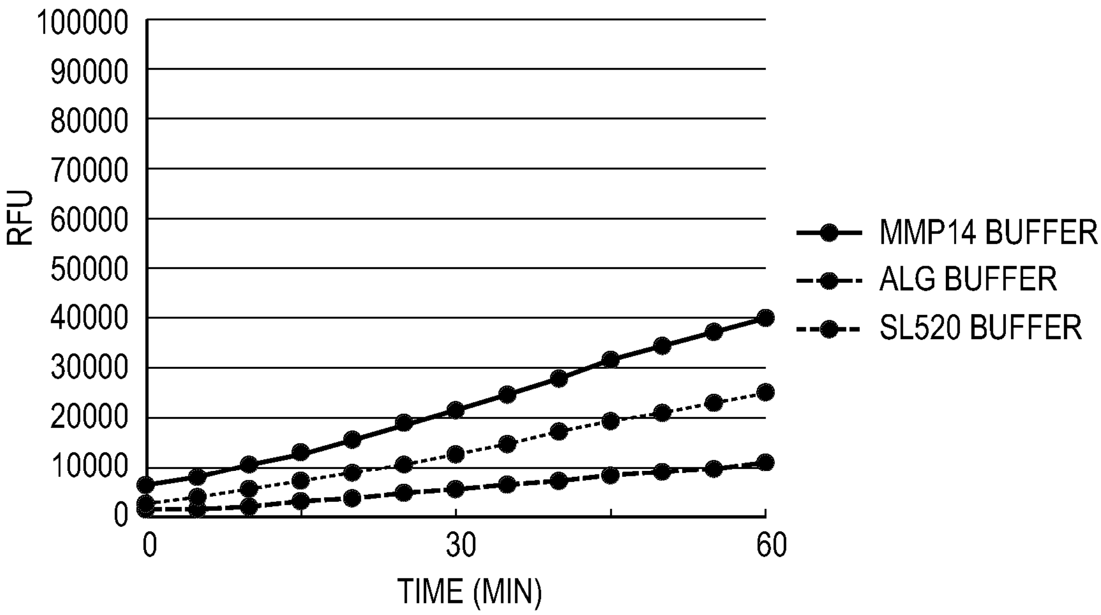
100000
90000
80000
70000
60000
50000
40000
30000
20000
10000
0
RFU
0
30
60
TIME (MIN)
MMP14 BUFFER
ALG BUFFER
SL520 BUFFER

FIG. 25B

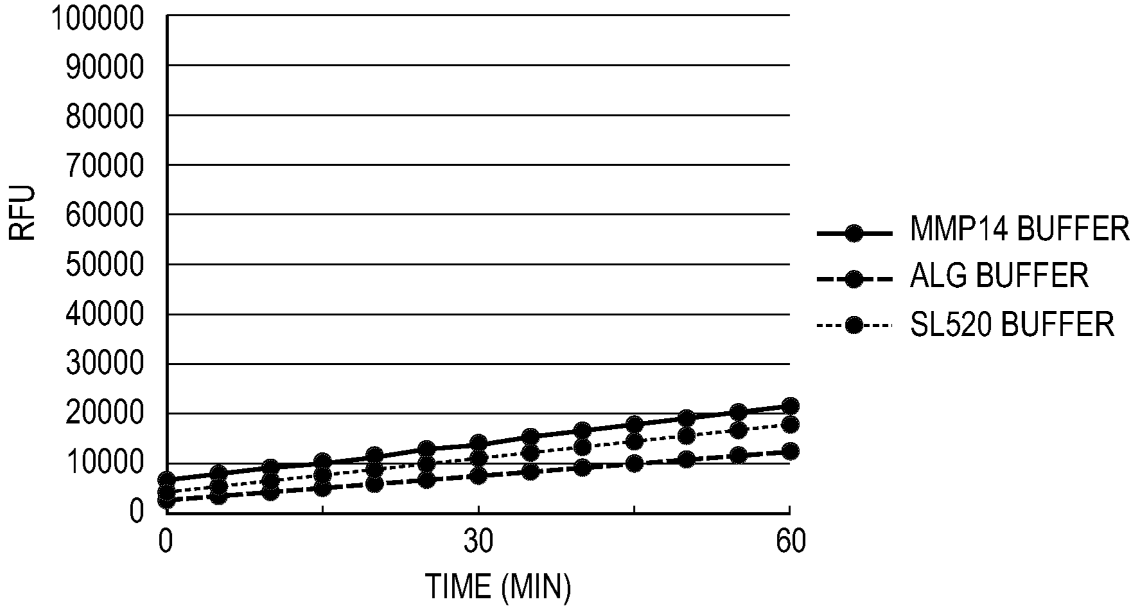

100000
90000
80000
70000
60000
50000
40000
30000
20000
10000
0
RFU
0
30
60
TIME (MIN)
MMP14 BUFFER
ALG BUFFER
SL520 BUFFER

FIG. 25C

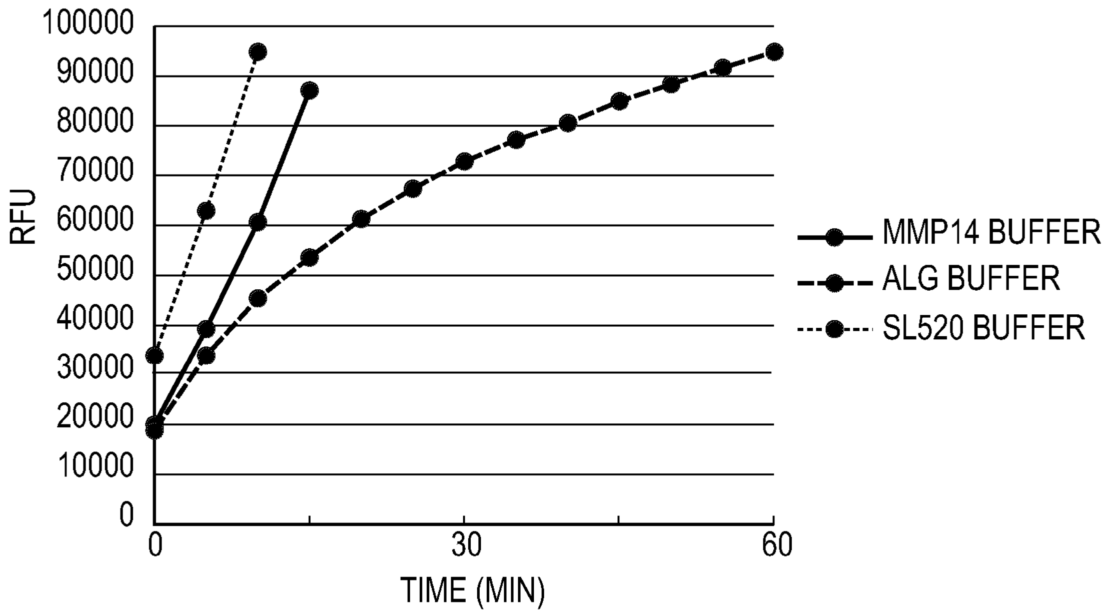
100000
90000
80000
70000
60000
50000
40000
30000
20000
10000
0
RFU
0
30
60
TIME (MIN)
MMP14 BUFFER
ALG BUFFER
SL520 BUFFER

FIG. 25D

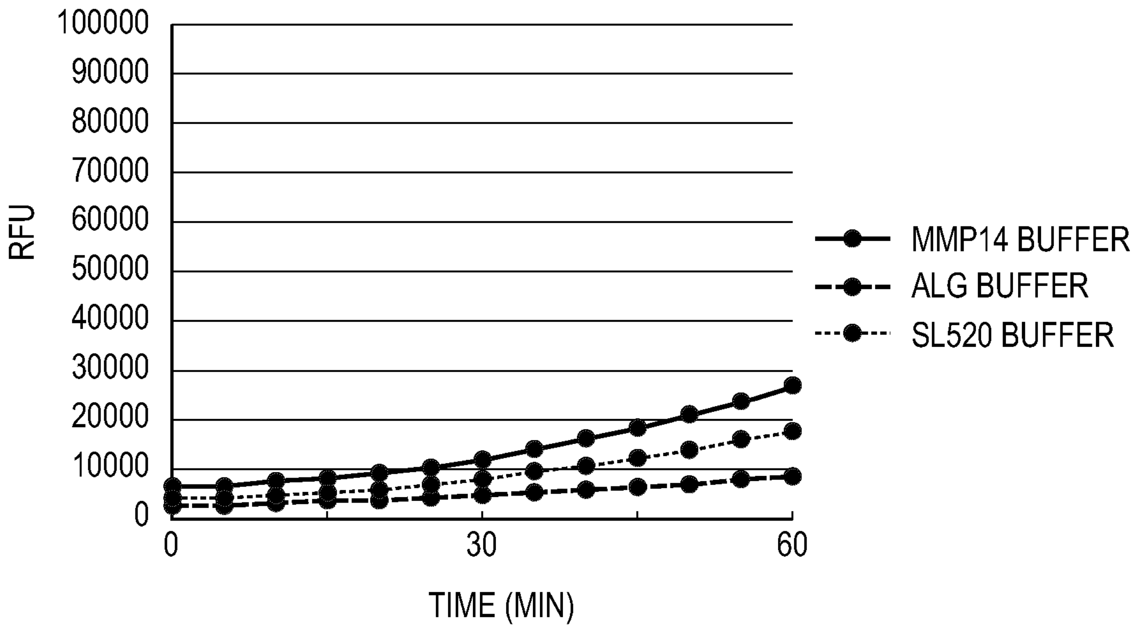
100000
90000
80000
70000
60000
50000
40000
30000
20000
10000
0
RFU
0
30
60
TIME (MIN)
MMP14 BUFFER
ALG BUFFER
SL520 BUFFER

FIG. 26A

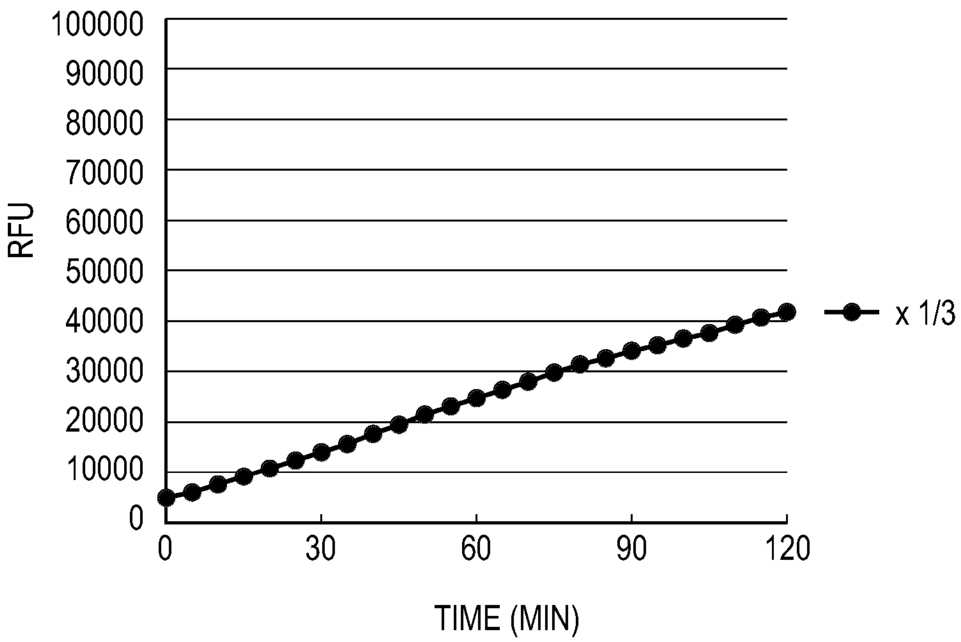
RFU
100000
90000
80000
70000
60000
50000
40000
30000
20000
10000
0
0
30
60
90
120
TIME (MIN)
x 1/3

FIG. 26B

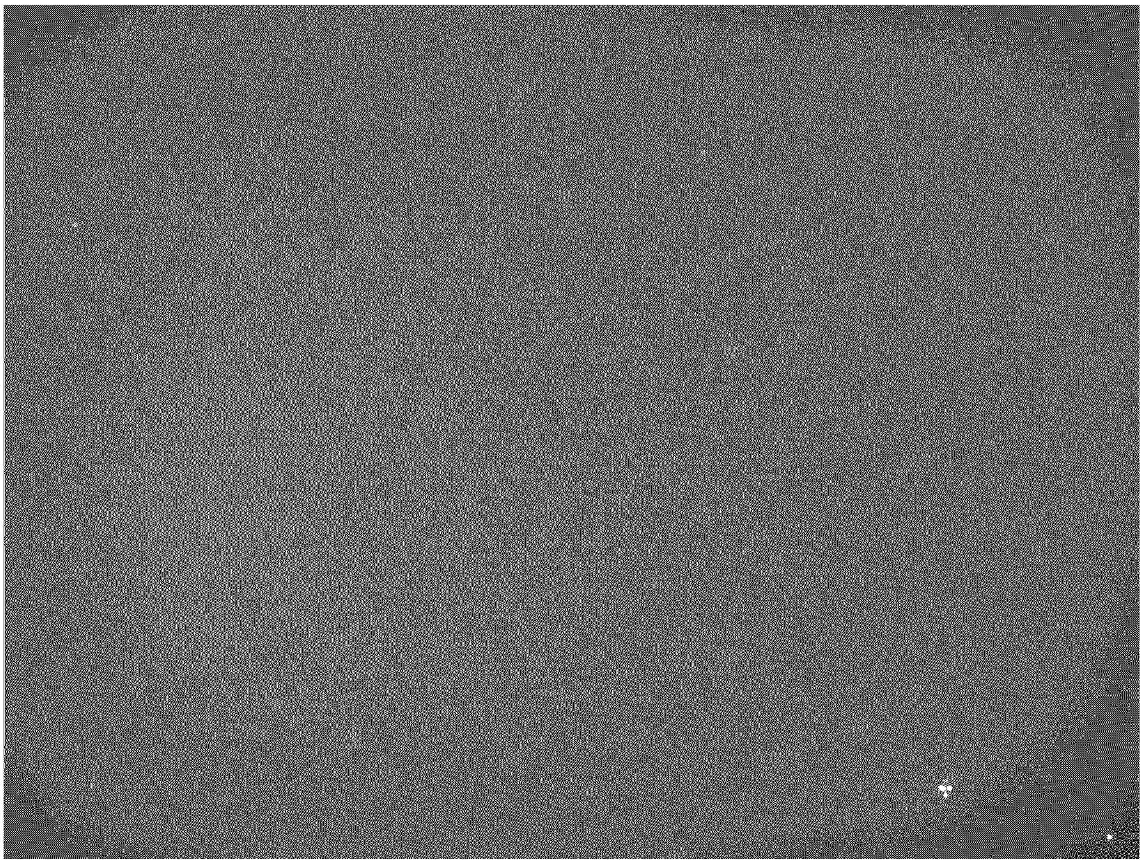

FIG. 26C

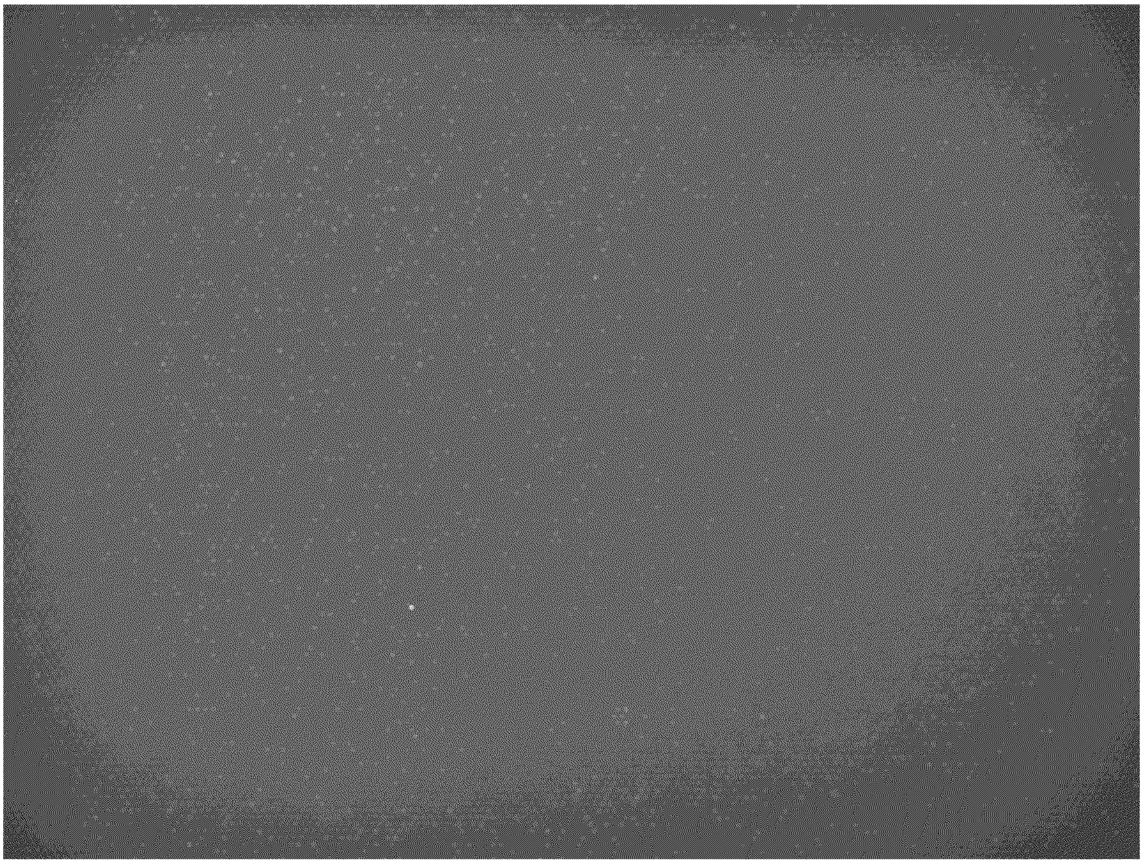

FIG. 26D

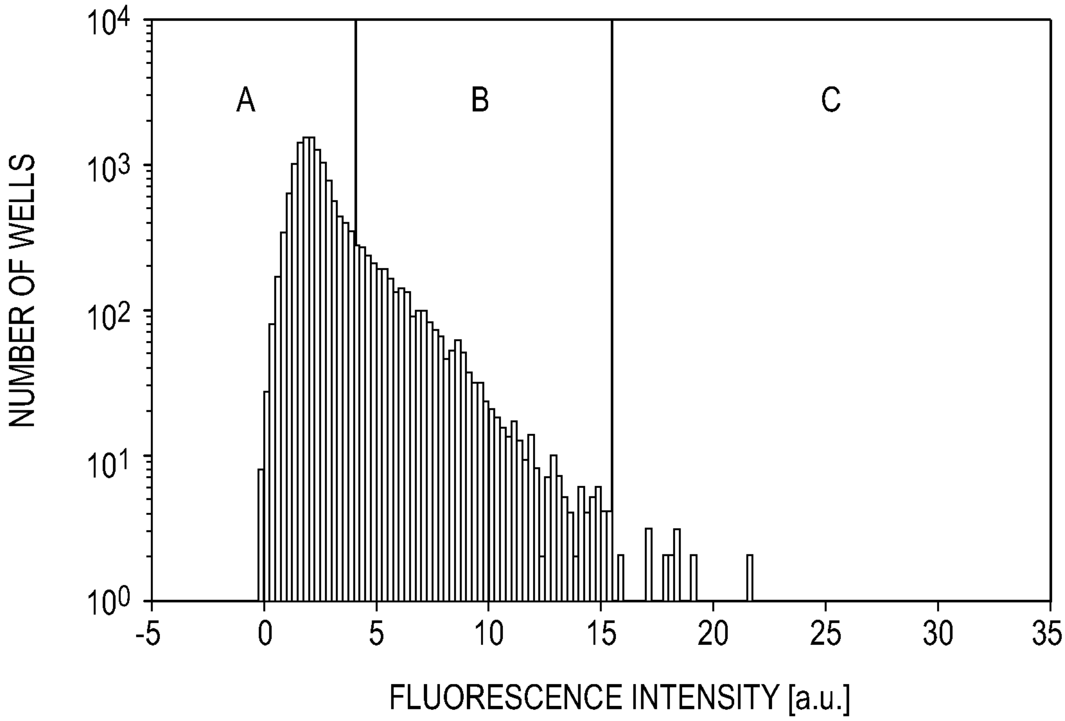

A
B
C
NUMBER OF WELLS
10^4
10^3
10^2
10^1
10^0
-5
0
5
10
15
20
25
30
35
FLUORESCENCE INTENSITY [a.u.]

FIG. 26E

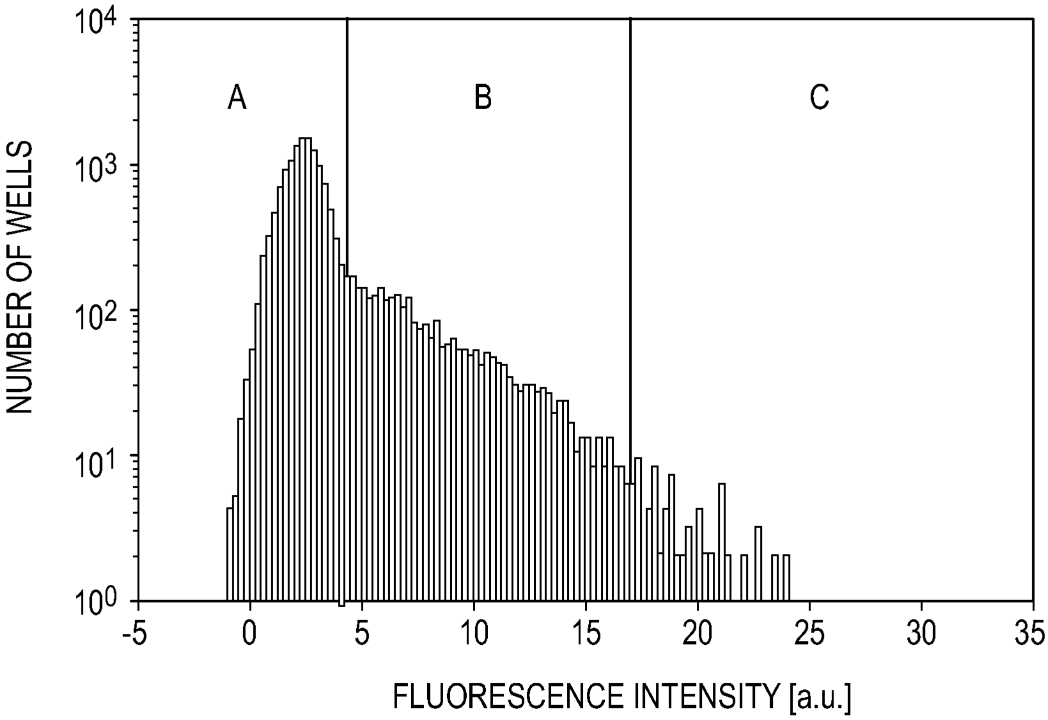
$10^4$
$10^3$
$10^2$
$10^1$
$10^0$
NUMBER OF WELLS
A
B
C
-5
0
5
10
15
20
25
30
35
FLUORESCENCE INTENSITY [a.u.]

FIG. 27

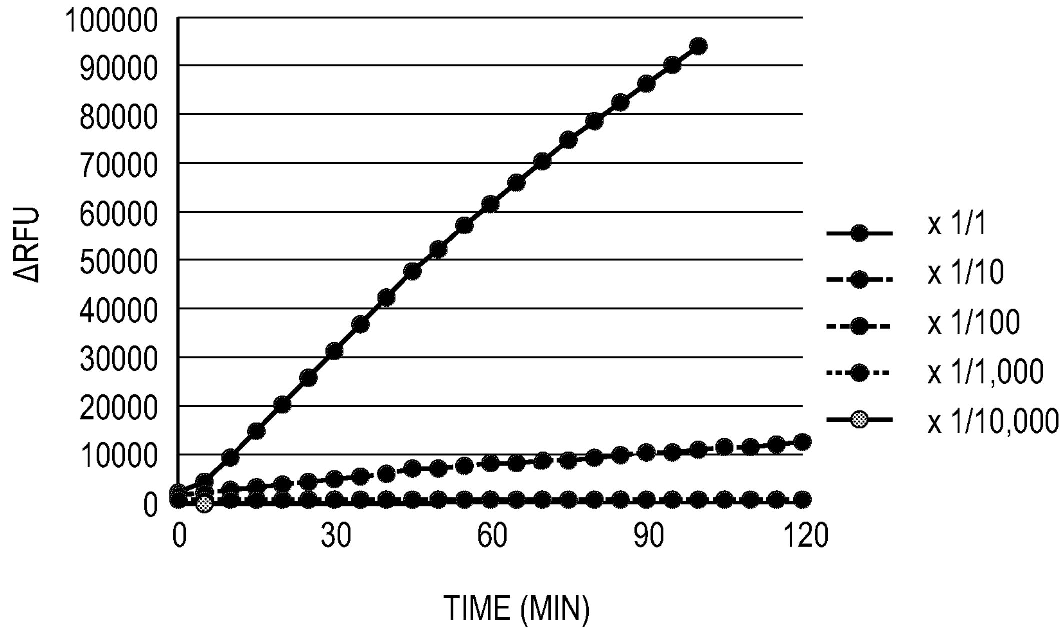
100000
90000
80000
70000
60000
50000
40000
30000
20000
10000
0
ΔRFU
0
30
60
90
120
TIME (MIN)
x 1/1
x 1/10
x 1/100
x 1/1,000
x 1/10,000

FIG. 28A

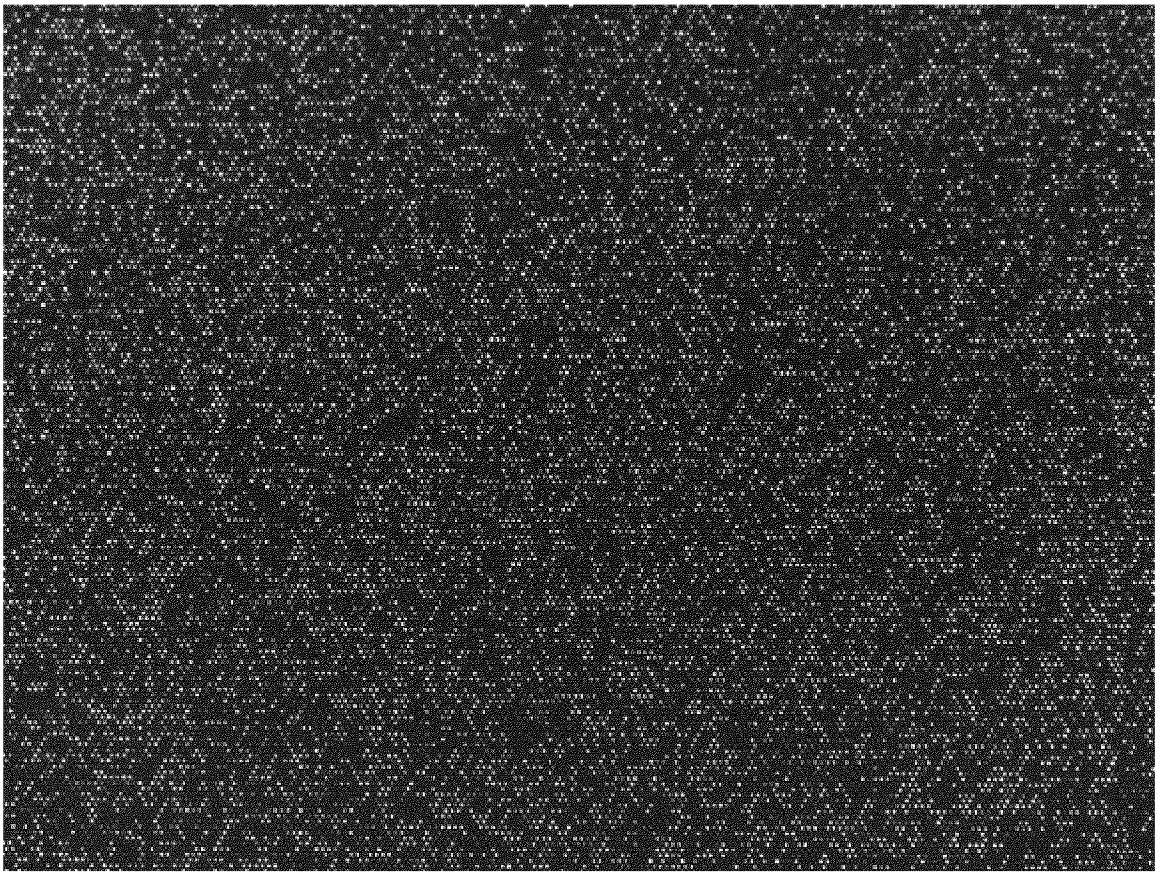

FIG. 28B

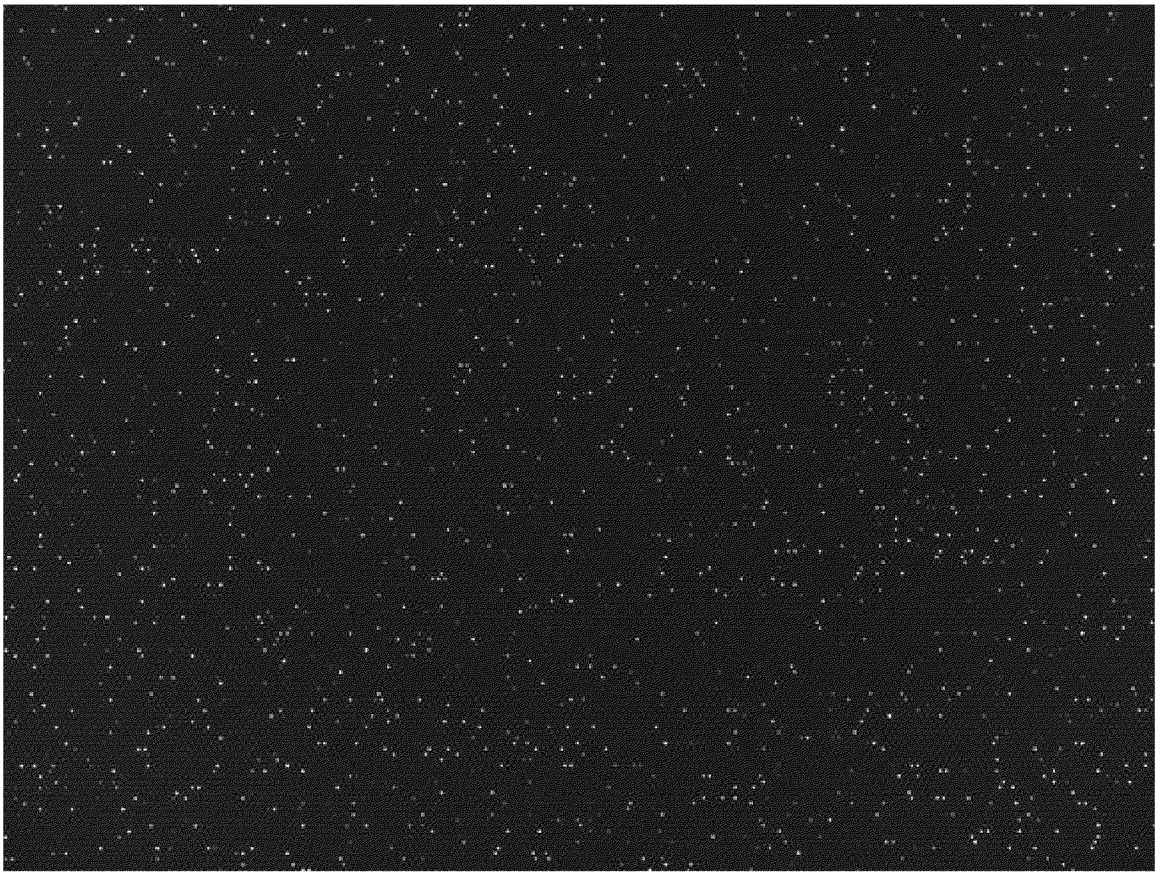

FIG. 28C

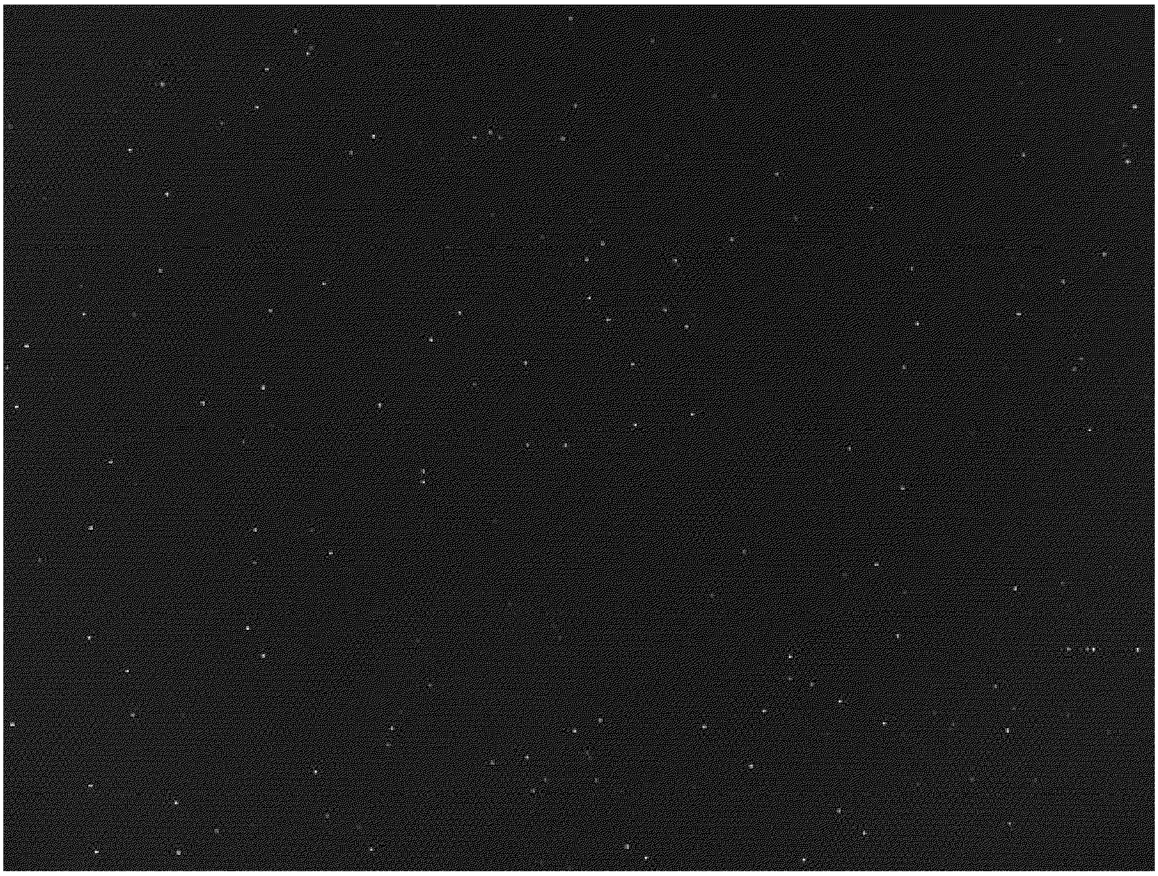

FIG. 28D

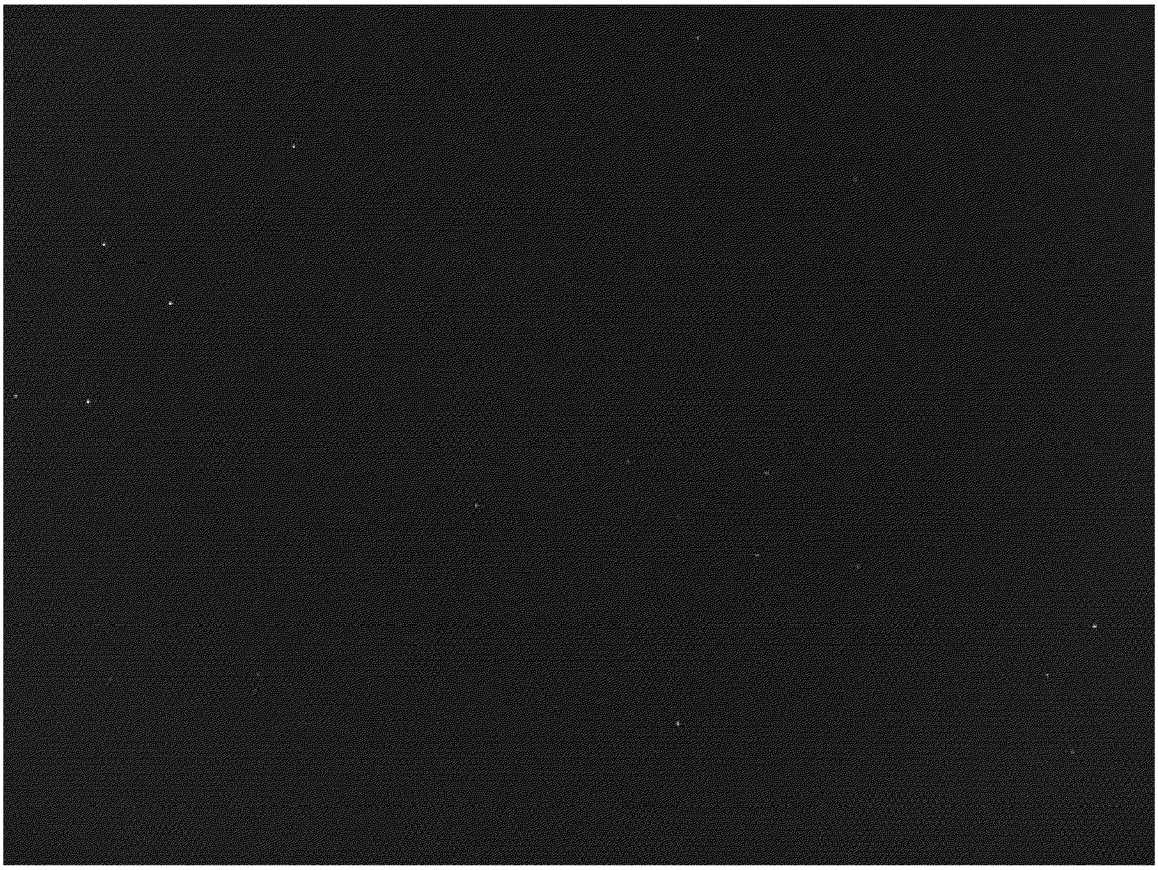

FIG. 28E

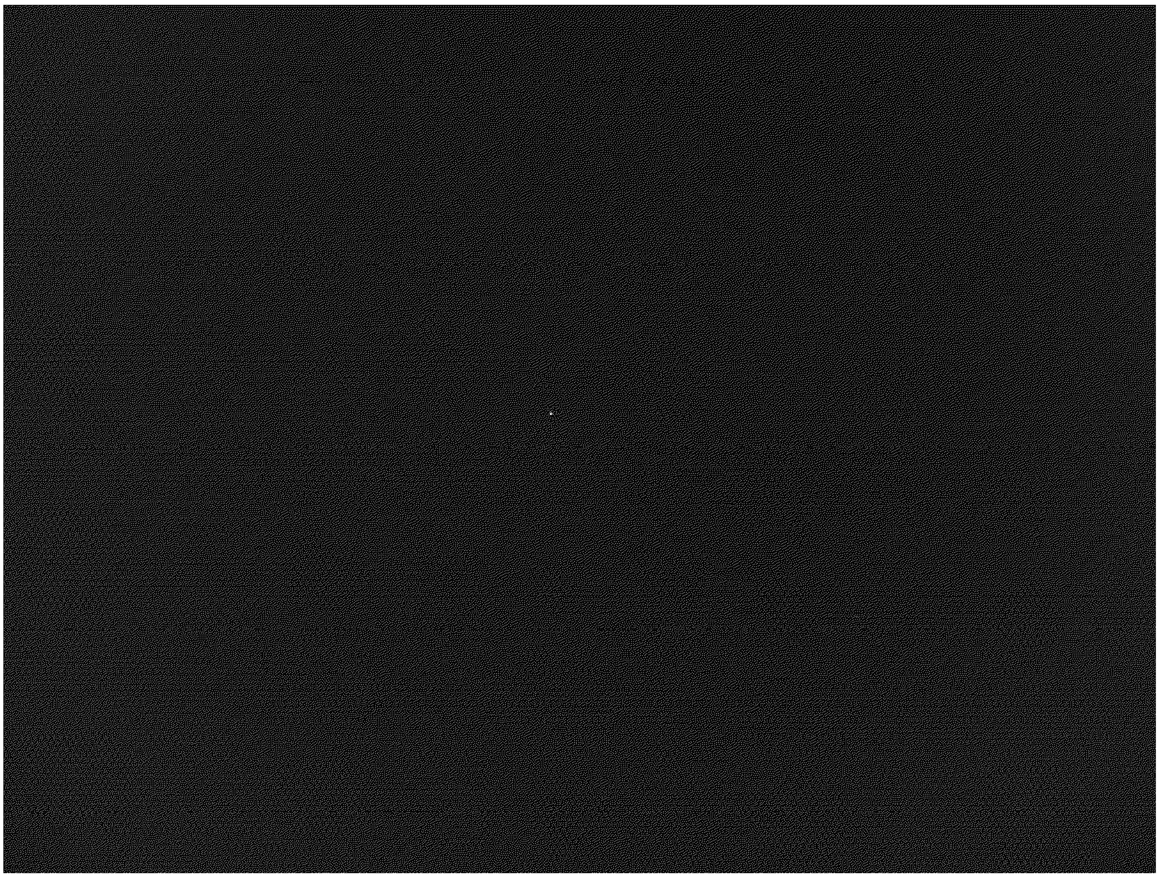

FIG. 28F

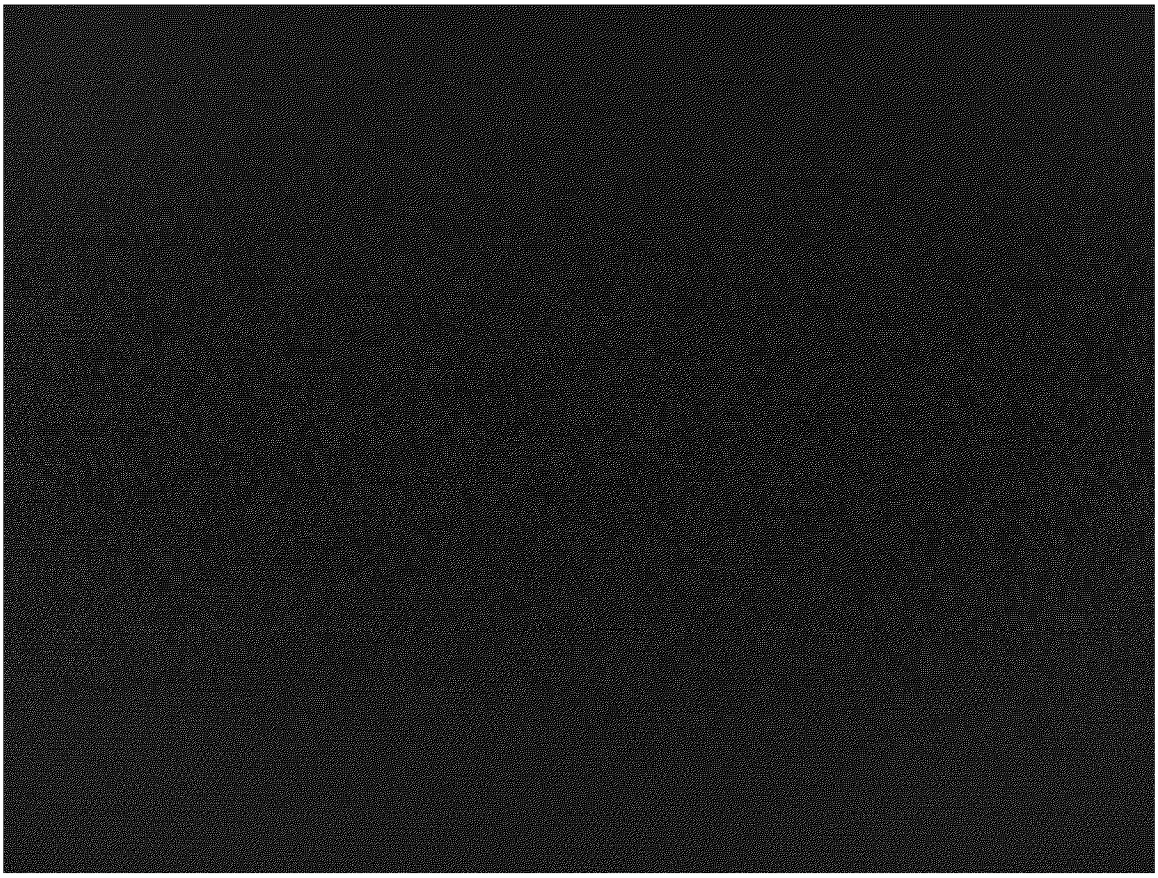

FIG. 28G

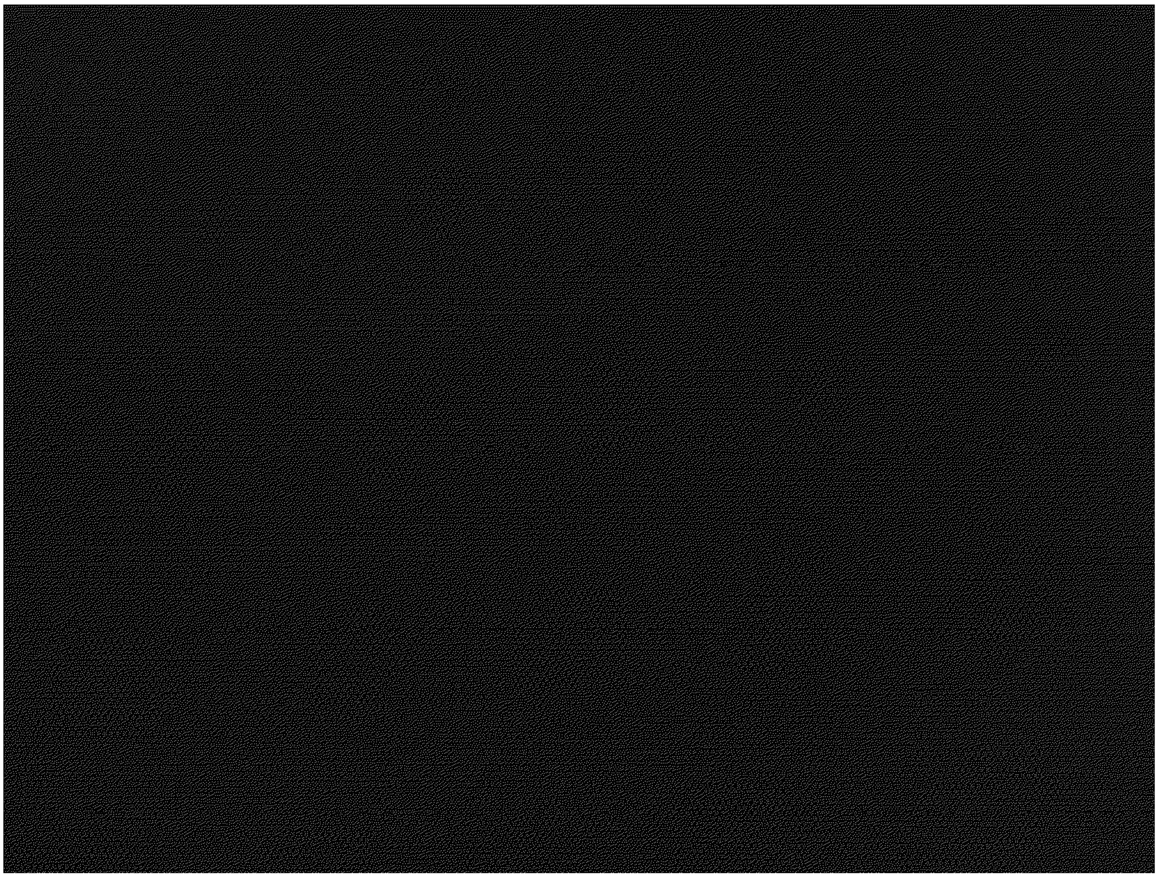

FIG. 28H

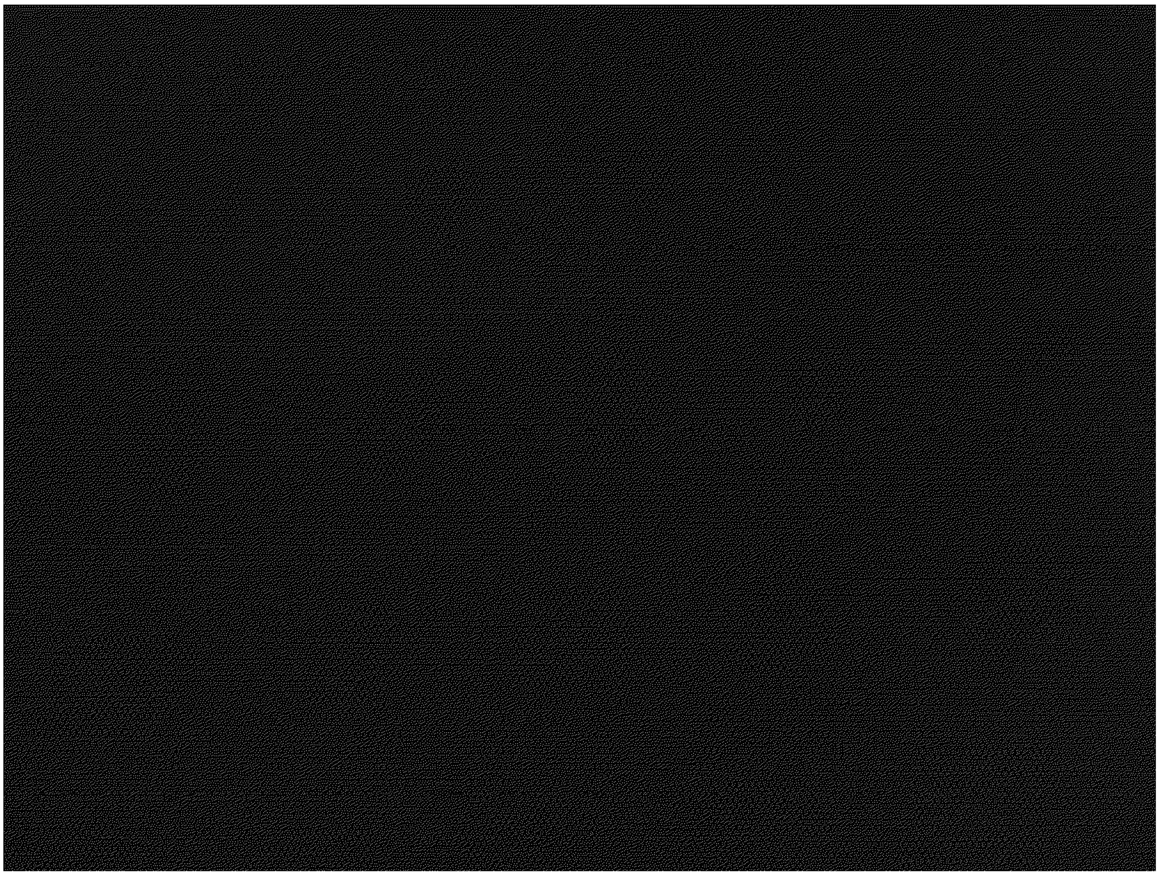

FIG. 28I

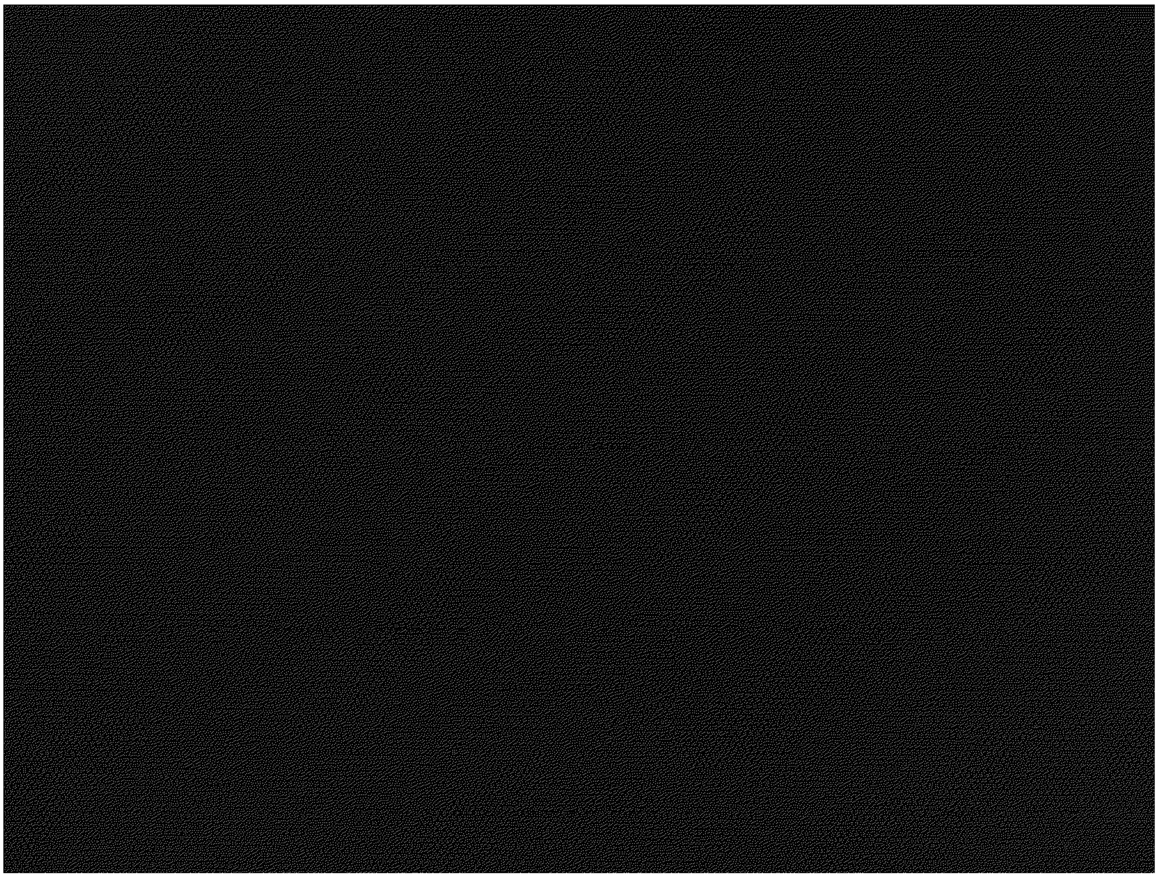

FIG. 28J

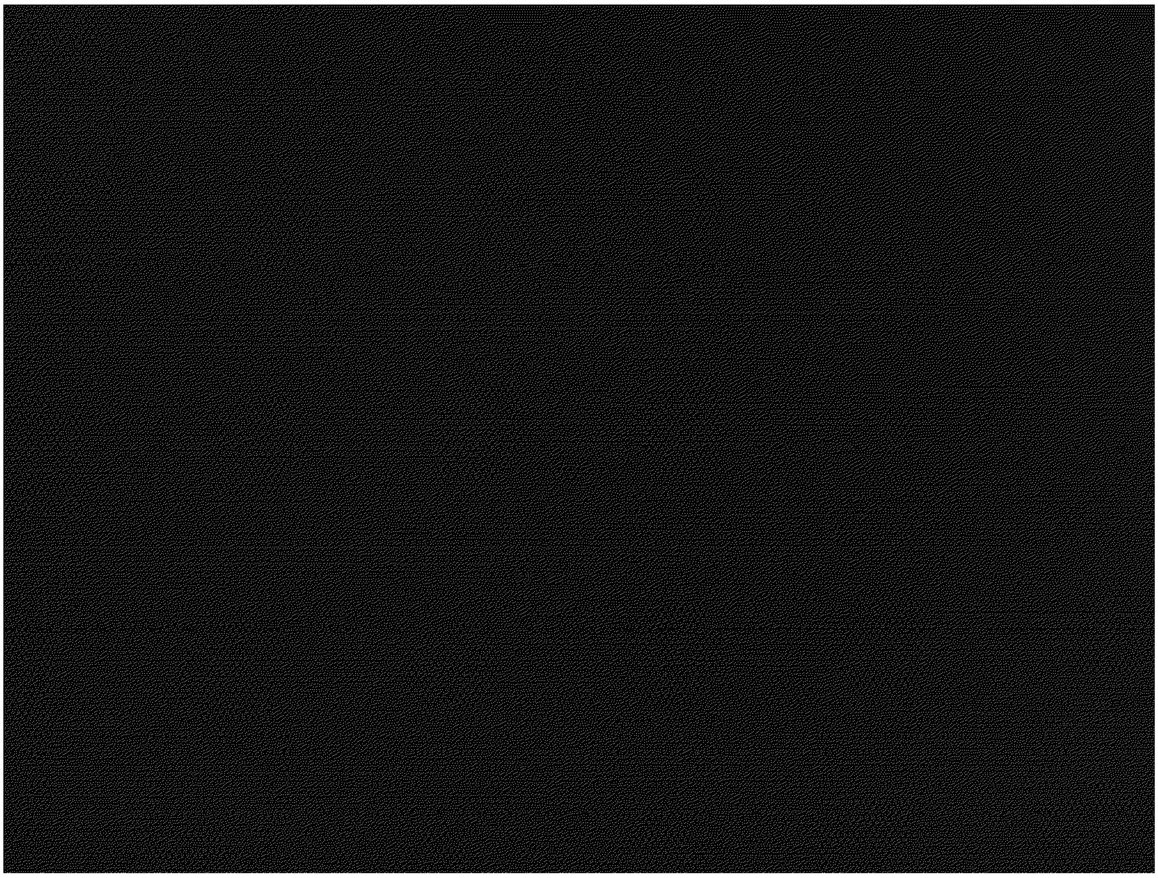

FIG. 28K

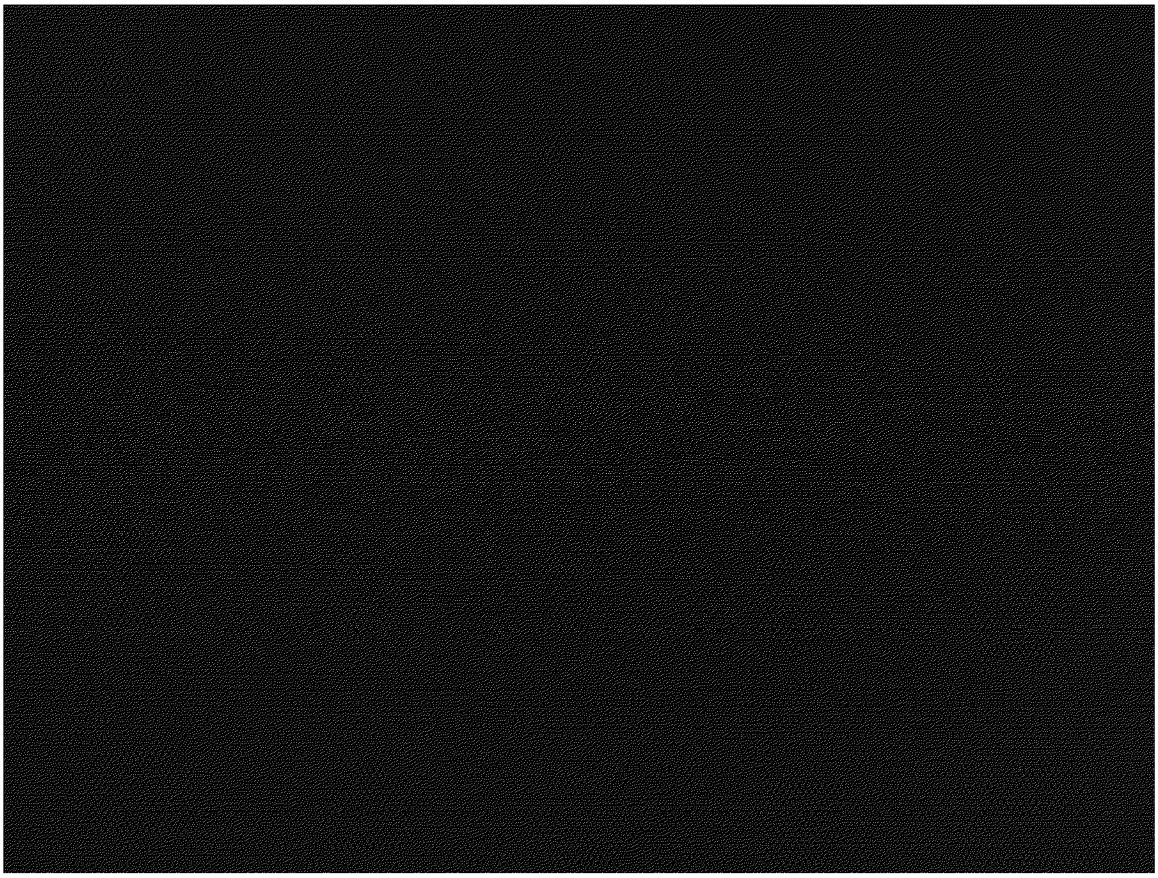

FIG. 28L

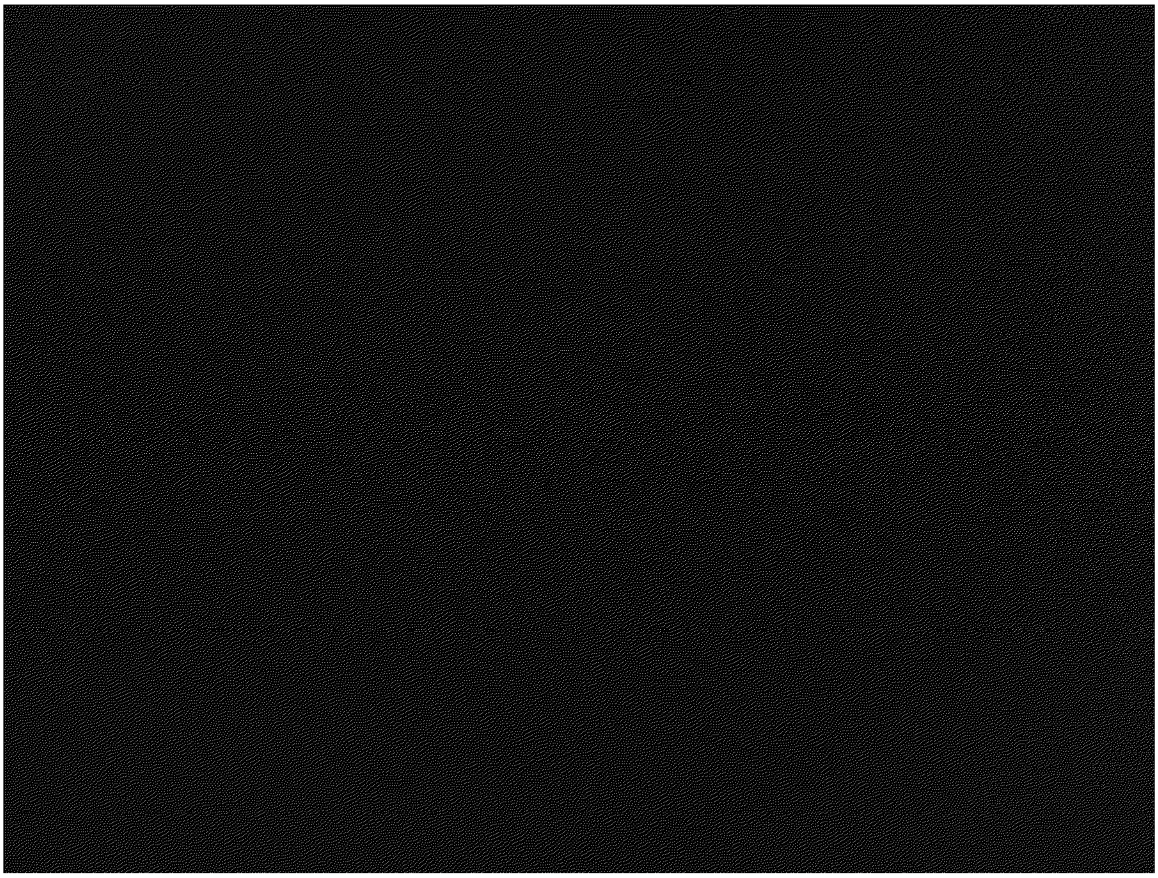

FIG. 29A

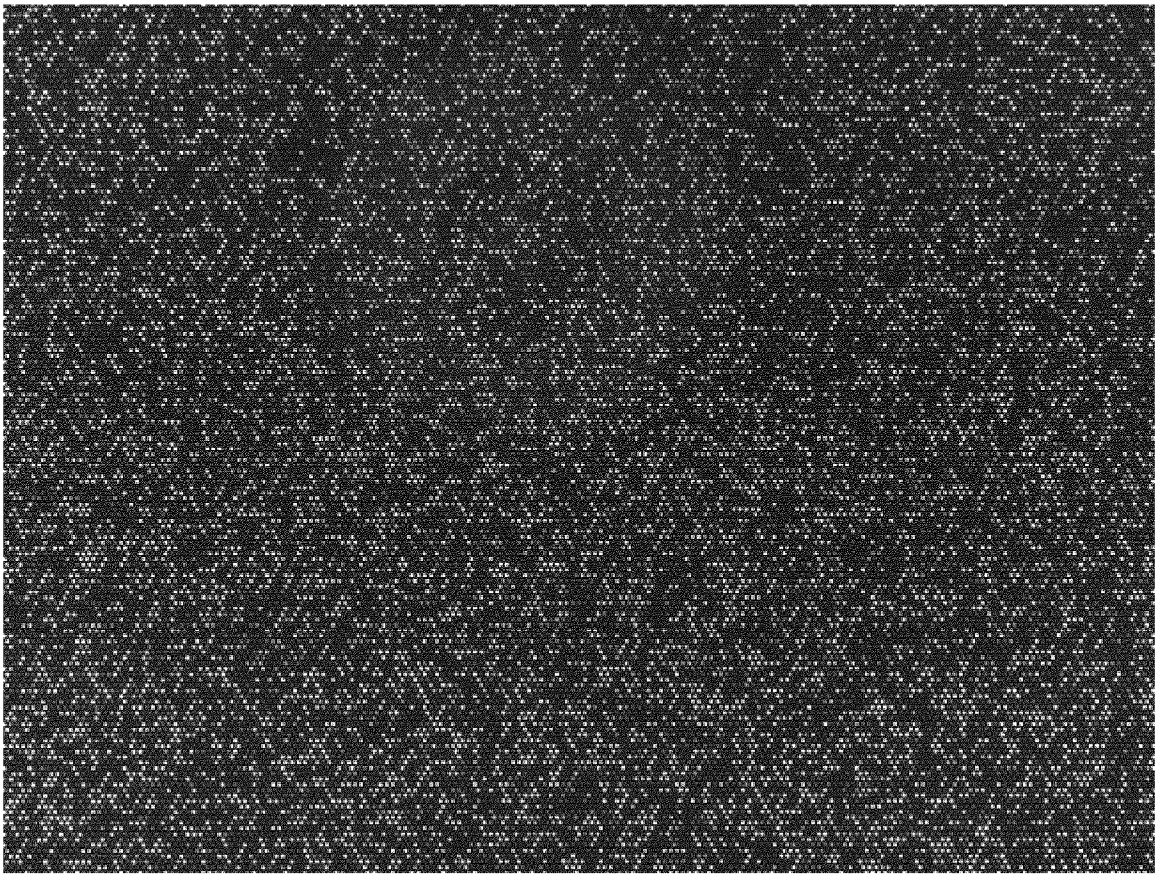

FIG. 29B

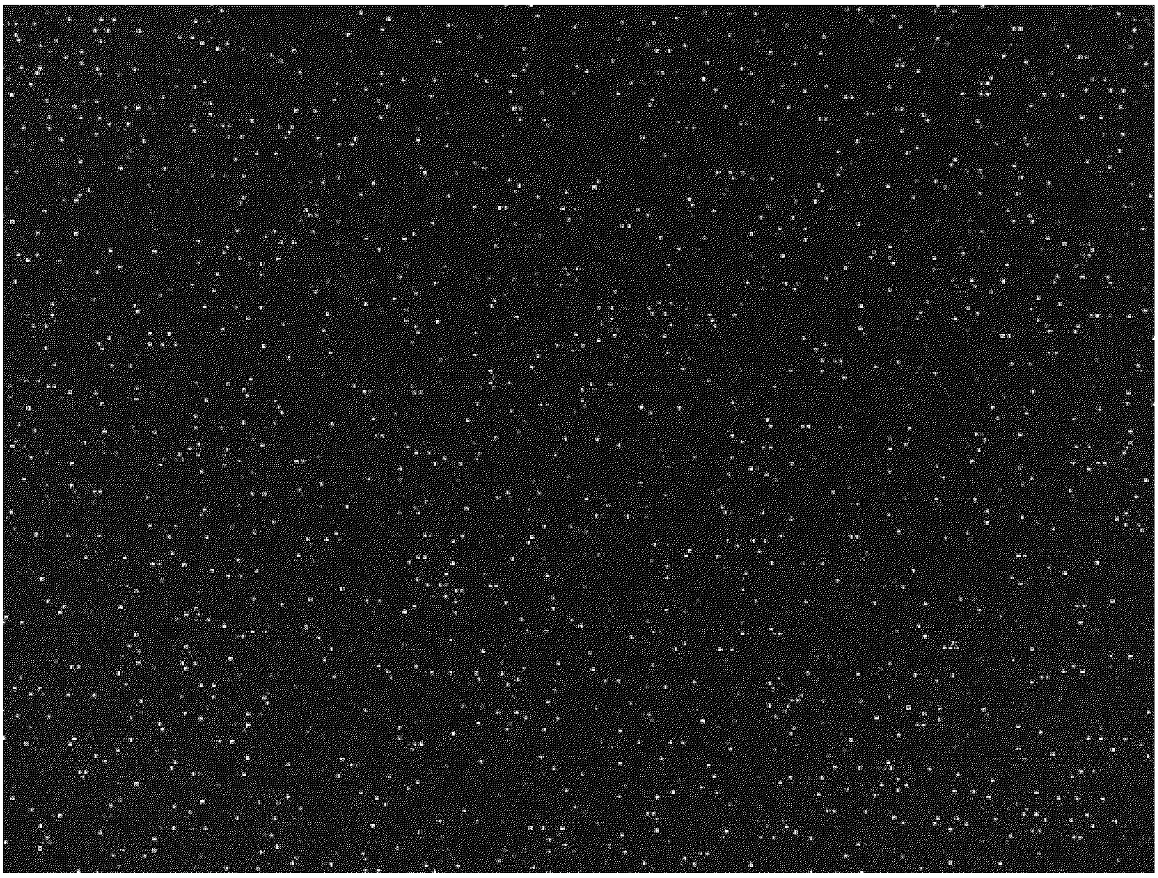

FIG. 29C

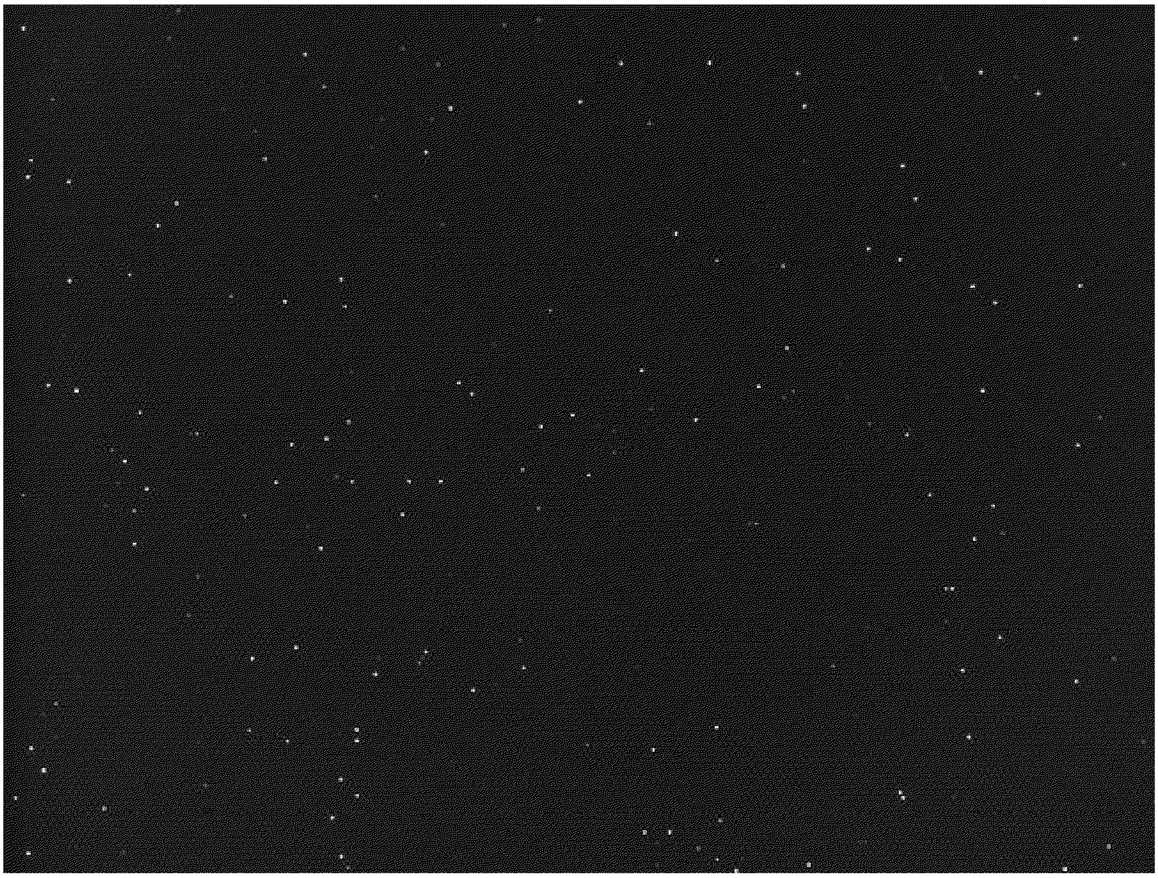

FIG. 29D

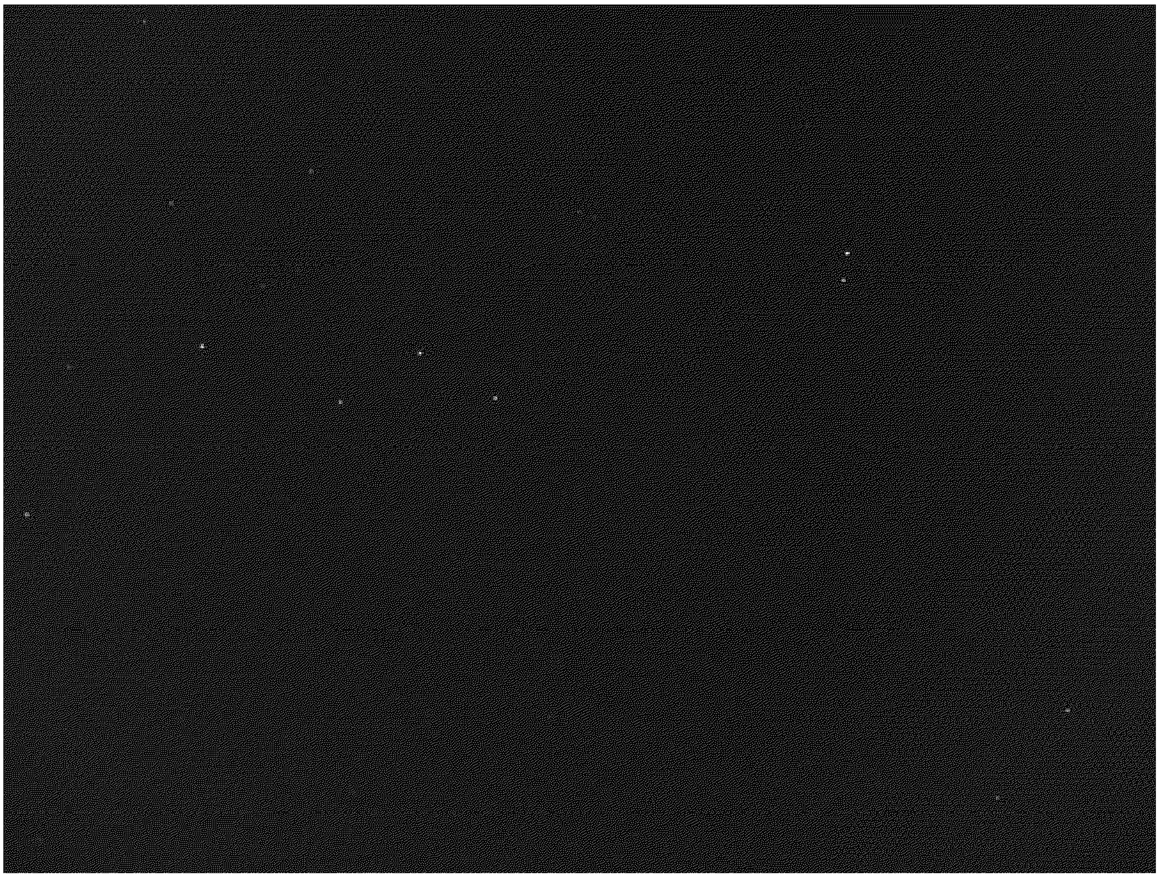

FIG. 29E

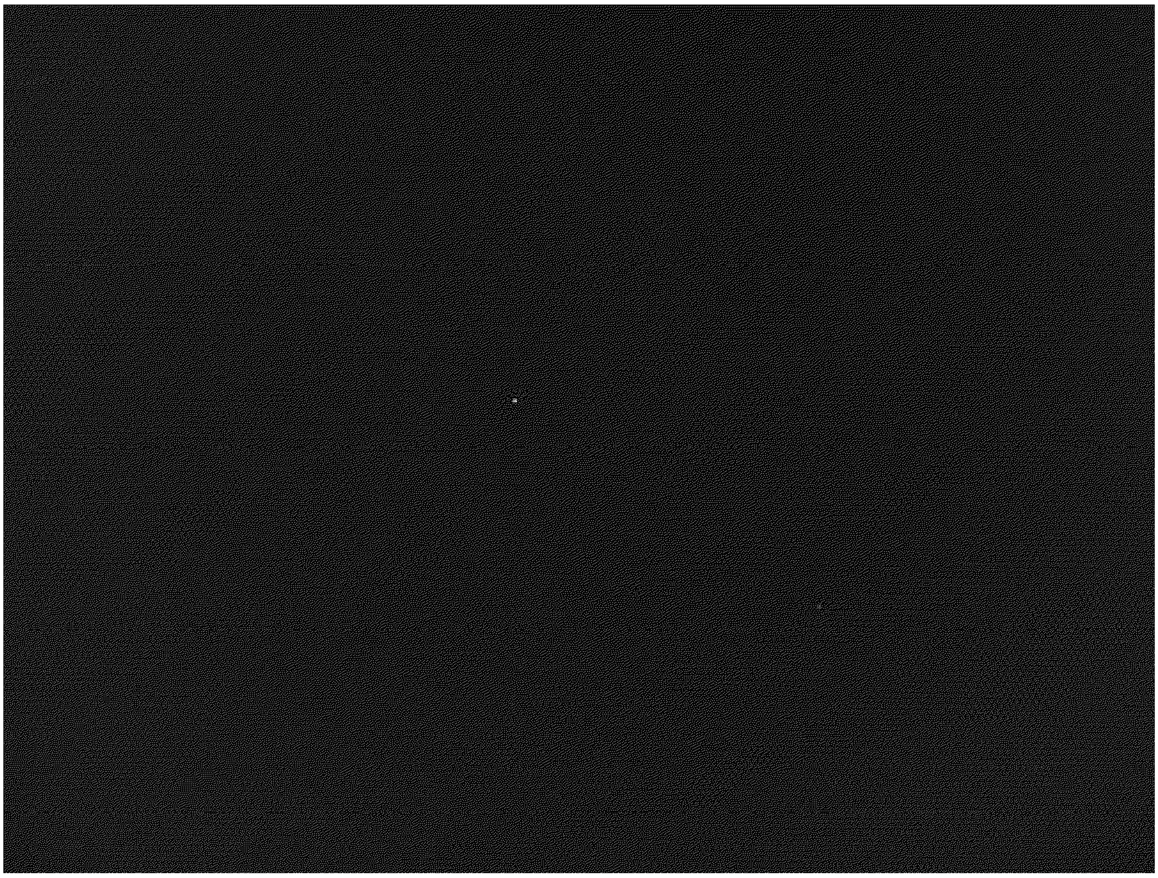

FIG. 29F

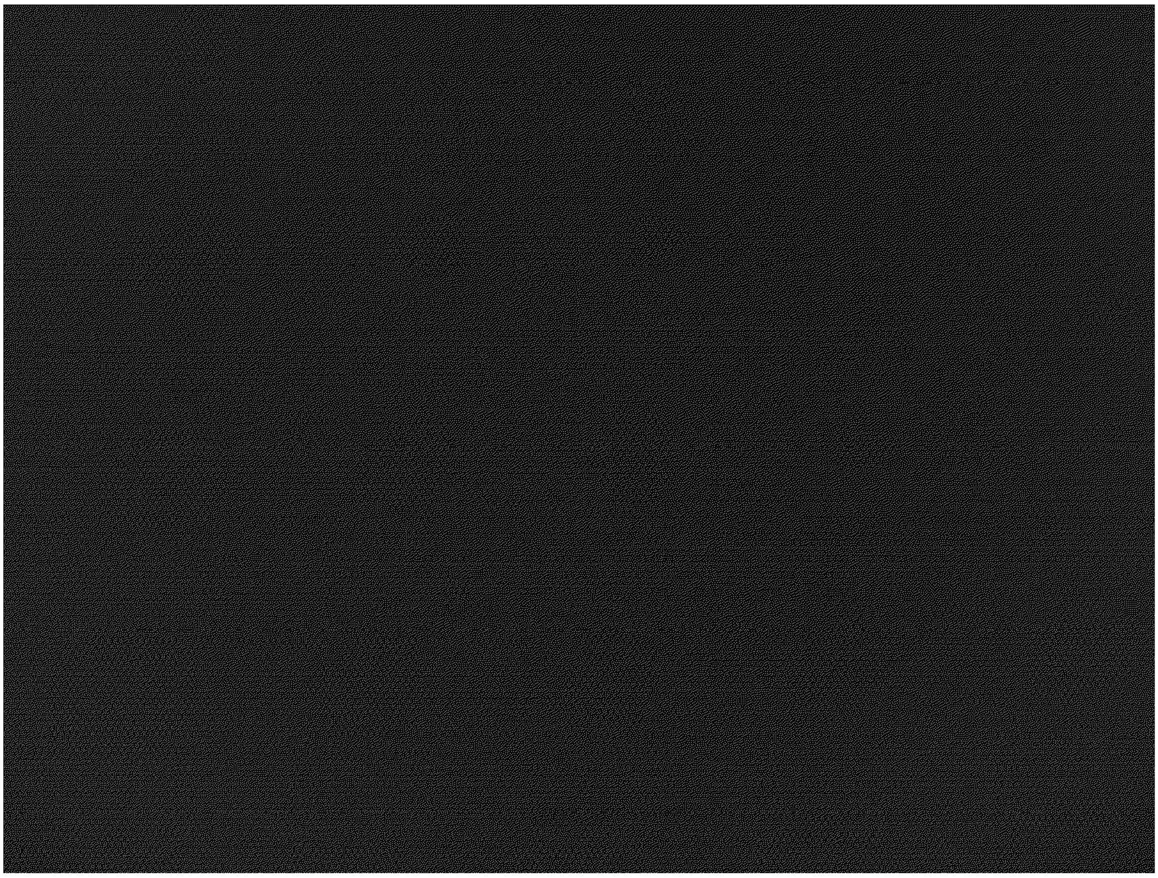

FIG. 29G

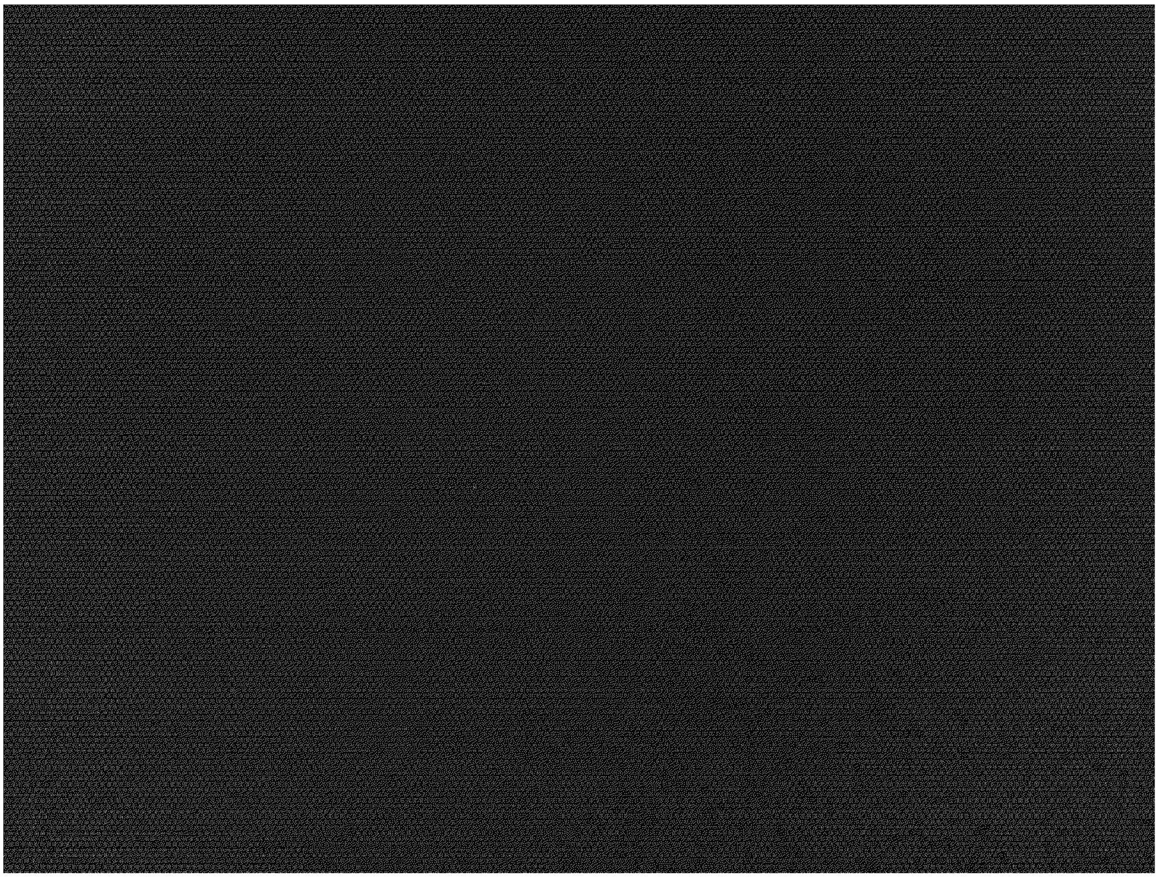

FIG. 29H

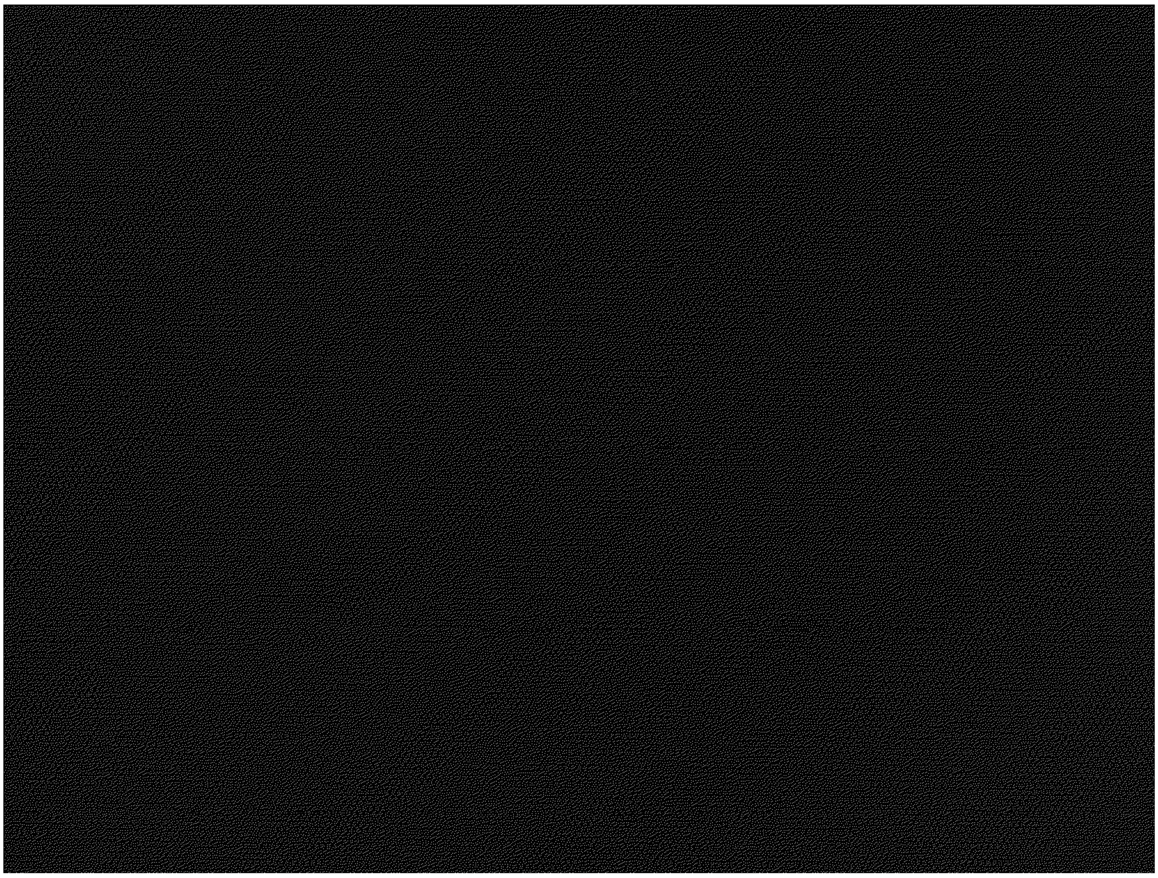

FIG. 29I

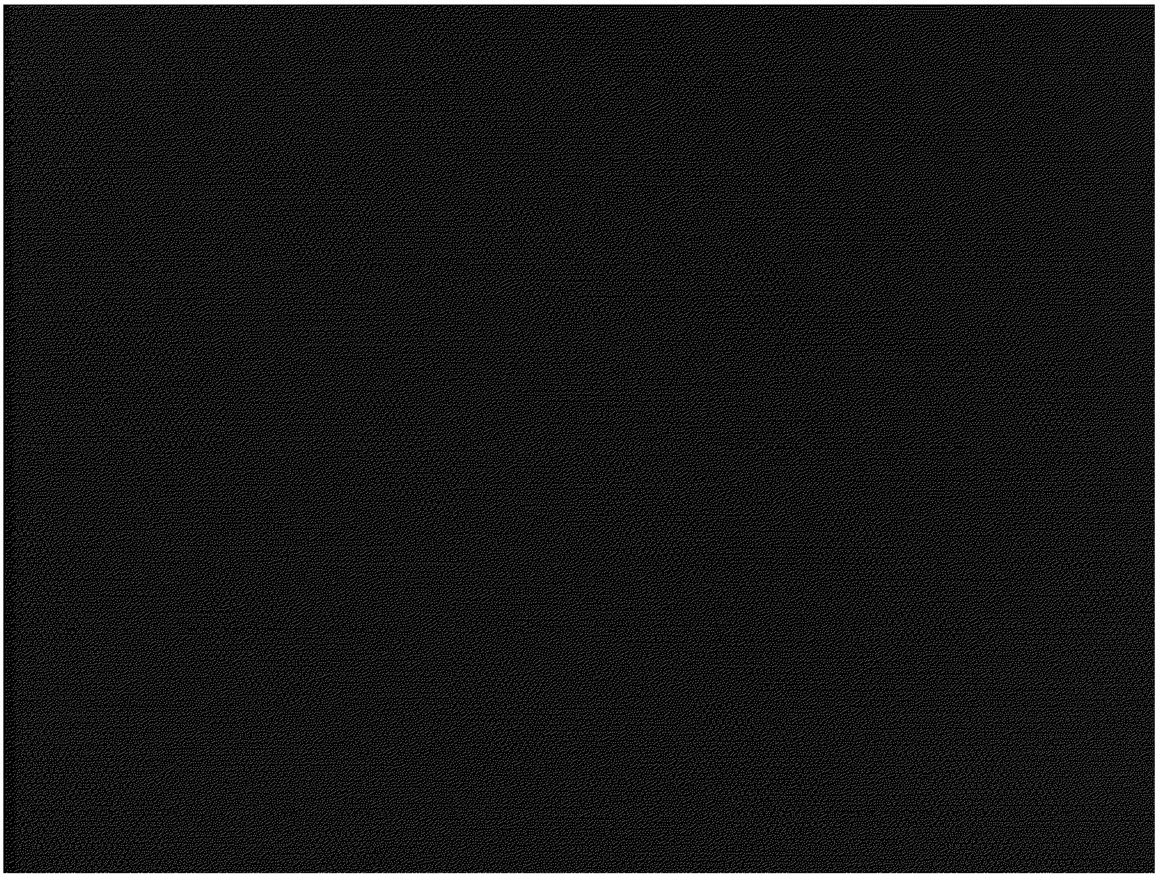

FIG. 29J

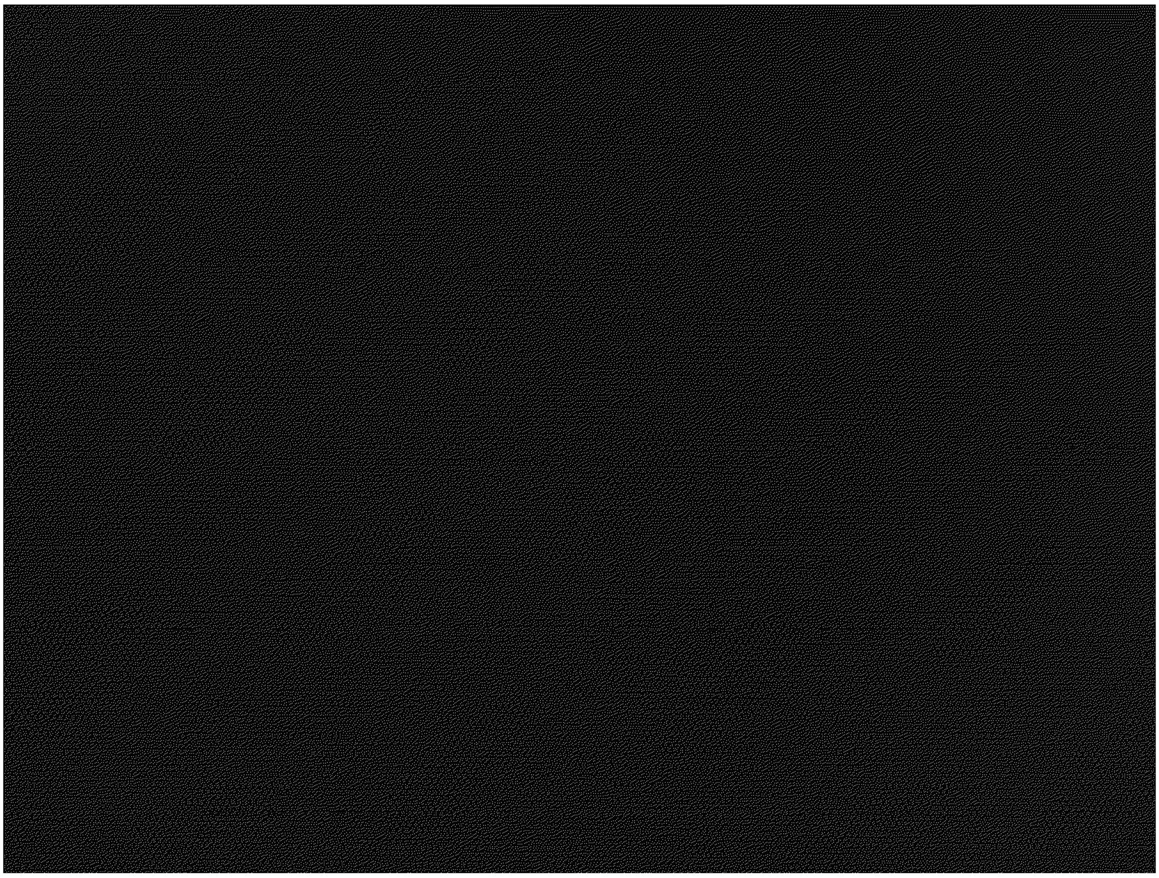

FIG. 29K

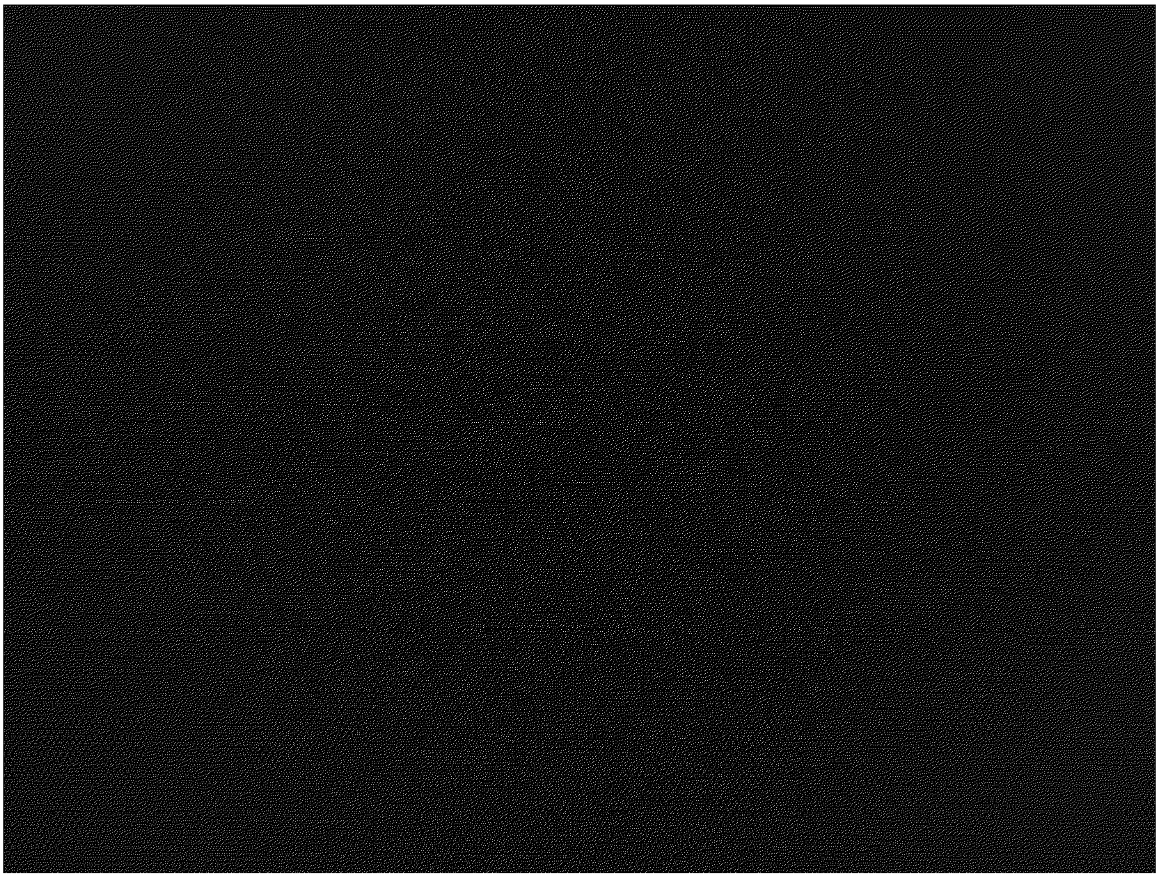

FIG. 29L

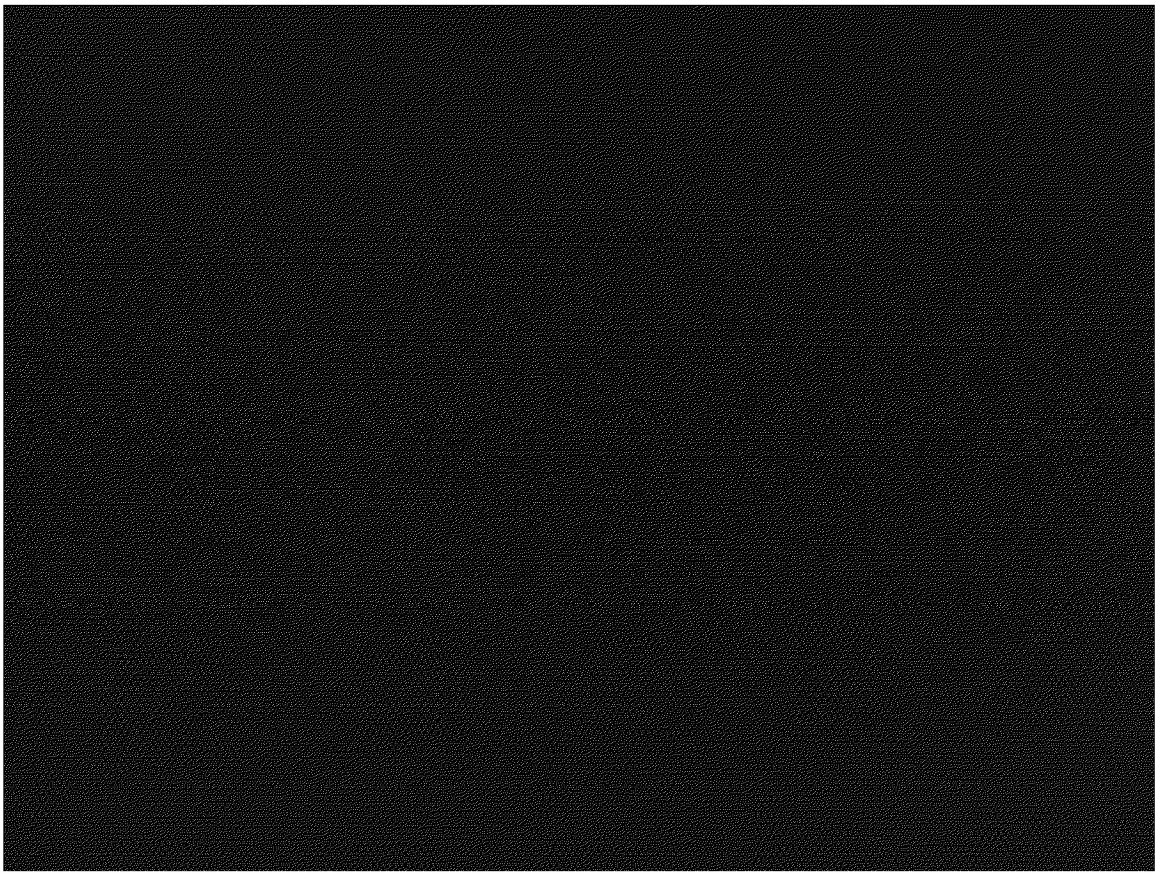

FIG. 30A

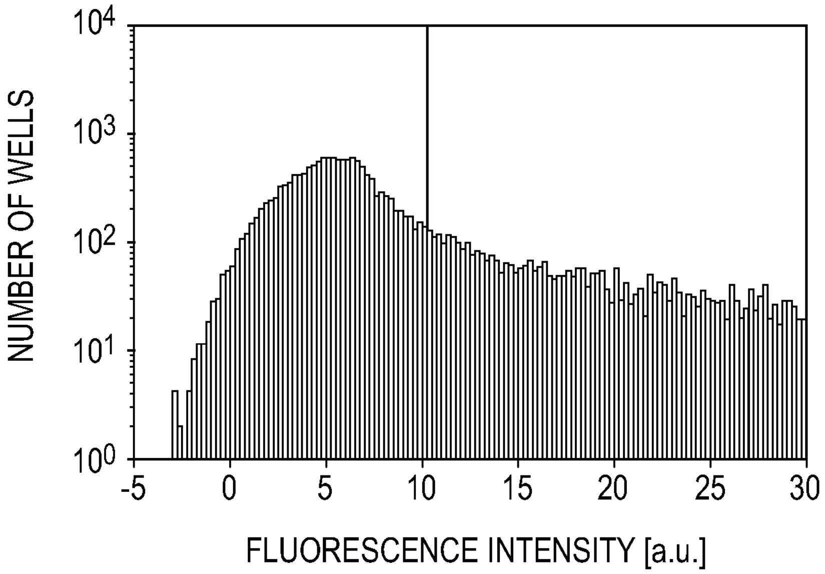
10^4
10^3
10^2
10^1
10^0
NUMBER OF WELLS
-5
0
5
10
15
20
25
30
FLUORESCENCE INTENSITY [a.u.]

FIG. 30B

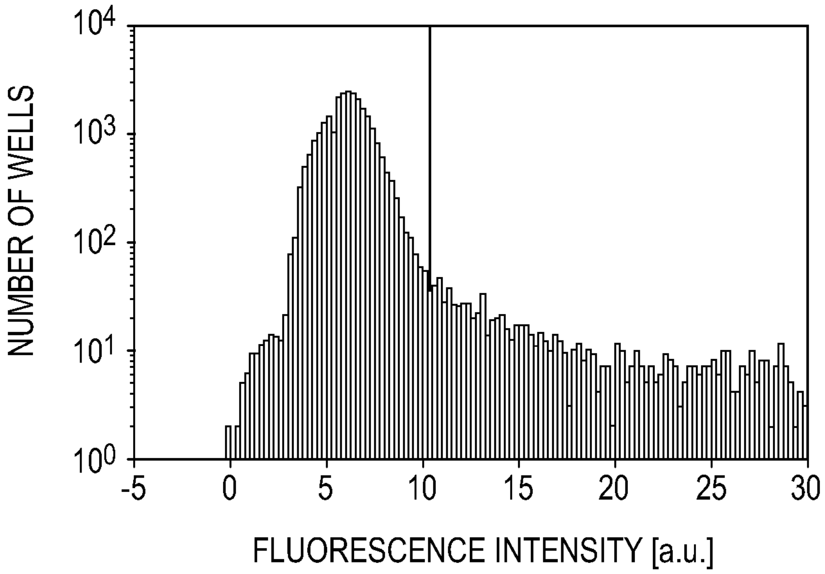

NUMBER OF WELLS
$10^4$
$10^3$
$10^2$
$10^1$
$10^0$
-5
0
5
10
15
20
25
30
FLUORESCENCE INTENSITY [a.u.]

FIG. 30C

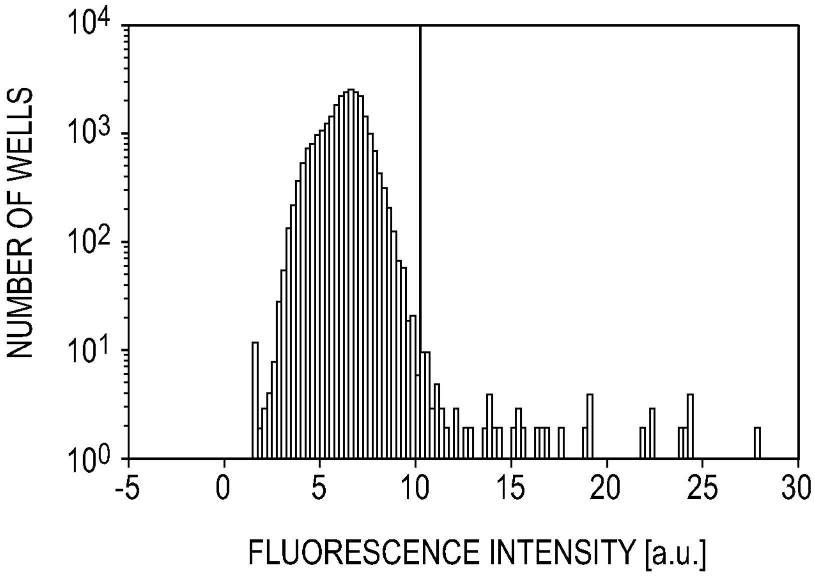

$10^4$
$10^3$
$10^2$
$10^1$
$10^0$
NUMBER OF WELLS
-5
0
5
10
15
20
25
30
FLUORESCENCE INTENSITY [a.u.]

FIG. 30D

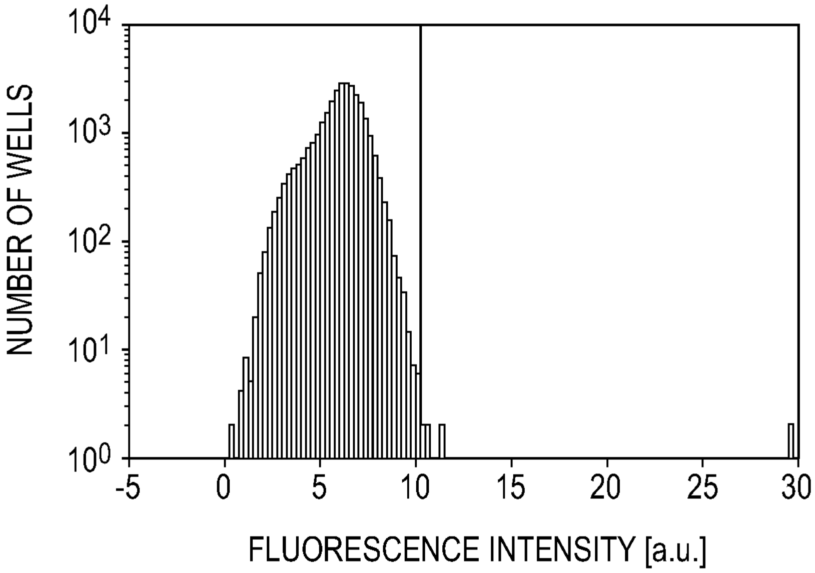
$10^4$
$10^3$
$10^2$
$10^1$
$10^0$
NUMBER OF WELLS
-5
0
5
10
15
20
25
30
FLUORESCENCE INTENSITY [a.u.]

FIG. 30E

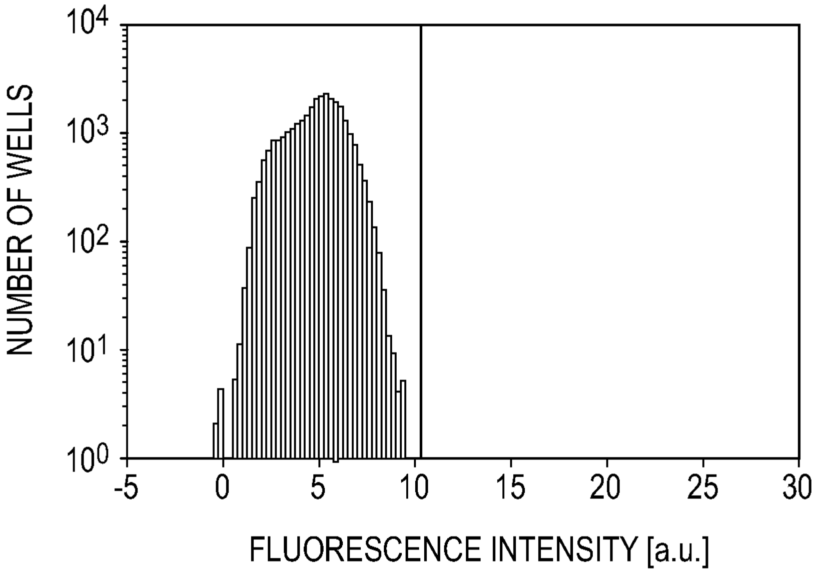
NUMBER OF WELLS
$10^4$
$10^3$
$10^2$
$10^1$
$10^0$
-5
0
5
10
15
20
25
30
FLUORESCENCE INTENSITY [a.u.]

FIG. 31A

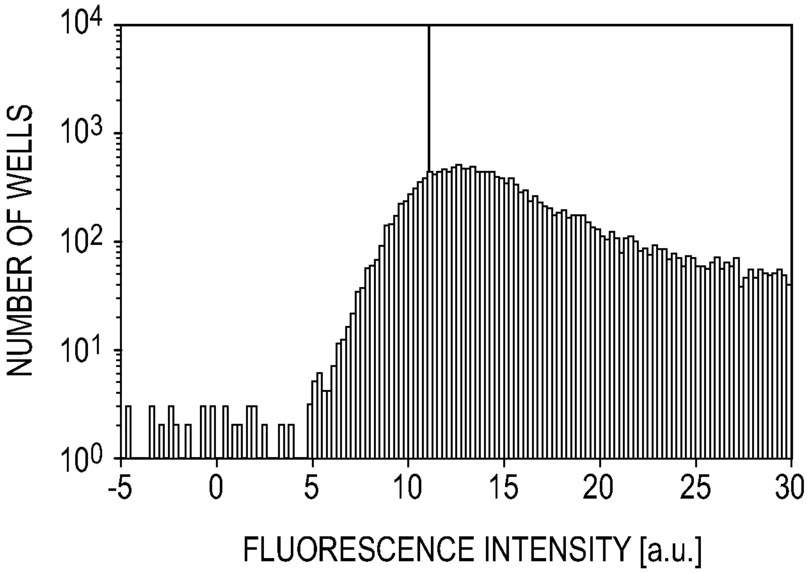

NUMBER OF WELLS
10^4
10^3
10^2
10^1
10^0
-5
0
5
10
15
20
25
30
FLUORESCENCE INTENSITY [a.u.]

FIG. 31B

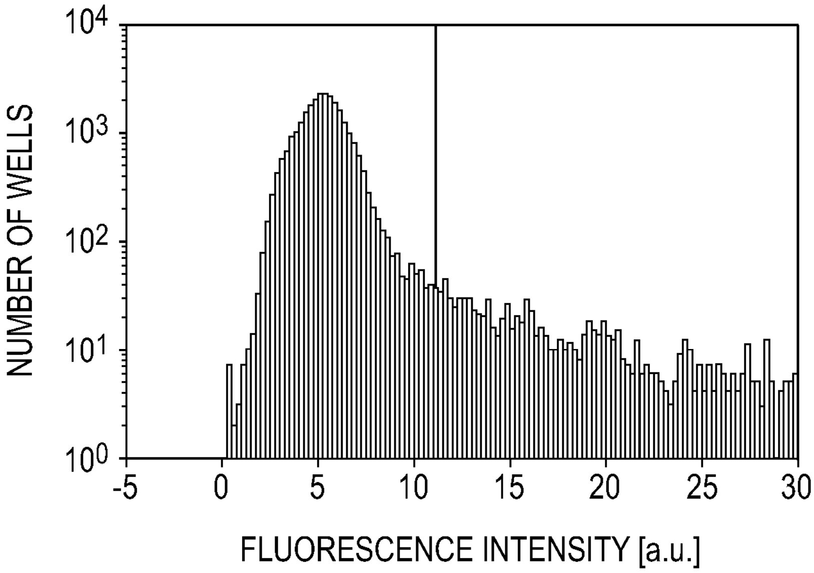
$10^4$
$10^3$
$10^2$
$10^1$
$10^0$
NUMBER OF WELLS
-5
0
5
10
15
20
25
30
FLUORESCENCE INTENSITY [a.u.]

FIG. 31C

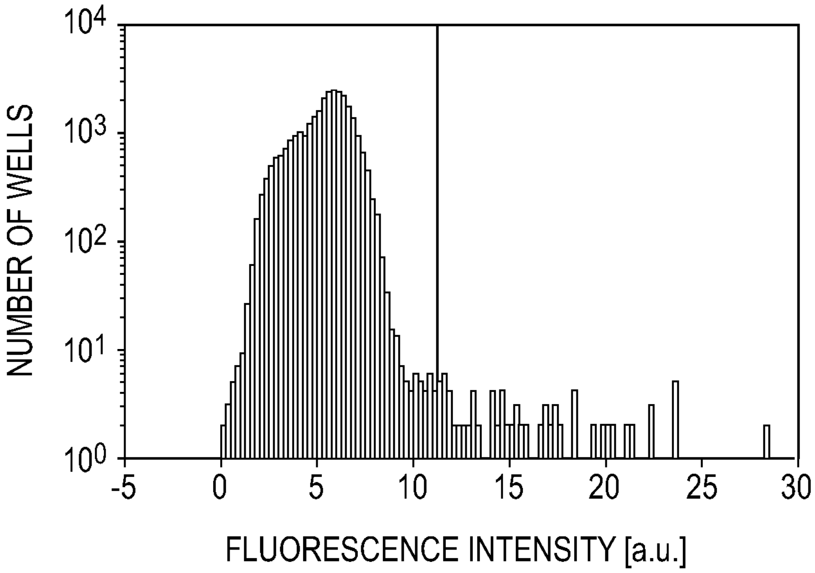
NUMBER OF WELLS
$10^4$
$10^3$
$10^2$
$10^1$
$10^0$
-5
0
5
10
15
20
25
30
FLUORESCENCE INTENSITY [a.u.]

FIG. 31D

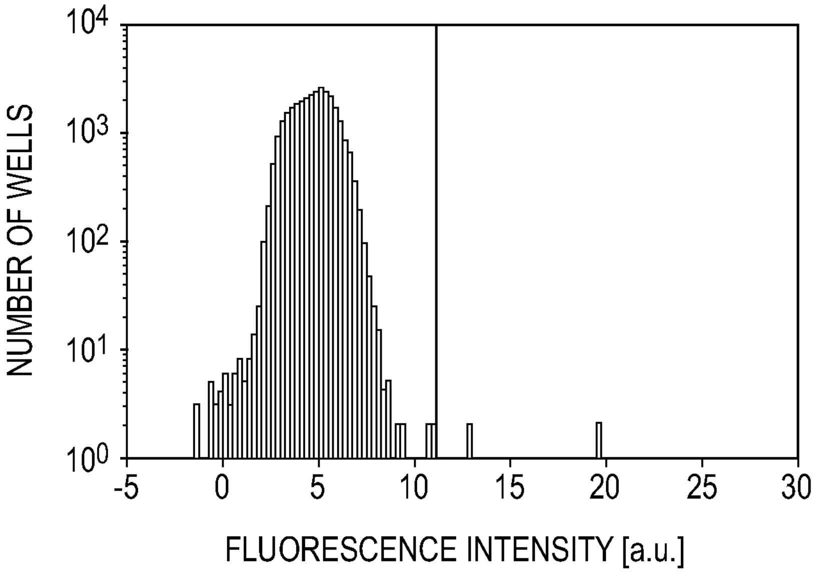
$10^4$
$10^3$
$10^2$
$10^1$
$10^0$
NUMBER OF WELLS
-5
0
5
10
15
20
25
30
FLUORESCENCE INTENSITY [a.u.]

FIG. 31E

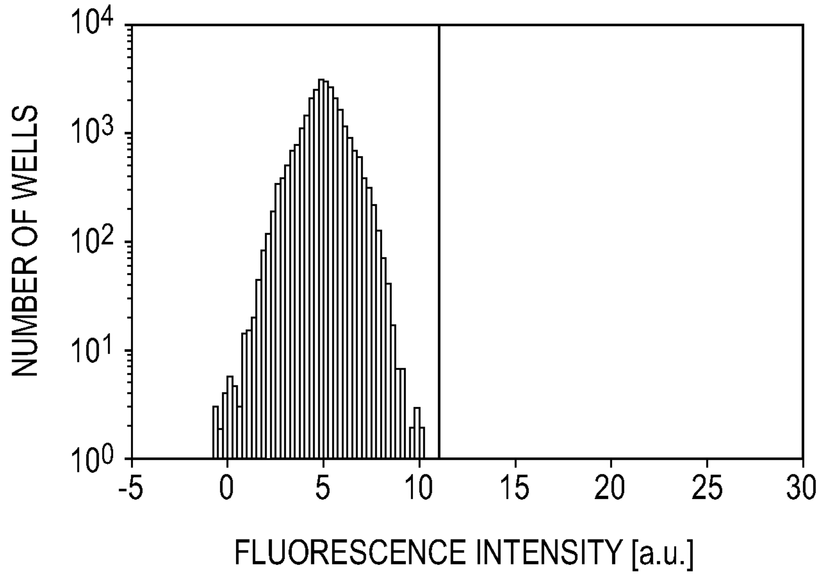
NUMBER OF WELLS
$10^4$
$10^3$
$10^2$
$10^1$
$10^0$
-5
0
5
10
15
20
25
30
FLUORESCENCE INTENSITY [a.u.]

FIG. 32

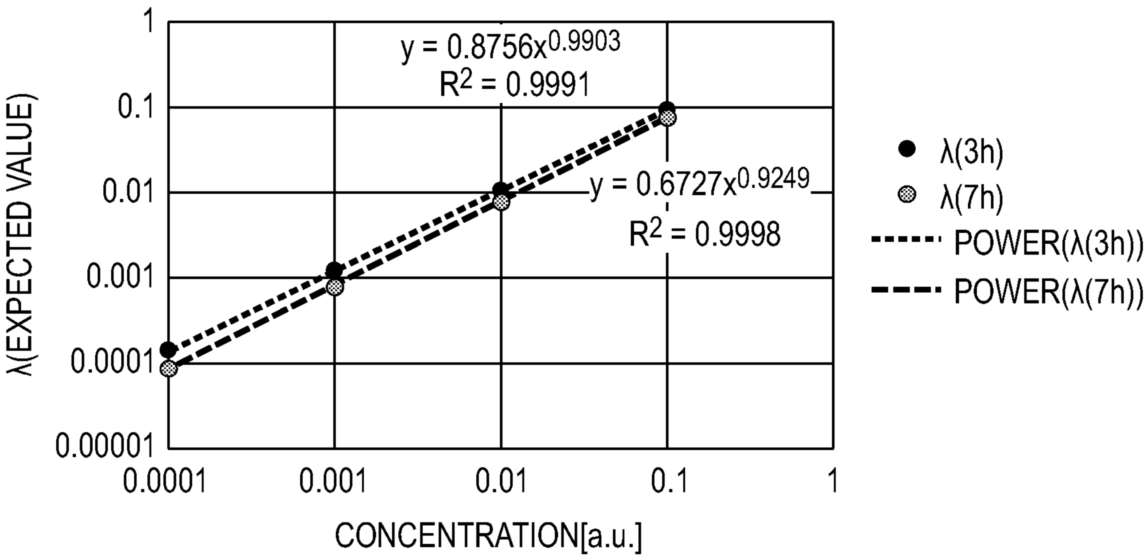
y = 0.8756x^0.9903
R2 = 0.9991
y = 0.6727x^0.9249
R2 = 0.9998
1
0.1
0.01
0.001
0.0001
0.00001
λ(EXPECTED VALUE)
0.0001
0.001
0.01
0.1
1
CONCENTRATION[a.u.]
λ(3h)
λ(7h)
POWER(λ(3h))
POWER(λ(7h))

FIG. 33

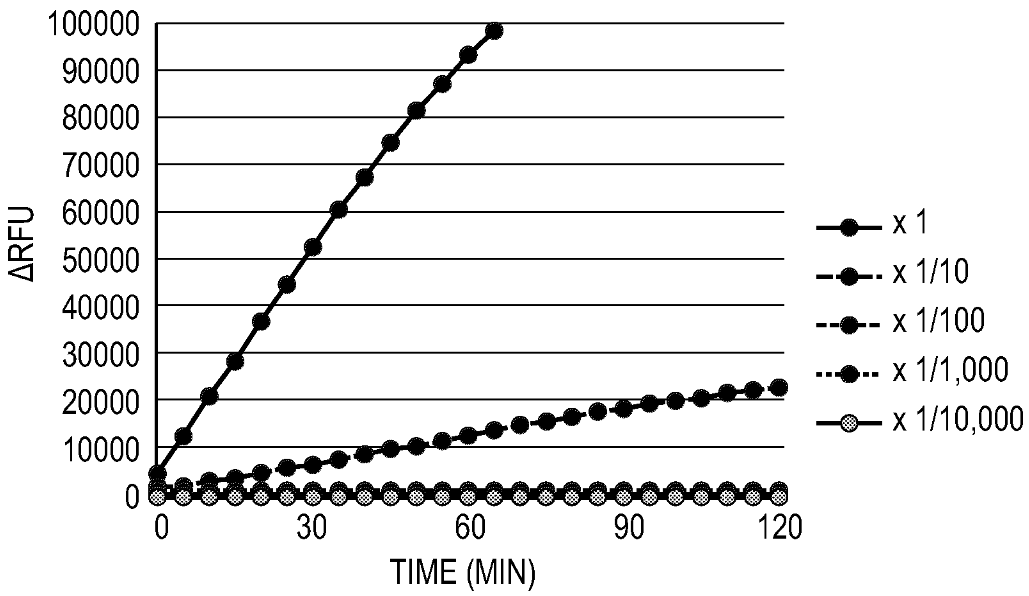

100000
90000
80000
70000
60000
50000
40000
30000
20000
10000
0
ΔRFU
0
30
60
90
120
TIME (MIN)
x 1
x 1/10
x 1/100
x 1/1,000
x 1/10,000

FIG. 34A

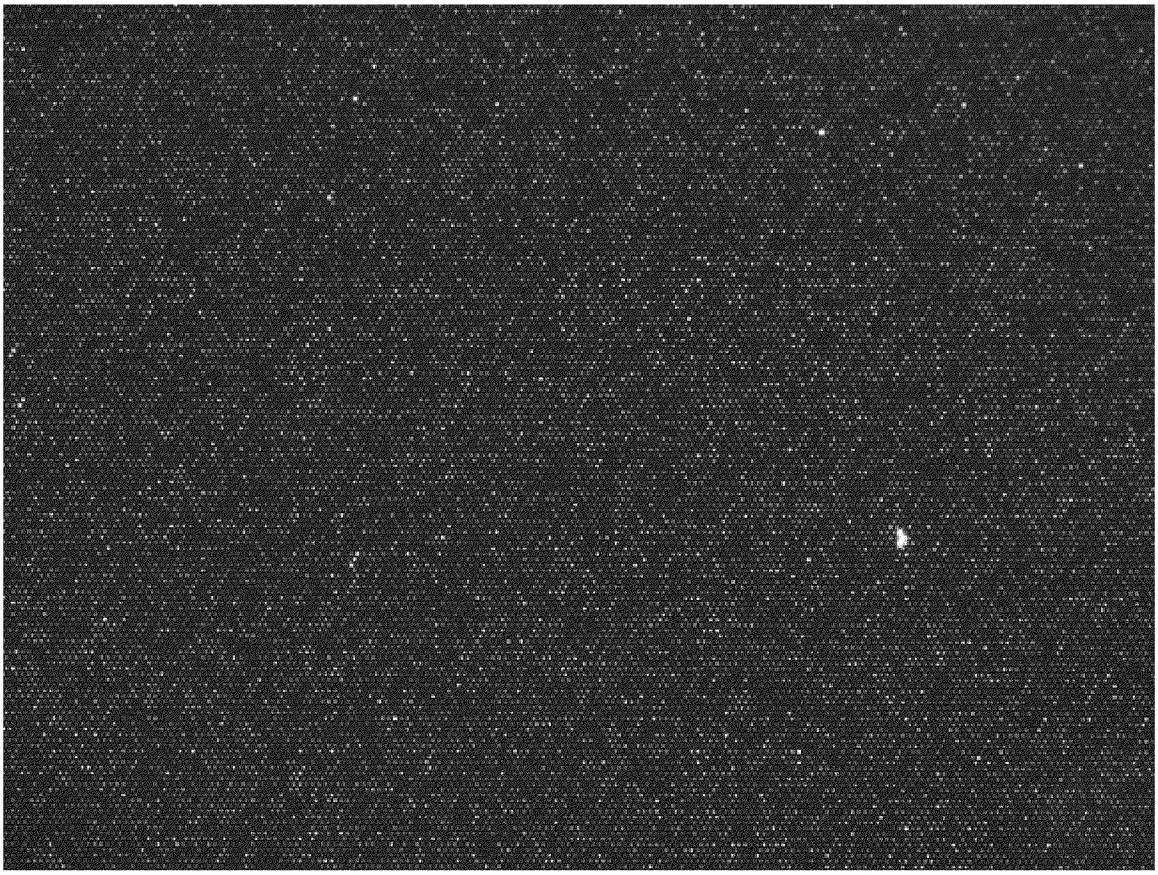

FIG. 34B

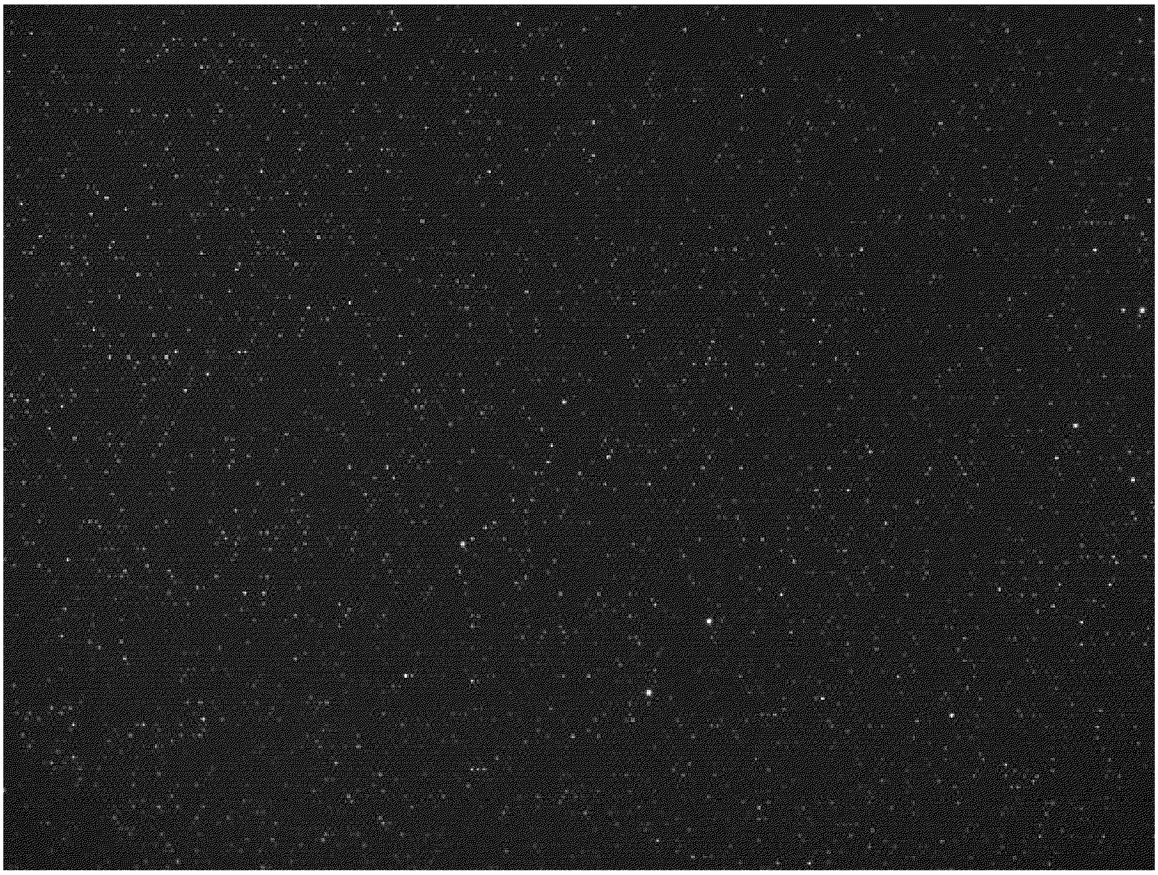

FIG. 34C

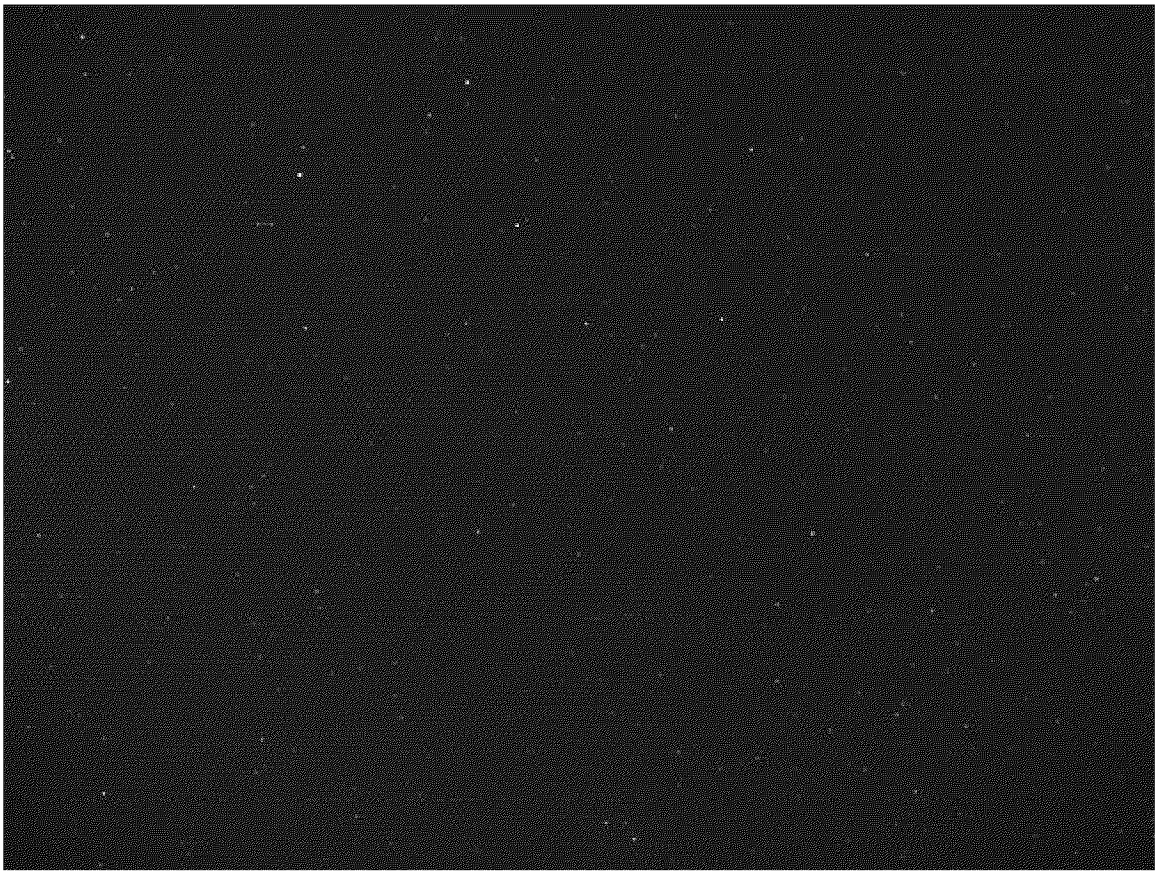

FIG. 34D

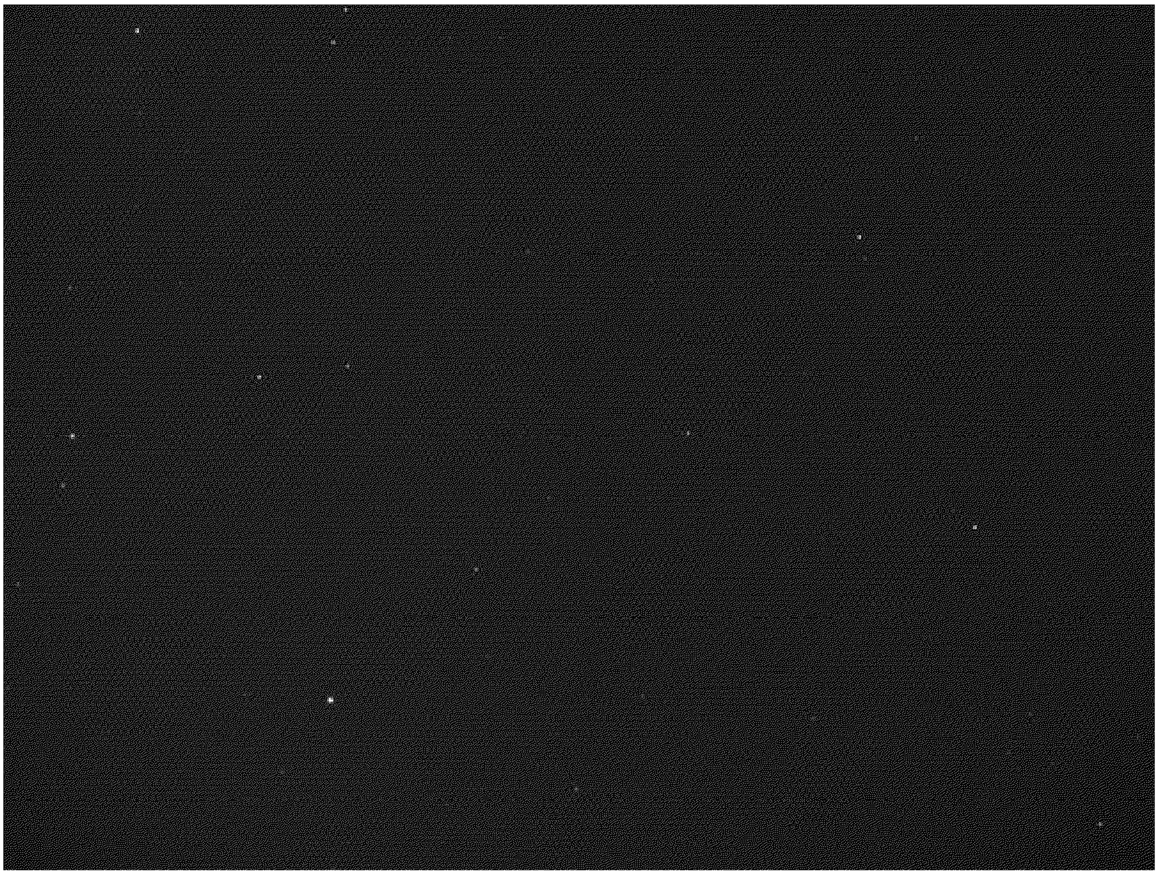

FIG. 34E

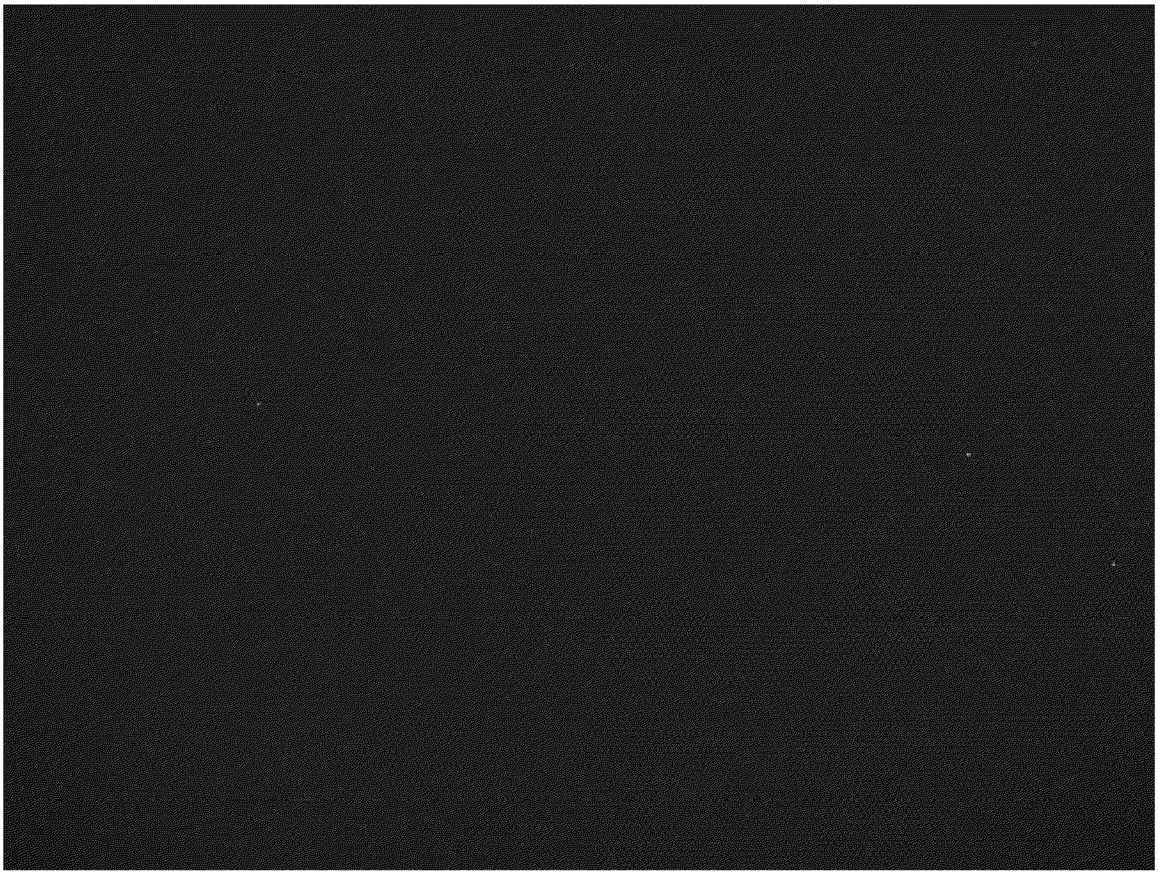

FIG. 34F

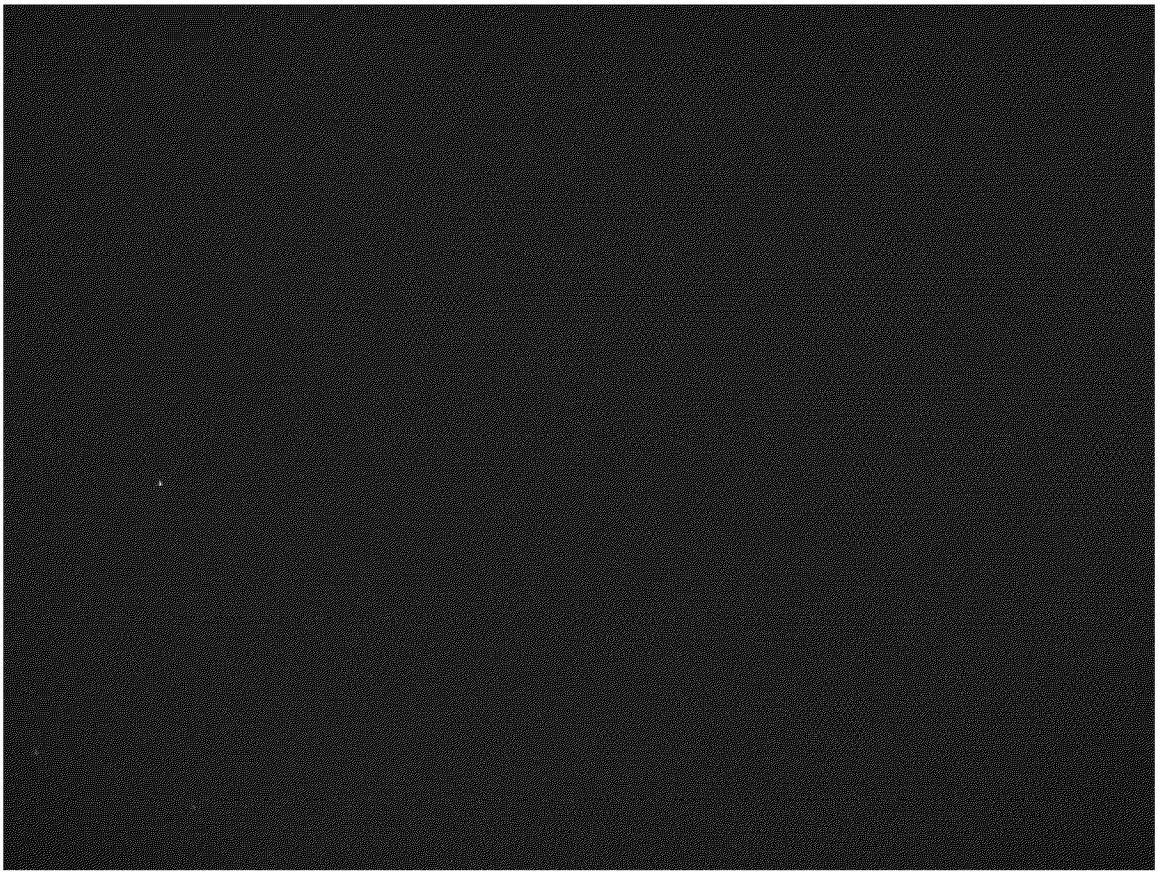

FIG. 34G

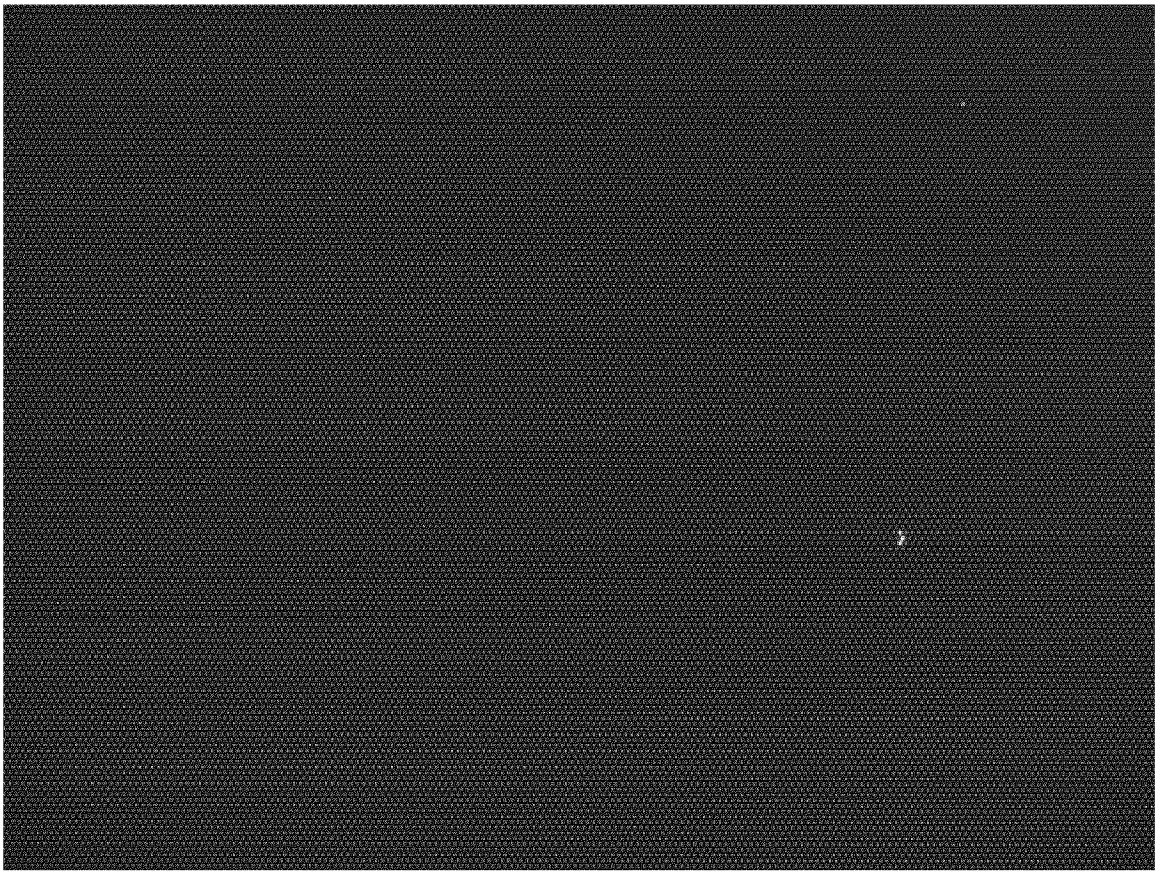

FIG. 34H

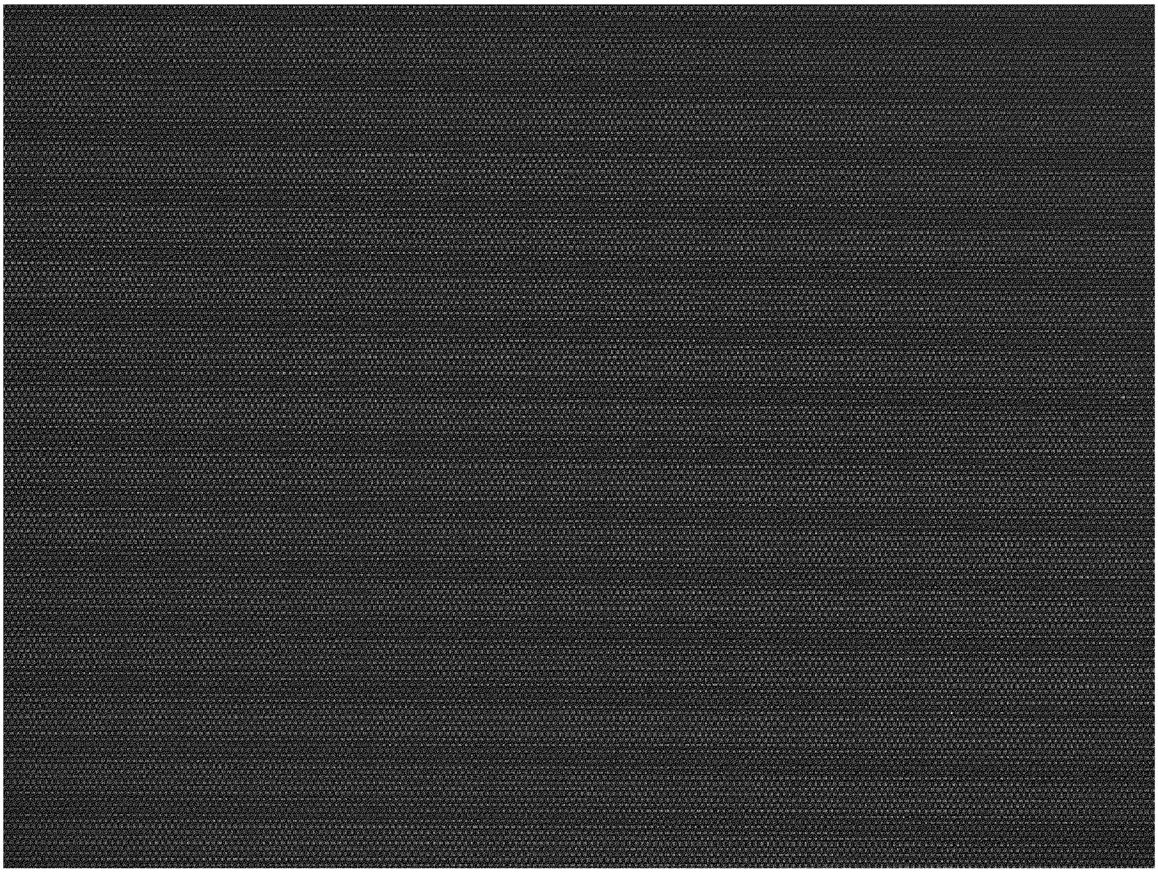

FIG. 34I

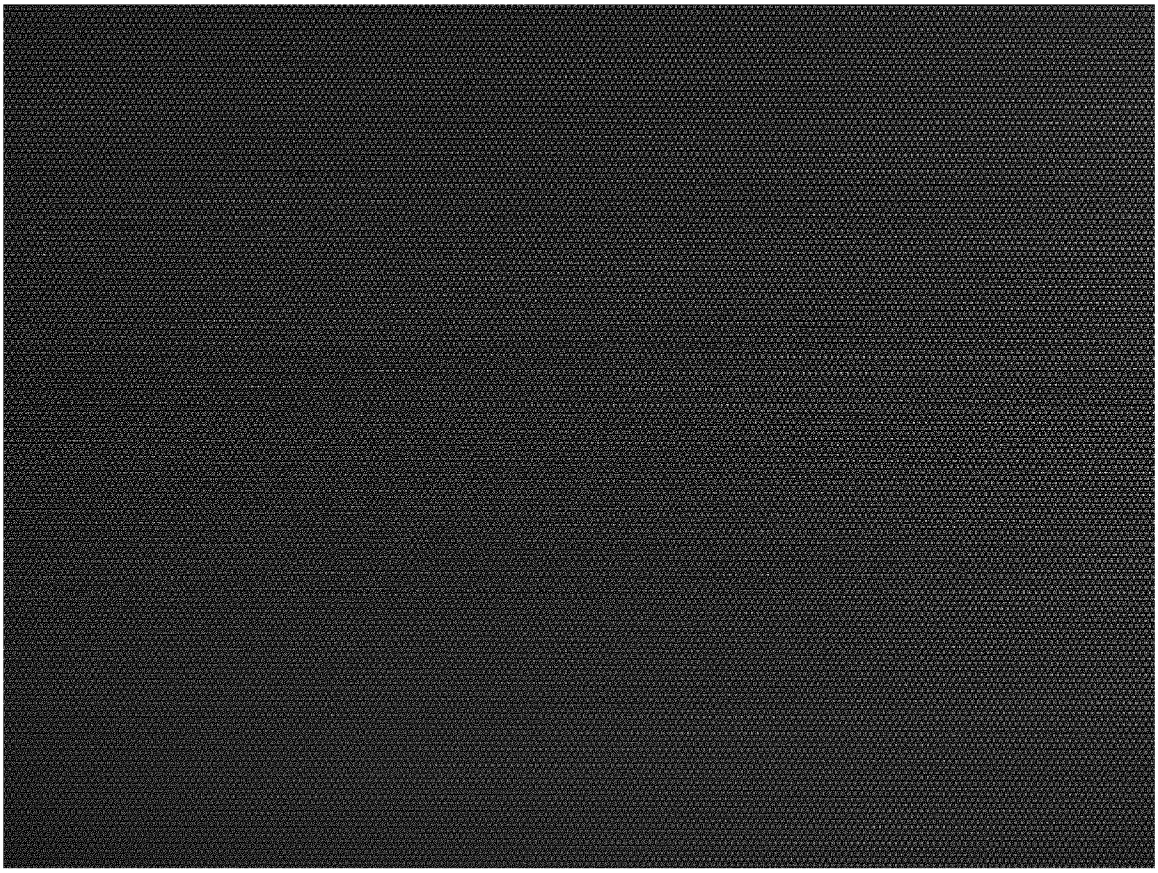

FIG. 34J

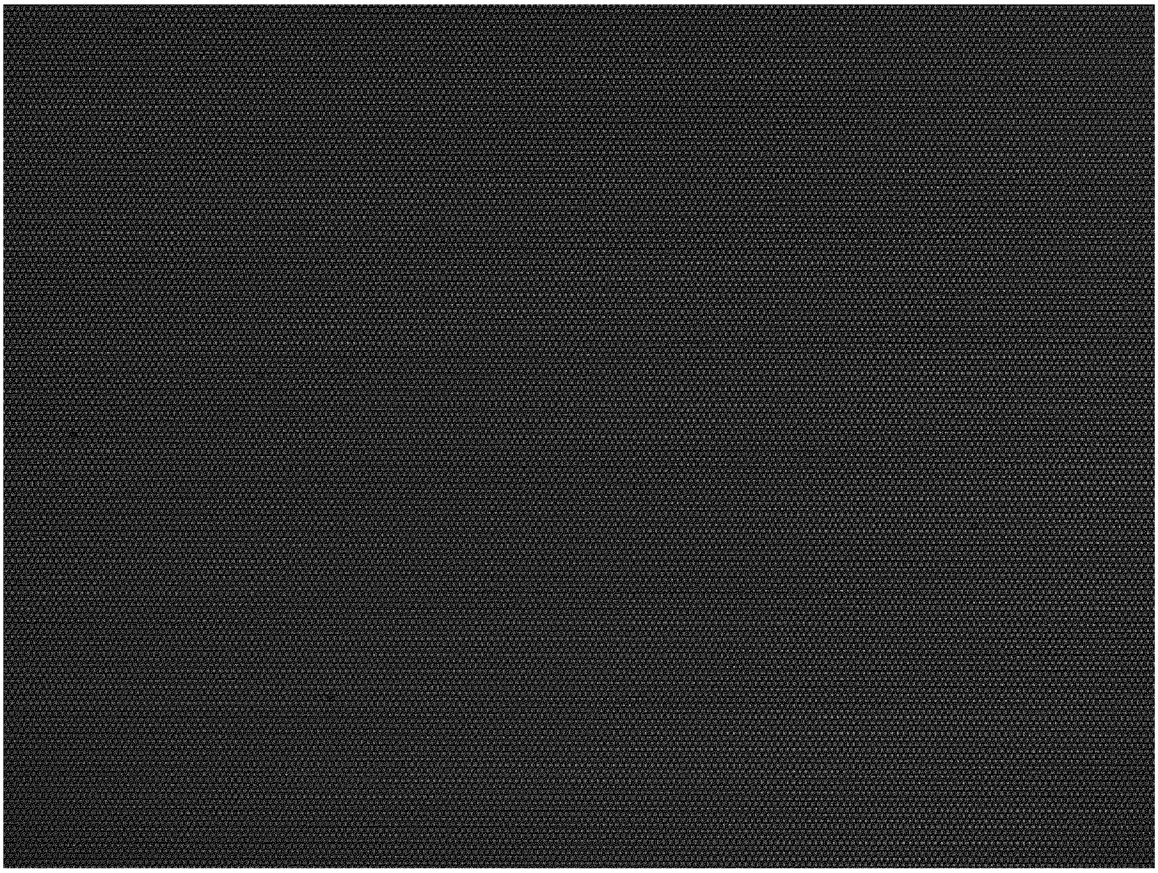

FIG. 34K

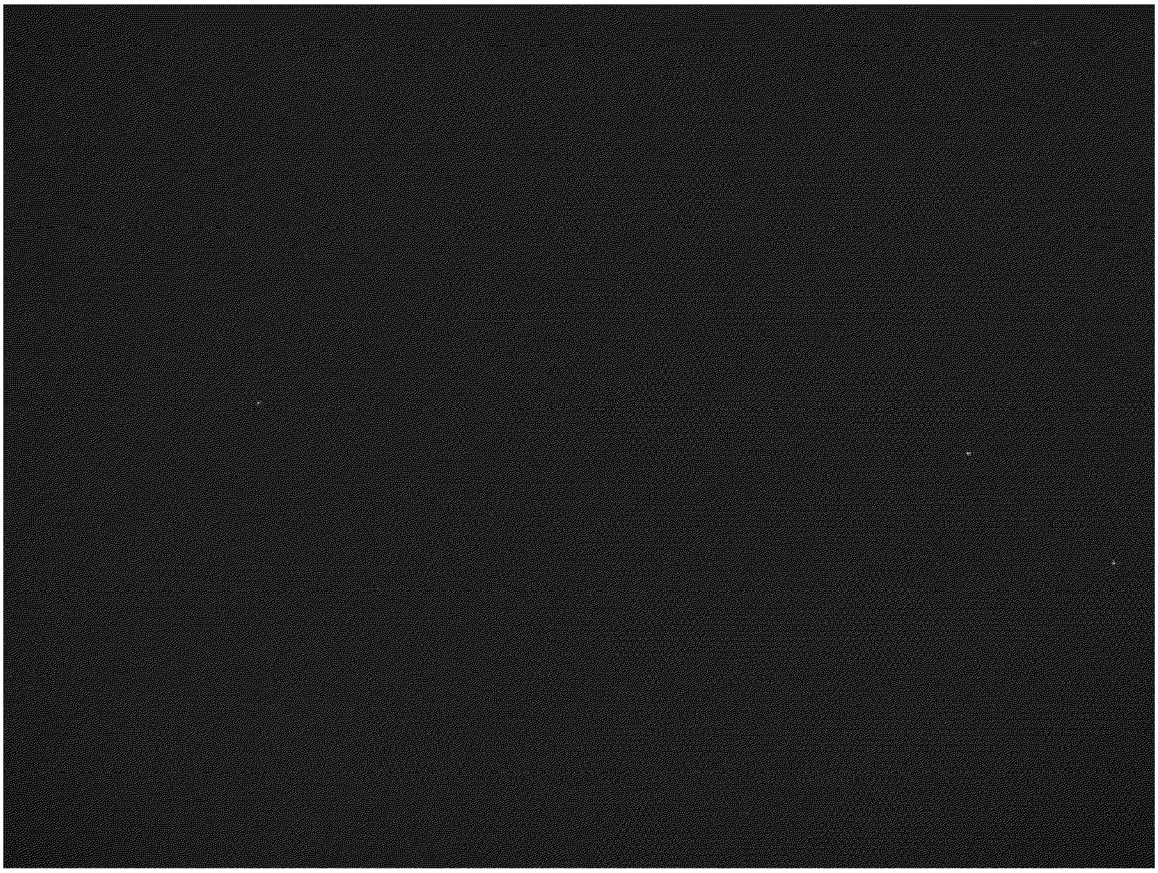

FIG. 34L

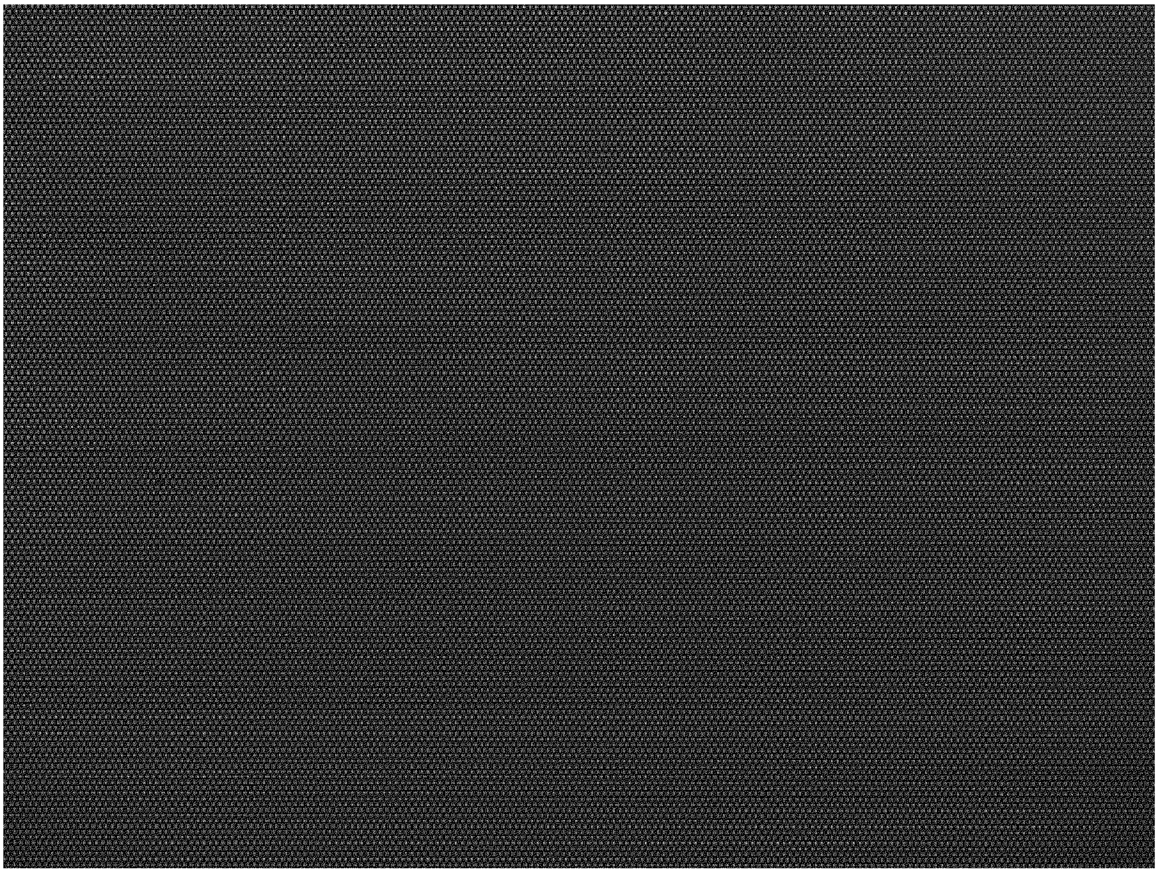

FIG. 35A

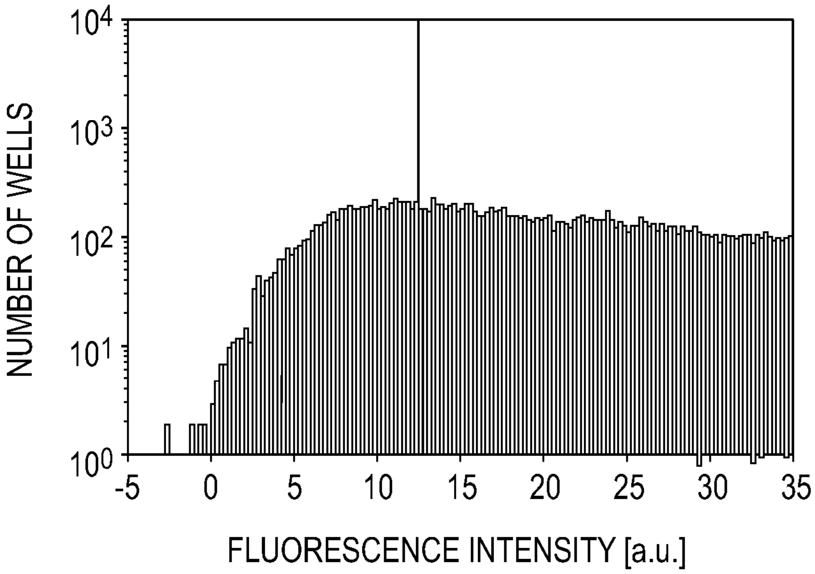
NUMBER OF WELLS
$10^4$
$10^3$
$10^2$
$10^1$
$10^0$
-5
0
5
10
15
20
25
30
35
FLUORESCENCE INTENSITY [a.u.]

FIG. 35B

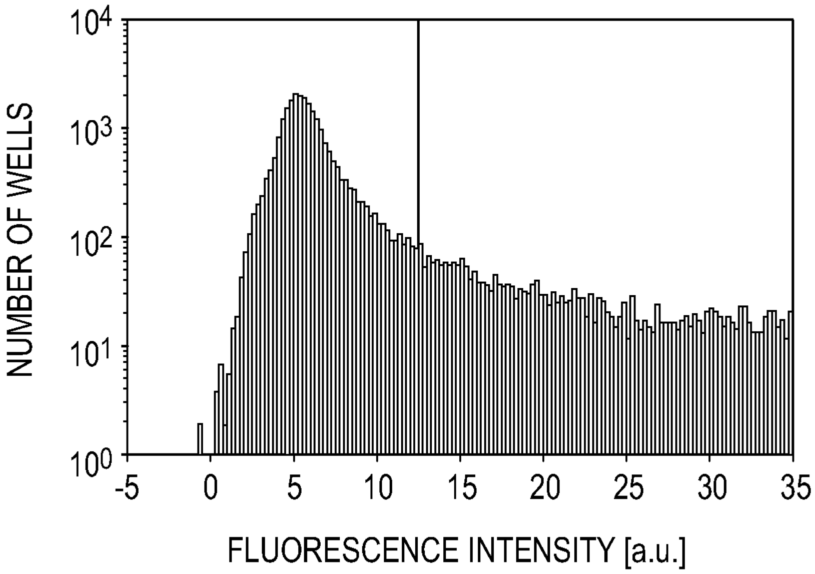
$10^4$
$10^3$
$10^2$
$10^1$
$10^0$
NUMBER OF WELLS
-5
0
5
10
15
20
25
30
35
FLUORESCENCE INTENSITY [a.u.]

FIG. 35C

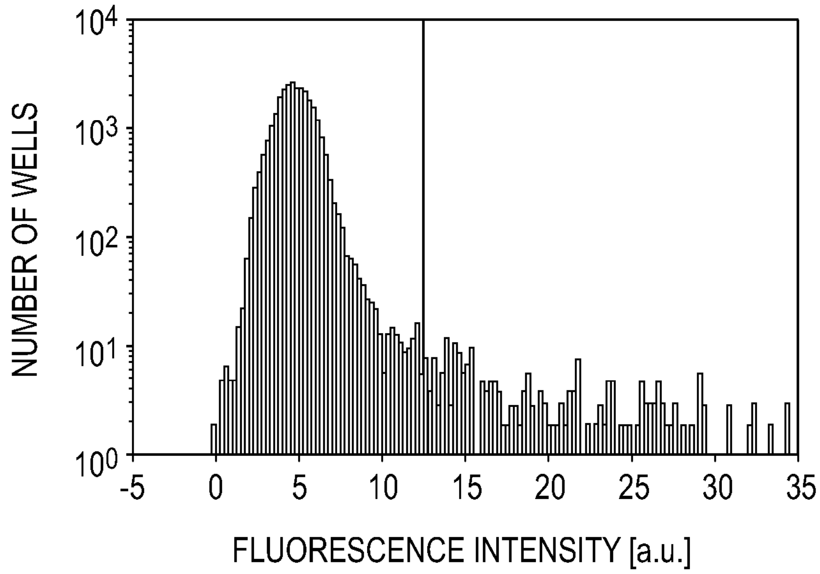
10^4
10^3
10^2
10^1
10^0
NUMBER OF WELLS
-5
0
5
10
15
20
25
30
35
FLUORESCENCE INTENSITY [a.u.]

FIG. 35D

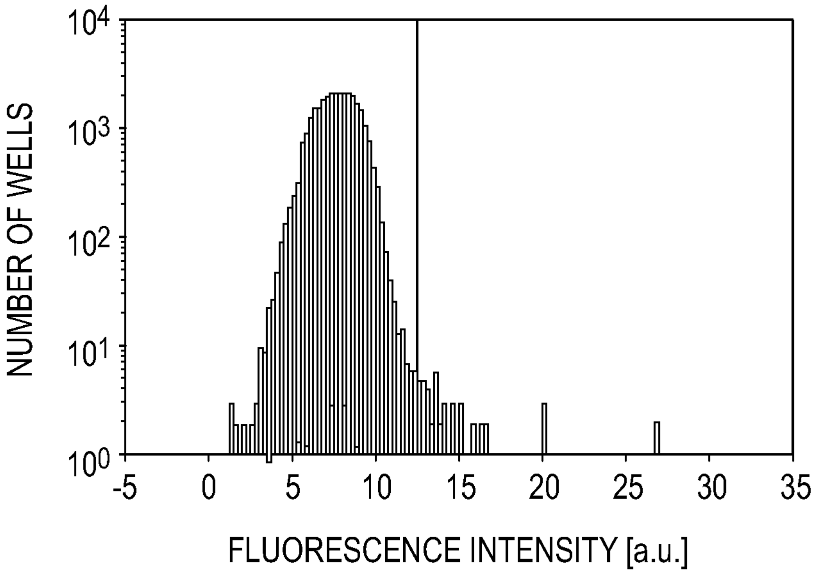
10^4
10^3
10^2
10^1
10^0
NUMBER OF WELLS
-5
0
5
10
15
20
25
30
35
FLUORESCENCE INTENSITY [a.u.]

FIG. 35E

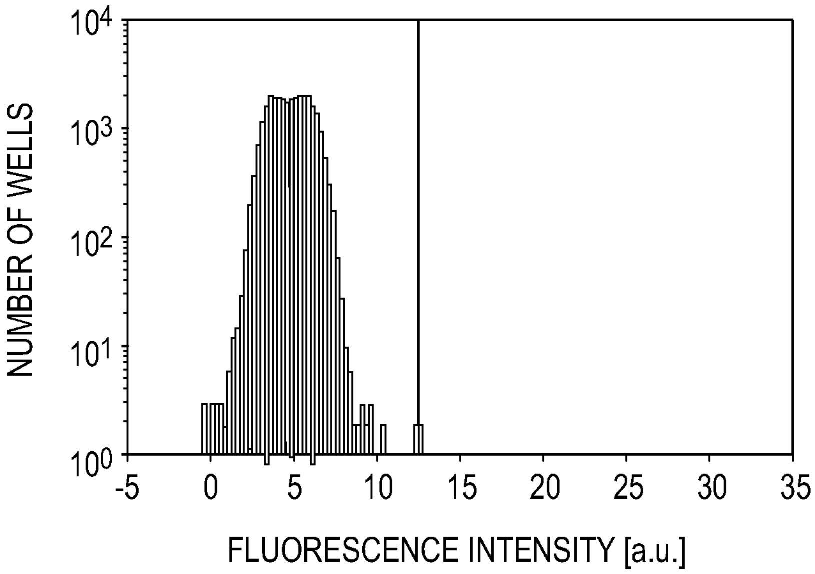
NUMBER OF WELLS
10^4
10^3
10^2
10^1
10^0
-5
0
5
10
15
20
25
30
35
FLUORESCENCE INTENSITY [a.u.]

FIG. 35F

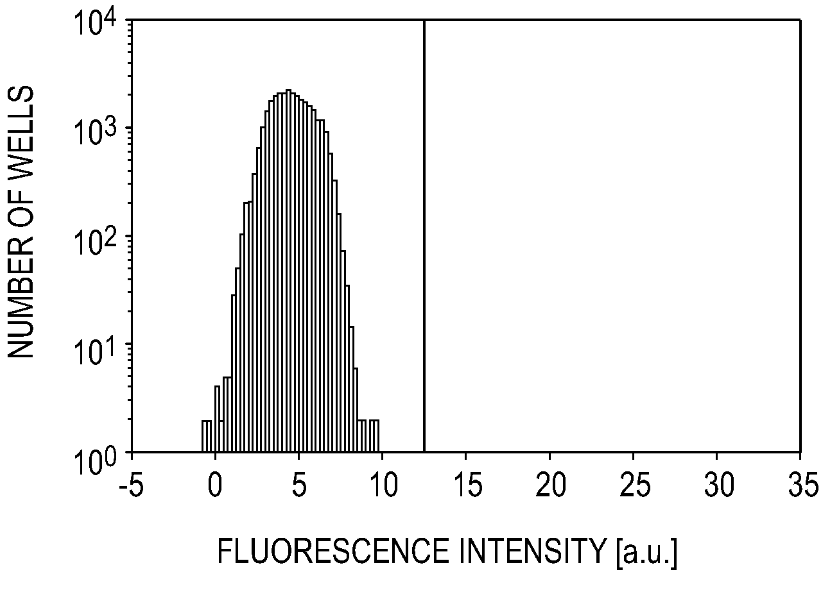

$10^4$
$10^3$
$10^2$
$10^1$
$10^0$
NUMBER OF WELLS
-5
0
5
10
15
20
25
30
35
FLUORESCENCE INTENSITY [a.u.]

FIG. 36

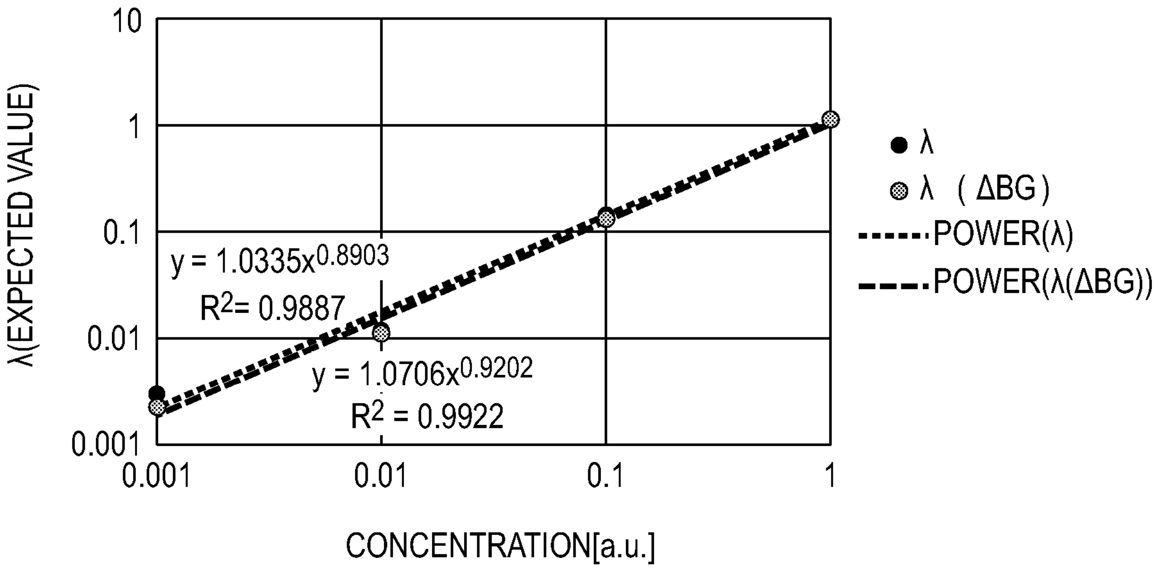
10
1
0.1
0.01
0.001
0.001
0.01
0.1
1
λ(EXPECTED VALUE)
CONCENTRATION[a.u.]
y = 1.0335x^0.8903
R^2= 0.9887
y = 1.0706x^0.9202
R^2 = 0.9922
λ
λ (ΔBG)
POWER(λ)
POWER(λ(ΔBG))

FIG. 37

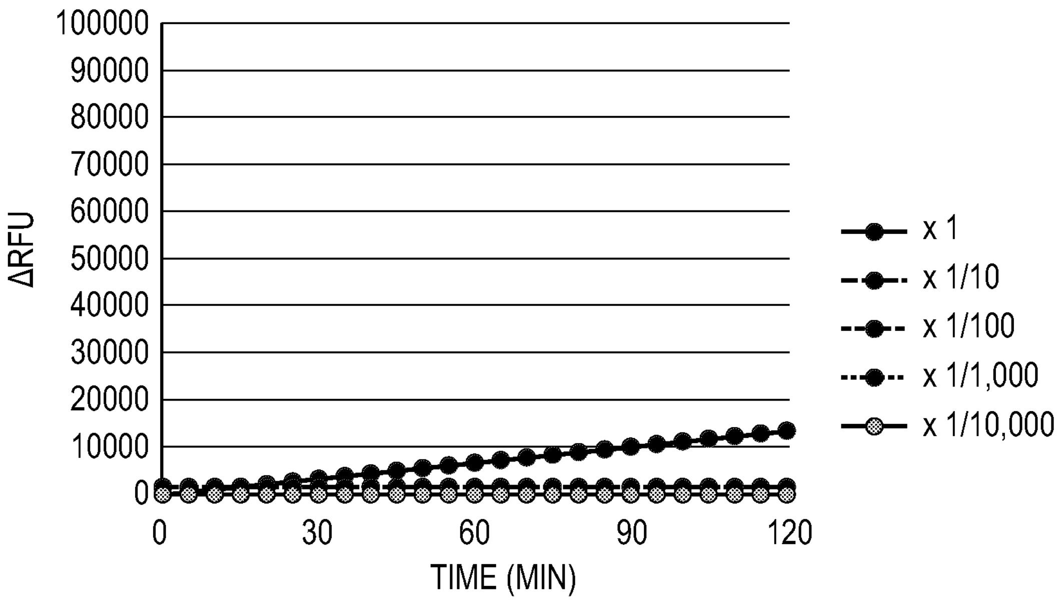
100000
90000
80000
70000
60000
50000
40000
30000
20000
10000
0
ΔRFU
0
30
60
90
120
TIME (MIN)
x 1
x 1/10
x 1/100
x 1/1,000
x 1/10,000

FIG. 38A

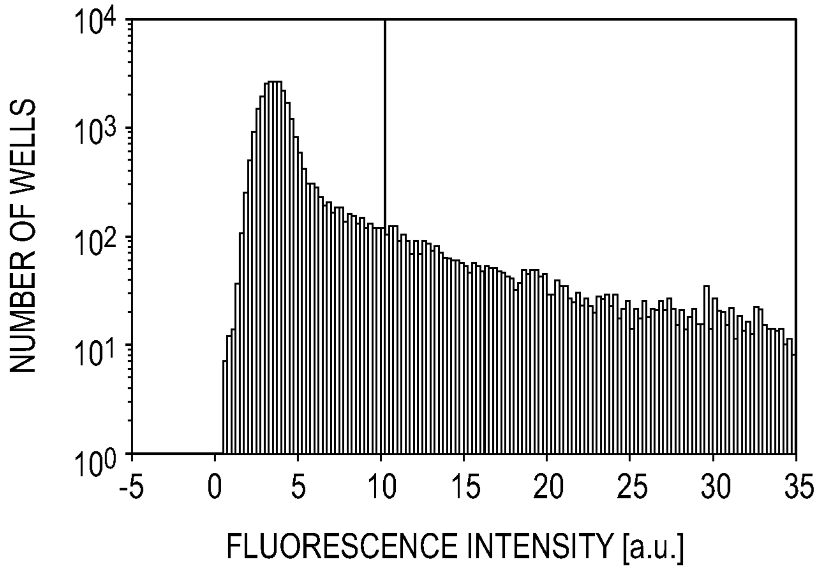
NUMBER OF WELLS
10^4
10^3
10^2
10^1
10^0
-5
0
5
10
15
20
25
30
35
FLUORESCENCE INTENSITY [a.u.]

FIG. 38B

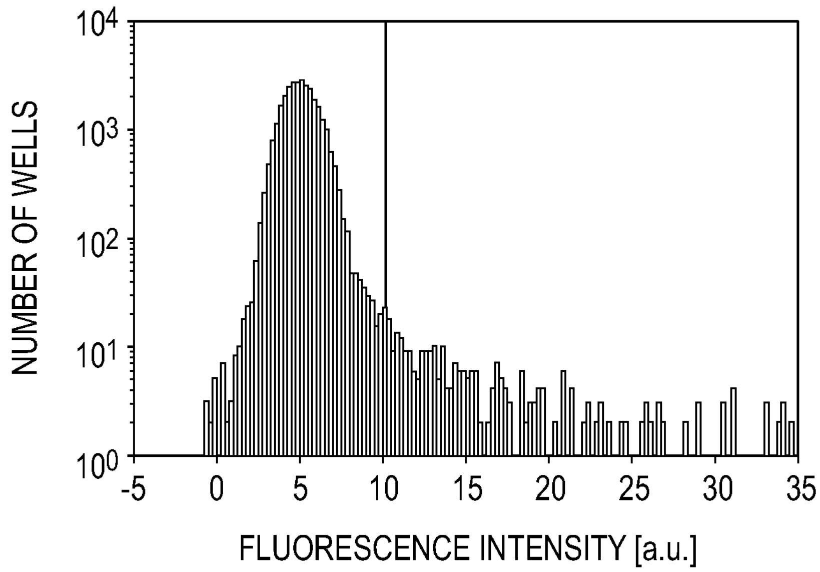
NUMBER OF WELLS
$10^4$
$10^3$
$10^2$
$10^1$
$10^0$
-5
0
5
10
15
20
25
30
35
FLUORESCENCE INTENSITY [a.u.]

FIG. 38C

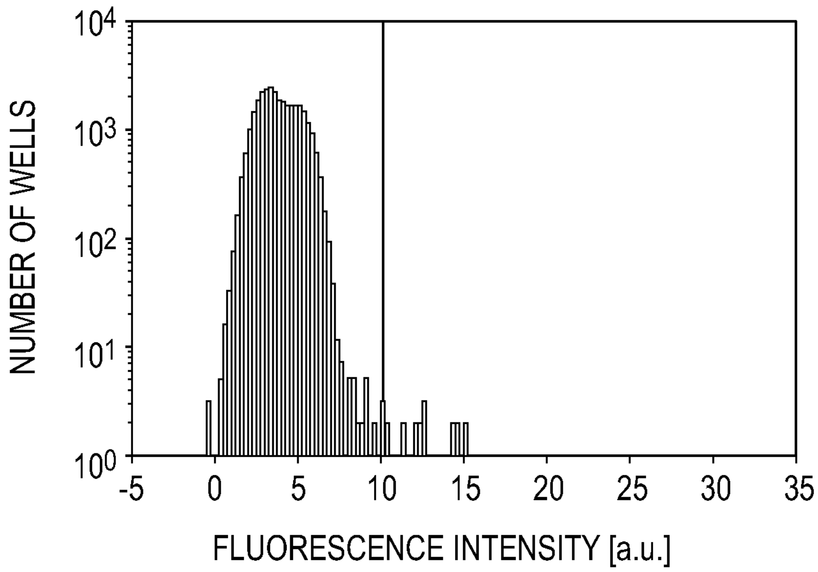
NUMBER OF WELLS
$10^4$
$10^3$
$10^2$
$10^1$
$10^0$
-5
0
5
10
15
20
25
30
35
FLUORESCENCE INTENSITY [a.u.]

FIG. 38D

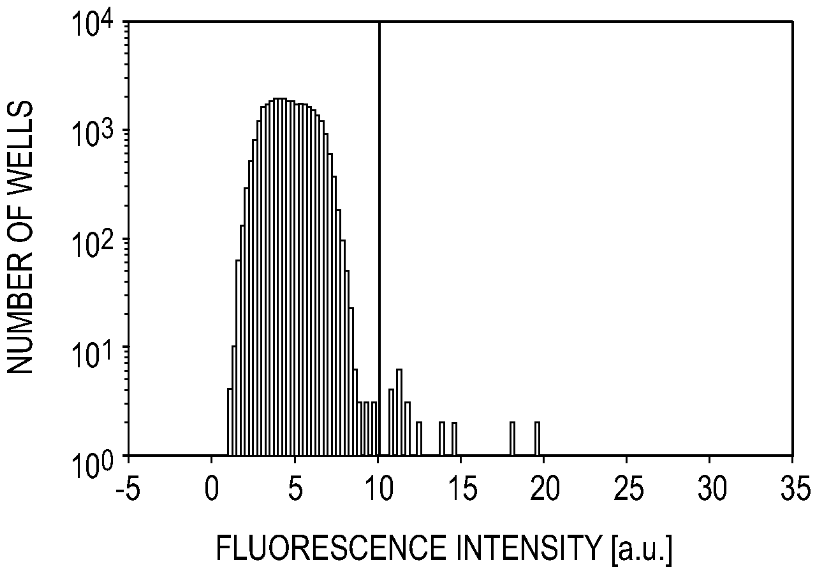
$10^4$
$10^3$
$10^2$
$10^1$
$10^0$
NUMBER OF WELLS
-5
0
5
10
15
20
25
30
35
FLUORESCENCE INTENSITY [a.u.]

FIG. 38E

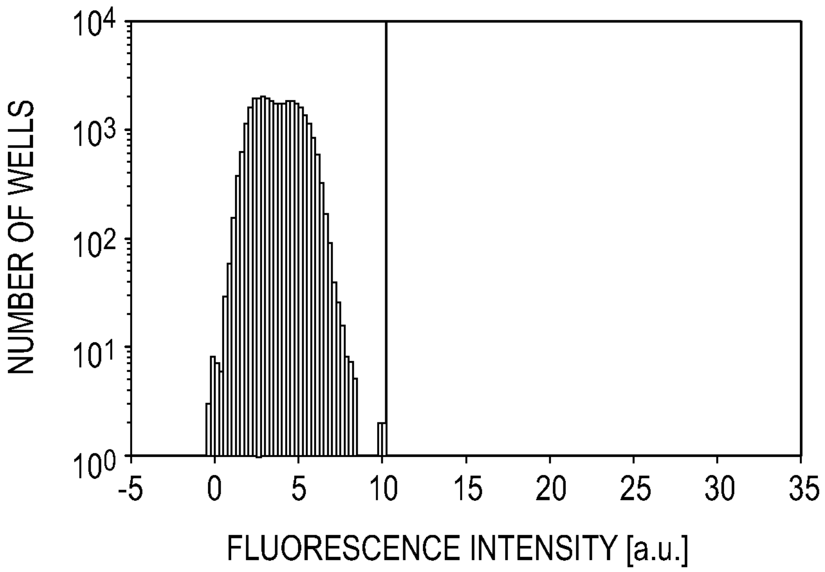
NUMBER OF WELLS
$10^4$
$10^3$
$10^2$
$10^1$
$10^0$
-5
0
5
10
15
20
25
30
35
FLUORESCENCE INTENSITY [a.u.]

FIG. 38F

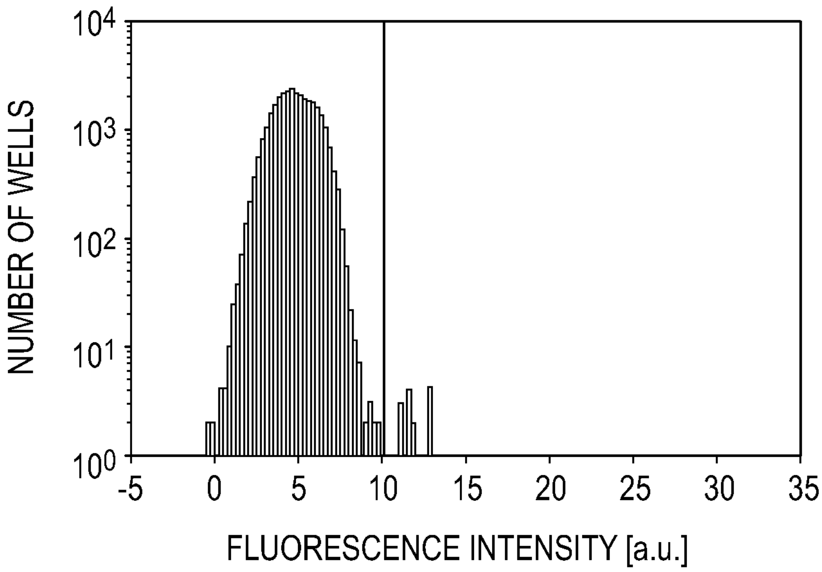
$10^4$
$10^3$
$10^2$
$10^1$
$10^0$
NUMBER OF WELLS
-5
0
5
10
15
20
25
30
35
FLUORESCENCE INTENSITY [a.u.]

FIG. 39

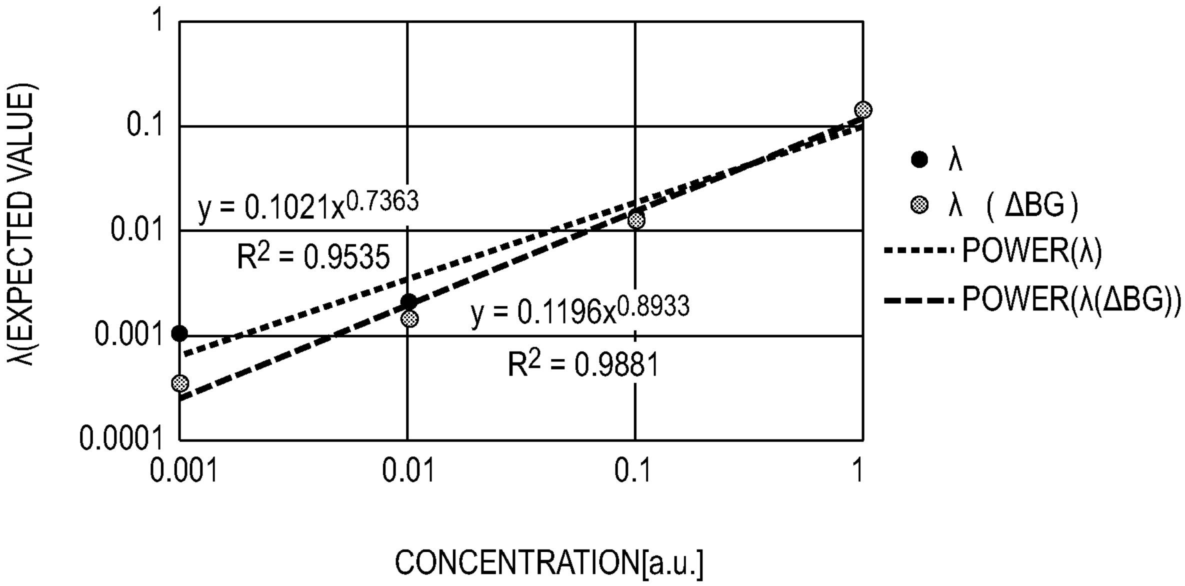
1
0.1
0.01
0.001
0.0001
λ(EXPECTED VALUE)
0.001
0.01
0.1
1
CONCENTRATION[a.u.]
y = 0.1021x^0.7363
R^2 = 0.9535
y = 0.1196x^0.8933
R^2 = 0.9881
λ
λ (ΔBG)
POWER(λ)
POWER(λ(ΔBG))

FIG. 40A

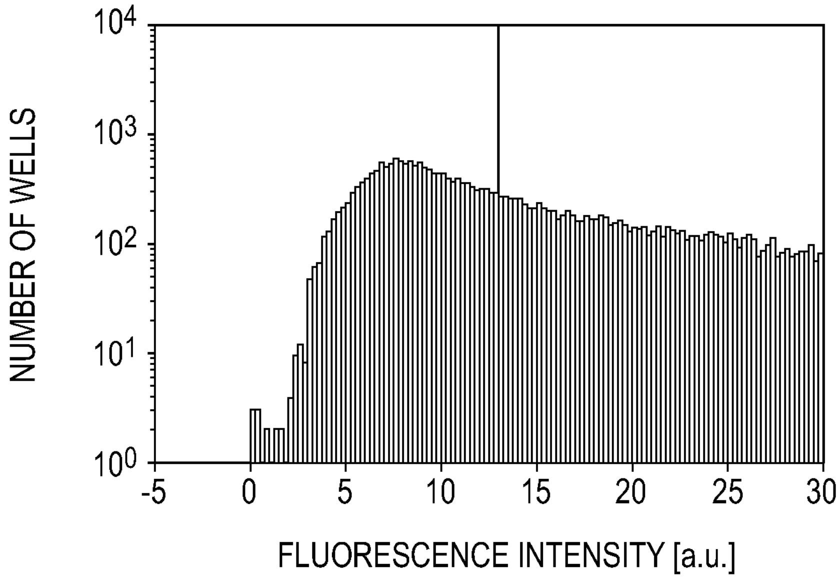
$10^4$
$10^3$
$10^2$
$10^1$
$10^0$
NUMBER OF WELLS
-5
0
5
10
15
20
25
30
FLUORESCENCE INTENSITY [a.u.]

FIG. 40B

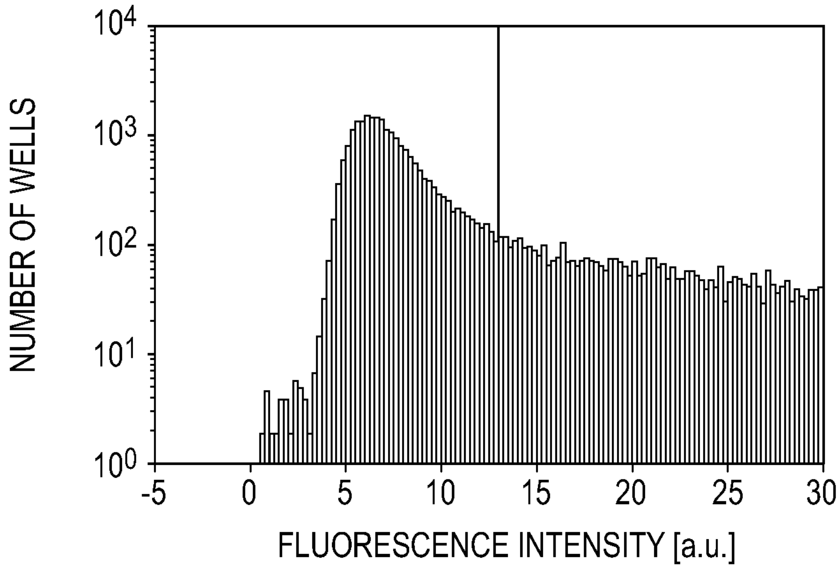
NUMBER OF WELLS
$10^4$
$10^3$
$10^2$
$10^1$
$10^0$
-5
0
5
10
15
20
25
30
FLUORESCENCE INTENSITY [a.u.]

FIG. 40C

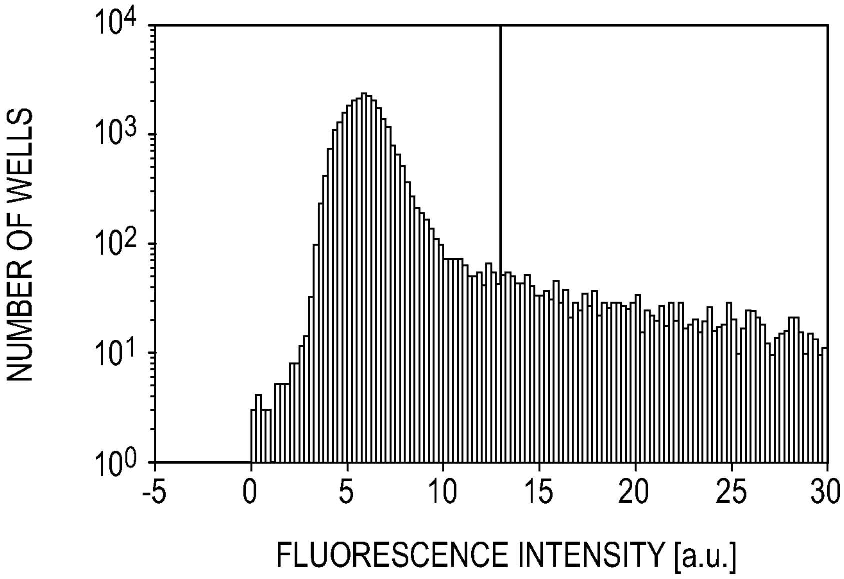
NUMBER OF WELLS
$10^4$
$10^3$
$10^2$
$10^1$
$10^0$
-5
0
5
10
15
20
25
30
FLUORESCENCE INTENSITY [a.u.]

FIG. 40D

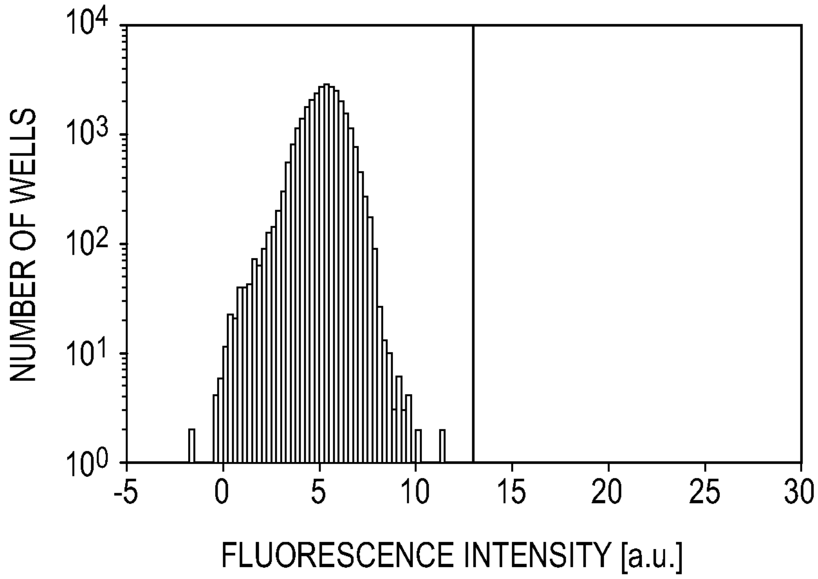
$10^4$
$10^3$
$10^2$
$10^1$
$10^0$
NUMBER OF WELLS
-5
0
5
10
15
20
25
30
FLUORESCENCE INTENSITY [a.u.]

FIG. 40E

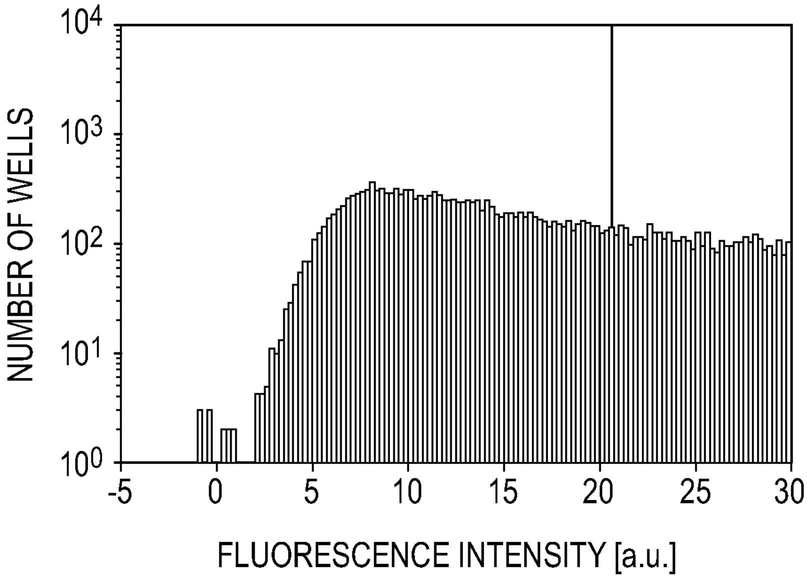
NUMBER OF WELLS
$10^4$
$10^3$
$10^2$
$10^1$
$10^0$
-5
0
5
10
15
20
25
30
FLUORESCENCE INTENSITY [a.u.]

FIG. 40F

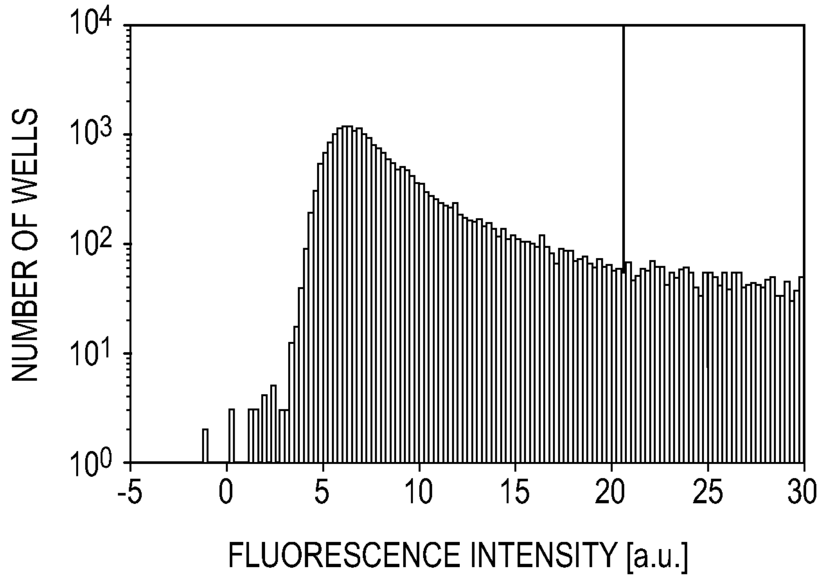
NUMBER OF WELLS
$10^4$
$10^3$
$10^2$
$10^1$
$10^0$
-5
0
5
10
15
20
25
30
FLUORESCENCE INTENSITY [a.u.]

FIG. 40G

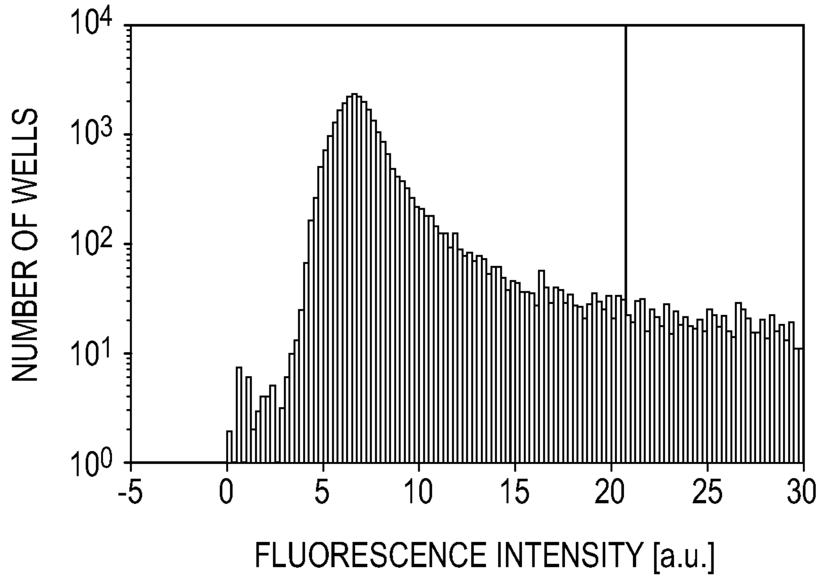
NUMBER OF WELLS
$10^4$
$10^3$
$10^2$
$10^1$
$10^0$
-5
0
5
10
15
20
25
30
FLUORESCENCE INTENSITY [a.u.]

FIG. 40H

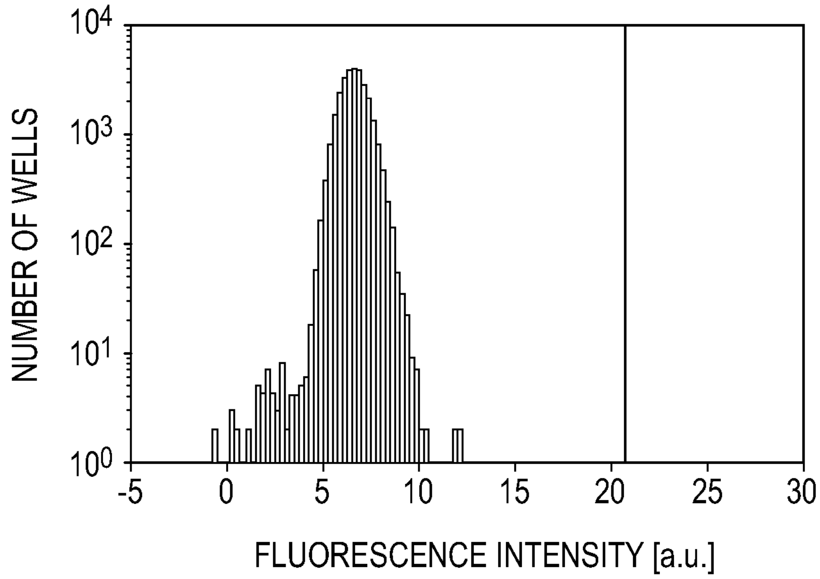

$10^4$
$10^3$
$10^2$
$10^1$
$10^0$
NUMBER OF WELLS
-5
0
5
10
15
20
25
30
FLUORESCENCE INTENSITY [a.u.]

FIG. 41A

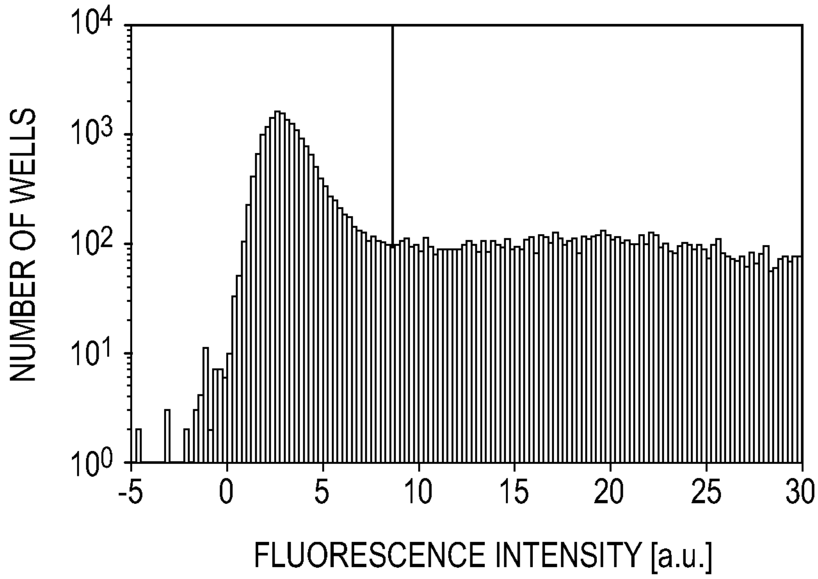
NUMBER OF WELLS
$10^4$
$10^3$
$10^2$
$10^1$
$10^0$
-5
0
5
10
15
20
25
30
FLUORESCENCE INTENSITY [a.u.]

FIG. 41B

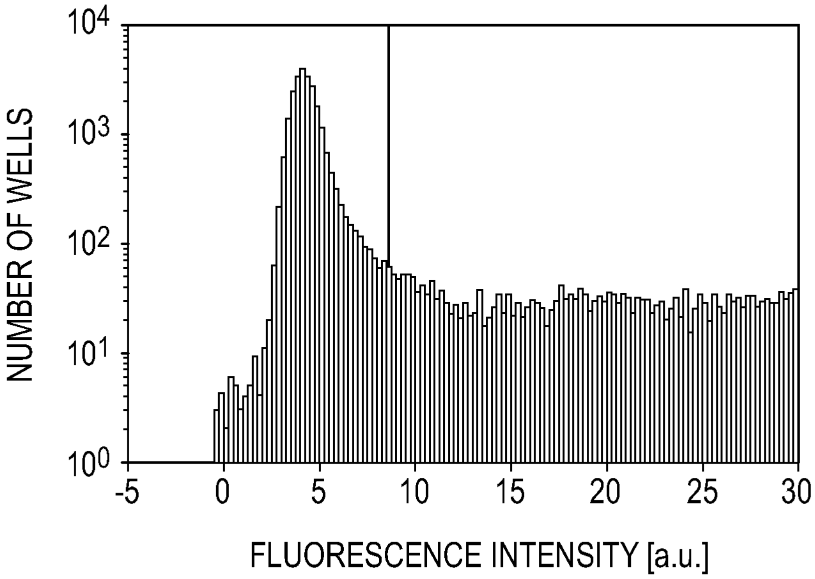
$10^4$
$10^3$
$10^2$
$10^1$
$10^0$
NUMBER OF WELLS
-5
0
5
10
15
20
25
30
FLUORESCENCE INTENSITY [a.u.]

FIG. 41C

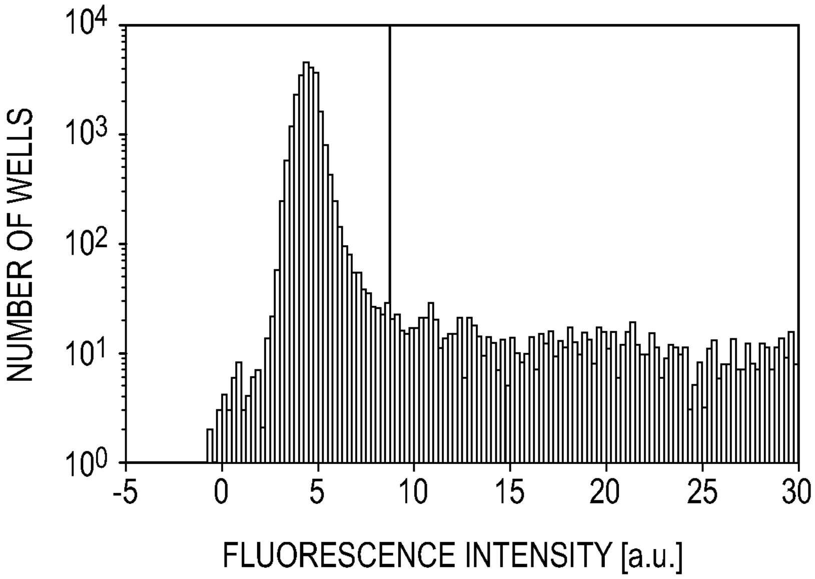
10^4
10^3
10^2
10^1
10^0
NUMBER OF WELLS
-5
0
5
10
15
20
25
30
FLUORESCENCE INTENSITY [a.u.]

FIG. 41D

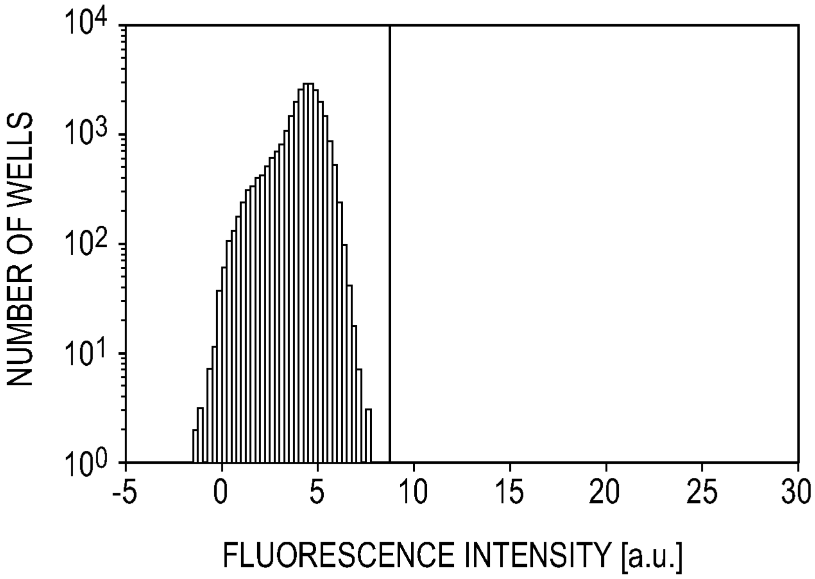
NUMBER OF WELLS
10^4
10^3
10^2
10^1
10^0
-5
0
5
10
15
20
25
30
FLUORESCENCE INTENSITY [a.u.]

FIG. 41E

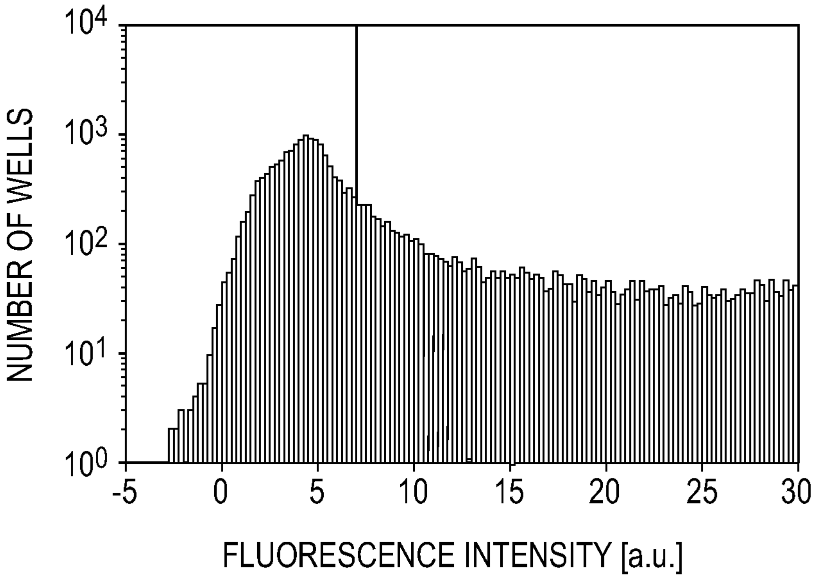
NUMBER OF WELLS
$10^4$
$10^3$
$10^2$
$10^1$
$10^0$
-5
0
5
10
15
20
25
30
FLUORESCENCE INTENSITY [a.u.]

FIG. 41F

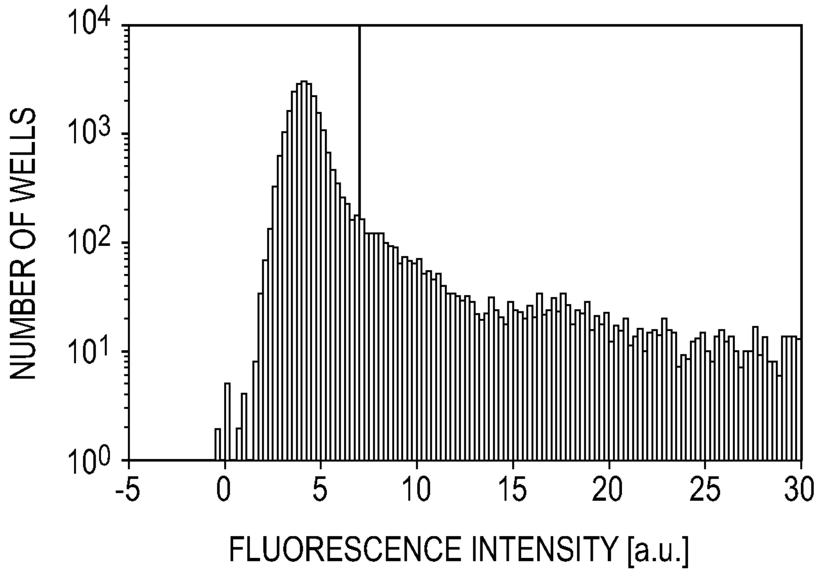
$10^4$
$10^3$
$10^2$
$10^1$
$10^0$
NUMBER OF WELLS
-5
0
5
10
15
20
25
30
FLUORESCENCE INTENSITY [a.u.]

FIG. 41G

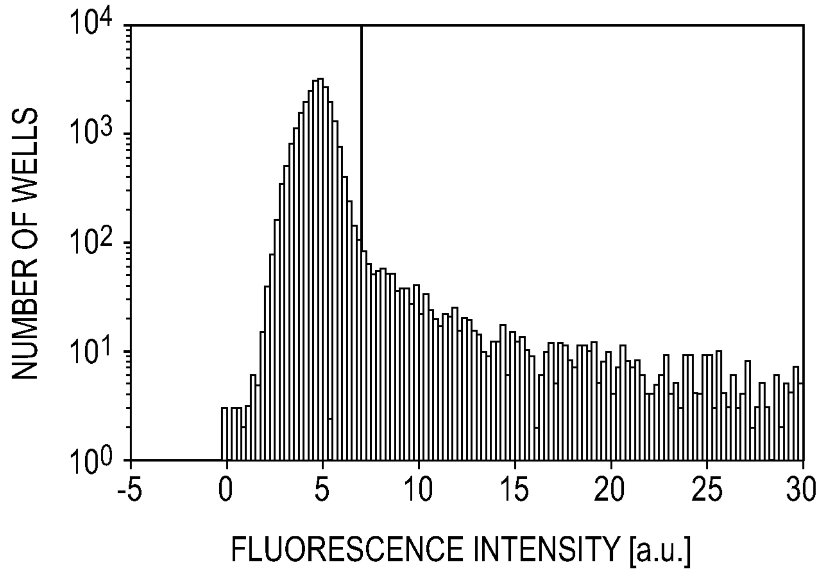

$10^4$
$10^3$
$10^2$
$10^1$
$10^0$
NUMBER OF WELLS
-5
0
5
10
15
20
25
30
FLUORESCENCE INTENSITY [a.u.]

FIG. 41H

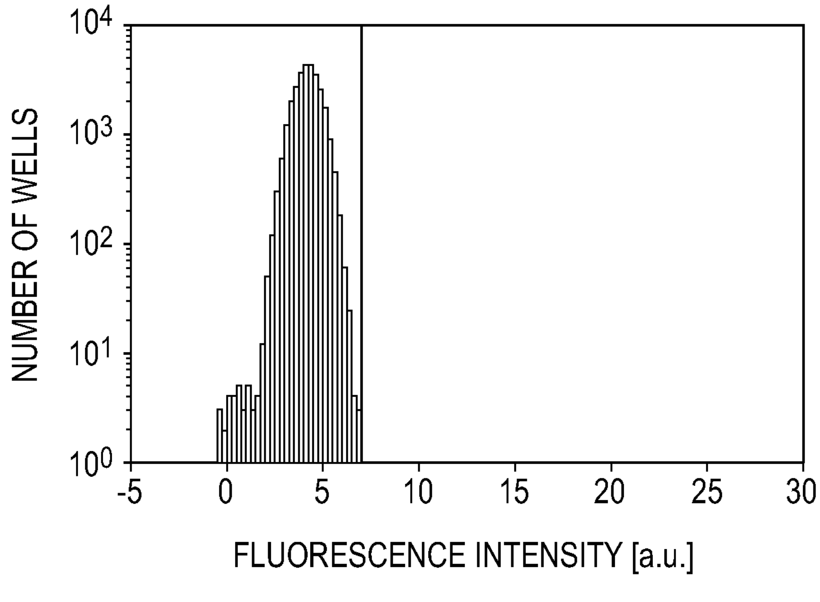
$10^4$
$10^3$
$10^2$
$10^1$
$10^0$
NUMBER OF WELLS
-5
0
5
10
15
20
25
30
FLUORESCENCE INTENSITY [a.u.]

FIG. 42A

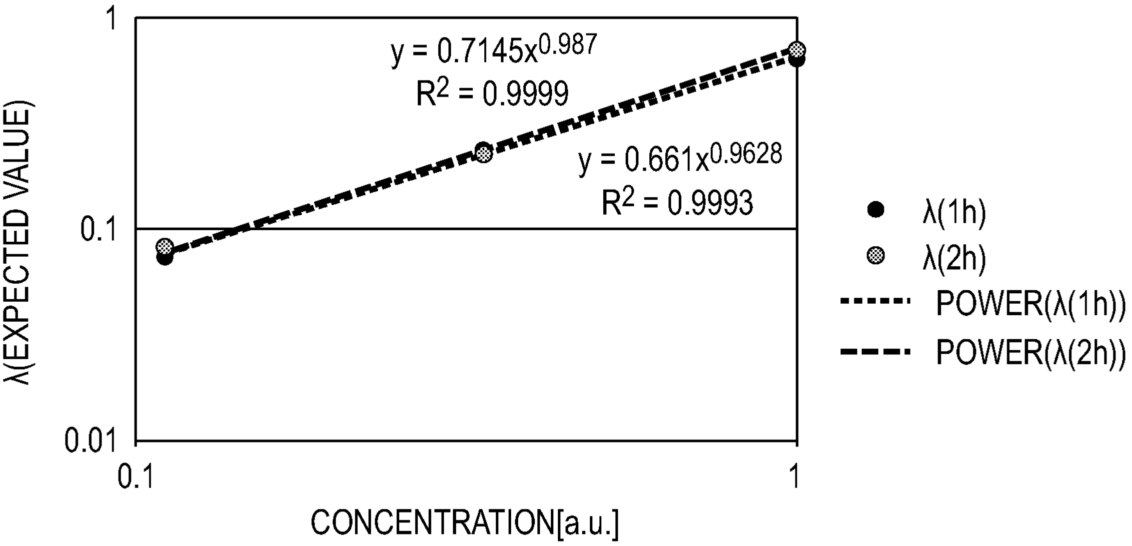
1
0.1
0.01
0.1
1
y = 0.7145x^0.987
R2 = 0.9999
y = 0.661x^0.9628
R2 = 0.9993
λ(EXPECTED VALUE)
CONCENTRATION[a.u.]
λ(1h)
λ(2h)
POWER(λ(1h))
POWER(λ(2h))

FIG. 42B

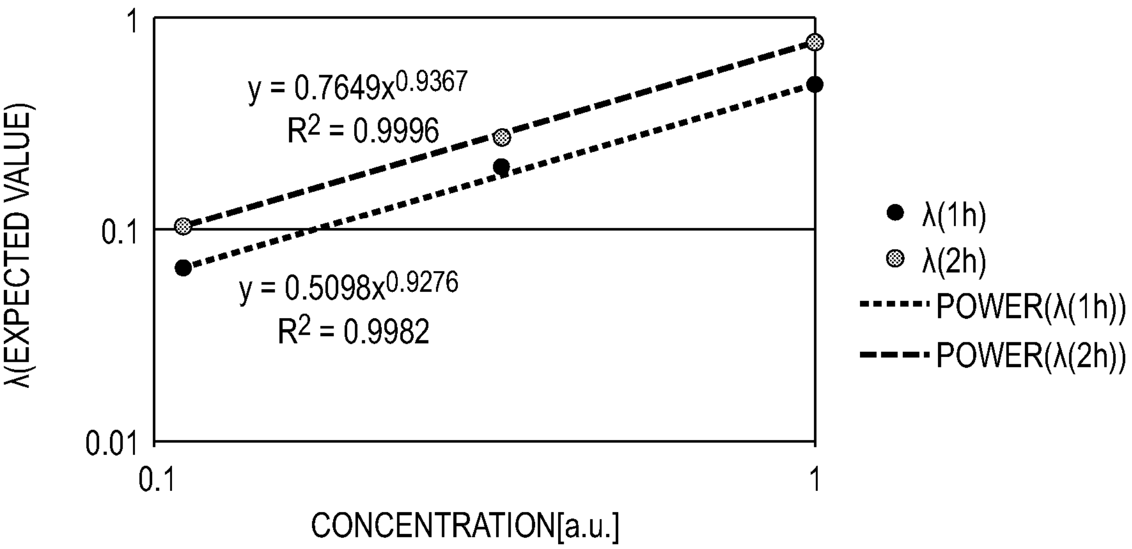
1
0.1
0.01
0.1
1
λ(EXPECTED VALUE)
CONCENTRATION[a.u.]
y = 0.7649x^0.9367
R² = 0.9996
y = 0.5098x^0.9276
R² = 0.9982
λ(1h)
λ(2h)
POWER(λ(1h))
POWER(λ(2h))

FIG. 43

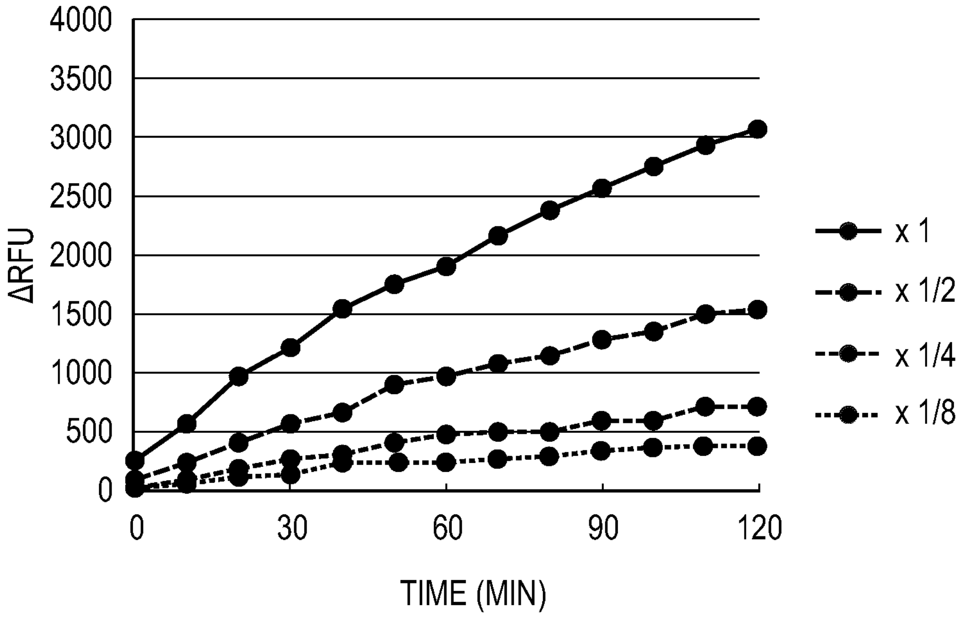
4000
3500
3000
2500
2000
1500
1000
500
0
ΔRFU
0
30
60
90
120
TIME (MIN)
x 1
x 1/2
x 1/4
x 1/8

FIG. 44A

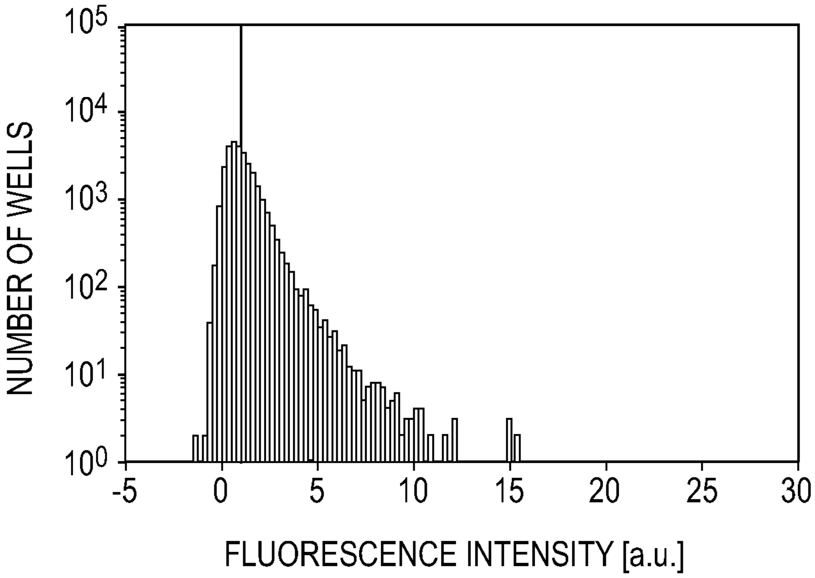
NUMBER OF WELLS
$10^5$
$10^4$
$10^3$
$10^2$
$10^1$
$10^0$
-5
0
5
10
15
20
25
30
FLUORESCENCE INTENSITY [a.u.]

FIG. 44B

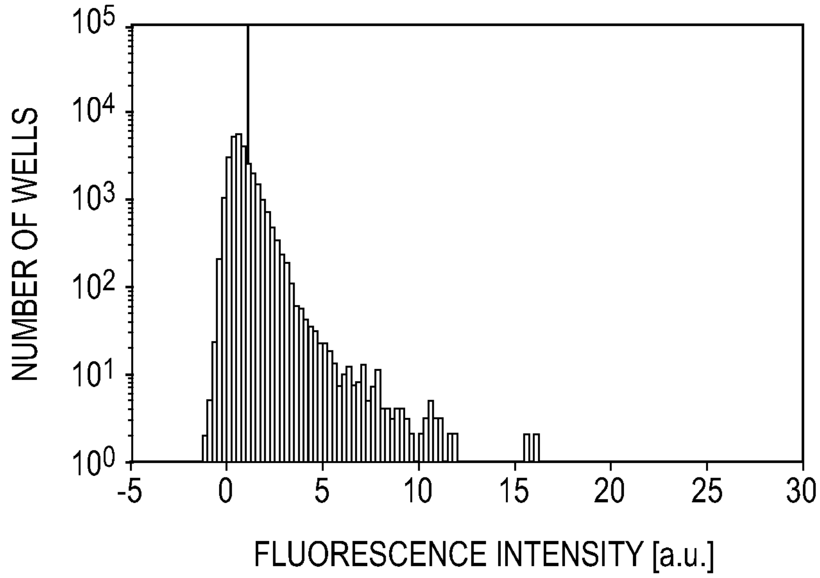
NUMBER OF WELLS
$10^5$
$10^4$
$10^3$
$10^2$
$10^1$
$10^0$
-5
0
5
10
15
20
25
30
FLUORESCENCE INTENSITY [a.u.]

FIG. 44C

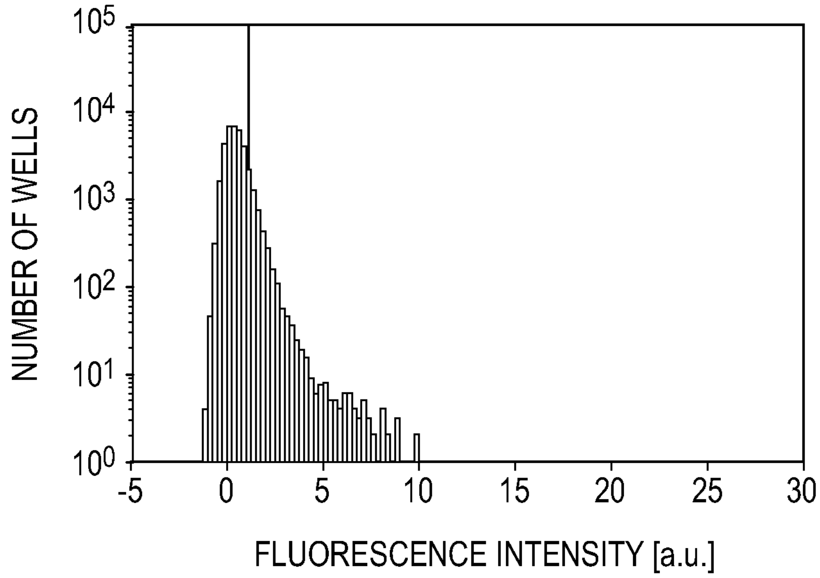

$10^5$
$10^4$
$10^3$
$10^2$
$10^1$
$10^0$
NUMBER OF WELLS
-5
0
5
10
15
20
25
30
FLUORESCENCE INTENSITY [a.u.]

FIG. 44D

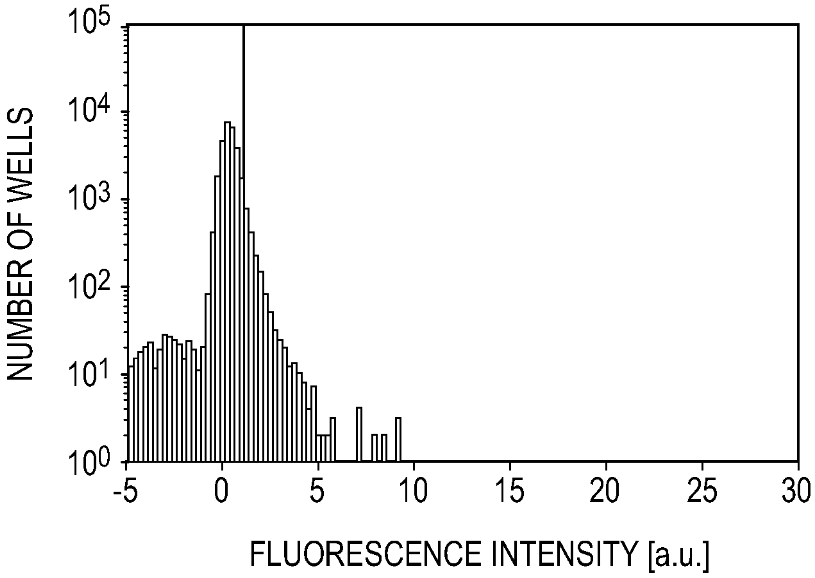
NUMBER OF WELLS
$10^5$
$10^4$
$10^3$
$10^2$
$10^1$
$10^0$
-5
0
5
10
15
20
25
30
FLUORESCENCE INTENSITY [a.u.]

FIG. 44E

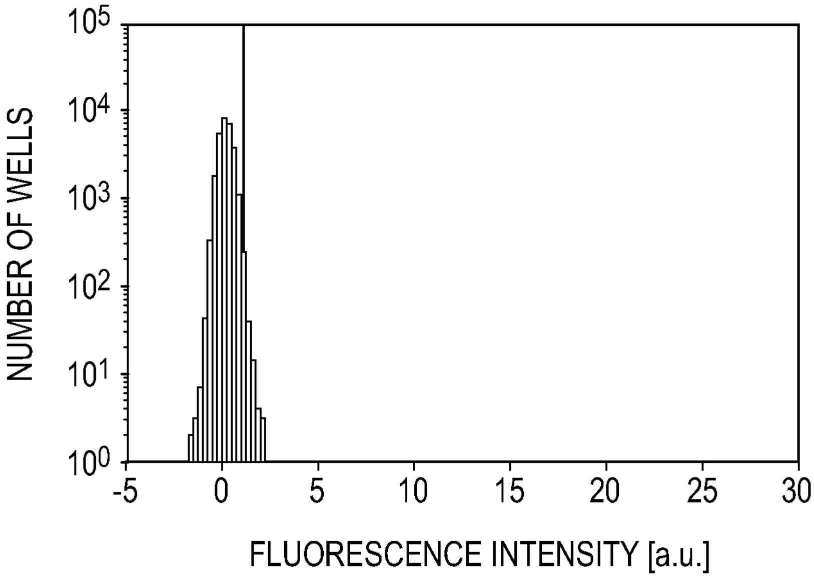
$10^5$
$10^4$
$10^3$
$10^2$
$10^1$
$10^0$
NUMBER OF WELLS
-5
0
5
10
15
20
25
30
FLUORESCENCE INTENSITY [a.u.]

FIG. 45

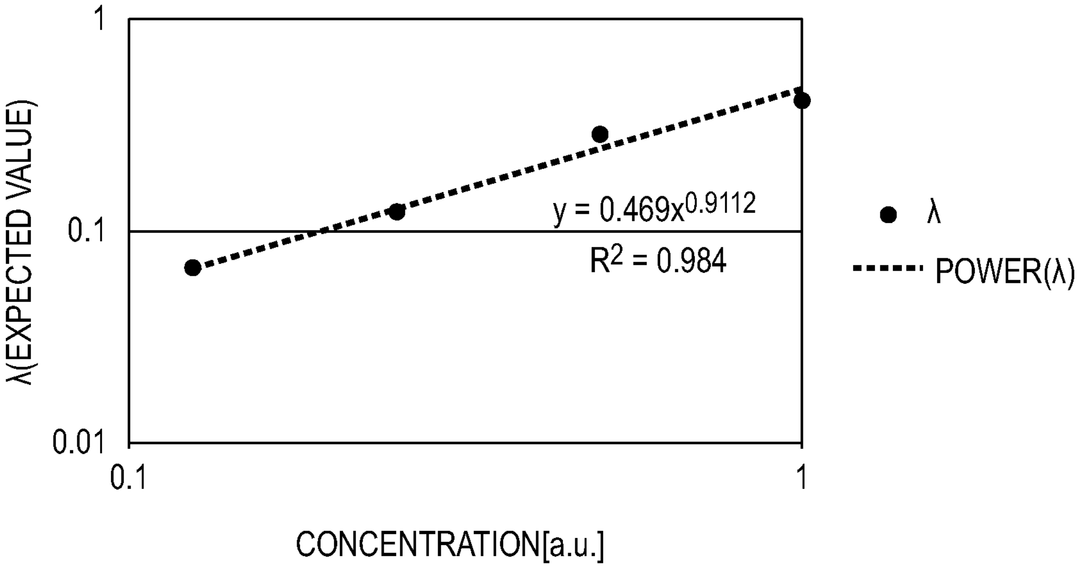
1
0.1
0.01
λ(EXPECTED VALUE)
0.1
1
CONCENTRATION[a.u.]
y = 0.469x^0.9112
R2 = 0.984
λ
POWER(λ)

FIG. 46

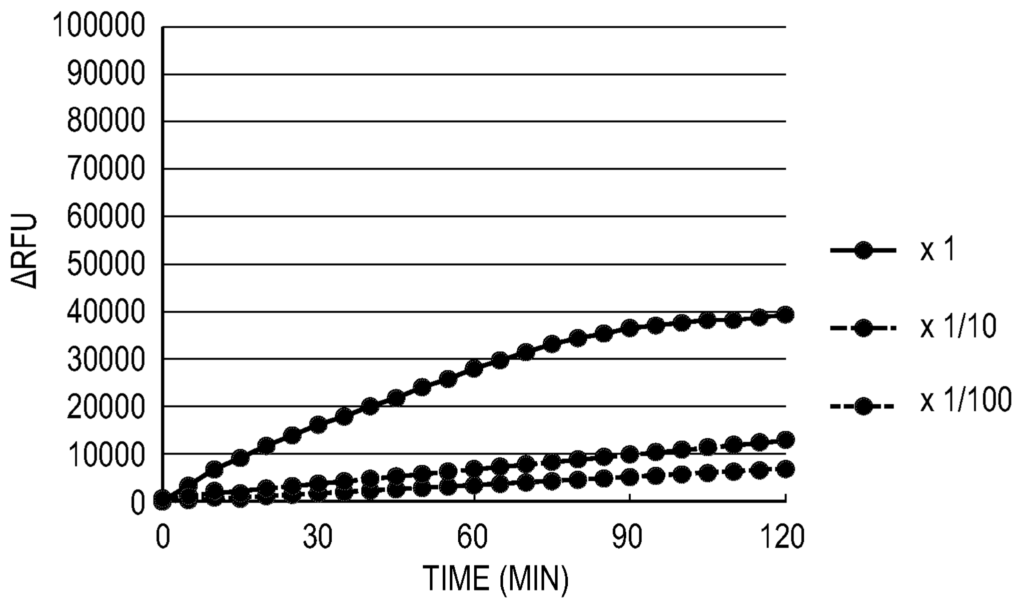
100000
90000
80000
70000
60000
50000
40000
30000
20000
10000
0
ΔRFU
0
30
60
90
120
TIME (MIN)
x 1
x 1/10
x 1/100

FIG. 47A

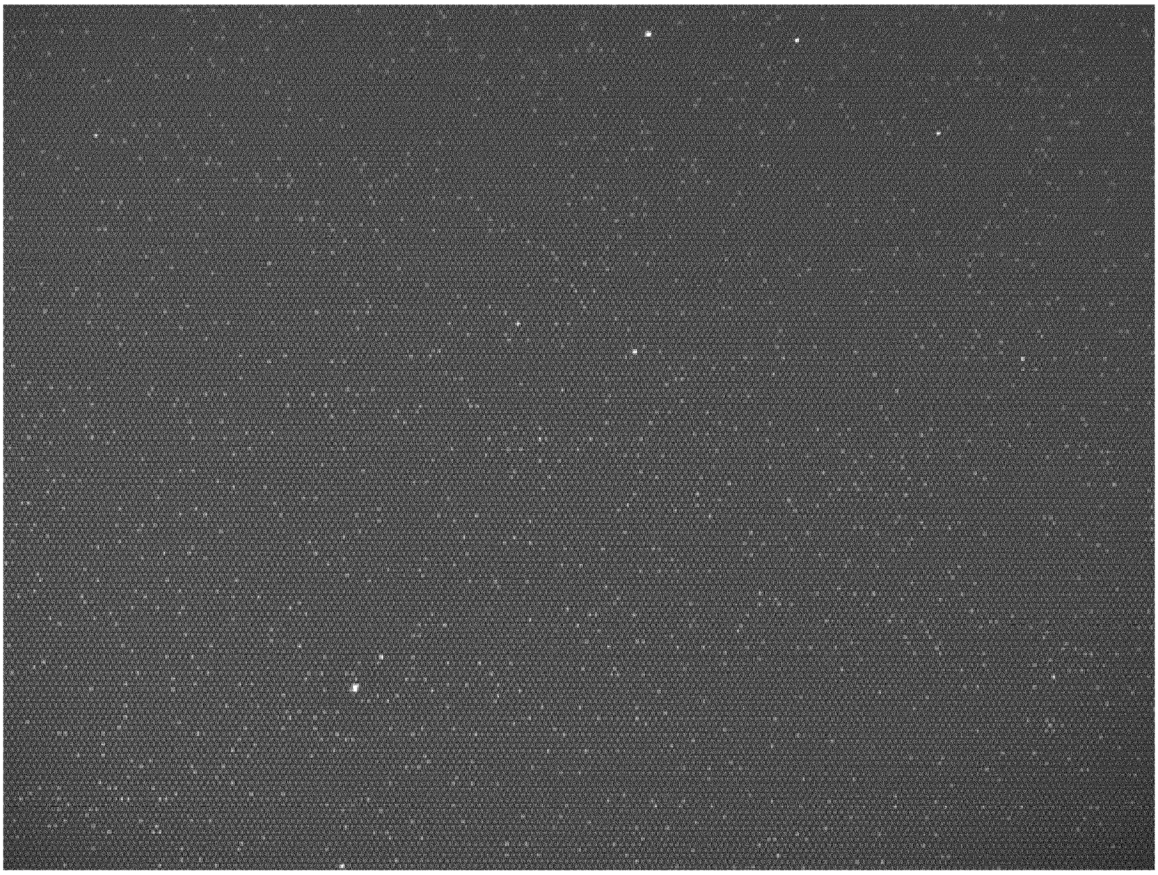

FIG. 47B

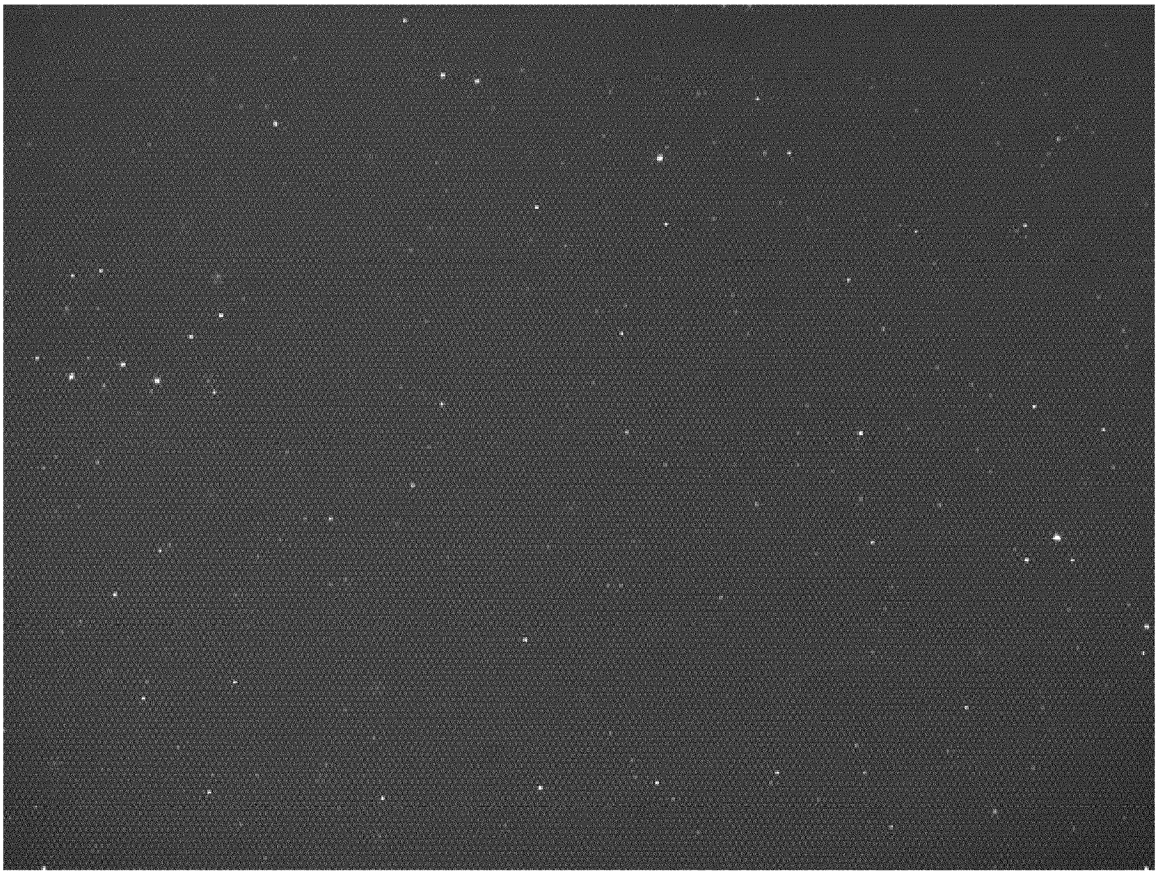

FIG. 47C

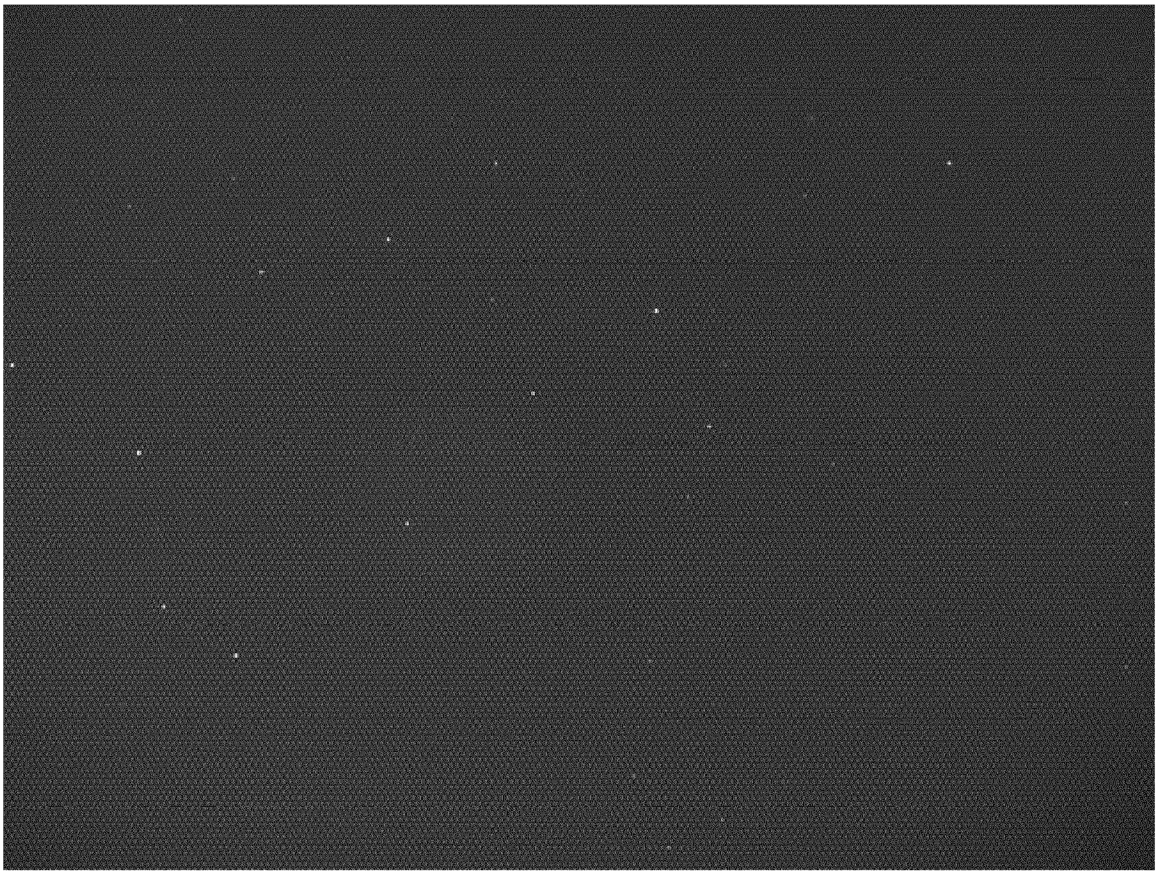

FIG. 47D

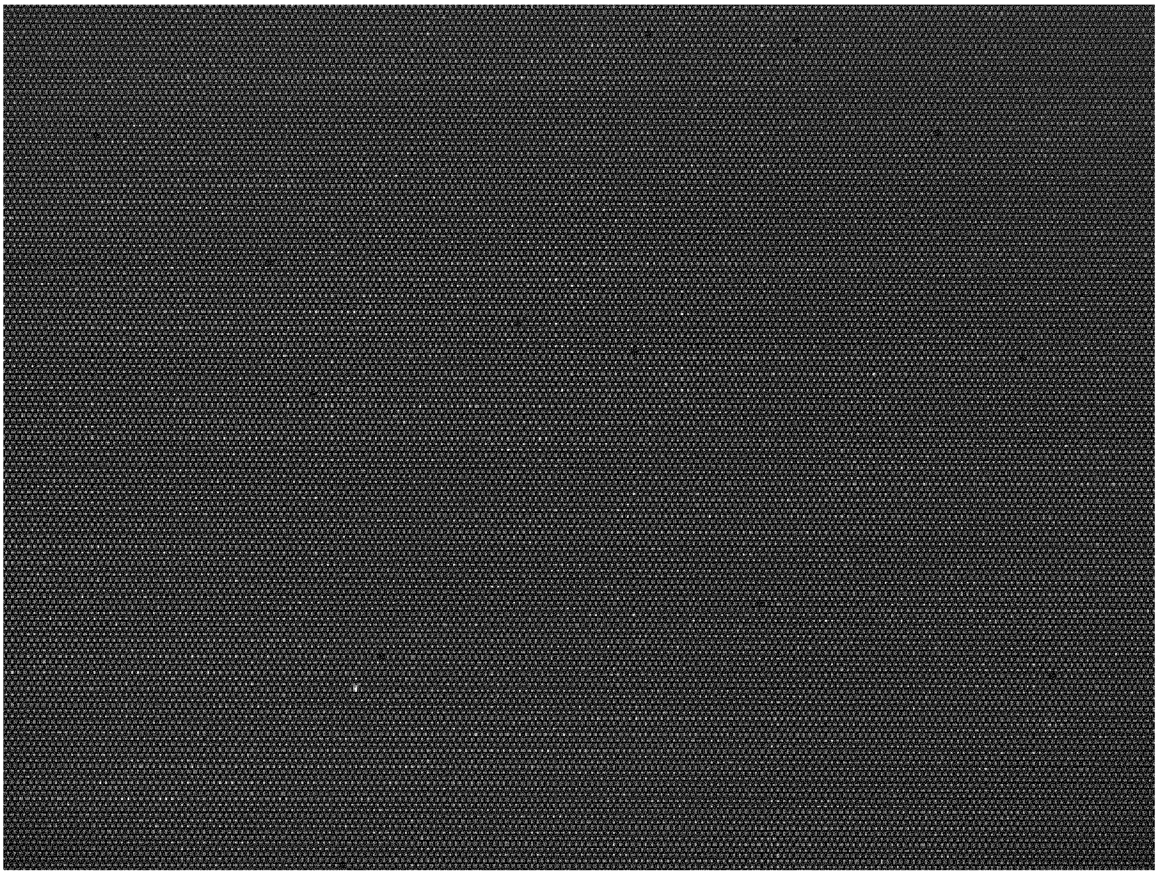

FIG. 47E

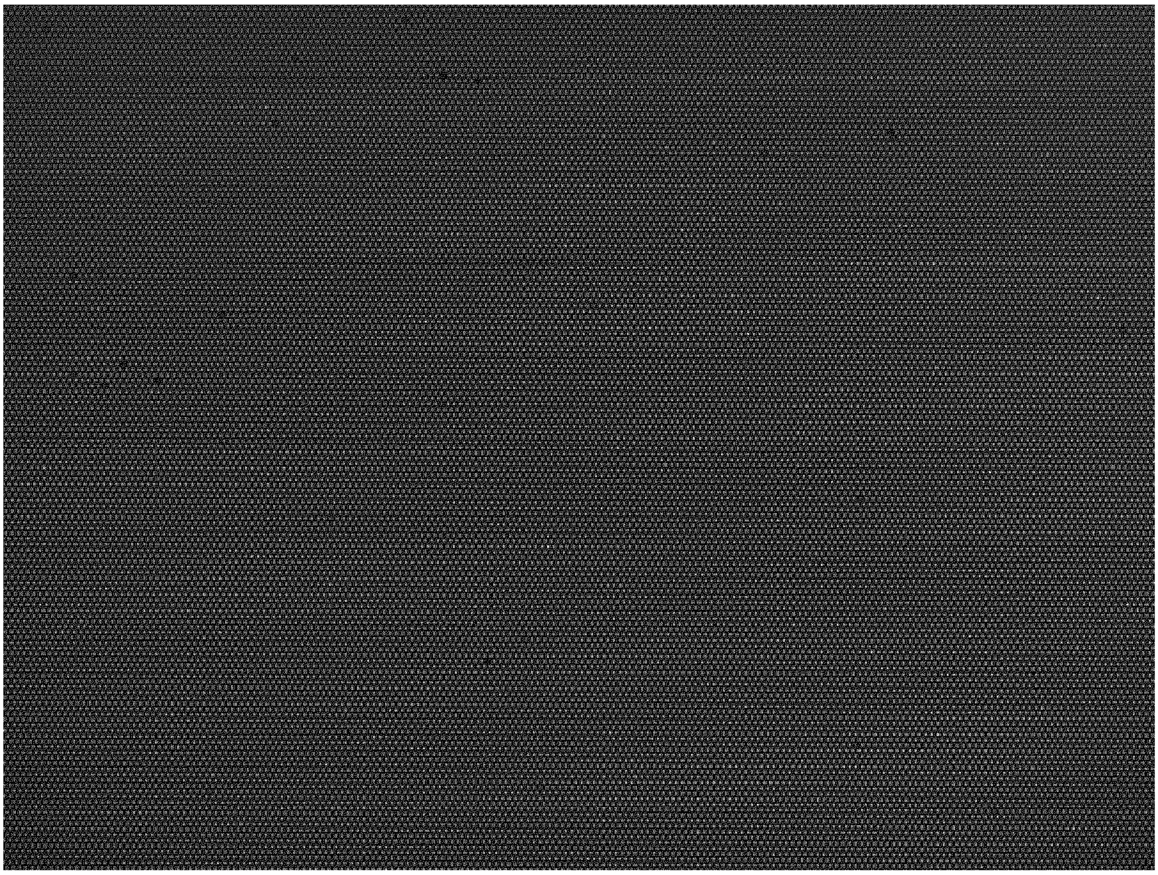

FIG. 47F

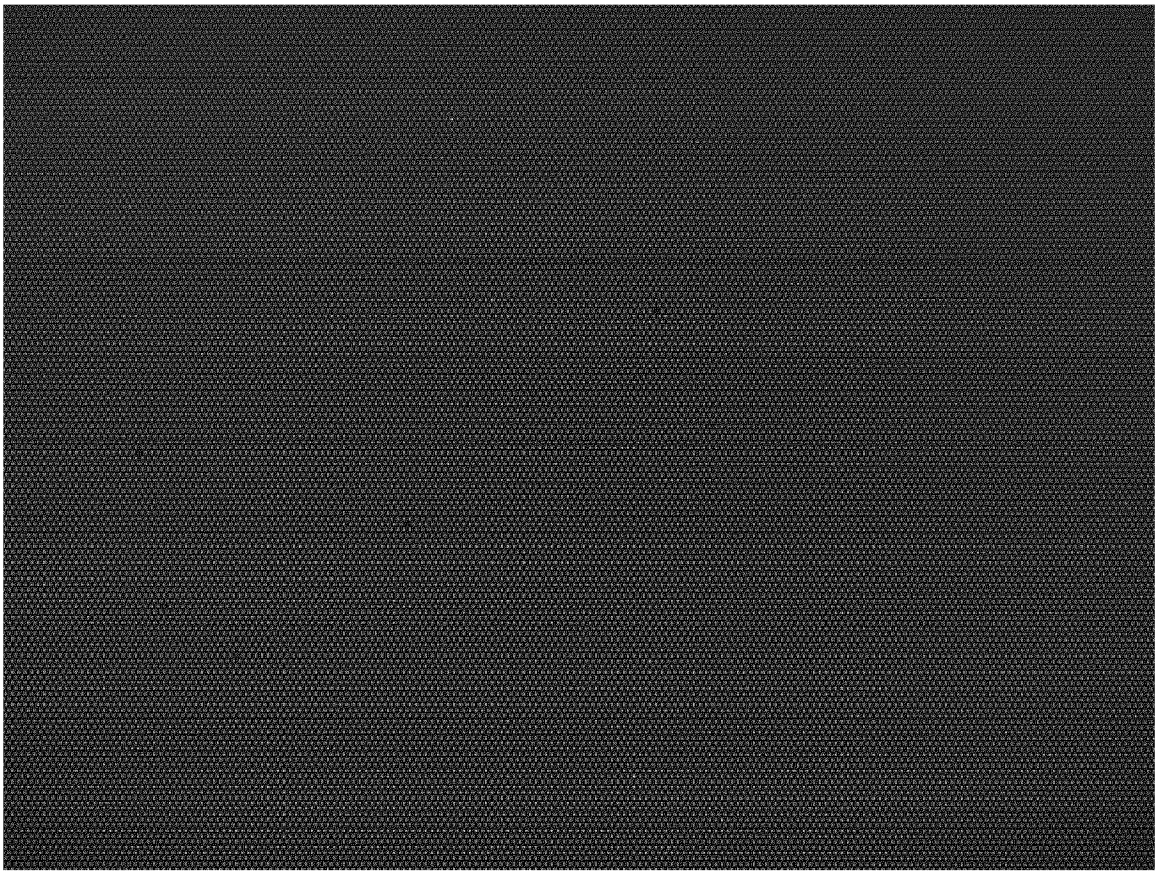

FIG. 48A

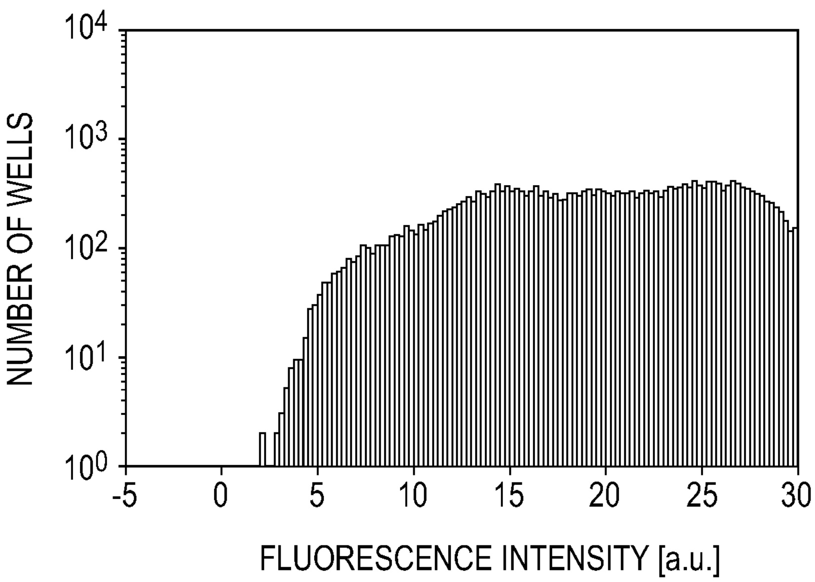
NUMBER OF WELLS
$10^4$
$10^3$
$10^2$
$10^1$
$10^0$
-5
0
5
10
15
20
25
30
FLUORESCENCE INTENSITY [a.u.]

FIG. 48B

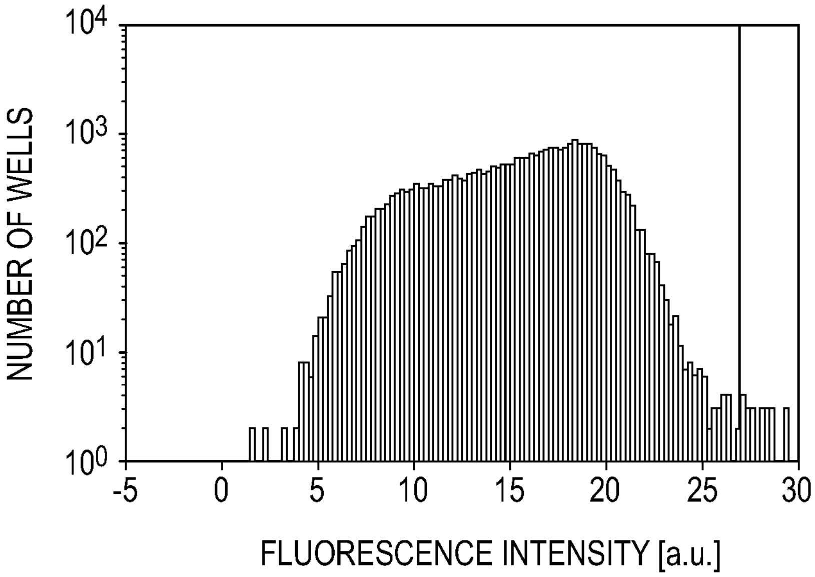

NUMBER OF WELLS
10^4
10^3
10^2
10^1
10^0
-5
0
5
10
15
20
25
30
FLUORESCENCE INTENSITY [a.u.]

FIG. 48C

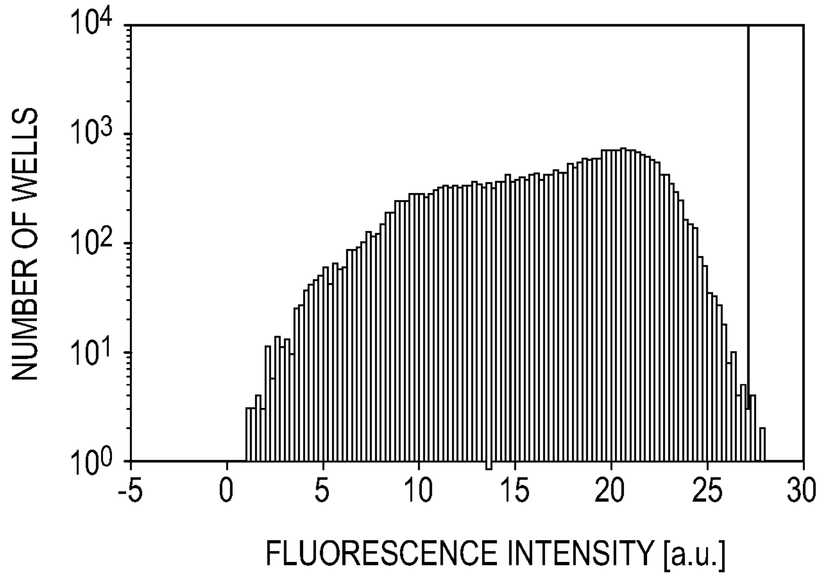

$10^4$
$10^3$
$10^2$
$10^1$
$10^0$
NUMBER OF WELLS
-5
0
5
10
15
20
25
30
FLUORESCENCE INTENSITY [a.u.]

FIG. 49

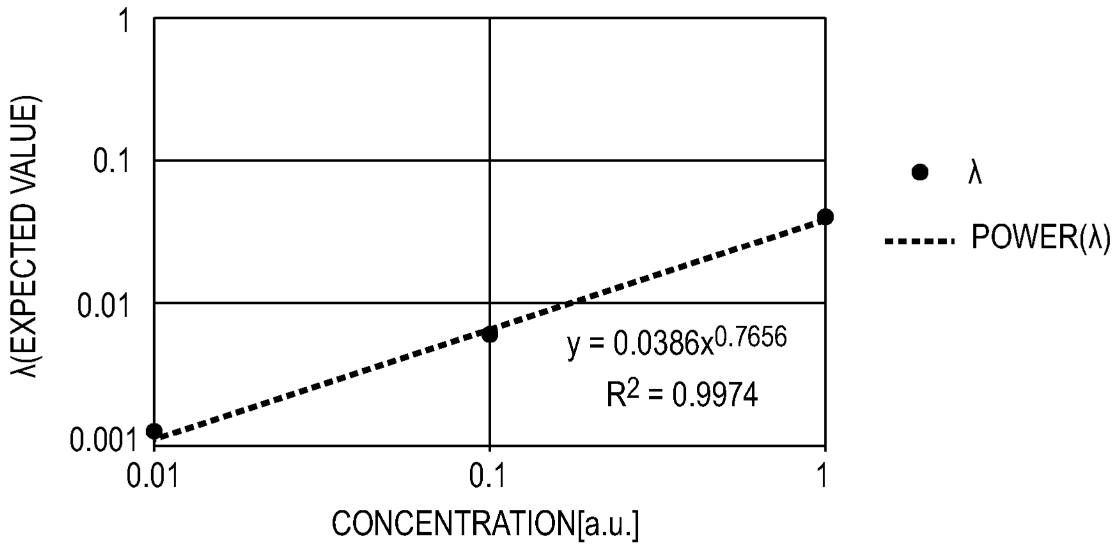
1
0.1
0.01
0.001
λ(EXPECTED VALUE)
0.01
0.1
1
CONCENTRATION[a.u.]
y = 0.0386x^0.7656
R^2 = 0.9974
λ
POWER(λ)

FIG. 50

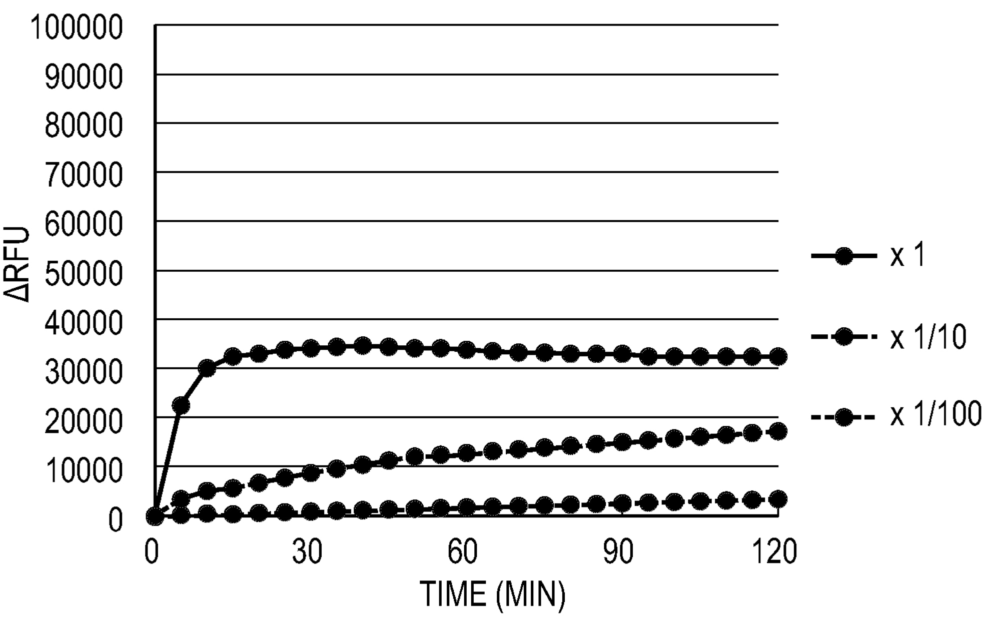
100000
90000
80000
70000
60000
50000
40000
30000
20000
10000
0
ΔRFU
0
30
60
90
120
TIME (MIN)
x 1
x 1/10
x 1/100

FIG. 51A

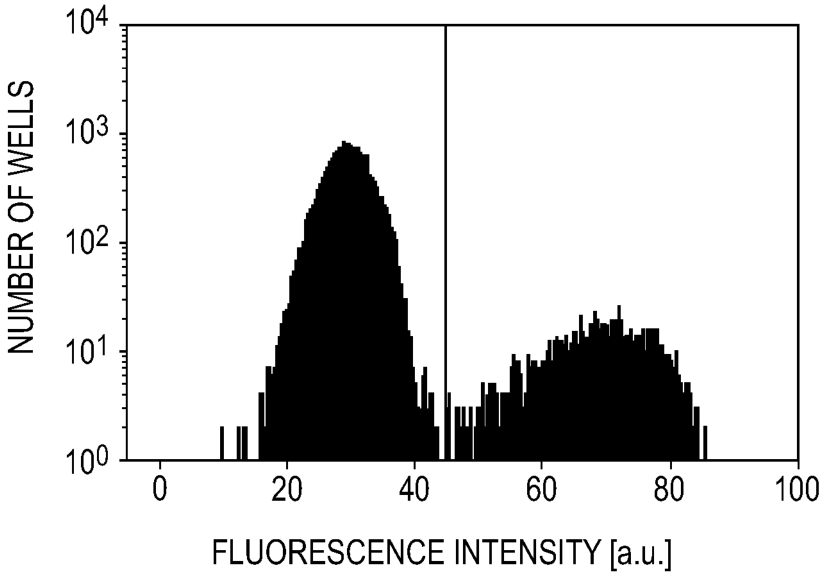
NUMBER OF WELLS
$10^4$
$10^3$
$10^2$
$10^1$
$10^0$
0
20
40
60
80
100
FLUORESCENCE INTENSITY [a.u.]

FIG. 51B

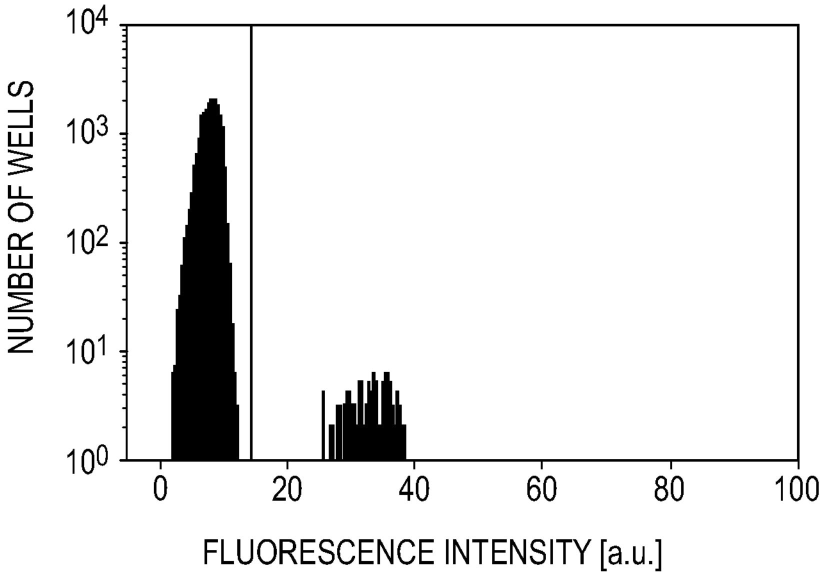
10^4
10^3
10^2
10^1
10^0
NUMBER OF WELLS
0
20
40
60
80
100
FLUORESCENCE INTENSITY [a.u.]

FIG. 51C

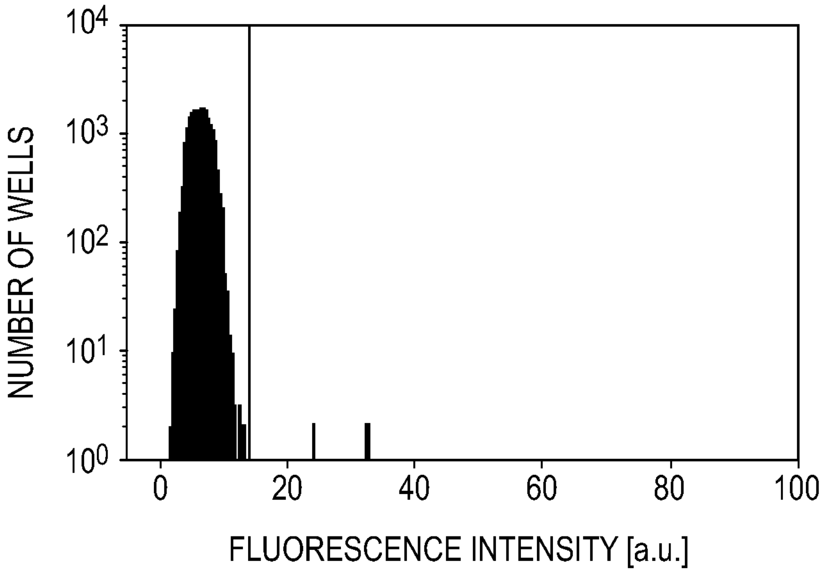

NUMBER OF WELLS
$10^4$
$10^3$
$10^2$
$10^1$
$10^0$
0
20
40
60
80
100
FLUORESCENCE INTENSITY [a.u.]

FIG. 51D

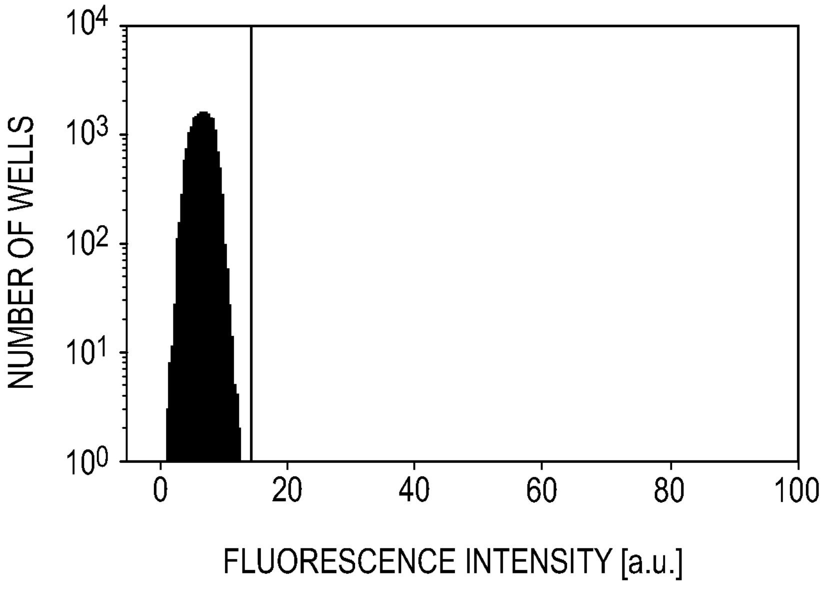
NUMBER OF WELLS
$10^4$
$10^3$
$10^2$
$10^1$
$10^0$
0
20
40
60
80
100
FLUORESCENCE INTENSITY [a.u.]

FIG. 52

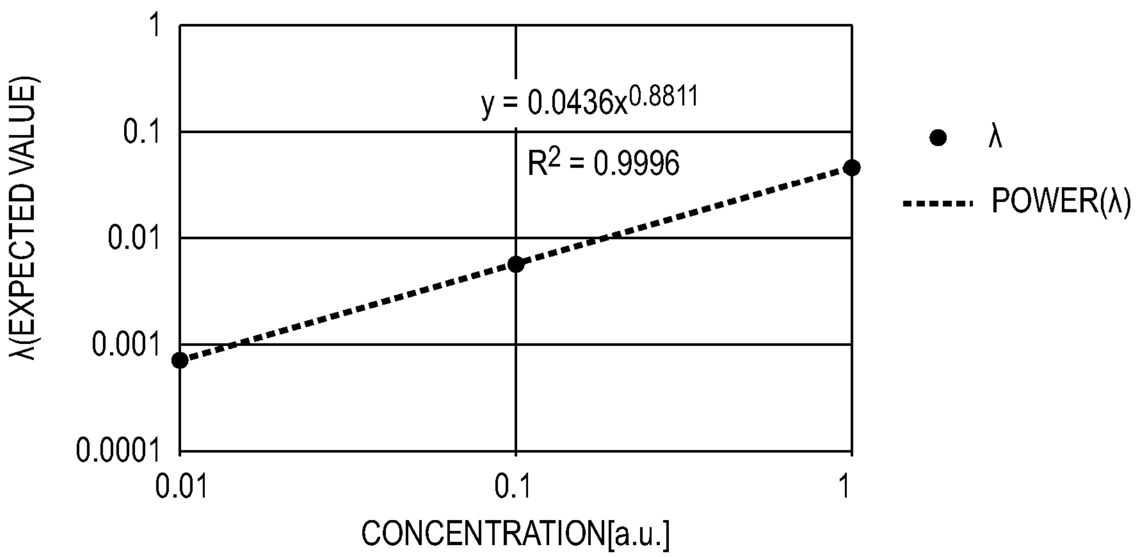
1
0.1
0.01
0.001
0.0001
λ(EXPECTED VALUE)
y = 0.0436x^0.8811
R^2 = 0.9996
0.01
0.1
1
CONCENTRATION[a.u.]
λ
POWER(λ)

FIG. 53

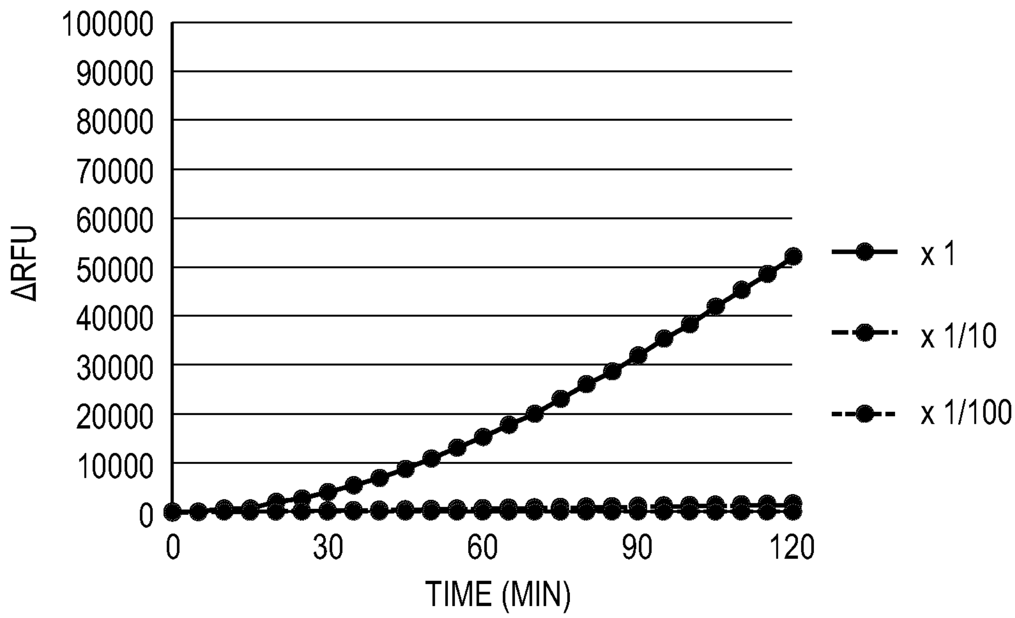
100000
90000
80000
70000
60000
50000
40000
30000
20000
10000
0
ΔRFU
0
30
60
90
120
TIME (MIN)
x 1
x 1/10
x 1/100

FIG. 54A

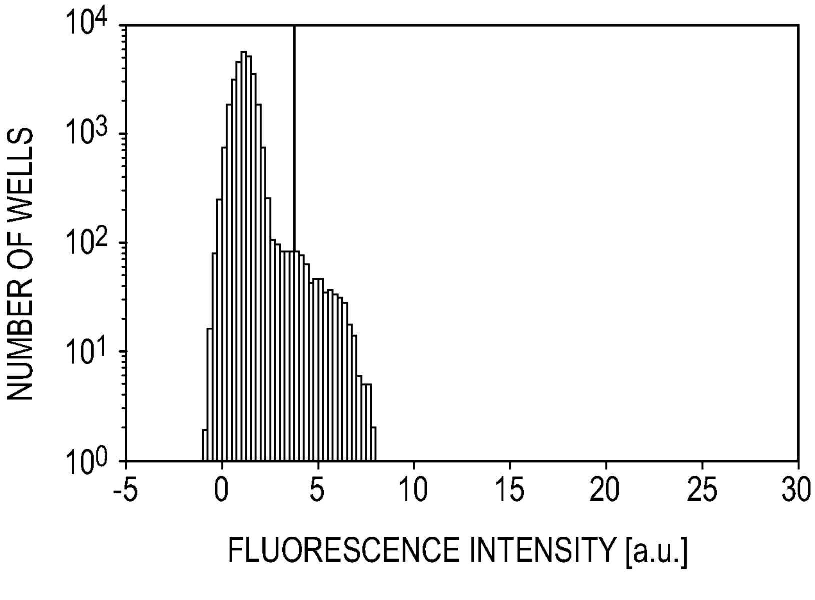
10^4
10^3
10^2
10^1
10^0
NUMBER OF WELLS
-5
0
5
10
15
20
25
30
FLUORESCENCE INTENSITY [a.u.]

FIG. 54B

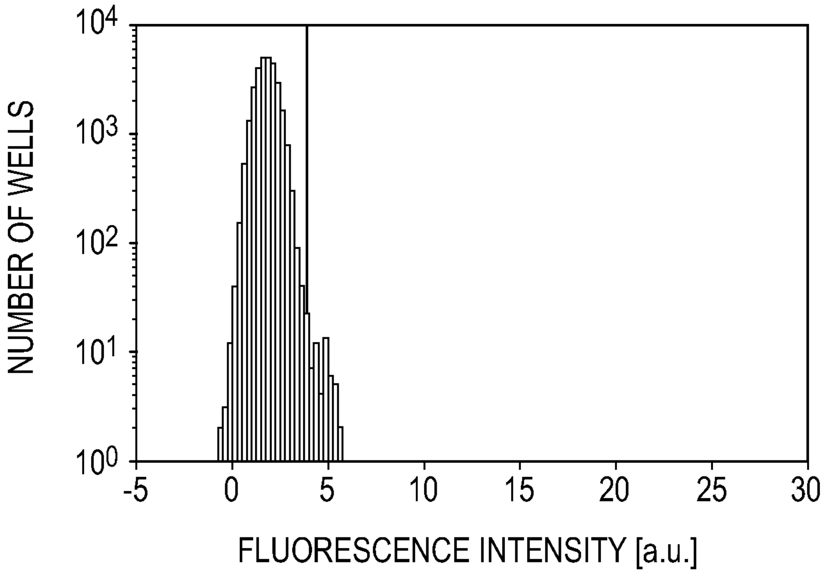
10^4
10^3
10^2
10^1
10^0
NUMBER OF WELLS
-5
0
5
10
15
20
25
30
FLUORESCENCE INTENSITY [a.u.]

FIG. 54C

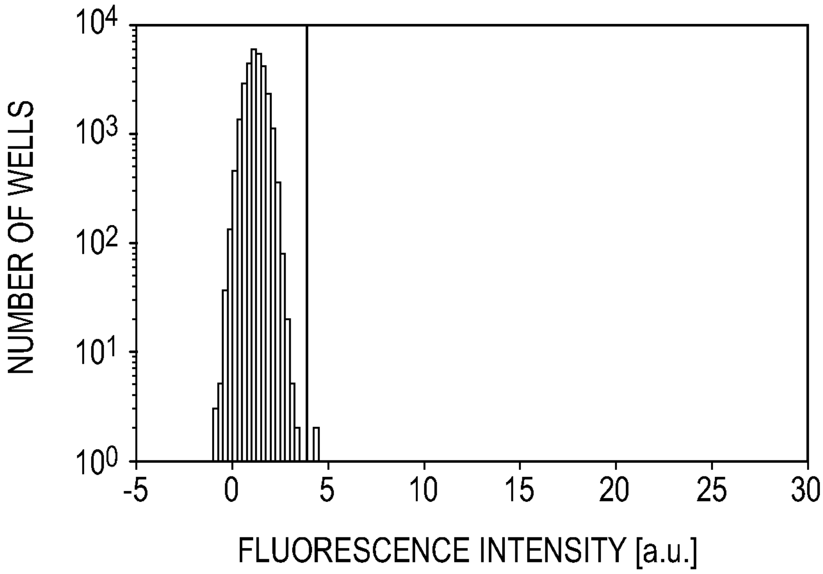
NUMBER OF WELLS
10^4
10^3
10^2
10^1
10^0
-5
0
5
10
15
20
25
30
FLUORESCENCE INTENSITY [a.u.]

FIG. 54D

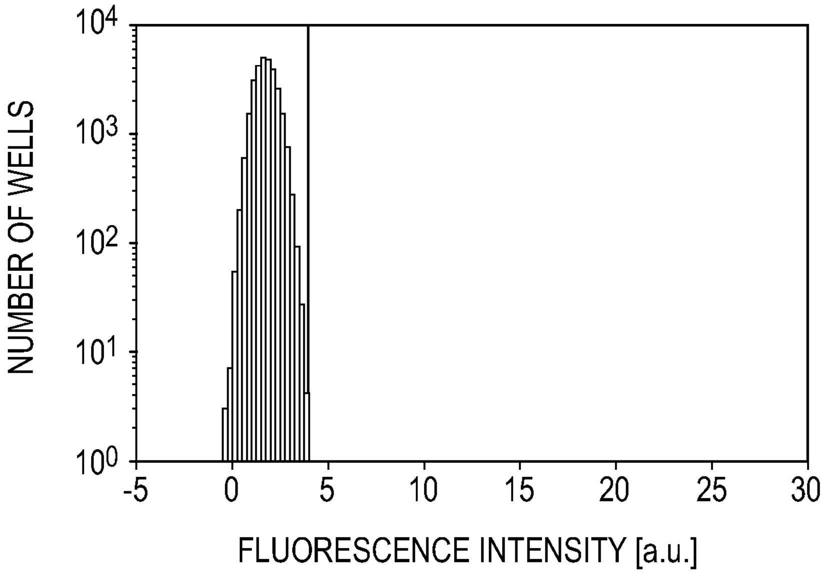
NUMBER OF WELLS
$10^4$
$10^3$
$10^2$
$10^1$
$10^0$
-5
0
5
10
15
20
25
30
FLUORESCENCE INTENSITY [a.u.]

FIG. 55

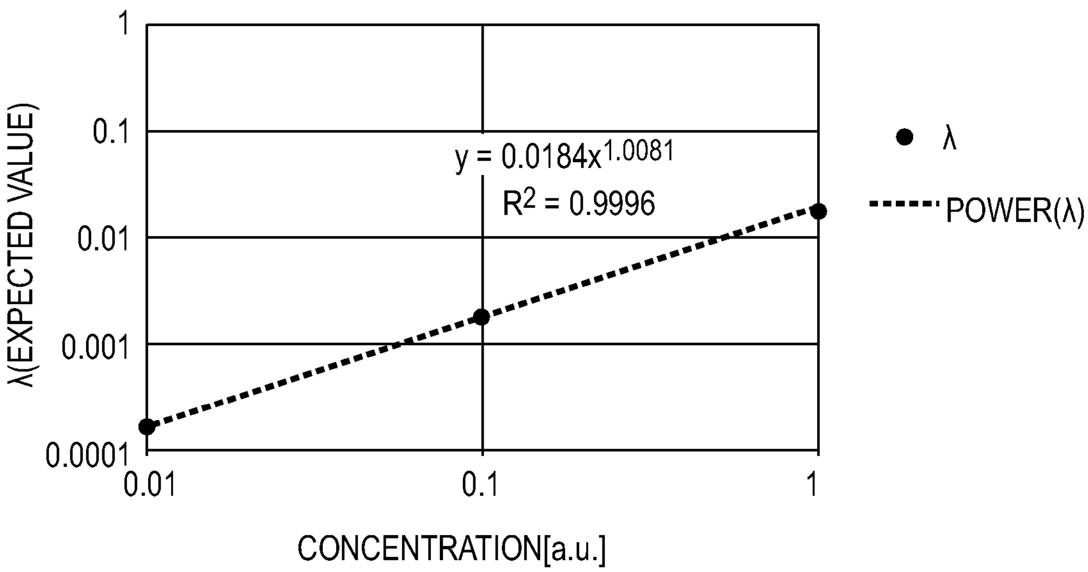
1
0.1
0.01
0.001
0.0001
λ(EXPECTED VALUE)
y = 0.0184x^1.0081
R2 = 0.9996
0.01
0.1
1
CONCENTRATION[a.u.]
λ
POWER(λ)

FIG. 56

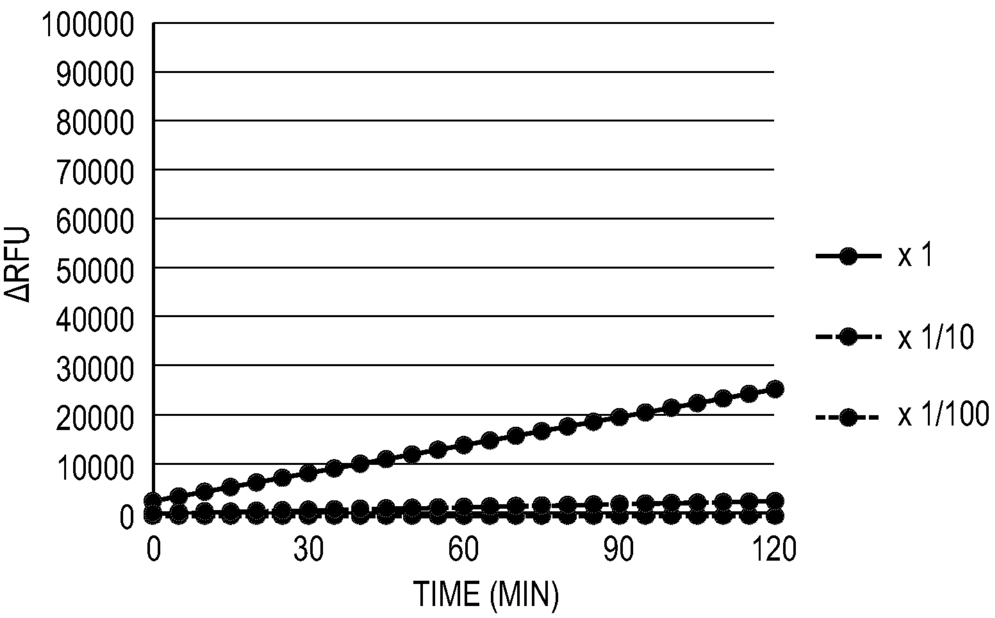
100000
90000
80000
70000
60000
50000
40000
30000
20000
10000
0
ΔRFU
0
30
60
90
120
TIME (MIN)
x 1
x 1/10
x 1/100

FIG. 57A

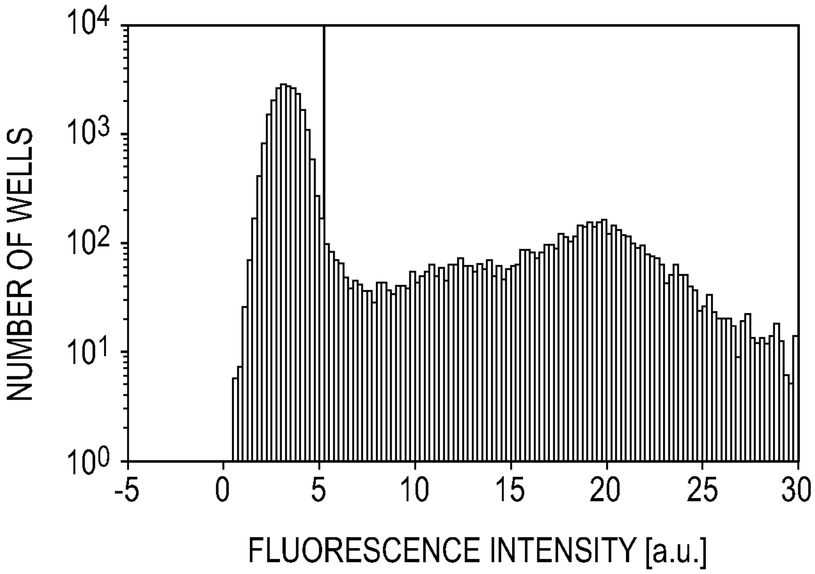
10^4
10^3
10^2
10^1
10^0
NUMBER OF WELLS
-5
0
5
10
15
20
25
30
FLUORESCENCE INTENSITY [a.u.]

FIG. 57B

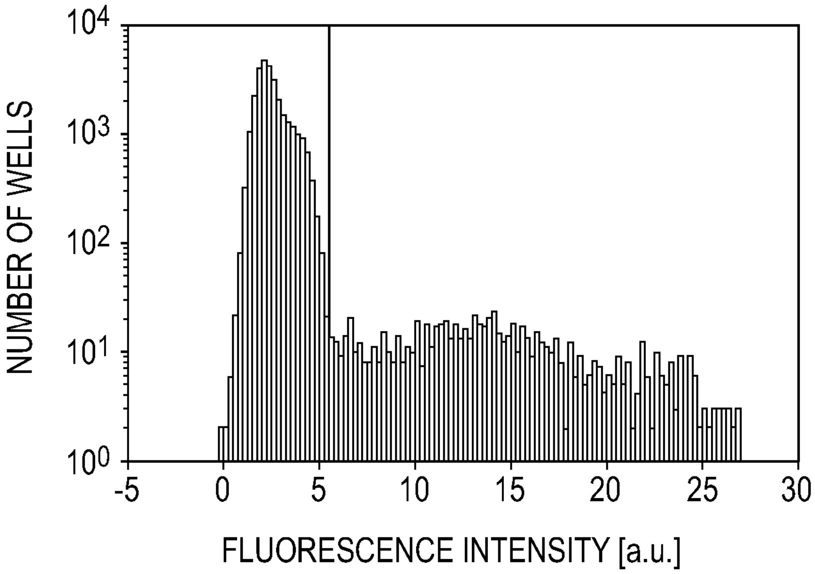

$10^4$
$10^3$
$10^2$
$10^1$
$10^0$
NUMBER OF WELLS
-5
0
5
10
15
20
25
30
FLUORESCENCE INTENSITY [a.u.]

FIG. 57C

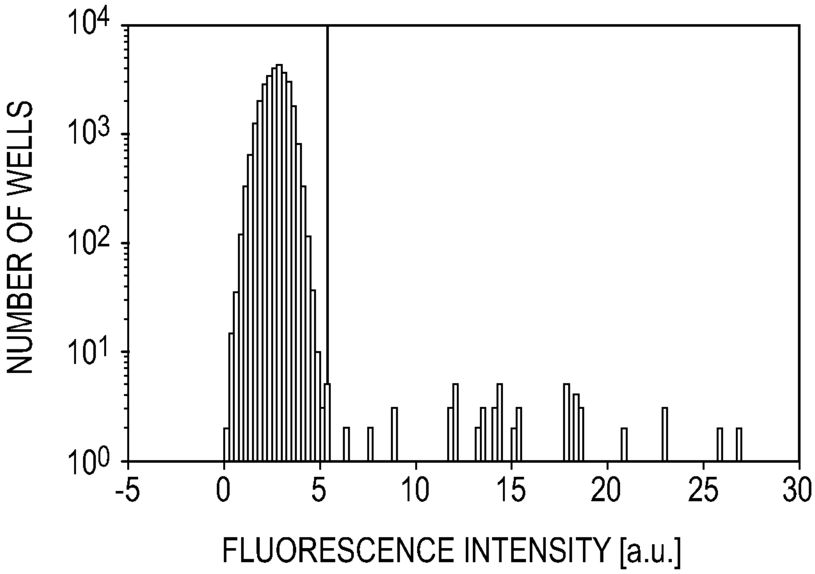

$10^4$
$10^3$
$10^2$
$10^1$
$10^0$
NUMBER OF WELLS
-5
0
5
10
15
20
25
30
FLUORESCENCE INTENSITY [a.u.]

FIG. 57D

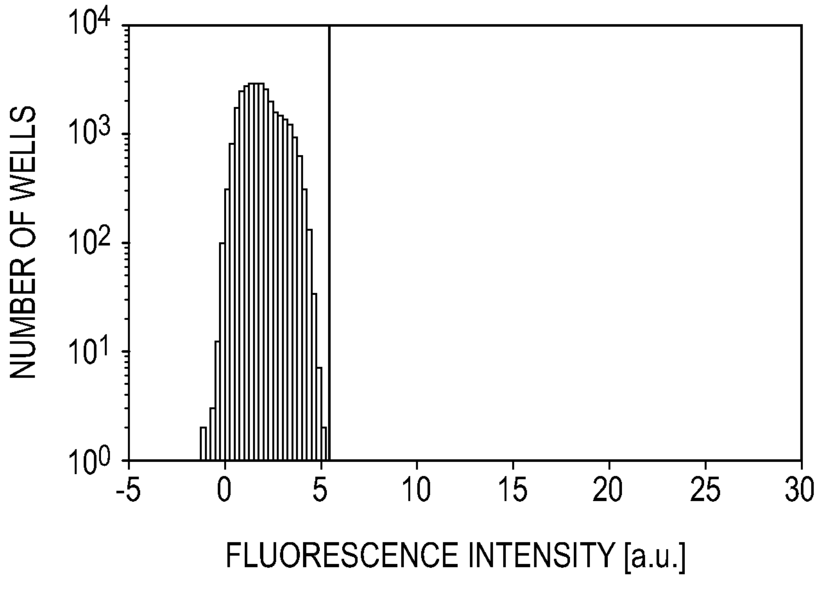

NUMBER OF WELLS
$10^4$
$10^3$
$10^2$
$10^1$
$10^0$
-5
0
5
10
15
20
25
30
FLUORESCENCE INTENSITY [a.u.]

FIG. 58

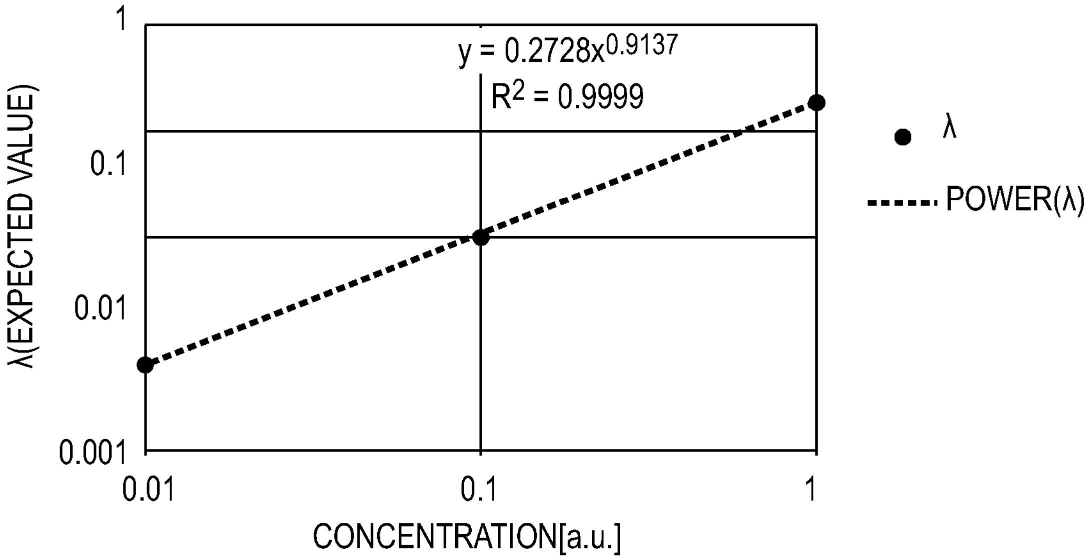

y = 0.2728x^0.9137
R2 = 0.9999
1
0.1
0.01
0.001
λ(EXPECTED VALUE)
0.01
0.1
1
CONCENTRATION[a.u.]
λ
POWER(λ)

FIG. 59A

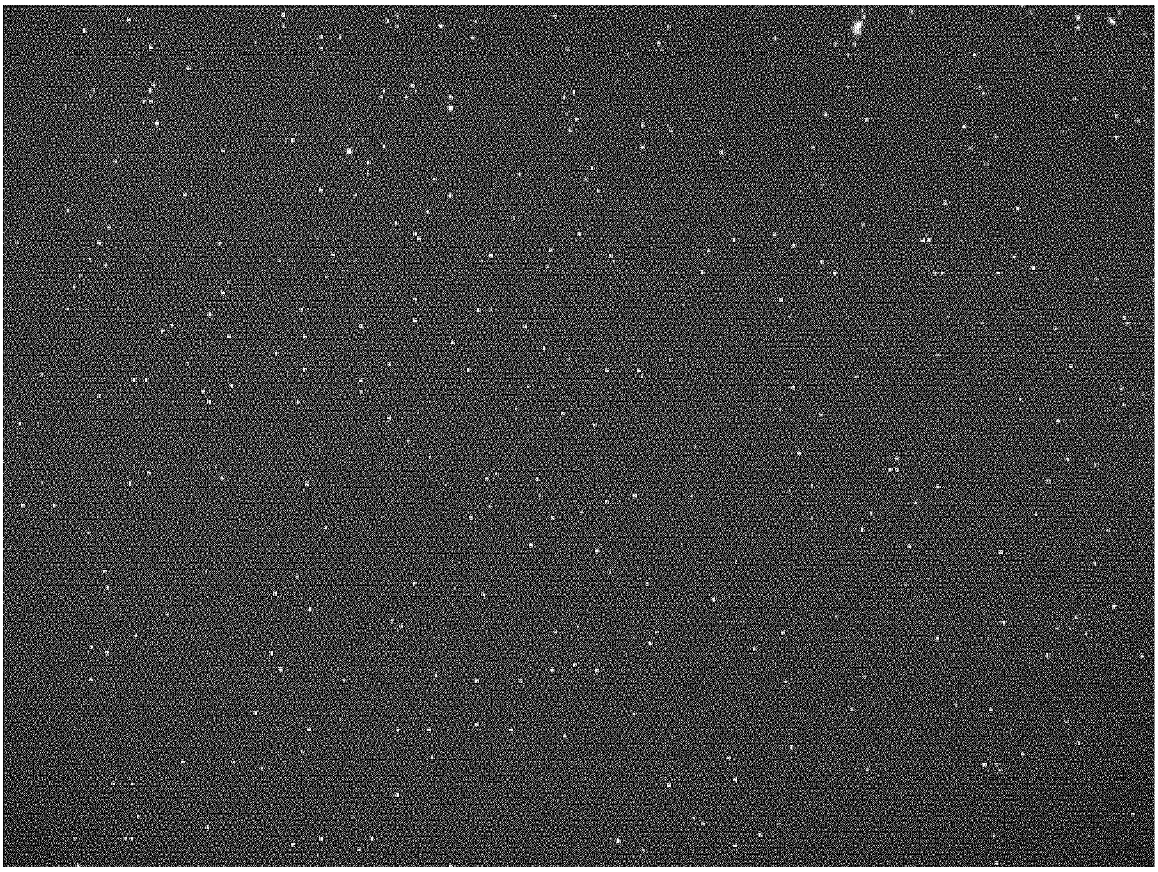

FIG. 59B

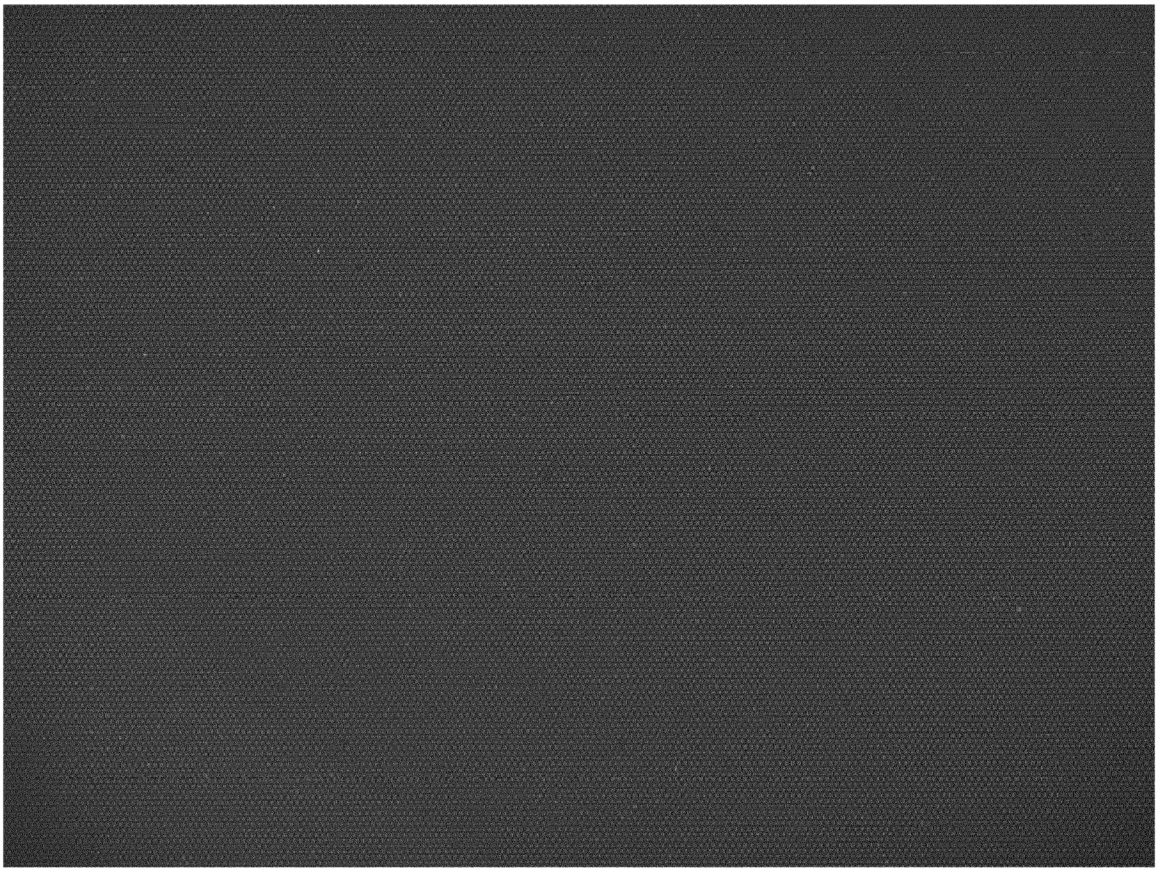

FIG. 59C

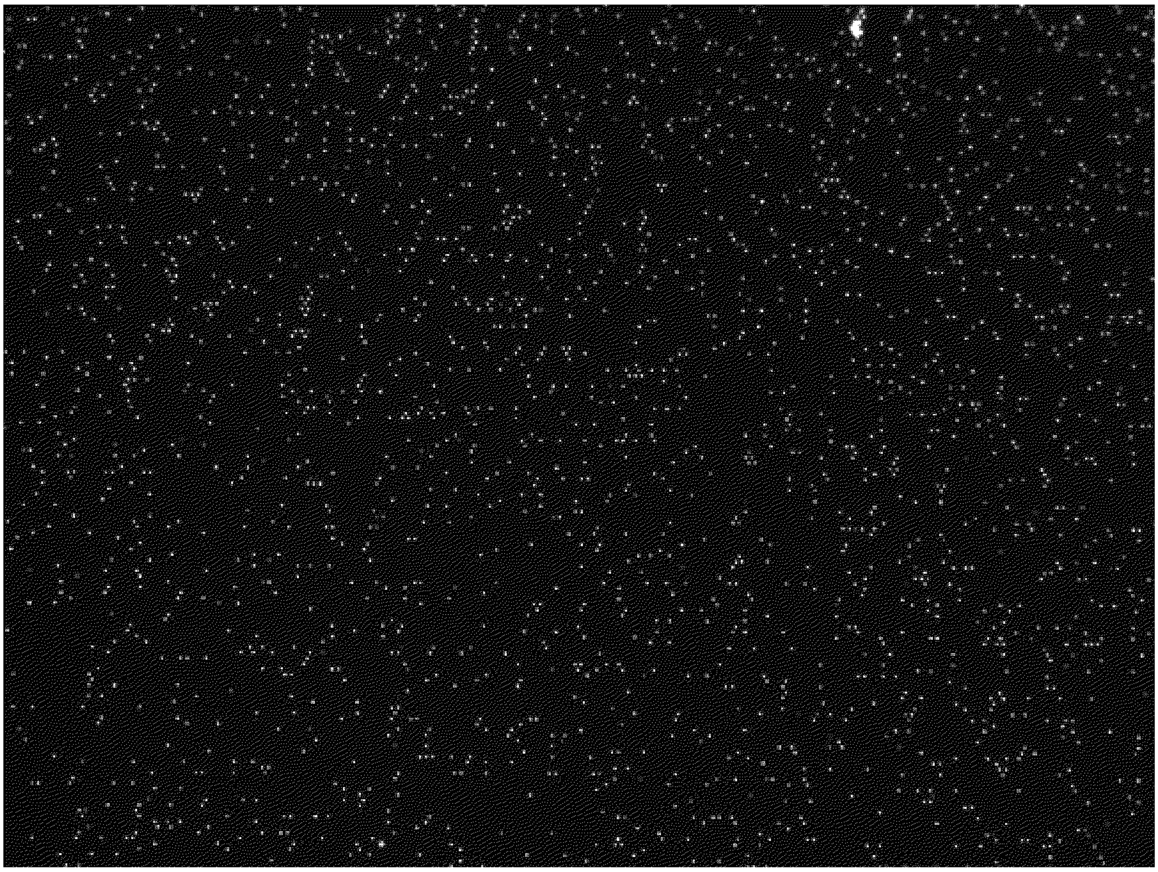

FIG. 59D

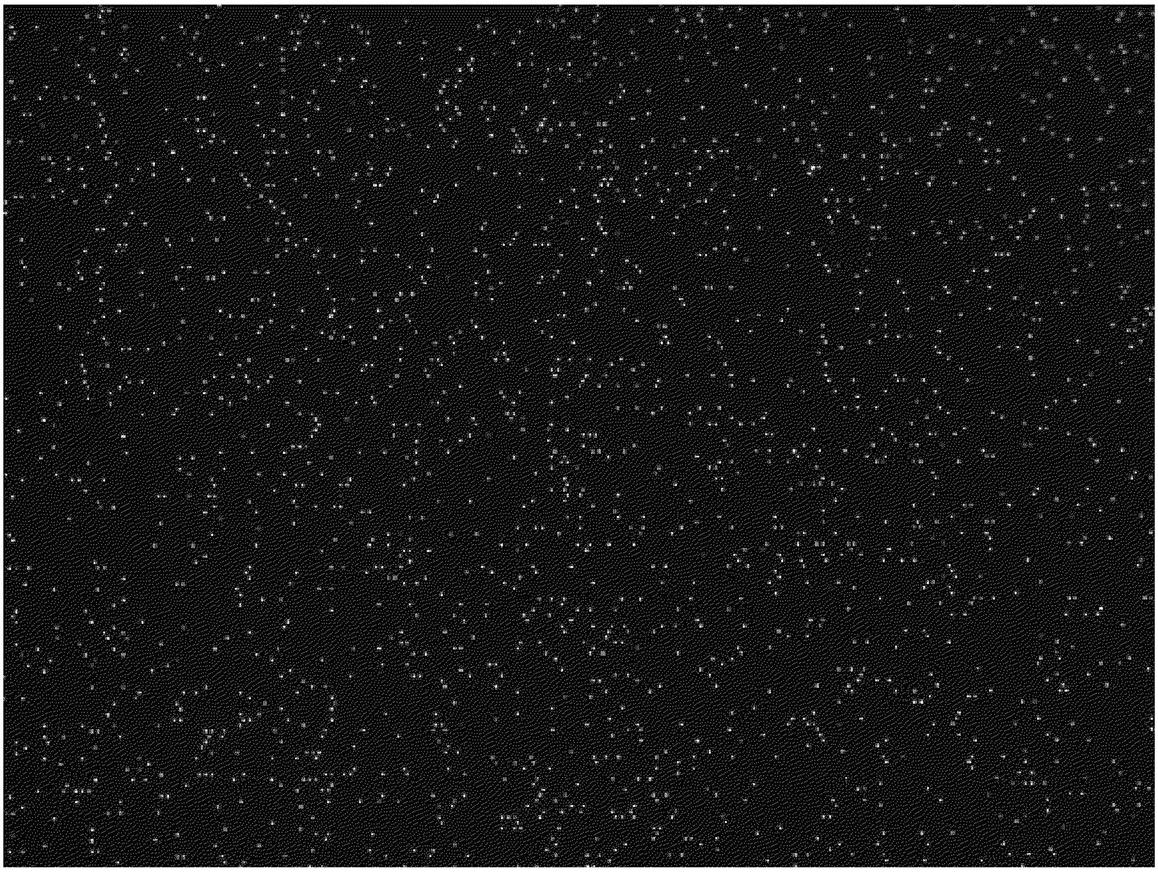

FIG. 60A

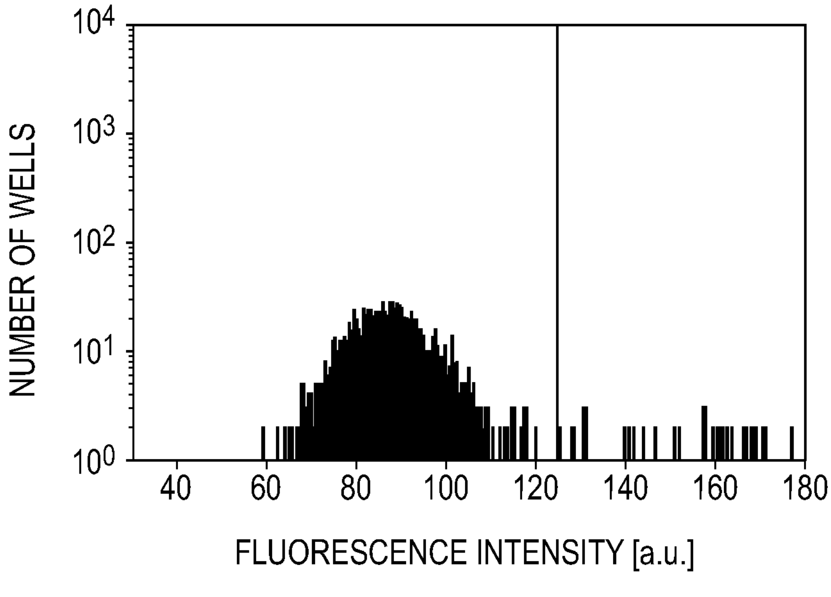

10^4
10^3
10^2
10^1
10^0
NUMBER OF WELLS
40
60
80
100
120
140
160
180
FLUORESCENCE INTENSITY [a.u.]

FIG. 60B

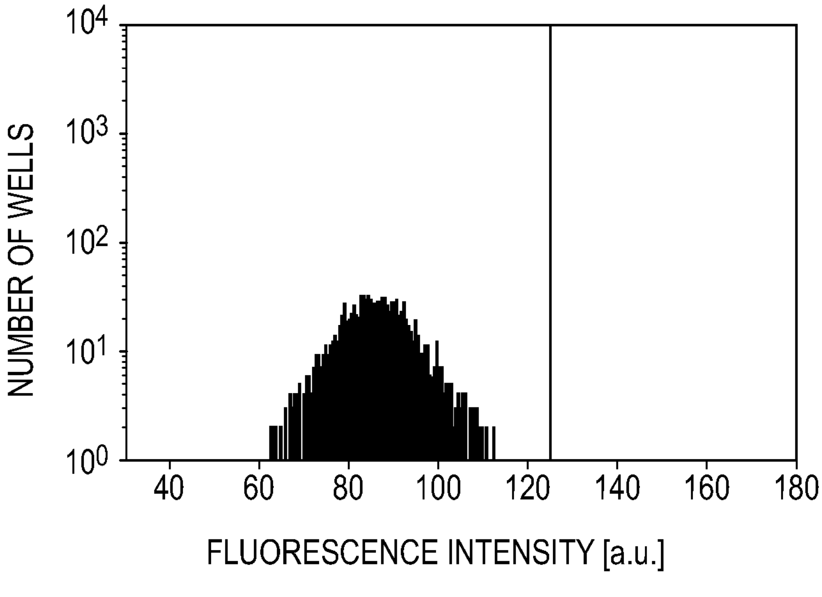
NUMBER OF WELLS
10^4
10^3
10^2
10^1
10^0
40
60
80
100
120
140
160
180
FLUORESCENCE INTENSITY [a.u.]

FIG. 61A

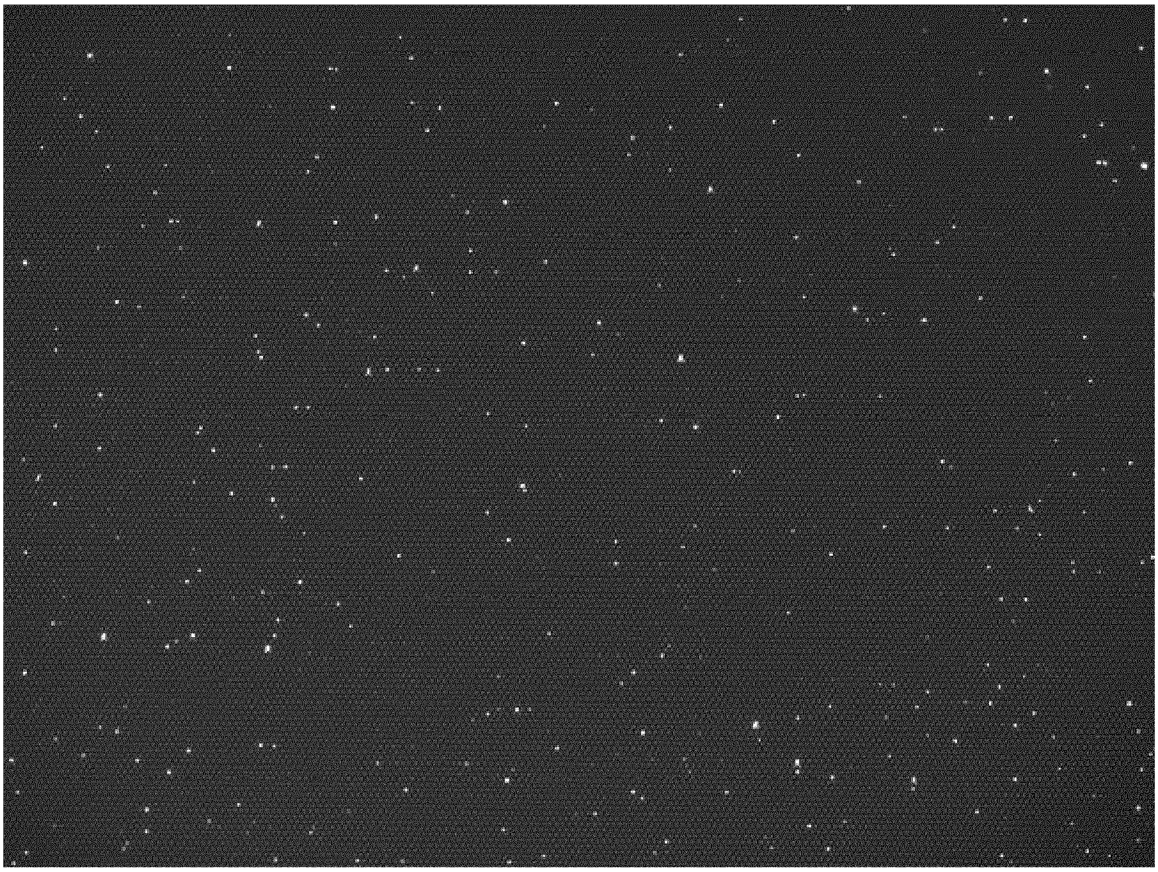

FIG. 61B

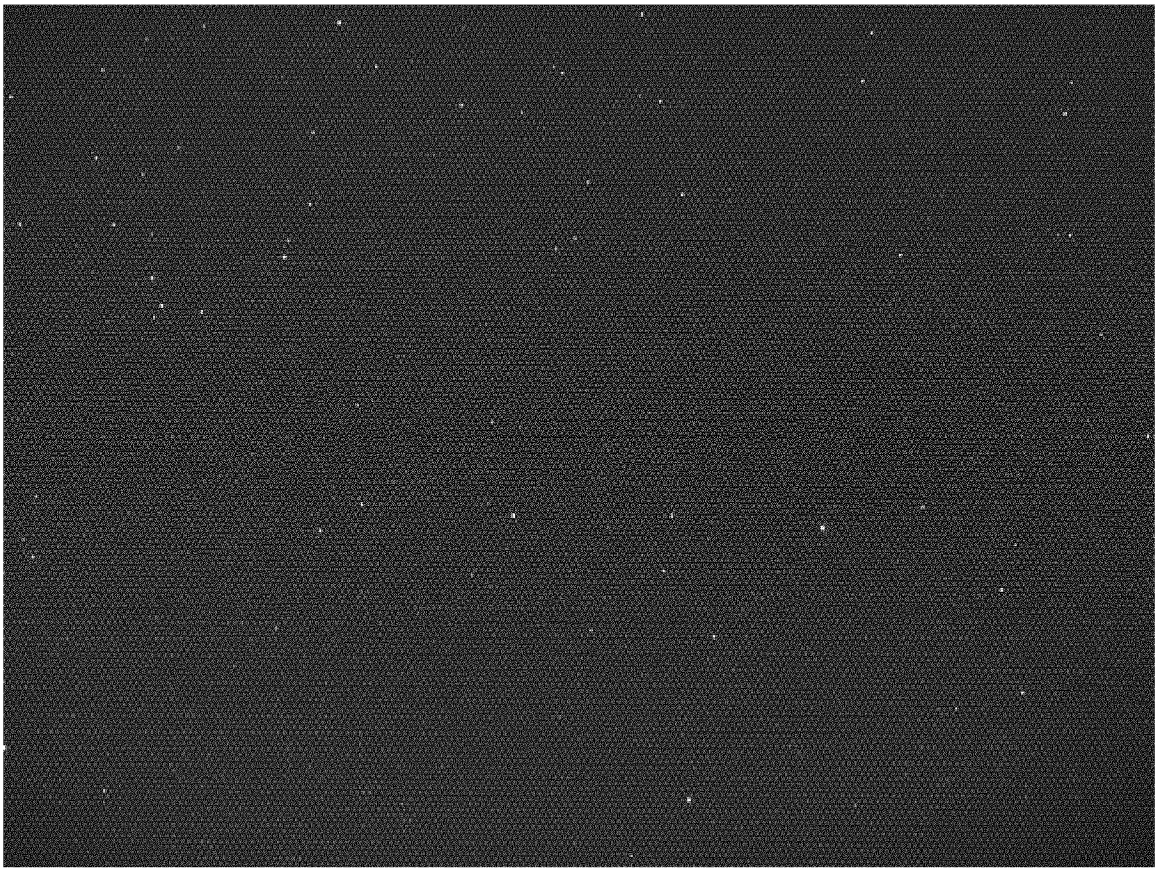

FIG. 61C

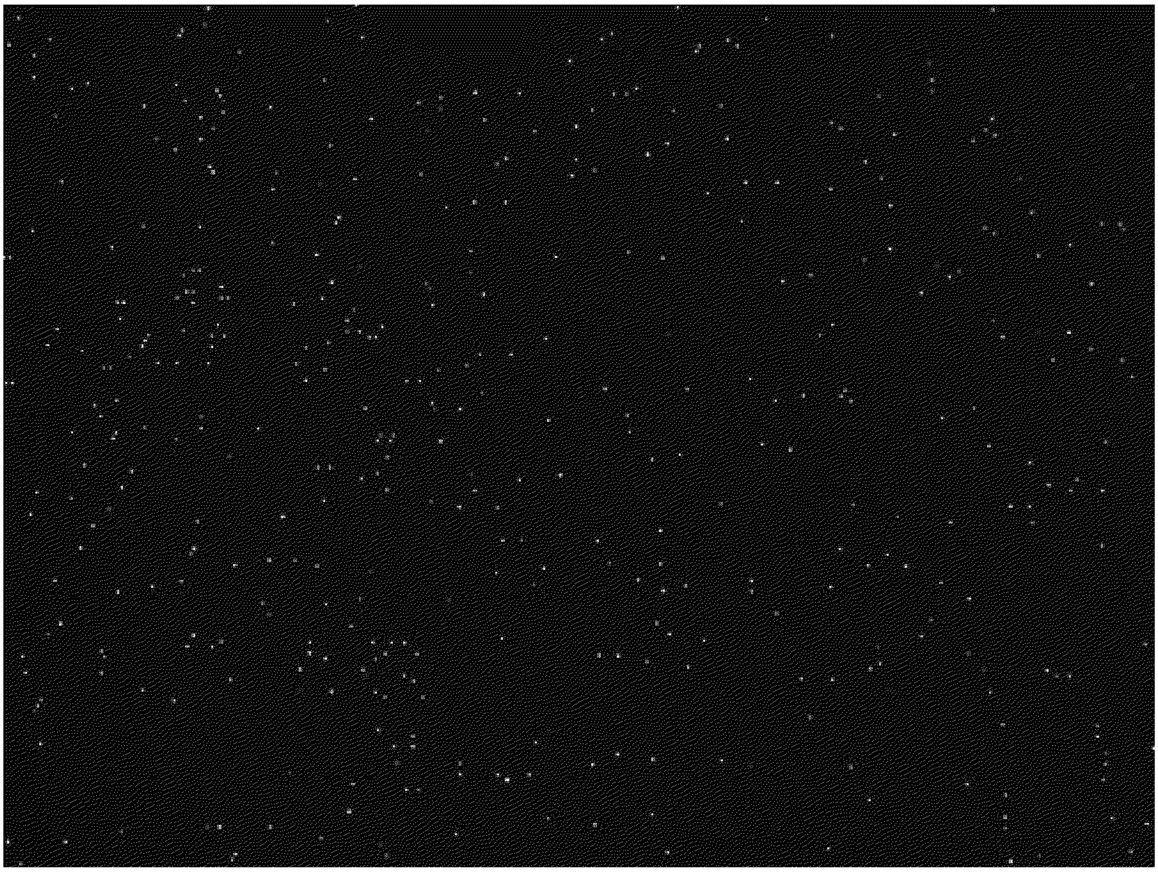

FIG. 61D

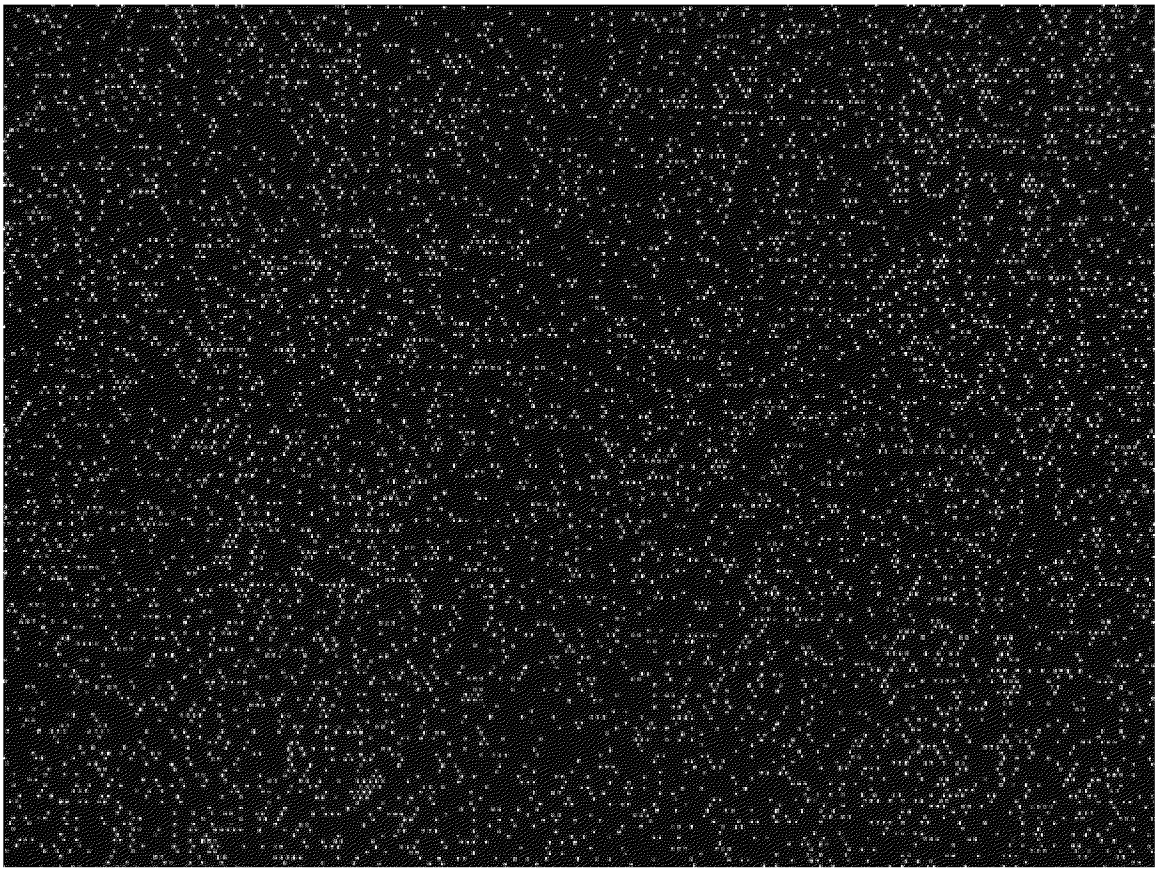

FIG. 62A

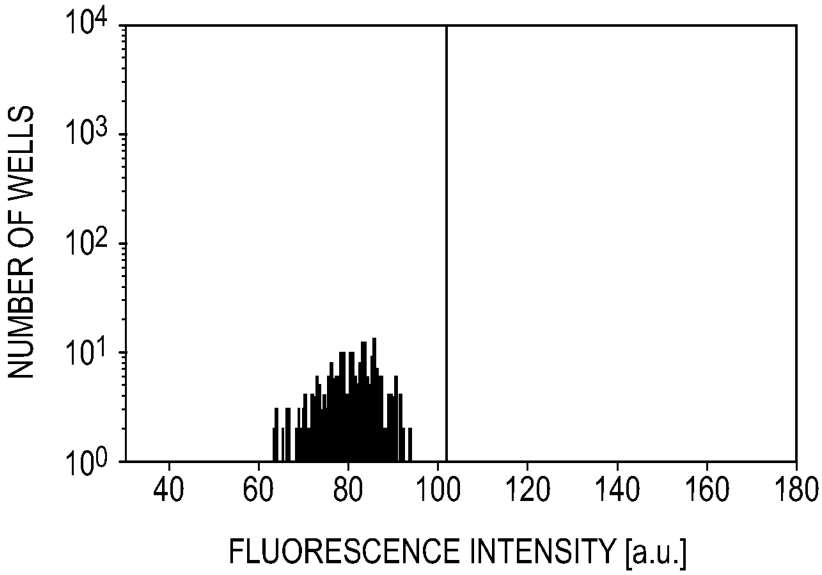
$10^4$
$10^3$
$10^2$
$10^1$
$10^0$
NUMBER OF WELLS
40
60
80
100
120
140
160
180
FLUORESCENCE INTENSITY [a.u.]

FIG. 62B

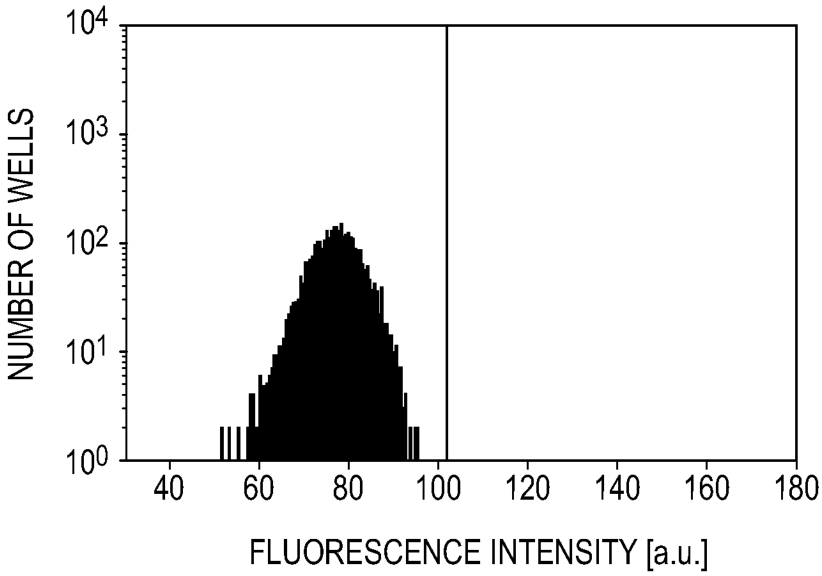

$10^4$
$10^3$
$10^2$
$10^1$
$10^0$
NUMBER OF WELLS
40
60
80
100
120
140
160
180
FLUORESCENCE INTENSITY [a.u.]

FIG. 63A

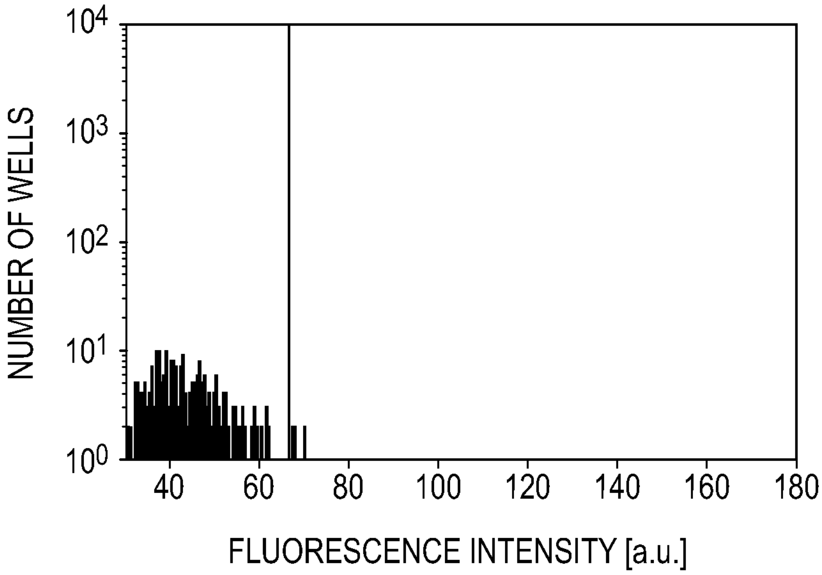
NUMBER OF WELLS
10^4
10^3
10^2
10^1
10^0
40
60
80
100
120
140
160
180
FLUORESCENCE INTENSITY [a.u.]

FIG. 63B

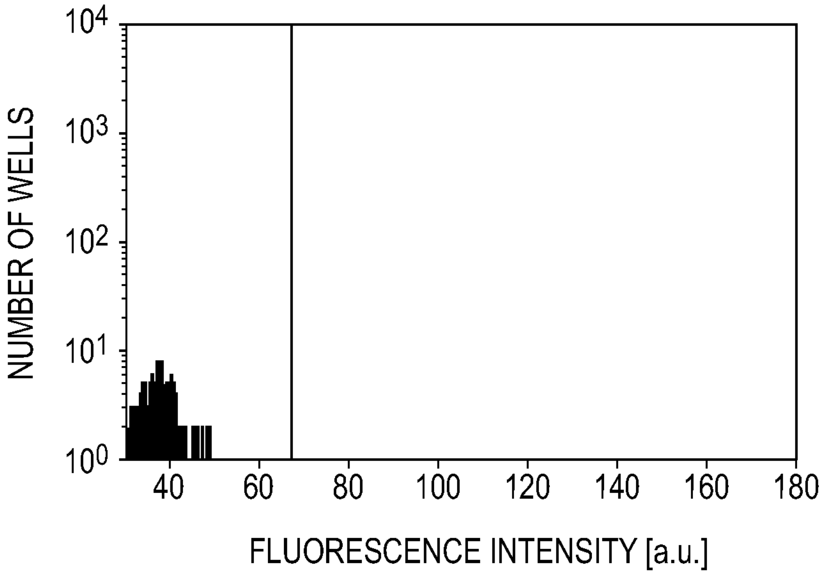
$10^4$
$10^3$
$10^2$
$10^1$
$10^0$
NUMBER OF WELLS
40
60
80
100
120
140
160
180
FLUORESCENCE INTENSITY [a.u.]

FIG. 64A

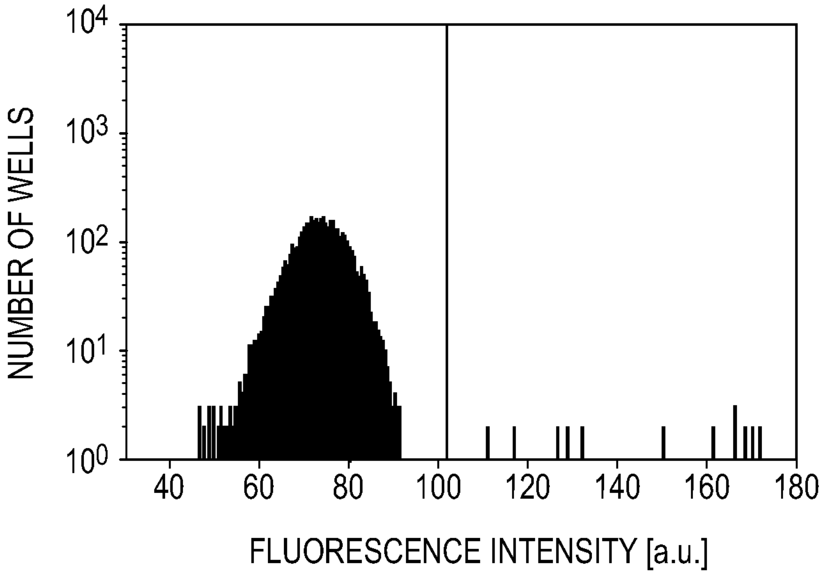
$10^4$
$10^3$
$10^2$
$10^1$
$10^0$
NUMBER OF WELLS
40
60
80
100
120
140
160
180
FLUORESCENCE INTENSITY [a.u.]

FIG. 64B

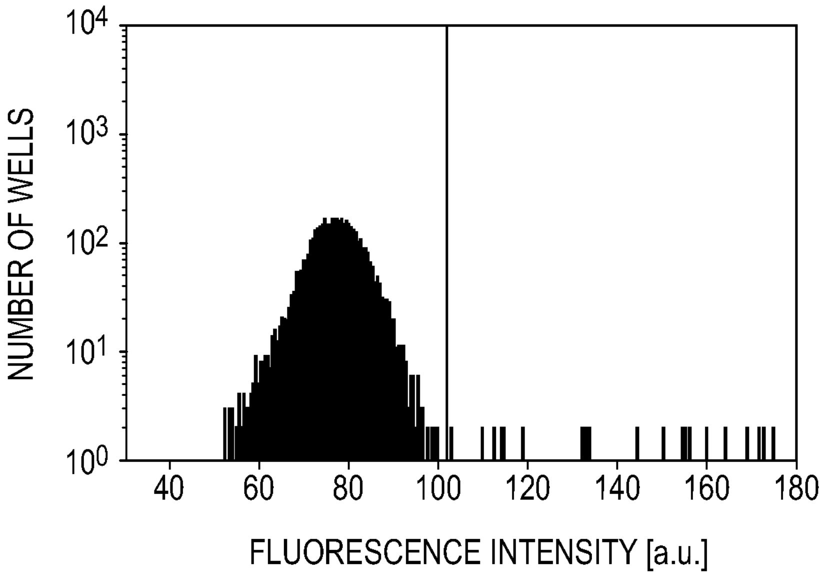
NUMBER OF WELLS
$10^4$
$10^3$
$10^2$
$10^1$
$10^0$
40
60
80
100
120
140
160
180
FLUORESCENCE INTENSITY [a.u.]

FIG. 65A

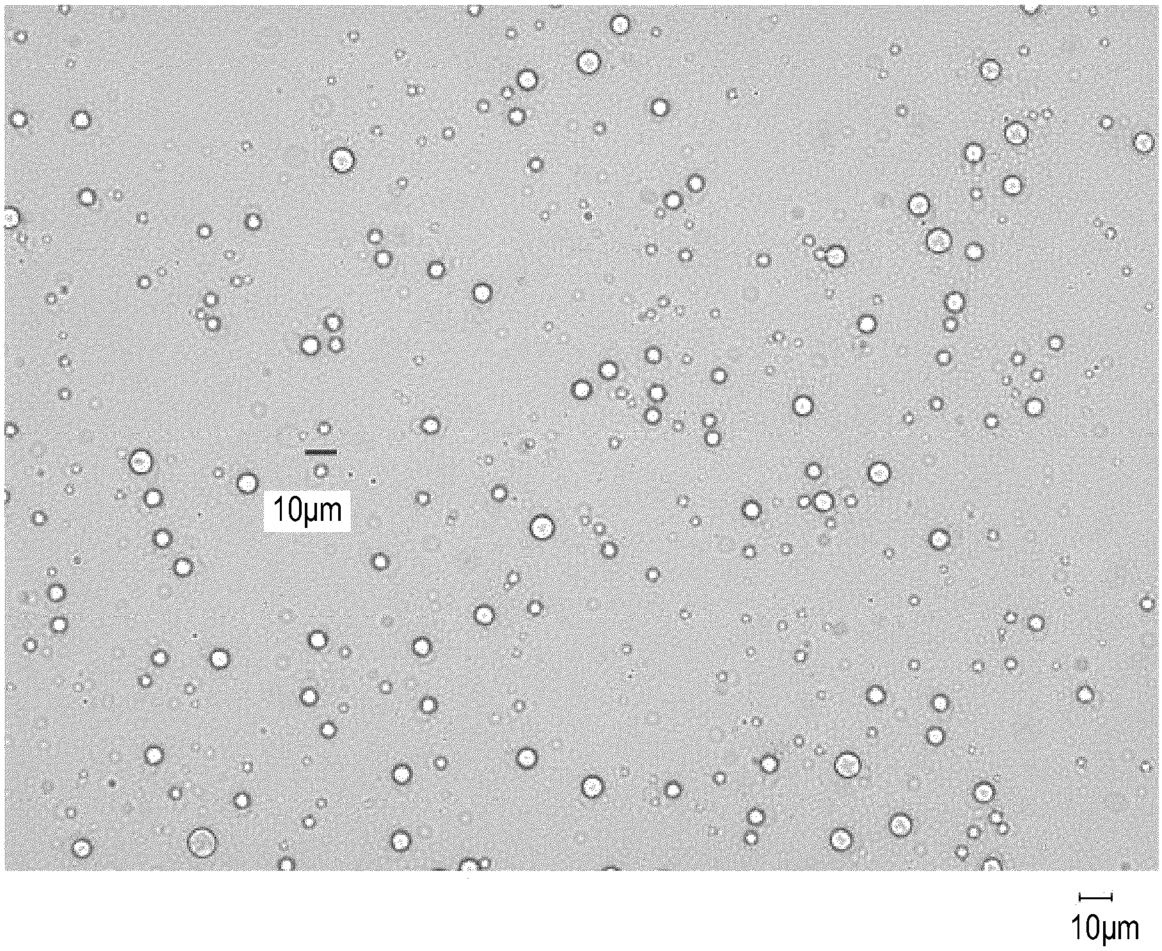

FIG. 65B

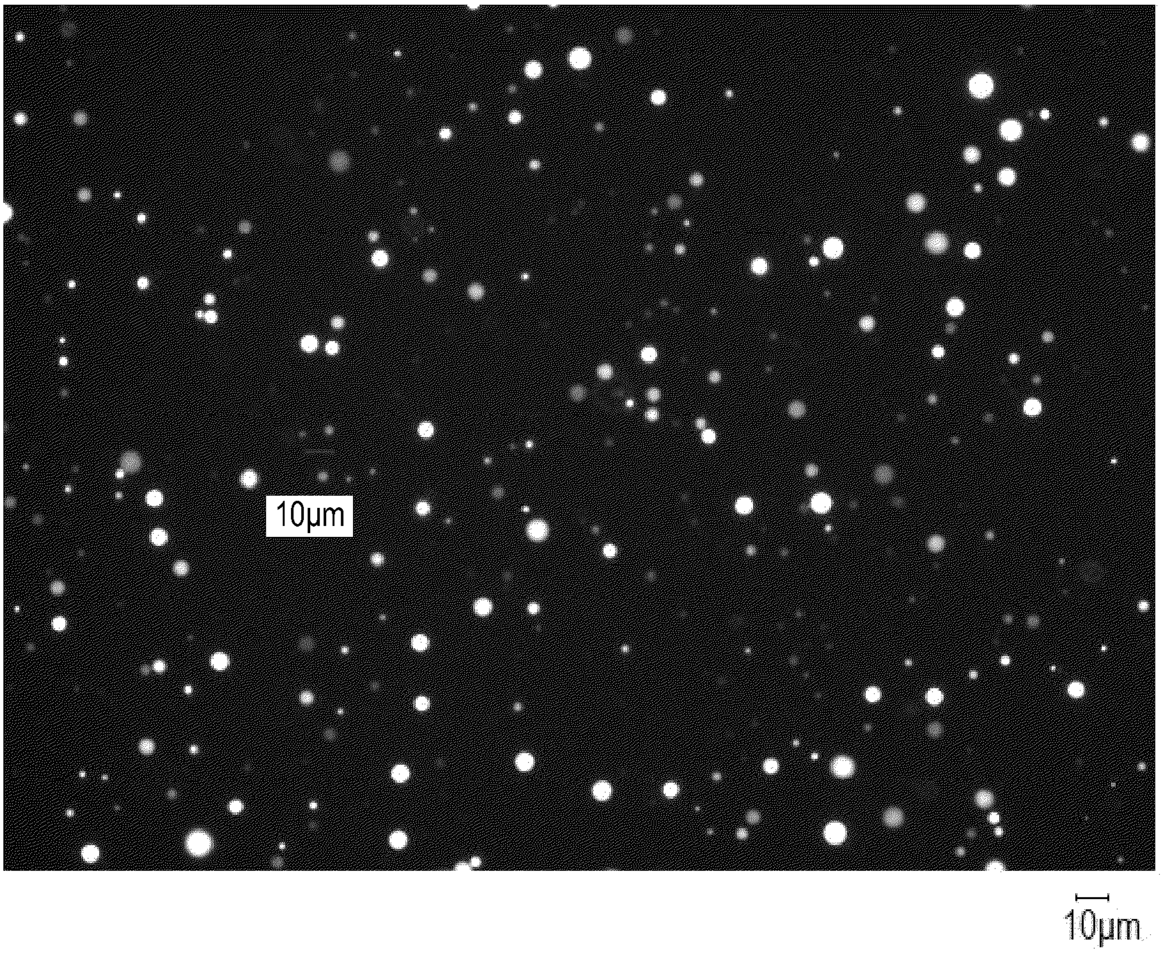

FIG. 65C

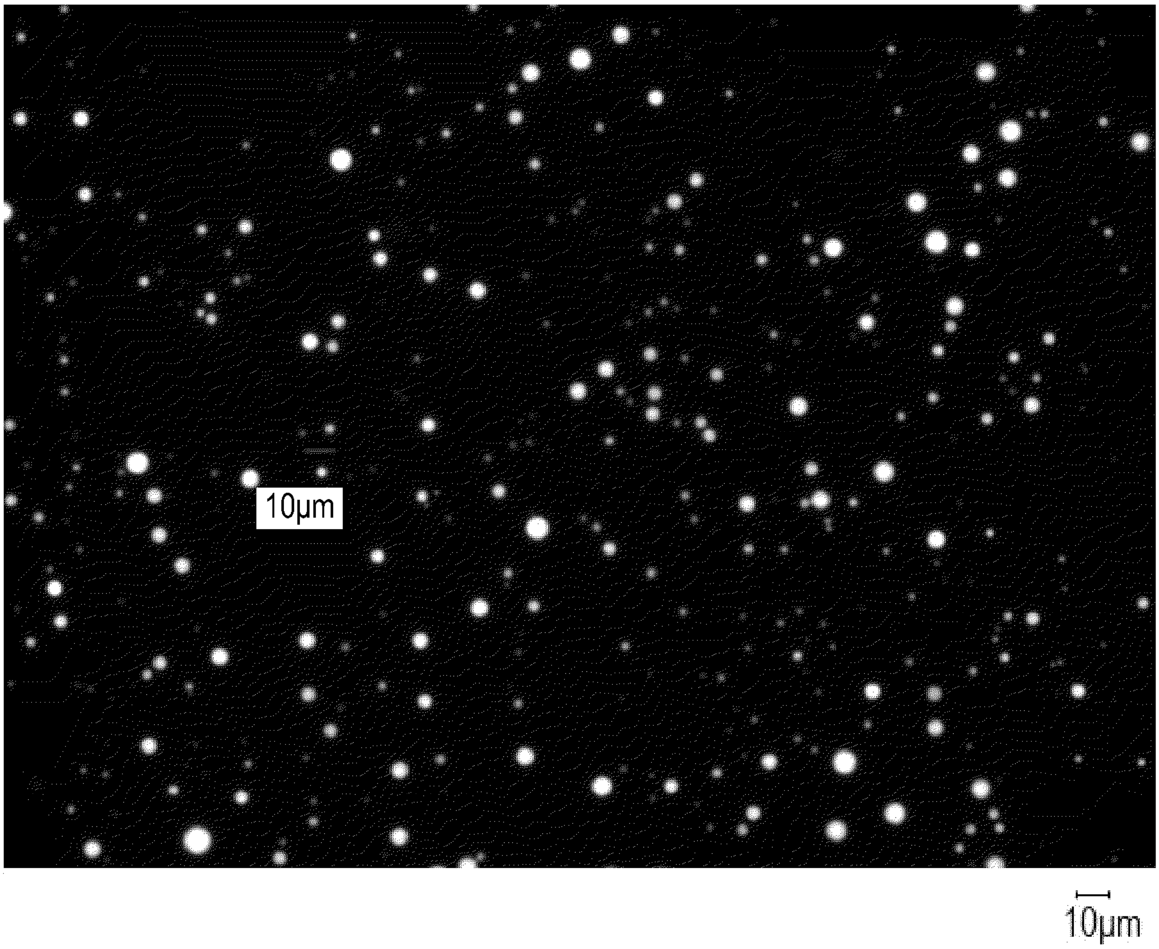

FIG. 66A

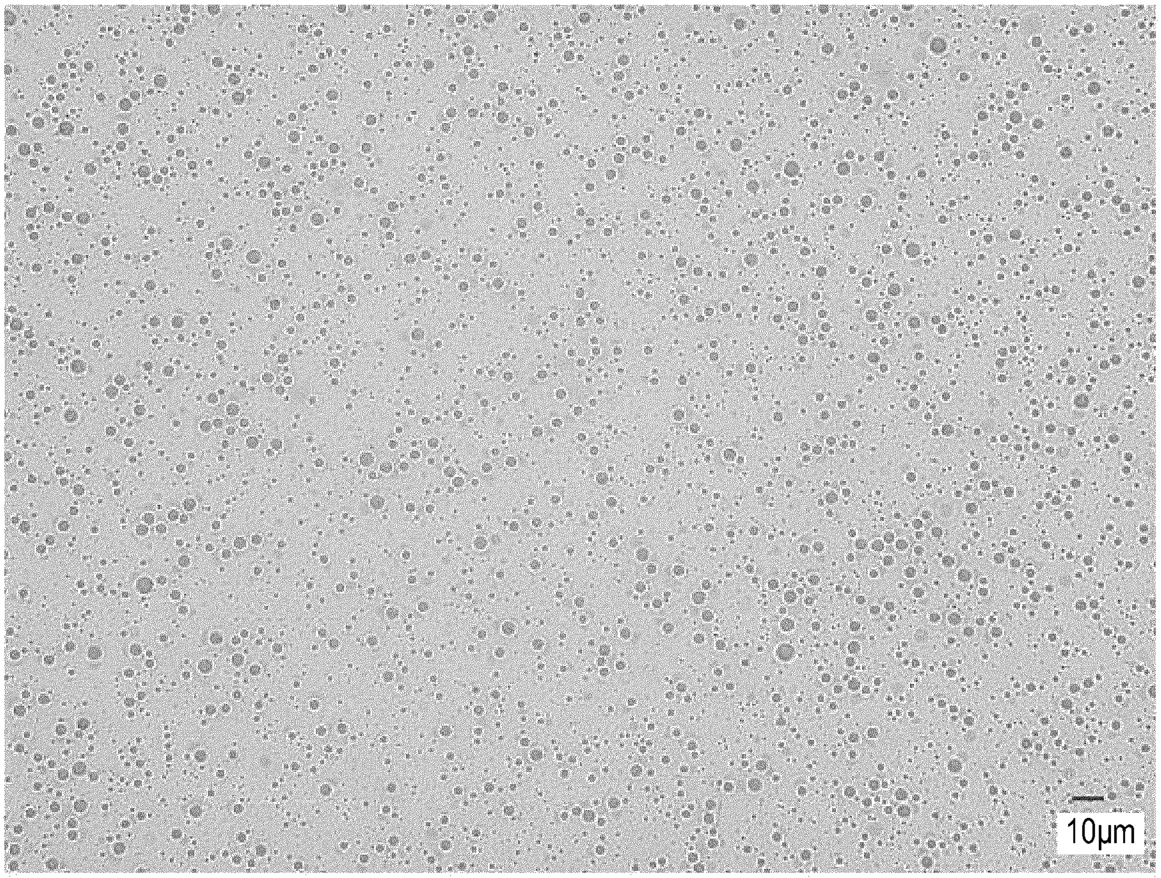


10μm

FIG. 66B

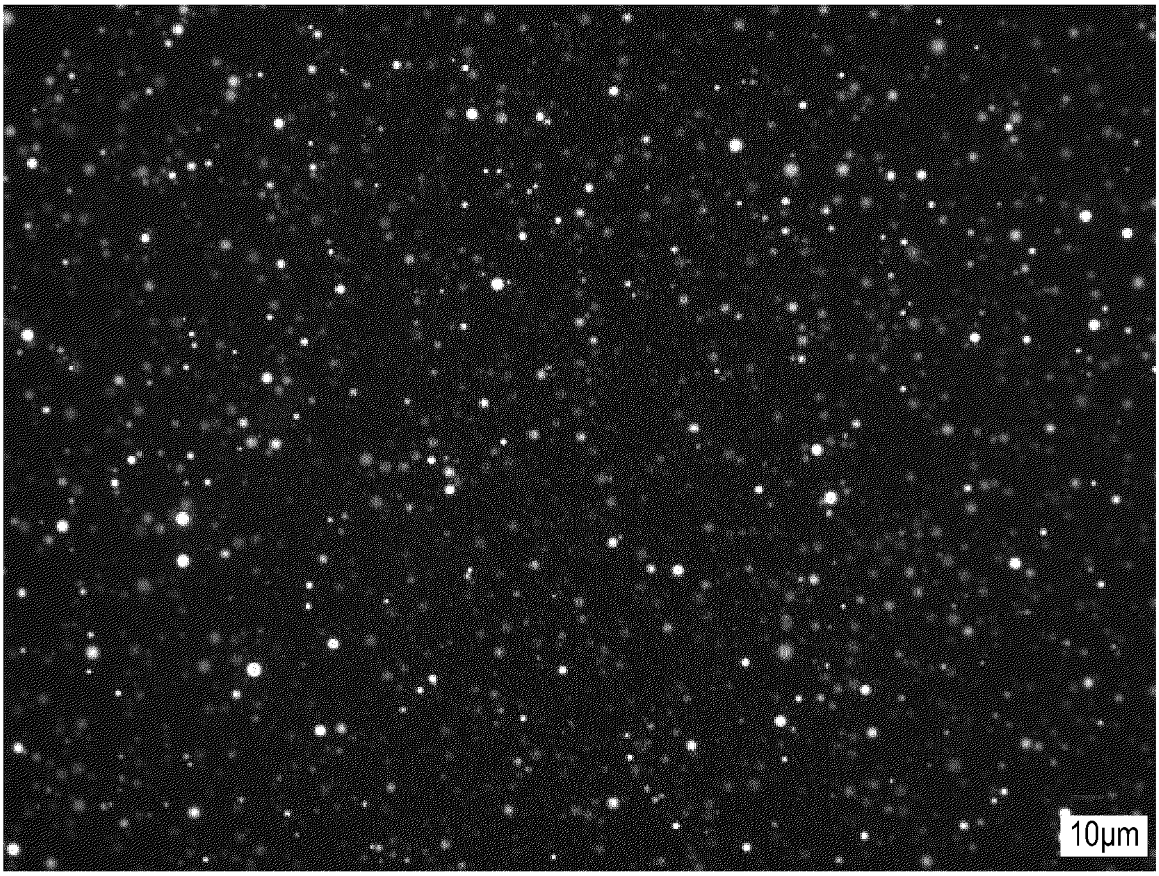


10μm

FIG. 66C

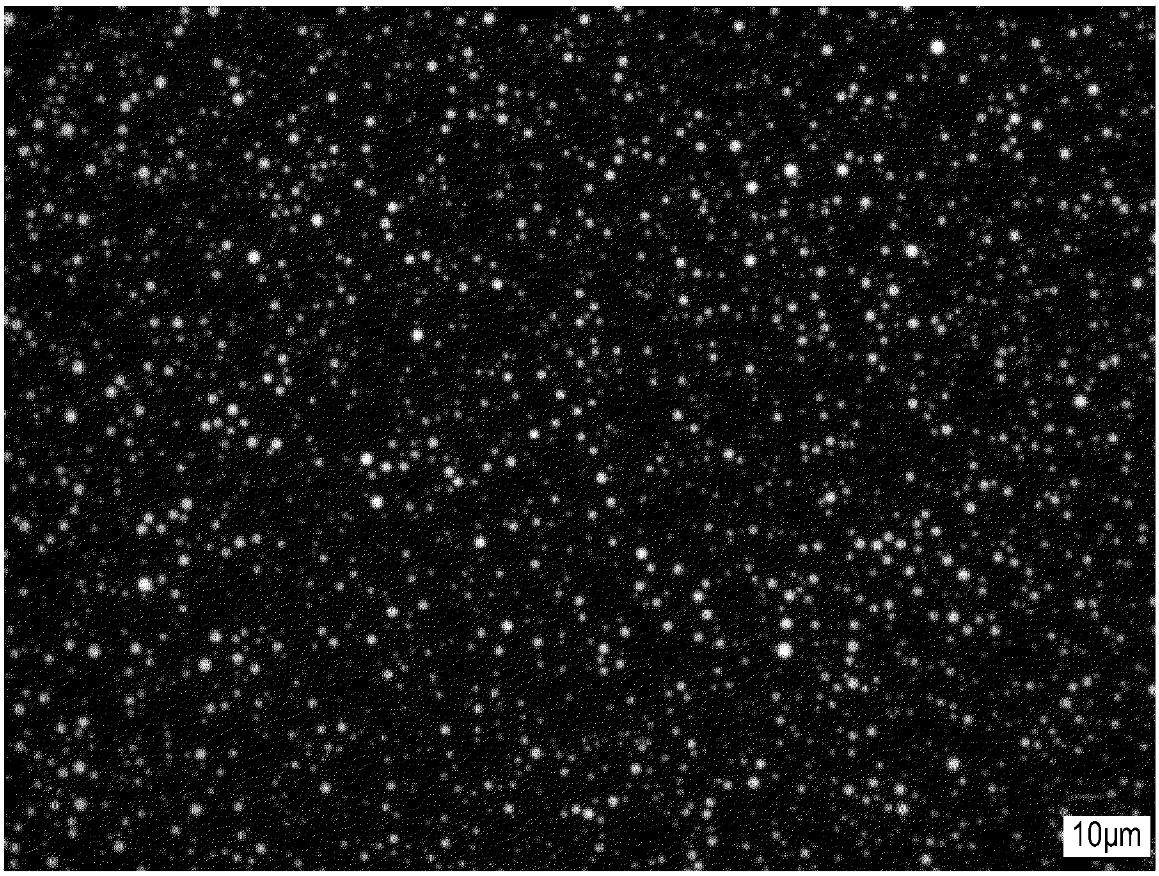


10μm

FIG. 67

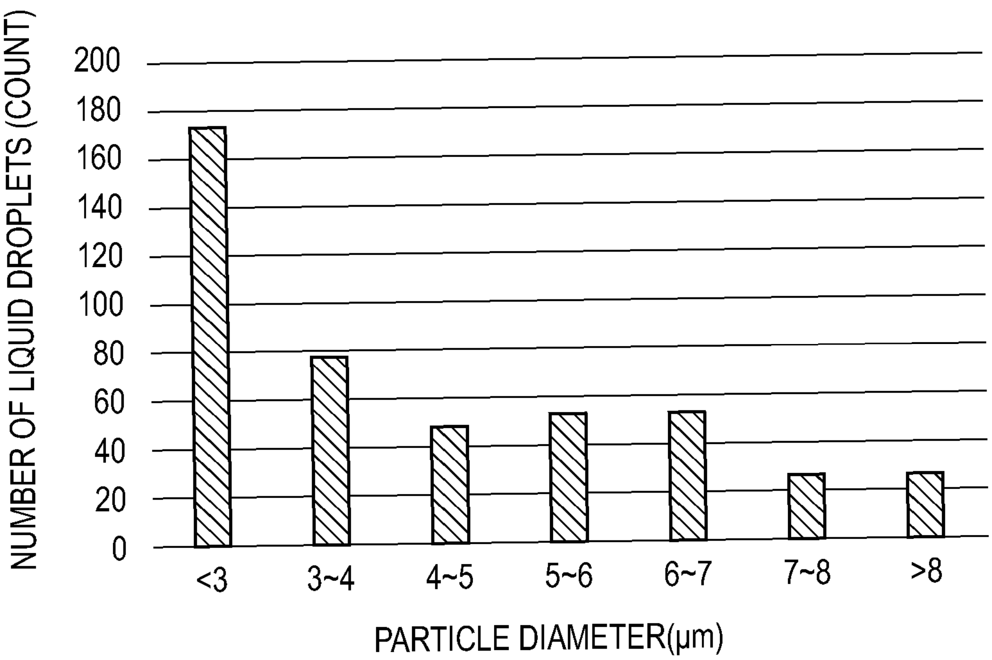
NUMBER OF LIQUID DROPLETS (COUNT)
200
180
160
140
120
100
80
60
40
20
0
<3
3~4
4~5
5~6
6~7
7~8
>8
PARTICLE DIAMETER(μm)

FIG. 68A

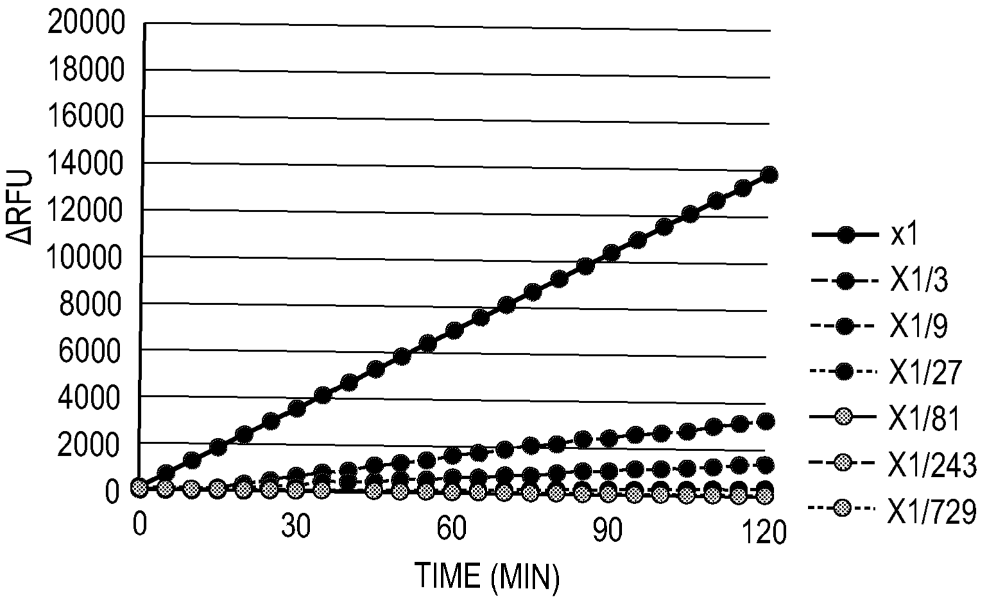
20000
18000
16000
14000
12000
10000
8000
6000
4000
2000
0
ΔRFU
0
30
60
90
120
TIME (MIN)
x1
X1/3
X1/9
X1/27
X1/81
X1/243
X1/729

FIG. 68B

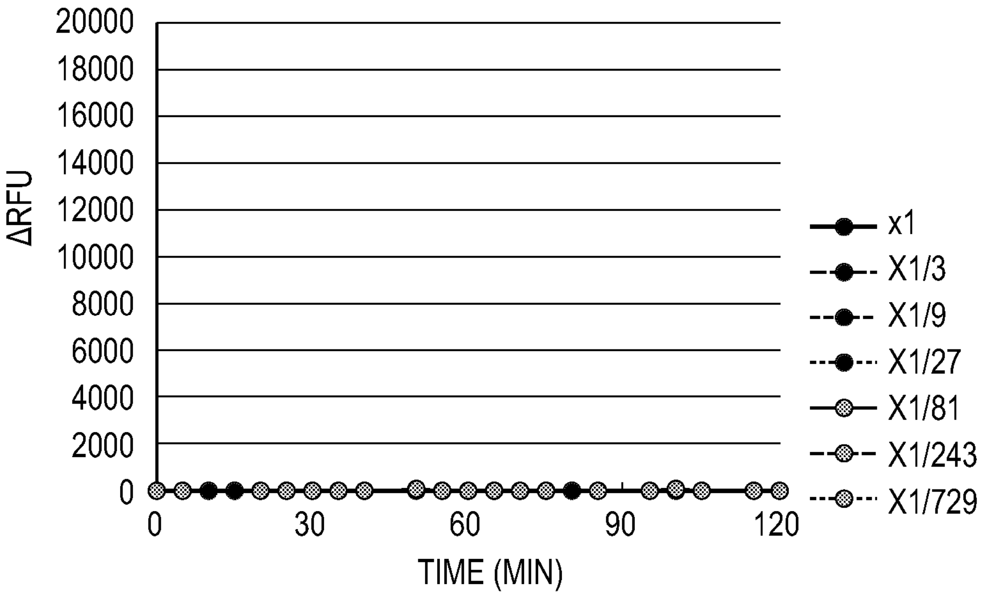
ΔRFU
20000
18000
16000
14000
12000
10000
8000
6000
4000
2000
0
0
30
60
90
120
TIME (MIN)
x1
X1/3
X1/9
X1/27
X1/81
X1/243
X1/729

FIG. 69A

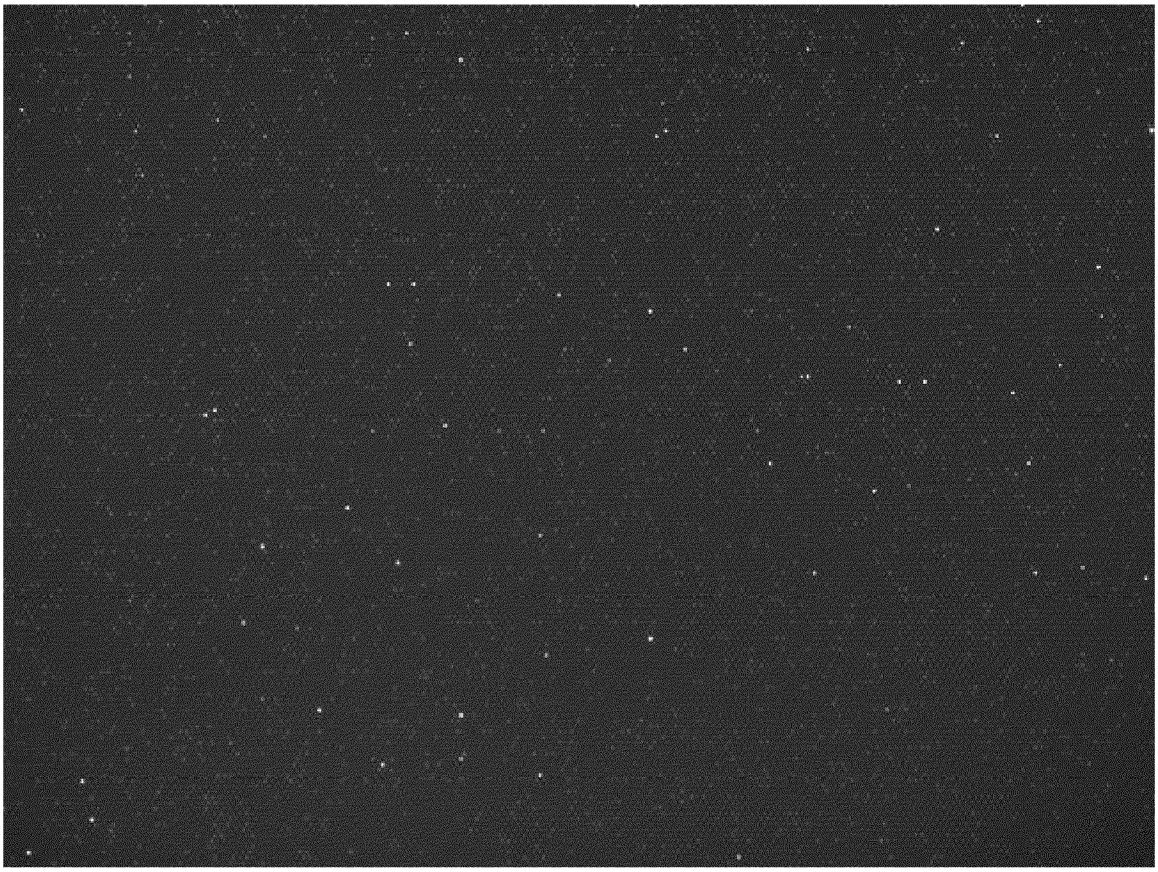

FIG. 69B

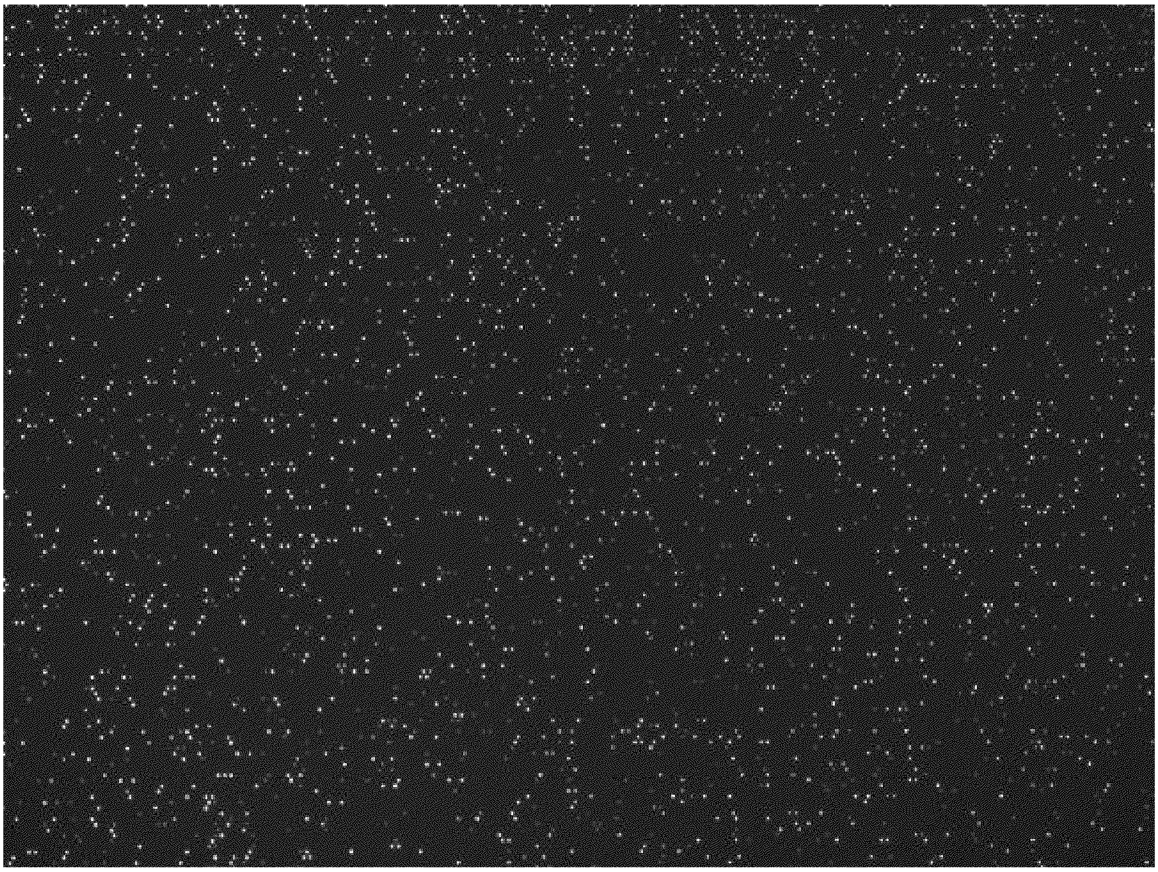

FIG. 69C

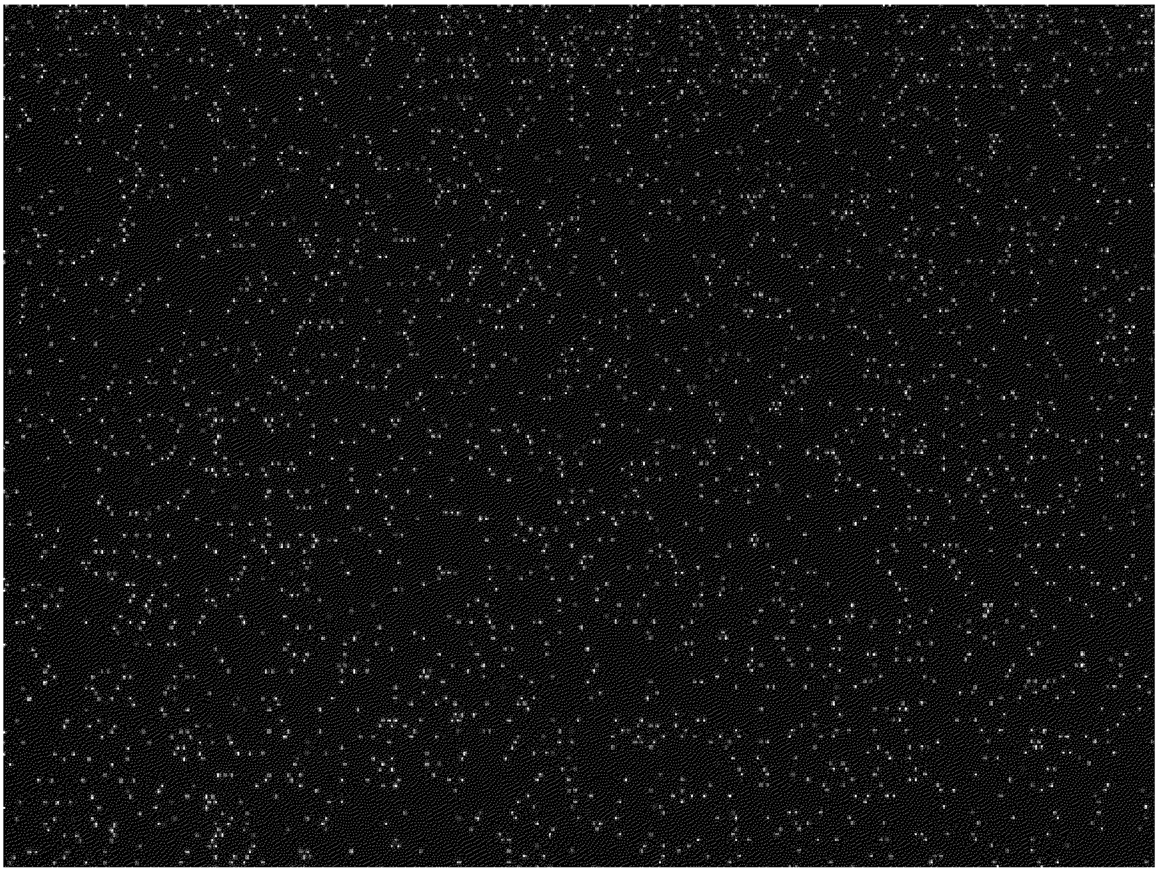

FIG. 70A

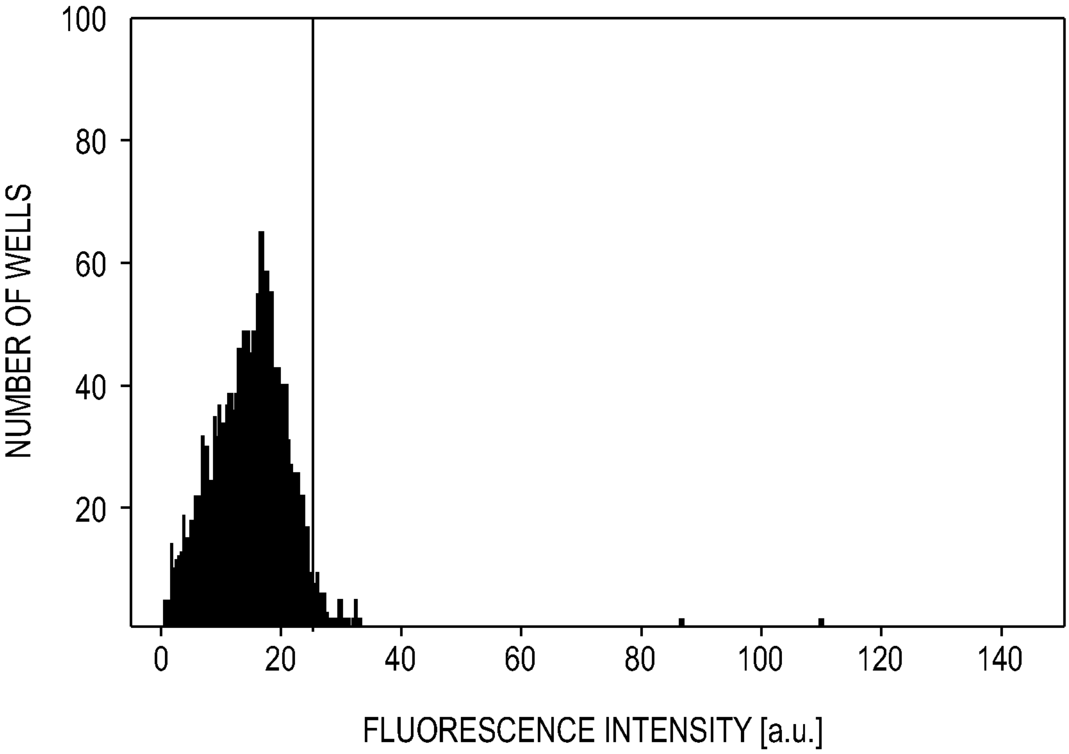
100
80
60
40
20
NUMBER OF WELLS
0
20
40
60
80
100
120
140
FLUORESCENCE INTENSITY [a.u.]

FIG. 70B

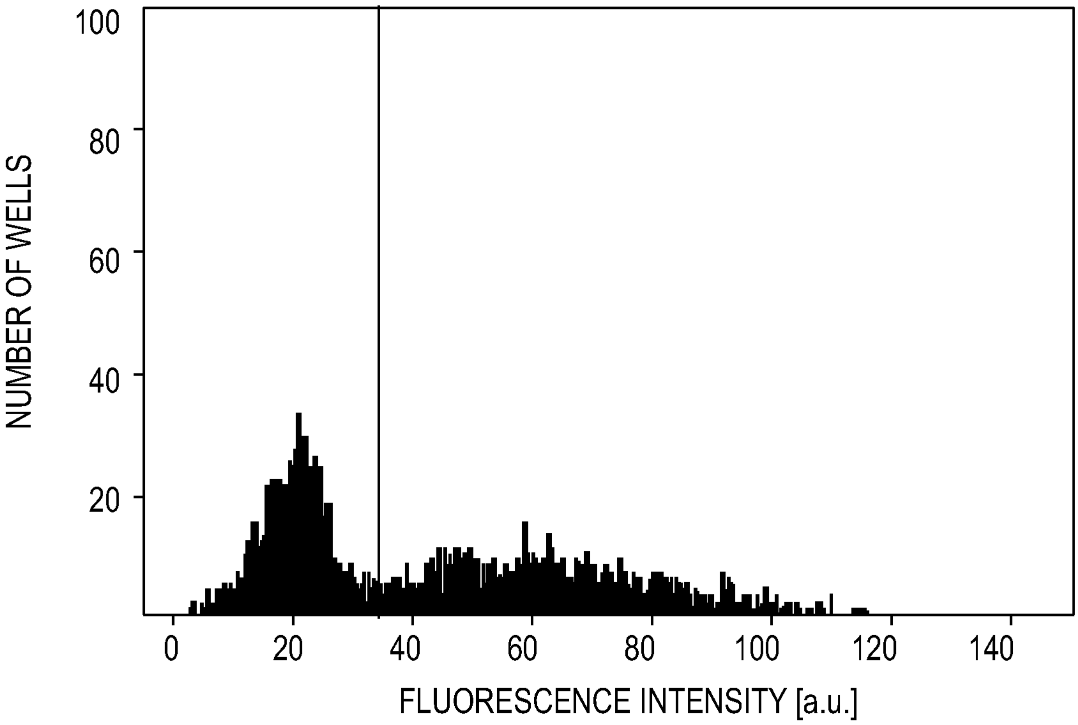
100
80
60
40
20
NUMBER OF WELLS
0
20
40
60
80
100
120
140
FLUORESCENCE INTENSITY [a.u.]

FIG. 71A

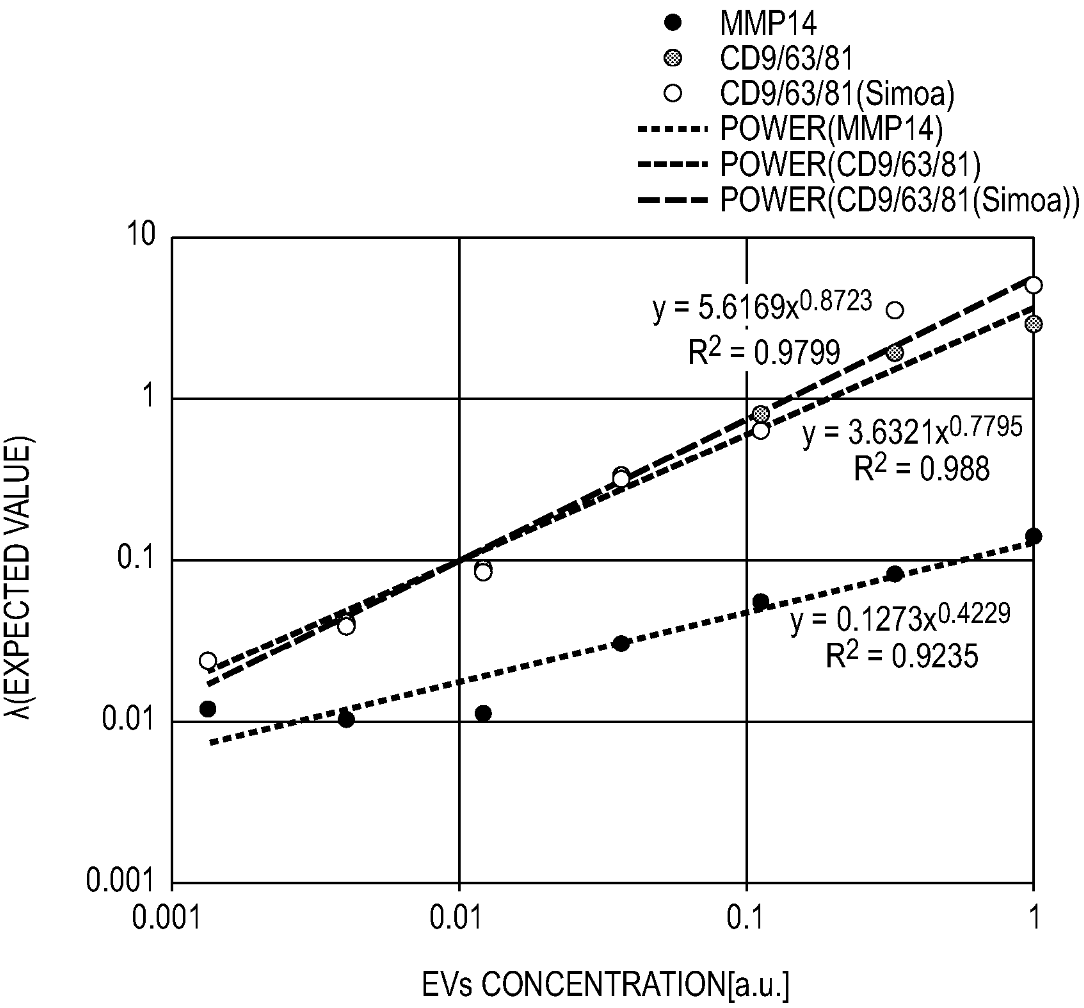
MMP14
CD9/63/81
CD9/63/81(Simoa)
POWER(MMP14)
POWER(CD9/63/81)
POWER(CD9/63/81(Simoa))
10
1
0.1
0.01
0.001
0.001
0.01
0.1
1
y = 5.6169x^0.8723
R^2 = 0.9799
y = 3.6321x^0.7795
R^2 = 0.988
y = 0.1273x^0.4229
R^2 = 0.9235
λ(EXPECTED VALUE)
EVs CONCENTRATION[a.u.]

FIG. 71B

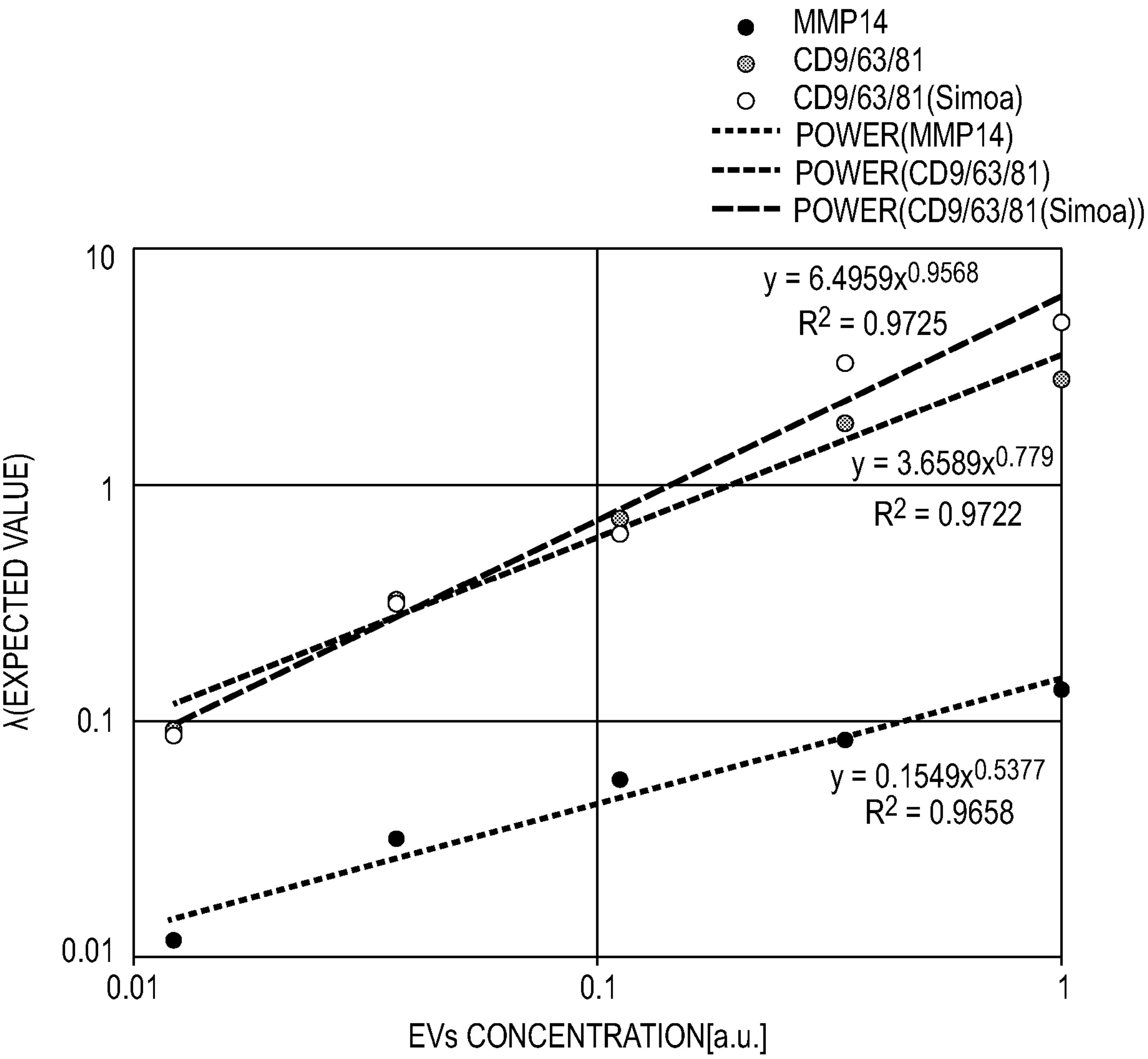
MMP14
CD9/63/81
CD9/63/81(Simoa)
POWER(MMP14)
POWER(CD9/63/81)
POWER(CD9/63/81(Simoa))
y = 6.4959x^0.9568
R^2 = 0.9725
y = 3.6589x^0.779
R^2 = 0.9722
y = 0.1549x^0.5377
R^2 = 0.9658
10
1
0.1
0.01
0.01
0.1
1
λ(EXPECTED VALUE)
EVs CONCENTRATION[a.u.]

FIG. 72

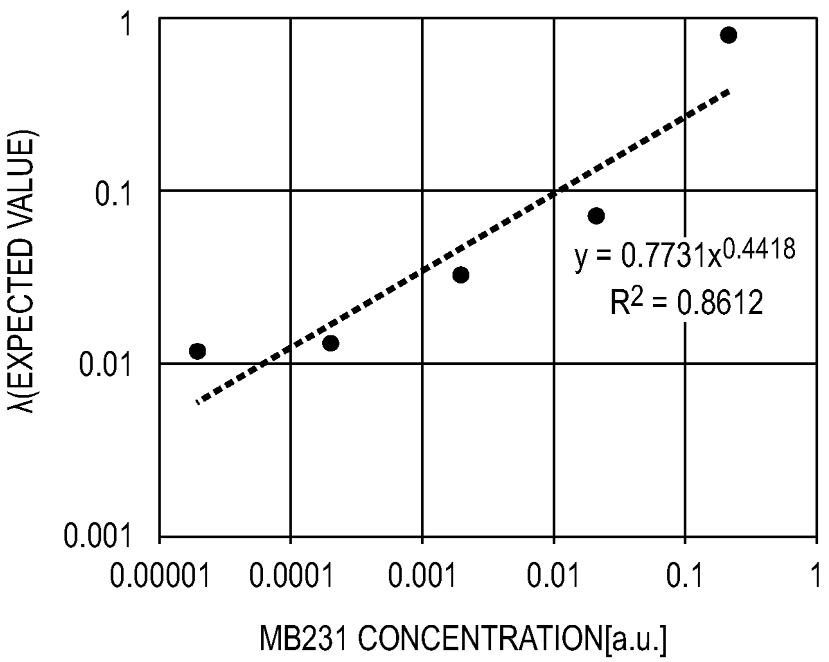
1
0.1
0.01
0.001
λ(EXPECTED VALUE)
y = 0.7731x^0.4418
R2 = 0.8612
0.00001
0.0001
0.001
0.01
0.1
1
MB231 CONCENTRATION[a.u.]

FIG. 73

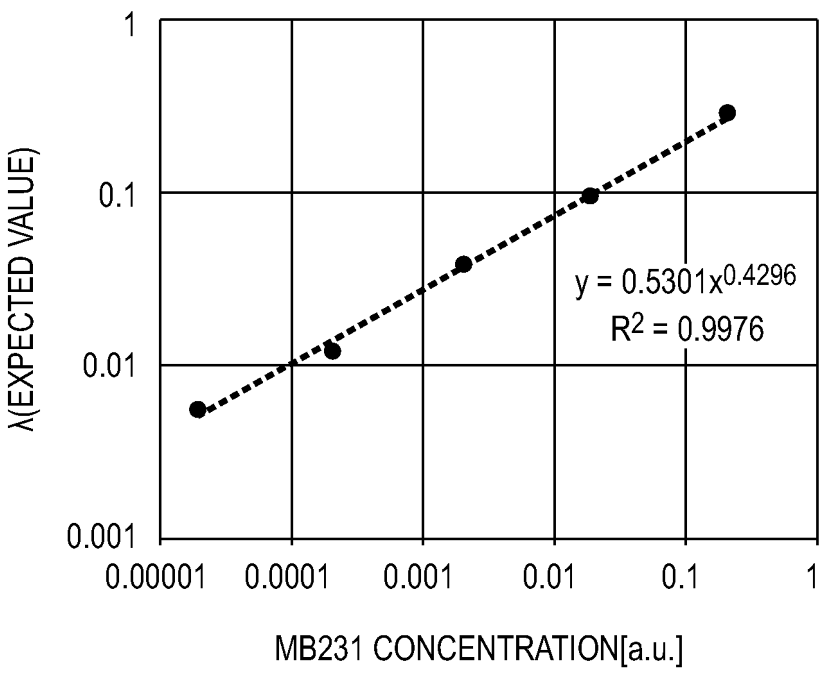
1
0.1
0.01
0.001
λ(EXPECTED VALUE)
y = 0.5301x^0.4296
R2 = 0.9976
0.00001
0.0001
0.001
0.01
0.1
1
MB231 CONCENTRATION[a.u.]

FIG. 74

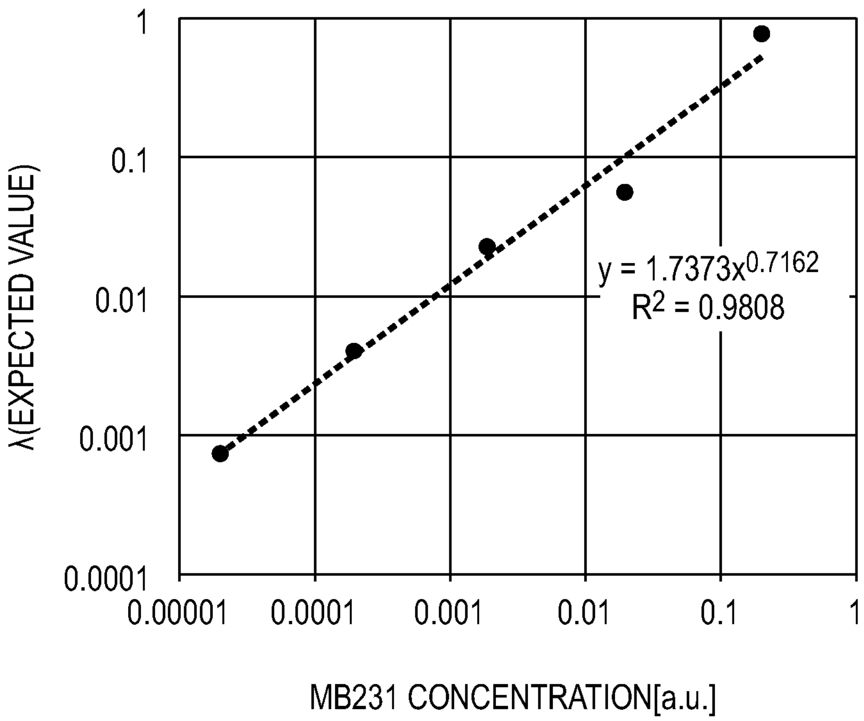
1
0.1
0.01
0.001
0.0001
λ(EXPECTED VALUE)
y = 1.7373x^0.7162
R² = 0.9808
0.00001
0.0001
0.001
0.01
0.1
1
MB231 CONCENTRATION[a.u.]

FIG. 75

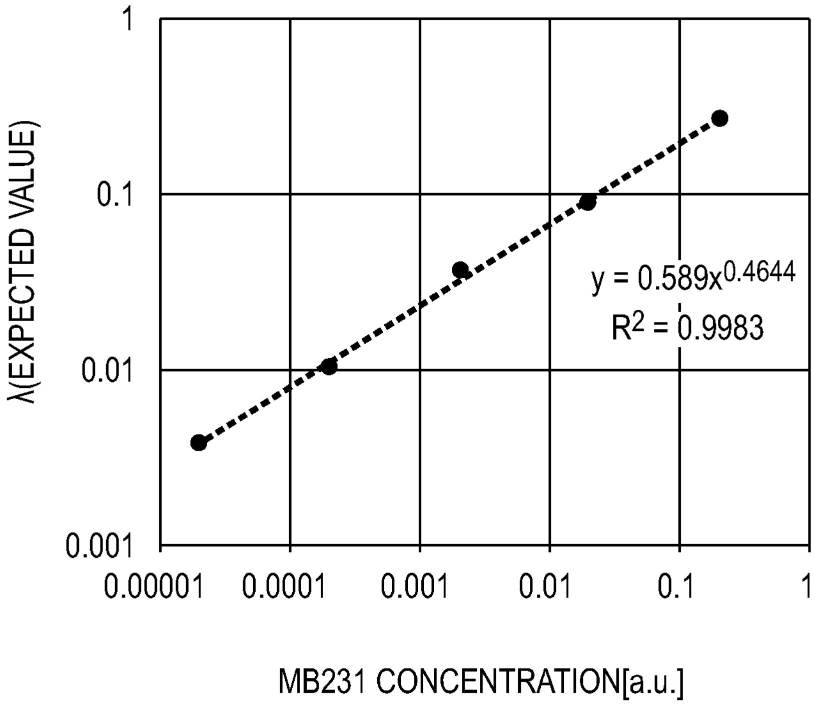
1
0.1
0.01
0.001
λ(EXPECTED VALUE)
y = 0.589x^0.4644
R2 = 0.9983
0.00001
0.0001
0.001
0.01
0.1
1
MB231 CONCENTRATION[a.u.]

FIG. 76

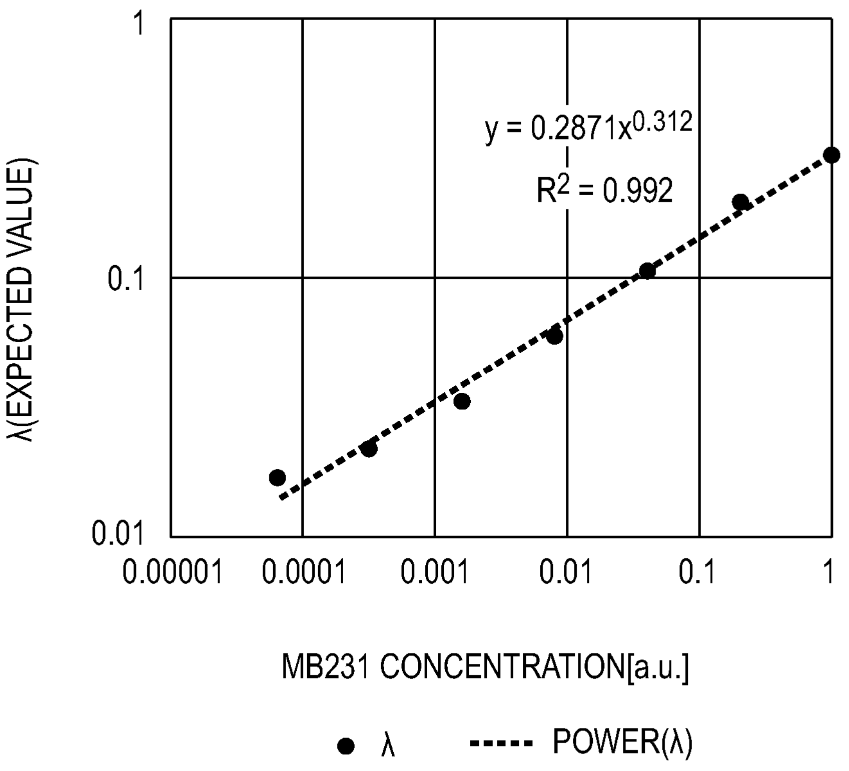
1
0.1
0.01
λ(EXPECTED VALUE)
y = 0.2871x^0.312
R^2 = 0.992
0.00001
0.0001
0.001
0.01
0.1
1
MB231 CONCENTRATION[a.u.]
λ
POWER(λ)

FIG. 77

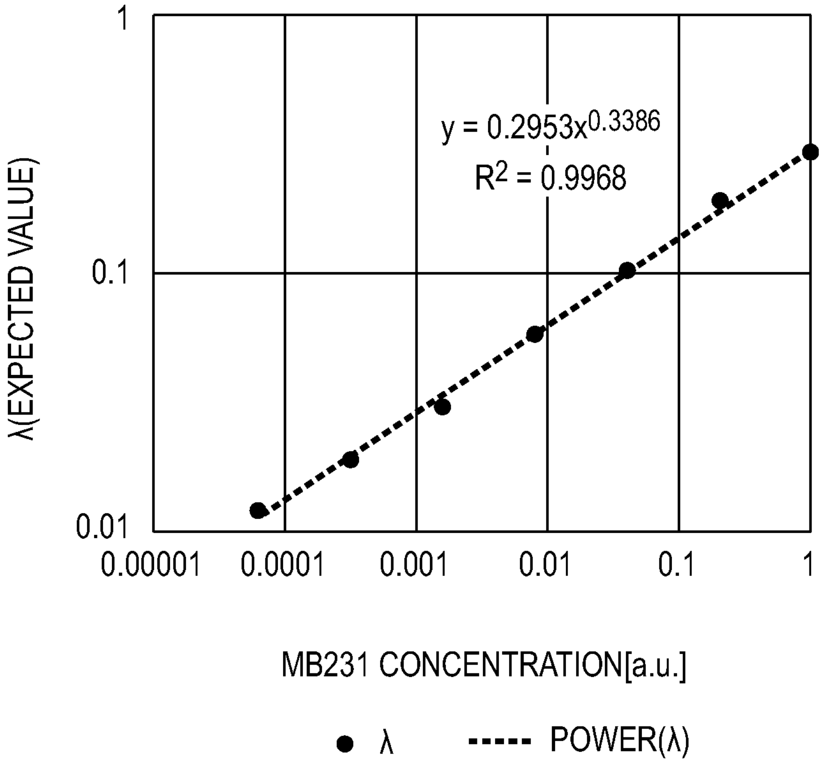
1
0.1
0.01
λ(EXPECTED VALUE)
y = 0.2953x^0.3386
R2 = 0.9968
0.00001
0.0001
0.001
0.01
0.1
1
MB231 CONCENTRATION[a.u.]
λ
POWER(λ)

## INTERNATIONAL SEARCH REPORT

International application No. **PCT/JP2023/043377**

### A. CLASSIFICATION OF SUBJECT MATTER

***C12Q 1/00***(2006.01)i; ***C12M 1/34***(2006.01)i; ***C12Q 1/25***(2006.01)i; ***C12Q 1/37***(2006.01)i; ***C12Q 1/44***(2006.01)i; ***G01N 21/64***(2006.01)i; ***G01N 33/50***(2006.01)i; ***G01N 33/53***(2006.01)i

FI: C12Q1/00 Z; C12M1/34 B; C12M1/34 E; C12Q1/25; C12Q1/37; C12Q1/44; G01N21/64 F; G01N33/50 Z; G01N33/53 D; G01N33/53 S

According to International Patent Classification (IPC) or to both national classification and IPC

### B. FIELDS SEARCHED

Minimum documentation searched (classification system followed by classification symbols)

C12Q1/00; C12M1/34; C12Q1/25; C12Q1/37; C12Q1/44; G01N21/64; G01N33/50; G01N33/53

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); PubMed

### C. DOCUMENTS CONSIDERED TO BE RELEVANT

| Category* | Citation of document, with indication, where appropriate, of the relevant passages | Relevant to claim No. |
|---|---|---|
| X | US 2015/0337356 A1 (FRIEDRICH-ALEXANDER-UNIVERSITAET ERLANGEN-NUERNBERG) 26 November 2015 (2015-11-26) claims 1-6, 13-14, paragraphs [0107], [0161], [0202] | 1-9, 12-31 |
| Y | | 1-31 |
| Y | SANDERSON, R. D. Proteases and glycosidases on the surface of exosomes: Newly discovered mechanisms for extracellular remodeling. Matrix Biol. 2019, vol. 75-76, pp. 160-169 Introduction, Summary and future perspectives | 1-31 |
| A | US 2014/0045915 A1 (THE GENERAL HOSPITAL CORPORATION) 13 February 2014 (2014-02-13) entire text, all drawings | 1-31 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 February 2024** | **27 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2023/043377

C. DOCUMENTS CONSIDERED TO BE RELEVANT

| Category* | Citation of document, with indication, where appropriate, of the relevant passages | Relevant to claim No. |
|---|---|---|
| A | WO 2021/178376 A1 (UNIVERSITY OF KANSAS) 10 September 2021 (2021-09-10) entire text, all drawings | 1-31 |
| A | JP 2022-148530 A (SYSMEX CORPORATION) 06 October 2022 (2022-10-06) entire text, all drawings | 1-31 |
| A | JP 2020-535416 A (ROSETTA EXOSOME) 03 December 2020 (2020-12-03) entire text, all drawings | 1-31 |
| A | EP 4063856 A1 (UNIVERSITEIT ANTWERPEN) 28 September 2022 (2022-09-28) entire text, all drawings | 1-31 |
| A | IRACI, N. et al. Extracellular vesicles are independent metabolic units with asparaginase activity. Nat Chem Biol. 2017, vol. 13, no. 9, pp. 951-955 entire text, all drawings | 1-31 |
| A | FluoroCet FCET96A-1 User Manual. [online]. 2017, [retrieved on 16 February 2024], Retrieved from the Internet: <URL: https://fnkprddata.blob.core.windows.net/domestic/data/datasheet/SBI/FCET96A-1.pdf> entire text, all drawings | 1-31 |

Form PCT/ISA/210 (second sheet) (July 2022)

**INTERNATIONAL SEARCH REPORT**
**Information on patent family members**

International application No.
**PCT/JP2023/043377**

| Patent document cited in search report | Publication date (day/month/year) | Patent family member(s) | Publication date (day/month/year) |
|---|---|---|---|
| US 2015/0337356 A1 | 26 November 2015 | WO 2014/108480 A1 | |
| US 2014/0045915 A1 | 13 February 2014 | WO 2012/031008 A2 | |
| WO 2021/178376 A1 | 10 September 2021 | US 2023/0024611 A1 | |
| JP 2022-148530 A | 06 October 2022 | US 2022/0308057 A1<br>entire text, all drawings<br>EP 4063515 A1 | |
| JP 2020-535416 A | 03 December 2020 | US 2020/0284770 A1<br>entire text, all drawings<br>WO 2019/066501 A1<br>EP 3690434 A1 | |
| EP 4063856 A1 | 28 September 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022524327 A
- JP 2022195994 A
- JP 2023111738 A
- JP 2023203313 A